# EUROPEAN PATENT SPECIFICATION

(11) **EP 4 291 193 B1**
(45) Date of publication and mention of the grant of the patent: **02.12.2026**
(21) Application number: 22752283.6
(22) Date of filing: 09.02.2022
(51) Int. Cl.: A61K 31/506, C07D 401/12, A61P 35/00

(54) **EGFR DEGRADERS AND METHODS OF USE**
EGFR-ABBAUER UND VERFAHREN ZUR VERWENDUNG
AGENTS DE DÉGRADATION D'EGFR ET PROCÉDÉS D'UTILISATION

(30) Priority: 10.02.2021 WO PCT/CN2021/076500
(43) Date of publication of application: 20.12.2023
(73) Proprietor: BeOne Medicines I GmbH, 4051 Basel (CH)
(72) Inventor: LIU, Huaqing, Beijing 102206 (CN); HAN, Songzhe, Beijing 102206 (CN); WANG, Zhiwei, Beijing 102206 (CN); LEI, Bailin, Beijing 102206 (CN)
(74) Representative: Mewburn Ellis LLP
(86) International application number: PCT/CN2022/075651
(87) International publication number: WO 2022/171123

(56) References cited:
- EP-A1- 3 725 771
- EP-A1- 3 778 590
- WO-A1-2017/197046
- WO-A1-2019/042444
- WO-A1-2019/113071
- WO-A1-2019/114770
- WO-A1-2019/133531
- WO-A1-2019/196812
- WO-A1-2020/010210
- WO-A1-2020/010210
- WO-A1-2020/216371
- WO-A1-2021/036922
- WO-A1-2021/173677
- WO-A1-2022/012622
- WO-A1-2022/012622
- WO-A1-2022/012623
- WO-A2-2017/204445
- CA-A1- 3 141 413
- CN-A- 110 684 015
- CN-A- 112 079 866
- XIUYUN SUN ET AL: "PROTACs: great opportunities for academia and industry", SIGNAL TRANSDUCTION AND TARGETED THERAPY, vol. 4, no. 1, 1 December 2019 (2019-12-01), pages 1 - 33, XP055767841, DOI: 10.1038/s41392-019-0101-6

## Description

### FIELD OF THE INVENTION

Disclosed herein are novel bifunctional compounds formed by conjugating EGFR inhibitor moieties with E3 ligase Ligand moieties, which function to recruit targeted proteins to E3 ubiquitin ligase for degradation, and methods of preparation and uses thereof.

### BACKGROUND OF THE INVENTION

Proteolysis targeting chimera (PROTAC) consists of two covalently linked protein-binding molecules: one capable of engaging an E3 ubiquitin ligase, and the other that binds to the protein of interest (POI) a target meant for degradation (Sakamoto KM et al., Proc. Natl. Acad. Sci. 2001, 98: 8554-9.; Sakamoto K. M. et al., Methods Enzymol. 2005; 399:833-847.). Rather than inhibiting the target protein's enzymatic activity, recruitment of the E3 ligase to the specific unwanted proteins results in ubiquitination and subsequent degradation of the target protein by the proteasome. The whole process of ubiquitination and proteasomal degradation is known as the ubiquitin-proteasome pathway (UPP) (Ardley H.et al., Essays Biochem. 2005, 41, 15-30; Komander D. et al., Biochem. 2012, 81, 203-229; Grice G. L. et al., Cell Rep. 2015, 12, 545-553; Swatek K. N. et al., Cell Res. 2016, 26, 399-422). Proteasomes are protein complexes which degrade unneeded, misfolded or abnormal proteins into small peptides to maintain health and productivity of the cells. Ubiquitin ligases, also called an E3 ubiquitin ligase, directly catalyze the transfer of ubiquitin from the E2 to the target protein for degradation. Although the human genome encodes over 600 putative E3 ligases, only a limited number of E3 ubiquitin ligases have been widely applied by small molecule PROTAC technology: cereblon (CRBN), Von Hippel-Lindau (VHL), mouse double minute 2 homologue (MDM2) and cellular inhibitor of apoptosis protein (cIAP) (Philipp O. et al., Chem. Biol. 2017, 12, 2570-2578), recombinant Human Ring Finger Protein 114 (RNF114) (Spradlin, J. N. et al. Nat. Chem. Biol. 2019, 15, 747-755) and DDB1 And CUL4 Associated Factor 16 (DCAF16) (Zhang, X. et al. Nat. Chem. Biol. 2019, 15, 737-746). For example, cereblon (CRBN) forms an E3 ubiquitin ligase complex with damaged DNA binding protein 1 (DDB1) and Cullin-4A (CUL4A) to ubiquitinate a number of other proteins followed by the degradation via proteasomes. (Yi-An Chen, et al., Scientific Reports 2015, 5, 1-13). Immunomodulatory drugs (IMiDs), including thalidomide, lenalidomide, and pomalidomide, function as monovalent promoters of PPIs by binding to the cereblon (CRBN) subunit of the CRL4A^{CRBN} E3 ligase complex and recruiting neosubstrate proteins. (Matyskiela, M. E. et al., Nat Chem Biol 2018, 14, 981-987.) As a consequence, the ability of thalidomide, and its derivatives, to recruit CRBN has been widely applied in proteolysis-targeting chimeras (PROTACs) related studies (Christopher T. et al. ACS Chem. Biol. 2019, 14, 342-347.; Honorine L. et al, ACS Cent. Sci. 2016, 2, 927-934). PROTACs have great potential to eliminate protein targets that are "undruggable" by traditional inhibitors or are non-enzymatic proteins. (Chu TT. et al., Cell Chem Biol. 2016; 23:453-461. Qin C. et al., J Med Chem 2018; 61: 6685-6704. Winter GE. et al., Science 2015;348:1376-1381.) In the recent years, PROTACs as useful modulators promote the selective degradation of a wide range of target proteins have been reported in antitumor studies. (Lu J. et al., Chem Biol. 2015;22(6):755-763; Ottis P. et al., Chem Biol. 2017; 12(4):892-898.; Crews C. M. et al., J Med Chem. 2018; 61(2):403-404; Neklesa T.K. et al., Pharmacol Ther. 2017, 174:138-144.; Cermakova K. et al., Molecules, 2018.23(8).; An S. et al., EBioMedicine, 2018.; Lebraud H. et al., Essays Biochem. 2017;61(5): 517-527.; Sun Y.H. et al., Cell Res. 2018;28:779-81; Toure M. et al., Angew Chem Int Ed Engl. 2016;55(6):1966-1973; Yonghui Sun et al., Leukemia, volume 33, pages2105-2110(2019); Shaodong Liu et al., Medicinal Chemistry Research, volume 29, pages 802-808(2020); and has been disclosed or discussed in patent publications, e.g., US20160045607, US20170008904, US20180050021, US20180072711, WO2002020740, WO2014108452, WO2016146985, WO2016149668, WO2016197032, WO2016197114, WO2017011590, WO2017030814, WO2017079267, WO2017182418, WO2017197036, WO2017197046, WO2017197051, WO2017197056, WO2017201449, and WO2018071606.

Epidermal growth factor receptor (EGFR) that belongs to the ErbB family is a transmembrane receptor tyrosine kinase (RTK), which plays a fundamentally key role in cell proliferation, differentiation, and motility (Y. Yarden, et al., Nat. Rev. Mol. Cell Biol. 2001; 2:127-137.). Homo- or heterodimerization of EGFR and other ErbB family members activates cytoplasmic tyrosine kinase domains to initiate intracellular signaling. Overexpression or activating mutations of EGFR are associated with the development of many types of cancers, such as pancreatic cancer, breast cancer, glioblastoma multiforme, head and neck cancer, and non-small cell lung cancer (Yewale C., et al. Biomaterials. 2013, 34 (34): 8690-8707.). The activating mutations in the EGFR tyrosine kinase domain (L858R mutation and exon-19 deletion) have been identified as oncogenic drivers for NSCLC (Konduri, K., et al. Cancer Discovery 2016, 6 (6), 601-611.). The first-generation EGFR tyrosine kinase inhibitors (EGFR-TKIs) gefitinib and erlotinib have been approved for NSCLC patients with EGFR activation mutations (M. Maemondo, N. Engl. J. Med. 362 (2010) 2380-2388.). Although most patients with EGFR mutant NSCLC respond to these therapies, patients typically develop resistance after an average of one year on treatment. There are several mechanisms of acquired resistance to gefitinib and erlotinib, including a secondary threonine 790 to methionine 790 mutation (T790M), which is also called "gatekeeper" T790M mutation (Xu Y., et al. Cancer Biol Ther. 2010, 9 (8): 572-582.). Therefore, the second-generation EGFR-TKIs afatinib and the third-generation EGFR-TKIs osimertinib (AZD9291) were developed as irreversible EGFR inhibitors that bind to Cys797 for the treatment of patients with T790M mutation. In particular, osimertinib that largely spares WT EGFR has achieved a greater clinical response rate in NSCLC patients with EGFR T790M. However, several recent studies have reported a tertiary Cys797 to Ser797 (C797S) point mutation with osimertinib clinical therapy (Thress KS, et al. Nat. Med. 2015, 21 (6): 560-562.). There is a need for drugs which can overcome EGFR (C797S) resistance obstacle in non-small cell lung cancer (NSCLC). EGFR-Targeting PROTACs serve as a potential strategy to overcome drug resistance mediated by these mutants, which has been disclosed or discussed in patent publications, e.g. WO2018119441, WO2019149922, WO2019183523, WO2019121562 and US20190106417.

Although, a number of EGFR-targeting PROTACs which were designed to degrade EGFR mutant proteins have been published (Zhang H., et al. ACS Med. Chem. Lett. 2022, doi.org/10.1021/acsmedchemlett.1c00645; Qu X., et al. Eur. J. Med. Chem. 2021, 218, 113328.; Zhang X., et al. Eur. J. Med. Chem. 2020, 192, 112199.; Zhang H, et al. Eur. J. Med. Chem. 2020, 189, 112061.; Lu X, Med. Res. Rev. 2018, 38(5):1550-1581. He K., et al. Bioorg. Med. Chem. Lett. 2020, 15, 127167.). Most of the published molecules are based on the first, second, and third generations of EGFR inhibitors. However, there were no data which showed those EGFR-Targeting PROTACs degrading all the main EGFR mutations, Such as Del19, L858R, Del19/T790M, L858R/T790M, Del19/T790M/C797S, L858R/T790M/C797S.

EP372577A1 describes ALK (Anaplastic Lymphoma Kinase) protein degradation agents and anti-tumor applications thereof.

The present application provides novel bifunctional compounds and compositions for the treatment of serious diseases.

### SUMMARY OF THE INVENTION

References to methods of treatment by therapy or surgery or in vivo diagnosis methods disclosed herein are to be interpreted as references to compounds of the present invention for use in those methods.

One objective of the present invention is to provide compounds and derivatives formed by conjugating EGFR inhibitor moieties with E3 ligase Ligand moieties, which function to recruit targeted proteins to E3 ubiquitin ligase for degradation, and methods of preparation and uses thereof.

The following aspects are for illustration only and should not be constructed as limiting the invention.

Aspect 1. A compound of Formula (X): 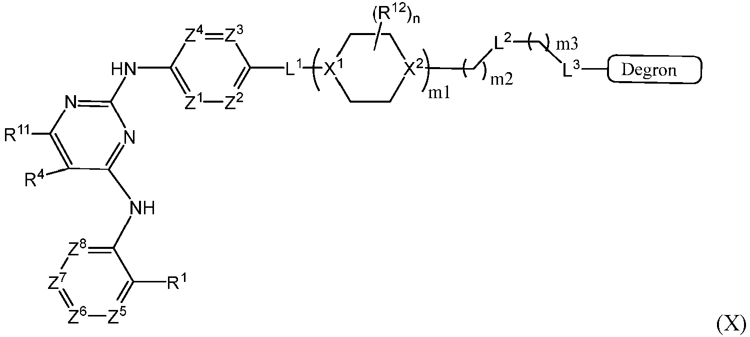 as defined by the claims.

Aspect 2. The compound of Aspect 1, wherein R¹ is selected from -P(O)(CH₃)₂, -NH-SO₂CH₃ or -N(CH₃)-SO₂CH₃.

Aspect 3. The compound of any one of Aspects 1-2, wherein the 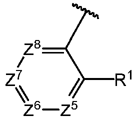 moiety is 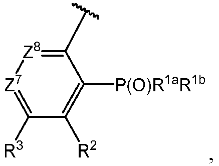 wherein R², R³, Z⁷, Z⁸, R^{1a} and R^{1b} are defined as in Aspect 1.

Aspect 4. The compound of Aspect 3, wherein the 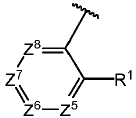 moiety is 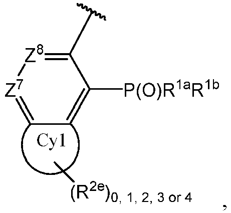 wherein Cy1 is a 5-6 membered aromatic ring, said ring comprising 0-3 heteroatoms independently selected from nitrogen, oxygen or sulfur;
preferable, the 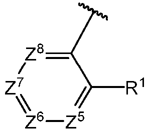 moiety is 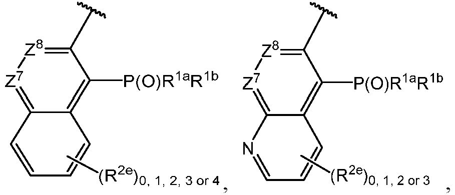 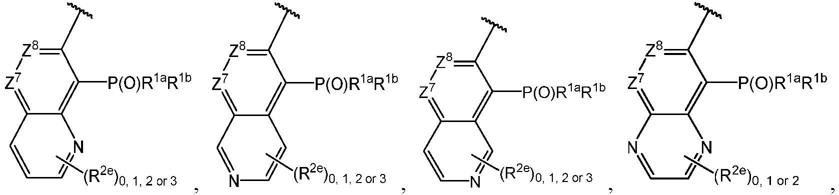 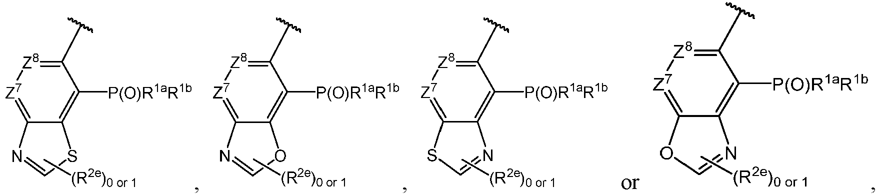
wherein R^{2e}, Z⁷, Z⁸, R^{1a} and R^{1b} are defined as in Aspect 1.

Aspect 5. The compound any one of Aspects 1-4, wherein R^{2e} at each occurrence, is independently hydrogen, -F, -Cl, -Br, -I, -CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CF₃,

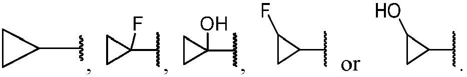

Aspect 6. The compound of one of Aspects 1-5, wherein the 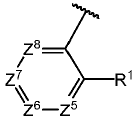 moiety is

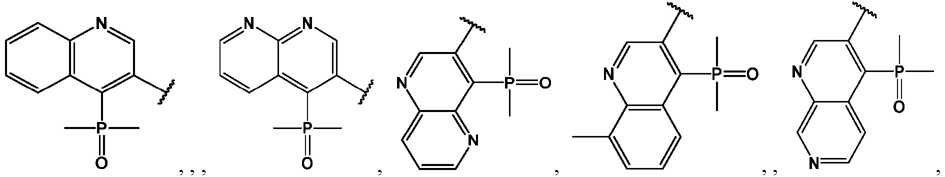

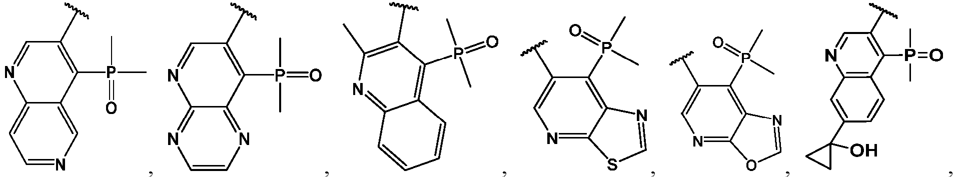

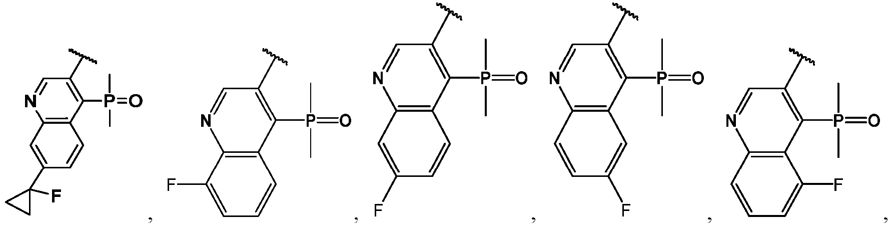

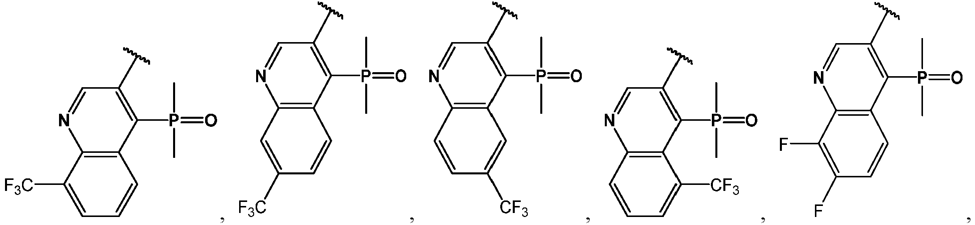

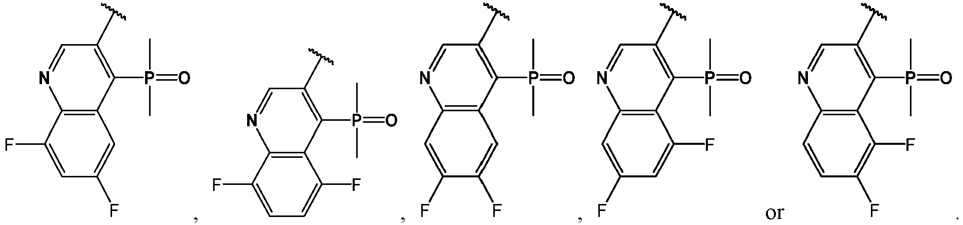

Aspect 7. The compound of any one of Aspects 1-6, wherein R⁹, R¹⁰, R¹¹ and R¹² are each independently selected from hydrogen, F, Cl, Br, -NH₂, -CH₃, -C₂H₅, -C₃H₇, -CH₂F, -CHF₂, -CF₃, -C₄H₉, -C₅H₁₁, -OCH₃, -OC₂H₅, -OC₃H₇, -OC₄H₉, -OC₅H₁₁, -CN, cyclopropyl or oxo; or
two R¹² together with the carbon atoms to which they are attached, form a 3, 4, 5, 6, 7 or 8-membered ring, said ring comprising 0, 1, 2 or 3 heteroatoms independently selected from nitrogen, oxygen or sulfur; said ring is optionally substituted with at least one substituent -H, -F, -Cl, -Br, -I, methyl, ethyl, propyl, butyl, -NH₂, -NHCH₃, -OH, -OCH₃, -OC₂H₅, cyclopropyl, cyclobutyl, cyclopentyl, or cyclohexyl.

Aspect 8. The compound of any one of Aspects 1-7, wherein R⁴ is selected from -H, -F, -Cl, -Br, -I, -CH₃, -C₂H₅, -C₃H₇, -C₄H₉, -C₅H₁₁, -OCH₃, -OC₂H₅, -OC₃H₇, -OC₄H₉, -OC₅H₁₁, cyclopropyl, cyclobutyl, cyclopentyl, cyclohexyl, cycloheptyl, cyclooctyl, phenyl or -CN, wherein each of -CH₃, -C₂H₅, -C₃H₇, -C₄H₉, -C₅H₁₁, -OCH₃, -OC₂H₅, -OC₃H₇, -OC₄H₉, -OC₅H₁₁, cyclopropyl, cyclobutyl, cyclopentyl, cyclohexyl, cycloheptyl, cyclooctyl or phenyl is optionally substituted with -F, -Cl, -Br, -I, -C₁₋₈alkyl, -C₂₋₈alkenyl, -C₂₋₈alkynyl, -C₃-C₈cycloalkyl, 3- to 8-membered heterocyclyl, C₆-C₁₂aryl, 5- to 12-membered heteroaryl, oxo, -CN, -OR^{4c}, -SO₂R^{4c}, -SO₂NR^{4c}R^{4d}, -COR^{4c}, -CO₂R^{4c}, -CONR^{4c}R^{4d}, -NR^{4c}R^{4d}, -NR^{4c}COR^{4d}, -NR^{4c}CO₂R^{4d}, or -NR^{4c}SO₂R^{4d};
R^{4c} and R^{4d} are each independently hydrogen, -C₁₋₈alkyl, -C₂₋₈alkenyl, -C₂₋₈alkynyl, C₃-C₈cycloalkyl, 3- to 8-membered heterocyclyl, C₆-C₁₂aryl, or 5- to 12-membered heteroaryl.

Aspect 9. The compound of any one of Aspects 1-8, wherein R⁴ is selected from -F, -Cl, -Br, -I, -CH₃, -CF₃, -CH₂F, or -CHF₂.

Aspect **10.** The compound of any one of Aspects 1-9, wherein L¹ is selected from a single bond, -O-, -SO₂-, -C(O)-, -NR^{L1a}-, -C₃-C₈cycloalkylene-, *^{L1}-O-C₁₋₈alkylene-**^{L1}, *^{L1}-C₁₋₈alkylene-O-**^{L1}, *^{L1}-SO₂-C₁₋₈alkylene-**^{L1}, *^{L1}-C₁₋₈alkylene-SO₂-**^{L1}, *^{L1}-CO-C₁₋₈alkylene-**^{L1}, *^{L1}-C₁₋₈alkylene-CO-**^{L1}, *^{L1}-NR^{L1a}-C₁₋₈alklene-**^{L1}, *^{L1}-C₁₋₈alkylene-NR^{L1a}-**^{L1} *^{L1}-NR^{L1a}C(O)-**^{L1}, *^{L1}-C(O)NR^{L1a}-**^{L1}, -C₁₋₈alkylene-, -C₂₋₈alkenlene-, -C₂₋₈alkynylene-, -[O(CR^{L1a}R^{L1b})ₘ₄]ₘ₅-, 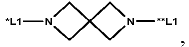 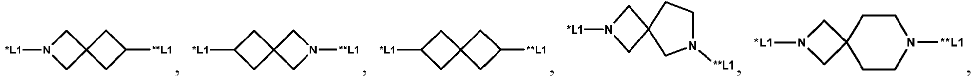 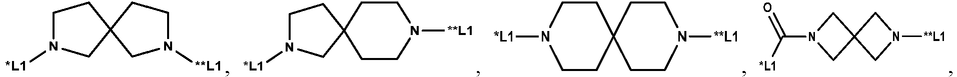 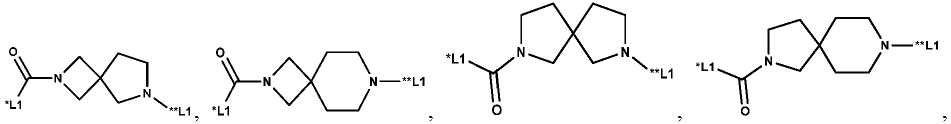 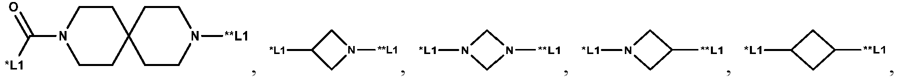 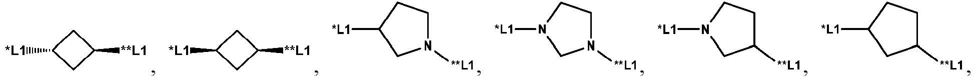 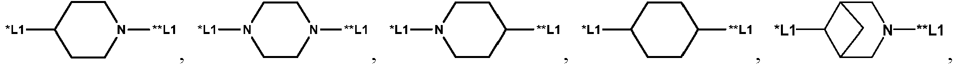 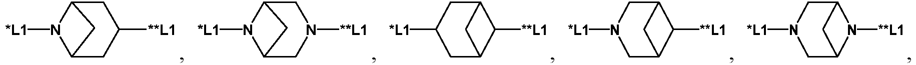 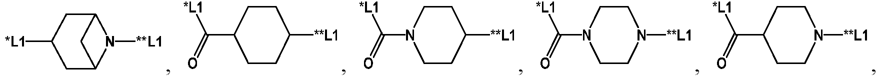 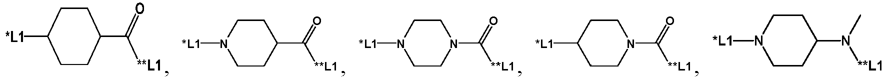 or 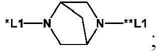
wherein each of said -C₃-C₈cycloalkylene-, *^{L1}-O-C₁₋₈alkylene-**^{L1}, *^{L1}-C₁₋₈alkylene-O-**^{L1}, *^{L1}-SO₂-C₁₋₈alkylene-**^{L1}, *^{L1}-C₁₋₈alkylene-SO₂-**^{L1}, *^{L1}-CO-C₁₋₈alkylene-**^{L1}, *^{L1}-C₁₋₈alkylene-CO-**^{L1}, *^{L1}-NR^{L1a}-C₁₋₈alkylene-**^{L1}, *^{L1}-C₁₋₈alkylene-NR^{L1a}-**^{L1}, -C₁₋₈alkylene-, -C₂₋₈alkenylene-, -C₂₋₈alkynylene-, 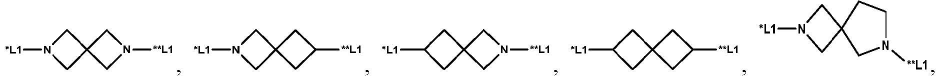 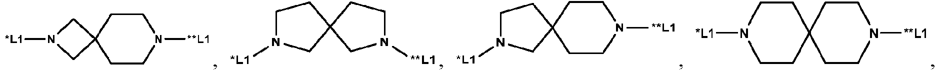 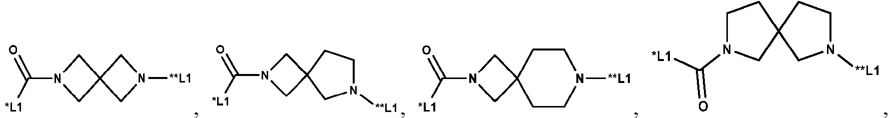 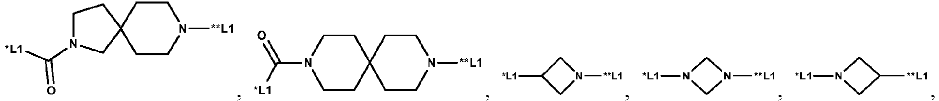 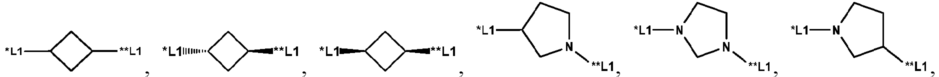 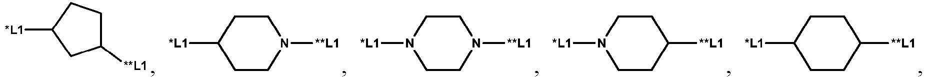 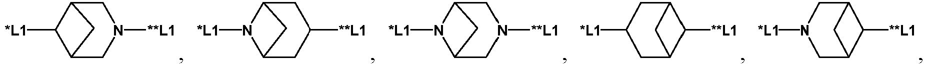 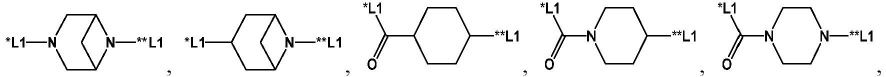 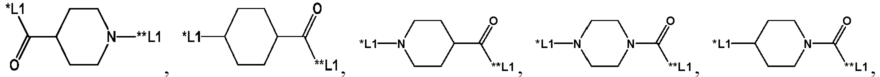 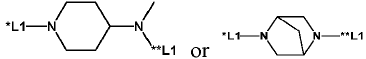 is optionally substituted with at least one R^{L1c};
R^{L1a} and R^{L1b} are each independently selected from hydrogen, methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl or pyrazinyl, wherein each of said methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl or pyrazinyl is optionally substituted with at least one substituent R^{L1d};
each of said R^{L1c} and R^{L1d} are independently -F, -Cl, -Br, -I, -OH, methyl, ethyl, propyl, butyl, pentyl, hexyl, heptyl, octyl, vinyl, ethynyl, methoxy, ethoxy, propoxy, butoxy, pentoxy, hexoxy, heptoxy, octoxy, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl, pyrazinyl or oxo;

Aspect **11.** The compound of any one of Aspects 1-10, wherein L¹ is selected from a single bond,-C₁₋₈alkylene- (preferably -CH₂-, -C₂H₄-, -C₃H₆-), -CO-, -O-, -N(CH₃)-, -NH-,

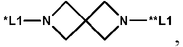

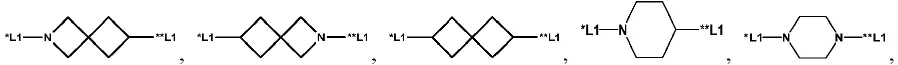

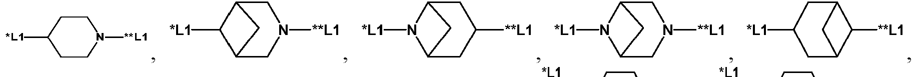

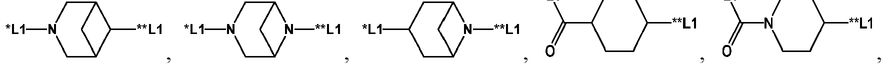

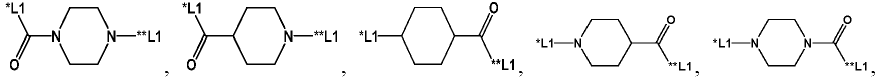

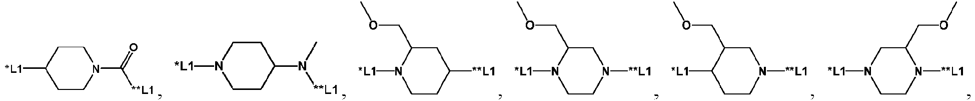

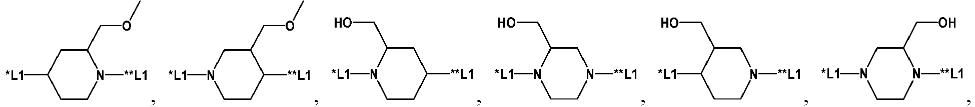

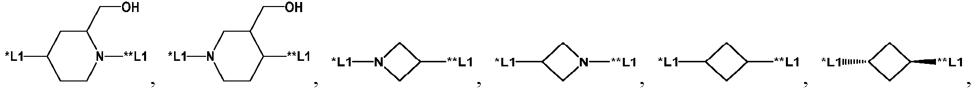

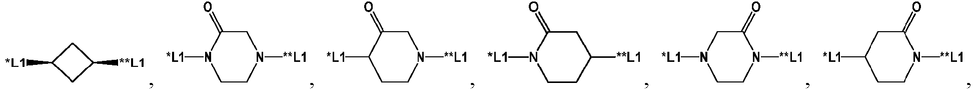

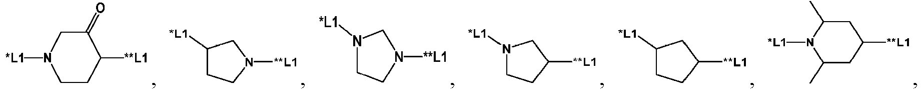

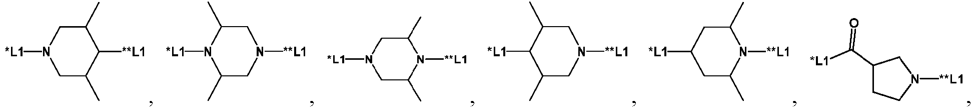

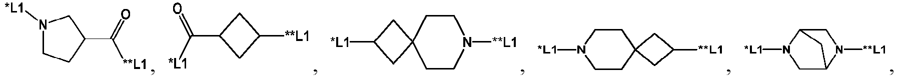

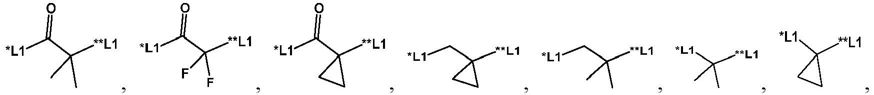

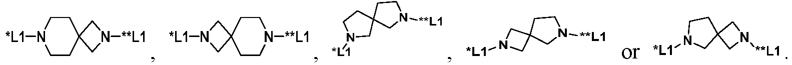

Aspect 12. The compound of any one of Aspects 1-11, wherein X¹ and X² are each independently selected from -CR^{a} or N;
R^{a} is selected from hydrogen, -F, -Cl, -Br, -I, CN, methyl, ethyl, methoxy, ethoxy, or cyclopropyl, wherein each of said methyl, ethyl, methoxy, ethoxy, or cyclopropyl is optionally substituted with at least one substituent -F, -Cl, -Br, -I, hydroxy, methyl, ethyl, (preferably, X¹ and X² are each independently selected from CH, C(F), C(CH₃) or N);
m1=1 or 0;
R¹² is hydrogen, oxo, methoxymethyl, hydroxymethyl, -CN or -CH₃.

**Aspect** 13. The compound of any one of Aspects 1-12, wherein m1 is 1; preferably, 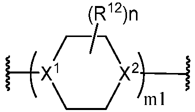 moiety is 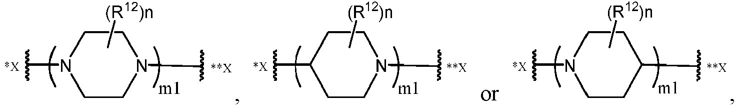 wherein *^{x} refers to the position attached to 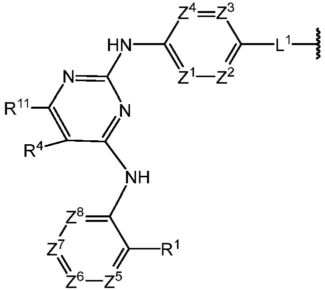 moiety, and **^{x} refers to the position attached to the 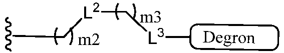 moiety.

Aspect 14. The compound of any one of Aspects 1-13, wherein m1 is 0.

Aspect 15. The compound of any one of Aspects 1-14, wherein 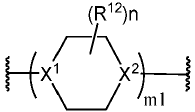 moiety is 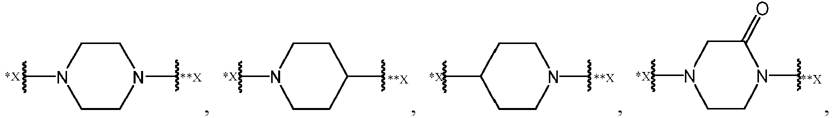 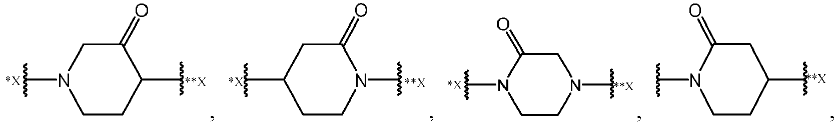 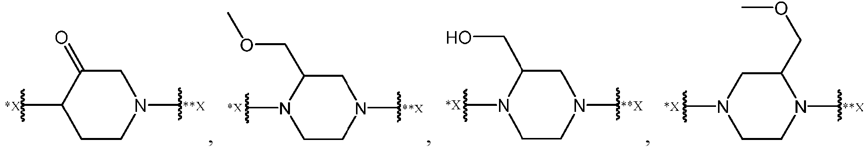 or

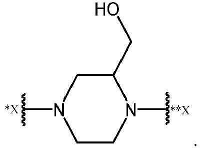

Aspect 16. The compound of any one of Aspects 1-15, wherein m2 is selected from 0, 1, 2, 3, 4 or 5.

Aspect 17. The compound of any one of Aspects 1-16, wherein L² is selected from a single bond, -O-, -SO₂-, -CO-, -NR^{L2a}-, -C₃-C₈cycloalkylene-, *^{L2}-O-C₁₋₈alkylene-**^{L2}, *^{L2}-C₁₋₈alkylene-O-**^{L2}, *^{L2}-SO₂-C₁₋₈alkylene-**^{L2}, *^{L2}-C₁₋₈alkylene-SO₂-**^{L2}, *^{L2}-CO-C₁₋₈alkylene-**^{L2}, *^{L2}-C₁₋₈alkylene-CO-**^{L2}, *^{L2}-NR^{L2a}-C₁₋₈alkylene-**^{L2}, *^{L2}-C₁₋₈alkylene-NR^{L22}-**^{L2}, *^{L2}-NR^{L2a}C(O)-**^{L2}, *^{L2}-C(O)NR^{L2a}-**^{L2}, -C₁₋₈alkylene-, -C₂₋₈alkenylene-, -C₂₋₈alkynylene-, -[O(CR^{L2a}R^{L2b})ₘ₄]ₘ₅-, 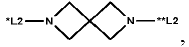 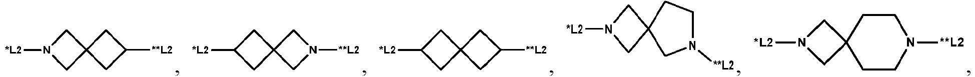 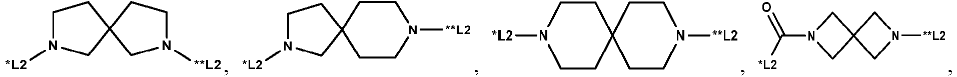 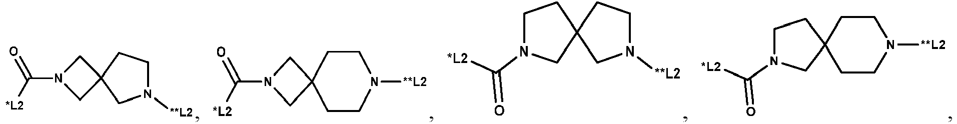 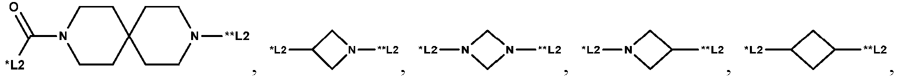 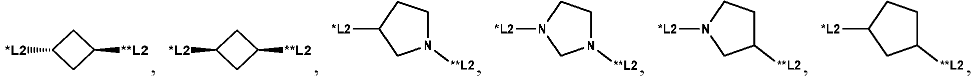 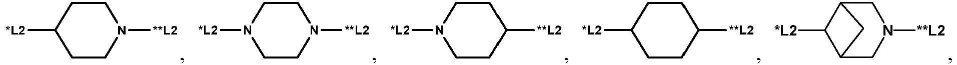 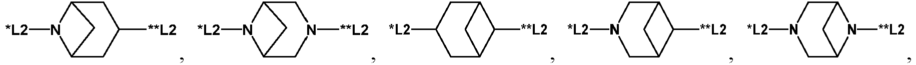 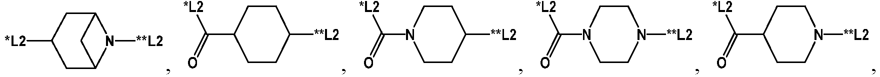 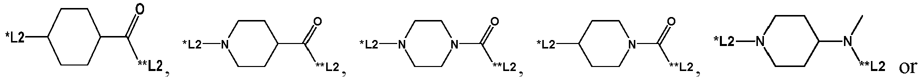 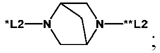
wherein each of said -C₃-C₈cycloalkylene-, *^{L2}-O-C₁₋₈alkylene-**^{L2}, *^{L2}-C₁₋₈alkylene-O-**^{L2}, *^{L2}-SO₂-C₁₋₈alkylene-**^{L2}, *^{L2}-C₁₋₈alkylene-SO₂-**^{L2}, *^{L2}-CO-C₁₋₈alkylene-**^{L2}, *^{L2}-C₁₋₈alkylene-CO-**^{L2}, *^{L2}-NR^{L2a}-C₁₋₈alkylene-**¹², *^{L2}-C₁₋₈alkylene-NR^{L2a}-**^{L2}, -C₁₋₈alkylene-, -C₂₋₈alkenylene-, -C₂₋₈alkynylene-, 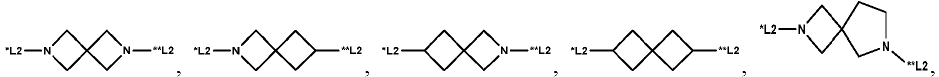 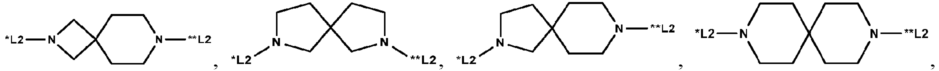 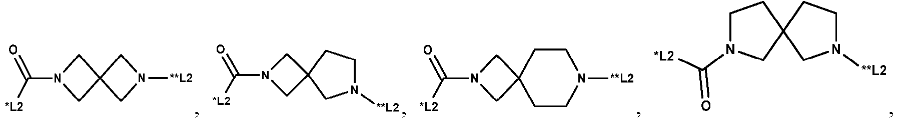 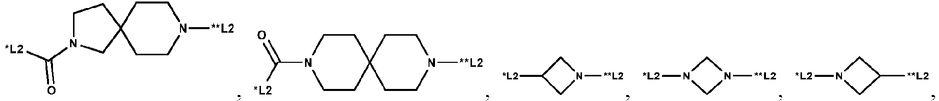 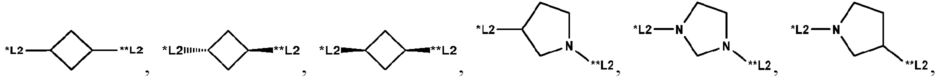 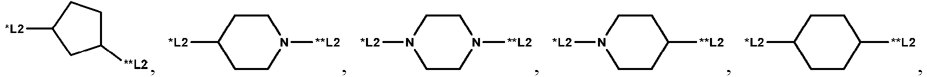 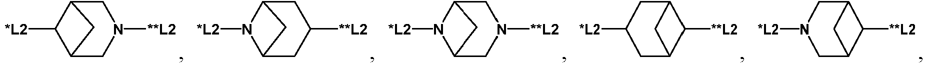 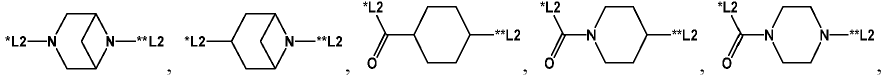 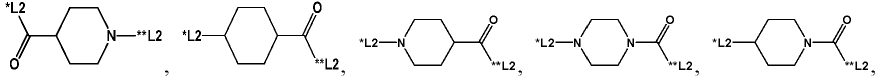 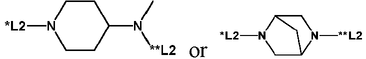 is optionally substituted with at least one substituent R^{L2c};
R^{L2a} and R^{L2b} are each independently selected from hydrogen, methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl or pyrazinyl, wherein each of said methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl or pyrazinyl is optionally substituted with at least one substituent R^{L2d};
each of said R^{L2c} and R^{L2d} are independently -F, -Cl, -Br, -I, -OH, methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl, pyrazinyl or oxo;

Aspect **18.** The compound of any one of Aspects 1-17, wherein L² is selected from a single bond,-C₁₋₈alkylene-(preferably -CH₂-, -C₂H₄-, -C₃H₆-), -CO-, -O-, -N(CH₃)-, -NH-,

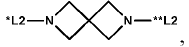

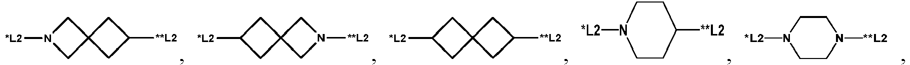

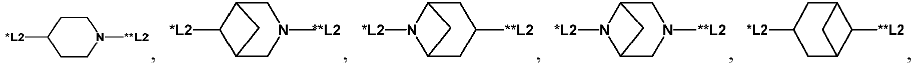

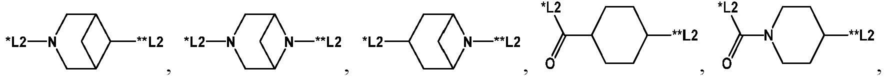

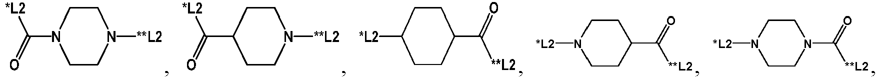

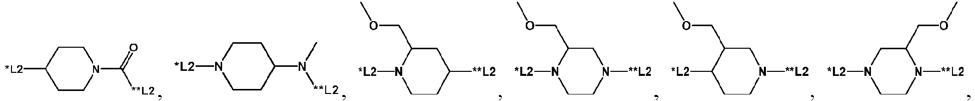

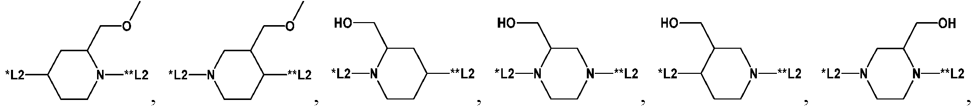

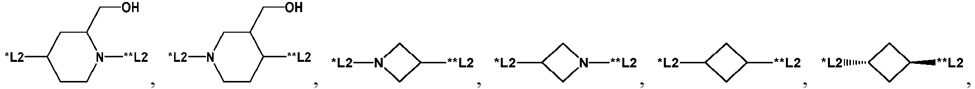

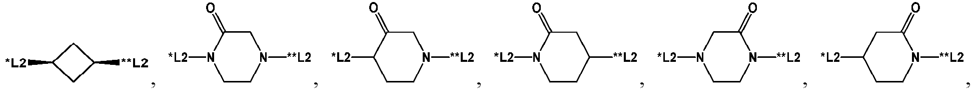

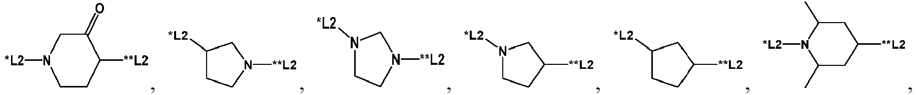

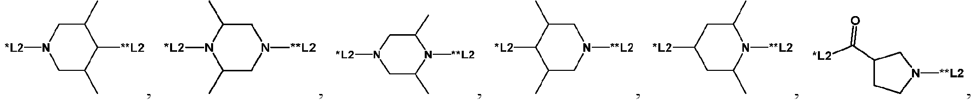

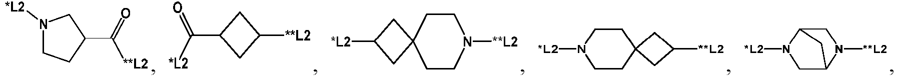

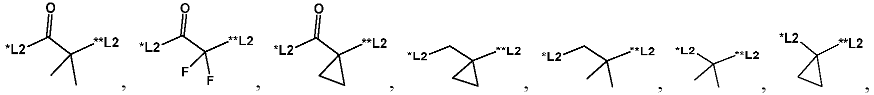

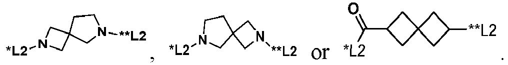

Aspect 19. The compound of any one of Aspects 1-18, wherein m3 is 0, 1, 2, 3, 4, 5 or 6.

Aspect 20. The compound of any one of Aspects 1-19, wherein L³ is selected from a single bond, -O-, -SO₂-, -CO-, -NR^{L3a}-, -C₃-C₈cycloalkylene-, *^{L3}-O-C₁₋₈alkylene-**^{L3}, *^{L3}-C₁₋₈alkylene-O-**^{L3}, *^{L3}-SO₂-C₁₋₈alkylene-**^{L3}, *^{L3}-C₁₋₈alkylene-SO₂-**^{L3}, *^{L3}-CO-C₁₋₈alkylene-**^{L3}, *^{L3}-C₁₋₈alkylene-CO-**^{L3}, *^{L3}-NR^{L3a-}C₁₋₈alkylene-**^{L3}, *^{L3-}C₁₋₈alkylene-NR^{L3a}-**^{L3}, *^{L3}-NR^{L3a}C(O)-**^{L3}, *^{L3}-C(O)NR^{L3a}-**^{L3}, -C₁₋₈alkylene-, -C₂₋₈alkenylene-, -C₂₋₈alkynylene-, -[O(CR^{L3a}R^{L3b})ₘ₄]ₘ₅-, 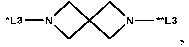 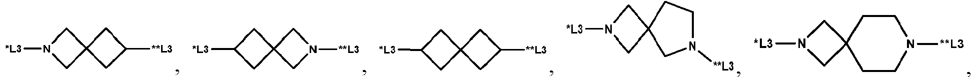 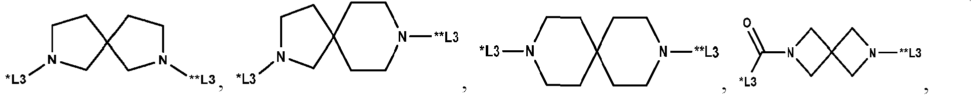 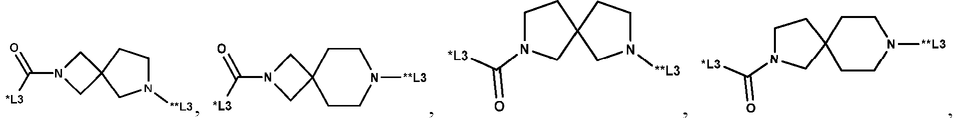 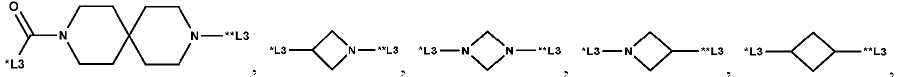 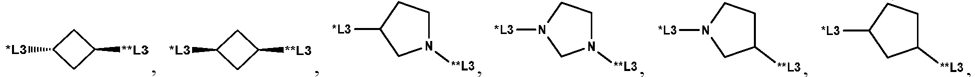 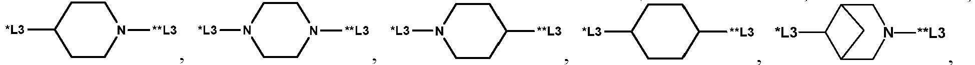 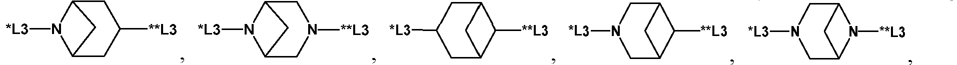 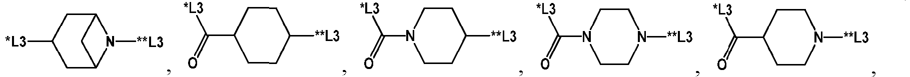 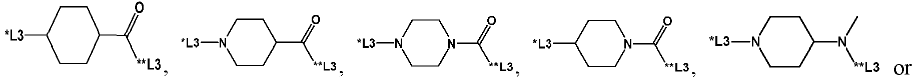 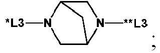
wherein each of said -C₃-C₈cycloalkylene-, *^{L3}-O-C₁₋₈alkylene-**^{L3}, *^{L3}-C₁₋₈alkylene-O-**^{L3}, *^{L3}-SO₂-C₁₋₈alkylene-**^{L3}, *^{L3}-C₁₋₈alkylene-SO₂-**^{L3}, *^{L3}-CO-C₁₋₈alkylene-**^{L3}, *^{L3}-C₁₋₈alkylene-CO-**^{L3}, *¹³-NR^{L3a}-C₁₋₈alkylene-**¹³, *^{L3}-C₁₋₈alkylene-NR^{L3a}-**^{L3}, -C₁₋₈alkylene-, -C₂₋₈alkenylene-, -C₂₋₈alkynylene-, 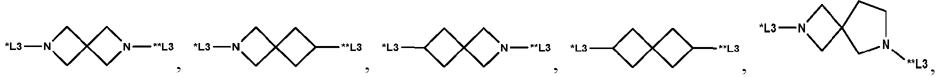 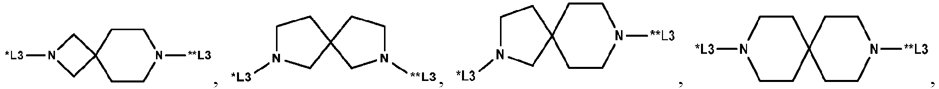 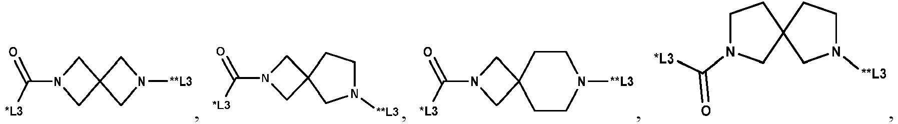 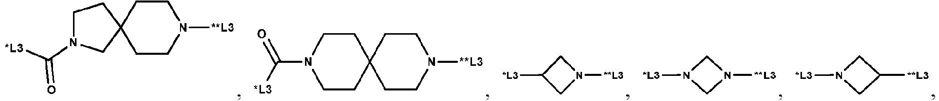 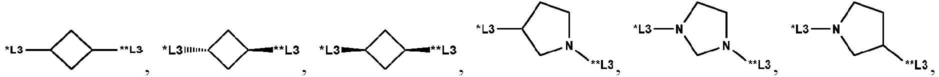 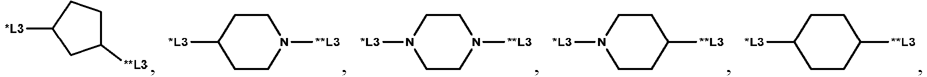 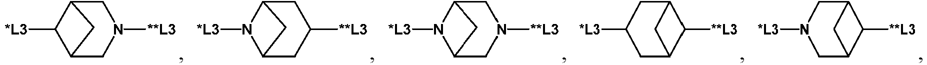 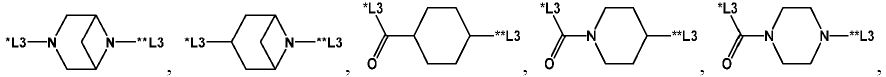 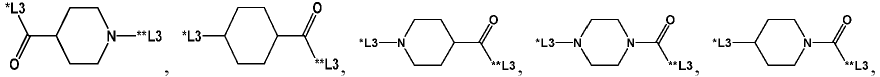 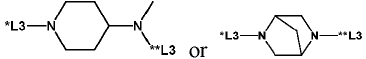 is optionally substituted with at least one substituent R^{13c};
R^{L3a} and R^{L3b} are each independently selected from hydrogen, methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl or pyrazinyl, wherein each of said methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl or pyrazinyl is optionally substituted with at least one substituent R^{L3d};
each of said R^{L3c} and R^{L3d} are independently -F, -Cl, -Br, -I, -OH, methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl, pyrazinyl or oxo.

Aspect **21.** The compound of any one of Aspects 1-20, wherein L³ is selected from single bond,-C₁₋₈alkylene-(preferably -CH₂-, -C₂H₄-, -C₃H₆-), -CO-, -O-, -N(CH₃)-, -NH-,

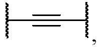

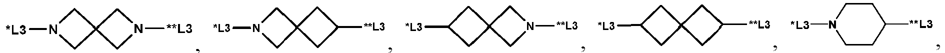

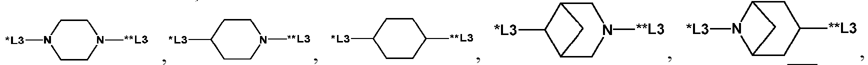

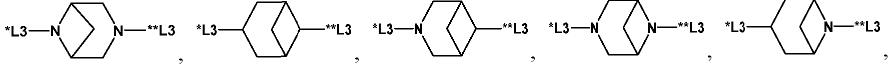

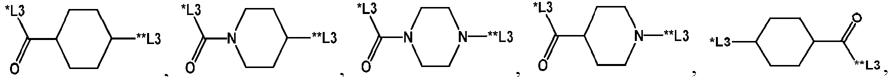

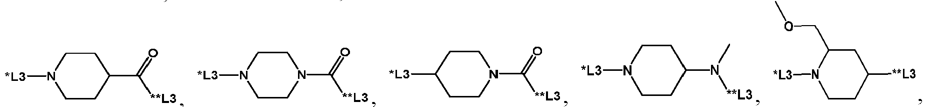

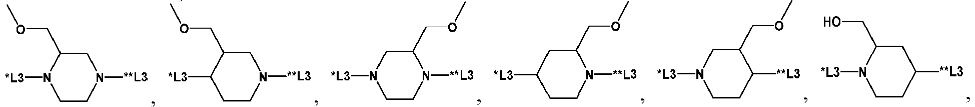

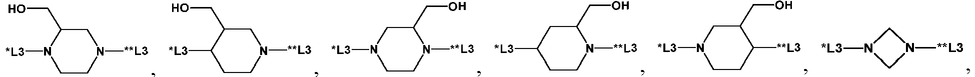

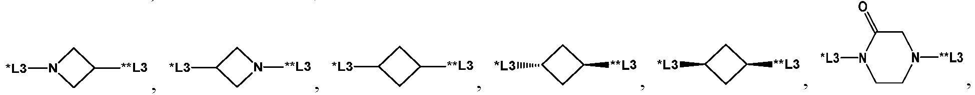

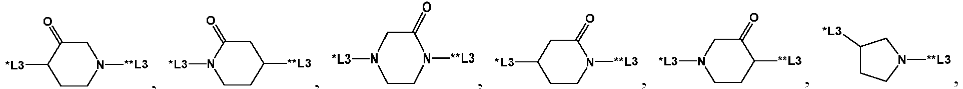

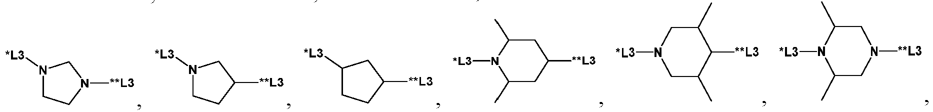

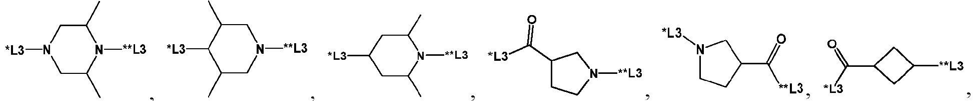

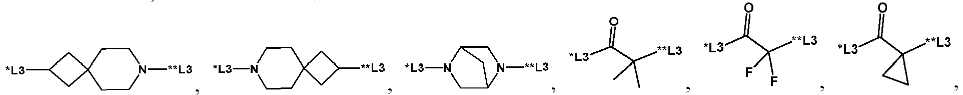

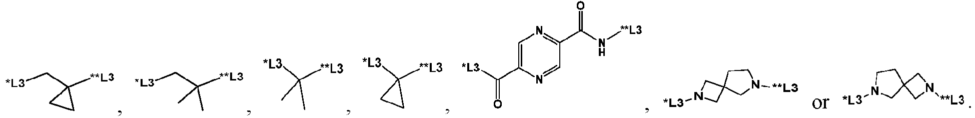

**Aspect 22.** The compound of any one of Aspects 1-21, wherein 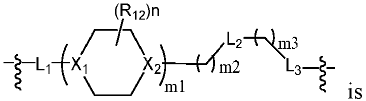 selected from -CH₂CH₂-, 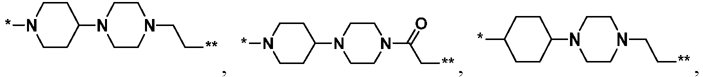 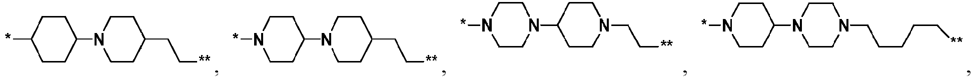 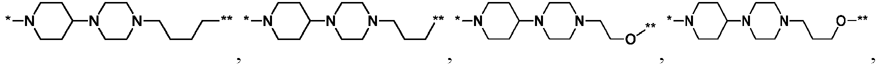 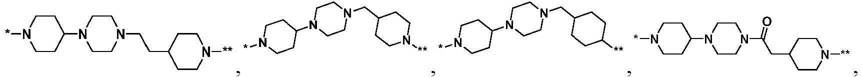 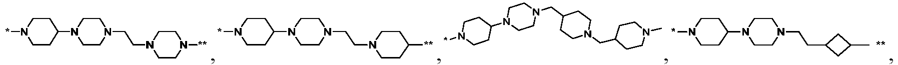 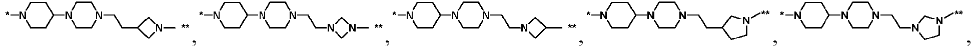 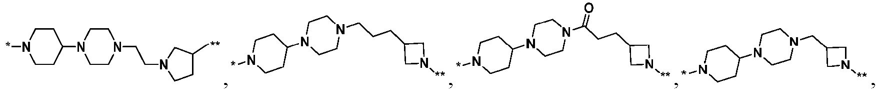 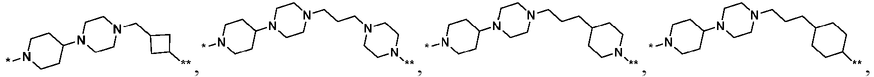 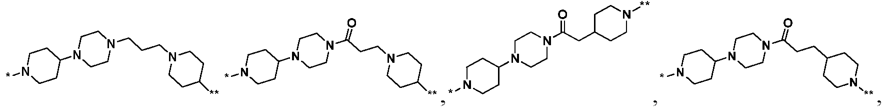 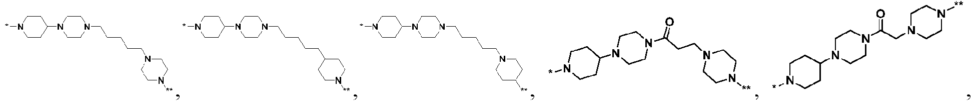 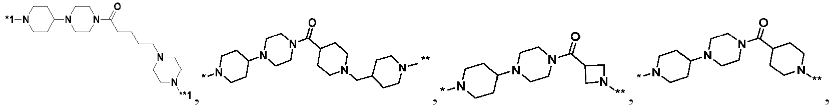 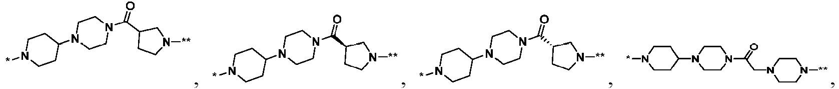 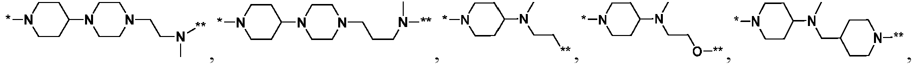 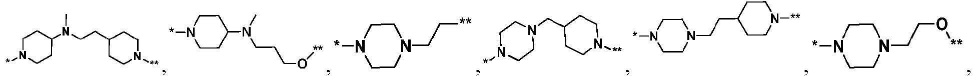 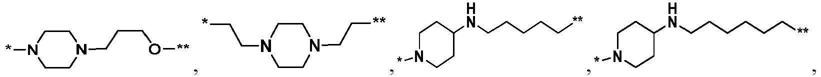 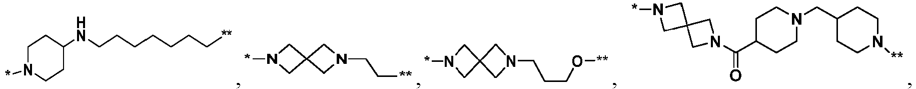 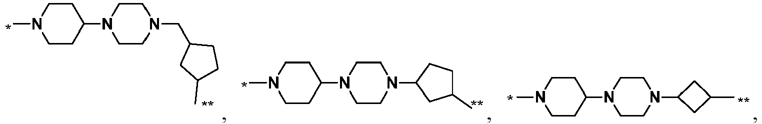 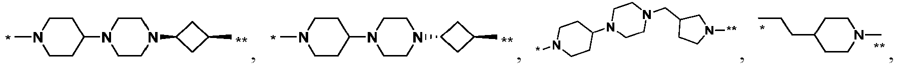 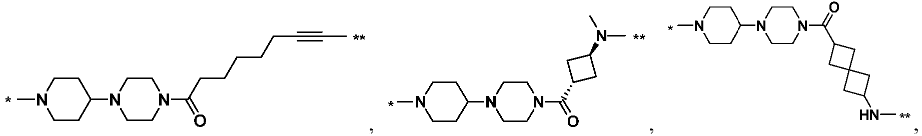 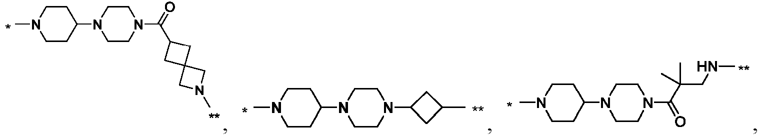 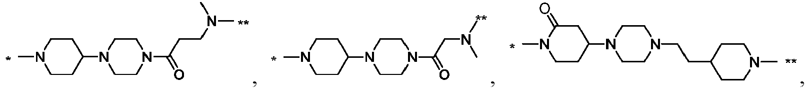 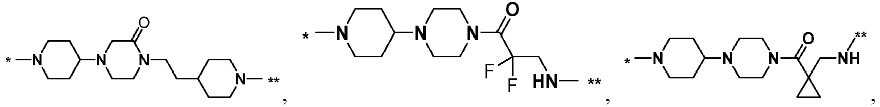 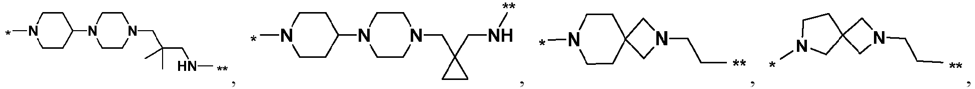 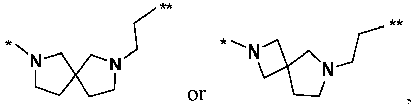 wherein * refers to the position attached to 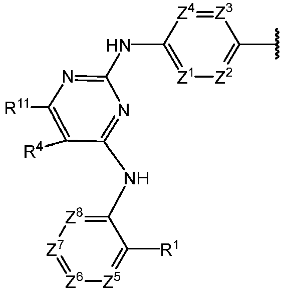 moiety, and ** refers to the position attached to the 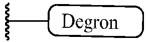 moiety.

**Aspect 23.** The compound of any one of Aspects 1-22, wherein 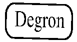 is selected from 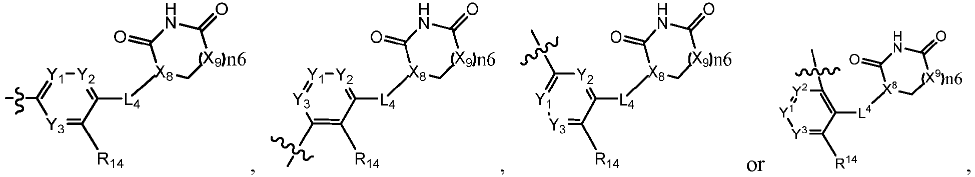 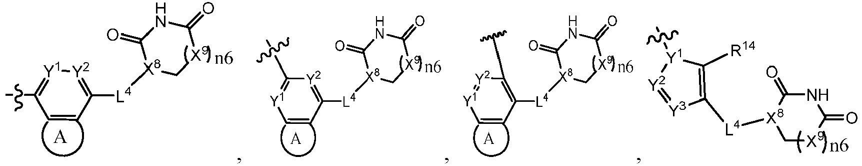 or 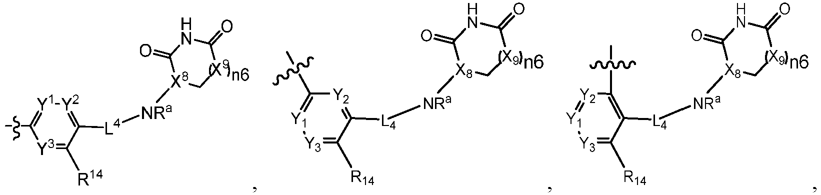 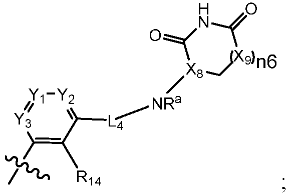
Ring A is selected from 5- to 7-membered cycloalkyl, 5- to 7-membered heterocyclyl, aryl, or heteroaryl;
R¹⁴ is independently selected from hydrogen, halogen, -C₁₋₈alkyl, -C₁₋₈alkoxy, or CN; said each -C₁₋₈alkyl, or -C₁₋₈alkoxy is optionally substituted by one or more halogen or -C₁₋₈alkyl; preferably R¹⁴ is independently selected from H, F, Cl, Br, I, CH₃, -OCH₃, CH₂F, CN, CHF₂, or CF₃;
X⁸ is independently selected from CH, CD, C(CH₃), C(C₂H₅), C(C₃H₇), C(CN) or N;
L⁴ is independently selected from a single bond, 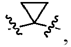 -O-, -NH-, -CH₂-, -CHF-, or -CF₂-;
Y¹, Y², and Y³ are each independently selected from CR^{a} or N;
X⁹ is CH₂;
R^{a} is each independently selected from hydrogen, halogen, -C₁₋₈alkyl, or -C₁₋₈alkoxy, wherein each of said -C₁₋₈alkyl or -C₁₋₈alkoxy is optionally substituted with at least one or more halogen, hydroxy, halogen, -C₁₋₈alkyl, or -C₁₋₈alkoxy; and
n6 is independently 0, 1 or 2.

Aspect 24. The compound of any one of Aspects 1-23, wherein 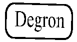 is 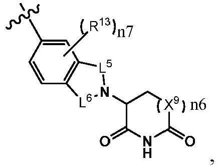 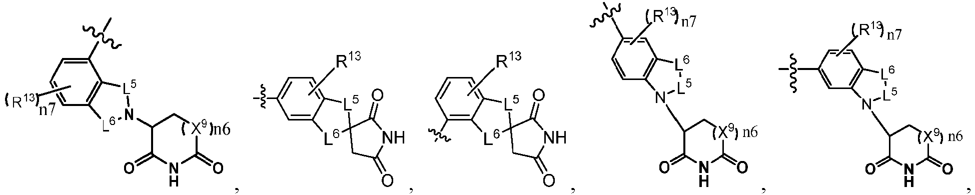 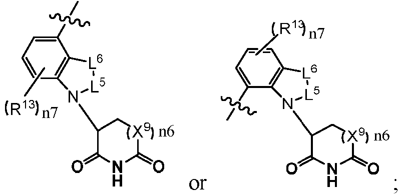
Wherein L⁵ and L⁶ is independently selected from a single bond, 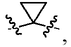 -O-, -NH-, -NMe-, -N(CH₂CH₃)-, -CH₂-, -CHF-, -CF₂-, -C(CH₃)₂- or -CO- (preferably L⁵ is -CO- or -CH₂-, and L⁶ is 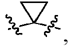 -O-, -NH-, -NMe-, -N(CH₂CH₃)-, -CH₂-, -CHF-, -CF₂-, -C(CH₃)₂- or -CO-);
X⁹ is CH₂;
each R¹³ is independently selected from hydrogen, -F, -Cl, -Br, -I, CN, -C₁₋₈alkyl, or -C₁₋₈alkoxy;
n₆ is 0 or 1; and
n₇ is 0, 1 2.

**Aspect** 25. The compound of any one of Aspects 1-24, wherein 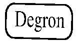 is 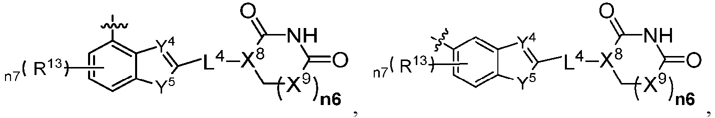 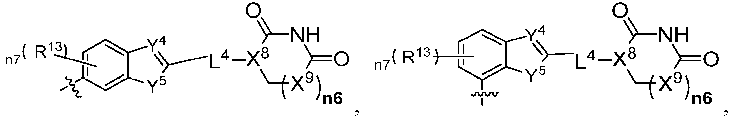 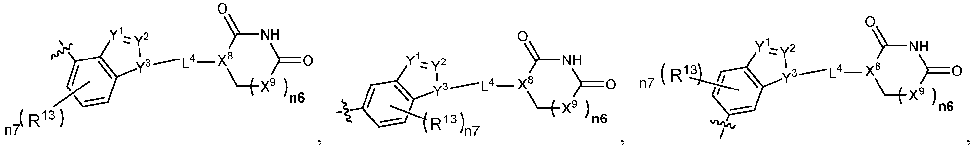 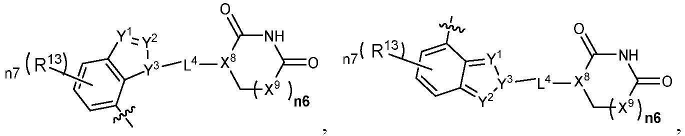 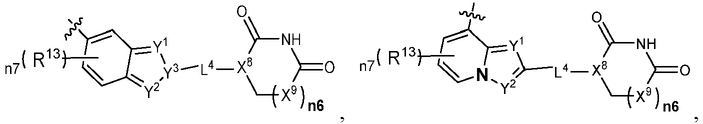 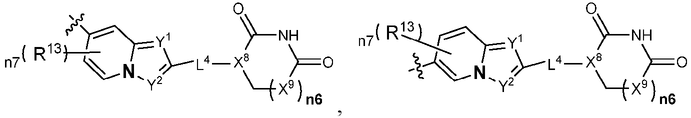 or 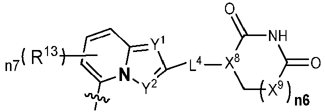
Wherein L⁴ is independently selected from a single bond, 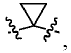 -O-, -NH-, -CH₂-, -CHF-, or -CF₂-;
X⁸ is independently selected from CH, CD, C(CH₃), C(C₂H₅), C(C₃H₇), C(F) or N;
X⁹ is CH₂;
each R¹³ is independently selected from hydrogen, -F, -Cl, -Br, -I, -CN, -C₁₋₈alkyl, or -C₁₋₈alkoxy;
Y¹, Y², Y³ and Y⁴ are each independently selected from CH, C(CH₃), C(CH₂CH₃), C(F), or N;
Y⁵ is selected from NH, N(CH₃), O or S;
n₆ is 0 or 1; and
n₇ is 0, 1 or 2.

**Aspect** 26. The compound of any one of Aspects 1-25, wherein 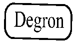 is selected from 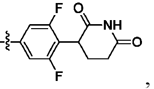 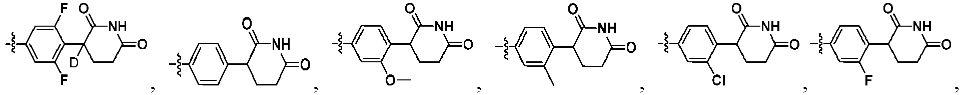 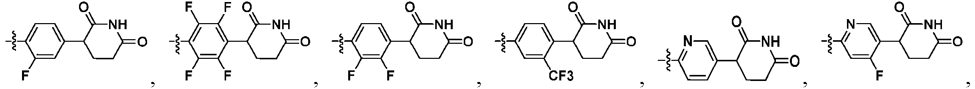 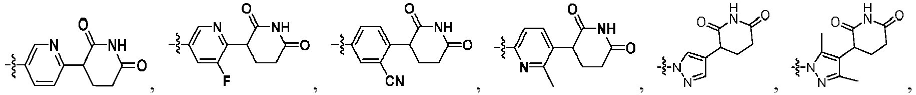 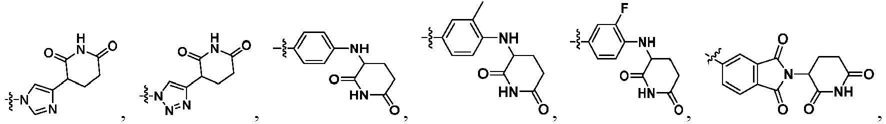 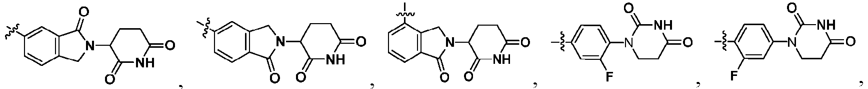 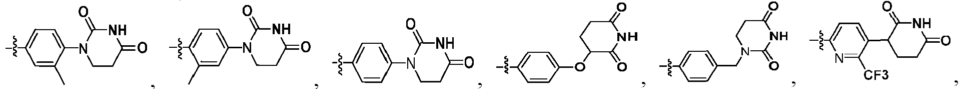 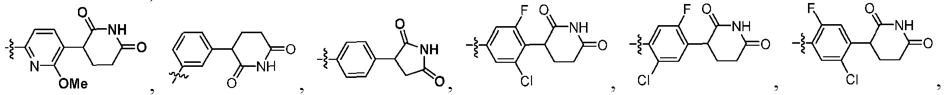 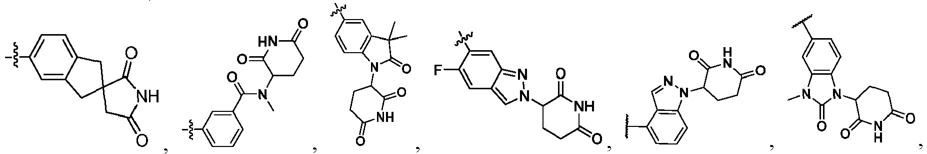 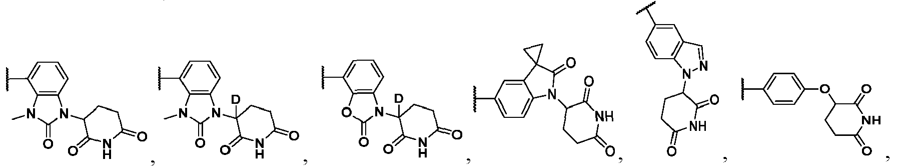 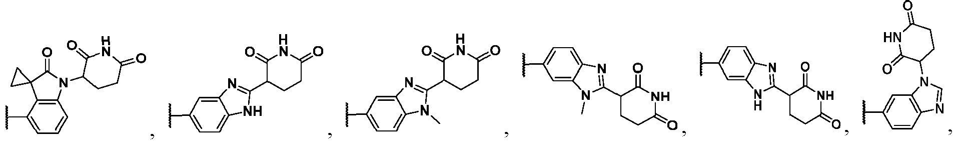 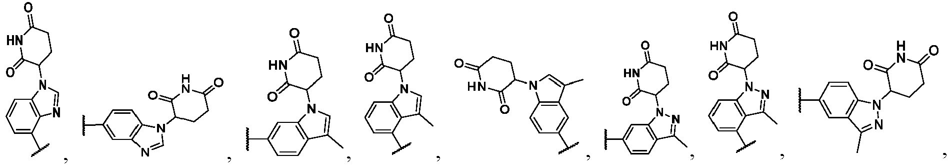 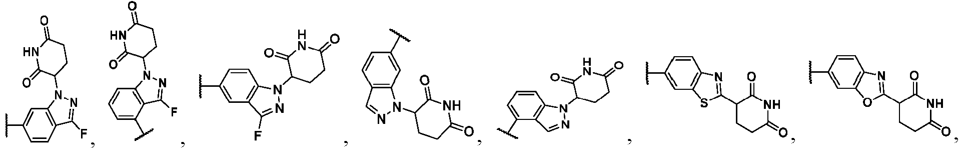 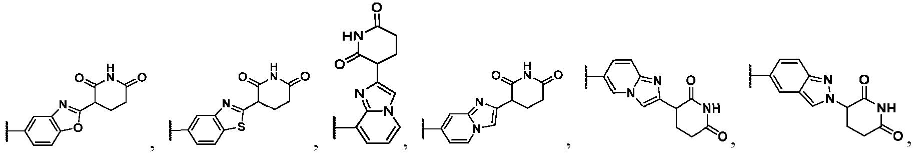 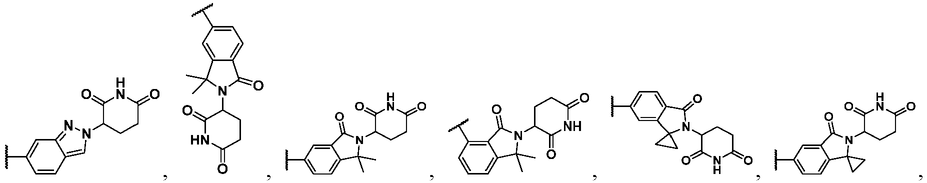 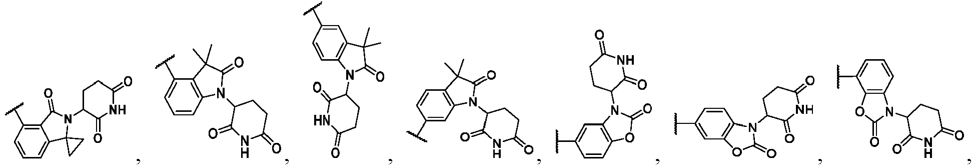 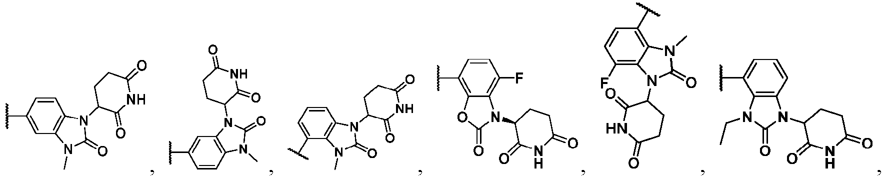 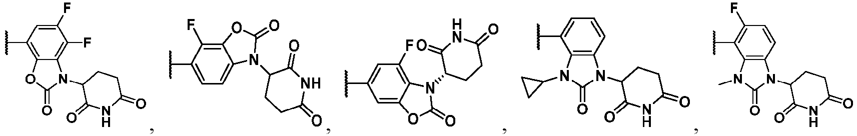 or

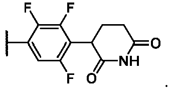

**Aspect 27.** The compound of any one of Aspects 1-26, wherein R^{z} is independently selected from H, -CH₃, -C₂H₅, F, -CH₂F, -CHF₂, -CF₃, -OCH₃, -OC₂H₅, -C₃H₇, -OCH₂F, -OCHF₂, -OCH₂CF₃, -OCF₃, -SCF₃, -CF₃ or -CH(OH)CH₃.

**Aspect 28.** The compound of any one of Aspects 1-27, wherein the deuterium substitution is on the Degron moiety, preferable, deuterium substitution is on X⁸.

**Aspect 29.** The compound of any one of Aspects 1-28, wherein the compound is selected from

| | |
|---|---|
| 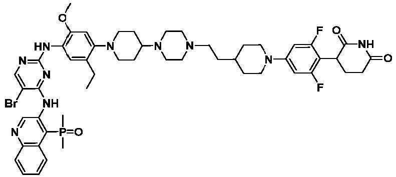 | 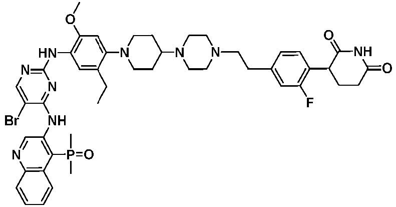 |
| 1 | 2 |
| 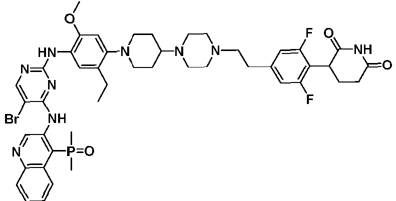 | 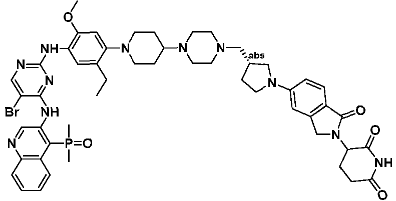 |
| 3 | 4 |
| 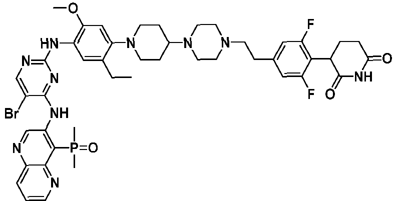 | 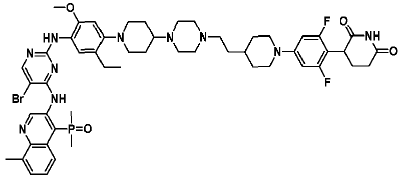 |
| 7 | 8 |
| 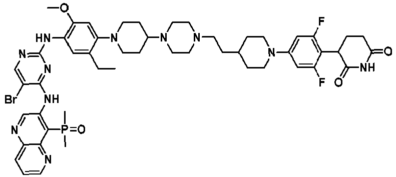 | 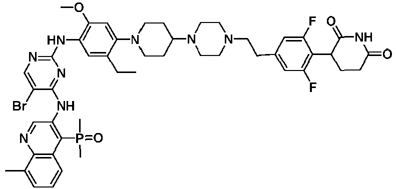 |
| 9 | 10 |
| 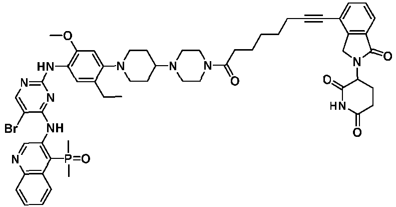 | 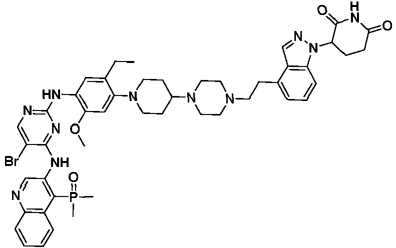 |
| 11 | 12 |
| 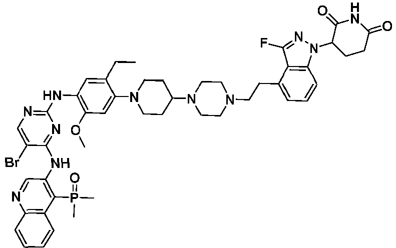 | 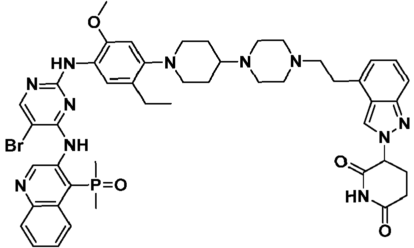 |
| 13 | 14 |
| 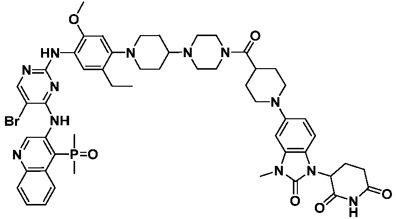 | 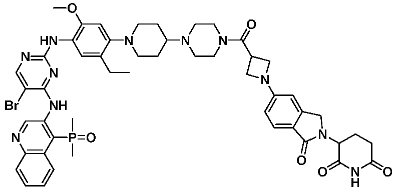 |
| 15 | 16 |
| 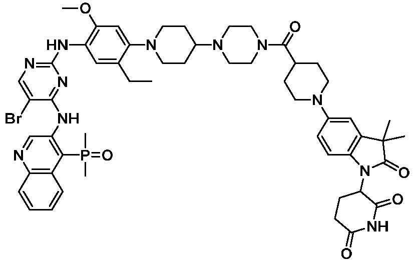 | 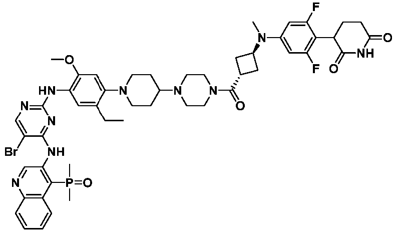 |
| 17 | 18 |
| 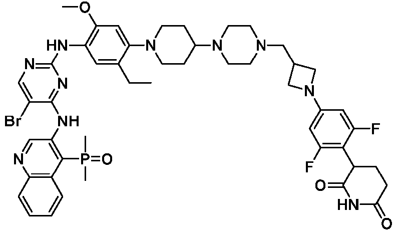 | 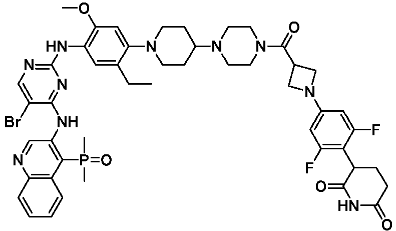 |
| 19 | 20 |
| 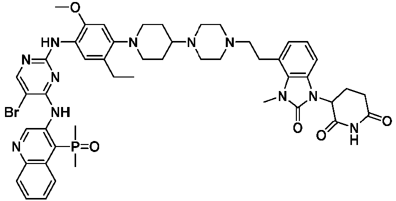 | 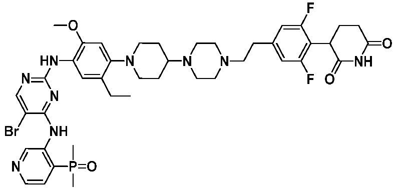 |
| 21 | 22 |
| 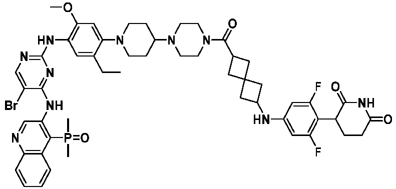 | 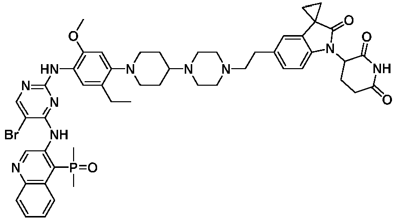 |
| 23 | 24 |
| 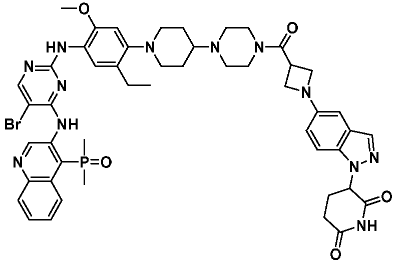 | 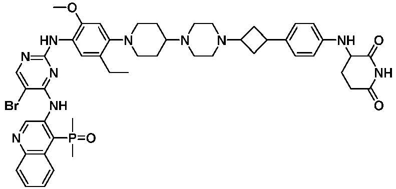 |
| 25 | 26 |
| 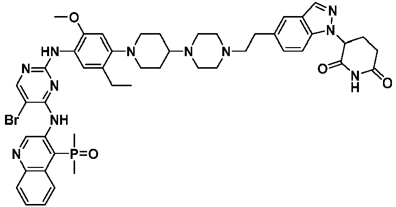 | 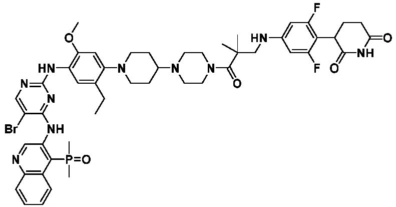 |
| 27 | 28 |
| 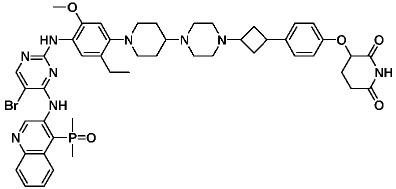 | 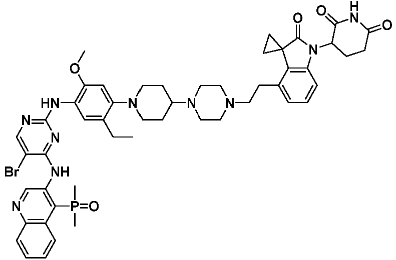 |
| 29 | 30 |
| 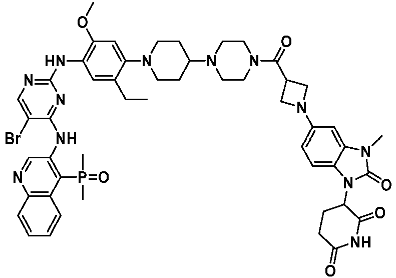 | 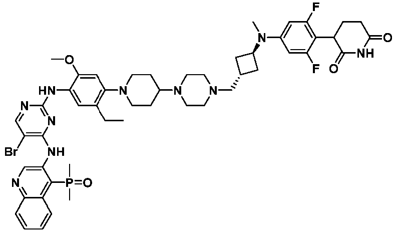 |
| 31 | 32 |
| 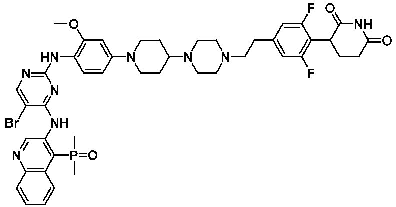 | 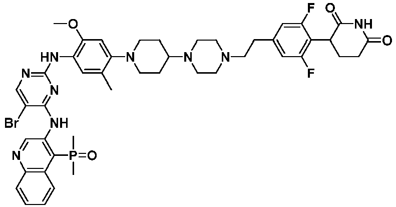 |
| 33 | 34 |
| 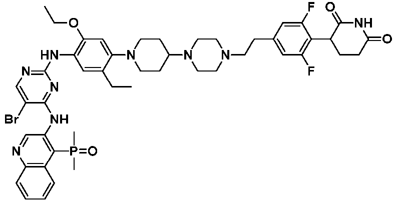 | 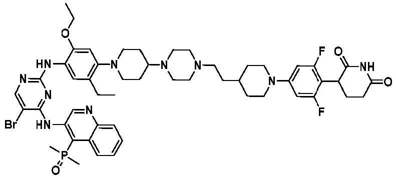 |
| 35 | 36 |
| 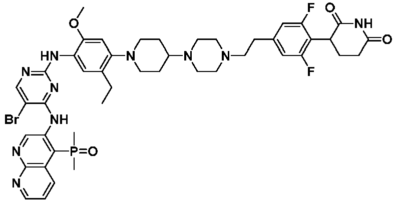 | 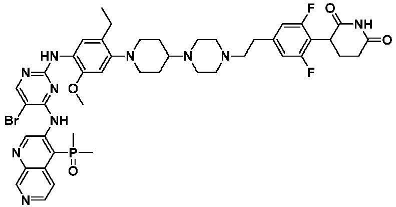 |
| 37 | 38 |
| | 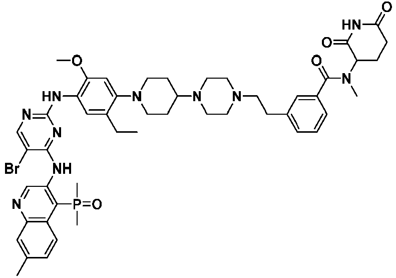 |
| | 40 |
| 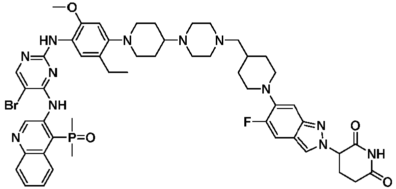 | 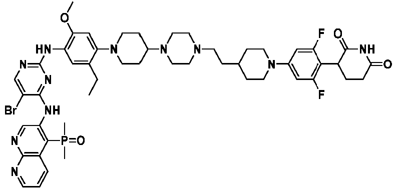 |
| 41 | 42 |
| 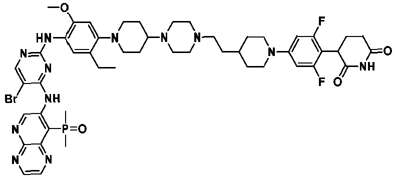 | 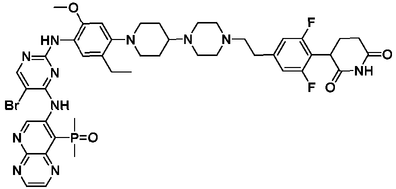 |
| 43 | 44 |
| 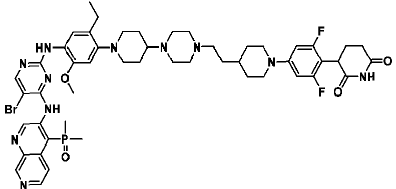 | 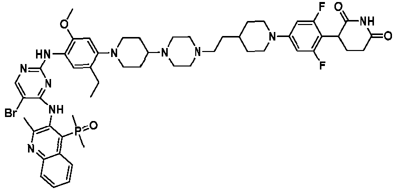 |
| 45 | 46 |
| 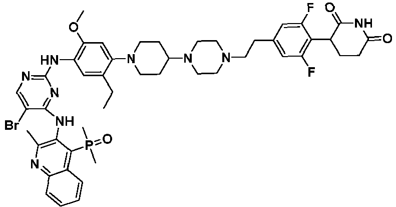 | 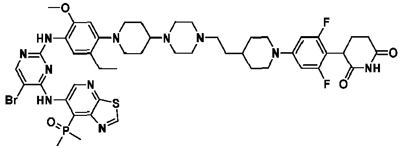 |
| 47 | 48 |
| 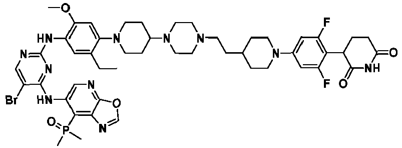 | 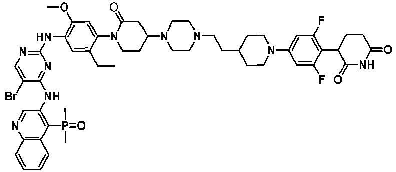 |
| 49 | 50 |
| 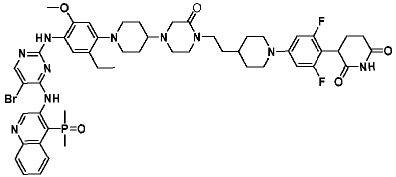 | 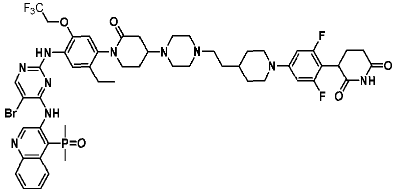 |
| 51 | 52 |
| 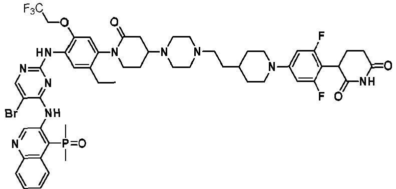 | 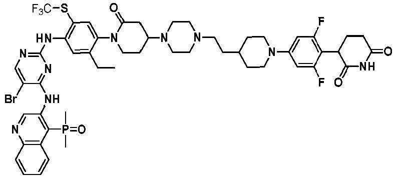 |
| 53 | 54 |
| 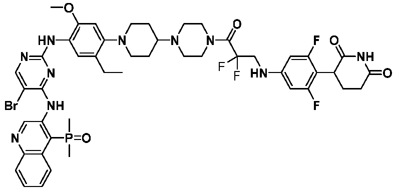 | 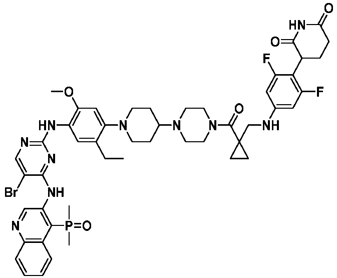 |
| 55 | 56 |
| 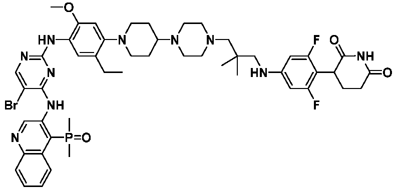 | 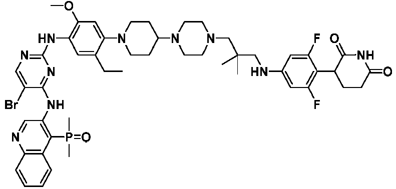 |
| 57 | 58 |
| 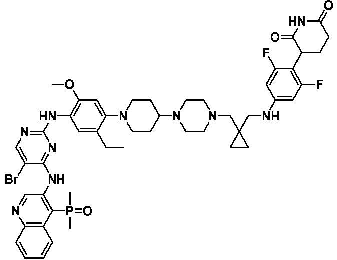 | 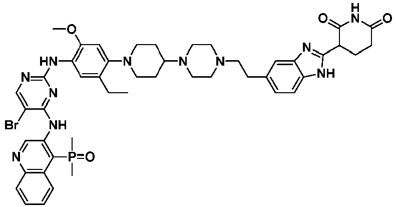 |
| 59 | 60 |
| 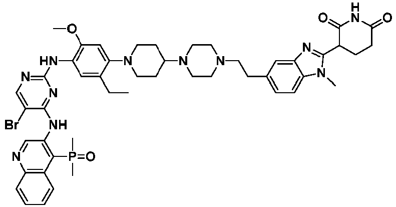 | 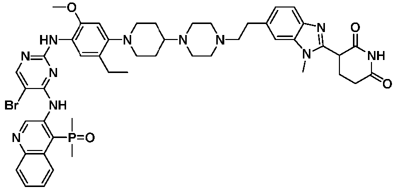 |
| 61 | 62 |
| 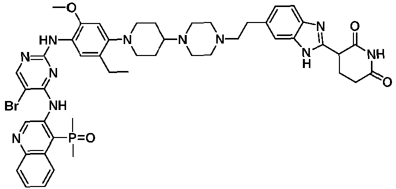 | 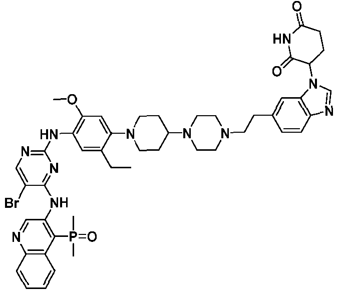 |
| 63 | 64 |
| 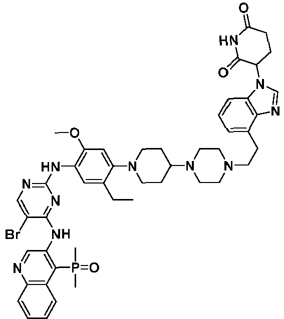 | 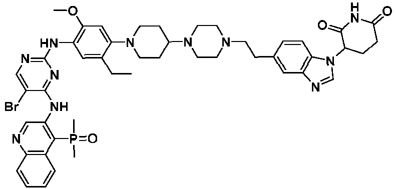 |
| 65 | 66 |
| 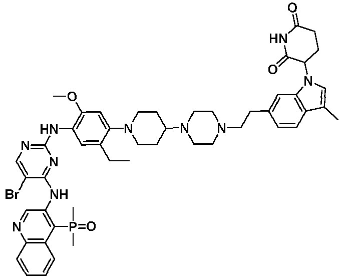 | 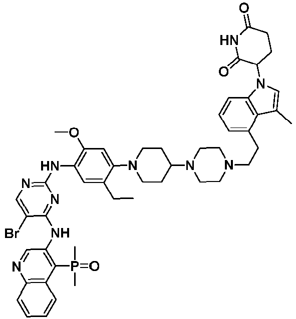 |
| 67 | 68 |
| 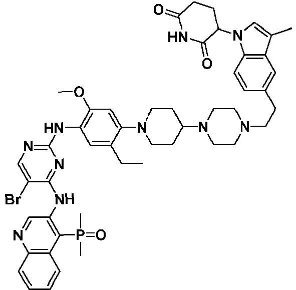 | 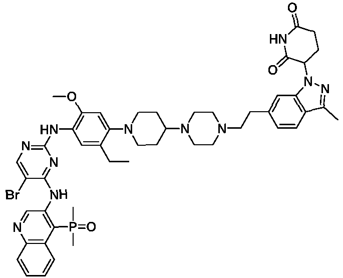 |
| 69 | 70 |
| 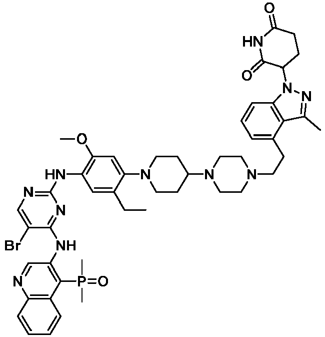 | 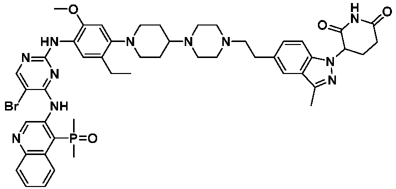 |
| 71 | 72 |
| 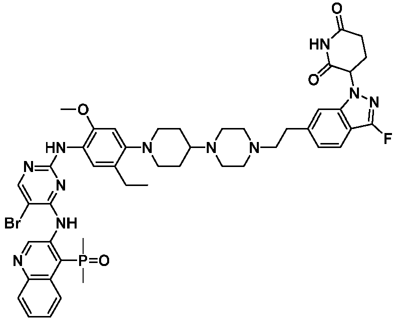 | 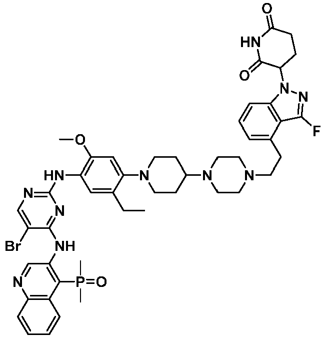 |
| 73 | 74 |
| 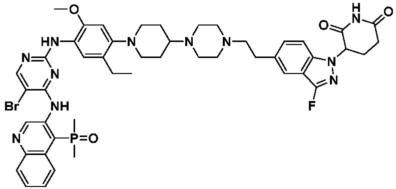 | 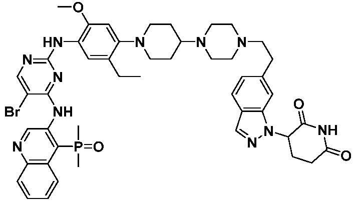 |
| 75 | 76 |
| 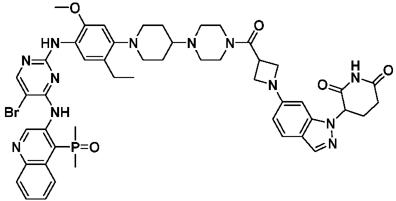 | 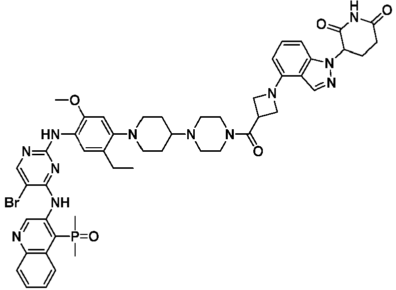 |
| 77 | 78 |
| 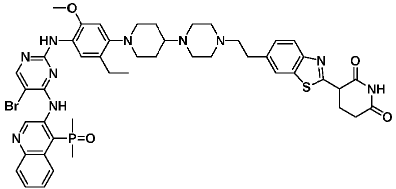 | 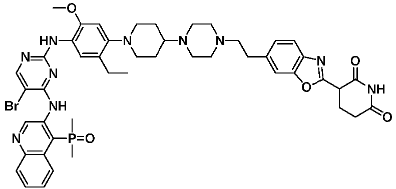 |
| 79 | 80 |
| 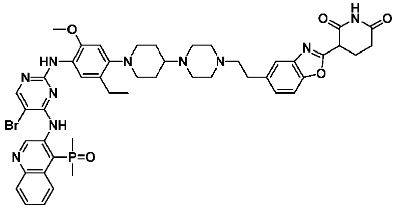 | 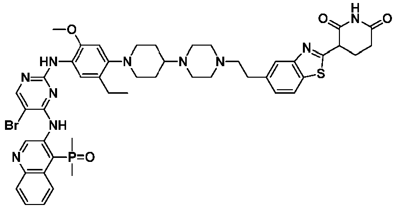 |
| 81 | 82 |
| 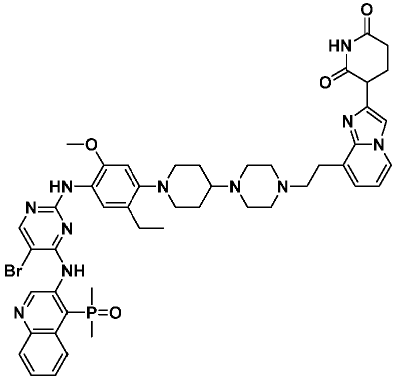 | 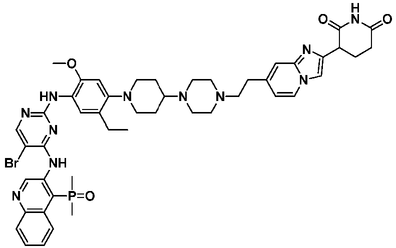 |
| 83 | 84 |
| 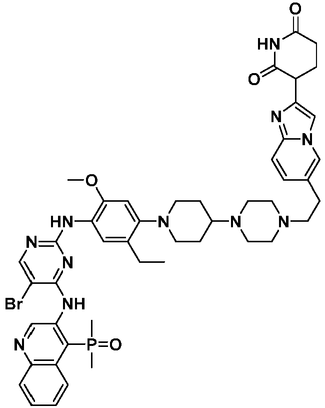 | 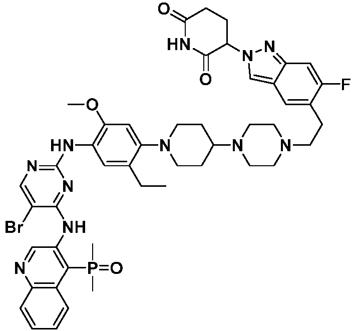 |
| 85 | 86 |
| 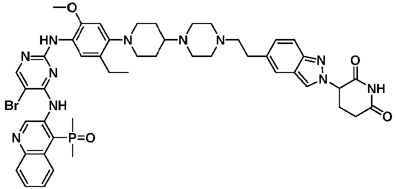 | 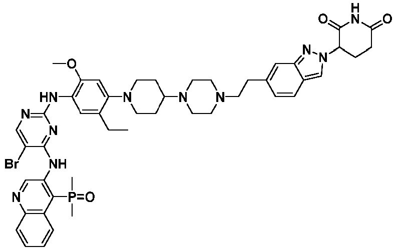 |
| 87 | 88 |
| 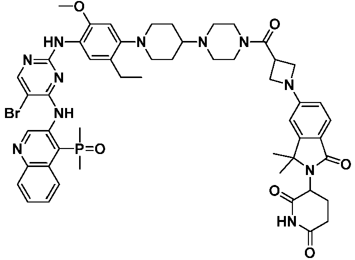 | 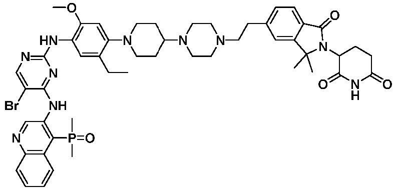 |
| 89 | 90 |
| 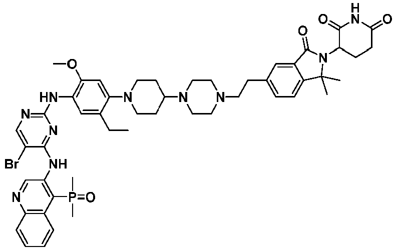 | 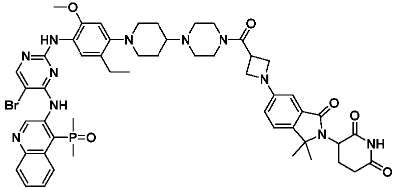 |
| 91 | 92 |
| 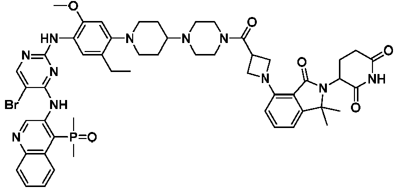 | 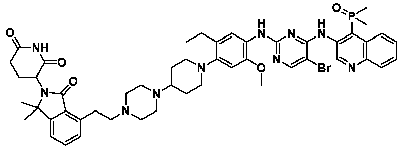 |
| 93 | 94 |
| 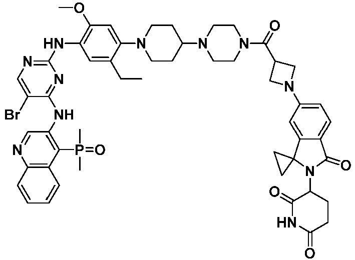 | 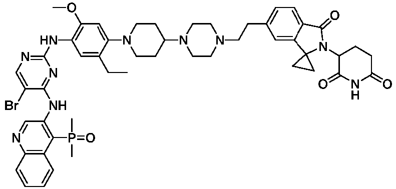 |
| 95 | 96 |
| 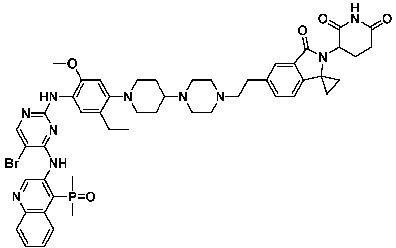 | 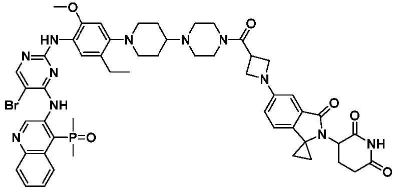 |
| 97 | 98 |
| 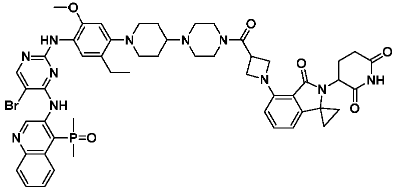 | 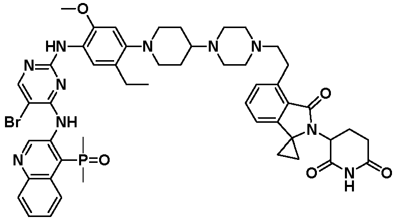 |
| 99 | 100 |
| 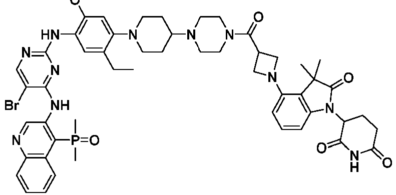 | 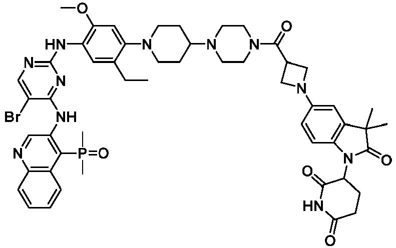 |
| 101 | 102 |
| 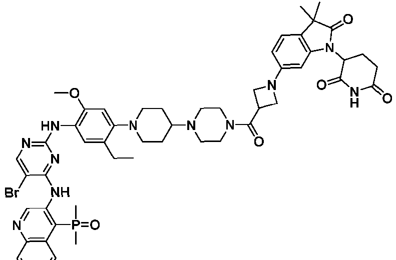 | 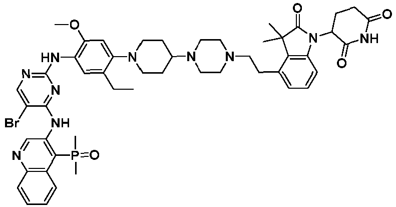 |
| 103 | 104 |
| 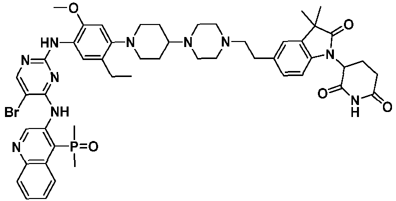 | 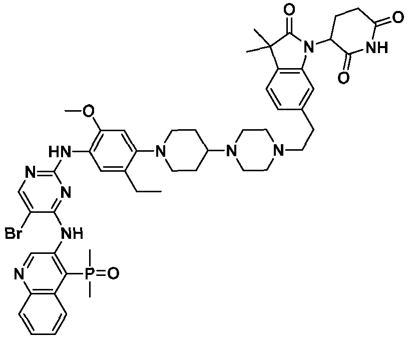 |
| 105 | 106 |
| 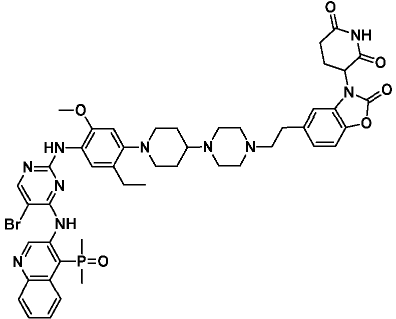 | 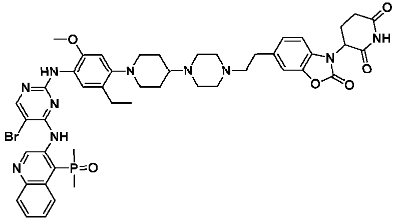 |
| 107 | 108 |
| 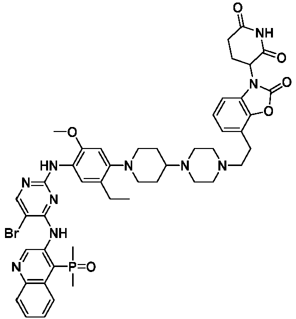 | 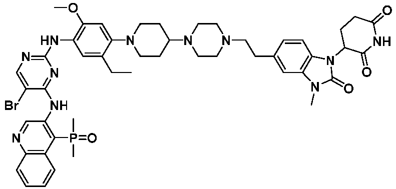 |
| 109 | 110 |
| 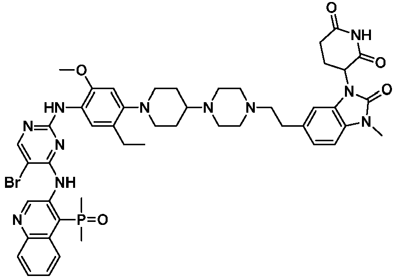 | 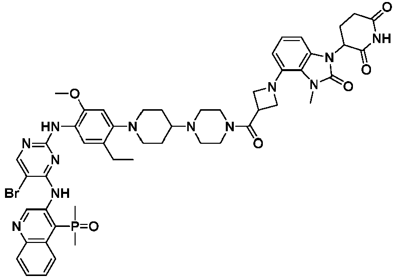 |
| 111 | 112 |
| 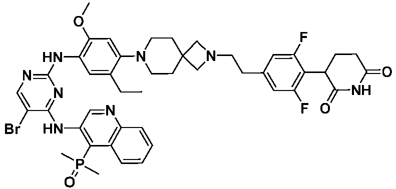 | 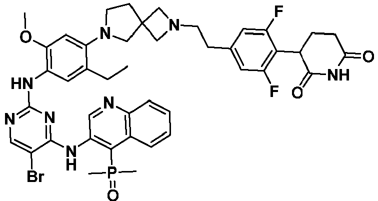 |
| 113 | 114 |
| 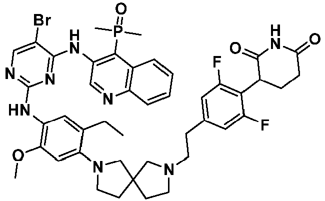 | 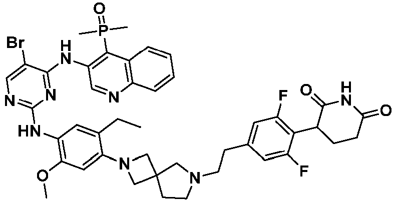 |
| 115 | 116 |
| 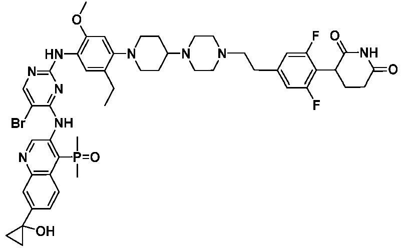 | 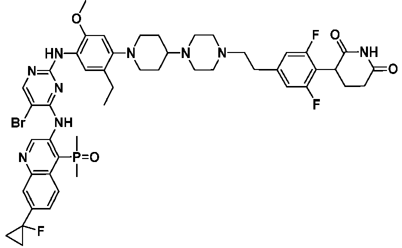 |
| 117 | 118 |
| 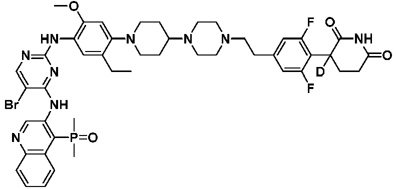 | 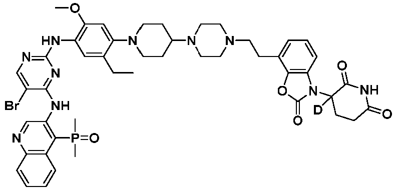 |
| 119 | 120 |
| 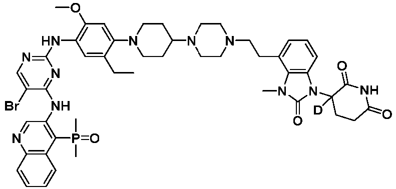 | 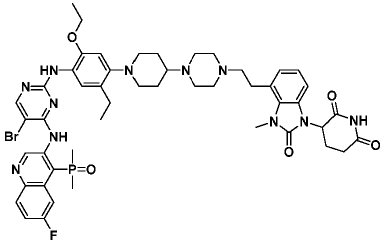 |
| 121 | 124 |
| 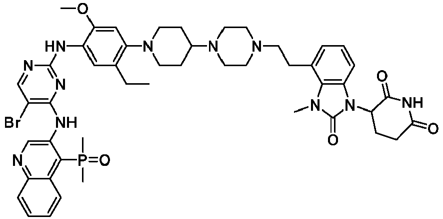 | 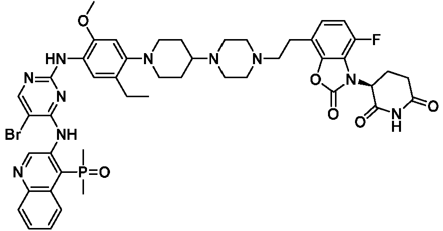 |
| 125 | 126 |
| 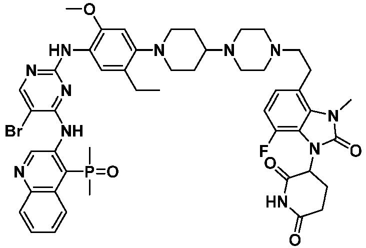 | 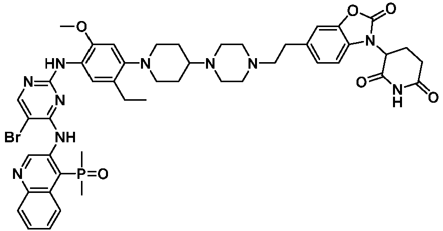 |
| 130 | 132 |
| 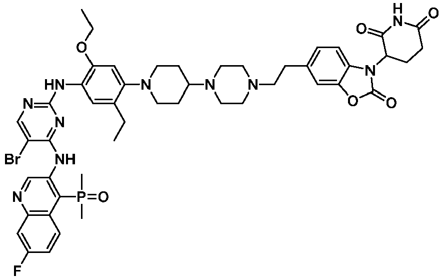 | 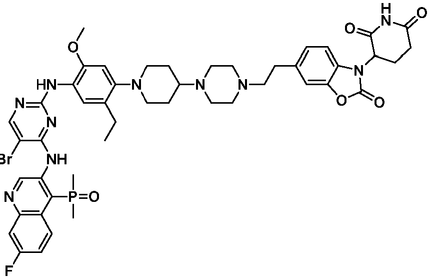 |
| 133 | 134 |
| 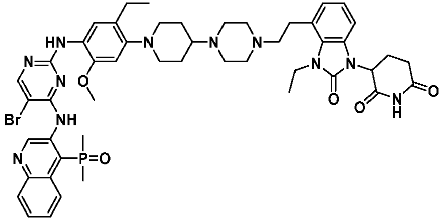 | 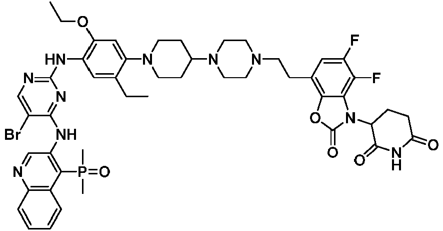 |
| 135 | 136 |
| 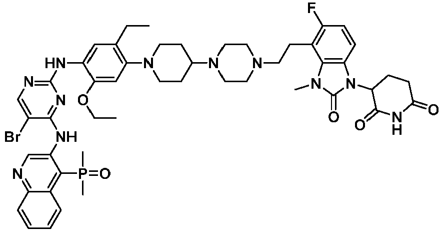 | 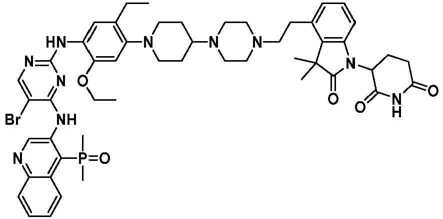 |
| 137 | 138 |
| 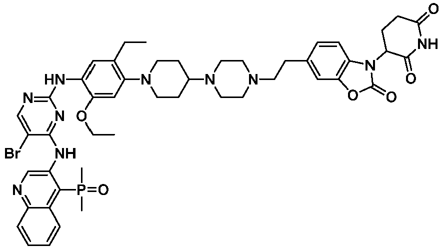 | 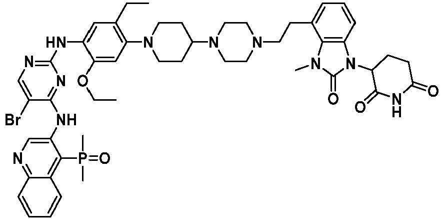 |
| 139 | 140 |
| 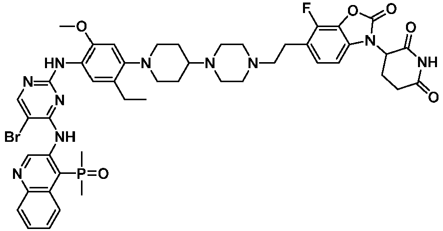 | 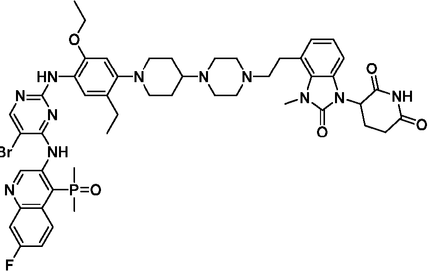 |
| 142 | 147 |
| 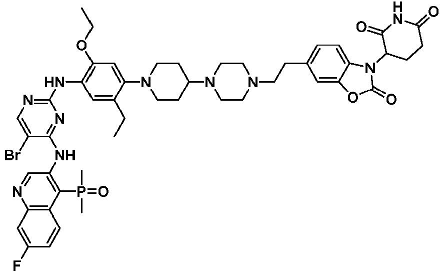 | 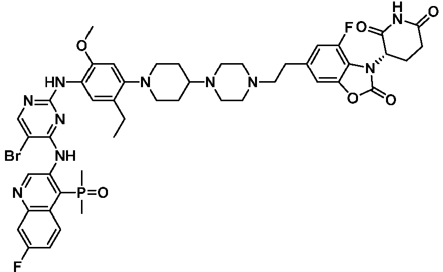 |
| 149 | 151 |
| 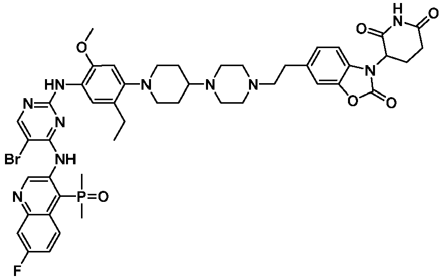 | 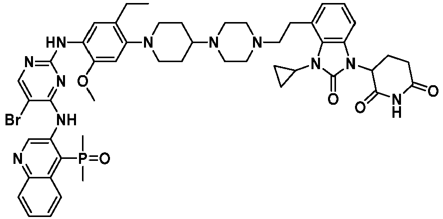 |
| 153 | 155 |
| | 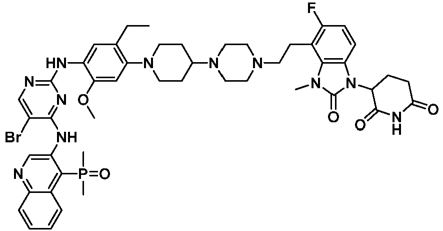 |
| | 159 |
| 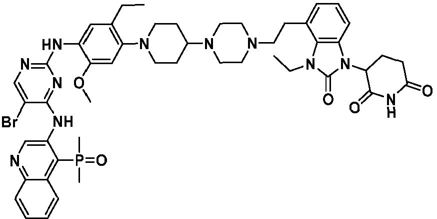 | 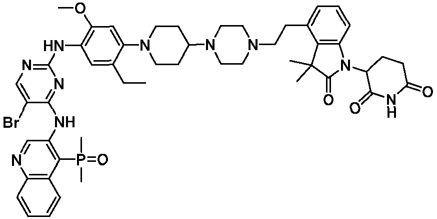 |
| 163 | 164 |
| 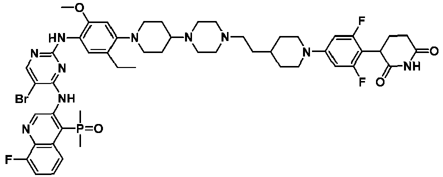 | 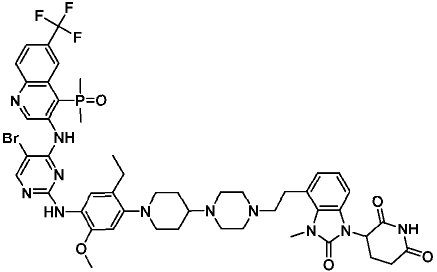 |
| 165 | 166 |
| 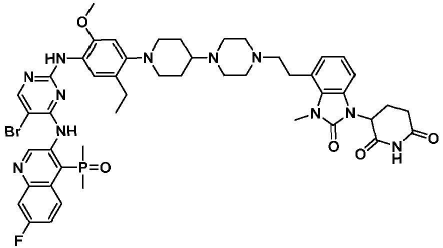 | 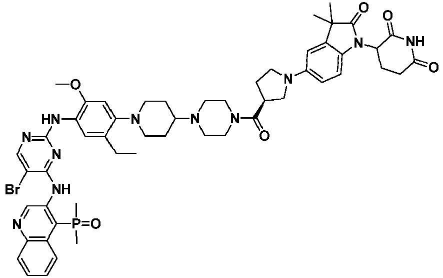 |
| 167 | 168 |
| 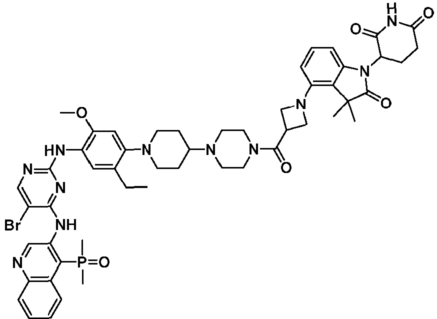 | 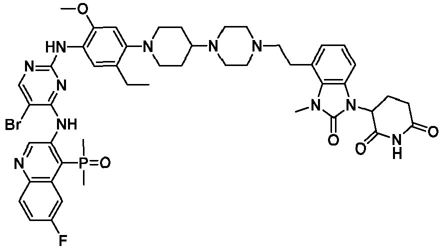 |
| 169 | 170 |
| 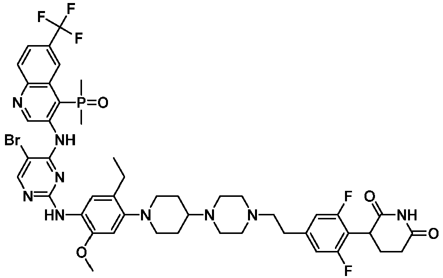 | 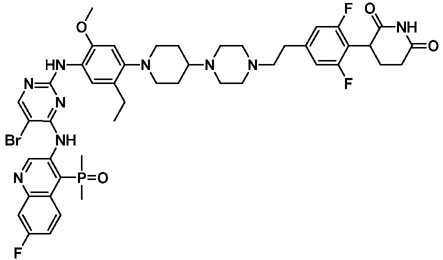 |
| 171 | 172 |
| 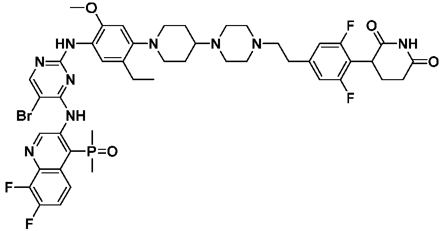 | 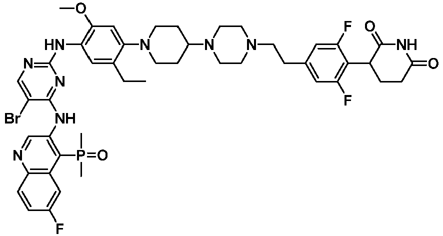 |
| 173 | 174 |
| 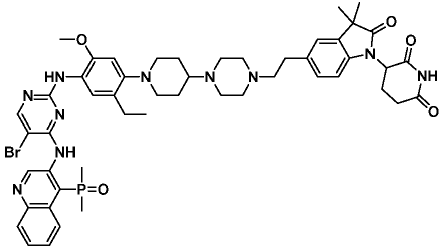 | 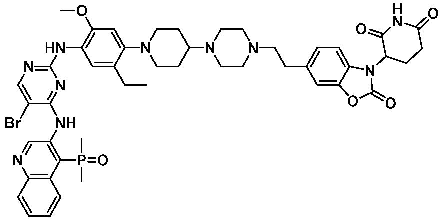 |
| 175 | 176 |
| | 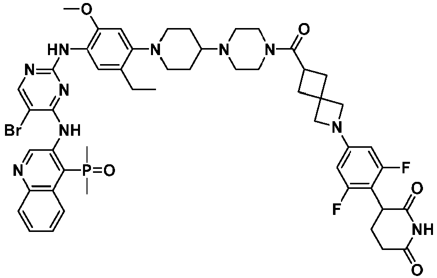 |
| | 178 |
| 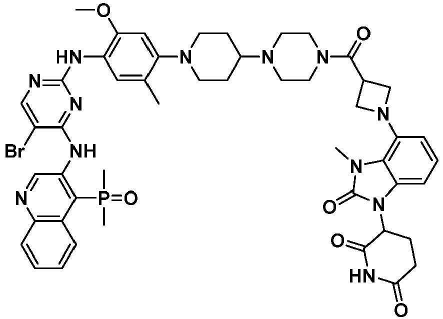 | 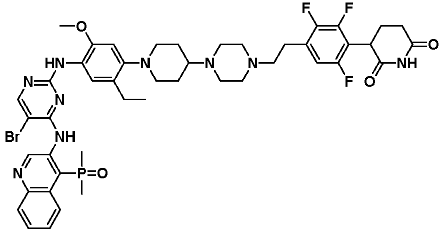 |
| 179 | 180 |
| 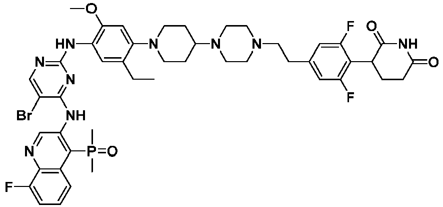 | 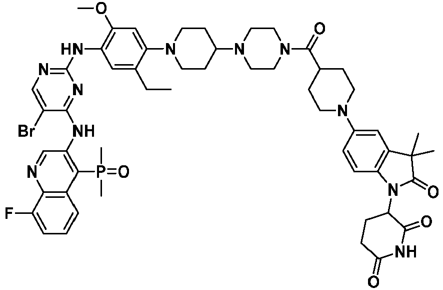 |
| 181 | 182 |
| 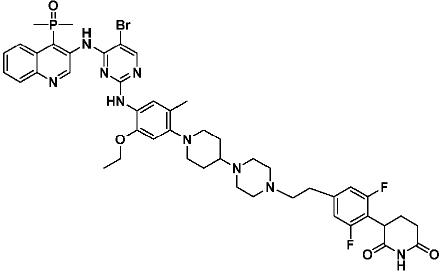 | 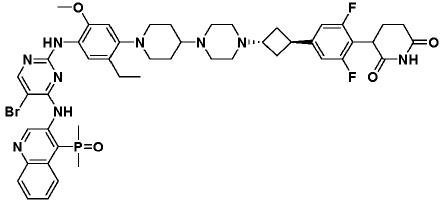 |
| 183 | 184 |
| 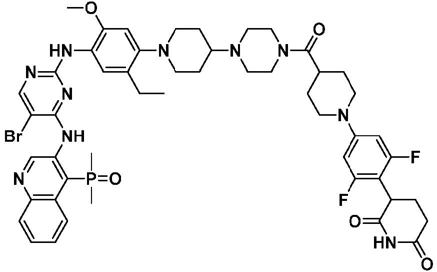 | 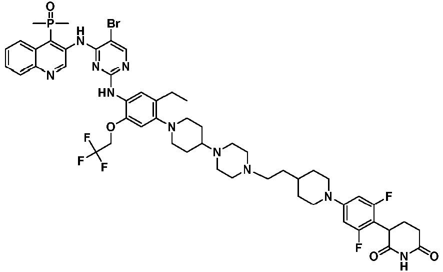 |
| 185 | 186 |
| 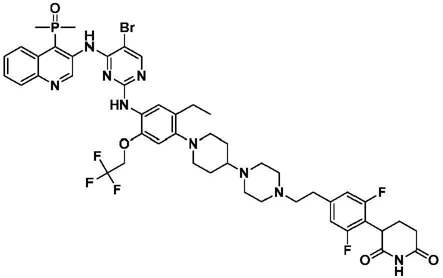 | 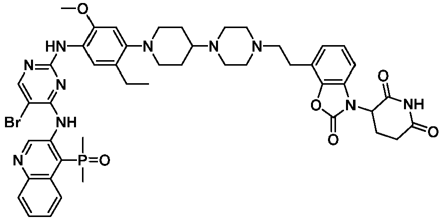 |
| 187 | 188 |
| 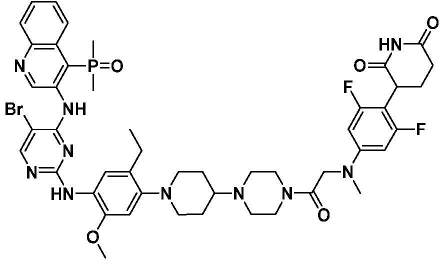 | |
| 189 | |

Aspect 30. A pharmaceutical composition comprising a compound of any one of Aspects 1-29 or a pharmaceutically acceptable salt, stereoisomer, tautomer or prodrug thereof, together with a pharmaceutically acceptable excipient.

Aspect 31. A method of decreasing EGFR activity by inhibition and/or degradation, which comprises administering to an individual the compound according to any one of Aspects 1-30, or a pharmaceutically acceptable salt thereof, including the compound of formula (I) or the specific compounds exemplified herein.

Aspect 32. The method of Aspect 32, wherein the disease is selected from cancer, preferred pancreatic cancer, breast cancer, glioblastoma multiforme, head and neck cancer, or non-small cell lung cancer.

Aspect 33. Use of a compound of any one of Aspects 1-29 or a pharmaceutically acceptable salt, stereoisomer, tautomer or prodrug thereof in the preparation of a medicament for treating a disease that can be affected by EGFR modulation.

Aspect 34. The use of Aspect 33, wherein the disease is cancer, preferred pancreatic cancer, breast cancer, glioblastoma multiforme, head and neck cancer, or non-small cell lung cancer.

Aspect 35. A method of treating a disease or disorder in a patient comprising administering to the patient a therapeutically effective amount of the compound any one of Aspects 1-29, or a pharmaceutically acceptable salt thereof as a EGFR kinase inhibitor and/or degrader, wherein the disease or disorder is associated with inhibition of EGFR.

Aspect 36. The method of Aspect 35, wherein the disease is selected from cancer, preferred pancreatic cancer, breast cancer, glioblastoma multiforme, head and neck cancer, or non-small cell lung cancer.

### DETAILED DESCRIPTION OF THE INVENTION

As mentioned, references to methods of treatment by therapy or surgery or in vivo diagnosis methods disclosed herein are to be interpreted as references to compounds of the present invention for use in those methods.

The following terms have the indicated meanings throughout the specification:
Unless specifically defined elsewhere in this document, all other technical and scientific terms used herein have the meaning commonly understood by one of ordinary skill in the art to which this invention belongs.

The following terms have the indicated meanings throughout the specification:
As used herein, including the appended claims, the singular forms of words such as "a", "an", and "the", include their corresponding plural references unless the context clearly indicates otherwise.

The term "or" is used to mean, and is used interchangeably with, the term "and/or" unless the context clearly dictates otherwise.

The term "alkyl" includes a hydrocarbon group selected from linear and branched, saturated hydrocarbon groups comprising from 1 to 18, such as from 1 to 12, further such as from 1 to 10, more further such as from 1 to 8, or from 1 to 6, or from 1 to 4, carbon atoms. Examples of alkyl groups comprising from 1 to 6 carbon atoms (i.e., C₁₋₆ alkyl) include, but not limited to, methyl, ethyl, 1-propyl or n-propyl ("n-Pr"), 2-propyl or isopropyl ("i-Pr"), 1-butyl or n-butyl ("n-Bu"), 2-methyl-1-propyl or isobutyl ("i-Bu"), 1-methylpropyl or s-butyl ("s-Bu"), 1,1-dimethylethyl or t-butyl ("t-Bu"), 1-pentyl, 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl and 3,3-dimethyl-2-butyl groups.

The term "propyl" includes 1-propyl or n-propyl ("n-Pr"), 2-propyl or isopropyl ("i-Pr").

The term "butyl" includes 1-butyl or n-butyl ("n-Bu"), 2-methyl-1-propyl or isobutyl ("i-Bu"), 1-methylpropyl or s-butyl ("s-Bu"), 1,1-dimethylethyl or t-butyl ("t-Bu").

The term "pentyl" includes 1-pentyl, 2-pentyl, 3-pentyl, 2-methyl-2-butyl, 3-methyl-2-butyl, 3-methyl-1-butyl, 2-methyl-1-butyl.

The term "hexyl" includes 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl and 3,3-dimethyl-2-butyl.

The term "alkylene" refers to a divalent alkyl group by removing two hydrogen from alkane. Alkylene includes but not limited to methylene, ethylene, propylene, and so on.

The term "cycloalkylene" refers to a divalent alkenyl group by removing two hydrogen from cycloalkylene. Cycloalkylene includes but not limited to, cyclopropyl, cyclobutyl, cyclopentyl, cyclohexyl, cycloheptyl, cyclooctyl, and so on.

The term "halogen" includes fluoro (F), chloro (Cl), bromo (Br) and iodo (I).

The term "alkenyl" includes a hydrocarbon group selected from linear and branched hydrocarbon groups comprising at least one C=C double bond and from 2 to 18, such as from 2 to 8, further such as from 2 to 6, carbon atoms. Examples of the alkenyl group, e.g., C₂₋₆ alkenyl, include, but not limited to ethenyl or vinyl, prop-1-enyl, prop-2-enyl, 2-methylprop-1-enyl, but-1-enyl, but-2-enyl, but-3-enyl, buta-1,3-dienyl, 2-methylbuta-1,3-dienyl, hex-1-enyl, hex-2-enyl, hex-3-enyl, hex-4-enyl, and hexa-1,3-dienyl groups.

The term "alkenylene" refers to a divalent alkenyl group by removing two hydrogen from alkene. Alkenylene includes but not limited to, vinylidene, butenylene, and so on.

The term "alkynyl" includes a hydrocarbon group selected from linear and branched hydrocarbon group, comprising at least one C≡C triple bond and from 2 to 18, such as 2 to 8, further such as from 2 to 6, carbon atoms. Examples of the alkynyl group, e.g., C₂₋₆ alkynyl, include, but not limited to ethynyl, 1-propynyl, 2-propynyl (propargyl), 1-butynyl, 2-butynyl, and 3-butynyl groups.

The term "alkynylene" refers to a divalent alkynyl group by removing two hydrogen from alkyne. Alkenylene includes but not limited to ethynylene and so on.

The term "cycloalkyl" includes a hydrocarbon group selected from saturated cyclic hydrocarbon groups, comprising monocyclic and polycyclic (e.g., bicyclic and tricyclic) groups including fused, bridged or spiro cycloalkyl.

For example, the cycloalkyl group may comprise from 3 to 12, such as from 3 to 10, further such as 3 to 8, further such as 3 to 6, 3 to 5, or 3 to 4 carbon atoms. Even further for example, the cycloalkyl group may be selected from monocyclic group comprising from 3 to 12, such as from 3 to 10, further such as 3 to 8, 3 to 6 carbon atoms. Examples of the monocyclic cycloalkyl group include cyclopropyl, cyclobutyl, cyclopentyl, cyclohexyl, cycloheptyl, cyclooctyl, cyclononyl, cyclodecyl, cycloundecyl, and cyclododecyl groups. In particular, examples of the saturated monocyclic cycloalkyl group, e.g., C₃₋₈cycloalkyl, include, but not limited to cyclopropyl, cyclobutyl, cyclopentyl, cyclohexyl, cycloheptyl, and cyclooctyl groups. In a preferred embodiment, the cycloalkyl is a monocyclic ring comprising 3 to 6 carbon atoms (abbreviated as C₃₋₆ cycloalkyl), including but not limited to, cyclopropyl, cyclobutyl, cyclopentyl, and cyclohexyl. Examples of the bicyclic cycloalkyl groups include those having from 7 to 12 ring atoms arranged as a fused bicyclic ring selected from [4,4], [4,5], [5,5], [5,6] and [6,6] ring systems, or as a bridged bicyclic ring selected from bicyclo[2.2.1]heptane, bicyclo[2.2.2]octane, and bicyclo[3.2.2]nonane. Further Examples of the bicyclic cycloalkyl groups include those arranged as a bicyclic ring selected from [5,6] and [6,6] ring systems.

The term "spiro cycloalkyl" includes a cyclic structure which contains carbon atoms and is formed by at least two rings sharing one atom.

The term "fused cycloalkyl" includes a bicyclic cycloalkyl group as defined herein which is saturated and is formed by two or more rings sharing two adjacent atoms.

The term "bridged cycloalkyl" includes a cyclic structure which contains carbon atoms and is formed by two rings sharing two atoms which are not adjacent to each other. The term "7 to 10 membered bridged cycloalkyl" includes a cyclic structure which contains 7 to 12 carbon atoms and is formed by two rings sharing two atoms which are not adjacent to each other.

Examples of fused cycloalkyl, fused cycloalkenyl, or fused cycloalkynyl include but are not limited to bicyclo[1.1.0]butyl, bicyclo[2.1.0]pentyl, bicyclo[3.1.0]hexyl, bicyclo[4.1.0]heptyl, bicyclo[3.3.0]octyl, bicyclo[4.2.0]octyl, decalin, as well as benzo 3 to 8 membered cycloalkyl, benzo C₄₋₆ cycloalkenyl, 2,3-dihydro-1H-indenyl, 1H-indenyl, 1, 2, 3,4-tetralyl, 1,4-dihydronaphthyl, etc. Preferred embodiments are 8 to 9 membered fused rings, which refer to cyclic structures containing 8 to 9 ring atoms within the above examples.

The term "aryl" used alone or in combination with other terms includes a group selected from:
5- and 6-membered carbocyclic aromatic rings, e.g., phenyl;
bicyclic ring systems such as 7 to 12 membered bicyclic ring systems, wherein at least one ring is carbocyclic and aromatic, e.g., naphthyl and indanyl; and,
tricyclic ring systems such as 10 to 15 membered tricyclic ring systems wherein at least one ring is carbocyclic and aromatic, e.g., fluorenyl.

The terms "aromatic hydrocarbon ring" and "aryl" are used interchangeably throughout the disclosure herein. In some embodiments, a monocyclic or bicyclic aromatic hydrocarbon ring has 5 to 10 ring-forming carbon atoms (i.e., C₅₋₁₀ aryl). Examples of a monocyclic or bicyclic aromatic hydrocarbon ring includes, but not limited to, phenyl, naphth-1-yl, naphth-2-yl, anthracenyl, phenanthrenyl, and the like. In some embodiments, the aromatic hydrocarbon ring is a naphthalene ring (naphth-1-yl or naphth-2-yl) or phenyl ring. In some embodiments, the aromatic hydrocarbon ring is a phenyl ring.

Specifically, the term "bicyclic fused aryl" includes a bicyclic aryl ring as defined herein. The typical bicyclic fused aryl is naphthalene.

The term "heteroaryl" includes a group selected from:
5-, 6- or 7-membered aromatic, monocyclic rings comprising at least one heteroatom, for example, from 1 to 4, or, in some embodiments, from 1 to 3, in some embodiments, from 1 to 2, heteroatoms, selected from nitrogen (N), sulfur (S) and oxygen (O), with the remaining ring atoms being carbon;
7- to 12-membered bicyclic rings comprising at least one heteroatom, for example, from 1 to 4, or, in some embodiments, from 1 to 3, or, in other embodiments, 1 or 2, heteroatoms, selected from N, O, and S, with the remaining ring atoms being carbon and wherein at least one ring is aromatic and at least one heteroatom is present in the aromatic ring; and
11- to 14-membered tricyclic rings comprising at least one heteroatom, for example, from 1 to 4, or in some embodiments, from 1 to 3, or, in other embodiments, 1 or 2, heteroatoms, selected from N, O, and S, with the remaining ring atoms being carbon and wherein at least one ring is aromatic and at least one heteroatom is present in an aromatic ring.

When the total number of S and O atoms in the heteroaryl group exceeds 1, those heteroatoms are not adjacent to one another. In some embodiments, the total number of S and O atoms in the heteroaryl group is not more than 2. In some embodiments, the total number of S and O atoms in the aromatic heterocycle is not more than 1. When the heteroaryl group contains more than one heteroatom ring member, the heteroatoms may be the same or different. The nitrogen atoms in the ring(s) of the heteroaryl group can be oxidized to form N-oxides.

Specifically, the term "bicyclic fused heteroaryl" includes a 7- to 12-membered, preferably 7- to 10-membered, more preferably 9- or 10-membered fused bicyclic heteroaryl ring as defined herein. Typically, a bicyclic fused heteroaryl is 5-membered/5-membered, 5-membered/6-membered, 6-membered/6-membered, or 6-membered/7-membered bicyclic. The group can be attached to the remainder of the molecule through either ring.

"Heterocyclyl", "heterocycle" or "heterocyclic" are interchangeable and include a non-aromatic heterocyclyl group comprising one or more heteroatoms selected from nitrogen, oxygen or optionally oxidized sulfur as ring members, with the remaining ring members being carbon, including monocyclic, fused, bridged, and spiro ring, i.e., containing monocyclic heterocyclyl, bridged heterocyclyl, spiro heterocyclyl, and fused heterocyclic groups.

The term "at least one substituent" disclosed herein includes, for example, from 1 to 4, such as from 1 to 3, further as 1 or 2, substituents, provided that the theory of valence is met. For example, "at least one substituent F" disclosed herein includes from 1 to 4, such as from 1 to 3, further as 1 or 2, substituents F.

The term "divalent" refers to a linking group capable of forming covalent bonds with two other moieties. For example, "a divalent cycloalkyl group" refers to a cycloalkyl group obtained by removing two hydrogen from the corresponding cycloalkane to form a linking group. the term "divalent aryl group", "divalent heterocyclyl group" or "divalent heteroaryl group" should be understood in a similar manner.

Compounds disclosed herein may contain an asymmetric center and may thus exist as enantiomers. "Enantiomers" refer to two stereoisomers of a compound which are non-superimposable mirror images of one another. Where the compounds disclosed herein possess two or more asymmetric centers, they may additionally exist as diastereomers. Enantiomers and diastereomers fall within the broader class of stereoisomers. All such possible stereoisomers as substantially pure resolved enantiomers, racemic mixtures thereof, as well as mixtures of diastereomers are intended to be included. All stereoisomers of the compounds disclosed herein and/or pharmaceutically acceptable salts thereof are intended to be included. Unless specifically mentioned otherwise, reference to one isomer applies to any of the possible isomers. Whenever the isomeric composition is unspecified, all possible isomers are included.

When compounds disclosed herein contain olefinic double bonds, unless specified otherwise, such double bonds are meant to include both E and Z geometric isomers.

When compounds disclosed herein contain a di-substituted cyclic ring system, substituents found on such ring system may adopt *cis* and *trans* formations. *Cis* formation means that both substituents are found on the upper side of the 2 substituent placements on the carbon, while *trans* would mean that they were on opposing sides. For example, the di-substituted cyclic ring system may be cyclohexyl or cyclobutyl ring.

It may be advantageous to separate reaction products from one another and/or from starting materials. The desired products of each step or series of steps is separated and/or purified (hereinafter separated) to the desired degree of homogeneity by the techniques common in the art. Typically such separations involve multiphase extraction, crystallization from a solvent or solvent mixture, distillation, sublimation, or chromatography. Chromatography can involve any number of methods including, for example: reverse-phase and normal phase; size exclusion; ion exchange; high, medium and low pressure liquid chromatography methods and apparatus; small scale analytical; simulated moving bed ("SMB") and preparative thin or thick layer chromatography, as well as techniques of small scale thin layer and flash chromatography. One skilled in the art could select and apply the techniques most likely to achieve the desired separation.

"Diastereomers" refer to stereoisomers of a compound with two or more chiral centers but which are not mirror images of one another. Diastereomeric mixtures can be separated into their individual diastereomers on the basis of their physical chemical differences by methods well known to those skilled in the art, such as by chromatography and/or fractional crystallization. Enantiomers can be separated by converting the enantiomeric mixture into a diastereomeric mixture by reaction with an appropriate optically active compound (e.g., chiral auxiliary such as a chiral alcohol or Mosher's acid chloride), separating the diastereomers and converting (e.g., hydrolyzing) the individual diastereoisomers to the corresponding pure enantiomers. Enantiomers can also be separated by use of a chiral HPLC column.

A single stereoisomer, e.g., a substantially pure enantiomer, may be obtained by resolution of the racemic mixture using a method such as formation of diastereomers using optically active resolving agents (Eliel, E. and Wilen, S. Stereochemistry of Organic Compounds. New York: John Wiley & Sons, Inc., 1994*;* Lochmuller, C. H., et al. "Chromatographic resolution of enantiomers: Selective review. "J. Chromatogr., 113(3) (1975): pp. 283-302*).* Racemic mixtures of chiral compounds of the invention can be separated and isolated by any suitable method, including: (1) formation of ionic, diastereomeric salts with chiral compounds and separation by fractional crystallization or other methods, (2) formation of diastereomeric compounds with chiral derivatizing reagents, separation of the diastereomers, and conversion to the pure stereoisomers, and (3) separation of the substantially pure or enriched stereoisomers directly under chiral conditions. See: Wainer, Irving W., Ed. Drug Stereochemistry: Analytical Methods and Pharmacology. New York: Marcel Dekker, Inc., 1993*.*

Some of the compounds disclosed herein may exist with different points of attachment of hydrogen, referred to as tautomers. For example, compounds including carbonyl -CH₂C(O)- groups (keto forms) may undergo tautomerism to form hydroxyl -CH=C(OH)- groups (enol forms). Both keto and enol forms, individually as well as mixtures thereof, are also intended to be included where applicable.

"Prodrug" refers to a derivative of an active agent that requires a transformation within the body to release the active agent. In some embodiments, the transformation is an enzymatic transformation. Prodrugs are frequently, although not necessarily, pharmacologically inactive until converted to the active agent.

"Pharmaceutically acceptable salts" refer to those salts which are, within the scope of sound medical judgment, suitable for use in contact with the tissues of humans and lower animals without undue toxicity, irritation, allergic response and the like, and are commensurate with a reasonable benefit/risk ratio. A pharmaceutically acceptable salt may be prepared in situ during the final isolation and purification of the compounds disclosed herein, or separately by reacting the free base function with a suitable organic acid or by reacting the acidic group with a suitable base. The term also includes salts of the stereoisomers (such as enantiomers and/or diastereomers), tautomers and prodrugs of the compound of the invention.

In addition, if a compound disclosed herein is obtained as an acid addition salt, the free base can be obtained by basifying a solution of the acid salt. Conversely, if the product is a free base, an addition salt, such as a pharmaceutically acceptable addition salt, may be produced by dissolving the free base in a suitable organic solvent and treating the solution with an acid, in accordance with conventional procedures for preparing acid addition salts from base compounds. Those skilled in the art will recognize various synthetic methodologies that may be used without undue experimentation to prepare non-toxic pharmaceutically acceptable addition salts.

The terms "administration", "administering", "treating" and "treatment" herein, when applied to an animal, human, experimental subject, cell, tissue, organ, or biological fluid, mean contact of an exogenous pharmaceutical, therapeutic, diagnostic agent, or composition to the animal, human, subject, cell, tissue, organ, or biological fluid. Treatment of a cell encompasses contact of a reagent to the cell, as well as contact of a reagent to a fluid, where the fluid is in contact with the cell. The term "administration" and "treatment" also means in vitro and ex vivo treatments, e.g., of a cell, by a reagent, diagnostic, binding compound, or by another cell. The term "subject" herein includes any organism, preferably an animal, more preferably a mammal (e.g., rat, mouse, dog, cat, and rabbit) and most preferably a human.

The term "effective amount" or "therapeutically effective amount" refers to an amount of the active ingredient, such as compound that, when administered to a subject for treating a disease, or at least one of the clinical symptoms of a disease or disorder, is sufficient to affect such treatment for the disease, disorder, or symptom. The term "therapeutically effective amount" can vary with the compound, the disease, disorder, and/or symptoms of the disease or disorder, severity of the disease, disorder, and/or symptoms of the disease or disorder, the age of the subject to be treated, and/or the weight of the subject to be treated. An appropriate amount in any given instance can be apparent to those skilled in the art or can be determined by routine experiments. In some embodiments, "therapeutically effective amount" is an amount of at least one compound and/or at least one stereoisomer, tautomer or prodrug thereof, and/or at least one pharmaceutically acceptable salt thereof disclosed herein effective to "treat" as defined herein, a disease or disorder in a subject. In the case of combination therapy, the term "therapeutically effective amount" refers to the total amount of the combination objects for the effective treatment of a disease, a disorder or a condition.

The term "disease" refers to any disease, discomfort, illness, symptoms or indications, and can be interchangeable with the term "disorder" or "condition".

Throughout this specification and the claims which follow, unless the context requires otherwise, the term "comprise", and variations such as "comprises" and "comprising" are intended to specify the presence of the features thereafter, but do not exclude the presence or addition of one or more other features. When used herein the term "comprising" can be substituted with the term "containing", "including" or sometimes "having".

Throughout this specification and the claims which follow, the term "Cₙ₋ₘ" indicates a range which includes the endpoints, wherein n and m are integers and indicate the number of carbons. Examples include C₁₋₈, C₁₋₆, and the like.

Unless specifically defined elsewhere in this document, all other technical and scientific terms used herein have the meaning commonly understood by one of ordinary skill in the art to which this invention belongs.

### EXAMPLES

The examples below are intended to be purely exemplary and should not be considered to be limiting in any way. Subject matter for which protection is sought is as defined by the claims. Accordingly, any compound described in this section which does not fall within the scope of the claims is present for explanatory purposes only and does not form part of the invention.

Efforts have been made to ensure accuracy with respect to numbers used (for example, amounts, temperature, etc.), but some experimental errors and deviations should be accounted for. Unless indicated otherwise, temperature is in degrees Centigrade. Reagents were purchased from commercial suppliers such as Sigma-Aldrich, Alfa Aesar, or TCI, and were used without further purification unless indicated otherwise. Unless indicated otherwise, the reactions set forth below were performed under a positive pressure of nitrogen or argon or with a drying tube in anhydrous solvents; the reaction flasks were fitted with rubber septa for the introduction of substrates and reagents via syringe; and glassware was oven dried and/or heat dried.

¹H NMR spectra were recorded on an Agilent instrument operating at 400 MHz. ¹HNMR
spectra were obtained using CDCl₃, CD₂Cl₂, CD₃OD, D₂O, d₆-DMSO, d₆-acetone or (CD₃)₂CO as solvent and tetramethylsilane (0.00 ppm) or residual solvent (CDCl₃: 7.25 ppm; CD₃OD: 3.31 ppm; D₂O: 4.79 ppm; d₆-DMSO: 2.50 ppm; d₆ -acetone: 2.05; (CD₃)₃CO: 2.05) as the reference standard. When peak multiplicities are reported, the following abbreviations are used: s (singlet), d (doublet), t (triplet), q (quartet), qn (quintuplet), sx (sextuplet), m (multiplet), br (broadened), dd (doublet of doublets), dt (doublet of triplets). Coupling constants, when given, are reported in Hertz (Hz).

LCMS-1: LC-MS spectrometer (Agilent 1260 Infinity) Detector: MWD (190-400 nm), Mass detector: 6120 SQ Mobile phase: A: water with 0.1% Formic acid, B: acetonitrile with 0.1% Formic acid Column: Poroshell 120 EC-C18, 4.6x50 mm, 2.7pm Gradient method: Flow: 1.8 mL/min Time (min) A (%) B (%)

| Time (min) | A(%) | B(%) |
|---|---|---|
| 0.00 | 95 | 5 |
| 1.5 | 5 | 95 |
| 2.0 | 5 | 95 |
| 2.1 | 95 | 5 |
| 3.0 | 95 | 5 |

LCMS, LCMS-3: LC-MS spectrometer (Agilent 1260 Infinity II) Detector: MWD (190-400 nm), Mass detector: G6125C SQ Mobile phase: A: water with 0.1% Formic acid, B: acetonitrile with 0.1% Formic acid Column: Poroshell 120 EC-C18, 4.6x50 mm, 2.7pm Gradient method: Flow: 1.8 mL/min Time (min) A (%) B (%)

| Time (min) | A(%) | B(%) |
|---|---|---|
| 0.00 | 95 | 5 |
| 1.5 | 5 | 95 |
| 2.0 | 5 | 95 |
| 2.1 | 95 | 5 |
| 3.0 | 95 | 5 |

LCMS-2: LC-MS spectrometer (Agilent 1290 Infinity II) Detector: MWD (190-400 nm), Mass detector: G6125C SQ Mobile phase: A: water with 0.1% Formic acid, B: acetonitrile with 0.1% Formic acid Column: Poroshell 120 EC-C18, 4.6x50 mm, 2.7pm Gradient method: Flow: 1.2 mL/min Time (min) A (%) B (%)

| Time (min) | A(%) | B(%) |
|---|---|---|
| 0.00 | 90 | 10 |
| 1.5 | 5 | 95 |
| 2.0 | 5 | 95 |
| 2.1 | 90 | 10 |
| 3.0 | 90 | 10 |

Preparative HPLC was conducted on a column (150 x 21.2 mm ID, 5 pm, Gemini NXC 18) at a flow rate of 20 ml/min, injection volume 2 ml, at room temperature and UV Detection at 214 nm and 254 nm.

In the following examples, the abbreviations below are used:

| | |
|---|---|
| (BPin)₂ | 4,4,4',4',5,5,5',5'-Octamethyl-2,2'-bi-1,3,2-dioxaborolane |
| Ac₂O | acetic anhydride |
| AcCl | Acetyl chloride |
| Ac | Acetyl |
| ACN or MeCN | Acetonitrile |
| AcOH or HOAc | Acetic acid |
| AcONa or NaOAc | Sodium acetate |
| Aq | Aqueous |
| BINAP | (±)-2,2'-Bis(diphenylphosphino)-1,1'-BINAPhthyl |
| Bn | benzyl |
| BnBr | Benzyl Bromide |
| Boc | t-Butyloxy carbonyl |
| Boc₂O | Di-tert-butyl pyrocarbonate |
| C:40691-33-6 | dichlorobis(tri-o-tolylphosphine)palladium(II) |
| Cbz | Benzyloxycarbonyl |
| CDI | N,N'-Carbonvldiimidazole |
| DCM | Dichloromethane |
| Con. or conc. | Concentrated |
| DavePhos | 2'-(Dicyclohexylphosphino)-N,N-dimethyl-2-biphenylamine |
| DBU | 1, 8-diazabicyclo[5.4.0]undec-7-ene |
| DCE | dichloroethane |
| DHP | 3,4-Dihydro-2H-pyran |
| DIBAL-H | Diisobutylaluminium hydride |
| DIEA or DIPEA | N, N-diisopropylethylamine |
| DMAP | 4-N, N-dimethylaminopyridine |
| DMP | Dess-Martin periodinane |
| DMF | N,N-Dimethylformamide |
| DMSO | Dimethyl sulfoxide |
| Dppf | 1, 1"-bis(diphenylphosphino)ferrocene |
| EA or EtOAc | Ethyl acetate |
| Et | Ethyl |
| EtOH | ethanol |
| FA | Formic acid |
| h or hr | Hour |
| HATU | 2-(7-Azabenzotriazol-1-yl)-N,N,N',N'-tetramethyluronium hexafluorophosphate |
| HBTU | O-(7- Benzotriazole-1-yl)-N, N, N', N'-tetramethyluronium hexafluorophosphate |
| Hex | Hexane |
| HPLC | High Performance Liquid Chromatography |
| hrs | hours |
| IBX | 2-Iodoxybenzoic acid |
| IPA | 2-propanol |
| i-PrOH or iPrOH | Isopropyl alcohol |
| KHMDS | Potassium bis(trimethylsilyl)amide |
| LiHMDS | Lithium bis(trimethylsilyl)amide |
| KOAc or AcOK | Potassium Acetate |
| MeCN or ACN | Acetonitrile |
| MeOH | Methanol |
| Min | Minutes |
| ms or MS | Mass spectrum |
| Ms | Methanesulfonyl |
| MsCl | Methanesulfonyl chloride |
| MsOH | Methanesulfonic acid |
| MTBE | Methyl tert-butyl ether |
| NMP | N-Methyl pyrrolidone |
| NMR | Nuclear Magnetic Resonance |
| o/n | overnight |
| Pd(dppf)Cl₂ | [1,1'-Bis(diphenylphosphino)ferrocene]dichloropalladium(II) |
| Pd₂(dba)₃ | Tris(dibenzylideneacetone)dipalladium |
| Pd(dtBPF)Cl₂ | [1,1'-Bis(di-tert-butylphosphino)ferrocene]dichloropalladium(II) |
| Pd(PPh₃)₂Cl₂ | Bis(triphenylphosphine)palladium(II) dichloride |
| PE | Petroleum ether |
| PhMe | Toluene |
| PPh₃ | triphenylphosphine |
| PMB | 4-Methoxybenzyl |
| PPA | Polyphosphoric acid |
| pre-HPLC or prep-HPLC | Preparation High Performance Liquid Chromatography |
| Prep-TLC | Preparation Thin Layer Chromatography |
| R.T. or r.t. | Room temperature |
| Rt | Retention time |
| Ruphos-G3 | (2-Dicyclohexylphosphino-2',6'-diisopropoxy-1,1'-biphenyl)[2-(2'-amin o-1,1'-biphenyl)]palladium(II) methanesulfonate |
| SEMCl | 2-(Trimethylsilyl)ethoxymethyl chloride |
| SEM | 2-(Trimethylsilyl)ethoxymethyl |
| STAB | Sodium Triacetoxyborohydride;Sodium triacetoborohydride |
| Sat. | saturated |
| TBAF | Tetra-butyl ammonium fluoride |
| TBDPS | tert-Butyldiphenylsilyl |
| TBS | tert-Butyldimethylsilyl |
| TBSCl | tert-Butyldimethylsilyl chloride |
| Ti(OiPr)4 | Titanium tetraisopropanolate |
| t-Bu | tert-butyl |
| t-BuOH | tert-Butanol |
| t-BuONa | Sodium tert-butoxide |
| t-BuOK | Potassium tert-butoxide |
| TEA | Triethylamine |
| Tf₂O | Triflic anhydride |
| TFA | Trifluoroacetic acid |
| THF | Tetrahydrofuran |
| TLC | Thin layer chromatography |
| TMSOK | Potassium trimethylsilanolate |
| Ts | para-Toluenesulfonyl |
| TsCl | 4-Toluenesulfonyl chloride |
| TsOH | p-toluenesulfonic acid |
| TsOH,Py | Pyridinium toluene-4-sulphonate |
| Xphos or X-phos | 2-Dicyclohexylphosphino-2',4',6'-triisopropylbiphenyl |
| XantPhos | 4,5-Bis(diphenylphosphino)-9,9-dimethylxanthene |

### Example 1:

### 3-(4-(4-(2-(4-(1-(4-((5-bromo-4-((4-(dimethylphosphoryl)quinolin-3-yl)amino)pyrimidin-2-yl)amino)-2-ethyl-5-methoxyphenyl)piperidin-4-yl)piperazin-1-yl)ethyl)piperidin-1-yl)-2,6-difluorophenyl)piperidine-2,6-dione

### Step 1: 4-iodoquinolin-3-amine

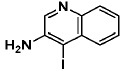

To a solution of quinolin-3-amine (10 g, 69.4 mmol) in AcOH (50 mL) was added iodine chloride(14.62 g, 90.22 mmol) in AcOH(20 mL) dropwise at 5 °C. After addition, the reaction mixture was stired at room temperature for 5 hours. The resulting mixture was diluted with water and extracted with EtOAc (3 x 500 mL). The combined organic layers were washed with brine (500 mL), dried over anhydrous Na₂SO₄. After filtration, the filtrate was concentrated under reduced pressure to afford the product (9.9 g, 52.8%). [M+H]⁺ = 271.1.

### Step 2: (3-aminoquinolin-4-yl)dimethylphosphine oxide

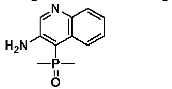

A mixture of 4-iodoquinolin-3-amine (9.0 g, 33.3 mmol), dimethylphosphine oxide (5.2 g, 66.6 mmol), Pd(OAc)₂ (746 mg, 3.33 mmol), Xantphos (3.85 g, 6.66mmol) and K₃PO₄ (21.18 g, 99.9 mmol) in dioxane (150 mL) was stirred in a round bottom flask at 100 °C under nitrogen atmosphere for 16 hrs. The reaction mixture was allowed to cool down to room temperature. The resulting mixture was diluted with water (200 mL) and extracted with DCM (3 x 200 mL). The combined organic layers were washed with brine (1000 mL), dried over anhydrous Na₂SO₄. After filtration, the filtrate was concentrated under reduced pressure. The residue was purified by silica gel column chromatography, eluted with DCM/MeOH (10: 1) to afford the product (6.5 g, 81.3%), [M+H]⁺ = 221.3.

### Step 3: (3-((5-bromo-2-chloropyrimidin-4-yl)amino)quinolin-4-yl)dimethylphosphine oxide

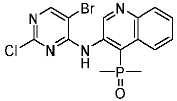

A mixture of (3-aminoquinolin-4-yl)dimethylphosphine oxide (5.5 g, 25.1 mmol), 5-bromo-2,4-dichloropyrimidine (11.4 g, 50.2 mmol) in THF (90 mL) was stirred in a round bottom flask at 0 °C under nitrogen atmosphere, LiHMDS (32.6 mL, 32.6 mmol) was added. The reaction mixture was allowed to warm up to room temperature for 2 hours. The reaction was quenched with water and the mixture was extracted with DCM (3 x 50 mL), washed triple with saturated brine, dried over anhydrous Na₂SO₄. After filtration, the filtrate was concentrated under reduced pressure. The residue was purified by silica gel column chromatography, eluted with DCM/MeOH (10: 1) to afford the product (4.7 g, 46.1%). [M+H]⁺ = 411.2.

### Step 4:

### (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide

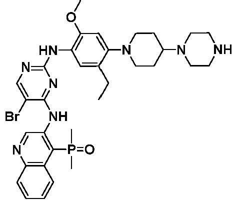

A mixture of (3-((5-bromo-2-chloropyrimidin-4-yl)amino)quinolin-4-yl)dimethylphosphine oxide (2.2 g, 5.35 mmol), tert-butyl 4-(1-(4-amino-2-ethyl-5-methoxyphenyl)piperidin-4-yl)piperazine-1-carboxylate (2.46 g, 5.89 mmol) and TsOH (2.76 g, 16.05 nm) in n-BuOH (40 mL) was stirred in a round bottom flask at 100 °C under nitrogen atmosphere for 16 h. The reaction mixture was concentrated under reduced pressure. The residue was dissolved in water and adjusted pH > 8. The resulting mixture was extracted with DCM (3 x 60 mL). The combined organic layers were washed with brine (100 mL), dried over anhydrous Na₂SO₄. After filtration, the filtrate was concentrated under reduced pressure. the residue was purified by silica gel column chromatography to afford the product (1.5 g, 40.5%). [M+H]⁺ = 693.1.

### Step 5: 2,6-bis(benzyloxy)-3-(4,4,5,5-tetramethyl-1,3,2-dioxaborolan-2-yl)pyridine

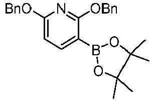

A mixture of 2,6-bis(benzyloxy)-3-bromopyridine (15 g, 40.65 mmol) and 4,4,4',4',5,5,5',5'-octamethyl-2,2'-bi(1,3,2-dioxaborolane) (12.6 g, 49.61 mmol), Pd(dppf)Cl₂ (3.32 g, 4.07mmol), KOAc (12 g, 122.45mmol) in dioxane (200 mL) was stirred overnight at 100 °C under nitrogen atmosphere. The resulting mixture was filtered, the filter cake was washed with MeOH and DCM. The filtrate was concentrated under reduced pressure. The residue was purified by silica gel column chromatography, eluted with PE/EtOAc (8:1) to afford the product (9.00 g, 53%). m/z[M+H]⁺ = 418.3.

### Step 6: 2,6-bis(benzyloxy)-3-(4-bromo-2,6-difluorophenyl)pyridine

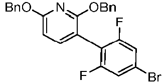

A mixture of 2,6-bis(benzyloxy)-3-(4,4,5,5-tetramethyl-1,3,2-dioxaborolan-2-yl)pyridine (9.00 g, 21.56 mmol) and 5-bromo-1,3-difluoro-2-iodobenzene (6.88 g, 21.57 mmol), K₂CO₃ (10.43 g, 75.48 mmol), Pd(dppf)Cl₂ (789 mg, 1.078 mmol) in dioxane (90 mL) and H₂O (30 mL) was stirred for 16h at 100 °C under nitrogen atmosphere. The resulting mixture was extracted with EtOAc (50 mL x 3). The combined organic layers were washed with brine, dried over anhydrous Na₂SO₄. After filtration, the filtrate was concentrated under reduced pressure. The residue was purified by silica gel column chromatography, eluted with PE/EtOAc (5:1) to afford the product (4 g, 38%). [M+H] ⁺ = 482.4.

### Step 7: ethyl 2-(1-(4-(2,6-bis(benzyloxy)pyridin-3-yl)-3,5-difluorophenyl)piperidin-4-yl)acetate

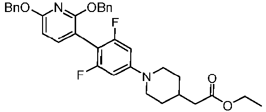

A mixture of 2,6-bis(benzyloxy)-3-(4-bromo-2,6-difluorophenyl)pyridine (4.00 g, 8.29 mmol), ethyl 2-(piperidin-4-yl)acetate (2.13 g, 12.43 mmol), Cs₂CO₃ (8.11 g, 24.89 mmol), DavePhos (652.7 mg, 1.659 mmol), Pd₂(dba)₃ (759.4 mg, 0.829 mmol) in 2-methyl-THF (50 mL) and H₂O (5 mL) was stirred overnight at 100 °C under nitrogen atmosphere. The resulting mixture was extracted with EtOAc. The combined organic layers were washed with brine, dried over anhydrous Na₂SO₄. After filtration, the filtrate was concentrated under reduced pressure. The residue was purified by silica gel column chromatography, eluted with PE/EtOAc (1:1) to afford the product (2 g, 42 %). [M+1]⁺ = 573.1.

### Step 8: 2-(1-(4-(2,6-bis(benzyloxy)pyridin-3-yl)-3,5-difluorophenyl)piperidin-4-yl)ethan-1-ol

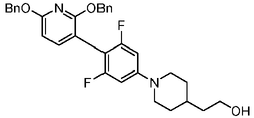

To a stirred mixture of ethyl 2-(1-(4-(2,6-bis(benzyloxy)pyridin-3-yl)-3,5-difluorophenyl)piperidin-4-yl)acetate (2.00 g, 3.49 mmol) in THF (50 mL) was added LiBH₄ (1.52 g, 69.77 mmol) in portions at room temperature overnight. The reaction was quenched with water (20 mL) at room temperature. The resulting mixture was extracted with EtOAc (3 x 50 mL). The combined organic layers were washed with brine, dried over anhydrous Na₂SO₄. After filtration, the filtrate was concentrated under reduced pressure to afford the product (1.8 g, 97%) [M+1] ⁺= 531.2.

### Step 9: 3-(2,6-difluoro-4-(4-(2-hydroxyethyl)piperidin-1-yl)phenyl)piperidine-2,6-dione

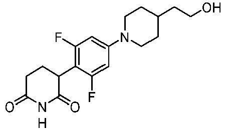

To a stirred mixture of 2-(1-(4-(2,6-bis(benzyloxy)pyridin-3-yl)-3,5-difluorophenyl)piperidin-4-yl)ethan-1-ol (1.80 g, 3.39 mmol) and Pd/C (1 g, 10% wt) in EtOH (20 mL) and DCM (20 mL) were added AcOH (20 mL) at rt and stirred at 40 °C under hydrogen atmosphere overnight. The resulting mixture was filtered, the filter cake was washed with MeOH (20 mL). The filtrate was concentrated under reduced pressure to afford the product (1.2 g, 100%). [M+1] ⁺=353.3.

### Step 10: 2-(1-(4-(2,6-dioxopiperidin-3-yl)-3,5-difluorophenyl)piperidin-4-yl)acetaldehyde

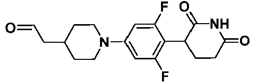

The titled compound (1.4 g, 92%) was prepared in a manner similar to that in Example 2 step 6 from 3-(2,6-difluoro-4-(4-(2-hydroxyethyl)piperidin-1-yl)phenyl)piperidine-2,6-dione and IBX. [M+H]⁺ = 351.2.

### Step 11:

### 3-(4-(4-(2-(4-(1-(4-((5-bromo-4-((4-(dimethylphosphoryl)quinolin-3-yl)amino)pyrimidin-2-yl)amino)-2-ethyl-5-methoxyphenyl)piperidin-4-yl)piperazin-1-yl)ethyl)piperidin-1-yl)-2,6-difluorophenyl)piperidine-2,6-dione

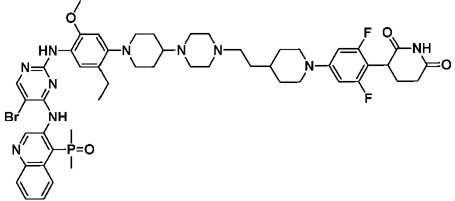

To a solution of (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide (66 mg, 0.1 mmol), and 2-(1-(4-(2,6-dioxopiperidin-3-yl)-3,5-difluorophenyl)piperidin-4-yl)acetaldehyde (42 mg, 0.12 mmol) in DCM (6 mL) and MeOH (1 mL) was added acetic acid (12 mg, 0.2 mmol) at room temperature. After stirring at room temperature for 1h, NaBH(OAc)₃ (64 mg, 0.3 mmol) was added and the resulting mixture was stirred at room temperature for 2h. The reaction was quenched with water (15 mL) and extracted with DCM (2 x 30 mL). The combined organic layers were dried over anhydrous Na₂SO₄, filtered and evaporated in vacuum to afford the crude residue, which was purified by silica gel column chromatography (DCM: MeOH = 100: 0~10: 1 gradient elution) to give an impure product, which was further purified with pre-HPLC (C-18 column chromatography (0.1% FA in water : acetonitrile = 90 : 10 ~ 60 : 40 gradient elution) to give the desired product (35 mg, 36%). ¹H NMR (400 MHz, DMSO) δ_{H} 11.68 (s, 1H), 10.86 (s, 1H), 9.45 (s, 1H), 8.25 (s, 1H), 8.18 (d, J = 8.3 Hz, 1H), 8.03 - 7.92 (m, 2H), 7.72 (t, J = 7.6 Hz, 1H), 7.64 (t, J = 7.8 Hz, 1H), 7.41 (s, 1H), 6.70 (s, 1H), 6.60 (d, J = 12.8 Hz, 2H), 4.04 (dd, J = 12.7, 5.1 Hz, 1H), 3.77 (s, 5H), 2.92 (d, J = 10.2 Hz, 2H), 2.75 (ddd, J = 32.2, 21.0, 8.5 Hz, 4H), 2.58 (dd, J = 32.2, 20.9 Hz, 5H), 2.41 - 2.19 (m, 9H), 2.06 (t, J = 9.4 Hz, 7H), 1.98 - 1.90 (m, 1H), 1.81 (d, J = 10.7 Hz, 2H), 1.71 (d, J = 11.2 Hz, 2H), 1.51 (dd, J = 21.9, 10.0 Hz, 3H), 1.41 - 1.33 (m, 2H), 1.22 - 1.13 (m, 2H), 0.75 (s, 3H); [M+H]⁺ = 1027.7.

### Example 2:

### 3-(4-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-2-fluorophenyl)piperidine-2,6-dione

### Step 1: 2-(4-bromo-3-fluorophenyl)ethan-1-ol

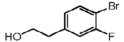

To a solution of 2-(4-bromo-3-fluorophenyl)acetic acid (45.0 g, 193 mmol) in THF (270 mL) was added BH₃·THF (1 M, 386 mL) at 0 °C. Then the mixture was stirred at 20 °C for 2 hrs. Under cooling with ice, MeOH (250 mL) was dropwise added until there was no foaming in the system and the solvent was distilled off under reduced pressure. To the resulting reside, water (50 mL) was added for extraction with EtOAc (3 x 100 mL). The combined organic phase was washed with brine (40 mL), dried over Na₂SO₄, filtered and concentrated in vacuum. 2-(4-bromo-3-fluorophenyl)ethan-1-ol (38.0 g, 89.8%) was obtained. ¹HNMR (400 MHz, CDCl₃-d) δ ppm 7.45 (t, *J =* 7.72 Hz, 1 H), 7.00 (dd, *J =* 9.48, 1.76 Hz, 1 H), 6.86 - 6.92 (m, 1 H), 3.82 (t, *J =* 6.50 Hz, 2 H), 2.80 (t, *J* = 6.50 Hz, 2 H), 2.03 (s, 1 H); [M+H]⁺ = 219.3.

### Step 2: (4-bromo-3-fluorophenethoxy)(tert-butyl)dimethylsilane

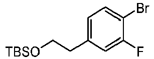

To a solution of 2-(4-bromo-3-fluorophenyl)ethan-1-ol (38.0 g, 173 mmol) in DCM (210 mL) was added imidazole (17.7 g, 260 mmol) at 20 °C. TBSCl (36.6 g, 242 mmol, 29.7 mL) was added to the reaction mixture at 0 °C. Then the mixture was stirred at 20 °C for 3 hrs. Then the mixture was adjusted to pH = 6 with 5 % citric acid (180 mL), and extracted with DCM (150 mL). The organic phase was adjusted to pH = 8 with NaHCO₃ and then aqueous phase was extracted with DCM (100 mL). The combined organic phase was washed with brine (150 mL), dried over Na₂SO₄, filtered and concentrated in vacuum.

(4-bromo-3-fluorophenethoxy)(tert-butyl)dimethylsilane (52.0 g, 156 mmol) was obtained. ¹HNMR (400 MHz, CDCl₃-*d*) δ ppm 7.43 (t, *J =* 7.72 Hz, 1 H), 7.00 (dd, *J* = 9.56, 1.87 Hz, 1 H), 6.89 (dd, *J* = 8.00, 1.87 Hz, 1 H), 3.80 (t, *J =* 6.48 Hz, 2 H), 2.78 (t, *J =* 6.48 Hz, 2 H), 0.84 - 0.89 (m, 9 H), - 0.05 - 0.01 (m, 6 H); [M+H]⁺ = 333.2.

### Step 3: 2,6-bis(benzyloxy)-3-(4-(2-((tert-butyldimethylsilyl)oxy)ethyl)-2-fluorophenyl)pyridine

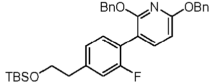

To a solution of (4-bromo-3-fluorophenethoxy)(tert-butyl)dimethylsilane (52.0 g, 156 mmol) and 2,6-bis(benzyloxy)-3-(4,4,5,5-tetramethyl-1,3,2-dioxaborolan-2-yl)pyridine (65.1 g, 156 mmol) in dioxane (320 mL) was added KOAc (45.9 g, 468 mmol) at 20 °C. Pd(dppf)Cl₂ (11.4 g, 15.6 mmol) was added to the mixture at 20 °C. The suspension was degassed under vacuum and purged with N₂ three times. Then the mixture was stirred at 90 °C for 16 hrs. Water (160 mL) was poured into the mixture, extrated with EtOAc (100 mL). The combined organic phase was washed with brine (100 mL), dried over Na₂SO₄, filtered and concentrated in vacuum. The residue was purified by column chromatography.

2,6-bis(benzyloxy)-3-(4-(2-((tert-butyldimethylsilyl)oxy)ethyl)-2-fluorophenyl)pyridine (32.0 g, 37.8%) was obtained. ¹HNMR (400 MHz, CDCl₃-d) δ ppm 7.55 (dd, J = 8.04, 0.99 Hz, 1 H), 7.43 - 7.47 (m, 2 H), 7.33 - 7.42 (m, 7 H), 7.25 - 7.33 (m, 3 H), 6.98 - 7.05 (m, 2 H), 5.40 (d, J = 18.4 Hz, 4 H), 3.87 (t, J = 6.84 Hz, 1 H), 3.84 - 3.89 (m, 1 H), 2.86 (t, J = 6.84 Hz, 2 H), 0.88 - 0.92 (m, 9 H), 0.01 - 0.03 (m, 6 H); [M+H]⁺ = 544.2.

### Step 4: 3-(4-(2-((tert-butyldimethylsilyl)oxy)ethyl)-2-fluorophenyl)piperidine-2,6-dione

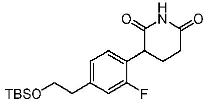

To a solution of 2,6-bis(benzyloxy)-3-(4-(2-((tert-butyldimethylsilyl)oxy)ethyl)-2-fluorophenyl)pyridine (32.0 g, 58.8 mmol) in THF (50.0 mL) was added Pd/C (0.800 g, 10.0% purity) under Ar at 20 °C. The suspension was degassed and purged with H₂ for 3 times. The mixture was stirred under H₂ (50 Psi) at 50 °C for 16 hrs. The suspension was filtered through a pad of celite and the filter cake was washed with THF (200 mL x 3). The combined filtrates were concentrated to dryness to give crude product. The crude product was triturated with petroleum ether (50.0 mL) at 20 °C for 1 hrs.

3-(4-(2-((tert-butyldimethylsilyl)oxy)ethyl)-2-fluorophenyl)piperidine-2,6-dione (12.0 g, 55.7%) was obtained. ¹HNMR (400 MHz, CDCl₃-*d*) δ ppm 7.06 - 7.12 (m, 1 H) 7.93 (br s, 1 H), 6.96 - 7.04 (m, 2 H), 3.91 (dd, *J =* 11.2, 5.04 Hz, 1 H), 3.81 (t, *J =* 6.80 Hz, 2 H), 2.82 (t, *J =* 6.80 Hz, 2 H), 2.58 - 2.73 (m, 2 H), 2.18 - 2.34 (m, 2 H), 0.87 (s, 9 H), 0.00 (s, 6 H); [M+H]⁺ = 366.3.

### Step 5: 3-(2-fluoro-4-(2-hydroxyethyl)phenyl)piperidine-2,6-dione

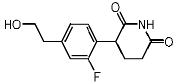

To a solution of 3-(4-(2-((tert-butyldimethylsilyl)oxy)ethyl)-2-fluorophenyl)piperidine-2,6-dione (12.0 g, 32.8 mmol) in MeOH (60 mL) was added HCl (12 M, 6 mL) at 20 °C. Then the mixture was stirred at 20 °C for 3 hrs. Water (60 mL) was poured into the mixture, extracted with EtOAc (40 mL). The combined organic phase was washed with brine (40 mL), dried over Na₂SO₄, filtered and concentrated in vacuum. The combined crude product was purified by re-crystallization from toluene (32.0 mL) at 100 °C.

3-(2-fluoro-4-(2-hydroxyethyl)phenyl)piperidine-2,6-dione (6.50 g, 78.8%) was obtained. ¹HNMR (400 MHz, DMSO-*d*₆)δ ppm 10.8 (s, 1 H), 7.19 (t, *J =* 7.84 Hz, 1 H), 6.99 - 7.08 (m, 2 H), 4.67 (t, *J =* 5.18 Hz, 1 H), 3.99 (dd, *J* = 12.6, 4.74 Hz, 1 H), 3.55 - 3.66 (m, 2 H), 2.68 - 2.75 (m, 3 H), 2.18 (qd, *J* = 12.8, 3.86 Hz, 1 H), 1.93 - 2.03 (m, 1 H); [M+H]⁺ = 252.1.

### Step 6: 2-(4-(2,6-dioxopiperidin-3-yl)-3-fluorophenyl)acetaldehyde

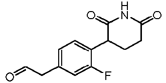

To a solution of 3-(2-fluoro-4-(2-hydroxyethyl)phenyl)piperidine-2,6-dione (200 mg, 0.8 mmol) in DMSO (10 mL) was added IBX (338 mg, 1.2 mmol). The mixture was stirred in a flask at rt overnight. After being determined the reaction to be completed by LCMS, the mixture was extracted with EA (30 mL). The combined organic phase was dried over anhydrous Na₂SO₄, and evaporated in vacuum to afford the crude product (100 mg, crude), which was used for next step without further purification. [M+H]⁺ = 250.2.

### Step 7:

### 3-(4-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-2-fluorophenyl)piperidine-2,6-dione

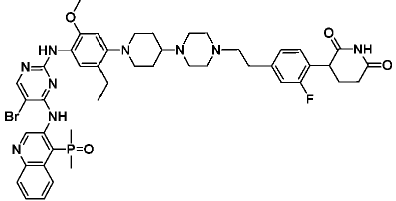

The titled compound (1.4 g, 92%) was prepared in a manner similar to that in Example 1 step 11 from (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide and 2-(4-(2,6-dioxopiperidin-3-yl)-3-fluorophenyl)acetaldehyde. ¹H NMR (500 MHz, DMSO) δ_{H} 11.68 (s, 1H), 10.86 (s, 1H), 9.45 (s, 1H), 8.25 (s, 1H), 8.19 (d, J = 8.3 Hz, 1H), 8.05 - 7.93 (m, 2H), 7.71 (d, J = 7.4 Hz, 1H), 7.65 (d, J = 8.0 Hz, 1H), 7.41 (s, 1H), 7.20 (t, J = 7.9 Hz, 1H), 7.06 (dd, J = 20.6, 9.8 Hz, 2H), 6.70 (s, 1H), 4.00 (dd, J = 12.5, 4.8 Hz, 1H), 3.77 (s, 3H), 2.93 (d, J = 10.9 Hz, 2H), 2.74 (dd, J = 15.1, 7.7 Hz, 3H), 2.65 - 2.51 (m, 10H), 2.48 - 2.40 (m, 3H), 2.33 (d, J = 7.1 Hz, 3H), 2.19 (dt, J = 13.0, 9.0 Hz, 1H), 2.05 (d, J = 13.5 Hz, 6H), 1.99 (dd, J = 9.1, 4.0 Hz, 1H), 1.83 (d, J = 11.2 Hz, 2H), 1.53 (d, J = 8.8 Hz, 2H), 0.75 (s, 3H); [M+H]⁺ = 926.5.

### Example 4:

3-(5-((R)-3-((4-(1-(4-((5-bromo-4-((4-(dimethylphosphoryl)quinolin-3-yl)amino)pyrimidin-2-yl)amino)-2-ethyl-5-methoxyphenyl)piperidin-4-yl)piperazin-1-yl)methyl)pyrrolidin-1-yl)-1-oxoisoindolin-2-yl)piperidine-2,6-dio ne

### Step 1: methyl (S)-2-cyano-4-(3-(hydroxymethyl)pyrrolidin-1-yl)benzoate

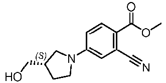

To a stirred mixture of methyl 2-cyano-4-fluorobenzoate (10.0 g, 55.819 mmol) and (S)-pyrrolidin-3-ylmethanol (6.8 g, 66.983 mmol) in DMSO (80 mL) was added DIEA (14.4 g, 111.638 mmol) and stirred overnight at 60 ° C. The resulting mixture was extracted with EtOAc (500 mL). The combined organic layers were washed with brine (100 mL), dried over anhydrous Na₂SO₄. After filtration, the filtrate was concentrated under reduced pressure. methyl (S)-2-cyano-4-(3-(hydroxymethyl)pyrrolidin-1-yl)benzoate (10.0 g, 98.3%) was obtained. [M+H]⁺ = 261.2.

### Step 2: methyl (S)-2-formyl-4-(3-(hydroxymethyl)pyrrolidin-1-yl)benzoate

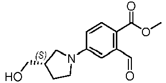

To a stirred mixture of methyl (S)-2-cyano-4-(3-(hydroxymethyl)pyrrolidin-1-yl)benzoate (10.0 g, 38.418 mmol) in AcOH (100 mL) and H₂O (50 mL) was added Raney-Ni (10.0 g, 116.720 mmol) in portions and stirred overnight at 40° C under hydrogen atmosphere. The resulting mixture was filtered, the filter cake was washed with DCM and MeOH (300 mL). The filtrate was concentrated under reduced pressure. The filtrate was extracted with EtOAc (500 mL). The combined organic layers were washed with brine (500 mL), dried over anhydrous Na₂SO₄. After filtration, the filtrate was concentrated under reduced pressure. The residue was purified by silica gel column chromatography, eluted with CH₂Cl₂/MeOH (9:1) to afford product (5.0 g, 49.4%). [M+H]⁺ = 264.1.

### Step 3: 3-(5-((S)-3-(hydroxymethyl)pyrrolidin-1-yl)-1-oxoisoindolin-2-yl)piperidine-2,6-dione

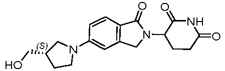

A mixture of 3-aminopiperidine-2,6-dione hydrochloride (4.7 g, 28.485 mmol) and DIEA (4.9 g, 37.980 mmol) in DMF (50 mL) was stirred for 5 h at room temperature . The mixture was acidified to pH < 7 with AcOH (5.7 g, 94.950 mmol) followed by the addition of methyl

(S)-2-formyl-4-(3-(hydroxymethyl)pyrrolidin-1-yl)benzoate (5.0 g, 18.990 mmol) in DCE (10.00 mL) and DMF (2 mL) at room temperature. The resulting mixture was stirred overnight at room temperature. To the above mixture was added NaBH₃CN (3.6 g, 56.970 mmol) in portions at room temperature. The resulting mixture was stirred for additional 2 h at room temperature. The reaction was quenched with water (50 mL) at room temperature. The resulting mixture was concentrated under reduced pressure. The residue was purified by silica gel column chromatography, eluted with CH₂Cl₂ /MeOH (5: 1) to afford crude product, which was purified by trituration with DCM (40 mL) to afford the product (2.1 g, 33.2%). [M+H]⁺ = 344.0.

### Step 4: ((3S)-1-(2-(2,6-dioxopiperidin-3-yl)-1-oxoisoindolin-5-yl)pyrrolidin-3-yl)methyl methanesulfonate

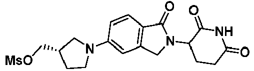

The titled compound (120 mg, 44%) was prepared in a manner similar to that in Example 19 step 4 from 3-(5-((S)-3-(hydroxymethyl)pyrrolidin-1-yl)-1-oxoisoindolin-2-yl)piperidine-2,6-dione and MsCl. [M+H]⁺ = 422.2.

### Step 5:

### 3-(5-((R)-3-((4-(1-(4-((5-bromo-4-((4-(dimethylphosphoryl)quinolin-3-yl)amino)pyrimidin-2-yl)amino)-2-ethyl-5-methoxyphenyl)piperidin-4-yl)piperazin-1-yl)methyl)pyrrolidin-1-yl)-1-oxoisoindolin-2-yl)piperidine-2,6-dio ne

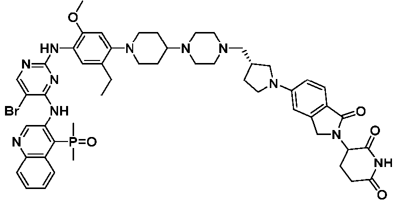

The titled compound (12 mg, 25%) was prepared in a manner similar to that in Example 19 step 5 from (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide and ((3S)-1-(2-(2,6-dioxopiperidin-3-yl)-1-oxoisoindolin-5-yl)pyrrolidin-3-yl)methyl methanesulfonate. ¹H NMR (400 MHz, DMSO) δ_{H} 11.68 (s, 1H), 10.93 (s, 1H), 9.44 (s, 1H), 8.25 (s, 1H), 8.19 (d, J = 8.3 Hz, 1H), 8.03 - 7.92 (m, 2H), 7.72 (t, J = 7.7 Hz, 1H), 7.64 (t, J = 7.4 Hz, 1H), 7.48 (d, J = 8.3 Hz, 1H), 7.41 (s, 1H), 6.70 (s, 1H), 6.62 (d, J = 9.3 Hz, 2H), 5.03 (dd, J = 13.3, 5.4 Hz, 1H), 4.30 (d, J = 20.1 Hz, 1H), 4.18 (d, J = 16.5 Hz, 1H), 3.78 (s, 3H), 3.42 (d, J = 29.0 Hz, 2H), 3.05 (d, J = 10.2 Hz, 1H), 2.97 - 2.85 (m, 4H), 2.64 - 2.54 (m, 8H), 2.40 - 2.28 (m, 9H), 2.05 (d, J = 13.5 Hz, 8H), 1.95 (s, 1H), 1.83 (s, 2H), 1.74 (s, 1H), 1.53 (d, J = 26.3 Hz, 2H), 0.75 (s, 3H); [M+H]⁺ = 1018.7.

### Example 7:

### 3-(4-(2-(4-(1-(4-((5-bromo-4-((4-(dimethylphosphoryl)-1,5-naphthyridin-3-yl)amino)pyrimidin-2-yl)amino)-2-et hyl-5-methoxyphenyl)piperidin-4-yl)piperazin-1-yl)ethyl)-2,6-difluorophenyl)piperidine-2,6-dione

### Step 1: 4-iodo-1,5-naphthyridin-3-amine

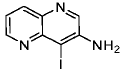

To a solution of 1,5-naphthyridin-3-amine (1.0 g, 6.9 mmol) and Ag₂SO₄ (2.4 g, 6.9 mmol) in EtOH (40 mL) was added I₂ (1.91 g, 6.9 mmol) at 0 °C. Then the mixture was stirred at 30 °C for 2 hrs. The resulting mixture was extracted with DCM (3 x 100 mL). The combined organic layers were washed with brine (100 mL), dried over anhydrous Na₂SO₄. After filtration, the filtrate was concentrated under reduced pressure. The residue was purified by silica gel column chromatography, eluted with DCM/MeOH(15: 1) to afford the product (0.4 g, 23%). [M+H]⁺ = 271.2.

### Step 2: (3-amino-1,5-naphthyridin-4-yl)dimethylphosphine oxide

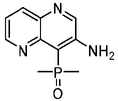

A mixture of 4-iodo-1,5-naphthyridin-3-amine (100 mg, 1.5 mmol), dimethylphosphine oxide (233 mg, 3.0 mmol), Pd(OAc)₂ ( 33 mg, 0.15 mmol) Xanphos (170 mg, 0.3 mmol) and K₃PO₄ ( 950 mg, 4.5 mmol) in dioxane(20 mL) was stirred in a round bottom flask at 100 °C under nitrogen atmosphere for 16 hrs. The reaction mixture was allowed to cool down to room temperature. The resulting mixture was extracted with DCM (3 x 100 mL). The combined organic layers were washed with brine (100 mL), dried over anhydrous Na₂SO₄. After filtration, the filtrate was concentrated under reduced pressure. The residue was purified by silica gel column chromatography, eluted with DCM/MeOH (10: 1) to afford the product (300 mg, 90%), [M+H]+ = 222.0.

### Step 3: (3-((5-bromo-2-chloropyrimidin-4-yl)amino)-1,5-naphthyridin-4-yl)dimethylphosphine oxide

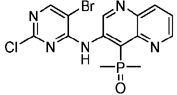

A mixture of (3-amino-1,5-naphthyridin-4-yl)dimethylphosphine oxide (300 mg, 1.35 mmol), 5-bromo-2,4-dichloropyrimidine (610 mg, 2.7 mmol) in DMF (20 mL) was stirred in a round bottom flask at 0 °C under nitrogen atmosphere, NaH (100 mg, 2.7 mmol ) was added. The reaction mixture was allowed to warm up to room temperature for 2 hours. The reaction was quenched with water and the mixture was extracted with DCM, washed with saturated brine. dried over anhydrous Na₂SO₄. After filtration, the filtrate was concentrated under reduced pressure. The residue was purified by silica gel column chromatography, eluted with DCM/MeOH (10: 1) to afford the product (300 mg, 54%). [M+H]⁺ = 412.0.

### Step 4:

### (3-((5-bromo-2-((5-ethyl-2-methoxy-4-(4-(piperazin-1-yl)piperidin-1-yl)phenyl)amino)pyrimidin-4-yl)amino)-1, 5-naphthyridin-4-yl)dimethylphosphine oxide

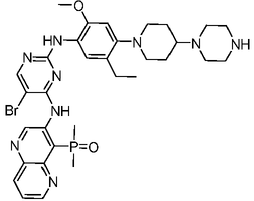

A mixture of (3-((5-bromo-2-chloropyrimidin-4-yl)amino)-1,5-naphthyridin-4-yl)dimethylphosphine oxide (2 g, 4.8 mmol), tert-butyl 4-(1-(4-amino-2-ethyl-5-methoxyphenyl)piperidin-4-yl)piperazine-1-carboxylate (2.6 g, 6.3 mmol) and MsOH (184 mg, 19.2 nm) in t-BuOH (20 mL) was stirred in a round bottom flask at 90 °C under nitrogen atmosphere for 16 h. The reaction mixture was concentrated under reduced pressure. The residue was purified by HPLC chromatography (C-18 column chromatography (0.1% FA in water : acetonitrile = 90 : 10 ~ 60 : 40 gradient elution) to afford the product (2 g, 60%). [M+H]+ = 694.0.

### Step 5:

### 3-(4-(2-(4-(1-(4-((5-bromo-4-((4-(dimethylphosphoryl)-1,5-naphthyridin-3-yl)amino)pyrimidin-2-yl)amino)-2-et hvl-5-methoxyphenyl)piperidin-4-yl)piperazin-1-yl)ethyl)-2,6-difluorophenyl)piperidine-2,6-dione

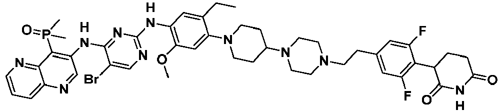

A mixture of (3-((5-bromo-2-((5-ethyl-2-methoxy-4-(4-(piperazin-1-yl)piperidin-1-yl)phenyl)amino)pyrimidin-4-yl)amino)-1, 5-naphthyridin-4-yl)dimethylphosphine oxide (40 mg, 0.057 mmol) and 2-(4-(2,6-dioxopiperidin-3-yl)-3,5-difluorophenyl)acetaldehyde (the material was synthesized from the similar method of example 2 step 6) (20 mg, 0.069 mmol) and NaOAc ( 14 mg, 0.17 mmol) in DCM (4 mL) and EtOH (0.5 mL) was stirred in a round bottom flask at room temperature for 2 hour. The mixture was added NaBH₃CN (10 mg, 0.17 mmol) and stirred in a round bottom flask at room temperature 2 h. Then the mixture was evaporated in vacuum to afford the crude product, which was purified with HPLC chromatography (C-18 column chromatography (0.1% FA in water : acetonitrile = 90 : 10 ~ 60 : 40 gradient elution)) to give the product (16 mg, 28%). ¹H NMR (500 MHz, DMSO) δ 12.25 (s, 1H), 10.95 (s, 1H), 9.83 (s, 1H), 8.93 (dd,*J* = 4.2, 1.6 Hz, 1H), 8.34 (d, *J =* 8.3 Hz, 1H), 8.29 (s, 1H), 8.21 (s, 1H), 7.71 (dd, *J =* 8.4, 4.2 Hz, 1H), 7.43 (s, 1H), 7.03 (d, *J =* 10.0 Hz, 2H), 6.75 (s, 1H), 4.20 (dd, *J =* 12.8, 5.2 Hz, 1H), 3.80 (d, *J =* 16.5 Hz, 3H), 3.00 (d, *J* = 11.5 Hz, 2H), 2.86 - 2.71 (m, 3H), 2.69 - 2.61 (m, 3H), 2.55-5.41 (m, 6H), 2.49 - 2.40 (m, 6H), 2.30 -2.20 (m,1H), 2.11-2.06 (m, 7H), 2.03 - 1.94 (m, 1H), 1.85 (d, *J =* 10.6 Hz, 2H), 1.61 - 1.49 (m, 2H), 0.87-0.68 (m, 3H); [M+H]⁺ = 945.5.

### Example 9:

### 3-(4-(4-(2-(4-(1-(4-((5-bromo-4-((4-(dimethylphosphoryl)-1,5-naphthyridin-3-yl)amino)pyrimidin-2-yl)amino)-2-ethyl-5-methoxyphenyl)piperidin-4-yl)piperazin-1-yl)ethyl)piperidin-1-yl)-2,6-difluorophenyl)piperidine-2,6-d ione

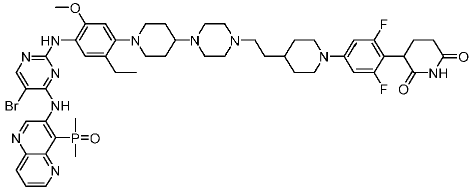

The titled compound was synthesized in the procedures similar to Example 1. ¹H NMR (500 MHz, DMSO) δ 12.26 (s, 1H), 10.86 (s, 1H), 9.84 (s, 1H), 8.93 (dd, *J =* 4.2*,* 1.5 Hz, 1H), 8.36 - 8.29 (m, 1H), 8.29 (s, 1H), 8.21 (s, 1H), 7.71 (dd, *J=* 8.4, 4.2 Hz, 1H), 7.43 (s, 1H), 6.74 (s, 1H), 6.60 (d, *J* = 12.8 Hz, 2H), 4.04 (dd, *J* = 12.6, 5.1 Hz, 1H), 3.81 - 3.68 (m, 5H), 3.00 (d, *J* = 11.0 Hz, 2H), 3.01-2.71 (m, 8H), 2.49 - 2.26 (m, 11H), 2.08 (d, *J* = 14.6 Hz, 7H), 2.01 - 1.91 (m, 1H), 1.84 (d, *J* = 10.7 Hz, 2H), 1.72 (d, *J* = 11.3 Hz, 2H), 1.60 - 1.33 (m, 5H), 1.25 - 1.12 (m, 2H), 0.88 (t, *J* = 5.9 Hz, 3H); [M+H]⁺ = 1028.5.

### Example 10:

### 3-(4-(2-(4-(1-(4-((5-bromo-4-((4-(dimethylphosphoryl)-8-methylquinolin-3-yl)amino)pyrimidin-2-yl)amino)-2-e thyl-5-methoxyphenyl)piperidin-4-yl)piperazin-1-yl)ethyl)-2,6-difluorophenyl)piperidine-2,6-dione

### Step 1: N-(8-methylquinolin-3-yl)-1,1-diphenylmethanimine

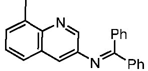

To a mixture of 3-bromo-8-methylquinoline (2 g, 9.05 mmol), diphenylmethanimine (2.46 g, 13.6 mmol), Pd₂(dba)₃ (834 mg, 0.91 mmol), BINAP (1.14 g, 1.82 mmol), Cs₂CO₃ (5.9 g, 18.1 mmol) in dioxane (60 mL) was stirred at 100 °C under nitrogen atmosphere for 18 hrs. After cooling to r.t, the reaction mixture was filtered, the filtrate was concentrated in vacuo. The residue was purified by silica gel column (PE: EA=4:1) to afford the product (2.6 g, 89.7%). [M+H]⁺ = 323.2.

### Step 2: 8-methylquinolin-3-amine

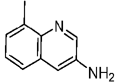

To a solution of N-(8-methylquinolin-3-yl)-1,1-diphenylmethanimine (2.6 g, 8.1 mmol) in dioxane (30 mL) was added HCl (6 mL, 2N). The mixture was stirred at 20 °C for 30 min and then sat. aq. Na₂CO₃ solution was added until PH=8-9, the resulting mixture was extracted with EtOAc (50 mL x 3). The combined organic phase was washed with brine (50 mL), dried over Na₂SO₄, filtered and concentrated in vacuum. The residue was purified by silica gel column (PE:EA=1:1) to afford 8-methylquinolin-3-amine (1.3 g, 82%). [M+H]⁺ = 159.1.

### Step 3: 4-iodo-8-methylquinolin-3-amine

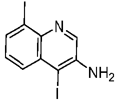

To a solution of 8-methylquinolin-3-amine (1.3 g, 8.23 mmol) in HOAc (10 mL) was added ICl (1.99 g, 12.3 mmol). The mixture was stirred at 20 °C for 3 hrs and then sat. aq. Na₂CO₃ solution was added to PH=8-9, the resulting mixture was extracted with EtOAc (70 mL x 3). The combined organic phase was washed with brine (60 mL), dried over Na₂SO₄, filtered and concentrated in vacuum to afford

4-iodo-8-methylquinolin-3-amine (1.8 g, 77%). [M+H]⁺ =285.2.

### Step 4: (3-amino-8-methylquinolin-4-yl)dimethylphosphine oxide

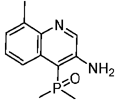

To a mixture of 4-iodo-8-methylquinolin-3-amine (1.8 g, 6.32 mmol), dimethylphosphine oxide (720 mg, 9.47 mmol), Pd(OAc)₂ (141 mg, 0.63 mmol), Xantphos (731 mg, 1.26 mmol), K₃PO₄ (2.68 g, 12.64 mmol) in dioxane (40 mL) was stirred at 100 °C under nitrogen atmosphere for 8 hrs. After cooling to r.t, the reaction mixture was filtered, the filtrate was concentrated in vacuo. The residue was purified by silica gel column (DCM:CH₃OH=15:1) to afford (3-amino-8-methylquinolin-4-yl)dimethylphosphine oxide (1.1 g, 74%). [M+H]⁺ = 235.2.

### Step 5: (3-((5-bromo-2-chloropyrimidin-4-yl)amino)-8-methylquinolin-4-yl)dimethylphosphine oxide

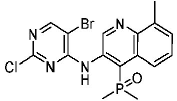

To a solution of (3-amino-8-methylquinolin-4-yl)dimethylphosphine oxide (1.1 g, 4.68 mmol) in DMF (25 mL) was added NaH (468 mg, 11.7 mmol, 60%) at 0 °C. The mixture was stirred at 0 °C for 20 min, then 5-bromo-2,4-dichloropyrimidine (2.12 g, 9.36 mmol) was added, the resulting solution was stirred at rt for 2 hrs. The reaction was quenched by sat. aq. NH₄Cl solution and the resulting mixture was extracted with EtOAc (40 mL x 3). The combined organic phase was washed with brine (30 mL), dried over Na₂SO₄, filtered and concentrated in vacuum. The residue was purified by silica gel column (DCM:CH₃OH=15:1) to afford the product (1.05 g, 52.8%). [M+H]⁺ =425.3.

### Step 6:

### (3-((5-bromo-2-((5-ethyl-2-methoxy-4-(4-(piperazin-1-yl)piperidin-1-yl)phenyl)amino)pyrimidin-4-yl)amino)-8-methylquinolin-4-yl)dimethylphosphine oxide

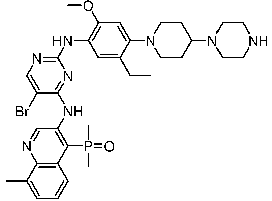

To a mixture of (3-((5-bromo-2-chloropyrimidin-4-yl)amino)-8-methylquinolin-4-yl)dimethylphosphine oxide (400 mg, 0.94 mmol), tert-butyl
4-(1-(4-amino-2-ethyl-5-methoxyphenyl)piperidin-4-yl)piperazine-1-carboxylate (392 mg, 0.94 mmol), TsOH·H₂O (536 mg, 2.82 mmol) in n-BuOH (8 mL) was stirred at 100 °C for 14 hrs. After cooling to r.t, the reaction mixture was concentrated in vacuo. The residue was diluted with DCM, washed with NaOH solution (20 mL, 1N) and brine, dried over Na₂SO₄ and then concentrated in vacuo. The residue was purified by silica gel column (DCM:CH₃OH=2:1) to afford the product (350 mg, 52.7%). [M+H]⁺ = 707.3.

### Step 7:

### 3-(4-(2-(4-(1-(4-((5-bromo-4-((4-(dimethylphosphoryl)-8-methylquinolin-3-yl)amino)pyrimidin-2-yl)amino)-2-e thyl-5-methoxyphenyl)piperidin-4-yl)piperazin-1-yl)ethyl)-2,6-difluorophenyl)piperidine-2,6-dione

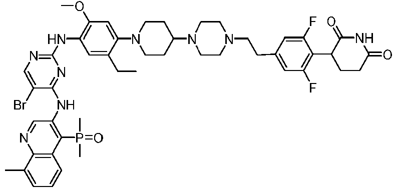

The titled compound (4 mg, 12%) was prepared in a manner similar to that in Example 2 step 7 from 2-(4-(2,6-dioxopiperidin-3-yl)-3,5-difluorophenyl)acetaldehyde and(3-((5-bromo-2-((5-ethyl-2-methoxy-4-(4-(piperazin-1-yl)piperidin-1-yl)phenyl)amino)pyrimidin-4-yl)amino )-8-methylquinolin-4-yl)dimethylphosphine oxide . ¹H NMR (500 MHz, DMSO) δ 11.76 (s, 1H), 10.95 (s, 1H), 9.51 (s, 1H), 8.25 (s, 1H), 8.07 - 7.87 (m, 2H), 7.58 (d, *J* = 7.0 Hz, 1H), 7.54 - 7.47 (m, 1H), 7.39 (s, 1H), 7.03 (d, *J =* 10.0 Hz, 2H), 6.62 (d, *J* = 84.3 Hz, 1H), 4.20 (dd, *J* = 12.7, 5.0 Hz, 1H), 3.80 (s, 3H), 2.92 (d, *J* = 11.0 Hz, 2H), 2.84 - 2.75 (m, 2H), 2.73 (s, 4H), 2.61 (dd, *J* = 20.2, 9.0 Hz, 3H), 2.53 (d, *J* = 5.8 Hz, 5H), 2.47 - 2.41 (m, 3H), 2.35 (dd, *J* = 10.1, 4.5 Hz, 3H), 2.27 (t, *J =* 11.1 Hz, 1H), 2.13 (dt, *J =* 13.1, 9.3 Hz, 1H), 2.05 (s, 3H), 2.04 - 1.93 (m, 5H), 1.82 (d, *J* = 11.1 Hz, 2H), 1.52 (dd, *J =* 20.2, 11.3 Hz, 2H), 0.75 (s, 3H). [M+H]⁺ = 958.4.

### Example 8:

### 3-(4-(4-(2-(4-(1-(4-((5-bromo-4-((4-(dimethylphosphoryl)-8-methylquinolin-3-yl)amino)pyrimidin-2-yl)amino)-2-ethyl-5-methoxyphenyl)piperidin-4-yl)piperazin-1-yl)ethyl)piperidin-1-yl)-2,6-difluorophenyl)piperidine-2,6-d ione

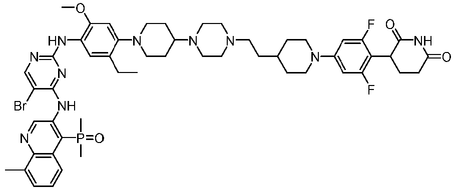

The titled compound was synthesized in the procedures similar to Example 10. ¹H NMR (500 MHz, DMSO) δ 11.76 (s, 1H), 10.86 (s, 1H), 9.51 (s, 1H), 8.24 (s, 1H), 8.08 - 7.87 (m, 2H), 7.57 (d, *J* = 7.0 Hz, 1H), 7.56 - 7.48 (m, 1H), 7.39 (s, 1H), 6.70 (s, 1H), 6.60 (d, *J* = 12.8 Hz, 2H), 4.04 (dd, *J* = 12.5, 5.1 Hz, 1H), 3.80 (s, 3H), 3.73 (d*, J =* 12.4 Hz, 2H), 2.91 (d, *J =* 10.9 Hz, 2H), 2.84 - 2.75 (m, 1H), 2.74 (d, *J =* 11.6 Hz, 4H), 2.69 (d, *J =* 11.4 Hz, 2H), 2.65 - 2.55 (m, 3H), 2.54 (s, 2H), 2.44 - 2.17 (m, 9H), 2.09 (dd, *J =* 12.6, 9.2 Hz, 1H), 2.04 (d, *J =* 13.5 Hz, 7H), 1.96 (dd, *J* = 14.3, 9.0 Hz, 1H), 1.81 (d, *J* = 10.8 Hz, 2H), 1.71 (d, *J* = 11.6 Hz, 2H), 1.51 (dd, *J =* 21.7, 12.3 Hz, 3H), 1.42 - 1.32 (m, 2H), 1.24 - 1.06 (m, 2H), 0.74 (s, 3H). [M+H]⁺ = 1041.4.

### Example 12:

### 3-(4-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-1H-indazol-1-yl)piperidine-2,6-dione

### Step 1: 3-(4-bromo-1H-indazol-1-yl)piperidine-2,6-dione

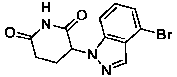

To a solution of 4-bromo-1H-indazole (1.96 g, 10 mmol) in THF (40 mL) was added NaH (60%, 0.48 g, 12 mmol) at 0 °C. Then the mixture was stirred at 60 °C for 1 h. To the above solution was added 3-bromopiperidine-2,6-dione (1.97 g, 10 mmol) at 60 °C. The resulting mixture was stirred overnight at 60 °C under nitrogen atmosphere. The mixture was cooled to room temperature, quenched with water (20 mL) and extracted with EtOAc (100 mL). The combined organic phase was washed with brine (40 mL), dried over Na₂SO₄, filtered and concentrated in vacuum. The residue was purified by column chromatography (DCM: MeOH=10:1) to afford the product (1.2 g, 39%) [M+H]⁺ = 308.2.

### Step 2: (E)-3-(4-(2-ethoxyvinyl)-1H-indazol-1-yl)piperidine-2,6-dione

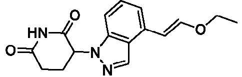

To a solution of 3-(4-bromo-1H-indazol-1-yl)piperidine-2,6-dione (0.616 g, 2 mmol) and (E)-2-(2-ethoxyvinyl)-4,4,5,5-tetramethyl-1,3,2-dioxaborolane (0.475 g, 2.4 mmol) in DMF (16 mL) and H₂O (4 mL) was added CsF (0.608 g, 4 mmol) and Pd(dtBPF)Cl₂ (0.163 g, 0.2 mmol) at room temperature. Then the mixture was stirred for 3 hrs at 80 °C under nitrogen atmosphere. The mixture was cooled to room temperature, diluted with water (20 mL) and extracted with EtOAc (100 mL). The combined organic phase was washed with brine (40 mL), dried over Na₂SO₄, filtered and concentrated in vacuum. The residue was purified by column chromatography (DCM: MeOH=10:1) to afford the product (366 mg, 61%). [M+H]⁺ = 300.3.

### Step 3: 2-(1-(2,6-dioxopiperidin-3-yl)-1H-indazol-4-yl)acetaldehyde

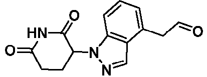

A solution of (E)-3-(4-(2-ethoxyvinyl)-1H-indazol-1-yl)piperidine-2,6-dione (60 mg, 0.2 mmol) in HCOOH (3 mL) was stirred for 3 hrs at room temperature. The mixture was concentrated in vacuum to afford the product (70 mg, crude) which was used in next step without further purification. [M+H]⁺ = 272.

### Step 4:

### 3-(4-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-1H-indazol-1-yl)piperidine-2,6-dione

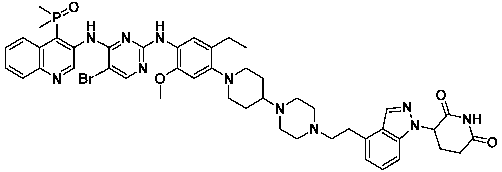

To a solution of 2-(1-(2,6-dioxopiperidin-3-yl)-1H-indazol-4-yl)acetaldehyde (70 mg crude, 0.2 mmol) in DCM (8 mL) and EtOH (4 mL) was added (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide (69.2 mg, 0.1 mmol) at room temperature. After 3 hrs, NaBH(OAc)₃ (42.4 mg, 0.2 mmol) was added to the mixture. The resulting mixture was stirred for 16 h at room temperature. The mixture was diluted with water (10 mL) and extracted with DCM (50 mL). The combined organic phase was washed with brine (20 mL), dried over Na₂SO₄, filtered and concentrated in vacuum. The residue was purified by prep. HPLC (C-18 column chromatography (0.1% FA in water : acetonitrile = 90 : 10 ~ 60 : 40 gradient elution) to afford the product (5.44 mg, 5.7%) was obtained. ¹H NMR (500 MHz, DMSO-*d₆*) δ ppm 11.68 (s, 1H), 11.10 (s, 1H), 9.45 (s, 1H), 8.27-8.14 (m, 3H), 8.04-7.96 (m, 2H), 7.72 (t, *J* =5 Hz, 1H), 7.64 (t, *J =*5 Hz, 1H), 7.48-7.41 (m, 2H), 7.32 (t*, J =*5 Hz, 1H), 7.01 (d, *J* =5 Hz, 1H), 6.70 (s, 1H), 5.83 (dd, *J =* 10, 5 Hz, 1H), 3.78 (s, 3H), 3.07 (t, *J =*5 Hz, 2H), 2.96-2.82 (m, 3H), 2.78-2.69 (m, 2H), 2.66-2.51 (m, 11H), 2.37-2.21 (m, 5H), 2.06 (s, 3H), 2.04 (s, 3H), 1.86-1.80 (m, 2H), 1.58-1.49 (m, 2H), 0.75 (t, *J =*10 Hz, 3H); [M+H]⁺ = 948.2.

### Example 14:

### 3-(4-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-2H-indazol-2-yl)piperidine-2,6-dione

### Step 1: 3-(4-bromo-2H-indazol-2-yl)piperidine-2,6-dione

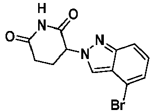

To a solution of 4-bromo-1H-indazole (1.96 g, 10 mmol) in THF (40 mL) was added NaH (60%, 0.48 g, 12 mmol) at 0 °C. Then the mixture was stirred at 60 °C for 1 h. To the above solution was added 3-bromopiperidine-2,6-dione (1.97 g, 10 mmol) at 60 °C.

The resulting mixture was stirred overnight at 60 °C under nitrogen atmosphere. The mixture was cooled to room temperature, quenched with water (20 mL) and extracted with EtOAc (100 mL). The combined organic phase was washed with brine (40 mL), dried over Na₂SO₄, filtered and concentrated in vacuum. The residue was purified by column chromatography (DCM: MeOH=10:1) to afford the product (0.98 g, 32%). [M+H]⁺ = 308.2.

### Step 2: (E)-3-(4-(2-ethoxyvinyl)-2H-indazol-2-yl)piperidine-2,6-dione

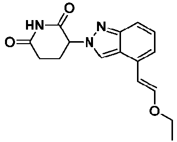

To a solution of 3-(4-bromo-2H-indazol-2-yl)piperidine-2,6-dione (0.616 g, 2 mmol) and (E)-2-(2-ethoxyvinyl)-4,4,5,5-tetramethyl-1,3,2-dioxaborolane (0.475 g, 2.4 mmol) in DMF (16 mL) and H₂O (4 mL) was added CsF (0.608 g, 4 mmol) and Pd(dtBPF)Cl₂ (0.163 g, 0.2 mmol) at room temperature. Then the mixture was stirred for 3 hrs at 80 °C under N₂. The mixture was cooled to room temperature, diluted with water (20 mL) and extraction with EtOAc (100 mL). The combined organic phase was washed with brine (40 mL), dried over Na₂SO₄, filtered and concentrated in vacuum. The residue was purified by column chromatography (DCM: MeOH=10:1) to afford the product (0.3 g, 50%). [M+H]⁺ = 300.2.

### Step 3: 2-(2-(2,6-dioxopiperidin-3-yl)-2H-indazol-4-yl)acetaldehyde

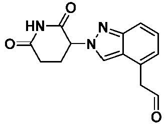

A solution of (E)-3-(4-(2-ethoxyvinyl)-2H-indazol-2-yl)piperidine-2,6-dione (60 mg, 0.2 mmol) in HCOOH (3 mL) was stirred for 3 hrs at room temperature. The mixture was concentrated in vacuum to afford the product (68 mg, crude) which was used in next step without further purification. [M+H]⁺ = 272.3.

### Step 4:

### 3-(4-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-2H-indazol-2-yl)piperidine-2,6-dione

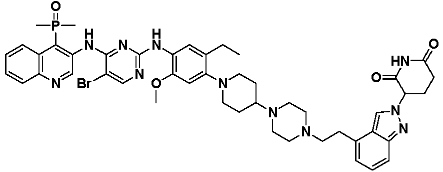

To a solution of 2-(2-(2,6-dioxopiperidin-3-yl)-2H-indazol-4-yl)acetaldehyde (68 mg, 0.2 mmol) in DCM (8 mL) and EtOH (4 mL) was added (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide (69.2 mg, 0.1 mmol) at room temperature. After 3 hrs, NaBH(OAc)₃ (42.4 mg, 0.2 mmol) was added to the mixture. The resulting mixture was stirred for 16 hrs at room temperature. The mixture was diluted with water (10 mL) and extracted with DCM (50 mL). The combined organic phase was washed with brine (20 mL), dried over Na₂SO₄, filtered and concentrated in vacuum. The residue was purified by prep. HPLC (C-18 column chromatography (0.1% FA in water : acetonitrile = 90 : 10 ~ 60 : 40 gradient elution)) to afford the product (8.26 mg, 8.7%). ¹H NMR (500 MHz, DMSO-*d₆*) δ ppm 11.68 (s, 1H), 11.18 (s, 1H), 9.45 (s, 1H), 8.57 (s, 1H), 8.25 (s, 1H), 8.17 (s, 1H), 8.04-7.95 (m, 2H), 7.72 (t*, J =*5 Hz, 1H), 7.64 (t, *J* =5 Hz, 1H), 7.44-7.38 (m, 2H), 7.18-7.14 (m, 1H), 6.88 (d, *J =*10 Hz, 1H), 6.70 (s, 1H), 5.68 (dd, *J* = 10, 5 Hz, 1H), 3.78 (s, 3H), 3.02-2.70 (m, 8H), 2.69-2.51 (m, 10H), 2.39-2.25 (m, 5H), 2.06 (s, 3H), 2.04 (s, 3H), 1.86-1.80 (m, 2H), 1.58-1.49 (m, 2H), 0.75 (t, *J* =10 Hz, 3H); [M+H]⁺ = 948.3.

### Example 15:

### 3-(5-(4-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazine-1-carbonyl)piperidin-1-yl)-3-methyl-2-oxo-2,3-dihydro-1H-benzo[d]imid azol-1-yl)piperidine-2,6-dione

### Step 1: 6-bromo-1-methyl-3H-1,3-benzodiazol-2-one

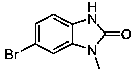

Into a 1000-mL round-bottom flask, were placed 5-bromo-N1-methylbenzene-1,2-diamine (10.0 g, 50.0 mmol), CH₃CN (200 mL), 1,1'-Carbonyldiimidazole (9.4 g, 58.0 mmol). The resulting solution was stirred for 5 h at 70 °C. The filtrate was concentrated after filtration. This resulted in 10.0 g (88.6%) of 6-bromo-1-methyl-3H-1,3-benzodiazol-2-one. ¹H NMR (500 MHz, DMSO-d6) δ ppm 10.98 (s, 1H), 7.31 (t*, J =* 1.5 Hz, 1H), 7.11 (ddd, *J=* 8.2 Hz, 1H), 6.91 (d, *J* = 8.2 Hz, 1H), 3.27 (s, 3H). [M+H]⁺ = 227.2.

### Step 2:

### 3-(5-bromo-3-methyl-2-oxo-2,3-dihydro-1H-benzo[d]imidazol-1-yl)-1-(4-methoxybenzyl)piperidine-2,6-dione

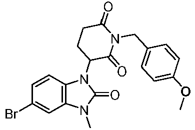

Into a 500-mL round-bottom flask purged and maintained with an inert atmosphere of nitrogen, were placed 6-bromo-1-methyl-3H-1,3-benzodiazol-2-one (10.0 g, 44.0 mmol), THF (100 mL), t-BuOK (4.9 g, 44.0 mmol), the resulting solution was stirred for 0.5 h at 0 °C and then was added 3-bromo-1-((4-methoxyphenyl)methyl)piperidine-2,6-dione (13.7 g, 44.0 mmol). The resulting solution was allowed to react, with stirring, for an additional 3 days at rt. The resulting solution was diluted with (200 mL) of EtOAc. The resulting mixture was washed with (3 x200 ml) of H₂O. The organic phase was dried over anhydrous sodium sulfate and concentrated. The residue was purified by combi-flash (EtOAc/PE=0-50%) to give the product (2.2 g, 11% yield). ¹H NMR (500 MHz, DMSO-d6) δ ppm 7.48 (d, *J* = 1.9 Hz, 1H), 7.27 - 7.14 (m, 3H), 7.02 (d, *J* = 8.4 Hz, 1H), 6.97 - 6.81 (m, 2H), 4.89 - 4.71 (m, 2H), 3.74 (s, 3H), 3.36 (s, 3H), 3.15 - 2.97 (m, 1H), 2.89 - 2.78 (m, 1H), 2.72 (td, J = 13.1 Hz, 1H), 2.13 - 1.97 (m, 1H). [M+H]⁺ = 458.1.

### Step 3: tert-butyl

### 1-(1-(1-(4-methoxybenzyl)-2,6-dioxopiperidin-3-yl)-3-methyl-2-oxo-2,3-dihydro-1H-benzo[d]imidazol-5-yl)pip eridine-4-carboxylate

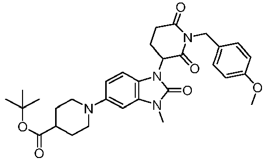

Into a 50-mL vial purged and maintained with an inert atmosphere of nitrogen, were placed 3-(5-bromo-3-methyl-2-oxo-2,3-dihydro-1H-benzo[d]imidazol-1-yl)-1-(4-methoxybenzyl)piperidine-2,6-dione (800.0 mg, 1.7 mmol), dioxane (20 mL), tert-butyl piperidine-4-carboxylate (485.1 mg, 2.6 mmol), Cs₂CO₃ (1.1 g, 3.5 mmol), Ruphos-G3 (225.2 mg, 0.3 mmol). The resulting solution was stirred for 15 h at 100 °C. The resulting solution was diluted with EtOAc (50 mL). The resulting mixture was washed with water (3 x 50 mL). The organic phase was dried over anhydrous sodium sulfate and concentrated. The residue was purified by combi-flash (EtOAc/PE=0-25%) to give the product (400 mg, 41% yield). [M+H]⁺ = 563.2.

### Step 4:

### 1-(1-(2,6-dioxopiperidin-3-yl)-3-methyl-2-oxo-2,3-dihydro-1H-benzo[d]imidazol-5-yl)piperidine-4-carboxylic acid

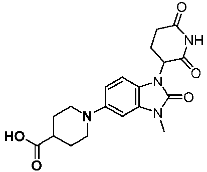

Into a 10-mL vial purged and maintained with an inert atmosphere of nitrogen, were placed tert-butyl 1-(1-(1-(4-methoxybenzyl)-2,6-dioxopiperidin-3-yl)-3-methyl-2-oxo-2,3-dihydro-1H-benzo[d]imidazol-5-yl)pip eridine-4-carboxylate (300.0 mg, 0.53 mmol), Toluene (2 mL), CH₃SO₃H (1 mL). The resulting solution was stirred for 2 h at 100 °C. The resulting solution was diluted with EtOAc (10 mL). The solids were collected by filtration. The solids were washed with EtOAc (3x20 mL). The solids were dried in vacuum. The product 1-(1-(2,6-dioxopiperidin-3-yl)-3-methyl-2-oxo-2,3-dihydro-1H-benzo[d]imidazol-5-yl)piperidine-4-carboxylic acid (190 mg, 92.22%) was obtained. [M+H]⁺ = 387.2.

### Step 5:

### 3-(5-(4-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazine-1-carbonyl)piperidin-1-yl)-3-methyl-2-oxo-2,3-dihydro-1H-benzo[d]imid azol-1-yl)piperidine-2,6-dione

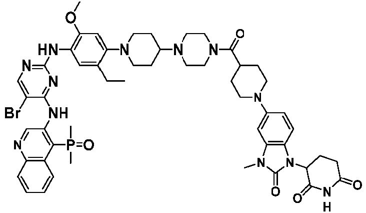

A mixture of (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide (29 mg, 0.075 mmol), HATU (24.2 mg, 0.063 mmol) and DIEA (22.4 mg, 0.17 mmol) in DMF (4 mL) was stirred at rt for 10 min. Then, 1-(1-(2,6-dioxopiperidin-3-yl)-3-methyl-2-oxo-2,3-dihydro-1H-benzo[d]imidazol-5-yl)piperidine-4-carboxylic acid (40 mg, 0.058 mmol) was added to the mixture above. The resulting mixture was stirred at rt for 16 h. The mixture was diluted with water (50 mL), extracted with EA (3x50 mL). The combined organic layers were dried over Na₂SO₄, filtered and concentrated under vacuum. The residue was purified by Prep-HPLC with the following conditions: Anal Column: SunFire Prep C18 OBD, 5 µm, 19x150 mm, Column Temp: Room temperature, Mobile Phase A: H₂O (0.1% FA), Mobile Phase B: ACN, Gradient Table: Mobile Phase B (15-28%), Time (min): 0-17 min to afford the product (11.33 mg, 18.5%). [M+H]⁺ = 1061.4. ¹H NMR (500 MHz, DMSO) δH 11.68 (s, 1H), 11.06 (s, 1H), 9.44 (s, 1H), 8.25 (s, 1H), 8.19 (d, *J=* 10.0 Hz, 1H), 8.02-7.97 (m, 2H), 7.75-7.70 (m, 1H), 7.67-7.62 (m, 1H), 7.41 (s, 1H), 6.94 (d, *J* = 5.0 Hz, 1H), 6.84 (s, 1H), 6.70 (s, 1H), 6.66-6.20 (m, 1H), 5.32-5.26 (m, 1H), 3.78 (s, 3H), 3.65-3.59 (m,2H), 3.57-3.51 (m, 2H), 3.50-3.44 (m, 2H), 2.97-2.86 (m, 3H), 2.78-2.58 (m, 10H), 2.57-2.53 (m, 3H), 2.39-2.30 (m, 4H), 2.06 (s, 3H), 2.04 (s, 3H), 2.01-1.96 (m, 1H), 1.86-1.80 (m, 2H), 1.75-1.69 (m, 4H), 1.60-1.51 (m, 2H), 0.80-0.71 (m, 3H).

### Example 16:

### 3-(5-(3-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazine-1-carbonyl)azetidin-1-yl)-1-oxoisoindolin-2-yl)piperidine-2,6-dione

### Step 1: 1-(3-cyano-4-(methoxycarbonyl)phenyl)azetidine-3-carboxylic acid

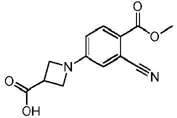

To a solution of methyl 2-cyano-4-fluorobenzoate (5.00 g, 27.9 mmol) and azetidine-3-carboxylic acid (4.99 g, 36.3 mmol) in DMSO (35.0 mL). The DIPEA (9.02 g, 69.8 mmol) was added to the reaction mixture. The reaction mixture was warmed to 100 °C for 0.5 hr and then stirred at 100 °C for another 3 hrs. The reaction mixture was diluted with ACN (35.0 mL) and stirred for 15 min, then filtered to get filtrate and adjusted the filtrate pH=8 by sat.aq.NaHCO₃ solution (40.0 mL). The mixture was extracted with EtOAc (50.0 mL). Water phase was adjusted pH=3 by 6N HCl (50.0 mL), extracted with EtOAc (50.0 mL x 2). The combine organic phase was washed with brine (50.0 mL), dried over anhydrous Na₂SO₄, filtered and concentrated in vacuum to obtain product (6.00 g, crude). [M-H] ⁻ = 259.1.

### Step 2: 1-(3-formyl-4-(methoxycarbonyl)phenyl)azetidine-3-carboxylic acid

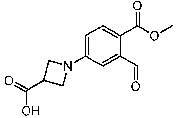

To a solution of Raney-Ni (2.00 g, 11.7 mmol) in HCOOH (20.0 mL) at 25 °C, 1-(3-cyano-4-(methoxycarbonyl)phenyl)azetidine-3-carboxylic acid in HCOOH (20.0 mL) was added to the reaction mixture at 25 °C. Then the mixture was stirred at 25 °C for 15 min. The reaction mixture was diluted with solvent H₂O (20.0 mL) and extracted with EtOAc (40.0 mL x 2). The combined organic phase was washed with brine (40.0 mL), dried over anhydrous Na₂SO₄, filtered and concentrated in vacuum to obtain product (5.50 g, crude). [M-H] ⁻ = 262.1.

### Step 3: 1-(2-(2,6-dioxopiperidin-3-yl)-1-oxoisoindolin-5-yl)azetidine-3-carboxylic acid

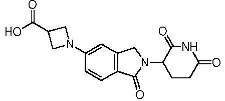

To a solution of 1-(3-formyl-4-(methoxycarbonyl)phenyl)azetidine-3-carboxylic acid (5.50 g, 20.9 mmol) and 3-aminopiperidine-2,6-dione hydrochloride (3.44 g, 20.9 mmol) in DMF (35.0 mL) was added DIPEA (8.10 g, 62.7 mmol) at 25 °C. The mixture was stirred at 25 °C for 1 hr. Then CH₃COOH (4.39 g, 73.1 mmol) and NaBH₃CN (2.63 g, 41.8 mmol) was added to the reaction mixture at 25 °C. The mixture was stirred at 25 °C for 1 hr. The mixture was adjust pH = 8 by DIPEA. The mixture was stirred at 25 °C for 1 hr. The mixture was concentrated under reduced pressure to give a residue and purified by prep-HPLC (Phase A: water (0.225%FA), Phase B: ACN; B%: 3%-35%, 20min) to obtain the product (1.70 g, 4.95 mmol, 23.7% yield). [M+H] + = 344.1.

### Step 4:

### 3-(5-(3-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazine-1-carbonyl)azetidin-1-yl)-1-oxoisoindolin-2-yl)piperidine-2,6-dione

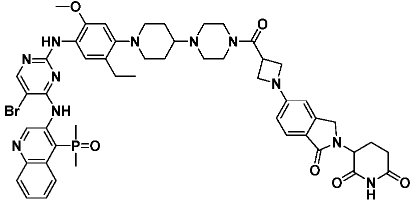

The titled compound (12 mg, 21%) was prepared in a manner similar to that in Example 15 step 5 from (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide and 1-(2-(2,6-dioxopiperidin-3-yl)-1-oxoisoindolin-5-yl)azetidine-3-carboxylic acid. 1H NMR (400 MHz, DMSO) δ 11.68 (s, 1H), 10.93 (s, 1H), 9.44 (s, 1H), 8.25 (s, 1H), 8.19 (d, J = 8.8 Hz, 1H), 8.02 - 7.97 (m, 2H), 7.72 (t, J = 7.4 Hz, 1H), 7.65 (d, J = 7.5 Hz, 1H), 7.50 (d, J = 8.4 Hz, 1H), 7.41 (s, 1H), 6.70 (s, 1H), 6.57 (s, 1H), 6.52 (d, J = 8.3 Hz, 1H), 5.04 (dd, J = 13.3, 5.0 Hz, 1H), 4.31 (d, J = 17.1 Hz, 1H), 4.19 (d, J = 17.1 Hz, 1H), 4.13 (t, J = 8.1 Hz, 2H), 4.00 (t, J = 6.3 Hz, 2H), 3.89-3.83 (m, 1H), 3.78 (s, 3H), 3.50 (s, 2H), 3.36-3.32 (m, 2H), 3.29 (s, 1H), 2.97-2.93 (m, 2H), 2.89-2.85 (m, 1H), 2.65 - 2.59 (m, 4H), 2.57-2.51 (m, 3H), 2.38-2.34 (m, 3H), 2.05 (d, J = 13.5 Hz, 6H), 1.98-1.93 (m, 1H), 1.82 (d, J = 10.4 Hz, 2H), 1.56 (d, J = 9.1 Hz, 2H), 0.75 (s, 3H); [M+H]⁺ = 1018.6.

### Example 17:

### 3-(5-(4-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazine-1-carbonyl)piperidin-1-yl)-3,3-dimethyl-2-oxoindolin-1-yl)piperidine-2,6 -dione

### Step 1: N-(2,6-bis(benzyloxy)pyridin-3-yl)-1,1-diphenylmethanimine

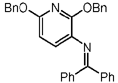

To a stirred solution of 2,6-bis(benzyloxy)-3-bromopyridine (30.0 g, 81.0 mmol) and diphenylmethanimine (17.6 g, 97.2 mmol) in dry 1,4-dioxane (400 mL), was added Cs₂CO₃ (52.8 g, 162 mmol) at room temperature. BINAP (5.0 g, 8.1 mmol) and Pd₂(dba)₃ (7.4 g, 8.1 mmol) were added to the mixture at room temperature. The suspension was degassed under vacuum and purged with N₂ three times. Then the mixture was stirred at 100 °C overnight. After cooling to room temperature, the mixture was filtered through a pad of celite, the filtrate was collected and concentrated to afford the residue which was purified by column chromatography (0-5% EA in PE). N-(2,6-bis(benzyloxy)pyridin-3-yl)-1,1-diphenylmethanimine (25.0 g, 65.6%) was obtained. [M+H]⁺ = 471.2.

### Step 2: 2,6-bis(benzyloxy)pyridin-3-aminium chloride

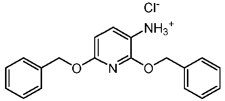

To a stirred solution of N-(2,6-bis(benzyloxy)pyridin-3-yl)-1,1-diphenylmethanimine (25.0 g, 53.1 mmol) in THF (200 mL), was added 1N HCl (200 mL) at room temperature. The suspension was stirred at room temperature for 16h, following by rotary evaporation in vacuum to remove the organic solvent. The solid precipitated out during evaporation was collected by filtration, then triturated with PE/EA (200 mL, 10/1) twice, and dried under vacuum to afford product (14.0 g, 76.9%). [M+H]⁺ = 307.2.

### Step 3: N-(2,6-bis(benzyloxy)pyridin-3-yl)-2-(2,5-dibromophenyl)-2-methylpropanamide

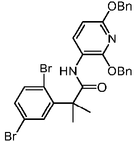

Into a stirred mixture of 2,6-bis(benzyloxy)pyridin-3-aminium chloride (14.0 g, 40.8 mmol) and 2-(2,5-dibromophenyl)-2-methylpropanoic acid (13.1 g, 40.8 mmol) in dry MeCN (200 mL) under N₂ atmosphere, was added 1-methyl-1H-imidazole (16.8 g, 204 mmol) via a syringe. The mixture was stirred at room temperature for 10 minutes, then a solution of N,N,N',N'-Tetramethylchloroformamidinium Hexafluorophosphate (13.8 g, 49.1 mmol) in dry MeCN (50 mL) was added into the mixture via a syringe. The mixture was stirred at 50 °C under N₂ atmosphere for 4 hrs. After cooling to room temperature, the reaction was quenched by addition of water (2 mL), then the mixture was concentrated in vacuum by an evaporator to afford the residue which was purified by column chromatography (5% EA in PE) to afford product (15.0 g, 60.2%). [M+H]⁺ = 611.2.

### Step 4: 1-(2,6-bis(benzyloxy)pyridin-3-yl)-5-bromo-3,3-dimethylindolin-2-one

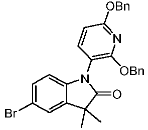

To a stirred mixture of N-(2,6-bis(benzyloxy)pyridin-3-yl)-2-(2,5-dibromophenyl)-2-methylpropanamide (10.0 g, 16.4 mmol) and K₂CO₃ (11.3 g, 81.9 mmol) in NMP (150 mL), was added CuCl (1.62 g, 16.4 mmol). The suspension was degassed under vacuum and purged with N₂ three times. Pentane-2,4-dione (3.28 g, 32.8 mmol) was added into the mixture via syringe. The suspension was stirred at 85 °C under N₂ atmosphere for 2 hrs. After cooling to room temperature, the mixture was poured into EA (500 mL), and washed with brine (200 mL). The organic layer was then dried over anhydrous Na₂SO₄ and concentrated in vacuum. The residue was purified by column chromatography (7% EA in PE) to afford product (6.0 g, 69.2%). [M+H]⁺ = 529.2.

### Step 5: tert-butyl 1-(1-(2,6-bis(benzyloxy)pyridin-3-yl)-3,3-dimethyl-2-oxoindolin-5-yl)piperidine-4-carboxylate

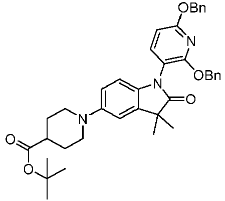

A mixture of 1-(2,6-bis(benzyloxy)pyridin-3-yl)-5-bromo-3,3-dimethylindolin-2-one (1.0 g, 1.889 mmol) and tert-butyl piperidine-4-carboxylate (419.9 mg, 2.267 mmol), Cs₂CO₃ (923.1g, 2.833 mmol), XPhos (180.1mg, 0.378 mmol), Pd₂(dba)₃ (173.0 mg, 0.189 mmol) in dioxane (20 mL) was stirred overnight at 110 °C under nitrogen atmosphere. The resulting mixture was filtered, the filter cake was washed with EtOAc (150 mL). The filtrate was concentrated under reduced pressure. The residue was purified by Prep-TLC (PE/EtOAc 1: 1) to afford product (620.0 mg, 51.7%) . [M+H]⁺ = 634.1.

### Step 6: tert-butyl 1-(1-(2,6-dioxopiperidin-3-yl)-3,3-dimethyl-2-oxoindolin-5-yl)piperidine-4-carboxylate

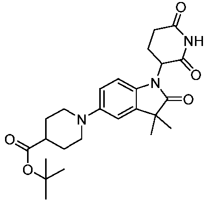

To a stirred mixture of tert-butyl 1-(1-(2,6-bis(benzyloxy)pyridin-3-yl)-3,3-dimethyl-2-oxoindolin-5-yl)piperidine-4-carboxylate (620.0mg, 0.978 mmol) and Pd/C (300.0 mg, 10% wt) in EtOH (10 mL) was added AcOH (50 mL, 872 mmol) dropwise and stirred overnight at 50 ° C under hydrogen atmosphere. The resulting mixture was filtered, the filter cake was washed with MeOH (150 mL). The filtrate was concentrated under reduced pressure to afford the product (300.0 mg, 67.3%). [M+H]⁺ = 456.2.

### Step 7: 1-(1-(2,6-dioxopiperidin-3-yl)-3,3-dimethyl-2-oxoindolin-5-yl)piperidine-4-carboxylic acid

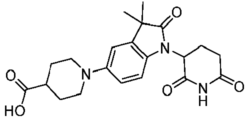

A mixture of tert-butyl 1-(1-(2,6-dioxopiperidin-3-yl)-3,3-dimethyl-2-oxoindolin-5-yl)piperidine-4-carboxylate (300.0 mg, 0.659 mmol) in TFA (1.5 mL) and DCM (3 mL) was stirred for 4 h at room temperature. The resulting mixture was concentrated under reduced pressure. The residue was purified by trituration with EtOAc (10 mL). 1-(1-(2,6-dioxopiperidin-3-yl)-3,3-dimethyl-2-oxoindolin-5-yl)piperidine-4-carboxylic acid (158.8 mg, 60.3%) was obtained. [M+H]⁺ =400.2.

### Step 8:

### 3-(5-(4-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazine-1-carbonyl)piperidin-1-yl)-3,3-dimethyl-2-oxoindolin-1-yl)piperidine-2,6 -dione

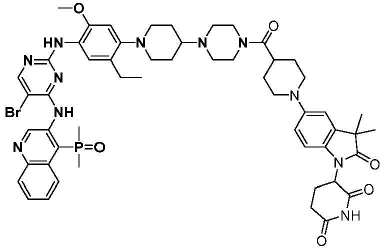

The titled compound (11 mg, 19%) was prepared in a manner similar to that in Example 15 step 5 from (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide and 1-(1-(2,6-dioxopiperidin-3-yl)-3,3-dimethyl-2-oxoindolin-5-yl)piperidine-4-carboxylic acid. 1H NMR (400 MHz, DMSO) δ 11.67 (s, 1H), 11.04 (s, 1H), 9.44 (s, 1H), 8.26-8.21 (m, 2H), 8.00 (d, J = 8.2 Hz, 2H), 7.71 (s, 1H), 7.63 (s, 1H), 7.42 (s, 1H), 7.10 (s, 1H), 6.77 (t, J = 9.4 Hz, 2H), 6.70 (s, 1H), 5.15 (s, 1H), 3.77 (s, 3H), 3.58 (d, J = 11.5 Hz, 2H), 3.53-3.46 (m, 4H), 2.94 (d, J = 10.2 Hz, 2H), 2.88-2.83 (m, 1H), 2.74-2.65 (m, 3H), 2.63-2.55 (m, 7H), 2.35 (d, J = 12.8 Hz, 3H), 2.04 (d, J = 13.4 Hz, 6H), 1.92 (s, 1H), 1.82 (d, J = 11.0 Hz, 2H), 1.73-1.68 (m, 4H), 1.55 (d, J = 9.8 Hz, 2H), 1.28 (d, J = 2.9 Hz, 7H), 0.74 (s, 3H); [M+H]⁺ = 1074.4.

### Example 18:

### 3-(4-(((1r,3r)-3-(4-(1-(4-((5-bromo-4-((4-(dimethylphosphoryl)quinolin-3-yl)amino)pyrimidin-2-yl)amino)-2-eth yl-5-methoxyphenyl)piperidin-4-yl)piperazine-1-carbonyl)cyclobutyl)(methyl)amino)-2,6-difluorophenyl)piperi dine-2,6-dione

### Step 1: methyl (1r,3r)-3-((tert-butoxycarbonyl)amino)cyclobutane-1-carboxylate

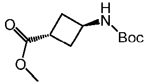

To a solution of methyl (1r,3r)-3-aminocyclobutane-1-carboxylate hydrochloride (1.5 g, 9.09 mmol) in dioxane (30 mL) and water (15 mL) was added Na₂CO₃ (2.89 g, 27.3 mmol) and Boc₂O (2.97 g, 13.6 mmol). The mixture was stirred at 20 °C for 13 hrs, the resulting mixture was extracted with EtOAc (70 mL x 3). The combined organic phase was washed with brine (60 mL), dried over Na₂SO₄, filtered and concentrated in vacuum. The residue was purified by silica gel column (PE:EA=1:1). Methyl (1r,3r)-3-((tert-butoxycarbonyl)amino)cyclobutane-1-carboxylate (1.7 g, 81.7%) was obtained. [M+H]⁺ =230.1.

### Step 2: methyl (1r,3r)-3-((tert-butoxycarbonyl)(methyl)amino)cyclobutane-1-carboxylate

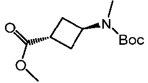

To a solution of methyl (1r,3r)-3-((tert-butoxycarbonyl)amino)cyclobutane-1-carboxylate (1.5 g, 6.52 mmol) in DMF (20 mL) was added NaH (522 mg, 13.04 mmol, 60%) at 0°C. The mixture was stirred at 20 °C for 1 hr, then MeI (1.39 g, 9.78 mmol) was added, the reaction was stirred at r.t for 3hrs and then quenched by sat. NH₄Cl solution (20 mL). The resulting mixture was extracted with EtOAc (40 mL x 3). The combined organic phase was washed with brine (30 mL), dried over Na₂SO₄, filtered and concentrated in vacuum. The residue was purified by silica gel column (PE:EA=1:1). Methyl (1r,3r)-3-((tert-butoxycarbonyl)(methyl)amino)cyclobutane-1-carboxylate (1.4 g, 88%) was obtained. [M+H]⁺ =244.2.

### Step 3: methyl (1r,3r)-3-(methylamino)cyclobutane-1-carboxylate hydrochloride

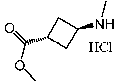

To a mixture of methyl (1r,3r)-3-((tert-butoxycarbonyl)(methyl)amino)cyclobutane-1-carboxylate (1.4 g, 5.76 mmol) and HCl in dioxane (20 mL, 6N) was stirred at 25 °C for 5 hr, then the resulting mixture was concentrated in vacuum. Methyl (1r,3r)-3-(methylamino)cyclobutane-1-carboxylate hydrochloride (700 mg, 84.4%) was obtained. [M+H]⁺ =144.2.

### Step 4: methyl

### (1r,3r)-3-((4-(2,6-bis(benzyloxy)pyridin-3-yl)-3,5-difluorophenyl)(methyl)amino)cyclobutane-1-carboxylate

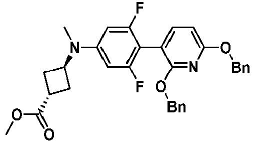

The titled compound (960 mg g, 52%) was prepared in a manner similar to that in Example 23 step 1 from methyl (1r,3r)-3-(methylamino)cyclobutane-1-carboxylate hydrochloride and 2,6-bis(benzyloxy)-3-(4-bromo-2,6-difluorophenyl)pyridine. [M+H]⁺ = 545.2.

### Step 5:

### (1r,3r)-3-((4-(2,6-bis(benzyloxy)pyridin-3-yl)-3,5-difluorophenyl)(methyl)amino)cyclobutane-1-carboxylic acid

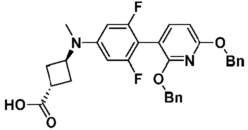

The titled compound (560 mg, 72%) was prepared in a manner similar to that in Example 23 step 2 from methyl (1r,3r)-3-((4-(2,6-bis(benzyloxy)pyridin-3-yl)-3,5-difluorophenyl)(methyl)amino)cyclobutane-1-carboxylate and LiOH. [M-H]⁻ = 529.2.

### Step 6: (1r,3r)-3-((4-(2,6-dioxopiperidin-3-yl)-3,5-difluorophenyl)(methyl)amino)cyclobutane-1-carboxylic acid

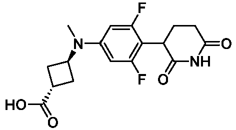

The titled compound (160 mg, 42%) was prepared in a manner similar to that in Example 23 step 3 from (1r,3r)-3-((4-(2,6-bis(benzyloxy)pyridin-3-yl)-3,5-difluorophenyl)(methyl)amino)cyclobutane-1-carboxylic acid and Pd/C. [M-H]⁻ = 351.1.

### Step 7:

### 3-(4-(((1r,3r)-3-(4-(1-(4-((5-bromo-4-((4-(dimethylphosphoryl)quinolin-3-yl)amino)pyrimidin-2-yl)amino)-2-eth yl-5-methoxyphenyl)piperidin-4-yl)piperazine-1-carbonyl)cyclobutyl)(methyl)amino)-2,6-difluorophenyl)piperi dine-2,6-dione

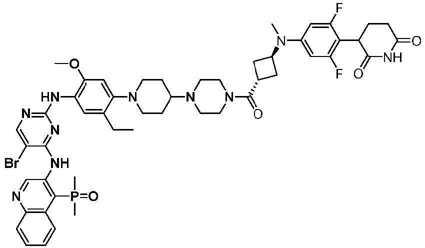

The titled compound (15 mg, 22%) was prepared in a manner similar to that in Example 15 step 5 from (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide and (1r,3r)-3-((4-(2,6-dioxopiperidin-3-yl)-3,5-difluorophenyl)(methyl)amino)cyclobutane-1-carboxylic acid. 1H NMR (400 MHz, DMSO) δ 11.68 (s, 1H), 10.86 (s, 1H), 9.44 (s, 1H), 8.25 (s, 1H), 8.19 (d, J = 9.0 Hz, 1H), 8.02 - 7.96 (m, 2H), 7.72 (t, J = 7.3 Hz, 1H), 7.64 (t, J = 7.5 Hz, 1H), 7.41 (s, 1H), 6.70 (s, 1H), 6.37 (d, J = 12.6 Hz, 2H), 4.10 - 4.02 (m, 2H), 3.77 (s, 3H), 3.50 (s, 2H), 3.31 - 3.28 (m, 2H), 3.23-3.20 (m, 1H), 2.93 (d, J = 10.8 Hz, 2H), 2.85 (s, 3H), 2.81-2.73 (m, 1H), 2.61 (t, J = 11.2 Hz, 2H), 2.51 (s, 2H), 2.49 - 2.46 (m, 4H), 2.39 - 2.31 (m, 5H), 2.08-2.05 (m, 8H), 1.97 - 1.92 (m, 1H), 1.81 (d, J = 11.0 Hz, 2H), 1.55 (d, J = 8.9 Hz, 2H), 0.75 (s, 3H); [M+H]⁺ = 1027.5.

### Example 19:

### 3-(4-(3-((4-(1-(4-((5-bromo-4-((4-(dimethylphosphoryl)quinolin-3-yl)amino)pyrimidin-2-yl)amino)-2-ethyl-5-m ethoxyphenyl)piperidin-4-yl)piperazin-1-yl)methyl)azetidin-1-yl)-2,6-difluorophenyl)piperidine-2,6-dione

### Step 1: methyl 1-(4-(2,6-bis(benzyloxy)pyridin-3-yl)-3,5-difluorophenyl)azetidine-3-carboxylate

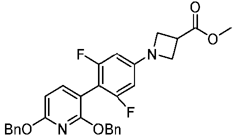

A mixture of 2,6-bis(benzyloxy)-3-(4-bromo-2,6-difluorophenyl)pyridine (3.00 g, 6.22 mmol), methyl azetidine-3-carboxylate hydrochloride (1.41 g, 9.33 mmol), Cs₂CO₃ (6.06 g, 18.66 mmol) and RuPhos Pd G3 (520.7 mg, 0.622 mmol) in toluene (50 mL) was stirred overnight at 100 °C under nitrogen atmosphere. The resulting mixture was extracted with EtOAc. The combined organic layers were washed with brine, dried over anhydrous Na₂SO₄. After filtration, the filtrate was concentrated under reduced pressure. The residue was purified by silica gel column chromatography, eluted with PE/EtOAc (2:1) to afford the product (1.7 g, 53 %). [M+1]⁺ = 517.1.

### Step 2: (1-(4-(2,6-bis(benzyloxy)pyridin-3-yl)-3,5-difluorophenyl)azetidin-3-yl)methanol

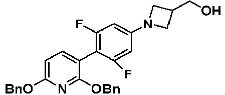

To a stirred mixture of methyl 1-(4-(2,6-bis(benzyloxy)pyridin-3-yl)-3,5-difluorophenyl)azetidine-3-carboxylate (1.7 g, 3.29 mmol) in THF (20 mL) was added LiAlH₄ (1 M in THF, 4.27 mL, 4.27 mmol) dropwise at 0 °C. Then the mixture was stirred for 2 hours, the reaction was quenched with water (10 mL) at 0 °C. The resulting mixture was extracted with EtOAc (3 x 50 mL). The combined organic layers were washed with brine, dried over anhydrous Na₂SO₄. After filtration, the filtrate was concentrated under reduced pressure to afford the product (1.4 g, 87%) [M+1] ⁺= 489.2.

### Step 3: 3-(2,6-difluoro-4-(3-(hydroxymethyl)azetidin-1-yl)phenyl)piperidine-2,6-dione

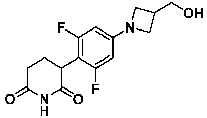

To a solution of (1-(4-(2,6-bis(benzyloxy)pyridin-3-yl)-3,5-difluorophenyl)azetidin-3-yl)methanol (1.40 g, 2.87 mmol) in iPrOH (20 mL) and DCM (20 mL) was added Pd/C (1.0 g, 10% wt) which was stirred at room temperature under hydrogen atmosphere for 48 hours. The resulting mixture was filtered, the filter cake was washed with MeOH (20 mL). The filtrate was concentrated under reduced pressure to afford the product (450 mg, 51%). [M+1]⁺=311.3.

### Step 4: (1-(4-(2,6-dioxopiperidin-3-yl)-3,5-difluorophenyl)azetidin-3-yl)methyl methanesulfonate

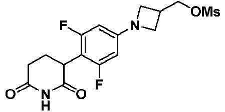

To a solution of 3-(2,6-difluoro-4-(3-(hydroxymethyl)azetidin-1-yl)phenyl)piperidine-2,6-dione (200 mg, 0.65 mmol) and Et₃N (262 mg, 2.6 mmol) in DCM (5 mL) and THF (5 mL), MsCl (150 mg, 1.30 mmol) was slowly added at 0 °C. The mixture was stirred at 25 °C for 2 hours. The mixture was quenched with water (10 mL). The organic phase was separated and concentrated in vacuum. The residue was purified by prep-TLC (DCM:MeOH=20: 1) to afford the product (180 mg, 72% yield). [M+H]⁺ = 389.1.

### Step 5:

### 3-(4-(3-((4-(1-(4-((5-bromo-4-((4-(dimethylphosphoryl)quinolin-3-yl)amino)pyrimidin-2-yl)amino)-2-ethyl-5-m ethoxyphenyl)piperidin-4-yl)piperazin-1-yl)methyl)azetidin-1-yl)-2,6-difluorophenyl)piperidine-2,6-dione

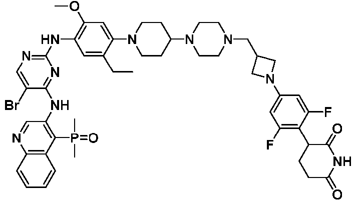

To a solution of (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide (50 mg, 0.072 mmol), (1-(4-(2,6-dioxopiperidin-3-yl)-3,5-difluorophenyl)azetidin-3-yl)methyl methanesulfonate (42 mg, 0.108 mmol), KI (36 mg, 0.216 mmol) and DIEA (37 mg, 0.288 mmol) in 5 mL ACN. The mixture was stirred at 80 °C for 16 hours. The mixture was concentrated in vacuum. The residue was dissolved in DCM and filtered. The organic phase was concentrated in vacuum and purified by prep-HPLC with C-18 column chromatography (0.1% FA in water : acetonitrile = 90 : 10 ~ 60 : 40 gradient elution) to afford the product (6.05 mg, 8.5% yield). 1H NMR (400 MHz, DMSO) δ 11.68 (s, 1H), 10.85 (s, 1H), 9.45 (s, 1H), 8.25 (s, 1H), 8.19 (d, J = 7.6 Hz, 1H), 8.02 - 7.97 (m, 2H), 7.71 (t, J = 7.7 Hz, 1H), 7.66-7.62 (m, 1H), 7.41 (s, 1H), 6.70 (s, 1H), 6.11 (d, J = 11.1 Hz, 2H), 4.02 (d, J = 7.8 Hz, 1H), 3.93 (t, J = 7.4 Hz, 2H), 3.77 (s, 3H), 3.49-3.45 (m, 2H), 3.29 (s, 2H), 2.99-2.87 (m, 3H), 2.79-2.73 (m, 1H), 2.65 - 2.57 (m, 3H), 2.54 (d, J = 6.8 Hz, 3H), 2.39-2.32 (m, 6H), 2.05 (d, J = 13.4 Hz, 8H), 1.97-1.93 (m, 2H), 1.83-1.80 (m, 2H), 1.55-1.50 (m, 2H), 0.75 (s, 3H); [M+H]⁺ = 985.5.

### Example 20:

### 3-(4-(3-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazine-1-carbonyl)azetidin-1-yl)-2,6-difluorophenyl)piperidine-2,6-dione

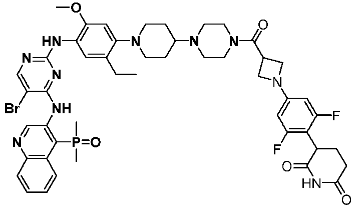

The titled compound was synthesized in the procedures similar to Example 18. 1H NMR (400 MHz, DMSO) δ 11.68 (s, 1H), 10.86 (s, 1H), 9.43 (s, 1H), 8.26-8.23 (m, 2H), 8.00 (d, J = 8.1 Hz, 2H), 7.71 (s, 1H), 7.63 (s, 1H), 7.43 (s, 1H), 6.70 (s, 1H), 6.17 (d, J = 11.2 Hz, 2H), 4.06 - 4.01 (m, 3H), 3.92 (t, J = 6.4 Hz, 2H), 3.85-3.81 (m, 1H), 3.77 (s, 3H), 3.49 (s, 2H), 2.93 (d, J = 10.2 Hz, 2H), 2.77 (dd, J = 21.2, 8.8 Hz, 1H), 2.62 (t, J = 11.1 Hz, 2H), 2.53-2.50 (m, 3H), 2.49 - 2.45 (m, 3H), 2.38-2.31 (m, 3H), 2.04 (d, J = 13.4 Hz, 8H), 1.95 (d, J = 5.6 Hz, 1H), 1.81 (d, J = 11.0 Hz, 2H), 1.58-1.52 (m, 2H), 0.74 (s, 3H); [M+H]⁺ = 999.5.

### Example 21:

### 3-(4-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-3-methyl-2-oxo-2,3-dihydro-1H-benzo[d]imidazol-1-yl)piperid ine-2,6-dione

### Step 1: 7-bromo-1-methyl-1,3-dihydro-2H-benzo[d]imidazol-2-one

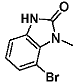

To a solution of 6-bromo-N1-methylbenzene-1,2-diamine (4 g, 19.9 mmol) in CH₃CN (50 mL) was added CDI (6.4 g, 39.8 mmol). The resulting solution was stirred for 6 h at 90 °C under nitrogen atmosphere. The solid was collected by filtration. This was resulted in 7-bromo-1-methyl-1,3-dihydro-2H-benzo[d]imidazol-2-one (4.1 g, 90.7%). [M+H]⁺ = 227.0.

### Step 2:

### 3-(4-bromo-3-methyl-2-oxo-2,3-dihydro-1H-benzo[d]imidazol-1-yl)-1-(4-methoxybenzyl)piperidine-2,6-dione

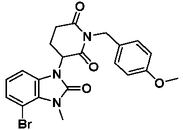

To a solution of 7-bromo-1-methyl-1,3-dihydro-2H-benzo[d]imidazol-2-one (600 mg, 2.6 mmol) in THF (10 mL) was added t-BuOK (1M in THF, 3.2 mL, 3.1 mmol) dropwise in 10 min at 0 °C, the reaction solution was stirred for 30 min at 0 °C , then to this was added 1-(4-methoxybenzyl)-2,6-dioxopiperidin-3-yl trifluoromethanesulfonate (This intermediate was prepared according the same manner described in WO 2020113233A1) (1.1 g, 2.9 mmol) in THF (5 mL) dropwise in 10 min. The resulting solution was stirred for 2 h at 0-10 °C. The reaction was quenched by the addition of sat.aq. NH₄Cl solution, extracted with EtOAc (10 mL x 3), combined the organic layer, and washed with brine, dried over anhydrous Na₂SO₄, after filtration, the filtrate was concentrated under reduced pressure. The residue was purified by a silica gel column, eluted with PE/EtOAc (1:1) to afford product (910 mg, 75.2%). [M+H]⁺ = 458.1.

### Step 3: 3-(4-bromo-3-methyl-2-oxo-2,3-dihydro-1H-benzo[d]imidazol-1-yl)piperidine-2,6-dione

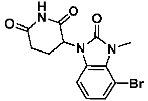

3-(4-bromo-3-methyl-2-oxo-2,3-dihydro-1H-benzo[d]imidazol-1-yl)-1-(4-methoxybenzyl)piperidine-2,6-di one (800 mg, 1.75 mmol) was dissolved in MeSO₃H/toluene(2 mL/ 6 mL). The resulting mixture was stirred for 3 h at 100 °C. Solvent was removed and the residue was poured into ice/water. The solid was collected by filtration. 3-(4-bromo-3-methyl-2-oxo-2,3-dihydro-1H-benzo[d]imidazol-1-yl)piperidine-2,6-dione was obtained (510 mg, 86.4%). [M+H]⁺ = 338.1.

### Step 4:

### (E)-3-(4-(2-ethoxyvinyl)-3-methyl-2-oxo-2,3-dihydro-1H-benzo[d]imidazol-1-yl)piperidine-2,6-dione

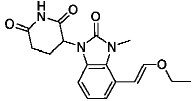

To a stirred solution of 3-(4-bromo-3-methyl-2-oxo-2,3-dihydro-1H-benzo[d]imidazol-1-yl)piperidine-2,6-dione (250 mg, 0.74 mmol) and (E)-2-(2-ethoxyvinyl)-4,4,5,5-tetramethyl-1,3,2-dioxaborolane (176 mg, 0.89 mmol) in DMF/H₂O (8 mL/ 2 mL) were added Pd(dtbpf)Cl₂ (48 mg, 0.074mmol) and CsF (225 mg, 1.48 mmol). The resulting mixture was stirred for 2 h at 80 °C under nitrogen atmosphere. The reaction solution was diluted with water, extracted with EtOAc (10 mL x 3). The organic layer was washed with water and brine, dried over anhydrous Na₂SO₄ which was evaporated to dryness. The residue was purified by a silica gel column, eluted with PE/EtOAc =1:1 to afford the product. (180 mg, 73.8%). [M+H]⁺ = 330.2.

### Step 5: 2-(1-(2,6-dioxopiperidin-3-yl)-3-methyl-2-oxo-2,3-dihydro-1H-benzo[d]imidazol-4-yl)acetaldehyde

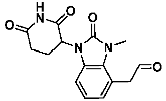

(E)-3-(4-(2-ethoxyvinyl)-3-methyl-2-oxo-2,3-dihydro-1H-benzo[d]imidazol-1-yl)piperidine-2,6-dione(180 mg, 0.55 mmol) was dissolved in HCOOH(2 mL). The resulting solution was stirred for 2 h at room temperature. The reaction solution was evaporated to dryness to afford product (125 mg, 75.3%) which was used directly in the next step. m/z [M+H]⁺ = 302.1.

### Step 6:

### 3-(4-(2-(4-(1-(4-((5-bromo-4-((4-(dimethylphosphoryl)quinolin-3-vl)amino)pyrimidin-2-yl)amino)-2-ethyl-5-me thoxyphenyl)piperidin-4-yl)piperazin-1-yl)ethyl)-3-methyl-2-oxo-2,3-dihydro-1H-benzo[d]imidazol-1-yl)piperid ine-2,6-dione

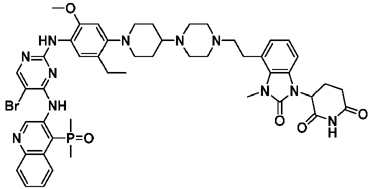

The titled compound (12 mg, 19%) was prepared in a manner similar to that in Example 1 step 11 from (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide and 2-(1-(2,6-dioxopiperidin-3-yl)-3-methyl-2-oxo-2,3-dihydro-1H-benzo[d]imidazol-4-yl)acetaldehyde. ¹H NMR (500 MHz, DMSO-*d*₆) δ_{H} 11.61 (s, 1H), 11.03 (s, 1H), 9.38 (s, 1H), 8.05-8.24 (m, 2H), 7.84-7.99 (m, 2H), 7.65 (t, J = 7.6 Hz, 1H), 7.57 (t, J = 7.6 Hz, 1H), 7.35 (s, 1H), 6.87-6.95 (m, 2H), 6.84 (dd, J = 6.2, 2.5 Hz, 1H), 6.64 (s, 1H), 5.30 (dd, J = 12.7, 5.3 Hz, 1H), 3.71 (s, 3H), 3.52 (s, 4H), 2.92-3.05 (m, 3H), 2.75-2.91 (m, 4H), 2.60-2.70 (m, 1H), 2.45-2.59 (m, 10H), 2.16-2.32 (m, 3H), 1.88-2.04 (m, 7H), 1.76 (d, J = 10.7 Hz, 2H), 1.46 (dd, J = 20.3, 11.1 Hz, 2H), 0.68 (s, 3H). [M+H]⁺ = 979.7

### Example 23:

### 3-(4-((6-(4-(1-(4-((5-bromo-4-((4-(dimethylphosphoryl)quinolin-3-yl)amino)pyrimidin-2-yl)amino)-2-ethyl-5-m ethoxyphenyl)piperidin-4-yl)piperazine-1-carbonyl)spiro[3.3]heptan-2-yl)amino)-2,6-difluorophenyl)piperidine-2,6-dione

### Step 1: Methyl

### 6-((4-(2,6-bis(benzyloxy)pyridin-3-yl)-3,5-difluorophenyl)amino)spiro[3.3]heptane-2-carboxylate

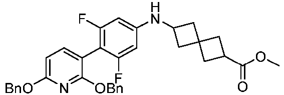

To a mixture of 2,6-bis(benzyloxy)-3-(4-bromo-2,6-difluorophenyl)pyridine (1 g, 2.08 mmol), HCl salt of methyl 6-aminospiro[3.3]heptane-2-carboxylate (640 mg, 3.12 mmol), Ruphos Pd G3 (176 mg, 0.21 mmol), Cs₂CO₃ (2.37 g, 7.28 mmol) in toluene (20 mL) was stirred at 100 °C under nitrogen atmosphere for 16 hrs. After cooling to rt, the reaction mixture was filtered, the filtrate was concentrated in vacuo. The residue was purified by silica gel column (PE: EA=1:1) to afford the product (530 mg, 45%). [M+H]⁺ = 571.2.

### Step 2: 6-((4-(2,6-bis(benzyloxy)pyridin-3-yl)-3,5-difluorophenyl)amino)spiro[3.3]heptane-2-carboxylic acid

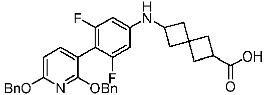

To a mixture of methyl 6-((4-(2,6-bis(benzyloxy)pyridin-3-yl)-3,5-difluorophenyl)amino)spiro[3.3]heptane-2-carboxylate (530 mg, 0.93 mmol) in THF (20 mL) and H₂O (10 mL) was added LiOH (111 mg, 4.64 mmol). The reaction solution was stirred at 25 °C for 4 hrs. HCl (2 N) was added to the mixture until PH=5-6. The resulting mixture was extracted with EA (25 mL x 3), washed with brine and dried over Na₂SO₄, concentrated in vacuo to afford the product (490 mg, 94.6%). [M+H]⁺ = 557.1.

### Step 3: 6-((4-(2,6-dioxopiperidin-3-yl)-3,5-difluorophenyl)amino)spiro[3.3]heptane-2-carboxylic acid

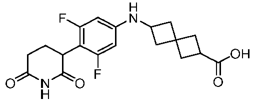

To a solution of 6-((4-(2,6-bis(benzyloxy)pyridin-3-yl)-3,5-difluorophenyl)amino)spiro[3.3]heptane-2-carboxylic acid (490 mg, 0.81 mmol) in MeOH (3 mL) and DCM (10 mL) was added Pd/C (200 mg, 10%). The reaction mixture was stirred at rt under H₂ balloon for 16 h. The catalyst was filtered off, the filtrate was concentrated in vacuum to afford the product (260 mg, 85%). [M+H]⁺ = 379.1.

### Step 4:

### 3-(4-((6-(4-(1-(4-((5-bromo-4-((4-(dimethylphosphoryl)quinolin-3-yl)amino)pyrimidin-2-yl)amino)-2-ethyl-5-m ethoxyphenyl)piperidin-4-yl)piperazine-1-carbonyl)spiro[3.3]heptan-2-yl)amino)-2,6-difluorophenyl)piperidine-2,6-dione

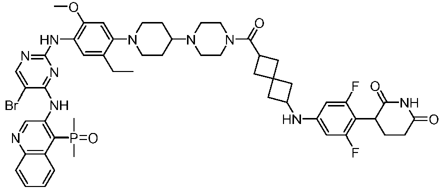

The titled compound (11 mg, 24%) was prepared in a manner similar to that in Example 15 Step 5 from (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide and 6-((4-(2,6-dioxopiperidin-3-yl)-3,5-difluorophenyl)amino)spiro[3.3]heptane-2-carboxylic acid. ¹H NMR (500 MHz, DMSO) δ 11.68 (s, 1H), 10.88 (d, *J* = 59.5 Hz, 1H), 9.44 (s, 1H), 8.25 (s, 1H), 8.23 - 8.14 (m, 1H), 8.04 - 7.89 (m, 2H), 7.72 (t, *J* = 7.5 Hz, 1H), 7.64 (t, *J =* 7.6 Hz, 1H), 7.41 (s, 1H), 6.66 (d, *J =* 37.1 Hz, 1H), 6.45 (d, *J* = 6.2 Hz, 1H), 6.12 (d, *J =* 11.8 Hz, 2H), 3.96 (dt, *J* = 24.5, 12.3 Hz, 1H), 3.77 (s, 3H), 3.71 - 3.58 (m, 1H), 3.43 (d, *J =* 4.7 Hz, 2H), 3.22 (dt, *J=* 17.1, 8.6 Hz, 2H), 2.93 (d, *J* = 10.5 Hz, 2H), 2.84 - 2.69 (m, 1H), 2.68 - 2.53 (m, 4H), 2.47 (s, 5H), 2.40 - 2.28 (m, 4H), 2.26 - 2.14 (m, 3H), 2.07 (d, *J* = 12.7 Hz, 4H), 2.03 (s, 4H), 1.96 - 1.91 (m, 1H), 1.89 - 1.68 (m, 4H), 1.53 (dd, *J=* 20.5, 11.6 Hz, 2H), 0.75 (s, 3H). [M+H]⁺ = 1053.4.

### Example 24:

### 3-(5'-(2-(4-(1-(4-((5-bromo-4-((4-(dimethylphosphoryl)quinolin-3-yl)amino)pyrimidin-2-yl)amino)-2-ethyl-5-m ethoxyphenyl)piperidin-4-yl)piperazin-1-yl)ethyl)-2'-oxospiro[cyclopropane-1,3'-indolin]-1'-yl)piperidine-2,6-di one

### Step 1: 1-(tert-butyl) 3-methyl 2-(5-bromo-2-nitrophenyl)malonate

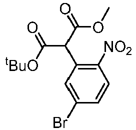

A mixture of 1-*tert*-butyl 3-methyl propanedioate (66.5 g, 381.823 mmol), Cs₂CO₃ (311 g, 954.558 mmol) and 4-bromo-2-fluoro-1-nitrobenzene (70 g, 318.186 mmol) in DMF (700 mL) was stirred for 16 h at 70 °C under nitrogen atmosphere. The resulting mixture was filtered, the filter cake was washed with EtOAc (3 x 2 L). The solution was acidified to pH= 6 with HCl (aq., 1 M) and diluted with water (5 L). The resulting solution was extracted with EtOAc (3 x 800 mL). The combined organic layers were washed with brine (500 mL), dried over anhydrous Na₂SO₄. After filtration, the filtrate was concentrated under reduced pressure. The residue was purified by silica gel column chromatography, eluted with EtOAc in PE (0%-2%) to afford the product (100 g, 84%). [M+H]⁺ =374.1.

### Step 2: methyl 2-(5-bromo-2-nitrophenyl)acetate

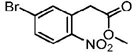

A solution of 1-(tert-butyl) 3-methyl 2-(5-bromo-2-nitrophenyl)malonate (80 g, 213.797 mmol) and *p*-toluenesulfonic acid.H₂O (20.33 g, 106.898 mmol) in toluene (800 mL) was stirred for 2 h at 110 °C .The resulting mixture was concentrated under reduced pressure. The resulting mixture was added water (200 mL) and extracted with EtOAc (3 x 1500 mL). The combined organic layers were washed with brine (2 x 500 mL), dried over anhydrous Na₂SO₄. After filtration, the filtrate was concentrated under reduced pressure. The residue was purified by silica gel column chromatography, eluted with EtOAc in PE (0%-2%) to afford the product (45.8 g, 69.5%). [M+H]⁺ =273.9.

### Step 3: methyl 2-(5-bromo-2-nitrophenyl)acrylate

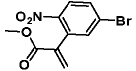

To a stirred solution of *N,N,N*-triethylanilinium iodide (13.36 g, 43.784 mmol) and methyl 2-(5-bromo-2-nitrophenyl)acetate (10 g, 36.487 mmol) in toluene (200 mL) were added paraformaldehyde (7.12 g, 237.166 mmol) and K₂CO₃ (10.09 g, 72.974 mmol) at room temperature. The resulting mixture was heated to 80 °C over 40 min. The resulting mixture was stirred for 1 h at 80 °C. The resulting mixture was concentrated under reduced pressure. The residue was purified by silica gel column chromatography, eluted with EtOAc in petroleum ether (0%-15%) to afford the product (8.7 g, 83.35%). [M+H]⁺ =287.1.0.

### Step 4: methyl 1-(5-bromo-2-nitrophenyl)cyclopropane-1-carboxylate

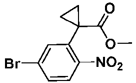

A mixture of *t*-BuOK (3.77 g, 33.557 mmol) and trimethylsulfoxonium iodide (7.38 g, 33.557 mmol) in THF (160 mL) was stirred for 0.5 h at 0 °C undernitrogen atmosphere. To the above mixture was added methyl 2-(5-bromo-2-nitrophenyl)acrylate (8 g, 27.964 mmol) in THF (20 mL) dropwise at 0 °C. The resulting mixture was stirred for 2 h at room temperature. The reaction was quenched with sat. NH₄Cl (aq.) (20 mL) at 0 °C and diluted with water (1L). The resulting mixture was extracted with EtOAc (3 x 200 mL). The combined organic layers were washed with brine (2 x 100 mL), dried over anhydrous Na₂SO₄. After filtration, the filtrate was concentrated under reduced pressure. The residue was purified by silica gel column chromatography, eluted with EtOAc in petroleum ether (0%-15%) to afford the product (6.6 g, 78.64%). [M+H]⁺ = 301.1

### Step 5: 5'-bromospiro[cyclopropane-1,3'-indolin]-2'-one

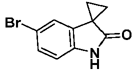

To a stirred solution of methyl 1-(5-bromo-2-nitrophenyl)cyclopropane-1-carboxylate (6.6 g, 21.992 mmol) in AcOH (66 mL) was added Fe (12.28 g, 219.921 mmol) in portions at room temperature under air atmosphere. The resulting mixture was stirred for 2 h at room temperature under air atmosphere. The resulting mixture was concentrated under reduced pressure. The residue was purified by silica gel column chromatography, eluted with EtOAc in petroleum ether (20%-90%) to afford the product (5 g, 95.49%). [M+H]⁺ = 237.9.

### Step 6: 3-(5'-bromo-2'-oxospiro[cyclopropane-1,3'-indolin]-1'-yl)-1-(4-methoxybenzyl)piperidine-2,6-dione

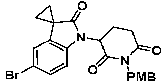

To a stirred mixture of 5'-bromospiro[cyclopropane-1,3'-indolin]-2'-one (5 g, 21.0 mmol) in THF (40 mL) was added *t*-BuOK (2.83 g, 25.201 mmol) in portions at 0 °C under nitrogen atmosphere. The resulting mixture was stirred for 1 h at room temperature under nitrogen atmosphere. To the above mixture was added 1-[(4-methoxyphenyl)methyl]-2,6-dioxopiperidin-3-yl trifluoromethanesulfonate (9.61 g, 25.201 mmol) in THF (20 mL) at 0 °C. The resulting mixture was stirred for 2 h at room temperature. The reaction was quenched with sat. NH₄Cl (aq.) (100 mL) at 0 °C. The resulting mixture was extracted with EtOAc (3 x 300 mL). The combined organic layers were washed with brine (2 x 200 mL), dried over anhydrous Na₂SO₄. After filtration, the filtrate was concentrated under reduced pressure. The residue was purified by silica gel column chromatography, eluted with EtOAc in petroleum ether (10%-50%) to afford the product (7 g,71.02%). [M+H]⁺ = 470.3.

### Step 7: 3-(5'-bromo-2'-oxospiro[cyclopropane-1,3'-indolin]-1'-yl)piperidine-2,6-dione

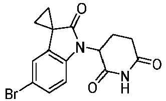

A solution of 3-(5'-bromo-2'-oxospiro[cyclopropane-1,3'-indolin]-1'-yl)-1-(4-methoxybenzyl)piperidine-2,6-dione (2 g, 4.261 mmol) and (NH₄)₂Ce(NO₃)₆ (9.38 g, 17.045 mmol) in MeCN (50 mL) and H₂O (10 mL) was stirred for 4 h at room temperature. The reaction was quenched by the addition of sat. NaHSO₃ (aq.) (10 mL) at room temperature. The resulting mixture was diluted with water (80 mL). The resulting mixture was extracted with EtOAc (3 x 100 mL). The combined organic layers were washed with brine (2 x 50 mL), dried over anhydrous Na₂SO₄. After filtration, the filtrate was concentrated under reduced pressure. The residue was purified by silica gel column chromatography, eluted with EtOAc in petroleum ether (10%-60%) to afford the product (950 mg, 63.84%). [M+H]⁺ = 349.0.

### Step 8:

### 3-(5'-bromo-2'-oxospiro[cyclopropane-1,3'-indolin]-1'-yl)-1-((2-(trimethylsilyl)ethoxy)methyl)piperidine-2,6-dio ne

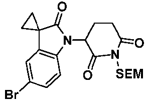

To a stirred mixture of 3-(5'-bromo-2'-oxospiro[cyclopropane-1,3'-indolin]-1'-yl)piperidine-2,6-dione (950 mg, 2.721 mmol) and DIEA (8.99 mL, 54.420 mmol) in DMF (45 mL) was added SEM-Cl (1.93 mL, 10.884 mmol) dropwise at 0 °C under nitrogen atmosphere. The resulting mixture was stirred for 3 h at room temperature. The reaction was quenched with water (300 mL) at room temperature. The resulting mixture was extracted with EtOAc (2 x 200 mL). The combined organic layers were washed with brine (3 x 80 mL), dried over anhydrous Na₂SO₄. After filtration, the filtrate was concentrated under reduced pressure. The residue was purified by silica gel column chromatography, eluted with EtOAc in petroleum ether (0-20%) to afford the product (848 mg, 65.01%). [M+H]⁺ = 479.1.

### Step 9:

### 3-(5'-allyl-2'-oxospirolcyclopropane-1,3'-indolin]-1'-yl)-1-((2-(trimethylsilyl)ethoxy)methyl)piperidine-2,6-dione

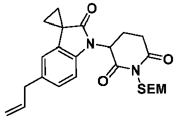

To a stirred solution of 3-(5'-bromo-2'-oxospiro[cyclopropane-1,3'-indolin]-1'-yl)-1-((2-(trimethylsilyl)ethoxy)methyl)piperidine-2,6-dio ne (850 mg, 1.773 mmol) and tributyl(prop-2-en-1-yl)stannane (0.88 g, 2.659 mmol) in DMF (16 mL) was added Pd(PPh₃)₂Cl₂ (0.19 mL, 0.266 mmol) at room temperature under nitrogen atmosphere. The resulting mixture was stirred for 2 h at 105 °C under nitrogen atmosphere. The resulting mixture was diluted with water (50 mL). The resulting mixture was extracted with EtOAc (3 x 100 mL). The combined organic layers were washed with brine (3 x 50 mL), dried over anhydrous Na₂SO₄. After filtration, the filtrate was concentrated under reduced pressure. The residue was purified by silica gel column chromatography, eluted with 1~40% EtOAc in PE to afford crude product (450 mg). The crude product was purified by reverse flash chromatography with the following conditions: column, C18; mobile phase, MeCN in water (0.1%FA), 50% to 90% gradient in 10 min; detector, UV 254 nm. to afford the product (350 mg, 44.81%). [M+Na]+ = 463.4.

### Step 10:

### 2-(1'-(2,6-dioxo-1-((2-(trimethylsilyl)ethoxy)methyl)piperidin-3-yl)-2'-oxospiro[cyclopropane-1,3'-indolin]-5'-yl )acetaldehyde

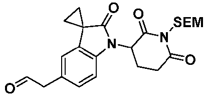

A mixture of 3-(5'-allyl-2'-oxospiro[cyclopropane-1,3'-indolin]-1'-yl)-1-((2-(trimethylsilyl)ethoxy)methyl)piperidine-2,6-dione (220 mg, 0.5 mmol), K₂OsO₄ (1.84 mg, 0.005 mmol), NaIO₄ (428 mg, 2 mmol) in dioxane (8 mL) and H₂O (2 mL) was stirred at rt for 1 h. The mixture was diluted with water (50 mL), extracted with DCM (3x50 mL). The combined organic layers were dried over Na₂SO₄, filtered and concentrated under vacuum. The residue was purified by silica gel column chromatography, eluted with EA/PE (2: 1) to afford product (120 mg, 54.3%). [M+H]⁺ = 443.2.

### Step 11:

### 3-(5'-(2-(4-(1-(4-((5-bromo-4-((4-(dimethylphosphoryl)quinolin-3-yl)amino)pyrimidin-2-yl)amino)-2-ethyl-5-m ethoxyphenyl)piperidin-4-yl)piperazin-1-yl)ethyl)-2'-oxospiro[cyclopropane-1,3'-indolin]-1'-yl)-1-((2-(trimethyl silyl)ethoxy)methyl)piperidine-2,6-dione

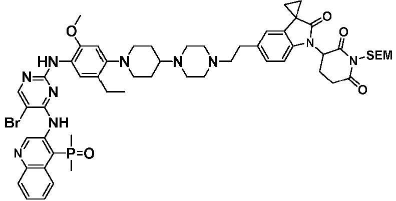

A mixture of (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide (62 mg, 0.09 mmol), 2-(1'-(2,6-dioxo-1-((2-(trimethylsilyl)ethoxy)methyl)piperidin-3-yl)-2'-oxospiro[cyclopropane-1,3'-indolin]-5'-yl )acetaldehyde (60 mg, 0.13 mmol) and AcOH (0.2 mL) in MeOH (5 mL) and DCM (5 mL) was stirred at rt for 16 h. Then, STAB (38.2 mg, 0.18 mmol) was added to the mixture above. The mixture was stirred at rt for 5 h. The mixture was diluted with water (50 mL), extracted with DCM (3x50 mL). The combined organic layers were dried over Na₂SO₄, filtered and concentrated under vacuum. The residue was purified by silica gel column chromatography, eluted with MeOH/DCM (1:9) to afford product (36 mg, 35.8%). [M+H]⁺ = 1119.4.

### Step 12:

### 3-(5'-(2-(4-(1-(4-((5-bromo-4-((4-(dimethylphosphoryl)quinolin-3-yl)amino)pyrimidin-2-yl)amino)-2-ethyl-5-m ethoxyphenyl)piperidin-4-yl)piperazin-1-yl)ethyl)-2'-oxospiro[cyclopropane-1,3'-indolin]-1'-yl)piperidine-2,6-di one

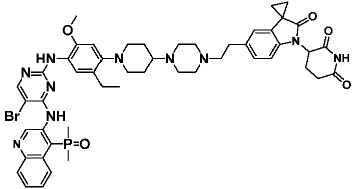

A mixture of 3-(5'-(2-(4-(1-(4-((5-bromo-4-((4-(dimethylphosphoryl)quinolin-3-yl)amino)pyrimidin-2-yl)amino)-2-ethyl-5-m ethoxyphenyl)piperidin-4-yl)piperazin-1-yl)ethyl)-2'-oxospiro[cyclopropane-1,3'-indolin]-1'-yl)-1-((2-(trimethyl silyl)ethoxy)methyl)piperidine-2,6-dione (35 mg, 0.03 mmol) in DCM (1 mL) was added TFA (5 mL) which was stirred at rt for 16 h. The mixture was concentrated under vacuum. The residue was dissolved in THF (5 mL) and added NH₃.H₂O (0.5 mL). The mixture was stirred at rt for 5 min. The mixture was diluted with water (50 mL), extracted with DCM (3x50 mL). The combined organic layers were dried over Na₂SO₄, filtered and concentrated under vacuum. The residue was purified by Prep-HPLC with the following conditions: Anal Column: SunFire Prep C18 OBD, 5 µm, 19x150 mm, Column Temp: Room temperature, Mobile Phase A: H₂O (0.1% FA), Mobile Phase B: ACN, Gradient Table: Mobile Phase B (15-35%), Time (min): 0-17 min to afford the product (6.56 mg, 20.6%). [M+H]⁺ = 989.4. ¹H NMR (500 MHz, DMSO) δH 11.68 (s, 1H), 11.07 (s, 1H), 9.44 (s, 1H), 8.25 (s, 1H), 8.21-8.14 (m, 1H), 8.02-7.96 (m, 2H), 7.75-7.69 (m, 1H), 7.67-7.62 (m, 1H), 7.43-7.39 (m, 1H), 7.07-7.04 (m, 1H), 6.93-6.88 (m, 2H), 6.71-6.69 (m, 1H), 5.36-5.27 (m, 1H), 3.77 (s, 3H), 2.97-2.83 (m, 4H), 2.75-2.54 (m, 14H), 2.39-2.25 (m, 4H), 2.06 (s, 3H), 2.04 (s, 3H), 1.99-1.92 (m, 1H), 1.87-1.78 (m, 2H), 1.66-1.60 (m, 2H), 1.57-1.49 (m, 4H), 0.81-0.69 (m, 3H).

### Example 25:

### 3-(5-(3-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazine-1-carbonyl)azetidin-1-yl)-1H-indazol-1-yl)piperidine-2,6-dione

### Step 1: Tert-butyl

### 1-(1-(1-(4-methoxybenzyl)-2 6-dioxopiperidin-3-yl)-1H-indazol-5-yl)azetidine-3-carboxylate

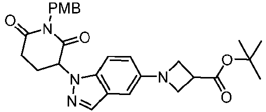

To a mixture of 3-(5-bromo-1H-indazol-1-yl)-1-(4-methoxybenzyl)piperidine-2,6-dione (1 g, 2.34 mmol), HCl salt of tert-butyl azetidine-3-carboxylate (677 mg, 3.51 mmol), Ruphos Pd G3 (198 mg, 0.23 mmol), Cs₂CO₃ (3.05 g, 9.36 mmol) in toluene (25 mL) was stirred at 100 °C for 15 hrs. After cooling to r.t, the solid was filtered off, the filtrate was concentrated in vacuo. The residue was purified by silica gel column (PE:EA=1:1) to afford the product (580 mg, 49%). [M+H]⁺ = 505.2.

### Step 2: 1-(1-(2,6-dioxopiperidin-3-yl)-1H-indazol-5-yl)azetidine-3-carboxylic acid

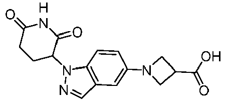

To a solution of tert-butyl 1-(1-(1-(4-methoxybenzyl)-2,6-dioxopiperidin-3-yl)-1H-indazol-5-yl)azetidine-3-carboxylate (580 mg, 1.15 mmol) in toluene (6 mL) and MsOH (2 mL) was stirred at 80 °C for 16 hrs. After cooling to r.t, the reaction mixture was concentrated in vacuum. The residue was purified by reversed phase column (CH₃CN:H₂O(0.1% FA)=20:80) to afford the product (140 mg, 37%). [M-H]⁻ = 327.1.

### Step 3:

### 3-(5-(3-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazine-1-carbonyl)azetidin-1-yl)-1H-indazol-1-yl)piperidine-2,6-dione

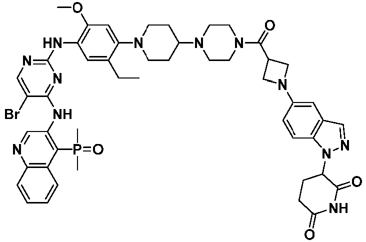

The titled compound (14 mg, 32%) was prepared in a manner similar to that in Example 15 step 5 from (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide and 1-(1-(2,6-dioxopiperidin-3-yl)-1H-indazol-5-yl)azetidine-3-carboxylic acid. ¹H NMR (500 MHz, DMSO) δ 11.61 (s, 1H), 10.99 (s, 1H), 9.38 (s, 1H), 8.19 (s, 1H), 8.11 (t, *J* = 11.0 Hz, 1H), 7.98 - 7.87 (m, 2H), 7.83 (s, 1H), 7.66 (t, *J =* 7.8 Hz, 1H), 7.58 (t, *J=* 7.2 Hz, 1H), 7.42 (d, *J* = 9.0 Hz, 1H), 7.35 (s, 1H), 6.69 (dd, *J =* 9.0*,* 2.1 Hz, 1H), 6.64 (s, 1H), 6.60 (d, *J* = 1.8 Hz, 1H), 5.69-5.66 (m, 1H), 3.97 (t, *J*= 7.3 Hz, 2H), 3.85-3.80 (m, 2H), 3.78 - 3.74 (m, 1H), 3.71 (s, 3H), 3.42 (s, 2H), 3.28 (s, 3H), 2.87 (d, *J* = 10.7 Hz, 2H), 2.82-2.75 (m, 1H), 2.69 - 2.60 (m, 2H), 2.58-2.54 (m, 2H), 2.47 (s, 2H), 2.34 - 2.21 (m, 4H), 2.20 - 2.09 (m, 1H), 1.96 *(t, J=* 19.2 Hz, 6H), 1.75 (d, *J=* 11.0 Hz, 2H), 1.55 - 1.39 (m, 2H), 0.69 (s, 3H). [M+H]⁺ = 1003.3.

### Example 26:

### 3-(5-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-1H-indazol-1-yl)piperidine-2,6-dione

### Step 1: 3-(5-bromo-1H-indazol-1-yl)-1-(4-methoxybenzyl)piperidine-2,6-dione and 3-(5-bromo-2H-indazol-2-yl)-1-(4-methoxybenzyl)piperidine-2,6-dione

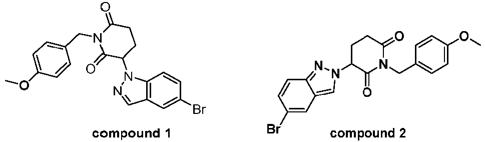

To a solution of 5-bromo-1H-indazole (2.0 g, 10.2 mmol) and 1-(4-methoxybenzyl)-2,6-dioxopiperidin-3-yl trifluoromethanesulfonate (This intermediate was prepared according to the same manner described in WO2020113233A1) (5.8 g, 15.3 mmol) in THF(50 mL) was added t-BuOK (15 mL , 15.3 mmol) at 0 °C. Then the mixture was stirred at 30 °C for 2 hrs. The reaction was quenched with water and the mixture was extracted with DCM (3 x 300 mL). The combined organic layers were washed with brine (300 mL), dried over anhydrous Na₂SO₄. After filtration, the filtrate was concentrated under reduced pressure. The residue was purified by silica gel column chromatography, eluted with DCM/MeOH(15: 1) to afford the title compound 1 (1.2 g, 28%).[M+H]⁺ = 428.2 and title compound 2 (1.3 g, 30%) [M+H]⁺ = 428.1

### Step 2: 3-(5-bromo-1H-indazol-1-yl)piperidine-2,6-dione

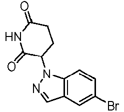

A mixture of 3-(5-bromo-1H-indazol-1-yl)-1-(4-methoxybenzyl)piperidine-2,6-dione (1.2 g, 2.8 mmol) in toluene (10 mL) and MsOH (5 mL) was stirred in a round bottom flask at 100 °C under nitrogen atmosphere for 3 hours. The reaction mixture was concentrated under reduced pressure. The residue was purified by purified by silica gel column chromatography, eluted with DCM/MeOH (15: 1) to afford the product (480 mg, 56%), [M+H]+ = 308.2.

### Step 3: (E)-3-(5-(2-ethoxyvinyl)-1H-indazol-1-yl)piperidine-2,6-dione

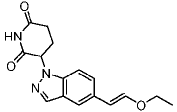

A mixture of 3-(5-bromo-1H-indazol-1-yl)piperidine-2,6-dione (70 mg, 0.23 mmol), (E)-2-(2-ethoxyvinyl)-4,4,5,5-tetramethyl-1,3,2-dioxaborolane (91 mg, 0.46 mmol), Pd(dppf)Cl₂ ( 15 mg, 0.023 mmol) and CsF (100 mg, 0.69 mmol) in DMF (5 mL) was stirred in a round bottom flask at 90 °C under nitrogen atmosphere for 2 hrs. The reaction mixture was allowed to cool down to room temperature. The resulting mixture was extracted with DCM (3 x 100 mL). The combined organic layers were washed with brine (100 mL), dried over anhydrous Na₂SO₄. After filtration, the filtrate was concentrated under reduced pressure. The residue was purified by silica gel column chromatography, eluted with DCM/MeOH (15: 1) to afford the product (50 mg, 72%), [M+H]+ = 300.0.

### Step 4: 2-(1-(2,6-dioxopiperidin-3-yl)-1H-indazol-5-yl)acetaldehyde

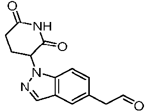

A mixture of (E)-3-(5-(2-ethoxvvinyl)-1H-indazol-1-yl)piperidine-2,6-dione (51 mg, 0.17 mmol) in HCOOH (2 mL) was stirred in a round bottom flask at 25 °C under nitrogen atmosphere for 30 min. The reaction mixture was concentrated under reduced pressure to afford crude product which was directly used for the next step. [M+H]+ = 272.2.

### Step 5:

### 3-(5-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-1H-indazol-1-yl)piperidine-2,6-dione

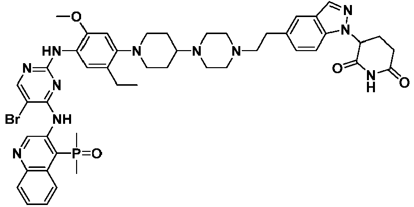

The titled compound (7 mg, 12%) was prepared in a manner similar to that in Example 1 step 11 from (3-((5-bromo-2-((5-ethyl-2-methoxy-4-(4-(piperazin-1-yl)piperidin-1-yl)phenyl)amino)pyrimidin-4-yl)amino)qu inolin-4-yl)dimethylphosphine oxide and 2-(1-(2,6-dioxopiperidin-3-yl)-1H-indazol-5-yl)acetaldehyde. ¹H NMR (500 MHz, DMSO) δ 11.68 (s, 1H), 11.09 (s, 1H), 9.44 (s, 1H), 8.29 - 8.13 (m, 2H), 8.00 (dd, *J* = 21.3, 13.0 Hz, 3H), 7.72 (t, *J =* 7.4 Hz, 1H), 7.64 (t, *J* = 7.7 Hz, 1H), 7.59 (s, 1H), 7.53 (d, *J* = 8.7 Hz, 1H), 7.42 (s, 1H), 7.30 (d, *J =* 8.7 Hz, 1H), 6.70 (s, 1H), 5.81 (dd, *J* = 11.4, 4.1 Hz, 1H), 3.77 (s, 3H), 2.98 - 2.66 (m, 8H), 2.65 - 2.50 (m, 10H), 2.37 - 2.19 (m, 5H), 2.05 (d, *J* = 13.4 Hz, 6H), 1.82 (d, *J =* 10.7 Hz, 2H), 1.59 - 1.44 (m, 2H), 0.75 (s, 3H); [M+H]⁺ = 948.3.

### Example 30:

### 3-(4'-(2-(4-(1-(4-((5-bromo-4-((4-(dimethylphosphoryl)quinolin-3-yl)amino)pyrimidin-2-yl)amino)-2-ethyl-5-m ethoxyphenyl)piperidin-4-yl)piperazin-1-yl)ethyl)-2'-oxospiro[cyclopropane-1,3'-indolin]-1'-yl)piperidine-2,6-di one

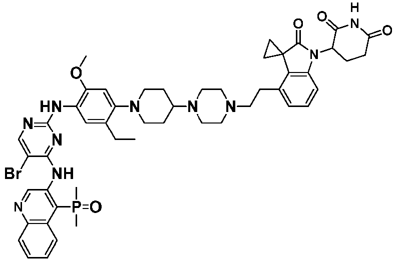

The titled compound was synthesized in the procedures similar to Example 24. [M+H]⁺ = 989.4. ¹H NMR (500 MHz, DMSO) δH 11.68 (s, 1H), 11.08 (s, 1H), 9.45 (s, 1H), 8.26 (s, 1H), 8.19 (d, *J=*10.0 Hz, 1H), 8.03-7.97 (m, 2H), 7.72 (t, *J*=10.0 Hz, 1H), 7.64 (t, *J=*10.0 Hz, 1H), 7.42 (s, 1H), 7.19-7.13 (m, 1H), 6.91-6.85 (m, 2H), 6.71 (s, 1H), 5.37-5.27 (m, 1H), 3.78 (s, 3H), 2.98-2.90 (m, 2H), 2.89-2.82 (m, 1H), 2.66-2.60 (m, 7H), 2.57-2.54 (m, 6H), 2.39-2.30 (m, 6H), 2.07 (s, 3H), 2.04 (s, 3H), 1.99-1.93 (m, 3H), 1.89-1.79 (m, 2H), 1.58-1.44 (m, 4H), 0.80-0.71 (m, 3H).

### Example 32:

### 3-(4-(((1r,3r)-3-((4-(1-(4-((5-bromo-4-((4-(dimethylphosphoryl)quinolin-3-yl)amino)pyrimidin-2-yl)amino)-2-et hyl-5-methoxyphenyl)piperidin-4-yl)piperazin-1-yl)methyl)cyclobutyl)(methyl)amino)-2,6-difluorophenyl)piper idine-2,6-dione

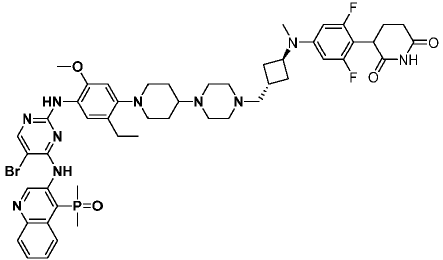

The titled compound was synthesized in the procedures similar to Example 1. 1H NMR (400 MHz, DMSO) δ 11.68 (s, 1H), 10.85 (s, 1H), 9.44 (s, 1H), 8.25 (s, 1H), 8.20 - 8.17 (m, 1H), 8.02 - 7.97 (m, 2H), 7.72 (t, J = 7.7 Hz, 1H), 7.64 (t, J = 7.5 Hz, 1H), 7.41 (s, 1H), 6.70 (s, 1H), 6.42 (s, 1H), 6.40 (s, 1H), 4.28 - 4.22 (m, 1H), 4.03 (dd, J = 12.5, 5.1 Hz, 1H), 3.77 (s, 3H), 2.92 (d, J = 10.9 Hz, 2H), 2.83 (s, 3H), 2.76 (dd, J = 17.0, 5.3 Hz, 1H), 2.61 (t, J = 11.3 Hz, 3H), 2.58-2.51 (m, 4H), 2.48 (d, J = 7.8 Hz, 3H), 2.40 (s, 2H), 2.35-2.31 (m, 3H), 2.27 (s, 1H), 2.23 - 2.18 (m, 2H), 2.09-2.04 (m, 9H), 2.02-2.00 (m, 1H), 1.97 - 1.93 (m, 1H), 1.82 (d, J = 10.5 Hz, 2H), 1.55 - 1.48 (m, 2H), 0.75 (s, 3H); [M+H]⁺ = 1013.4.

### Example 33:

### 3-(4-(2-(4-(1-(4-((5-bromo-4-((4-(dimethylphosphoryl)quinolin-3-yl)amino)pyrimidin-2-yl)amino)-3-methoxyph enyl)piperidin-4-yl)piperazin-1-yl)ethyl)-2,6-difluorophenyl)piperidine-2,6-dione

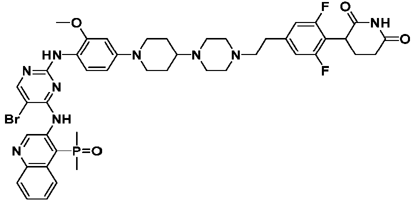

The titled compound was synthesized in the procedures similar to Example 1. 1H NMR (400 MHz, DMSO) δ 11.74 (s, 1H), 10.95 (s, 1H), 9.50 (s, 1H), 8.20 (s, 1H), 8.15 (d, J = 8.4 Hz, 1H), 8.03 (d, J = 8.2 Hz, 1H), 8.00 (s, 1H), 7.71 (t, J = 7.6 Hz, 1H), 7.63 (t, J = 7.6 Hz, 1H), 7.30 (d, J = 7.6 Hz, 1H), 7.02 (d, J = 10.1 Hz, 2H), 6.55 (d, J = 1.9 Hz, 1H), 6.22 (s, 1H), 4.20 (dd, J = 12.7, 5.1 Hz, 1H), 3.77 (s, 3H), 3.62 (d, J = 11.7 Hz, 2H), 2.86 - 2.79 (m, 1H), 2.76 (dd, J = 15.4, 8.1 Hz, 3H), 2.60 (t, J = 11.4 Hz, 3H), 2.56-2.51 (m, 3H), 2.46 - 2.36 (m, 5H), 2.31-2.26 (m, 1H), 2.12 (dd, J = 13.4, 4.1 Hz, 1H), 2.05 (d, J = 13.5 Hz, 7H), 2.02-1.97 (m, 1H), 1.82 (d, J = 11.2 Hz, 2H), 1.51 - 1.44 (m, 2H); [M+H]⁺ = 916.4.

### Example 34:

### 3-(4-(2-(4-(1-(4-((5-bromo-4-((4-(dimethylphosphoryl)quinolin-3-yl)amino)pyrimidin-2-yl)amino)-5-methoxy-2 -methylphenyl)piperidin-4-yl)piperazin-1-yl)ethyl)-2,6-difluorophenyl)piperidine-2,6-dione

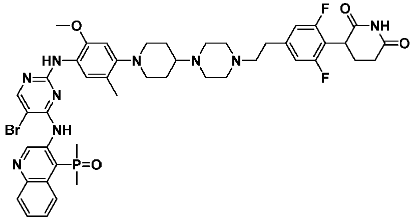

The titled compound was synthesized in the procedures similar to Example 1. 1H NMR (400 MHz, DMSO) δ 11.75 (s, 1H), 10.95 (s, 1H), 9.49 (d, J = 3.7 Hz, 1H), 8.24 (s, 1H), 8.18 (d, J = 8.3 Hz, 1H), 8.01 (d, J = 8.3 Hz, 1H), 7.96 (s, 1H), 7.72 (t, J = 7.6 Hz, 1H), 7.64 (t, J = 7.8 Hz, 1H), 7.37 (s, 1H), 7.04 (s, 1H), 7.02 (s, 1H), 6.64 (s, 1H), 4.20 (dd, J = 12.6, 5.1 Hz, 1H), 3.77 (s, 3H), 3.01 (d, J = 11.3 Hz, 2H), 2.86-2.81 (m, 1H), 2.76 (dd, J = 14.6, 6.9 Hz, 3H), 2.59 - 2.53 (m, 8H), 2.48 - 2.43 (m, 3H), 2.31-2.27 (m, 1H), 2.13 (dd, J = 13.0, 3.9 Hz, 1H), 2.05 (d, J = 13.5 Hz, 7H), 2.02-1.97 (m, 1H), 1.94 (s, 3H), 1.83 (d, J = 11.3 Hz, 2H), 1.57-1.52 (m, 2H); [M+H]⁺ = 930.4.

### Example 35:

### 3-(4-(2-(4-(1-(4-((5-bromo-4-((4-(dimethylphosphoryl)quinolin-3-yl)amino)pyrimidin-2-yl)amino)-5-ethoxy-2-e thylphenyl)piperidin-4-yl)piperazin-1-yl)ethyl)-2,6-difluorophenyl)piperidine-2,6-dione

### Step 1: 4-ethyl-3-fluorophenol

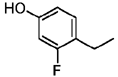

To a solution of 1-(2-fluoro-4-hydroxyphenyl)ethan-1-one (5.0 g, 32 mmol) in H₂O (17 mL) was added Zn powder (6.5g, 100 mmol) and HCl (conc., 17 mL). The reaction mixture was stirred at 115 °C for 18 hours. After cooling to room temperature, the mixture was extracted with MTBE (2 x 30 mL). The organic phase was combined and washed with brine (2 x 60 mL). The organic phase was concentrated to afford the crude product, which was used in next step without further purification.

### Step 2: 4-ethoxy-1-ethyl-2-fluorobenzene

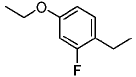

To a solution of 4-ethyl-3-fluorophenol (1.4 g, 10 mmol) in DMF (50 mL) was added K₂CO₃ (2.1g, 15 mmol), EtI (2.0g, 13 mmol). The reaction mixture was stirred at 20-30 °C for 18 hours. After quenching the reaction with H₂O (50 mL), the reaction mixture was extracted with PE/EA(1:1, 30 mL). The organic phase was combined and washed with brine (2 x 50 mL). Organic phase was concentrated and purified by silica gel column chromatography with PE (100%) to give product (1.2g, 71.4%).¹H NMR (500 MHz, CDCl3) δ 7.06 (t, *J =* 9.0 Hz, 1H), 6.62-6.55 (m, 2H), 3.98 (q, *J =* 7.0 Hz, 2H), 2.59 (q, *J* = 7.5 Hz, 2H), 1.39 (t, *J =* 7.0 Hz, 3H), 1.19 (t, J = 7.5 Hz, 3H).

### Step 3: 1-ethoxy-4-ethyl-5-fluoro-2-nitrobenzene

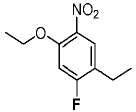

To a solution of 4-ethoxy-1-ethyl-2-fluorobenzene (1.4 g, 8.3 mmol) in Ac₂O (15 mL) was added HNO₃ (conc., 4 mL) dropwise. After stirring for 30min at 0°C, the reaction was quenching with H₂O (30 mL) and Na₂CO₃ solution (20 mL). The mixture was extracted with EA (2 x 30 mL). Organic phase was combined and washed with brine (2 x 50 ml), which was concentrated to afford the crude residue. The crude residue was purified by pre-flash with pure PE to give the product (1.2g, 67.8%). [M+H]⁺ = 214.2.

### Step 4: tert-butyl 4-(1-(5-ethoxy-2-ethyl-4-nitrophenyl)piperidin-4-yl)piperazine-1-carboxylate

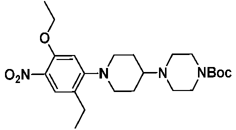

A solution of 1-ethoxy-4-ethyl-5-fluoro-2-nitrobenzene (0.7 g, 3.3 mmol), tert-butyl 4-(piperidin-4-yl)piperazine-1-carboxylate (0.97 g, 3.6 mmol) and K₂CO₃ (0.9 g, 6.6 mmol) in DMF was stirred at 120 °C for 18hs. The reaction was quenched with H₂O (50 mL) and extracted with EA (2 x 50 mL). The organic was combined and washed with brine (2 x 100 mL). After concentration, the crude product (0.9g) was purified by silica gel column chromatography ( DCM/MeOH= 100:1 to 10: 1) to afford the product (915 mg, 60%).¹H NMR (500 MHz, CDCl₃) δ7.81 (s, 1H), 6.58 (s, 1H), 4.17-4.11 (m, 3H), 3.48 (br,4H), 3.26 (d, *J=* 12 Hz, 2H), 2.69 (t, *J* = 12 Hz, 2H), 2.62-2.57 (m, 6H), 2.43 (br, 1H), 1.94 (br, 2H), 1.73 (br, 2H), 1.50 - 1.43 (m, 12H), 1.32 - 1.15 (t, *J* = 7.5 Hz, 3H).

### Step 5: tert-butyl 4-(1-(4-amino-5-ethoxy-2-ethylphenyl)piperidin-4-yl)piperazine-1-carboxylate

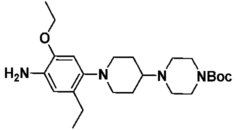

A solution of tert-butyl 4-(1-(5-ethoxy-2-ethyl-4-nitrophenyl)piperidin-4-yl)piperazine-1-carboxylate (0.9 g, 1.9 mmol) and Pd/C (0.09g) in THF (30 mL) was stirred at room temperature for 18 hours under H₂ atmosphere. After filtering off solid, the filtrate was concentrated to afford the crud product (0.50 g, 61%) for next step without further purification. [M+H]⁺ = 433.5.

### Step 6:

### (3-((5-bromo-2-((2-ethoxy-5-ethyl-4-(4-(piperazin-1-yl)piperidin-1-yl)phenyl)amino)pyrimidin-4-yl)amino)quin olin-4-yl)dimethylphosphine oxide

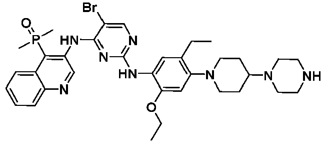

A solution of tert-butyl 4-(1-(4-amino-5-ethoxy-2-ethylphenyl)piperidin-4-yl)piperazine-1-carboxylate (120 mg, 0.3 mmol), TsOH (268 mg, 1.6 mmol) and (3-((5-bromo-2-chloropyrimidin-4-yl)amino)quinolin-4-yl)dimethylphosphine oxide (169 mg, 0.39 mmol) in n-BuOH was stirred at 105 °C for 18 hours. The solvent was evaporated to afford the residue and was washed with sat. aq. Na₂CO₃ solution (30 mL) and the mixture was extracted with DCM (2 x 20 mL). Organic phase was concentrated and purified by pre-TLC with DCM/MeOH (5:1) to afford the product (115 mg, 54%). [M+H]⁺ = 707.4.

### Step 7:

### 3-(4-(2-(4-(1-(4-((5-bromo-4-((4-(dimethylphosphoryl)quinolin-3-yl)amino)pyrimidin-2-yl)amino)-5-ethoxy-2-e thylphenyl)piperidin-4-yl)piperazin-1-yl)ethyl)-2,6-difluorophenyl)piperidine-2,6-dione

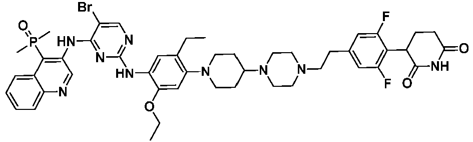

To a solution of (3-((5-bromo-2-((2-ethoxy-5-ethyl-4-(4-(piperazin-1-yl)piperidin-1-yl)phenyl)amino)pyrimidin-4-yl)amino)quin olin-4-yl)dimethylphosphine oxide (40 mg, 0.06 mmol), 2-(4-(2,6-dioxopiperidin-3-yl)-3,5-difluorophenyl)acetaldehyde (15 mg, 0.06 mmol) in DCM/EtOH (5:1, 30 mL) was added NaOAc (50 mg, 0.6 mmol). After stirring for 30min, NaBH(OAc)₃ (100 mg, 0.5 mmol) was added. The mixture was stirred for another 18hs. The solvent was evaporated to afford the crude residue which was purified by pre-HPLC (0.1% FA in water : acetonitrile = 90 : 10 ~ 60 : 40 gradient elution) to afford the product (6.8 mg, 12%). ¹H NMR (500 MHz, d-DMSO) δ 11.64 (s, 1H), 10.95 (s, 1H), 9.43 (d, *J =* 4.0 Hz, 1H), 8.27 (s, 1H), 8.22 (d, *J =* 8.5 Hz, 1H), 8.01 (d, *J =* 8.5 Hz, 1H), 7.90 (s, 1H), 7.73 (t, J = 7.5 Hz, 1H), 7.65 (t, *J =* 7.5 Hz, 1H), 7.45 (s, 1H), 7.04 (d, *J =* 10.0 Hz, 2H), 6.68 (s, 1H), 4.20 (dd, J = 12.5, 5.5 Hz, 1H), 4.03 (q, *J =* 7.0 Hz, 2H), 3.04 - 2.53 (m, 17H), 2.28 (d, *J* = 7.0 Hz, 3H), 2.18 - 1.95 (m, 9H), 1.89 (br, 2H), 1.56 (br, 2H), 1.29 (t, *J =* 7.0 Hz, 3H), 0.68 (s, 3H). [M+H]⁺ = 958.5.

### Example 36:

### 3-(4-(4-(2-(4-(1-(4-((5-bromo-4-((4-(dimethylphosphoryl)quinolin-3-yl)amino)pyrimidin-2-yl)amino)-5-ethoxy-2-ethylphenyl)piperidin-4-yl)piperazin-1-yl)ethyl)piperidin-1-yl)-2,6-difluorophenyl)piperidine-2,6-dione

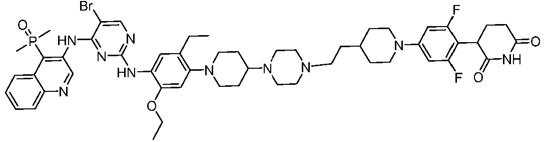

The titled compound was synthesized in the procedures similar to Example 35. ¹H NMR (500 MHz, d-DMSO) δ 11.64 (s, 1H), 10.86 (s, 1H), 9.43 (s, 1H), 8.29 - 8.15 (m, 2H), 8.00 (d, *J =* 7.5 Hz, 1H), 7.89 (s, 1H), 7.72 (s, 1H), 7.64 (s, 1H), 7.44 (s, 1H), 6.69 - 6.44 (m, 3H), 4.12 - 3.91 (m, 4H), 3.73 (d, *J =* 12.5 Hz, 2H), 2.98 - 2.53 (m, 10H), 2.40 - 2.15 (m, 8H), 2.15 - 1.86 (m, 10H), 1.85 - 1.64 (m, 4H), 1.56 - 1.07 (m, 10H), 0.67 (s, 3H). [M+H]⁺ = 1041.7

### Example 37:

### 3-(4-(2-(4-(1-(4-((5-bromo-2-((4-(dimethylphosphoryl)-1,8-naphthyridin-3-yl)amino)pyrimidin-4-yl)amino)-2-et hyl-5-methoxyphenyl)piperidin-4-yl)piperazin-1-yl)ethyl)-2,6-difluorophenyl)piperidine-2,6-dione

### Step 1: 4-iodo-1,8-naphthyridin-3-amine

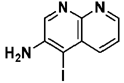

The titled compound (440 mg, 46%) was prepared in a manner similar to that in Example 1 step 1 from 1,8-naphthyridin-3-amine and iodine chloride. [M+H]+ = 272.0.

### Step 2: (3-amino-1,8-naphthyridin-4-yl)dimethylphosphine oxide

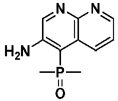

The titled compound (340 mg, 46%) was prepared in a manner similar to that in Example 1 step 2 from 4-iodo-1,8-naphthyridin-3-amine and dimethylphosphine oxide. [M+H]+ = 222.1

### Step 3: (3-((5-bromo-2-chloropyrimidin-4-yl)amino)-1,8-naphthyridin-4-yl)dimethylphosphine oxide

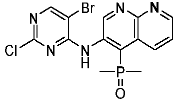

The titled compound (330 mg, 68%) was prepared in a manner similar to that in Example 1 step 3 from (3-amino-1,8-naphthyridin-4-yl)dimethylphosphine oxide and 5-bromo-2,4-dichloropyrimidine. [M+H]⁺ = 412.0.

### Step 4:

### (3-((5-bromo-2-((5-ethyl-2-methoxy-4-(4-(piperazin-1-yl)piperidin-1-yl)phenyl)amino)pyrimidin-4-yl)amino)-1, 8-naphthyridin-4-yl)dimethylphosphine oxide

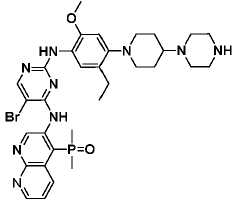

The titled compound (180 mg, 76%) was prepared in a manner similar to that in Example 1 step 4 from (3-((5-bromo-2-chloropyrimidin-4-yl)amino)-1,8-naphthyridin-4-yl)dimethylphosphine oxide and tert-butyl 4-(1-(4-amino-2-ethyl-5-methoxyphenyl)piperidin-4-yl)piperazine-1-carboxylate. [M+H]⁺ = 694.1.

### Step 5:

### 3-(4-(2-(4-(1-(4-((5-bromo-2-((4-(dimethylphosphoryl)-1,8-naphthyridin-3-yl)amino)pyrimidin-4-yl)amino)-2-et hyl-5-methoxyphenyl)piperidin-4-yl)piperazin-1-yl)ethyl)-2,6-difluorophenyl)piperidine-2,6-dione

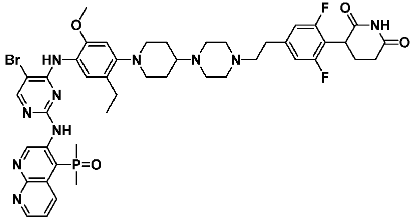

The titled compound (10.2 mg, 28%) was prepared in a manner similar to that in Example 2 step 7 from (3-((5-bromo-2-((5-ethyl-2-methoxy-4-(4-(piperazin-1-yl)piperidin-1-yl)phenyl)amino)pyrimidin-4-yl)amino)-1, 8-naphthyridin-4-yl) dimethylphosphine oxide and 2-(4-(2,6-dioxopiperidin-3-yl)-3,5-difluorophenyl)acetaldehyde. 1H NMR (500 MHz, DMSO) δ 11.47 (s, 1H), 10.94 (s, 1H), 9.59 (s, 1H), 9.00 (s, 1H), 8.79 (s, 1H), 8.26 (s, 1H), 7.98 (s, 1H), 7.63 (s, 1H), 7.40 (s, 1H), 7.03 (d, J = 10.0 Hz, 2H), 6.68 (s, 1H), 4.20 (dd, J = 12.6, 5.2 Hz, 1H), 3.77 (m, 4H), 2.92 (d, J = 10.5 Hz, 2H), 2.83 - 2.74 (m, 3H), 2.62 - 2.52 (m, 7H), 2.48 - 2.42 (m, 4H), 2.27 (s, 3H), 2.17 - 1.99 (m, 9H), 1.81 (d, J = 10.7 Hz, 2H), 1.57 - 1.47 (m, 2H), 0.67 (m, 3H). [M+H]⁺ = 945.5.

### Example 38:

### 3-(4-(2-(4-(1-(4-((5-bromo-4-((4-(dimethylphosphoryl)-1,7-naphthyridin-3-yl)amino)pyrimidin-2-yl)amino)-2-et hyl-5-methoxyphenyl)piperidin-4-yl)piperazin-1-yl)ethyl)-2,6-difluorophenyl)piperidine-2,6-dione

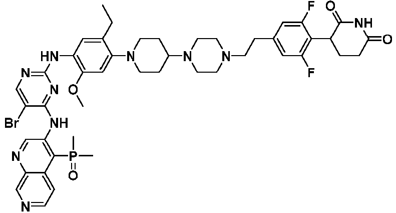

### Step 1: 4-iodo-1,7-naphthyridin-3-amine

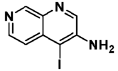

The titled compound (1.8 g, 97%) was prepared in a manner similar to that in Example 1 step 1 from 1,7-naphthyridin-3-amine and iodine monochloride. [M+H]⁺ = 271.9.

### Step 2: (3-amino-1,7-naphthyridin-4-yl)dimethylphosphine oxide

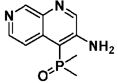

The titled compound (1.4 g, 95%) was prepared in a manner similar to that in Example 1 step 2 from 4-iodo-1,7-naphthyridin-3-amine and dimethylphosphine oxide. [M+H]⁺ = 222.1.

### Step 3: (3-((5-bromo-2-chloropyrimidin-4-yl)amino)-1,7-naphthyridin-4-yl)dimethylphosphine oxide

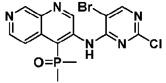

The titled compound (60 mg, 16%) was prepared in a manner similar to that in Example 1 step 3 from (3-amino-1,7-naphthyridin-4-yl)dimethylphosphine oxide and 5-bromo-2,4-dichloropyrimidine. [M+H]⁺ = 411.9.

### Step 4:

### (3-((5-bromo-2-((5-ethyl-2-methoxy-4-(4-(piperazin-1-yl)piperidin-1-yl)phenyl)amino)pyrimidin-4-yl)amino)-1 7-naphthyridin-4-yl)dimethylphosphine oxide

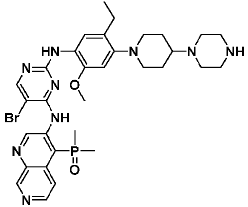

The titled compound (60 mg, 71%) was prepared in a manner similar to that in Example 1 step 4 from (3-((5-bromo-2-chloropyrimidin-4-yl)amino)-1,7-naphthyridin-4-yl)dimethylphosphine oxide and tert-butyl 4-(1-(4-amino-2-ethyl-5-methoxyphenyl)piperidin-4-yl)piperazine-1-carboxylate. [M+H]⁺ = 694.2.

### Step 5:

### 3-(4-(2-(4-(1-(4-((5-bromo-4-((4-(dimethylphosphoryl)-1,7-naphthyridin-3-yl)amino)pyrimidin-2-yl)amino)-2-et hyl-3-methoxyphenyl)piperidin-4-yl)piperazin-1-yl)ethyl)-2,6-difluorophenyl)piperidine-2,6-dione

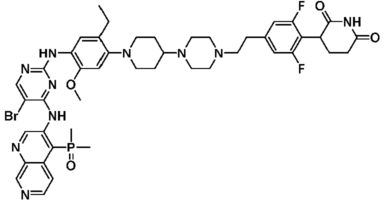

To a solution of (3-((5-bromo-2-((5-ethyl-2-methoxy-4-(4-(piperazin-1-yl)piperidin-1-yl)phenyl)amino)pyrimidin-4-yl)amino)-1, 7-naphthyridin-4-yl)dimethylphosphine oxide (30 mg, 0.04 mmol) in DCM (5 mL) was added 2-(4-(2,6-dioxopiperidin-3-yl)-3,5-difluorophenyl)acetaldehyde (11 mg, 0.04 mmol) at 20 °C. The mixture was stirred at 20 °C for 1 hr and STAB (18 mg, 0.08 mmol) was added. Then the mixture was stirred at 20 °C for 12 hrs. Water (10 mL) was poured into the mixture. Then the mixture was extracted with DCM (50 mL). The organic phase was washed with brine (50 mL), dried over Na₂SO₄, filtered and concentrated in vacuum. The residue was purified by prep-HPLC with C-18 column chromatography (0.1% FA in water: acetonitrile = 90 : 10 ~ 60 : 40 gradient elution) to afford the product (2.3 mg, 5.5%). ¹H NMR (500 MHz, DMSO) δ 12.16 (s, 1H), 10.96 (s, 1H), 9.68 (s, 1H), 9.25 (s, 1H), 8.56 (d, *J =* 6.0 Hz, 1H), 8.29 (d, *J* = 28.2 Hz, 2H), 7.97 (d, *J =* 5.8 Hz, 1H), 7.39 (s, 1H), 7.09 (d, *J* = 10.0 Hz, 2H), 6.74 (s, 1H), 4.22 (dd, *J* = 12.7, 4.9 Hz, 1H), 3.79 (s, 3H), 3.51-3.29 (m, 4H), 3.05 (d, *J =* 9.2 Hz, 4H), 2.93 - 2.75 (m, 5H), 2.72 - 2.55 (m, 5H), 2.43 - 2.36 (m, 3H), 2.23 - 1.94 (m, 10H), 1.75 (s, 2H), 0.83 (s, 3H). [M+H]⁺ = 945.6.

### Example 124:

### 3-(4-(2-(4-(1-(4-((5-bromo-4-((4-(dimethylphosphoryl)-6-fluoroquinolin-3-yl)amino)pyrimidin-2-yl)amino)-5-et hoxy-2-ethylphenyl)piperidin-4-yl)piperazin-1-yl)ethyl)-3-methyl-2-oxo-2,3-dihydro-1H-benzo[d]imidazol-1-yl) piperidine-2,6-dione

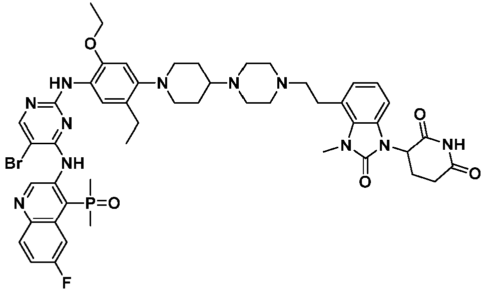

The title compound was prepared in a procedure similar to that in Example 1. [M+H]⁺ = 1010.4.

### Example 125:

### 3-(4-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-3-methyl-2-oxo-2,3-dihydro-1H-benzo[d]imidazol-1-yl)piperid ine-2,6-dione

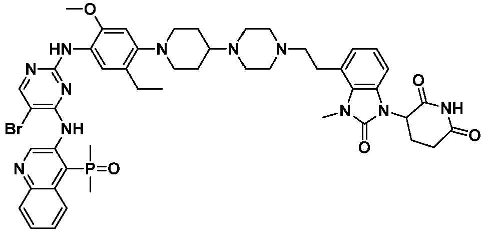

The title compound was prepared in a procedure similar to that in Example 1. [M+H]⁺ = 978.7.

### Example 126:

### (S)-3-(7-(2-(4-(1-(4-((5-bromo-4-((4-(dimethylphosphoryl)quinolin-3-yl)amino)pyrimidin-2-yl)amino)-2-ethyl-5 -methoxyphenyl)piperidin-4-yl)piperazin-1-yl)ethyl)-4-fluoro-2-oxobenzo[d]oxazol-3(2H)-yl)piperidine-2,6-dio ne

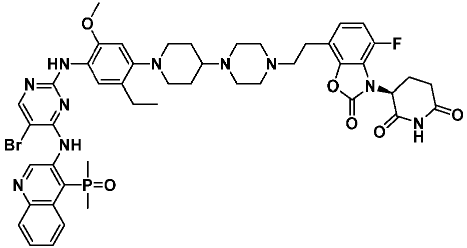

The title compound was prepared in a procedure similar to that in Example 1. [M+H]⁺ = 983.7.

### Example 130:

### 3-(4-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-7-fluoro-3-methyl-2-oxo-2,3-dihydro-1H-benzo[d]imidazol-1-yl)piperidine-2,6-dione

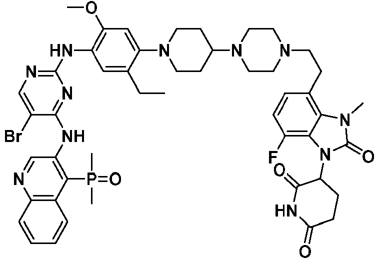

The title compound was prepared in a procedure similar to that in Example 1. [M+H]⁺ = 996.7.

### Example 132:

### 3-(6-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-2-oxobenzo[d]oxazol-3(2H)-yl)piperidine-2,6-dione

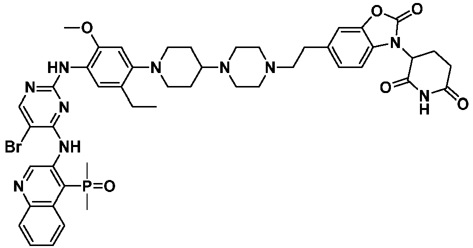

The title compound was prepared in a procedure similar to that in Example 1. [M+H]⁺ = 965.3.

### Example 133:

### 3-(6-(2-(4-(1-(4-((5-bromo-4-((4-(dimethylphosphoryl)-7-fluoroquinolin-3-yl)amino)pyrimidin-2-yl)amino)-5-et hoxy-2-ethylphenyl)piperidin-4-yl)piperazin-1-yl)ethyl)-2-oxobenzo[d]oxazol-3(2H)-yl)piperidine-2,6-dione

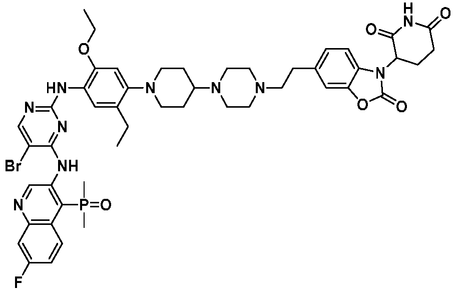

The title compound was prepared in a procedure similar to that in Example 1. [M+H]⁺ = 997.7.

### Example 134:

### 3-(6-(2-(4-(1-(4-((5-bromo-4-((4-(dimethylphosphoryl)-7-fluoroquinolin-3-yl)amino)pyrimidin-2-yl)amino)-2-et hyl-5-methoxyphenyl)piperidin-4-yl)piperazin-1-yl)ethyl)-2-oxobenzo[d]oxazol-3(2H)-yl)piperidine-2,6-dione

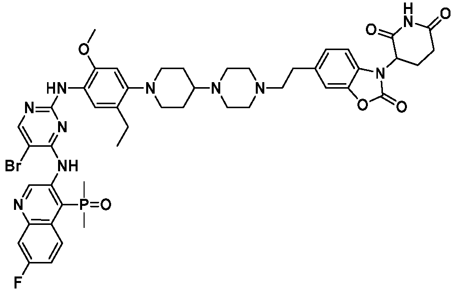

The title compound was prepared in a procedure similar to that in Example 1. [M+H]⁺ = 983.6.

### Example 135:

### 3-(4-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-3-ethyl-2-oxo-2,3-dihydro-1H-benzo[d]imidazol-1-yl)piperidin e-2,6-dione

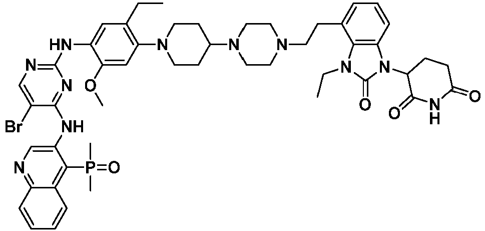

The title compound was prepared in a procedure similar to that in Example 1. [M+H]⁺ = 992.1.

### Example 136:

### 3-(7-(2-(4-(1-(4-((5-bromo-4-((4-(dimethylphosphoryl)quinolin-3-yl)amino)pyrimidin-2-yl)amino)-5-ethoxy-2-e thylphenyl)piperidin-4-yl)piperazin-1-yl)ethyl)-4,5-difluoro-2-oxobenzo[d]oxazol-3(2H)-yl)piperidine-2,6-dione

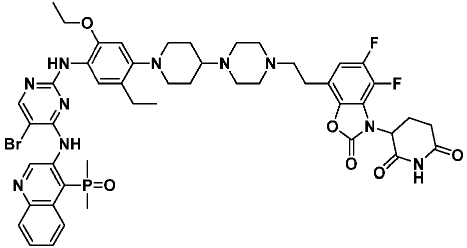

The title compound was prepared in a procedure similar to that in Example 1. [M+H]⁺ = 1015.6.

### Example 137:

### 3-(4-(2-(4-(1-(4-((5-bromo-4-((4-(dimethylphosphoryl)quinolin-3-yl)amino)pyrimidin-2-yl)amino)-5-ethoxy-2-e thylphenyl)piperidin-4-yl)piperazin-1-yl)ethyl)-5-fluoro-3-methyl-2-oxo-2,3-dihydro-1H-benzo[d]imidazol-1-yl) piperidine-2,6-dione

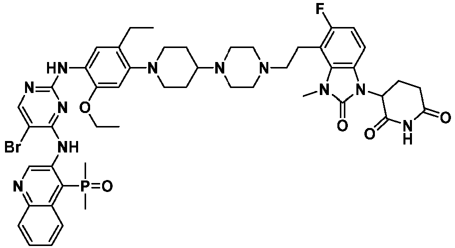

The title compound was prepared in a procedure similar to that in Example 1. [M+H]⁺ = 1010.2.

### Example 138:

### 3-(4-(2-(4-(1-(4-((5-bromo-4-((4-(dimethylphosphoryl)quinolin-3-yl)amino)pyrimidin-2-yl)amino)-5-ethoxy-2-e thylphenyl)piperidin-4-yl)piperazin-1-yl)ethyl)-3,3-dimethyl-2-oxoindolin-1-yl)piperidine-2,6-dione

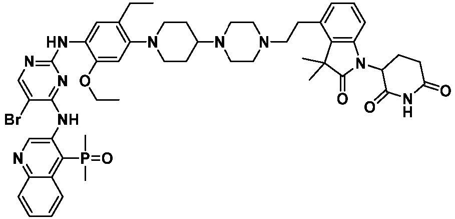

The title compound was prepared in a procedure similar to that in Example 1. [M+H]⁺ = 1005.2.

### Example 139:

### 3-(6-(2-(4-(1-(4-((5-bromo-4-((4-(dimethylphosphoryl)quinolin-3-yl)amino)pyrimidin-2-yl)amino)-5-ethoxy-2-e thylphenyl)piperidin-4-yl)piperazin-1-yl)ethyl)-2-oxobenzo[d]oxazol-3(2H)-yl)piperidine-2,6-dione

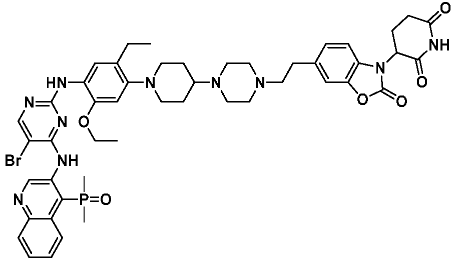

The title compound was prepared in a procedure similar to that in Example 1. [M+H]⁺ = 979.1.

### Example 140:

### 3-(4-(2-(4-(1-(4-((5-bromo-4-((4-(dimethylphosphoryl)quinolin-3-yl)amino)pyrimidin-2-yl)amino)-5-ethoxy-2-e thylphenyl)piperidin-4-yl)piperazin-1-yl)ethyl)-3-methyl-2-oxo-2,3-dihydro-1H-benzo[d]imidazol-1-yl)piperidin e-2,6-dione

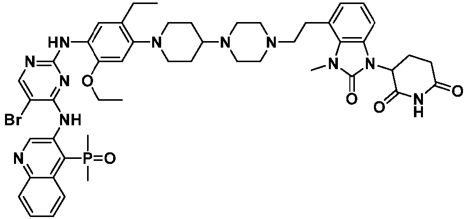

The title compound was prepared in a procedure similar to that in Example 1. [M+H]⁺ = 992.3.

### Example 142:

### 3-(6-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-7-fluoro-2-oxobenzo[d]oxazol-3(2H)-yl)piperidine-2,6-dione

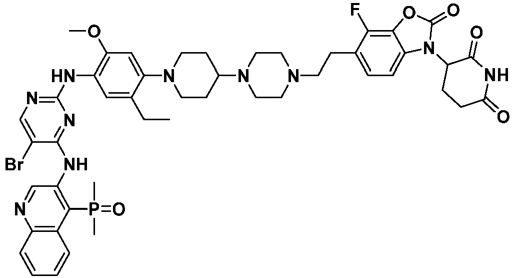

The title compound was prepared in a procedure similar to that in Example 1. [M+H]⁺ = 983.7.

### Example 147:

### 3-(4-(2-(4-(1-(4-((5-bromo-4-((4-(dimethylphosphoryl)-7-fluoroquinolin-3-yl)amino)pyrimidin-2-yl)amino)-5-et hoxy-2-ethylphenyl)piperidin-4-yl)piperazin-1-yl)ethyl)-3-methyl-2-oxo-2,3-dihydro-1H-benzo[d]imidazol-1-yl) piperidine-2,6-dione

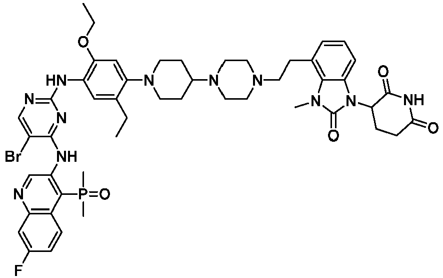

The title compound was prepared in a procedure similar to that in Example 1. [M+H]⁺ = 1010.7.

### Example 149:

### 3-(6-(2-(4-(1-(4-((5-bromo-4-((4-(dimethylphosphoryl)-7-fluoroquinolin-3-yl)amino)pyrimidin-2-yl)amino)-5-et hoxy-2-ethylphenyl)piperidin-4-yl)piperazin-1-yl)ethyl)-2-oxobenzo[d]oxazol-3(2H)-yl)piperidine-2,6-dione

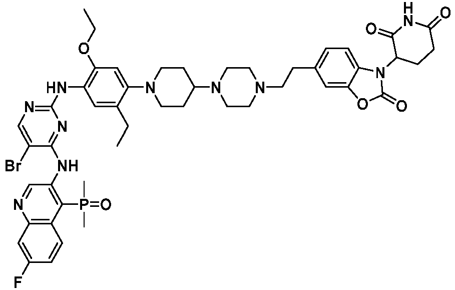

The title compound was prepared in a procedure similar to that in Example 1. [M+H]⁺ = 997.7.

### Example 151:

### (S)-3-(6-(2-(4-(1-(4-((5-bromo-4-((4-(dimethylphosphoryl)-7-fluoroquinolin-3-yl)amino)pyrimidin-2-yl)amino)-2-ethyl-5-methoxyphenyl)piperidin-4-yl)piperazin-1-yl)ethyl)-4-fluoro-2-oxobenzo[d]oxazol-3(2H)-yl)piperidin e-2,6-dione

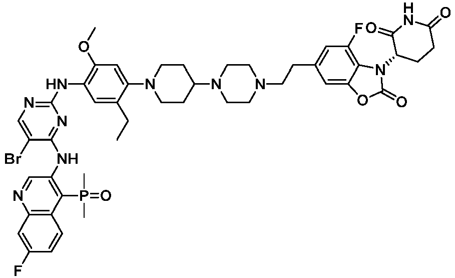

The title compound was prepared in a procedure similar to that in Example 1. [M+H]⁺ = 1001.7.

### Example 153:

### 3-(6-(2-(4-(1-(4-((5-bromo-4-((4-(dimethylphosphoryl)-7-fluoroquinolin-3-yl)amino)pyrimidin-2-yl)amino)-2-et hyl-5-methoxyphenyl)piperidin-4-yl)piperazin-1-yl)ethyl)-2-oxobenzo[d]oxazol-3(2H)-yl)piperidine-2,6-dione

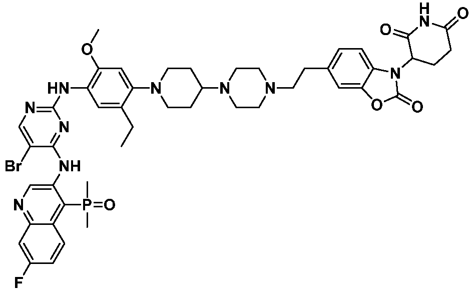

The title compound was prepared in a procedure similar to that in Example 1. [M+H]⁺ = 983.4.

### Example 155:

### 3-(4-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-3-cyclopropyl-2-oxo-2,3-dihydro-1H-benzo[d]imidazol-1-yl)pi peridine-2,6-dione

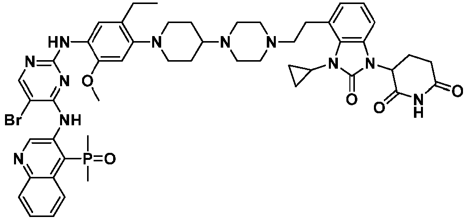

The title compound was prepared in a procedure similar to that in Example 1. [M+H]⁺ = 1004.2.

### Example 159:

### 3-(4-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-5-fluoro-3-methyl-2-oxo-2,3-dihydro-1H-benzo[d]imidazol-1-yl)piperidine-2,6-dione

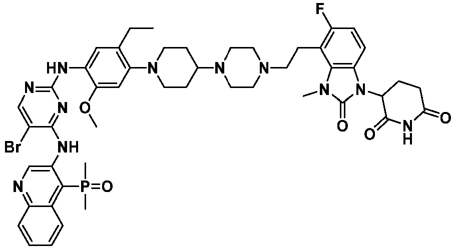

The title compound was prepared in a procedure similar to that in Example 1. [M+H]⁺ = 996.1.

### Example 163:

### 3-(4-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-3-ethyl-2-oxo-2,3-dihydro-1H-benzo[d]imidazol-1-yl)piperidin e-2,6-dione

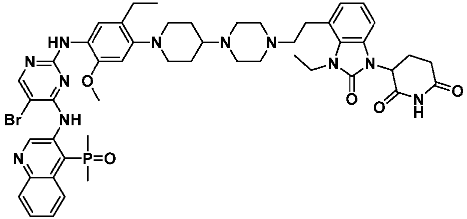

The title compound was prepared in a procedure similar to that in Example 1. [M+H]⁺ = 992.1.

### Example 164:

### 3-(4-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-3,3-dimethyl-2-oxoindolin-1-yl)piperidine-2,6-dione

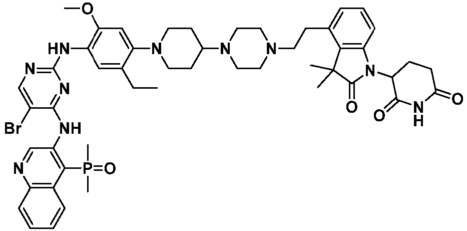

The title compound was prepared in a procedure similar to that in Example 1. [M+H]⁺ = 991.7.

### Example 165:

### 3-(4-(4-(2-(4-(1-(4-((5-bromo-4-((4-(dimethylphosphoryl)-8-fluoroquinolin-3-yl)amino)pyrimidin-2-yl)amino)-2 -ethyl-5-methoxyphenyl)piperidin-4-yl)piperazin-1-yl)ethyl)piperidin-1-yl)-2,6-difluorophenyl)piperidine-2,6-di one

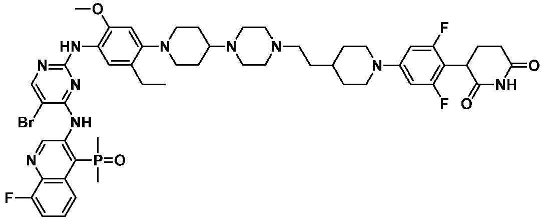

The title compound was prepared in a procedure similar to that in Example 1. [M+H]⁺ = 1045.7.

### Example 166:

### 3-(4-(2-(4-(1-(4-((5-bromo-4-((4-(dimethylphosphoryl)-6-(trifluoromethyl)quinolin-3-yl)amino)pyrimidin-2-yl)a mino)-2-ethyl-5-methoxyphenyl)piperidin-4-yl)piperazin-1-yl)ethyl)-3-methyl-2-oxo-2,3-dihydro-1H-benzo[d]i midazol-1-yl)piperidine-2,6-dione

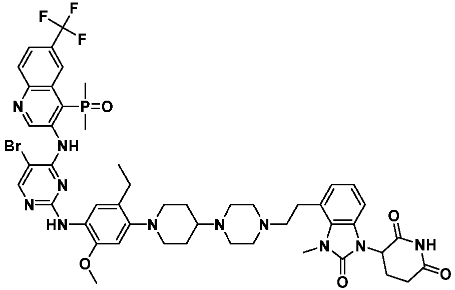

The title compound was prepared in a procedure similar to that in Example 1. [M+H]⁺ = 1046.1.

### Example 167:

### 3-(4-(2-(4-(1-(4-((5-bromo-4-((4-(dimethylphosphoryl)-7-fluoroquinolin-3-yl)amino)pyrimidin-2-yl)amino)-2-et hyl-5-methoxyphenyl)piperidin-4-yl)piperazin-1-yl)ethyl)-3-methyl-2-oxo-2,3-dihydro-1H-benzo[d]imidazol-1-yl)piperidine-2,6-dione

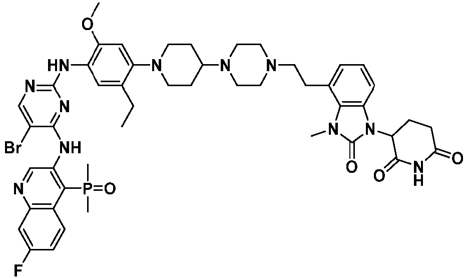

The title compound was prepared in a procedure similar to that in Example 1. [M+H]⁺ = 996.6.

### Example 168:

### 3-(5-((S)-3-(4-(1-(4-((5-bromo-4-((4-(dimethylphosphoryl)quinolin-3-yl)amino)pyrimidin-2-yl)amino)-2-ethyl-5 -methoxyphenyl)piperidin-4-yl)piperazine-1-carbonyl)pyrrolidin-1-yl)-3,3-dimethyl-2-oxoindolin-1-yl)piperidin e-2,6-dione

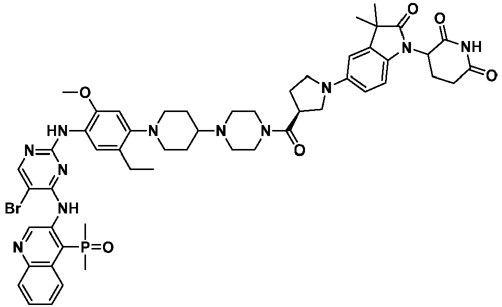

The title compound was prepared in a procedure similar to that in Example 23. [M+H]⁺ = 1060.7.

### Example 169:

### 3-(4-(3-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazine-1-carbonyl)azetidin-1-yl)-3,3-dimethyl-2-oxoindolin-1-yl)piperidine-2,6-dione

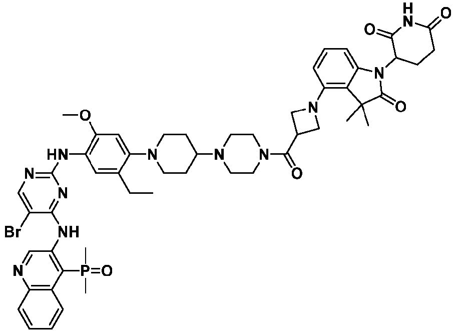

The title compound was prepared in a procedure similar to that in Example 23. [M+H]⁺ = 1046.7.

### Example 170:

### 3-(4-(2-(4-(1-(4-((5-bromo-4-((4-(dimethylphosphoryl)-6-fluoroquinolin-3-yl)amino)pyrimidin-2-yl)amino)-2-et hyl-5-methoxyphenyl)piperidin-4-yl)piperazin-1-yl)ethyl)-3-methyl-2-oxo-2,3-dihydro-1H-benzo[d]imidazol-1-yl)piperidine-2,6-dione

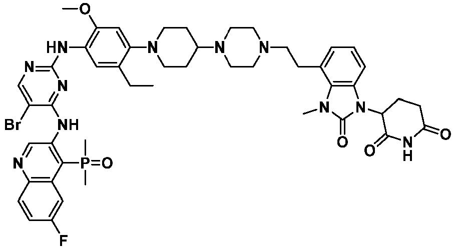

The title compound was prepared in a procedure similar to that in Example 1. [M+H]⁺ = 996.5.

### Example 171:

### 3-(4-(2-(4-(1-(4-((5-bromo-4-((4-(dimethylphosphoryl)-6-(trifluoromethyl)quinolin-3-yl)amino)pyrimidin-2-yl)a mino)-2-ethyl-5-methoxyphenyl)piperidin-4-yl)piperazin-1-yl)ethyl)-2,6-difluorophenyl)piperidine-2,6-dione

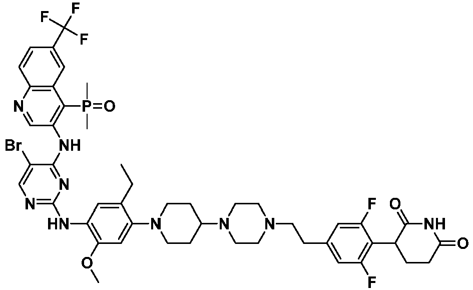

The title compound was prepared in a procedure similar to that in Example 1. [M+H]⁺ = 1012.3.

### Example 172:

### 3-(4-(2-(4-(1-(4-((5-bromo-4-((4-(dimethylphosphoryl)-7-fluoroquinolin-3-yl)amino)pyrimidin-2-yl)amino)-2-et hyl-5-methoxyphenyl)piperidin-4-yl)piperazin-1-yl)ethyl)-2,6-difluorophenyl)piperidine-2,6-dione

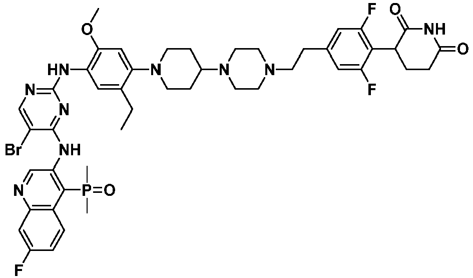

The title compound was prepared in a procedure similar to that in Example 1. [M+H]⁺ = 962.6.

### Example 173:

### 3-(4-(2-(4-(1-(4-((5-bromo-4-((4-(dimethylphosphoryl)-7,8-difluoroquinolin-3-yl)amino)pyrimidin-2-yl)amino)-2-ethyl-5-methoxyphenyl)piperidin-4-yl)piperazin-1-yl)ethyl)-2,6-difluorophenyl)piperidine-2,6-dione

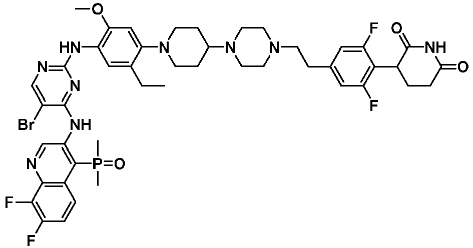

The title compound was prepared in a procedure similar to that in Example 1. [M+H]⁺ = 980.4.

### Example 174:

### 3-(4-(2-(4-(1-(4-((5-bromo-4-((4-(dimethylphosphoryl)-6-fluoroquinolin-3-yl)amino)pyrimidin-2-yl)amino)-2-et hyl-5-methoxyphenyl)piperidin-4-yl)piperazin-1-yl)ethyl)-2,6-difluorophenyl)piperidine-2,6-dione

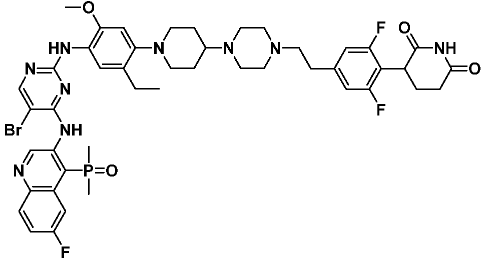

The title compound was prepared in a procedure similar to that in Example 1. [M+H]⁺ = 962.5.

### Example 175:

### 3-(5-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-3,3-dimethyl-2-oxoindolin- 1-yl)piperidine-2,6-dione

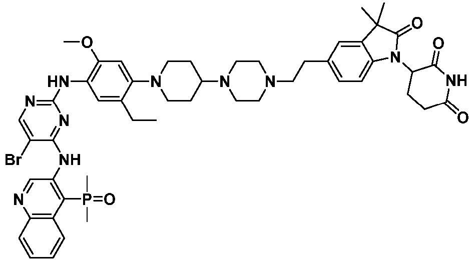

The title compound was prepared in a procedure similar to that in Example 1. [M+H]⁺ = 991.7.

### Example 176:

### 3-(6-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-2-oxobenzo[d]oxazol-3(2H)-yl)piperidine-2,6-dione

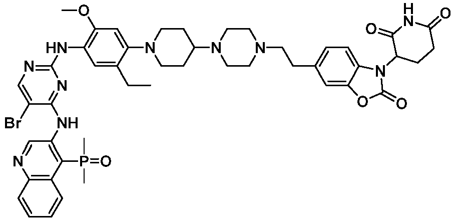

The title compound was prepared in a procedure similar to that in Example 1. [M+H]⁺ = 965.6.

### Example 178:

### 3-(4-(6-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazine-1-carbonyl)-2-azaspiro[3.3]heptan-2-yl)-2,6-difluorophenyl)piperidine-2, 6-dione

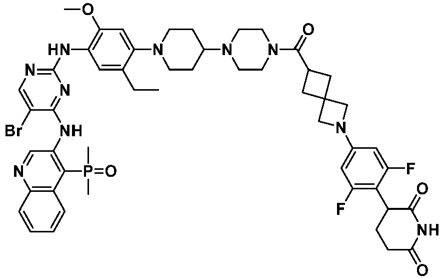

The title compound was prepared in a procedure similar to that in Example 23. [M+H]⁺ = 1039.6.

### Example 179:

### 3-(4-(3-(4-(1-(4-((5-bromo-4-((4-(dimethylphosphoryl)quinolin-3-yl)amino)pyrimidin-2-yl)amino)-5-methoxy-2 -methylphenyl)piperidin-4-yl)piperazine-1-carbonyl)azetidin-1-yl)-3-methyl-2-oxo-2,3-dihydro-1H-benzo[d]imi dazol-1-yl)piperidine-2,6-dione

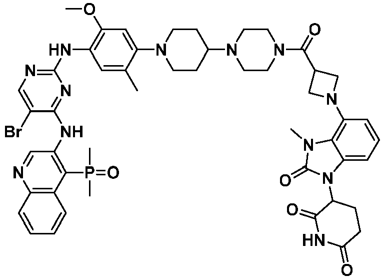

The title compound was prepared in a procedure similar to that in Example 23. [M+H]⁺ = 1019.6.

### Example 180:

### 3-(4-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-2,3,6-trifluorophenyl)piperidine-2,6-dione

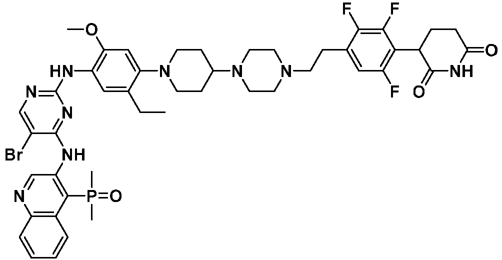

The title compound was prepared in a procedure similar to that in Example 1. [M+H]⁺ = 962.4.

### Example 181:

### 3-(4-(2-(4-(1-(4-((5-bromo-4-((4-(dimethylphosphoryl)-8-fluoroquinolin-3-yl)amino)pyrimidin-2-yl)amino)-2-et hyl-5-methoxyphenyl)piperidin-4-yl)piperazin-1-yl)ethyl)-2,6-difluorophenyl)piperidine-2,6-dione

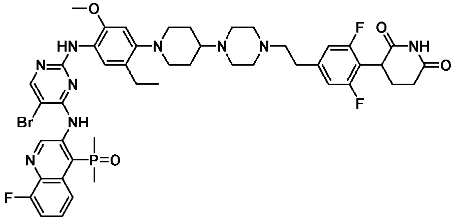

The title compound was prepared in a procedure similar to that in Example 1. [M+H]⁺ = 962.3.

### Example 182:

### 3-(5-(4-(4-(1-(4-((5-bromo-4-((4-(dimethylphosphoryl)-8-fluoroquinolin-3-yl)amino)pyrimidin-2-yl)amino)-2-et hyl-5-methoxyphenyl)piperidin-4-yl)piperazine-1-carbonyl)piperidin-1-yl)-3,3-dimethyl-2-oxoindolin-1-yl)piper idine-2,6-dione

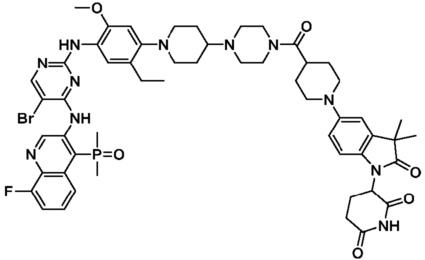

The title compound was prepared in a procedure similar to that in Example 23. [M+H]⁺ = 1092.4.

### Example 183:

### 3-(4-(2-(4-(1-(4-((5-bromo-4-((4-(dimethylphosphoryl)quinolin-3-yl)amino)pyrimidin-2-yl)amino)-5-ethoxy-2-methylphenyl)piperidin-4-yl)piperazin-1-yl)ethyl)-2,6-difluorophenyl)piperidine-2,6-dione

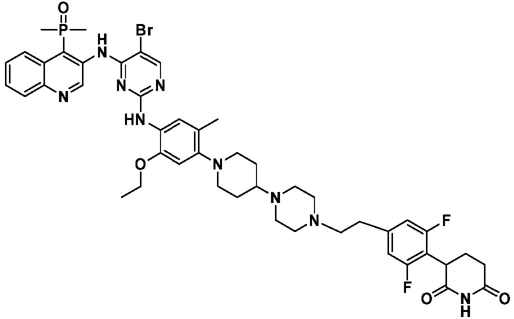

The title compound was prepared in a procedure similar to that in Example 1. [M+H]⁺ = 944.6.

### Example 184:

### 3-(4-((lr,3r)-3-(4-(1-(4-((5-bromo-4-((4-(dimethylphosphoryl)quinolin-3-yl)amino)pyrimidin-2-yl)amino)-2-eth yl-5-methoxyphenyl)piperidin-4-yl)piperazin-1-yl)cyclobutyl)-2,6-difluorophenyl)piperidine-2,6-dione

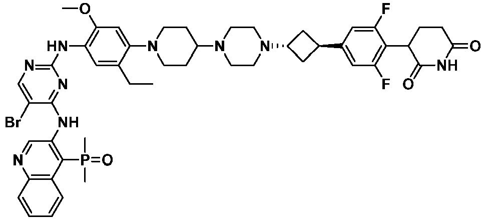

The title compound was prepared in a procedure similar to that in Example 1. [M+H]⁺ = 970.6.

### Example 185:

### 3-(4-(4-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazine-1-carbonyl)piperidin-1-yl)-2,6-difluorophenyl)piperidine-2,6-dione

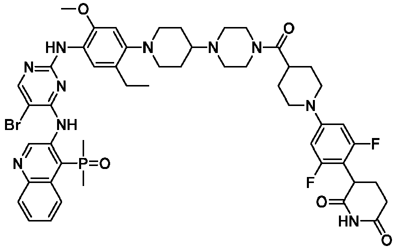

The title compound was prepared in a procedure similar to that in Example 23. [M+H]⁺ = 1027.7.

### Example 186:

### 3-(4-(4-(2-(4-(1-(4-((5-bromo-4-((4-(dimethylphosphoryl)quinolin-3-yl)amino)pyrimidin-2-yl)amino)-2-ethyl-5-(2,2,2-trifluoroethoxy)phenyl)piperidin-4-yl)piperazin-1-yl)ethyl)piperidin-1-yl)-2,6-difluorophenyl)piperidine-2 ,6-dione

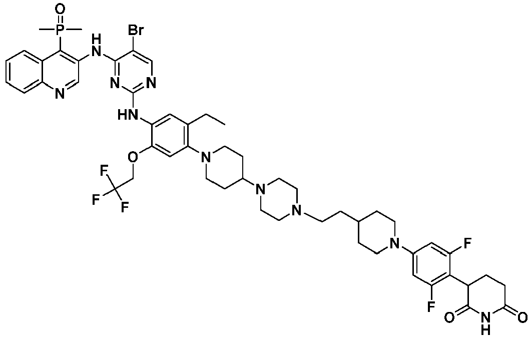

The title compound was prepared in a procedure similar to that in Example 1. [M+H]⁺ = 1095.2.

### Example 187:

### 3-(4-(2-(4-(1-(4-((5-bromo-4-((4-(dimethylphosphoryl)quinolin-3-yl)amino)pyrimidin-2-yl)amino)-2-ethyl-5-(2, 2,2-trifluoroethoxy)phenyl)piperidin-4-yl)piperazin-1-yl)ethyl)-2,6-difluorophenyl)piperidine-2,6-dione

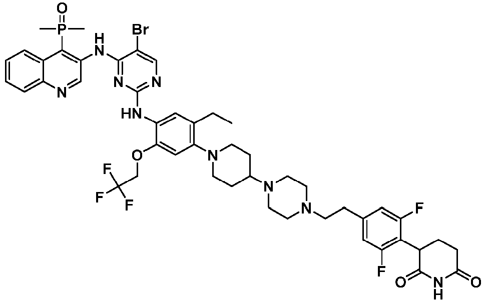

The title compound was prepared in a procedure similar to that in Example 1. [M+H]⁺ = 1012.2.

### Example 188:

### 3-(7-(2-(4-(1-(4-((5-bromo-4-((4-(dimethylphosphoryl)quinolin-3-yl)amino)pyrimidin-2-yl)amino)-2-ethyl-5-me thoxyphenyl)piperidin-4-yl)piperazin-1-yl)ethyl)-2-oxobenzo[d]oxazol-3(2H)-yl)piperidine-2,6-dione

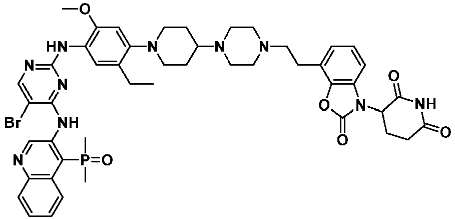

The title compound was prepared in a procedure similar to that in Example 1. [M+H]⁺ = 965.5.

### Example 189:

### 3-(4-((2-(4-(1-(4-((5-bromo-4-((4-(dimethylphosphoryl)quinolin-3-yl)amino)pyrimidin-2-yl)amino)-2-ethyl-5-m ethoxyphenyl)piperidin-4-yl)piperazin-1-yl)-2-oxoethyl)(methyl)amino)-2,6-difluorophenyl)piperidine-2,6-dione

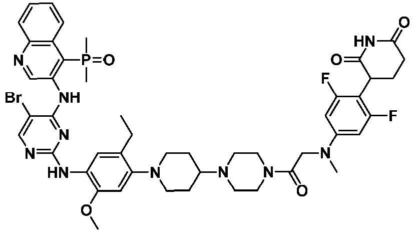

The title compound was prepared in a procedure similar to that in Example 23. [M+H]⁺ = 987.5.

### Cell Degradation of Example 1-28

### Cell treatment

BaF3-LTC(L858R/T790M/C797S) and BaF3-DTC(Del19/T790M/C797S) cells are seeded at 100000 cells/well in cell culture medium [RPMI1640(Gibco, phenol red free, Cat#11835-030), 10% heat-inactive FBS, 1%PS(Gibco, Cat#10378)] in Corning 96 well plate (Cat#3799).

BaF3-LTC(L858R/T790M/C797S) and BaF3-DTC(Del19/T790M/C797S) cells are treated with compounds diluted in 0.2%DMSO cell culture medium and incubate for 16h, 37°C, 5%CO₂. The final concentriation of compounds in all assay is start with 10 uM, 4-fold dilution, total 8 doses were included.

### HTRF assay

After 16h treatment, add HTRF lysis buffer to each well ; seal the plate and incubate 1 hour at room temperature on a plate shaker; Once the cells are lysed, 16 µL of cell lysate are transferred to a PE 384-well HTRF detection plate; 4 µL of pre-mixed HTRF antibodies are added to each well ; Cover the plate with a plate sealer, spin 1000 rpm for 1 min, Incubate overnight at room temperature; Read on BMG PheraStar with HTRF protocol (337nm-665nm-620nm).

The inhibition (degradation) percentage of the compound was calculated by the following equation: Inhibition percentage of Compound = 100-100 × (Signal-low control) / (High control-low control), wherein signal = each test compound group.
Low control = only lysis buffer without cells, indicating that EGFR is completely degraded;
High control = Cell group with added DMSO and without compound, indicating microplate readings without EGFR degradation;
Dmax is the maximum percentage of inhibition (degradation).

The IC₅₀ (DC₅₀) value of a compound can be obtained by fitting the following equation$Y = Bottom + \left(TOP-Bottom\right) / \left(1+\left({\left({IC}_{50}/X\right)}^{h}illslope\right)\right)$

Wherein, X and Y are known values, and IC₅₀, Hillslope, Top and Bottom are the parameters obtained by fitting with software. Y is the inhibition percentage (calculated from the equation), X is the concentration of the compound; IC₅₀ is the concentration of the compound when the 50% inhibition is reached. The smaller the IC₅₀ value is, the stronger the inhibitory ability of the compound is. Vice versa, the higher the IC₅₀ value is, the weaker the ability the inhibitory ability of the compound is; Hillslope represents the slope of the fitted curve, generally around 1 *; Bottom represents the minimum value of the curve obtained by data fitting, which is generally 0% ± 20%; Top represents the maximum value of the curve obtained by data fitting, which is generally 100% ± 20%. The experimental data were fitted by calculating and analyzing with Dotmatics data analysis software. When Dmax <50%, DC₅₀ will be automatically assigned as larger than 10000 µM.

**Table 1. Degradation (BaF3) result for Example 1 to Example 28**

| | DTC | | LTC | |
|---|---|---|---|---|
| Example | DC₅₀(nM) | Dmax (%) | DC₅₀(nM) | Dmax (%) |
| 1 | 10.43 | 96.55 | 24.96 | 90.66 |
| 2 | 18.49 | 86.75 | 146.4 | 85.03 |
| 4 | 3.94 | 91.36 | 7.75 | 90.47 |
| 7 | 15.52 | 93.86 | 45.26 | 82.68 |
| 8 | 11.77 | 96.88 | 30.16 | 89.62 |
| 9 | 12.65 | 96.14 | 29.32 | 87.96 |
| 10 | 24.79 | 88.58 | 218.9 | 88.39 |
| 12 | 14.45 | 91.82 | 68.83 | 89.09 |
| 14 | 185.6 | 81.73 | 546.1 | 84.57 |
| 15 | 1.23 | 94.57 | 3.23 | 86.4 |
| 16 | 2.14 | 96.98 | 5.05 | 89.67 |
| 17 | 1.94 | 96.58 | 3.86 | 92.5 |
| 18 | 3.95 | 97.59 | 14.57 | 90.69 |
| 19 | 4.24 | 97.44 | 8.02 | 89.37 |
| 20 | 4.65 | 96.7 | 19.52 | 89.53 |
| 21 | 1.11 | 94.17 | 5.02 | 93.31 |
| 23 | 2.22 | 94.5 | 11.07 | 88.64 |
| 24 | 2.88 | 84.3 | 27.54 | 89.6 |
| 25 | 1.8 | 88.68 | 6.77 | 85.01 |
| 27 | 3.08 | 83.05 | 19.31 | 87.55 |
| 28 | 5.4 | 90.9 | 8.95 | 81.86 |

### Cell Degradation of Example 124-189

### Cell treatment

On day 1, H1975-clone#28(Del19/T790M/C797S) and H1975-clone#25 (L858R/T790M/C797S) cells are seeded at 5000 cells/well correspondingly in cell culture medium [RPMI1640(Gibco, Cat#72400-047), 10% heat-inactive FBS, 1%PS(Gibco, Cat#10378)] in Corning 96 well plate (Cat#3599) . H1975-#28 and H1975-#25 cells are treated with compounds diluted in 0.2%DMSO cell culture medium on day 2, incubate for 16h, 37°C, 5%CO2 .the final concentriation of compounds in all assay is start with 10uM, 5-fold dilution, total 8 doses were included.

### HTRF assay

After 16h treatment, add HTRF lysis buffer to each well ; seal the plate and incubate 1 hour at room temperature on a plate shaker; Once the cells are lysed, 16 µL of cell lysate are transferred to a PE 384-well HTRF detection plate; 4 µL of pre-mixed HTRF antibodies are added to each well ; Cover the plate with a plate sealer, spin 1000 rpm for 1 min, Incubate overnight at room temperature; Read on BMG PheraStar with HTRF protocol (337nm-665nm-620nm).

The inhibition (degradation) percentage of the compound was calculated by the following equation: $Inhibition percentage of Compound = 100 - 100 \times \left(Signal-low control\right) / \left(High control-low control\right) ,$ wherein signal = each test compound group.
Low control = only lysis buffer without cells, indicating that EGFR is completely degraded;
High control = Cell group with added DMSO and without compound, indicating microplate readings without EGFR degradation;
Dmax is the maximum percentage of inhibition (degradation).

The IC₅₀ (DC₅₀) value of a compound can be obtained by fitting the following equation $Y = Bottom + \left(TOP-Bottom\right) / \left(1+\left({\left({IC}_{50}/X\right)}^{∧}hillslope\right)\right)$

Wherein, X and Y are known values, and IC₅₀, Hillslope, Top and Bottom are the parameters obtained by fitting with software. Y is the inhibition percentage (calculated from the equation), X is the concentration of the compound; IC₅₀ is the concentration of the compound when the 50% inhibition is reached. The smaller the IC₅₀ value is, the stronger the inhibitory ability of the compound is. Vice versa, the higher the IC₅₀ value is, the weaker the ability the inhibitory ability of the compound is; Hillslope represents the slope of the fitted curve, generally around 1 *; Bottom represents the minimum value of the curve obtained by data fitting, which is generally 0% ± 20%; Top represents the maximum value of the curve obtained by data fitting, which is generally 100% ± 20%. The experimental data were fitted by calculating and analyzing with Dotmatics data analysis software. When Dmax <50%, DC₅₀ will be automatically assigned as larger than 10000 µM.

**Table 2. Degradation (H1975) result for Example 122 to Example 189**

| | | H1975-clone#28 DTC | | H1975-clone#25 LTC |
|---|---|---|---|---|
| | H1975-clone#28 DTC | | H1975-clone#25 LTC | |
| Example | DC₅₀(nM) | Dmax (%) | DC₅₀(nM) | Dmax (%) |
| 124 | 1.4 | 73 | 3.4 | 77 |
| 126 | 1.3 | 84 | | |
| 130 | 1.0 | 86 | | |
| 132 | 1.0 | 80 | | |
| 133 | 3.0 | 71 | | |
| 134 | 0.8 | 79 | | |
| 135 | 0.9 | 83 | | |
| 136 | 0.6 | 81 | | |
| 137 | 2.1 | 86 | | |
| 138 | 3.0 | 83 | | |
| 139 | 1.5 | 75 | | |
| 140 | 4.4 | 84 | | |
| 142 | 0.9 | 81 | | |
| 147 | 0.4 | 84 | 15.3 | 77 |
| 149 | 1.3 | 73 | | |
| 151 | 1.1 | 79 | | |
| 153 | 0.8 | 77 | | |
| 155 | 0.7 | 85 | | |
| 159 | 1.4 | 85 | | |
| 163 | 1.1 | 86 | | |
| 164 | 2.1 | 81 | | |
| 165 | 4.2 | 84 | | |
| 166 | 2.8 | 80 | 4.2 | 81 |
| 167 | 0.8 | 74 | | |
| 168 | 2.0 | 47 | | |

| | H1975-clone#28 DTC | | H1975-clone#25 LTC | |
|---|---|---|---|---|
| 169 | 4.6 | 65 | | |
| 170 | 2.1 | 82 | | |
| 171 | 4.3 | 79 | | |
| 172 | 1.4 | 81 | | |
| 173 | 2.7 | 82 | | |
| 174 | 1.8 | 80 | | |
| 175 | 5.5 | 47 | | |
| 176 | 1.6 | 73 | | |
| 178 | 2.6 | 67 | | |
| 179 | 2.6 | 54 | | |
| 180 | 2.0 | 83 | | |
| 181 | 4.2 | 82 | | |
| 182 | 0.8 | 56 | | |
| 183 | 4.6 | 73 | | |
| 184 | 1.5 | 81 | | |
| 185 | 1.2 | 71 | | |
| 186 | 8.0 | 72 | | |
| 187 | 3.3 | 69 | | |
| 188 | 0.8 | 84 | | |
| 189 | 2.4 | 77 | | |

The foregoing examples and description of certain embodiments should be taken as illustrating, rather than as limiting the present invention as defined by the claims. As will be readily appreciated, numerous variations and combinations of the features set forth above can be utilized without departing from the present invention as set forth in the claims.

## Claims

1. A compound of Formula (X): 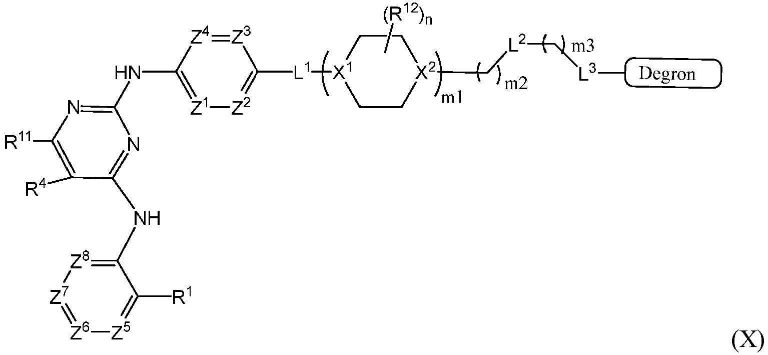 or a N-oxide thereof, or a pharmaceutically acceptable salt thereof, or a stereoisomer thereof, or a deuterated analog thereof, wherein:
R¹ is -P(O)R^{1a}R^{1b}, wherein R^{1a} and R^{1b} are each independently selected from -CH₃, - CH₂CH₃, -CH₂CH₂CH₃, *-iso-*C₃H₇, -CH₂CH₂CH₂CH₃, *-iso*-C₄H₉, *-sec*-C₄H₉ or-*tert*-C₄H₉, cyclopropyl, cyclobutyl or cyclopentyl;
Z⁵ is -CR²;
Z⁶ is -CR³;
Z⁷ is N;
Z⁸ is -CR¹⁰;
R² and R³ together with the carbon atoms to which they are attached, form a 5-6 membered aromatic ring, said ring comprising 0-3 heteroatoms independently selected from nitrogen, oxygen and sulfur; said ring is optionally substituted with at least one substituent R^{2e};
R^{2e} at each occurrence, is independently hydrogen, -F, -Cl, -Br, -I, methyl, ethyl, propyl, butyl, pentyl, cyclopropyl, cyclobutyl, or cyclopentyl, wherein each of methyl, ethyl, propyl, butyl, pentyl, cyclopropyl, cyclobutyl, or cyclopentyl is optionally substituted with at least one R^{2d}; and
R^{2d}, at each occurrence, is independently -F, -Cl, -Br, -I, -OH, methyl, ethyl, propyl, butyl, pentyl, hexyl, heptyl, octyl, methoxy, ethoxy, propoxy, butoxy, pentoxy, hexoxy, heptoxy, octoxy, cyclopropyl, cyclobutyl, cyclopentyl, cyclohexyl, cycloheptyl, cyclooctyl or phenyl;
R⁴ is selected from hydrogen, halogen, -C₁₋₈alkyl, -C₂₋₈alkenyl, -C₂₋₈alkynyl, -C₁₋₈alkoxy, - C₃-C₈cycloalkyl, 3- to 8-membered heterocyclyl, -C₆-C₁₂aryl, 5- to 12-membered heteroaryl, - CN, -SO₂R^{4a}, -SO₂NR^{4a}R^{4b}, -COR^{4a}, -CO₂R^{4a}, -CONR^{4a}R^{4b}, -NR^{4a}R^{4b}, -NR^{4a}COR^{4b}, - NR^{4a}CO₂R^{4b}, and -NR^{4a}SO₂R^{4b}, wherein each of -C₁₋₈alkyl, -C₂₋₈alkenyl, -C₂₋₈alkynyl, -C₁₋₈alkoxy, -C₃-C₈cycloalkyl, 3- to 8-membered heterocyclyl, -C₆-C₁₂aryl or 5- to 12-membered heteroaryl is optionally substituted with halogen, -C₁₋₈alkoxy, -C₁₋₈alkyl, -C₂₋₈alkenyl, -C₂₋₈alkynyl, -C₃-C₈cycloalkyl, 3- to 8-membered heterocyclyl, C₆-C₁₂aryl, 5- to 12-membered heteroaryl, oxo, -CN, -OR^{4c}, -SO₂R^{4c}, -SO₂NR^{4c}R^{4d}, -COR^{4c}, -CO₂R^{4c}, -CONR^{4c}R^{4d}, -NR^{4c}R^{4d}, - NR^{4c}COR^{4d}, -NR^{4c}CO₂R^{4d}, or -NR^{4c}SO₂R^{4d};
R^{4a}, R^{4b}, R^{4c} and R^{4d} are each independently hydrogen, -C₁₋₈alkyl, -C₂₋₈alkenyl, -C₂₋₈alkynyl, C₃-C₈cycloalkyl, 3- to 8-membered heterocyclyl, -C₆-C₁₂aryl, or 5- to 12-membered heteroaryl;
R¹⁰, R¹¹ and R¹² are each independently selected from hydrogen, -F, -Cl, -Br, -I, methyl, ethyl, propyl, butyl, pentyl, hexyl, heptyl, octyl, methoxy, ethoxy, propoxy, butoxy, pentoxy, hexoxy, heptoxy, octoxy, cyclopropyl, cyclobutyl, cyclopentyl, cyclohexyl, cycloheptyl, cyclooctyl, -NH₂ and oxo, wherein each of methyl, ethyl, propyl, butyl, pentyl, hexyl, heptyl, octyl, methoxy, ethoxy, propoxy, butoxy, pentoxy, hexoxy, heptoxy, octoxy, cyclopropyl, cyclobutyl, cyclopentyl, cyclohexyl, cycloheptyl, or cyclooctyl is optionally substituted with at least one substituent R^{9c}; or
two R¹² together with the carbon atoms to which they are attached, form a 3, 4, 5, 6, 7 or 8-membered ring, said ring comprising 0, 1, 2 or 3 heteroatoms independently selected from nitrogen, oxygen and sulfur; said ring is optionally substituted with at least one substituent R^{9c}; wherein
R^{9c} is independently -F, -Cl, -Br, -I, hydroxy, methyl, ethyl, propyl, butyl, pentyl, hexyl, heptyl, octyl, methoxy, ethoxy, propoxy, butoxy, pentoxy, hexoxy, heptoxy, octoxy, cyclopropyl, cyclobutyl, cyclopentyl, cyclohexyl, cycloheptyl, cyclooctyl, -C₂₋₈alkenyl, -C₂₋₈alkynyl, 3- to 8-membered heterocyclyl, phenyl, 5- to 12-membered heteroaryl, NH₂, or - NHCH₃;
Z¹, Z², Z³ and Z⁴ are each independently -CR^{z};
R^{Z}, at each occurrence, is independently selected from hydrogen, halogen, -C₁₋₈alkyl, -C₂₋₈alkenyl, -C₂₋₈alkynyl, -NR^{Za}R^{Zb}, -OR^{Za}, -SR^{Za}, C₃-C₈cycloalkyl, 3- to 8-membered heterocyclyl, C₆-C₁₂aryl, 5- to 12-membered heteroaryl, and CN, wherein each of -C₁₋₈alkyl, -C₂₋₈alkenyl, -C₂₋₈alkynyl, C₃-C₈cycloalkyl, 3- to 8-membered heterocyclyl, C₆-C₁₂aryl, or 5- to 12-membered heteroaryl is optionally substituted with at least one R^{Zc};
R^{Za} and R^{Zb} is independently selected from hydrogen, methyl, ethyl, propyl, butyl, pentyl, hexyl, heptyl, octyl, -C₂₋₈alkenyl, -C₂₋₈alkynyl, cyclopropyl, cyclobutyl, cyclopentyl, cyclohexyl, cycloheptyl, cyclooctyl, 3- to 8-membered heterocyclyl, phenyl and 5- to 12-membered heteroaryl, wherein each of said hydrogen, methyl, ethyl, propyl, butyl, pentyl, hexyl, heptyl, octyl, -C₂₋₈alkenyl, -C₂₋₈alkynyl, cyclopropyl, cyclobutyl, cyclopentyl, cyclohexyl, cycloheptyl, cyclooctyl, 3- to 8-membered heterocyclyl, phenyl or 5- to 12-membered heteroaryl is optionally substituted with at least one substituent R^{Zd}; and
R^{Zc} and R^{Zd} are each independently -F, -Cl, -Br, -I, hydroxy, methyl, ethyl, propyl, butyl, pentyl, hexyl, heptyl, octyl, -C₁₋₈alkoxy, -C₂₋₈alkenyl, -C₂₋₈alkynyl, cyclopropyl, cyclobutyl, cyclopentyl, cyclohexyl, cycloheptyl, cyclooctyl, 3- to 8-membered heterocyclyl, phenyl, or 5-to 12-membered heteroaryl;
L¹ is selected from a single bond, -O-, -SO₂-, -C(O)-, -NR^{L1a}-, -C₃-C₈cycloalkylene-, *^{L1}-O-C₁₋₈alkylene-**^{L1}, *^{L1}-C₁₋₈alkylene-O-**^{L1}, *^{L1}-SO₂-C₁₋₈alkylene-**^{L1}, *^{L1}-C₁₋₈alkylene-SO₂-**^{L1}, *^{L1}-CO-C₁₋₈alkylene-**^{L1}, *^{L1}-C₁₋₈alkylene-CO-**^{L1}, *^{L1}-NR^{L1a}-C₁₋₈alkylene-**^{L1}, *^{L1}-C₁₋₈alkylene-NR^{L1a}-**^{L1}, *^{L1}-NRL^{1a}C(O)-**^{L1}, *^{L1}-C(O)NR^{L1a}-**^{L1}, -C₁₋₈alkylene-, -C₂₋₈alkenylene-, -C₂₋₈alkynylene-, -[O(CR^{L1a}R^{L1b})ₘ₄]ₘ₅-, 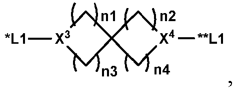 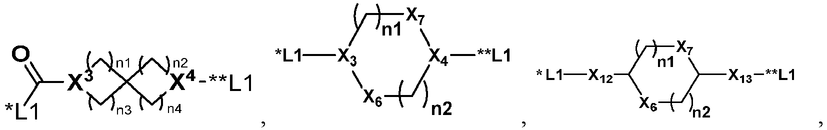 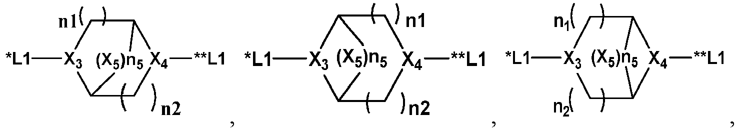 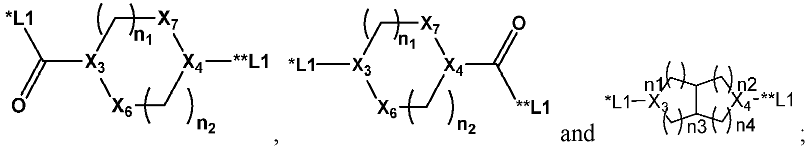
wherein each of said -C₃-C₈cycloalkylene-, *^{L1}-O-C₁₋₈alkylene-**^{L1}, *^{L1}-C₁₋₈alkylene-O-**^{L1}, *^{L1}-SO₂-C₁₋₈alkylene-**^{L1}, *^{L1}-C₁₋₈alkylene-SO₂-**^{L1}, *^{L1}-CO-C₁₋₈alkylene-**^{L1}, *^{L1}-C₁₋₈alkylene-CO-**^{L1}, *^{L1}-NR^{L1a}-C₁₋₈alkylene-**^{L1}, *^{L1}-C₁₋₈alkylene-NR^{L1a}-**^{L1}, -C₁₋₈alkylene-, - C₂₋₈alkenylene-, -C₂₋₈alkynylene-, 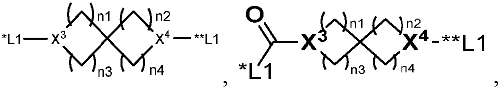 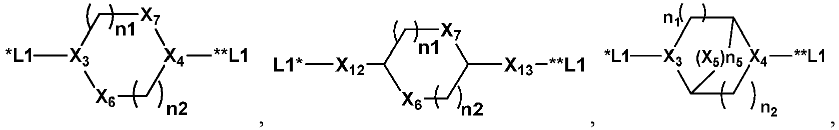 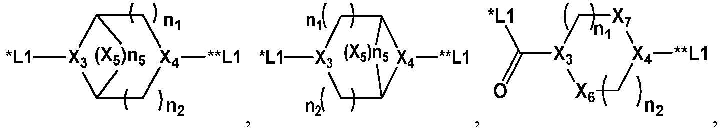 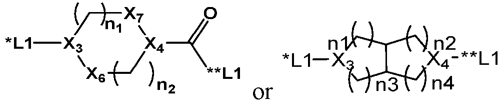 is optionally substituted with at least one R^{L1c};
wherein *^{L1} refers to the position attached to 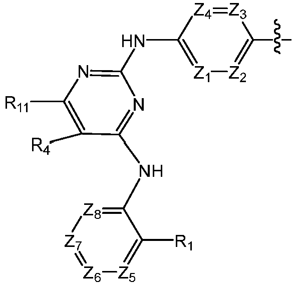 moiety, and **^{L1} refers to the position attached to the 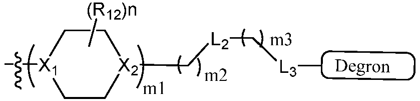 moiety;
R^{L1a} and R^{L1b} are each independently selected from hydrogen, -C₁₋₈alkyl, -C₂₋₈alkenyl, -C₂₋₈alkynyl, C₃-C₈cycloalkyl, 3- to 8-membered heterocyclyl, C₆-C₁₂aryl and 5- to 12-membered heteroaryl, wherein each of said -C₁₋₈alkyl, -C₂₋₈alkenyl, -C₂₋₈alkynyl, C₃-C₈cycloalkyl, 3- to 8-membered heterocyclyl, C₆-C₁₂aryl or 5- to 12-membered heteroaryl is optionally substituted with at least one substituent R^{L1d};
each of said R^{L1c} and R^{L1d} are independently halogen, hydroxy, -C₁₋₈alkyl, -C₁₋₈alkoxy, C₁₋₈alkoxy-C₁₋₈alkyl-, -C₂₋₈alkenyl, -C₂₋₈alkynyl, C₃-C₈cycloalkyl, 3- to 8-membered heterocyclyl, C₆-C₁₂aryl, 5- to 12-membered heteroaryl or oxo;
L² is selected from a single bond, -O-, -SO₂-, -CO-, -NR^{L2a}-, -C₃-C₈cycloalkylene-, *^{L2}-O-C₁₋ₛ₈lkylene-**^{L2}, *^{L2}-C₁₋₈alkylene-O-**^{L2}, *^{L2}-SO₂-C₁₋₈alkylene-**^{L2}, *^{L2}-C₁₋₈alkylene-SO₂-**L2, *^{L2}-CO-C₁₋₈alkylene-**^{L2}, *^{L2}-C₁₋₈alk_{y}lene-CO-**^{L2}, *^{L2}-NR^{L2a}-C₁₋₈alkylene-**^{L2}, *^{L2}-C₁₋₈alkylene-NR^{L2a}-**^{L2}, *^{L2}-NR^{L2a}C(O)-**^{L2}, *^{L2}-C(O)NR^{L2a}-**^{L2}, -C₁₋₈alkylene-, -C₂₋₈alkenylene-, -C₂₋₈alkynylene-, -[O(CR^{L2a}R^{L2b})ₘ₄]ₘ₅-, 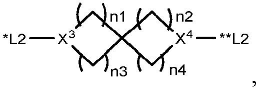 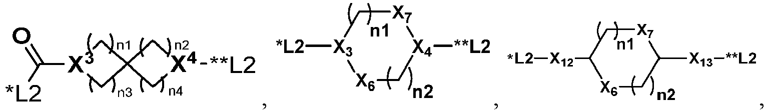 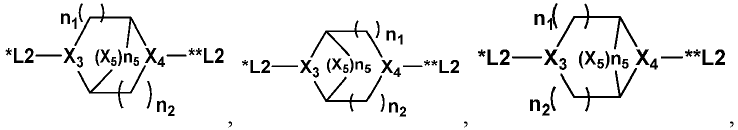 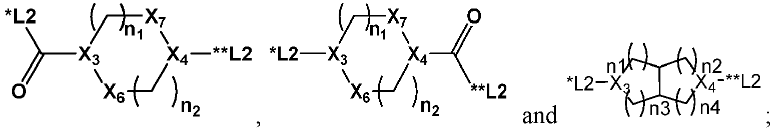
wherein each of said -C₃-C₈cycloalkylene-, *^{L2}-O-C₁₋₈alkylene-**^{L2}, *^{L2}-C₁₋₈alkylene-O-**L2, *^{L2}-SO₂-C₁₋₈alkylene-**^{L2}, *^{L2}-C₁₋₈alkylene-SO₂-**^{L2}, *^{L2}-CO-C₁₋₈alkylene-**^{L2}, *^{L2}-C₁₋₈alkylene-CO-**^{L2}, *^{L2}-NR^{L2a}-C₁₋₈alkylene-**^{L2}, *^{L2}-C₁₋₈alkylene-NR^{L2a}-**^{L2}, -C₁₋₈alkylene-, - C₂₋₈alkenylene-, -C₂₋₈alkynylene-, 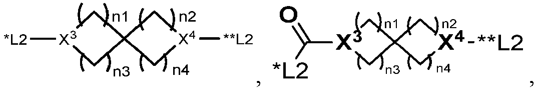 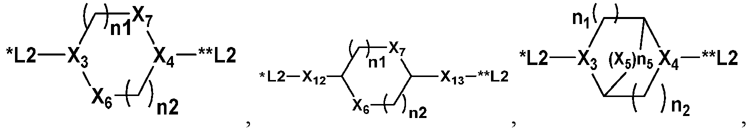 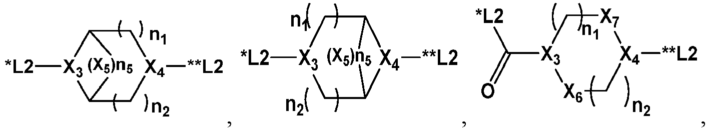 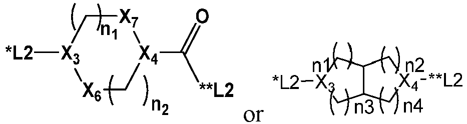 is optionally substituted with at least one substituent R^{L2c};
wherein *^{L2} refers to the position attached to 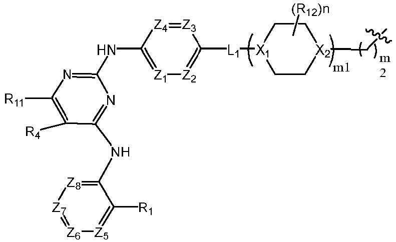 moiety, and **^{L2} refers to the position attached to the 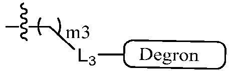 moiety;
R^{L2a} and R^{L2b} are each independently selected from hydrogen, -C₁₋₈alkyl, -C₂₋₈alkenyl, -C₂₋₈alkynyl, C₃-C₈cycloalkyl, 3- to 8-membered heterocyclyl, C₆-C₁₂aryl and 5- to 12-membered heteroaryl, wherein each of said -C₁₋₈alkyl, -C₂₋₈alkenyl, -C₂₋₈alkynyl, C₃-C₈cycloalkyl, 3- to 8-membered heterocyclyl, C₆-C₁₂aryl or 5- to 12-membered heteroaryl is optionally substituted with at least one substituent R^{L2d};
each of said R^{L2c} and R^{L2d} are independently halogen, hydroxy, -C₁₋₈alkyl, -C₁₋₈alkoxy, C₁₋₈alkoxy-C₁₋₈alkyl-, -C₂₋₈alkenyl, -C₂₋₈alkynyl, C₃-C₈cycloalkyl, 3- to 8-membered heterocyclyl, C₆-C₁₂aryl, 5- to 12-membered heteroaryl or oxo;
L³ is selected from a single bond, -O-, -SO₂-, -CO-, -NR^{L3a}-, -C₃-C₈cycloalkylene-, *^{L3}-O-C₁₋₈alkylene-**^{L3}, *^{L3}-C₁₋₈alkylene-O-**^{L3}, *^{L3}-SO₂-C₁₋₈alkylene-**^{L3}, *^{L3}-C₁₋₈alkylene-SO₂-**^{L3}, *^{L3}-CO-C₁₋₈alkylene-**^{L3}, *^{L3}-C₁₋₈alkylene-CO-**^{L3}, *^{L3}-NR^{L3a}-C₁₋₈alkylene-**^{L3}, *^{L3}-C₁₋₈alkylene-NR^{L3a}-**^{L3}, *^{L3}-W^{3a}C(O)-**^{L3}, *^{L3}-C(O)NR^{L3a}-**^{L3}, -C₁₋₈alkylene-, -C₂₋₈alkenylene-, -C₂₋₈alkynylene-, -[O(CR^{L3a}R^{L3b})ₘ₄]ₘ₅-, 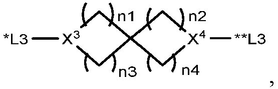 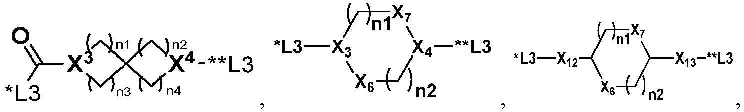 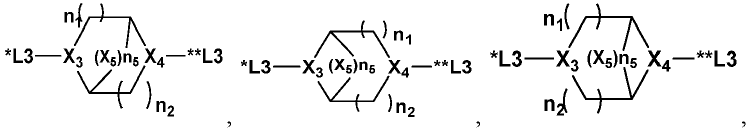 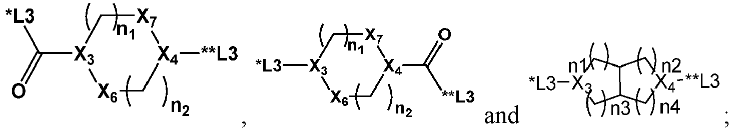
wherein each of said -C₃-C₈cycloalkylene-, *^{L3}-O-C₁₋₈alkylene-**^{L3}, *^{L3}-C₁₋₈alkylene-O-**^{L3}, *^{L3}-SO₂-C₁₋₈alkylene-**^{L3}, *^{L3}-C₁₋₈alkylene-SO₂-**^{L3}, *^{L3}-CO-C₁₋₈alkylene-**^{L3}, *^{L3}-C₁₋₈alkylene-CO-**^{L3}, *^{L3}-NR^{L3a}-C₁₋₈alkylene-**^{L3}, *^{L3}-C₁₋₈alkylene-NR^{L3a}-**^{L3}, -C₁₋₈alkylene-, - C₂₋₈alkenylene-, -C₂₋₈alkynylene-, 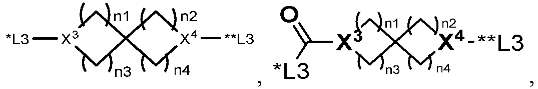 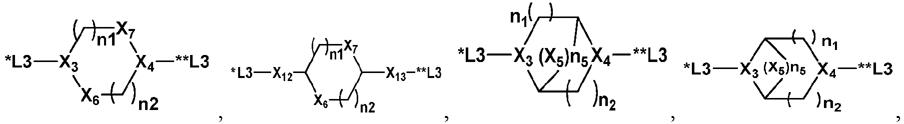 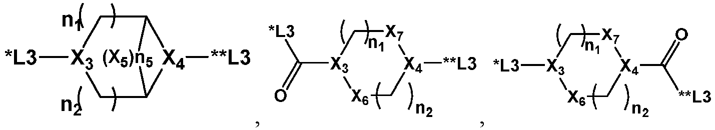 or 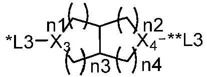 is optionally substituted with at least one substituent R^{L3c};
wherein *^{L3} refers to the position attached to 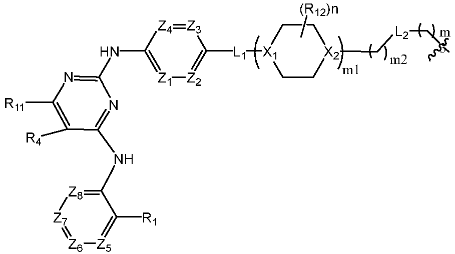 moiety, and **^{L3} refers to the position attached to the 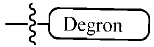 moiety;
R^{L3a} and R^{L3b} are each independently selected from hydrogen, -C₁₋₈alkyl, -C₂₋₈alkenyl, -C₂₋₈alkynyl, C₃-C₈cycloalkyl, 3- to 8-membered heterocyclyl, C₆-C₁₂aryl and 5- to 12-membered heteroaryl, wherein each of said -C₁₋₈alkyl, -C₂₋₈alkenyl, -C₂₋₈alkynyl, C₃-C₈cycloalkyl, 3- to 8-membered heterocyclyl, C₆-C₁₂aryl or 5- to 12-membered heteroaryl is optionally substituted with at least one substituent R^{L3d};
each of said R^{L3c} and R^{L3d} are independently halogen, hydroxy, -C₁₋₈alkyl, -C₁₋₈alkoxy, C₁₋₈alkoxy-C₁₋₈alkyl-, -C₂₋₈alkenyl, -C₂₋₈alkynyl, C₃-C₈cycloalkyl, 3- to 8-membered heterocyclyl, C₆-C₁₂aryl, 5- to 12-membered heteroaryl or oxo; 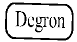 is selected from 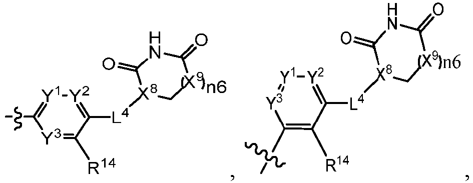 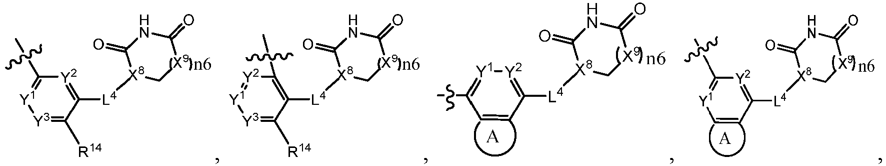 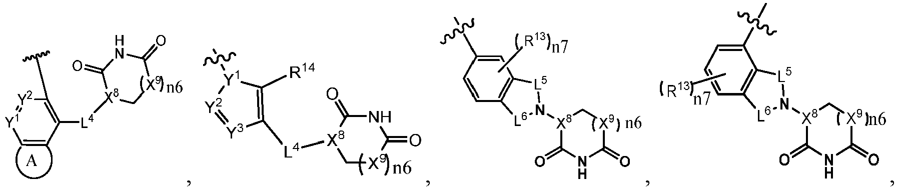 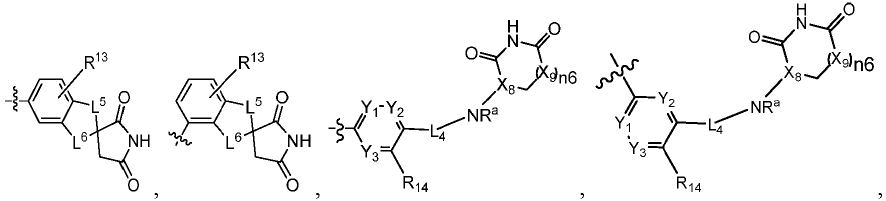 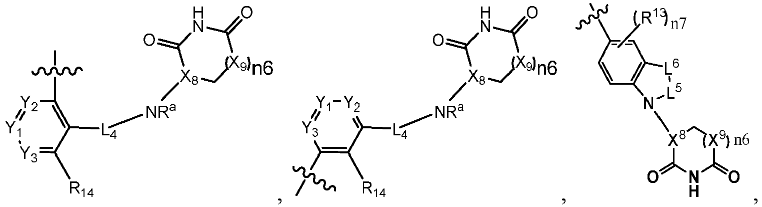 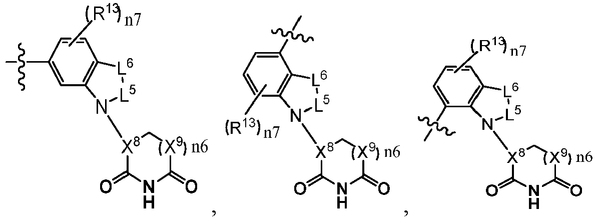 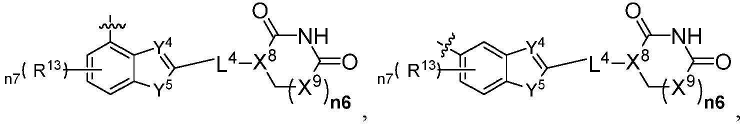 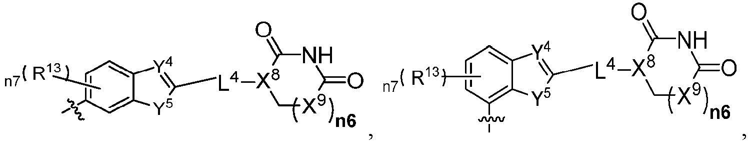 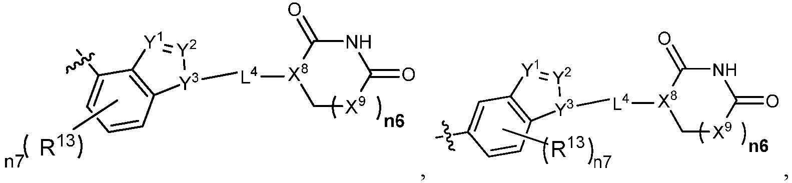 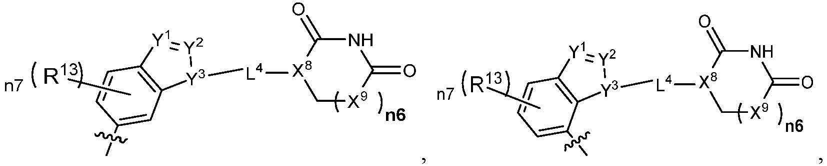 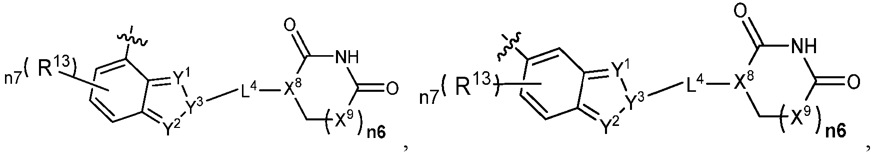 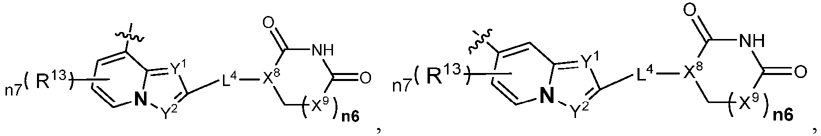 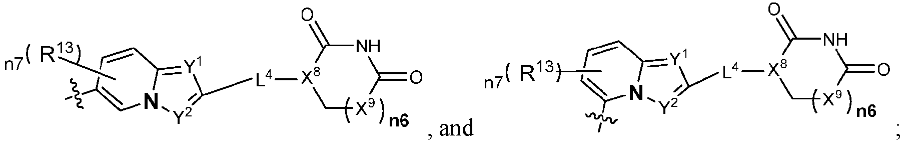
Ring A is selected from 3-12 membered cycloalkyl, 3-12 membered heterocyclyl, aryl, and heteroaryl;
R¹³ and R¹⁴ are independently selected from hydrogen, halogen, CN, -C₁₋₈alkyl, -C₂₋₈alkenyl, -C₂₋₈alkynyl, -C₁₋₈alkoxy, C₃-C₈cycloalkyl, 3- to 8-membered heterocyclyl, C₆-C₁₂aryl and 5- to 12-membered heteroaryl; said each -C₁₋₈alkyl, -C₂₋₈alkenyl, -C₂₋₈alkynyl, -C₁₋₈alkoxy, C₃-C₈cycloalkyl, 3- to 8-membered heterocyclyl, C₆-C₁₂aryl or 5- to 12-membered heteroaryl is optionally substituted with at least one substituent halogen, -C₁₋₈alkyl, C₁₋₈alkoxy-C₁₋₈alkyl-, -C₂₋₈alkenyl, -C₂₋₈alkynyl, C₃-C₈cycloalkyl, 3- to 8-membered heterocyclyl, C₆-C₁₂aryl or 5- to 12-membered heteroaryl;
X¹, X², X³, X⁴ and X⁸ are each independently selected from -CR^{a} and N;
X⁵, X⁶, X⁷ and X⁹ are each independently selected from -NR^{a}-, -O-, -S- and -CR^{a}R^{b}-;
X¹² and X¹³ are each independently selected from -NR^{a}- and -O-;
L⁴, L⁵ and L⁶ are each independently selected from a single bond, -O-, -NR^{a}-, -(CR^{a}R^{b})ₙ₈-, - O(CR^{a}R^{b})ₙ₈-, -NR^{a}(CR^{a}R^{b})ₙ₈- and -C(O)-;
Y¹, Y², Y³ and Y⁴ are each independently selected from CR^{a} and N;
Y⁵ is selected from NR^{a}, O and S;
R^{a} and R^{b} are each independently selected from hydrogen (H, D or T), halogen, CN, -C₁₋₈alkyl, -C₁₋₈alkoxy, -C₂₋₈alkenyl, -C₂₋₈alkynyl, -C₃-C₈cycloalkyl, 3- to 8-membered heterocyclyl, -C₆-C₁₂aryl and 5- to 12-membered heteroaryl, wherein each of said -C₁₋₈alkyl, -C₁₋₈alkoxy, -C₂₋₈alkenyl, -C₂₋₈alkynyl, -C₃-C₈cycloalkyl, 3- to 8-membered heterocyclyl, -C₆-C₁₂aryl or 5- to 12-membered heteroaryl is optionally substituted with at least one substituent halogen, hydroxy, halogen, -C₁₋₈alkyl, -C₁₋₈alkoxy, -C₂₋₈alkenyl, -C₂₋₈alkynyl, -C₃-C₈cycloalkyl, 3- to 8-membered heterocyclyl, -C₆-C₁₂aryl or 5- to 12-membered heteroaryl; or
R^{a} and R^{b} together with the carbon atoms to which they are attached, form a 3- to 12-membered ring, said ring comprising 0-3 heteroatoms independently selected from nitrogen, oxygen and sulfur; said ring is optionally substituted with at least one substituent halogen, hydroxy, -C₁₋₈alkyl, -C₂₋₈alkenyl, -C₂₋₈alkynyl, -C₁₋₈alkoxy, -C₂₋₈alkenyl, -C₂₋₈alkynyl, C₃₋C₈cycloalkyl, 3- to 8-membered heterocyclyl, C₆-C₁₂aryl or 5- to 12-membered heteroaryl;
m₁ is 0 or 1;
m₂ and m₃ are each independently 0, 1, 2, 3, 4, 5, 6, 7 or 8;
m₄ and m₅ are each independently 0, 1, 2 or 3;
n, n₁, n₂, n₃, n₄ and n₅ are each independently 0, 1, 2 or 3; and
n₆ is 0, 1, 2, 3 or 4;
n₇ is 0, 1, 2 or 3;
n₈ is 0, 1, 2, 3, 4, 5, 6, 7 or 8.

2. The compound of claim 1, wherein R¹ is -P(O)(CH₃)₂.

3. The compound of any one of claims 1-2, wherein:
R² and R³ together with the carbon atoms to which they are attached, form a 5 or 6 membered aromatic ring, said ring comprising 0, 1 or 2 heteroatoms independently selected from nitrogen, oxygen, and sulfur; said ring is optionally substituted with at least one substituent R^{2e};
R^{2e}, at each occurrence, is independently hydrogen, -F, -Cl, -Br, -I, methyl, ethyl, propyl, butyl, pentyl, hexyl, heptyl, octyl, methoxy, ethoxy, propoxy, butoxy, pentoxy, hexoxy, heptoxy, octoxy, cyclopropyl, cyclobutyl, cyclopentyl, cyclohexyl, cycloheptyl, cyclooctyl or oxo, wherein each of methyl, ethyl, propyl, butyl, pentyl, hexyl, heptyl, octyl, methoxy, ethoxy, propoxy, butoxy, pentoxy, hexoxy, heptoxy, octoxy, cyclopropyl, cyclobutyl, cyclopentyl, cyclohexyl, cycloheptyl or cyclooctyl is optionally substituted with at least one substituent R^{2d}; and
R^{2d}, at each occurrence, is independently -F, -Cl, -Br, -I, -OH, methyl, ethyl, propyl, butyl, pentyl, hexyl, heptyl, octyl, methoxy, ethoxy, propoxy, butoxy, pentoxy, hexoxy, heptoxy, octoxy, cyclopropyl, cyclobutyl, cyclopentyl, cyclohexyl, cycloheptyl, cyclooctyl or phenyl.

4. The compound of any one of claims 1-3, wherein the 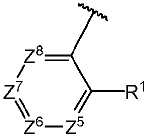 moiety is 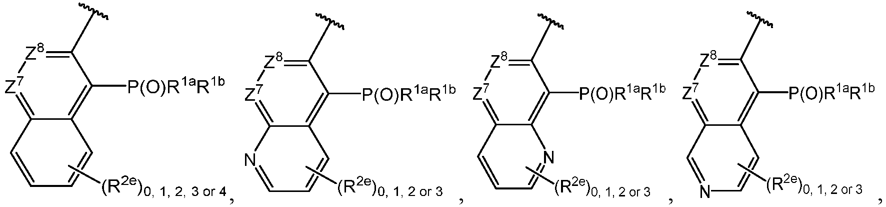 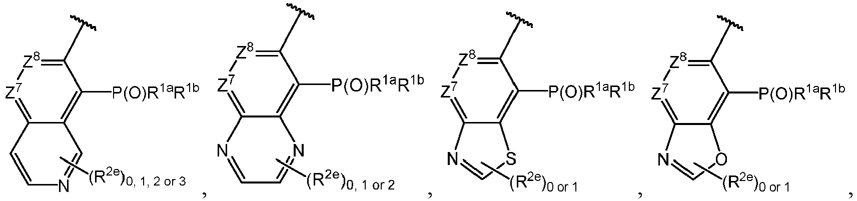 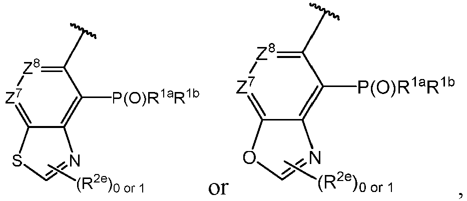 wherein R^{2e}, Z⁷, Z⁸, R^{1a} and R^{1b} are defined as in claim 1.

5. The compound of any one of claims 1--4, wherein:
(i) R^{2e} at each occurrence, is independently hydrogen, -F, -Cl, -Br, -I, -CH₃, -CH₂CH₃, - CH₂CH₂CH₃, -CH(CH₃)₂, -CH₂CH₂CH₂CH₃, -CH(CH₃)CH₂CH₃, -CH₂CH(CH₃)₂, -C(CH₃)₃, - CF₃, 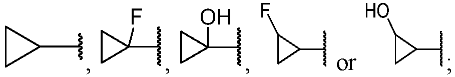 and/or
(ii) the 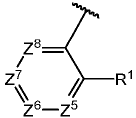 moiety is

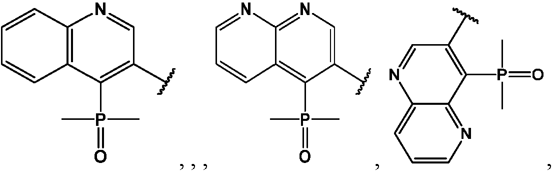

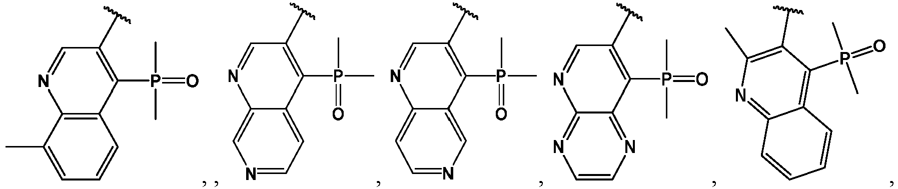

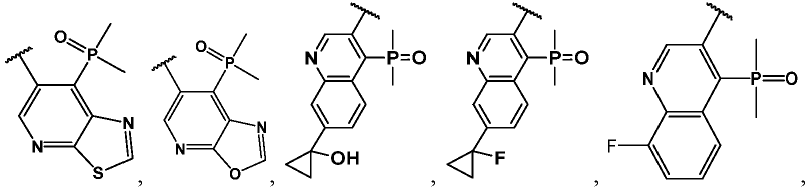

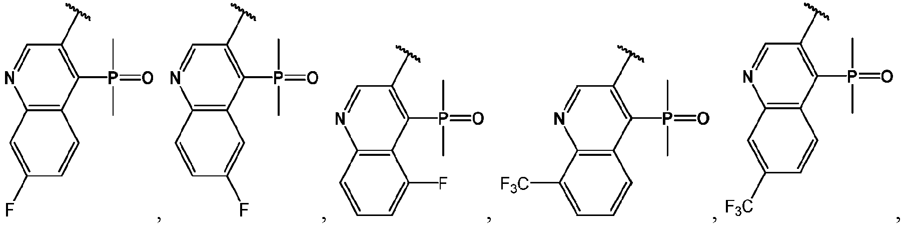

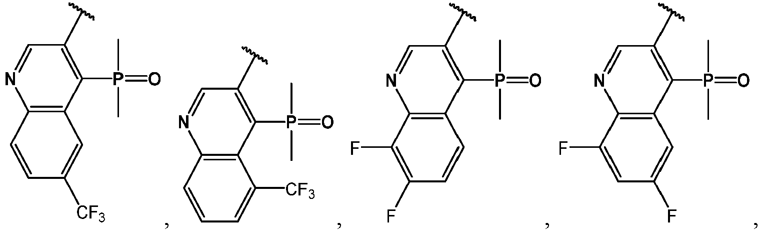

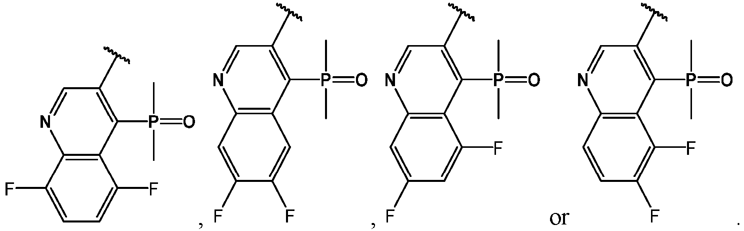

6. The compound of any one of claims 1-5, wherein:
R¹⁰, R¹¹ and R¹² are each independently selected from hydrogen, F, Cl, Br, -NH₂, -CH₃, - C₂H₅, -C₃H₇, -CH₂F, -CHF₂, -CF₃, -C₄H₉, -C₅H₁₁, -OCH₃, -OC₂H₅, -OC₃H₇, -OC₄H₉, -OC₅H₁₁, - CN, cyclopropyl and oxo; or
two R¹² together with the carbon atoms to which they are attached, form a 3, 4, 5, 6, 7 or 8-membered ring, said ring comprising 0, 1, 2 or 3 heteroatoms independently selected from nitrogen, oxygen and sulfur; said ring is optionally substituted with at least one substituent -H, - F, -Cl, -Br, -I, methyl, ethyl, propyl, butyl, -NH₂, -NHCH₃, -OH, -OCH₃, -OC₂H₅, cyclopropyl, cyclobutyl, cyclopentyl, or cyclohexyl.

7. The compound of any of claims 1-6, wherein R⁴ is selected from -H, -F, -Cl, -Br, -I, - CH₃, -C₂H₅, -C₃H₇, -C₄H₉, cyclopropyl, and cyclobutyl, wherein each of -CH₃, -C₂H₅, -C₃H₇, - C₄H₉, cyclopropyl, or cyclobutyl is optionally substituted with -F, -Cl, -Br, -I, -C₁₋₈alkyl, -C₂₋₈alkenyl, -C₂₋₈alkynyl, -C₃-C₈cycloalkyl, 3- to 8-membered heterocyclyl, C₆-C₁₂aryl, 5- to 12-membered heteroaryl, oxo, -CN, -OR^{4c}, -SO₂R^{4c}, -SO₂NR^{4c}R^{4d}, -COR^{4c}, -CO₂R^{4c}, -CONR^{4c}R^{4d}, -NR^{4c}R^{4d}, -NR^{4c}COR^{4d}, -NR^{4c}CO₂R^{4d}, or -NR^{4c}SO₂R^{4d};
R^{4c} and R^{4d} are each independently hydrogen, -C₁₋₈alkyl, -C₂₋₈alkenyl, -C₂₋₈alkynyl, C₃-C₈cycloalkyl, 3- to 8-membered heterocyclyl, C₆-C₁₂aryl, or 5- to 12-membered heteroaryl.

8. The compound of any one of claims 1-7, wherein:
(i) L¹ is selected from a single bond, -O-, -SO₂-, -C(O)-, -NR^{L1a}-, -C₃-C₈cycloalkylene-, *^{L1}-O-C₁₋₈alkylene-**^{L1}, *^{L1}-C₁₋₈alkylene-O-**^{L1}, *^{L1}-SO₂-C₁₋₈alkylene-**^{L1}, *^{L1}-C₁₋₈alkylene-SO₂-**^{L1}, *^{L1}-CO-C₁₋₈alkylene-**^{L1}, *^{L1}-C₁₋₈alkylene-CO-**^{L1}, *^{L1}-NR^{L1a}-C₁₋₈alkylene-**^{L1}, *^{L1}-C₁₋₈alkylene-NR^{L1a}-**^{L1}, *L1-NR^{L1a}C(O)-**^{L1}, *^{L1}-C(O)NR^{L1a}-**^{L1}, -C₁₋₈alkylene-, -C₂₋₈alkenylene-, -C₂₋₈alkynylene-, -[O(CR^{L1a}R^{L1b})ₘ₄]ₘ₅-, 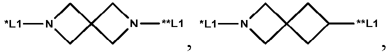 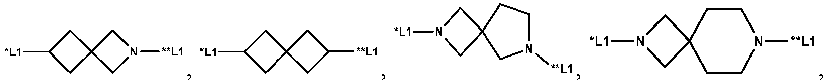 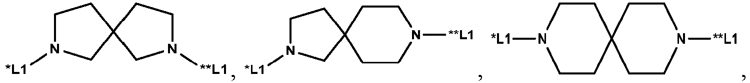 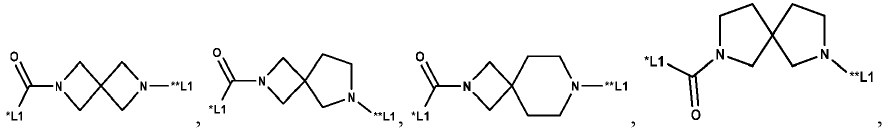 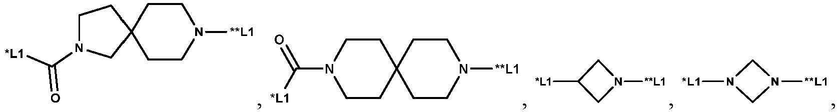 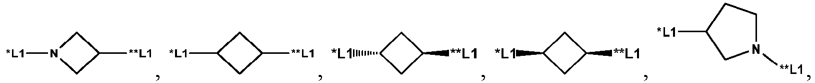 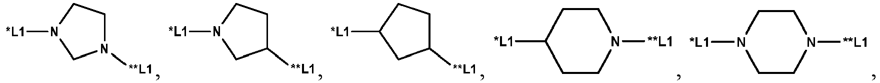 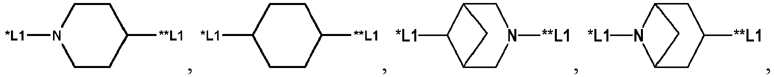 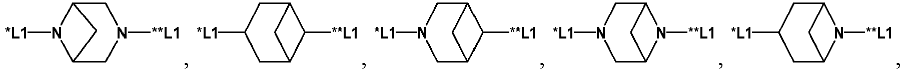 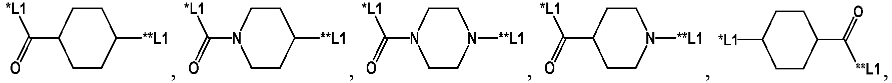 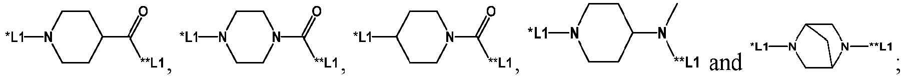
wherein each of said -C₃-C₈cycloalkylene-, *^{L1}-O-C₁₋₈alkylene-**^{L1}, *^{L1}-C₁₋₈alkylene-O-**^{L1}, *^{L1}-SO₂-C₁₋₈alkylene-**^{L1}, *^{L1}-C₁₋₈alkylene-SO₂-**^{L1}, *^{L1}-CO-C₁₋₈alkylene-**^{L1}, *^{L1}-C₁₋₈alkylene-CO-**^{L1}, *^{L1}-NR^{1a}-C₁₋₈alkylene-**^{L1}, *^{L1}-C₁₋₈alkylene-NR^{L1a}-**^{L1}, -C₁₋₈alkylene-, - C₂₋₈alkenylene-, -C₂₋₈alkynylene-, 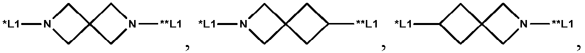 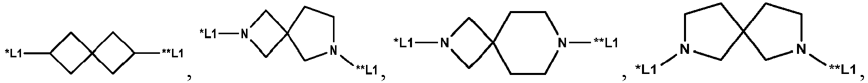 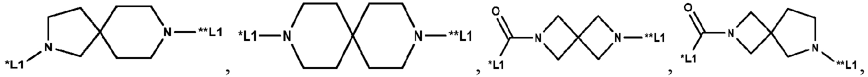 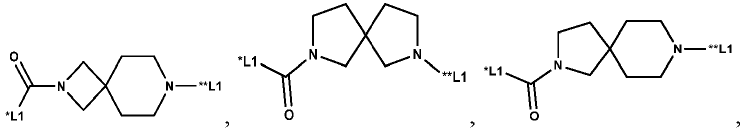 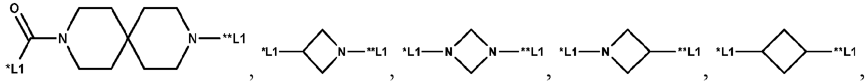 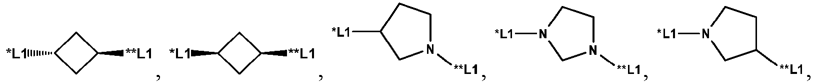 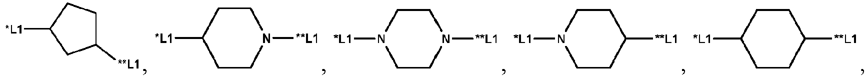 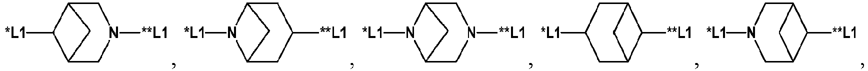 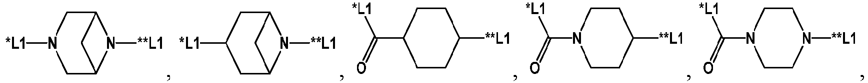 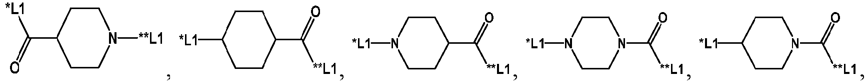 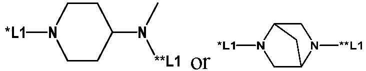 is optionally substituted with at least one R^{L1c};
R^{L1a} and R^{L1b} are each independently selected from hydrogen, methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl and pyrazinyl, wherein each of said methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl or pyrazinyl is optionally substituted with at least one substituent R^{L1d}; and
each of said R^{L1c} and R^{L1d} are independently -F, -Cl, -Br, -I, -OH, methyl, ethyl, propyl, butyl, pentyl, hexyl, heptyl, octyl, vinyl, ethynyl, methoxy, ethoxy, propoxy, butoxy, pentoxy, hexoxy, heptoxy, octoxy, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl, pyrazinyl or oxo;
or
(ii) L¹ is selected from a single bond,-C₁₋₈alkylene- (preferably -CH₂-, -C₂H₄-, -C₃H₆-), - CO-, -O-, -N(CH₃)-, -NH-,

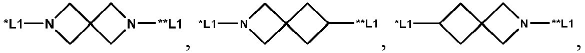

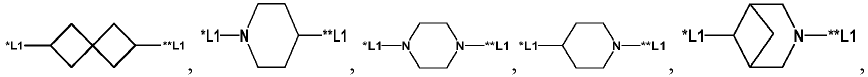

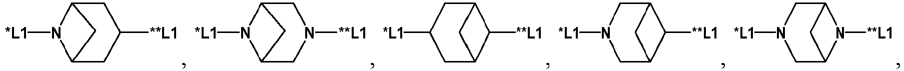

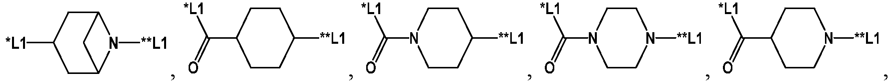

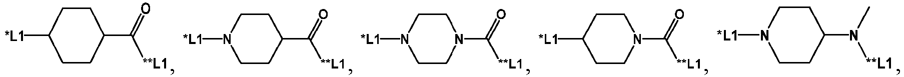

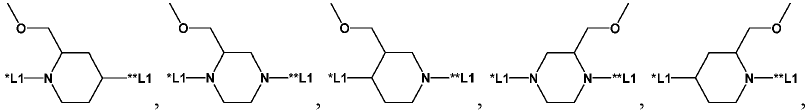

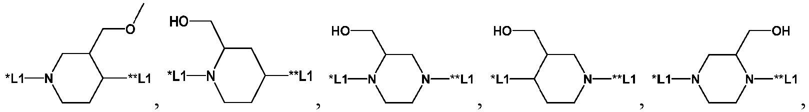

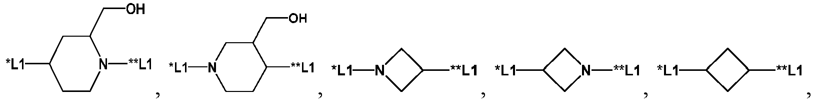

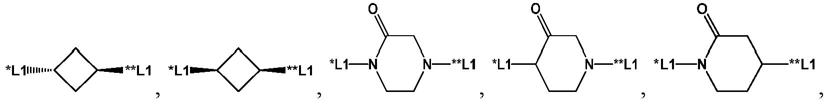

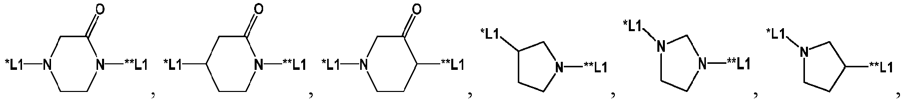

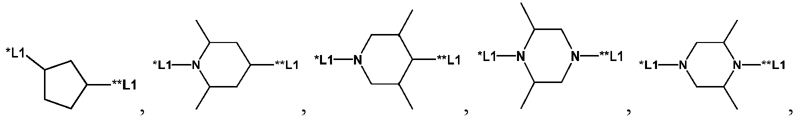

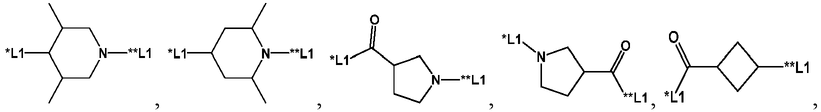

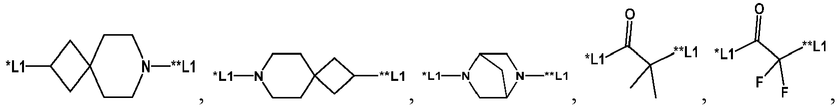

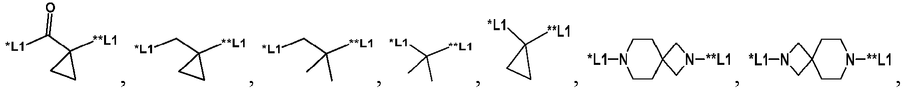

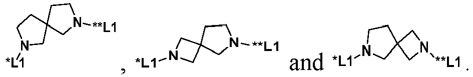

9. The compound of any one of claims 1-8, wherein:
(i) X¹ and X² are each independently selected from -CR^{a} and N;
R^{a} is selected from hydrogen, -F, -Cl, -Br, -I, CN, methyl, ethyl, methoxy, ethoxy, and cyclopropyl, wherein each of said methyl, ethyl, methoxy, ethoxy, or cyclopropyl is optionally substituted with at least one substituent -F, -Cl, -Br, -I, hydroxy, methyl, ethyl (preferably, X¹ and X² are each independently selected from CH, C(F), C(CH₃) or N);
m₁ is 1 or 0; and
R¹² is hydrogen, oxo, methoxymethyl, hydroxymethyl, -CN or -CH₃;
and/or
(ii) m1 is 1; preferably, the 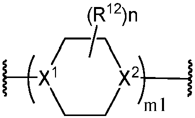 moiety is 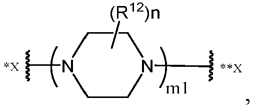 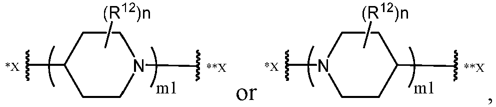 wherein *^{X} refers to the position attached to 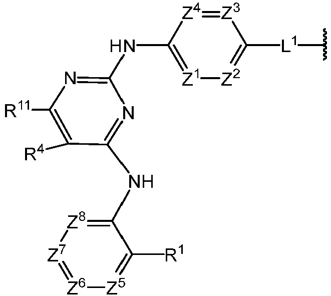 moiety, and **^{X} refers to the position attached to the 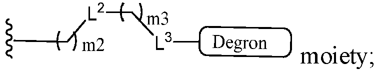 and/or
(iii) m₁ is 0.

10. The compound of any one of claims 1-9, wherein:
(i) the 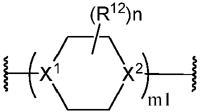 moiety is 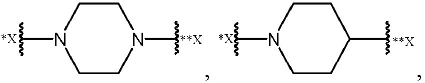 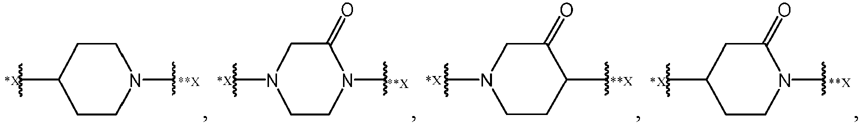 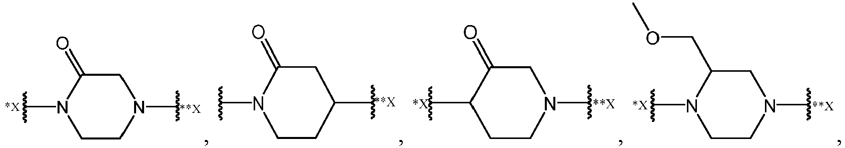 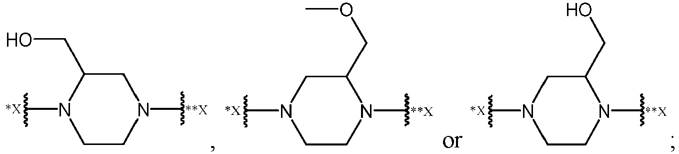 or
(ii) m₂ is selected from 0, 1, 2, 3, 4 and 5;
or
(iii) L² is selected from a single bond, -O-, -SO₂-, -CO-, -NR^{L2a}-, -C₃-C₈cycloalkylene-, *^{L2}-O-C₁₋₈alkylene-**^{L2}, *^{L2}-C₁₋₈alkylene-O-**^{L2}, *^{L2}-SO₂-C₁₋₈alkylene-**^{L2}, *^{L2}-C₁₋₈alkylene-SO₂-**^{L2}, *^{L2}-CO-C₁₋₈alkylene-**^{L2}, *^{L2}-C₁₋₈alkylene-CO-**^{L2}, *^{L2}-NR^{L2a}-C₁₋₈alkylene-**^{L2}, *^{L2}-C₁₋₈alkylene-NR^{L2a}-**^{L2}, *^{L2}-NR^{L2a}C(O)-**^{L2}, *^{L2}-C(O)NR^{L2a}-**^{L2}, -C₁₋₈alkylene-, -C₂₋₈alkenylene-, -C₂₋₈alkynylene-, -[O(CR^{L2a}R^{L2b})ₘ₄]ₘ₅-, 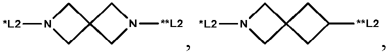 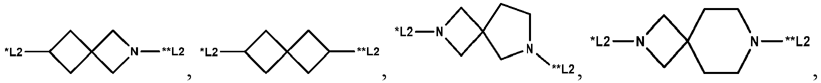 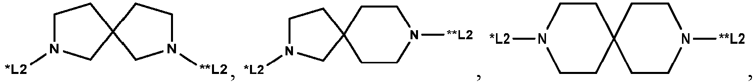 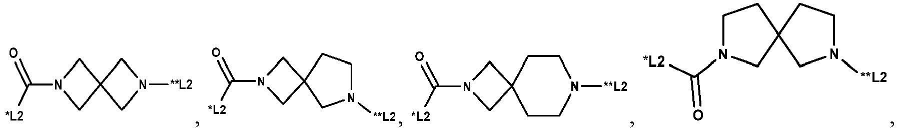 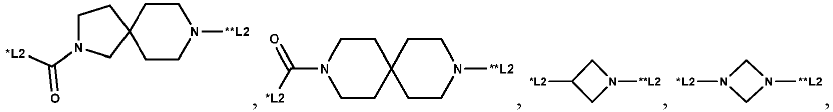 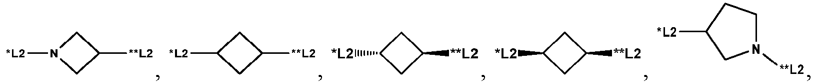 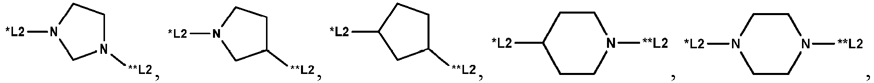 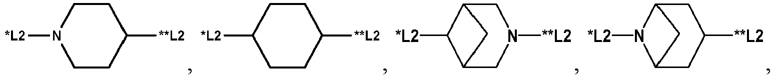 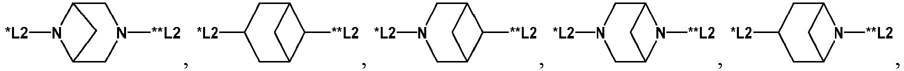 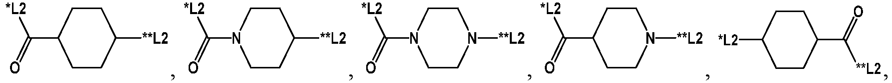 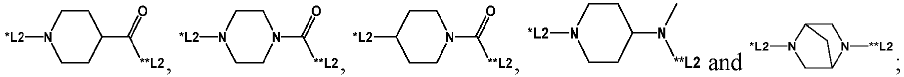
wherein each of said -C₃-C₈cycloalkylene-, *^{L2}-O-C₁₋₈alkylene-**^{L2}, *^{L2}-C₁₋₈alkylene-O-**L2, *^{L2}-SO₂-C₁₋₈alkylene-**^{L2}, *^{L2}-C₁₋₈alkylene-SO₂-**^{L2}, *^{L2}-CO-C₁₋₈alkylene-**^{L2}, *^{L2}-C₁₋₈alkylene-CO-**^{L2}, *^{L2}-NR^{L2a}-C₁₋₈alkylene-**^{L2}, *^{L2}-C₁₋₈alkylene-NR^{L2a}-**^{L2}, -C₁₋₈alkylene-, - C₂₋₈alkenylene-, -C₂₋₈alkynylene-, 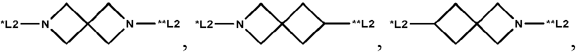 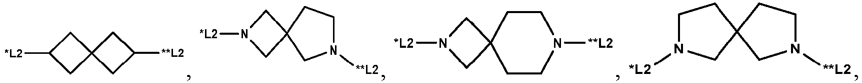 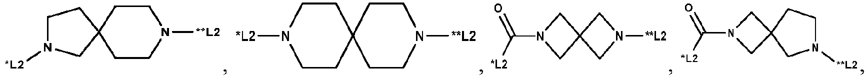 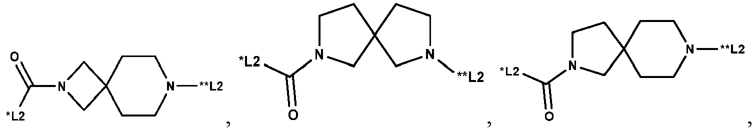 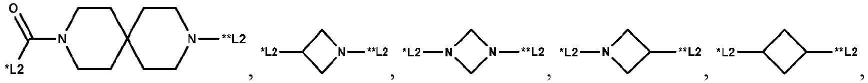 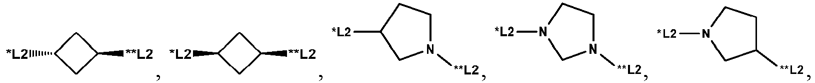 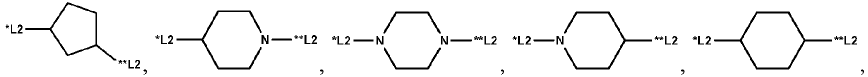 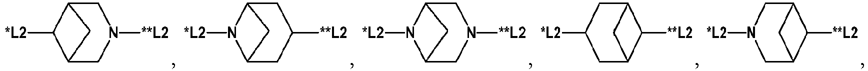 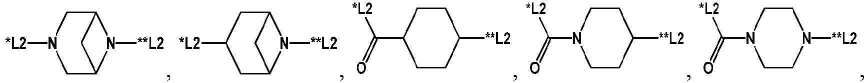 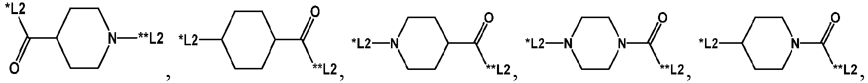 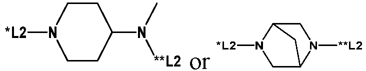 is optionally substituted with at least one substituent R^{L2c};
R^{L2a} and R^{L2b} are each independently selected from hydrogen, methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl and pyrazinyl, wherein each of said methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl or pyrazinyl is optionally substituted with at least one substituent R^{L2d}; and
each of said R^{L2c} and R^{L2d} are independently -F, -Cl, -Br, -I, -OH, methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl, pyrazinyl or oxo.

11. The compound of any one of claims 1-10, wherein:
(i) L² is selected from a single bond,-C₁₋₈alkylene-(preferably -CH₂-, -C₂H₄-, -C₃H₆-), -CO-, -O-, -N(CH₃)-, -NH-, 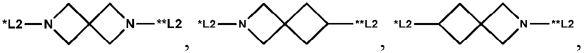 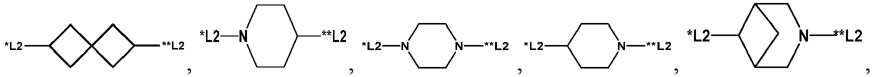 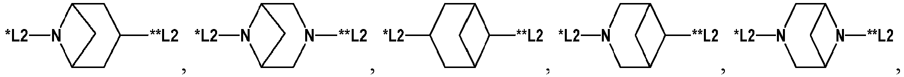 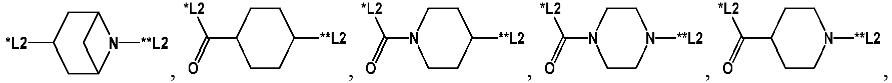 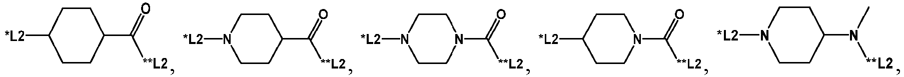 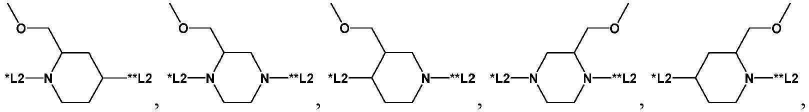 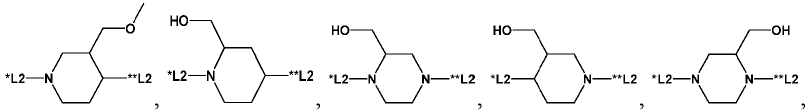 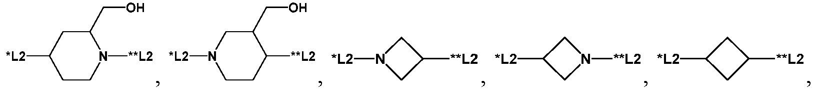 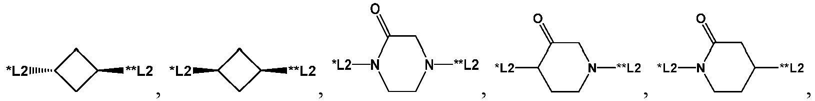 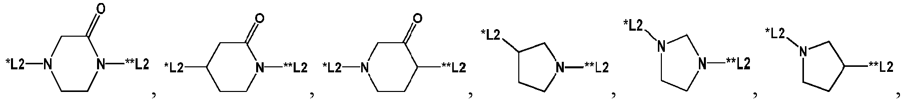 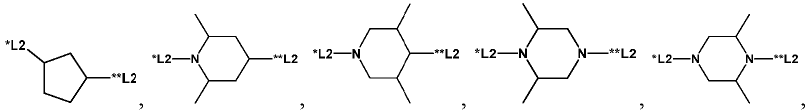 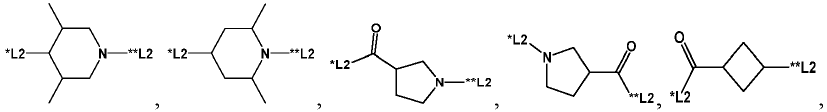 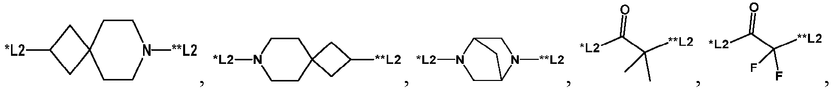 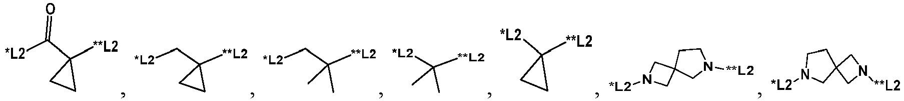 and 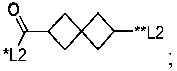 and/or
(ii) m₃ is 0, 1, 2, 3, 4, 5 or 6;
and/or
(iii) L³ is selected from a single bond, -O-, -SO₂-, -CO-, -NR^{L3a}-, -C₃-C₈cycloalkylene-, *^{L3}-O-C₁₋₈alkylene-**^{L3}, *^{L3}-C₁₋₈alkylene-O-**^{L3}, *^{L3}-SO₂-C₁₋₈alkylene-**^{L3}, *^{L3}-C₁₋₈alkylene-SO₂-**^{L3}, *^{L3}-CO-C₁₋₈alkylene-**^{L3}, *^{L3}-C₁₋₈alkylene-CO-**^{L3}, *^{L3}-NR^{L3a}-C₁₋₈alkylene-**^{L3}, *^{L3}-C₁₋₈alkylene-NR^{L3a}-**^{L3}, *^{L3}-NR^{L3a}C(O)-**^{L3}, *L3-C(O)NR^{L3a}-**^{L3}, -C₁₋₈alkylene-, -C₂₋₈alkenylene-, -C₂₋₈alkynylene-, -[O(CR^{L3a}R^{L3b})ₘ₄]ₘ₅-, 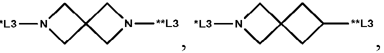 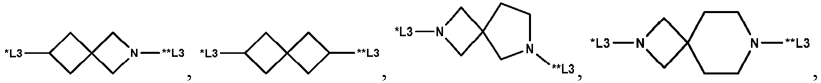 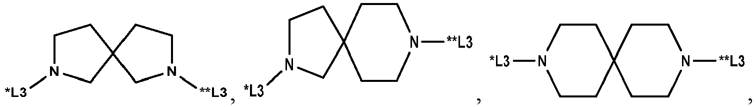 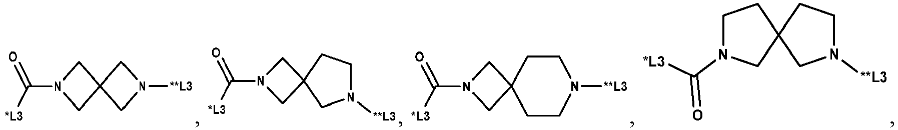 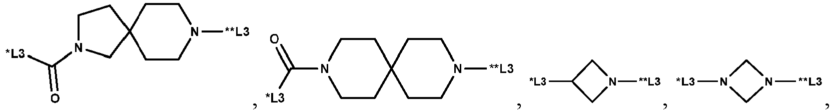 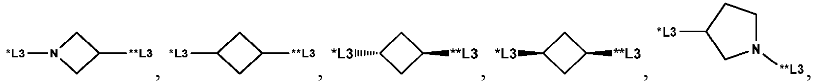 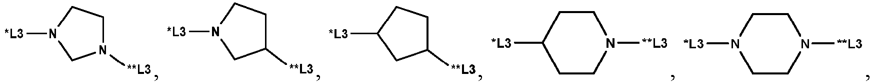 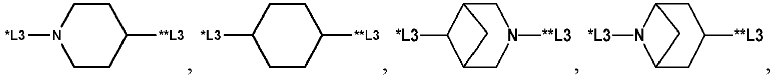 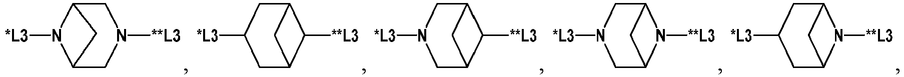 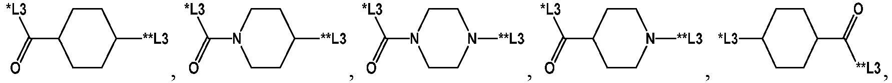 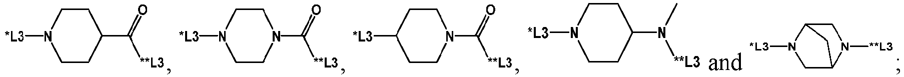
wherein each of said -C₃-C₈cycloalkylene-, *^{L3}-O-C₁₋₈alkylene-**^{L3}, *^{L3}-C₁₋₈alkylene-O-**^{L3}, *^{L3}-SO₂-C₁₋₈alkylene-**^{L3}, *^{L3}-C₁₋₈alkylene-SO₂-**^{L3}, *^{L3}-CO-C₁₋₈alkylene-**^{L3}, *^{L3}-C₁₋₈alkylene-CO-**^{L3}, *^{L3}-NR^{L3a}-C₁₋₈alkylene-**^{L3}, *^{L3}-C₁₋₈alkylene-NR^{L3a}-**^{L3}, -C₁₋₈alkylene-, - C₂₋₈alkenylene-, -C₂₋₈alkynylene-, 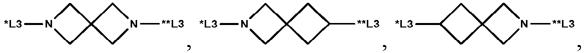 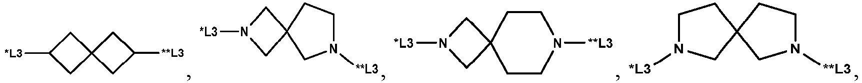 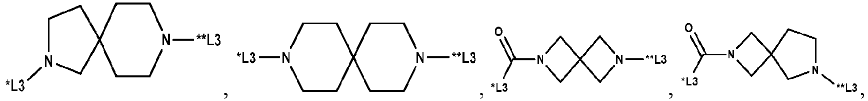 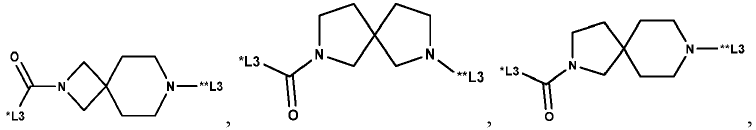 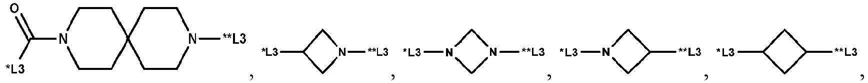 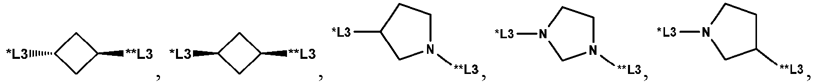 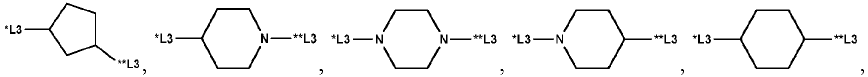 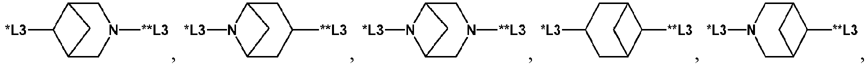 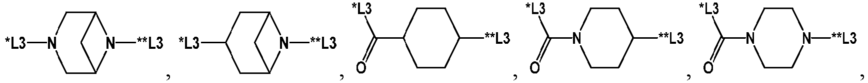 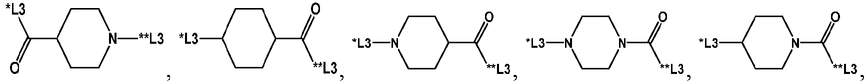 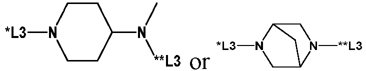 is optionally substituted with at least one substituent R^{L3c};
R^{L3a} and R^{L3b} are each independently selected from hydrogen, methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl and pyrazinyl, wherein each of said methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl or pyrazinyl is optionally substituted with at least one substituent R^{L3d}; and
each of said R^{L3c} and R^{L3d} are independently -F, -Cl, -Br, -I, -OH, methyl, ethyl, propyl, butyl, pentyl, hexyl, heptyl, octyl, vinyl, ethynyl, cyclopropyl, cyclobutyl, cyclopentyl, cyclohexyl, cycloheptyl, cyclooctyl, oxazolidinyl, imidazolidinyl, thiazolidinyl, pyrazolidinyl, morpholinyl, piperidinyl, piperazinyl, oxazinyl, imidazolyl, thiazolyl, oxazolyl, pyridyl, pyrimidinyl, pyrazinyl, pyridazinyl, phenyl, pyrrolyl, imidazolyl, pyrazolyl, thiazolyl, oxazolyl, triazolyl, thiophenyl, furanyl, pyridyl, pyrimidinyl, pyrazinyl or oxo;
and/or
(iv) L³ is selected from single bond,-C₁₋₈alkylene-(preferably -CH₂-, -C₂H₄-, -C₃H₆-), -CO-, -O-, -N(CH₃)-, -NH-, 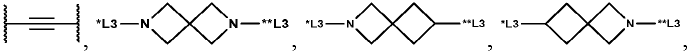 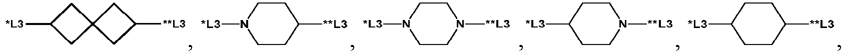 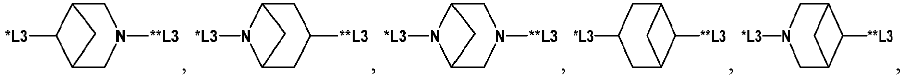 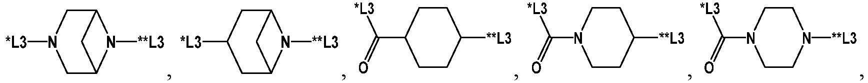 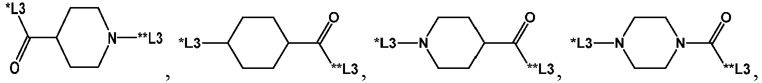 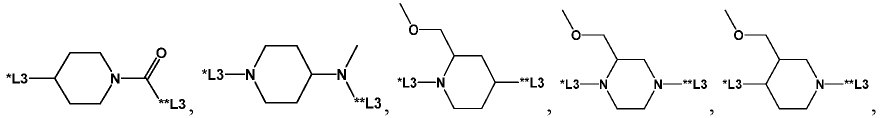 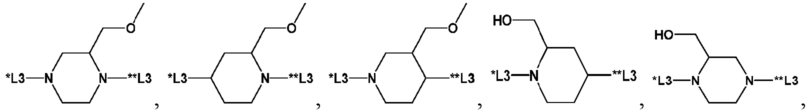 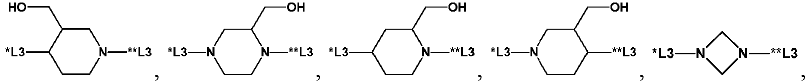 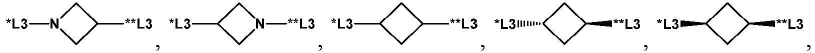 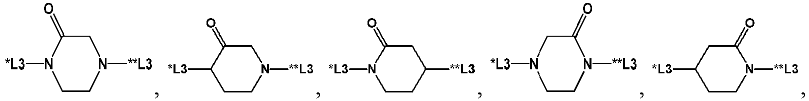 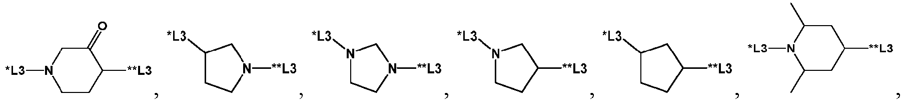 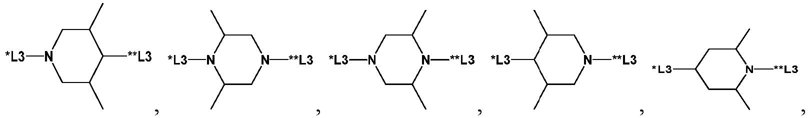 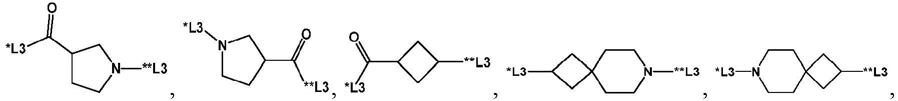 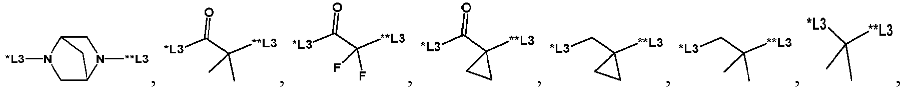 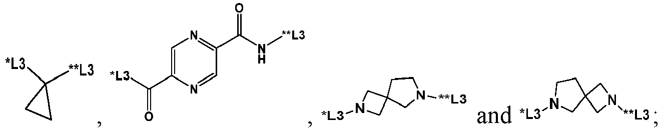 and/or
(v) 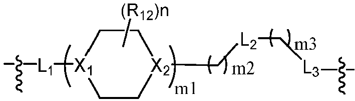 is selected from -CH₂CH₂-, 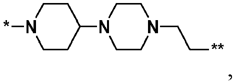 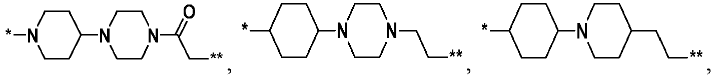 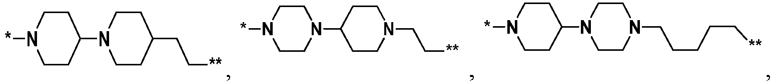 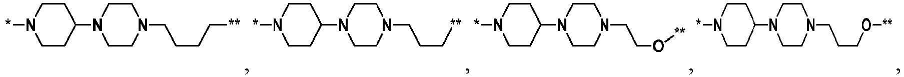 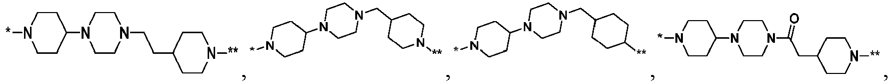 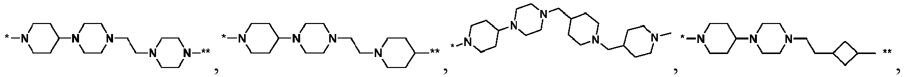 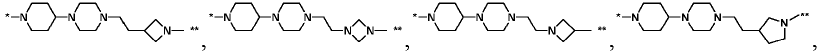 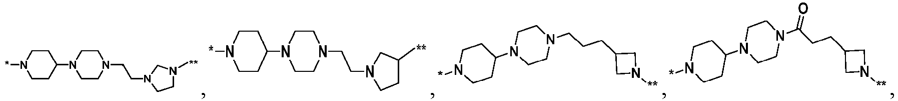 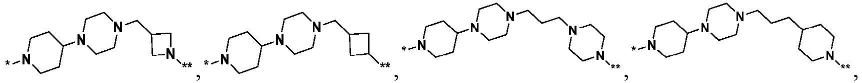 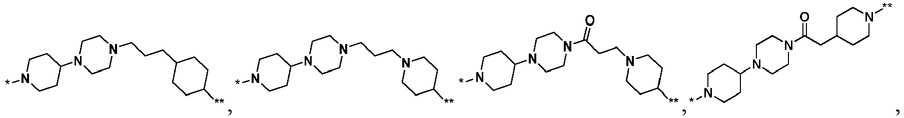 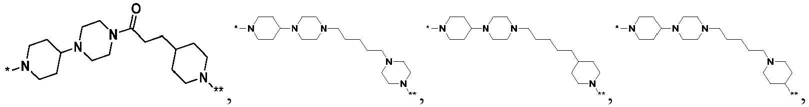 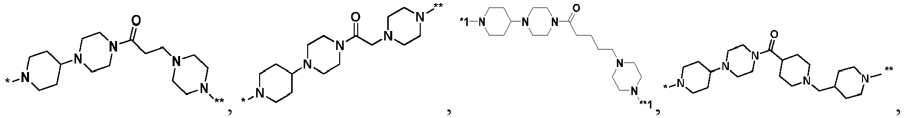 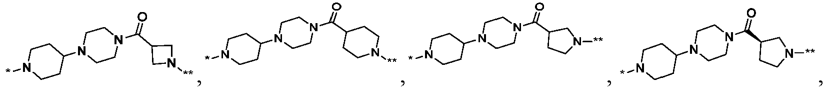 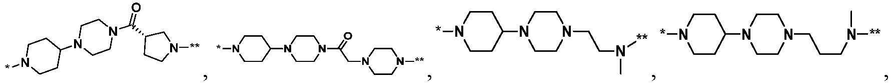 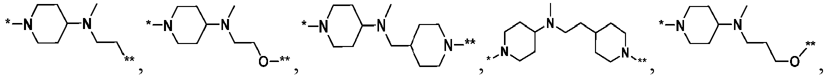 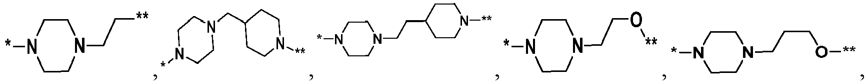 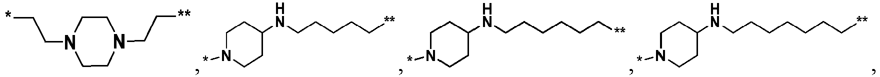 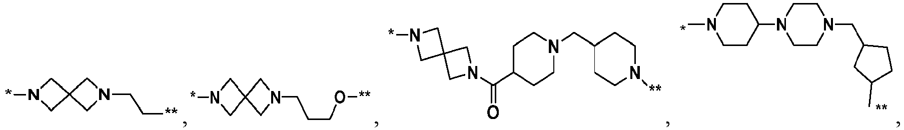 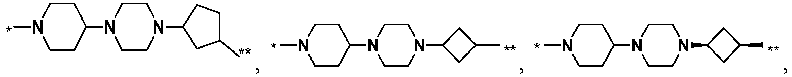 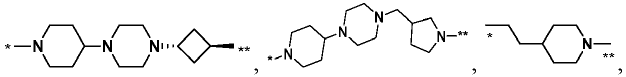 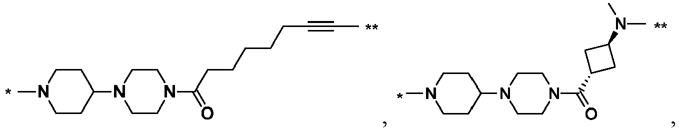 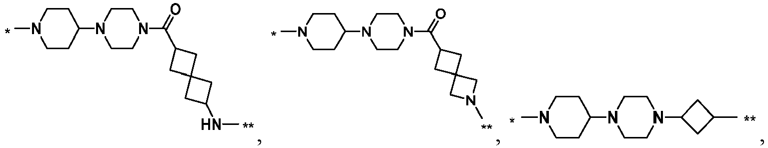 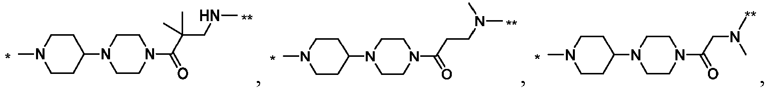 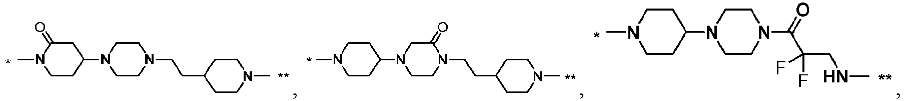 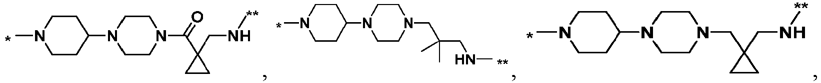 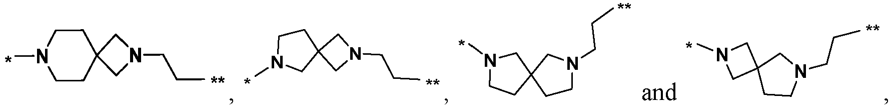 wherein * refers to the position attached to 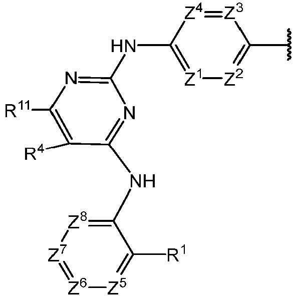 moiety, and ** refers to the position attached to the 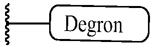 moiety.

12. The compound of any one of claims 1-11, wherein 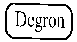 is selected from:
(i) 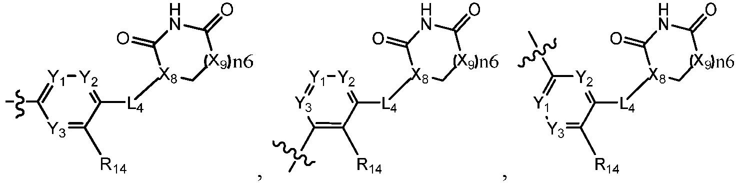 or 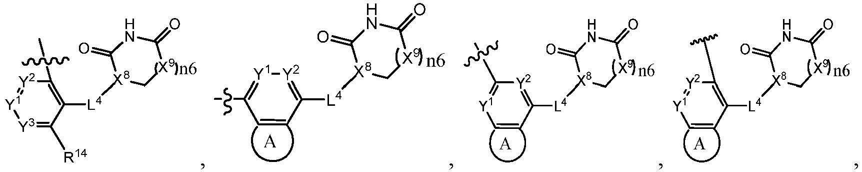 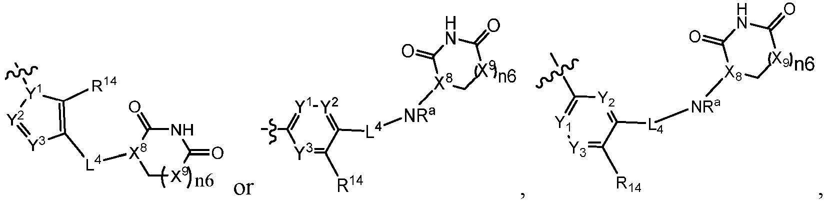 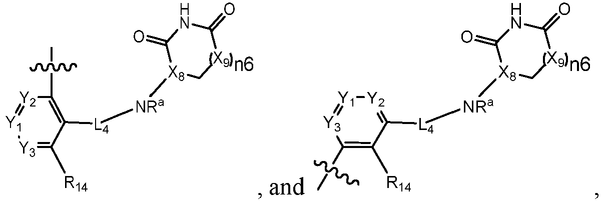
wherein Ring A is selected from 5- to 7-membered cycloalkyl, 5- to 7-membered heterocyclyl, aryl, and heteroaryl;
R¹⁴ is independently selected from hydrogen, halogen, -C₁₋₈alkyl, -C₁₋₈alkoxy, and CN; said each -C₁₋₈alkyl, or -C₁₋₈alkoxy is optionally substituted by one or more halogen or -C₁₋₈alkyl; preferably R¹⁴ is independently selected from H, F, Cl, Br, I, CH₃, -OCH₃, CH₂F, CN, CHF₂, and CF₃;
X⁸ is independently selected from CH, CD, C(CH₃), C(C₂H₅), C(C₃H₇), C(CN) and N;
L⁴ is independently selected from a single bond, 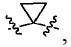 -O-, -NH-, -CH₂-, -CHF-, and - CF₂-;
Y¹, Y², and Y³ are each independently selected from CR^{a} and N;
X⁹ is CH₂;
R^{a} is each independently selected from hydrogen, halogen, -C₁₋₈alkyl, and -C₁₋₈alkoxy, wherein each of said -C₁₋₈alkyl or -C₁₋₈alkoxy is optionally substituted with at least one or more halogen, hydroxy, halogen, -C₁₋₈alkyl, or -C₁₋₈alkoxy; and
n₆ is independently 0,1 or 2;
or
(ii) 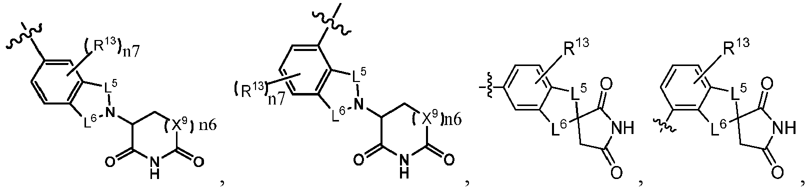 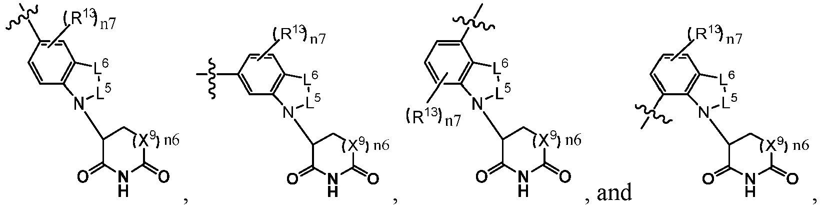
wherein L⁵ and L⁶ is independently selected from a single bond, 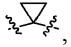 -O-, -NH-, - NMe-, -N(CH₂CH₃)-, -CH₂-, -CHF-, -CF₂-, -C(CH₃)₂- and -CO- (preferably L⁵ is -CO- or - CH₂-, and L⁶ is 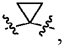 -O-, -NH-, -NMe-, -N(CH₂CH₃)-, -CH₂-, -CHF-, -CF₂-, -C(CH₃)₂- or - CO-);
X⁹ is CH₂;
each R¹³ is independently selected from hydrogen, -F, -Cl, -Br, -I, CN, -C₁₋₈alkyl, and - C₁₋₈alkoxy;
n₆ is 0 or 1; and
n₇ is 0, 1 or 2;
or
(iv) 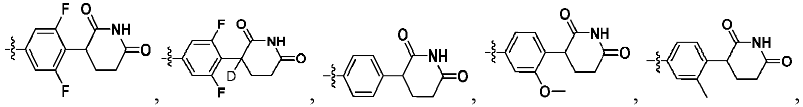 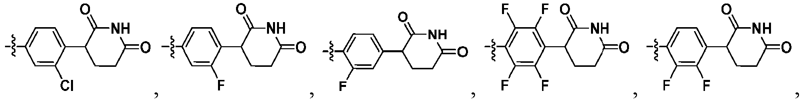 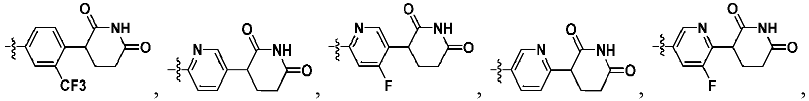 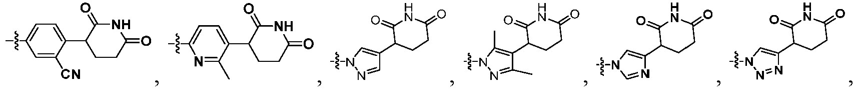 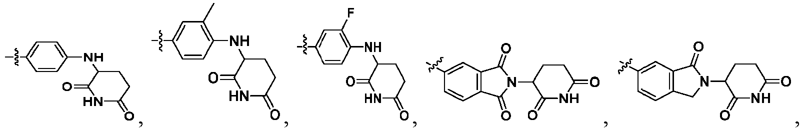 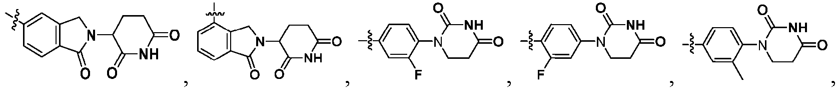 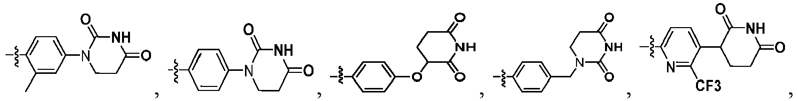 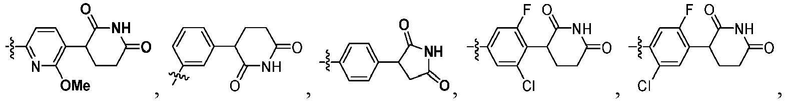 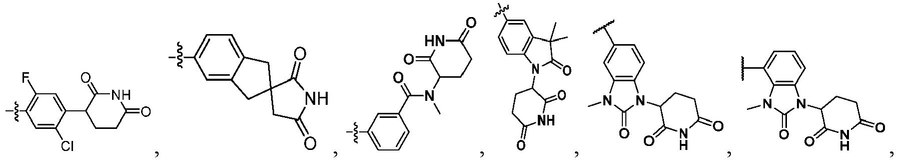 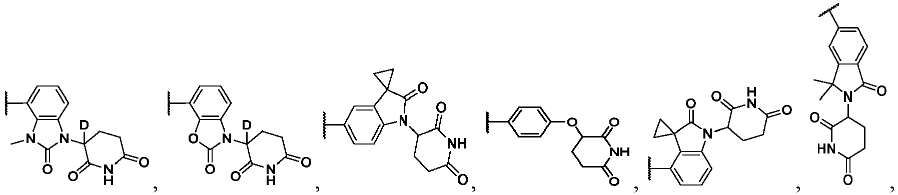 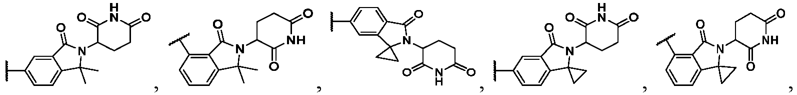 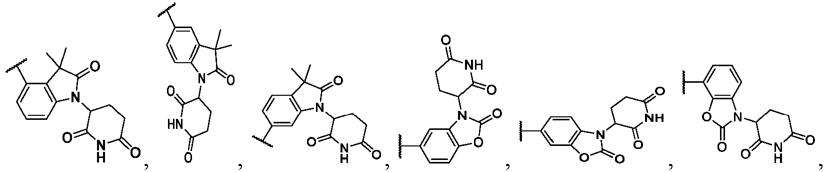 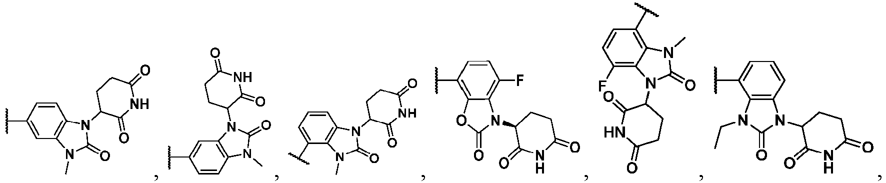 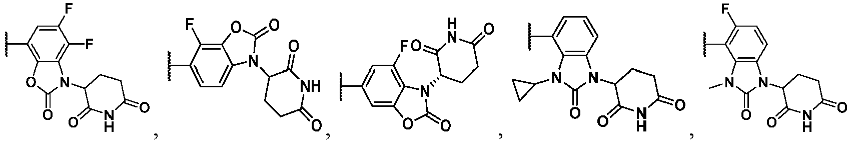 and

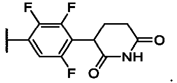

13. The compound of any one of claims 1-12, wherein:
R^{z} is independently selected from H, -CH₃, -C₂H₅, F, -CH₂F, -CHF₂, -CF₃, -OCH₃, - OC₂H₅, -C₃H₇, -OCH₂F, -OCHF₂, -OCH₂CF₃, -OCF₃, -SCF₃, -CF₃ and -CH(OH)CH₃.

14. A compound selected from
| | |
|---|---|
| 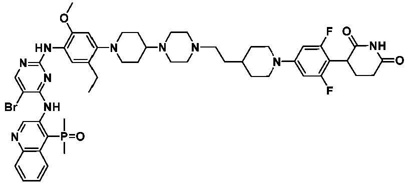 | 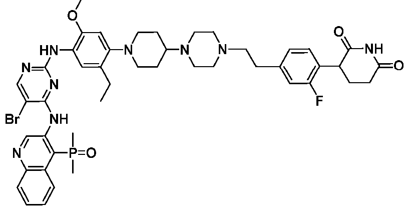 |
| 1 | 2 |
| 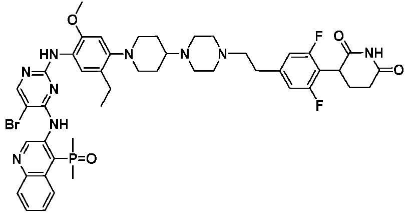 | 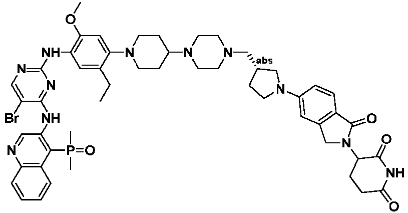 |
| 3 | 4 |
| 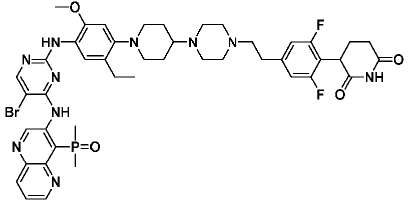 | 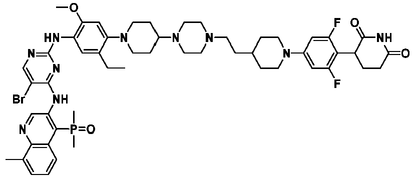 |
| 7 | 8 |
| 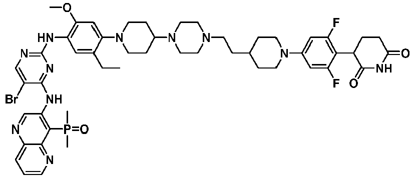 | 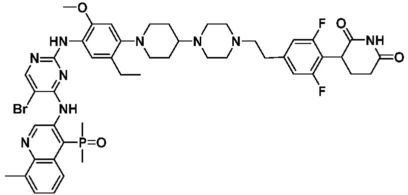 |
| 9 | 10 |
| 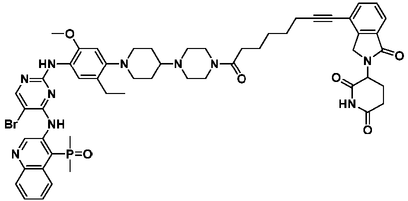 | 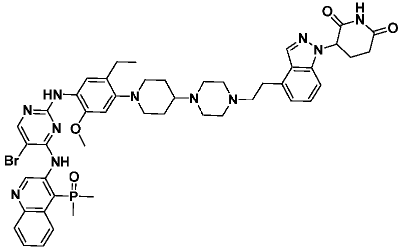 |
| 11 | 12 |
| 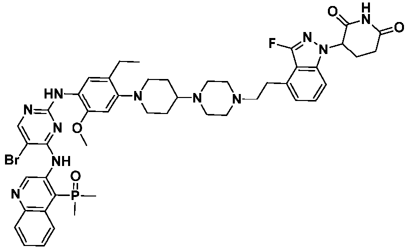 | 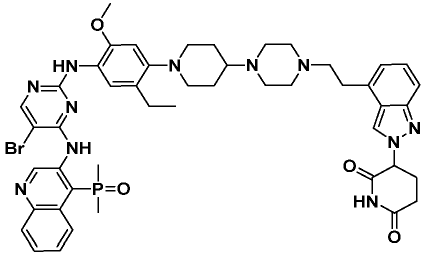 |
| 13 | 14 |
| 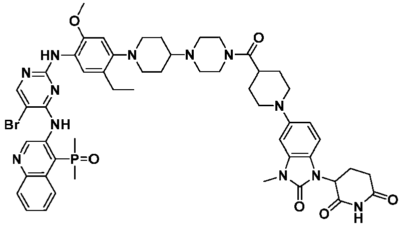 | 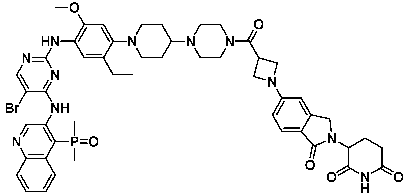 |
| 15 | 16 |
| 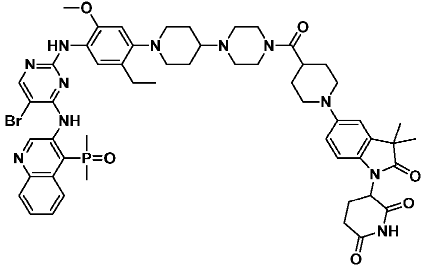 | 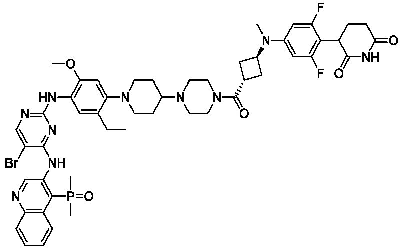 |
| 17 | 18 |
| 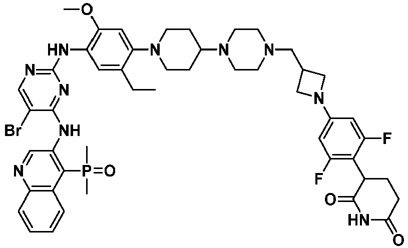 | 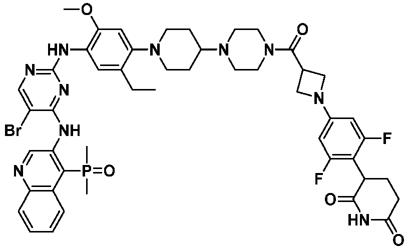 |
| 19 | 20 |
| 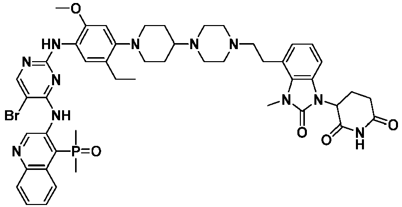 | 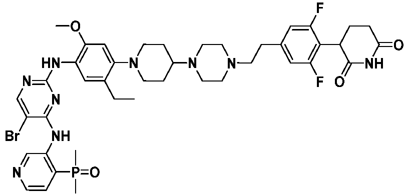 |
| 21 | 22 |
| 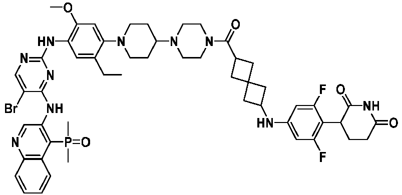 | 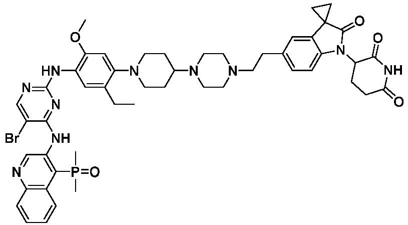 |
| 23 | 24 |
| 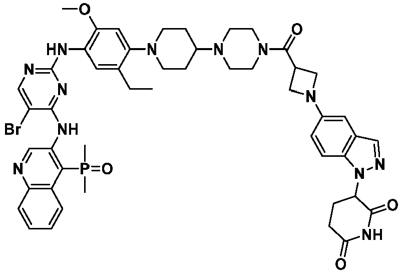 | 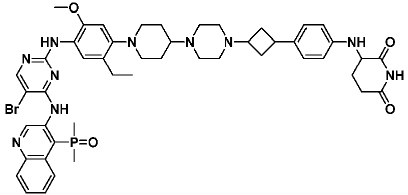 |
| 25 | 26 |
| 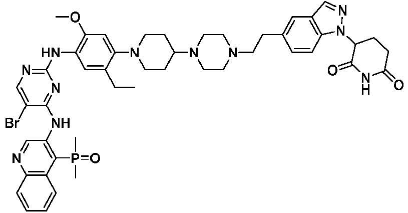 | 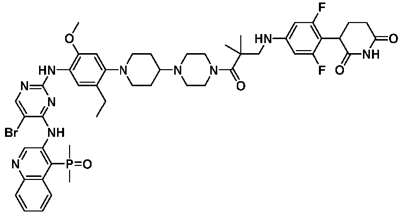 |
| 27 | 28 |
| 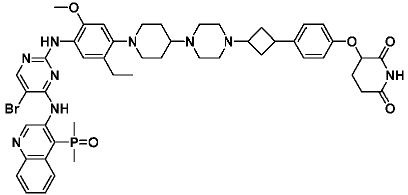 | 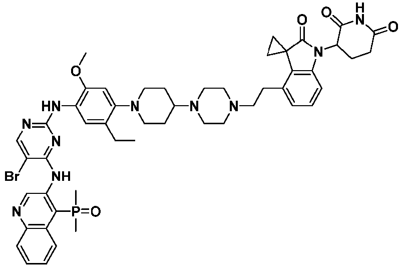 |
| 29 | 30 |
| 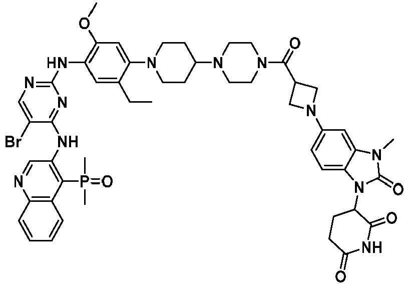 | 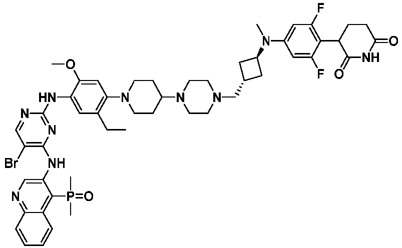 |
| 31 | 32 |
| 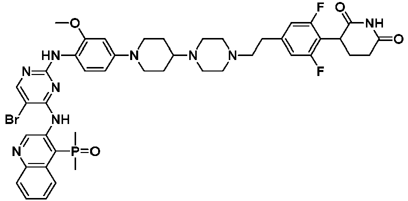 | 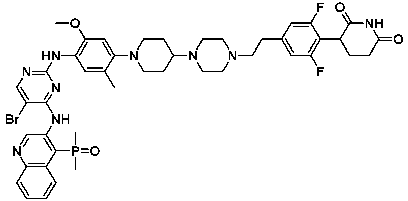 |
| 33 | 34 |
| 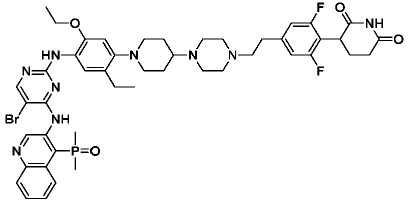 | 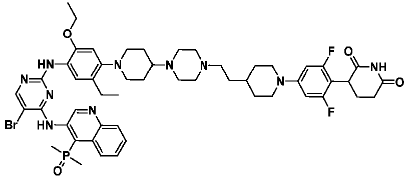 |
| 35 | 36 |
| 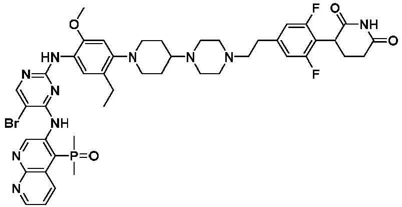 | 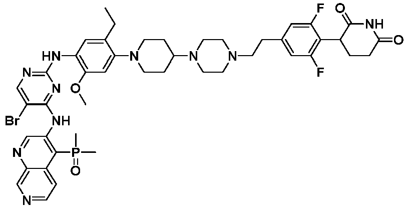 |
| 37 | 38 |
| | 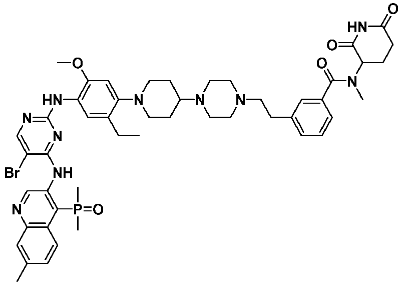 |
| | 40 |
| 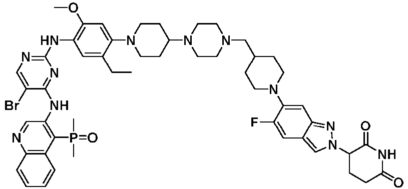 | 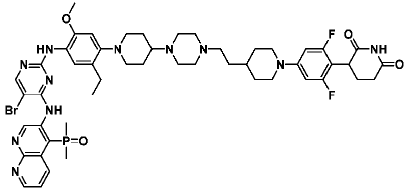 |
| 41 | 42 |
| 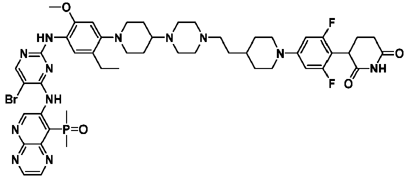 | 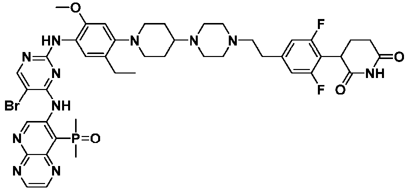 |
| 43 | 44 |
| 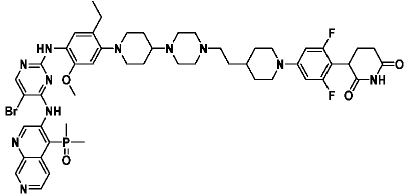 | 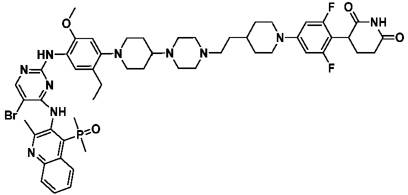 |
| 45 | 46 |
| 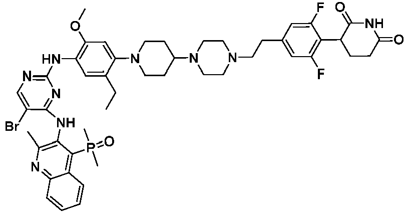 | 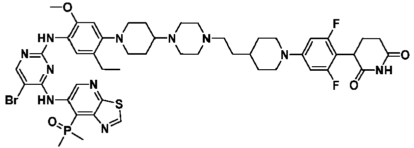 |
| 47 | 48 |
| 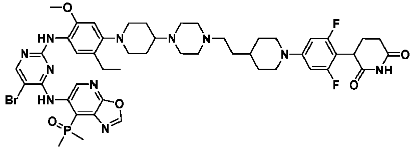 | 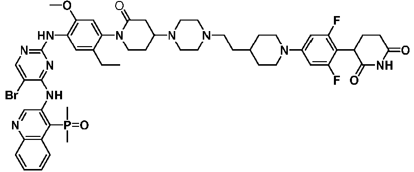 |
| 49 | 50 |
| 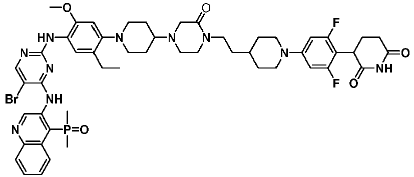 | 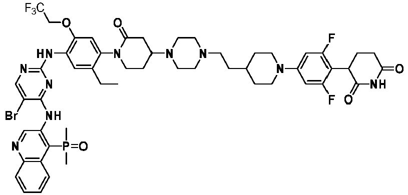 |
| 51 | 52 |
| 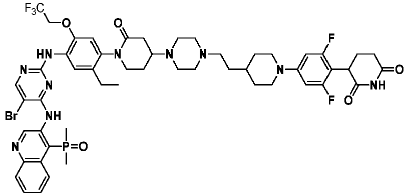 | 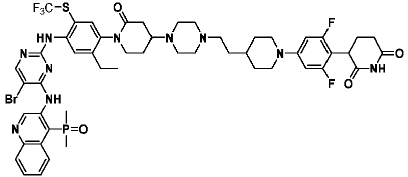 |
| 53 | 54 |
| 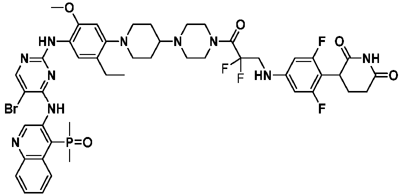 | 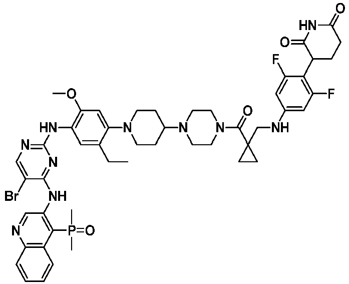 |
| 55 | 56 |
| 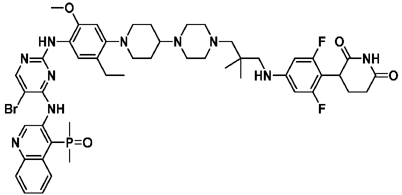 | 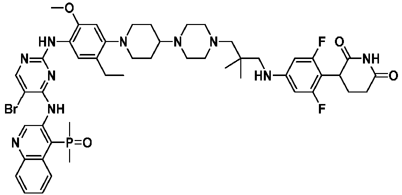 |
| 57 | 58 |
| 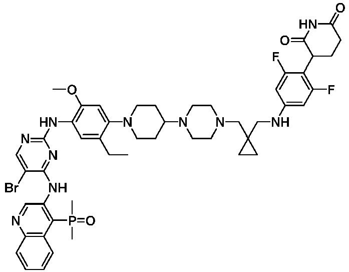 | 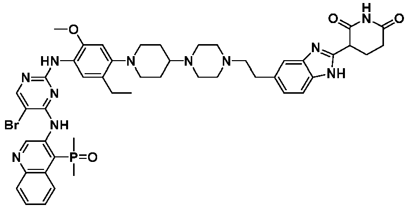 |
| 59 | 60 |
| 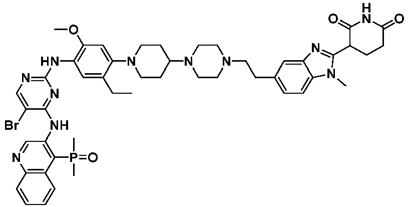 | 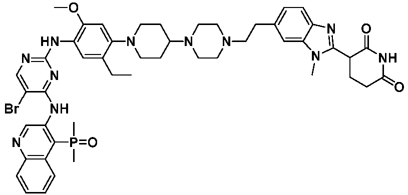 |
| 61 | 62 |
| 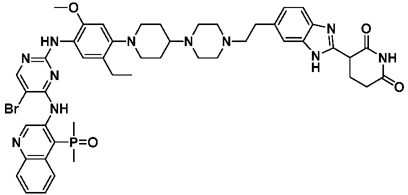 | 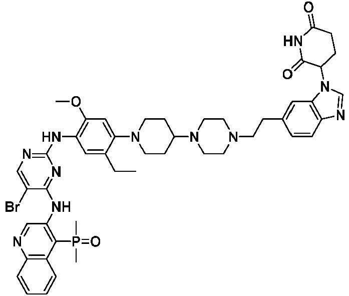 |
| 63 | 64 |
| 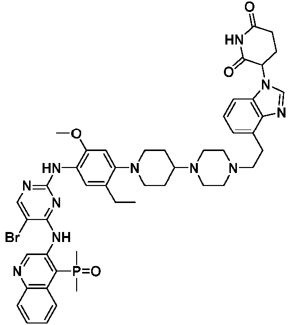 | 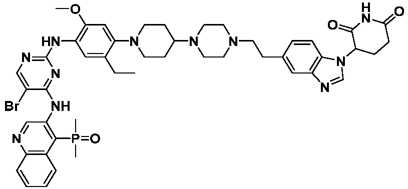 |
| 65 | 66 |
| 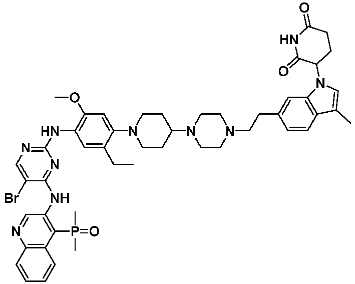 | 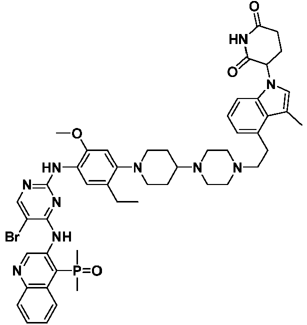 |
| 67 | 68 |
| 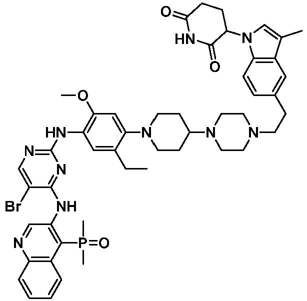 | 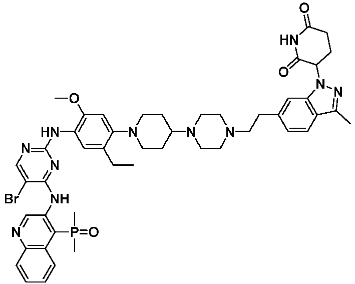 |
| 69 | 70 |
| 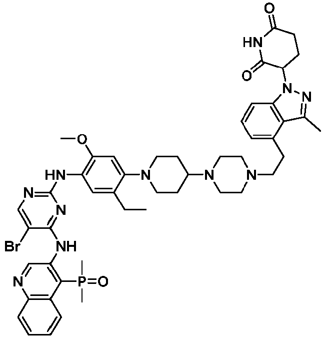 | 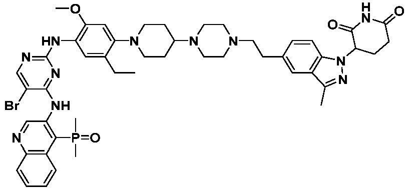 |
| 71 | 72 |
| 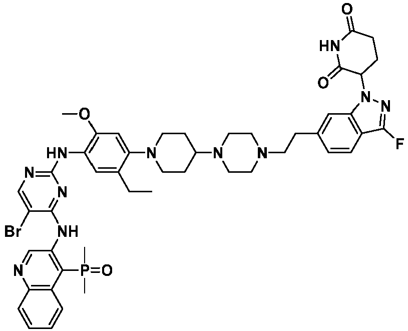 | 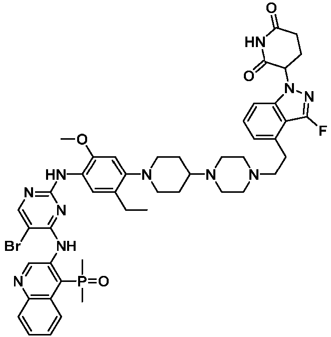 |
| 73 | 74 |
| 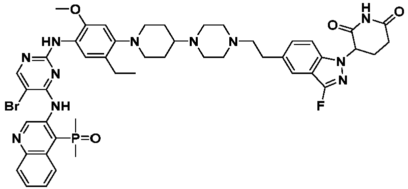 | 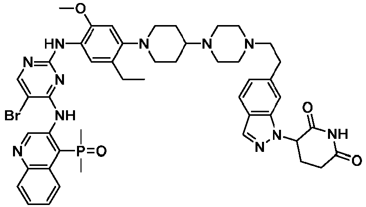 |
| 75 | 76 |
| 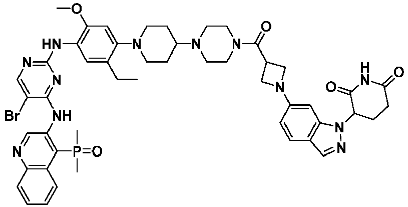 | 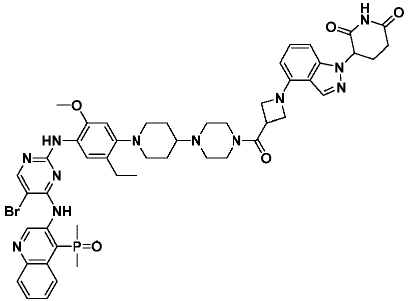 |
| 77 | 78 |
| 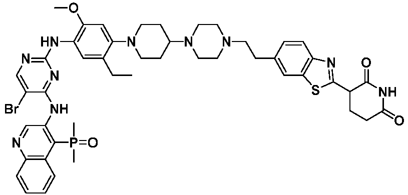 | 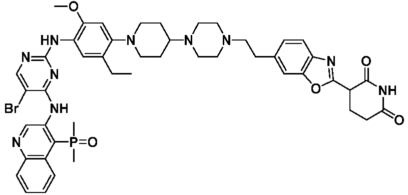 |
| 79 | 80 |
| 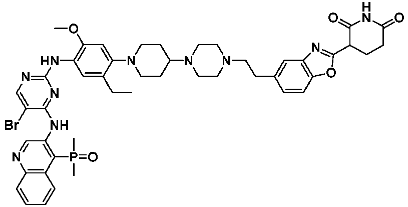 | 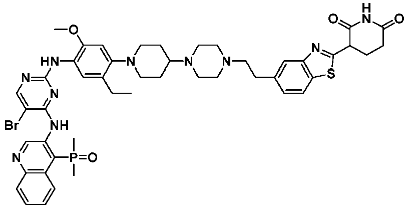 |
| 81 | 82 |
| 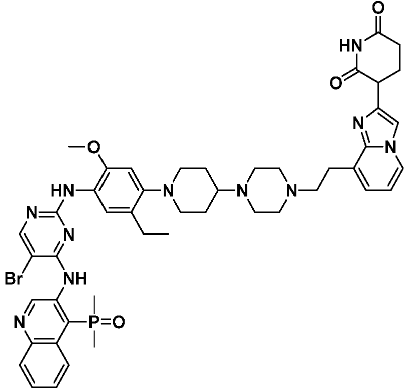 | 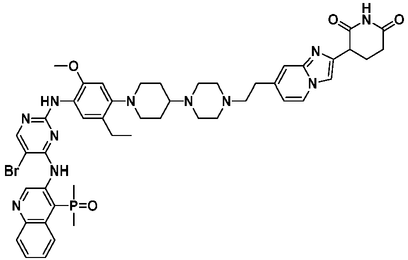 |
| 83 | 84 |
| 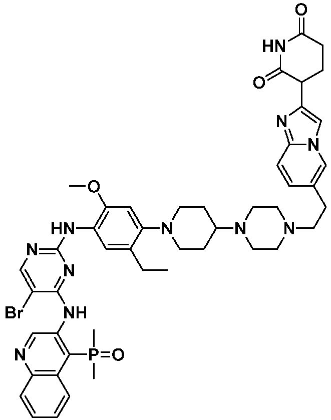 | 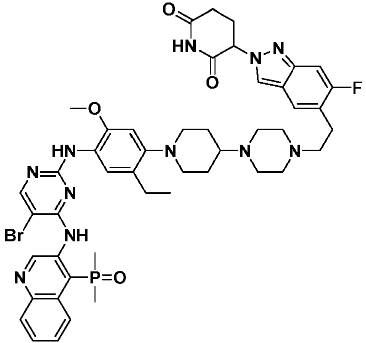 |
| 85 | 86 |
| 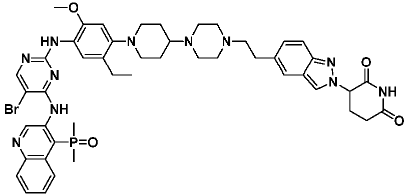 | 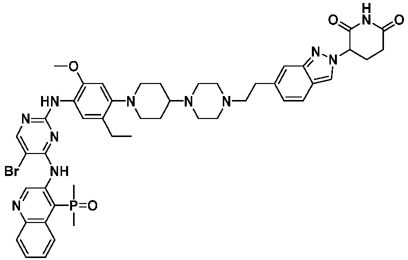 |
| 87 | 88 |
| 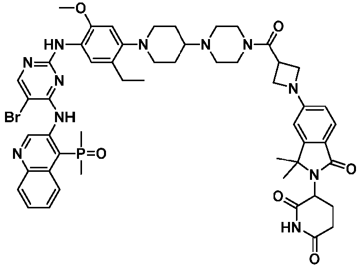 | 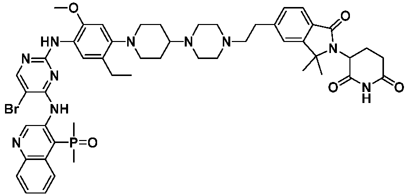 |
| 89 | 90 |
| 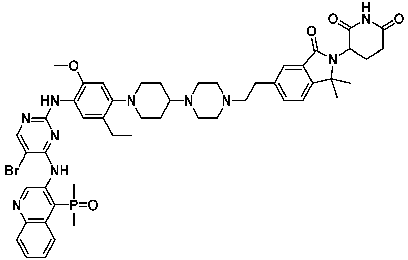 | 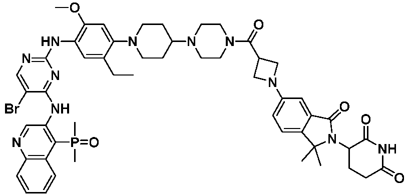 |
| 91 | 92 |
| 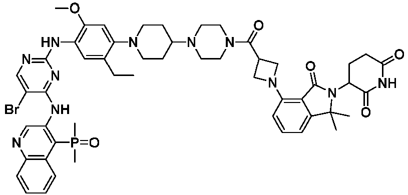 | 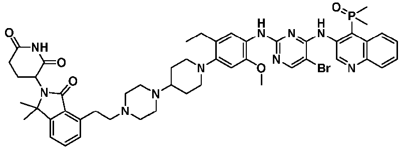 |
| 93 | 94 |
| 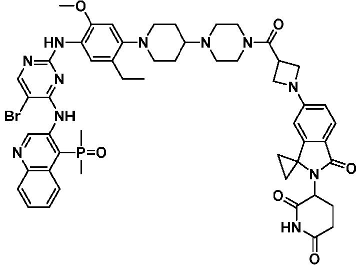 | 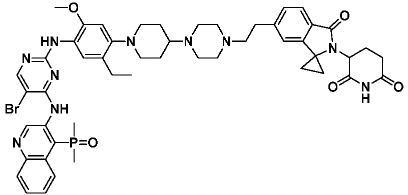 |
| 95 | 96 |
| 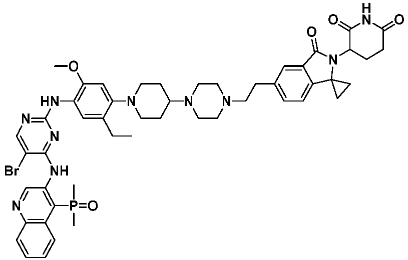 | 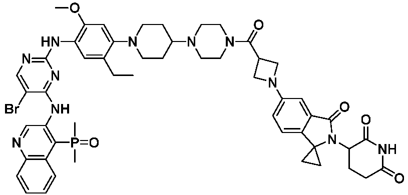 |
| 97 | 98 |
| 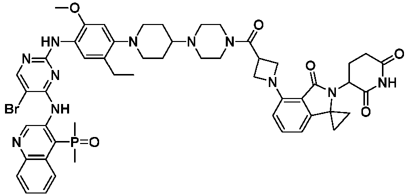 | 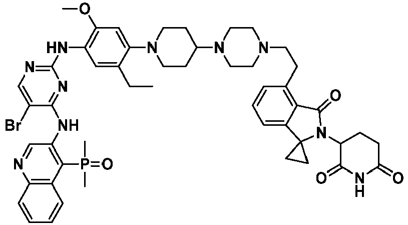 |
| 99 | 100 |
| 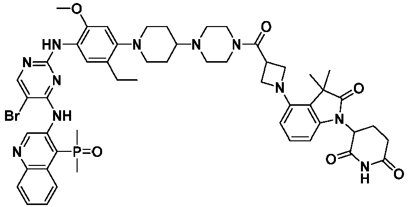 | 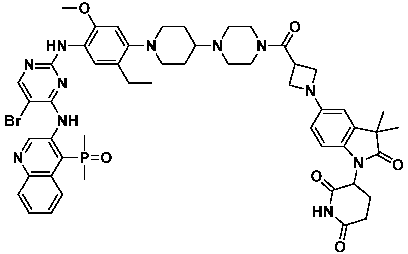 |
| 101 | 102 |
| 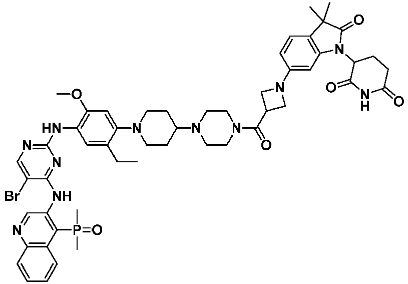 | 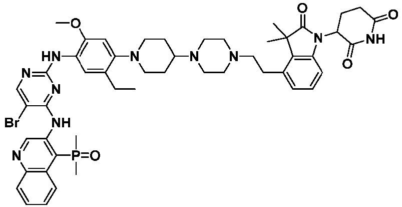 |
| 103 | 104 |
| 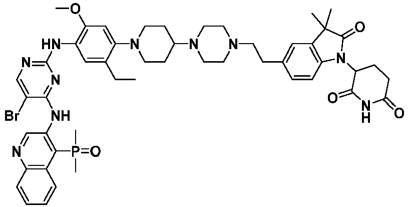 | 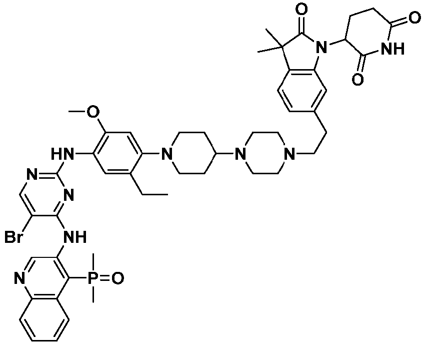 |
| 105 | 106 |
| 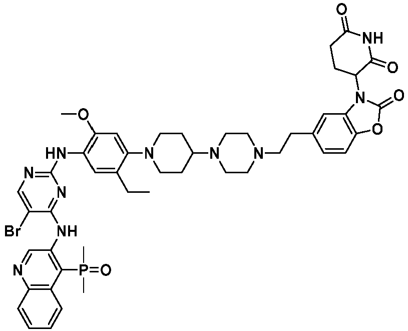 | 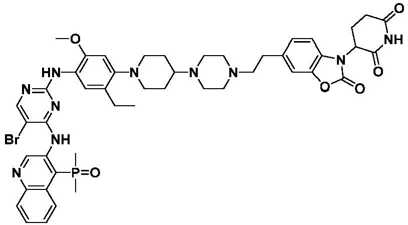 |
| 107 | 108 |
| 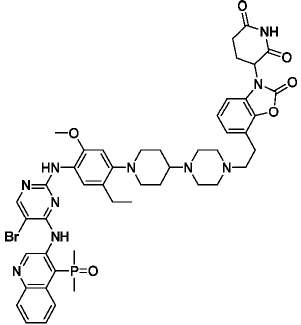 | 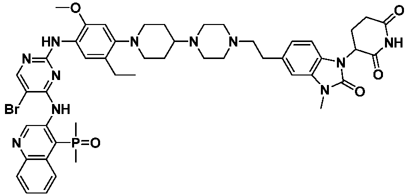 |
| 109 | 110 |
| 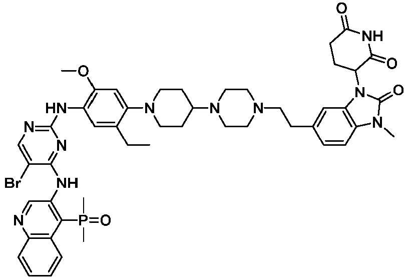 | 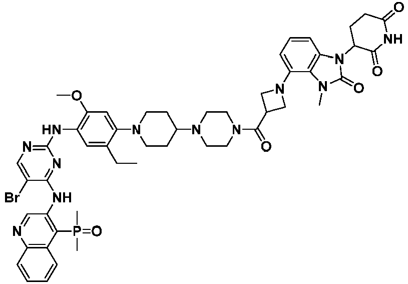 |
| 111 | 112 |
| 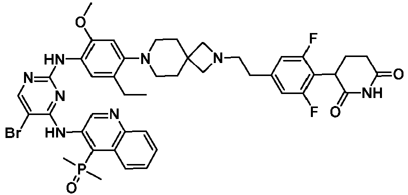 | 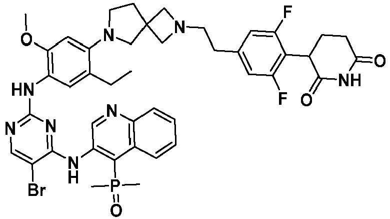 |
| 113 | 114 |
| 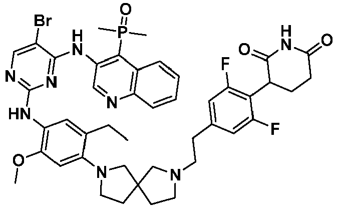 | 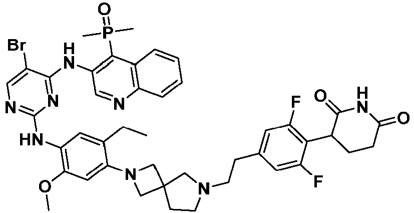 |
| 115 | 116 |
| 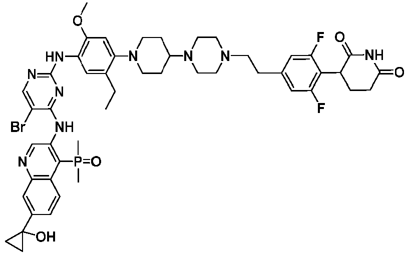 | 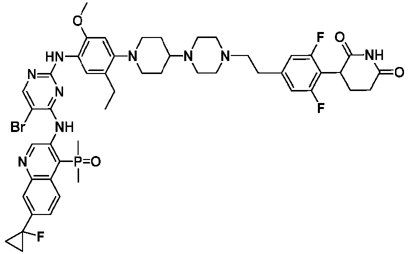 |
| 117 | 118 |
| 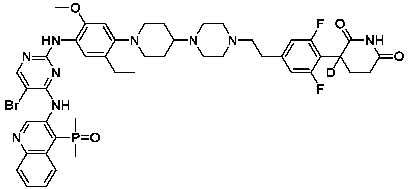 | 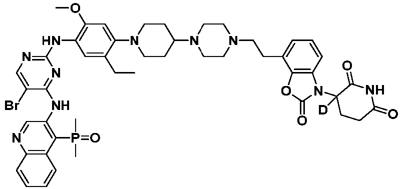 |
| 119 | 120 |
| 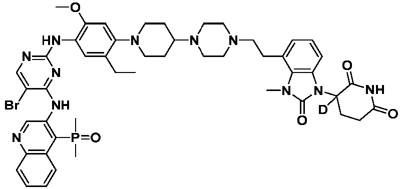 | 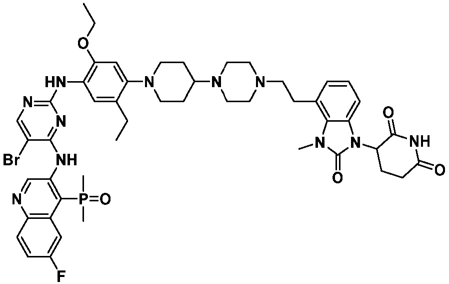 |
| 121 | 124 |
| 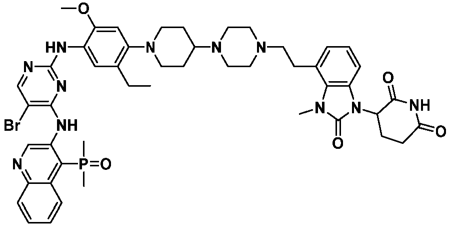 | 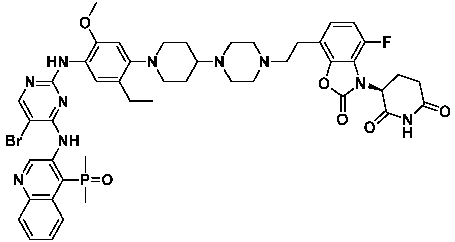 |
| 125 | 126 |
| 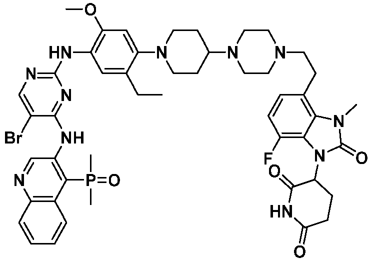 | 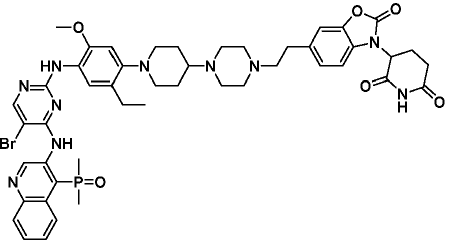 |
| 130 | 132 |
| 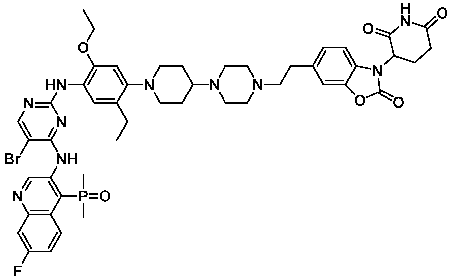 | 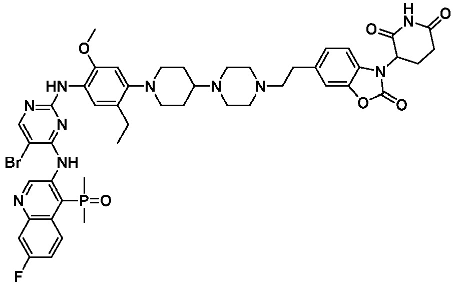 |
| 133 | 134 |
| 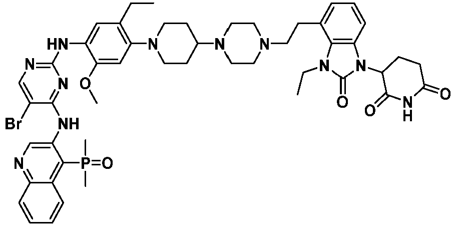 | 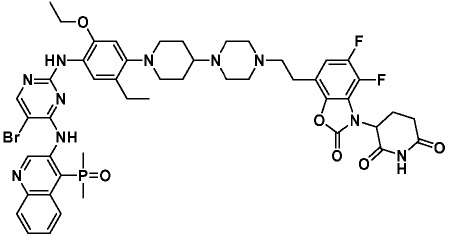 |
| 135 | 136 |
| 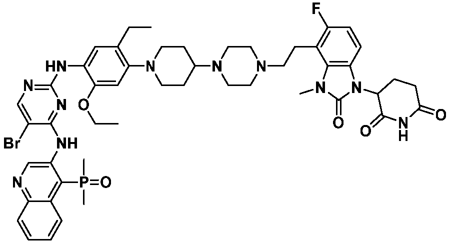 | 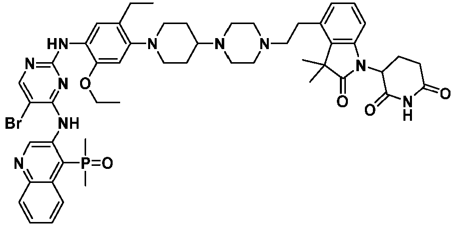 |
| 137 | 138 |
| 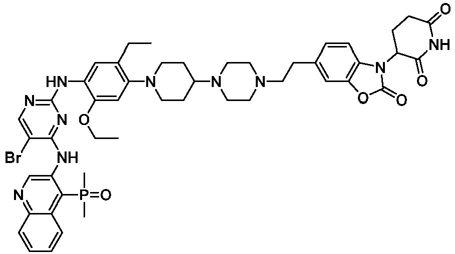 | 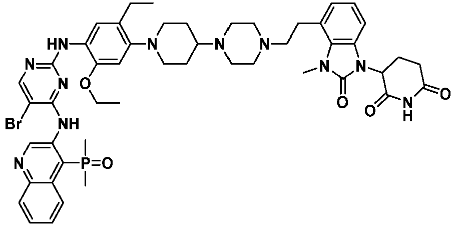 |
| 139 | 140 |
| 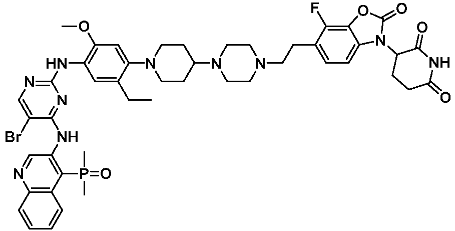 | 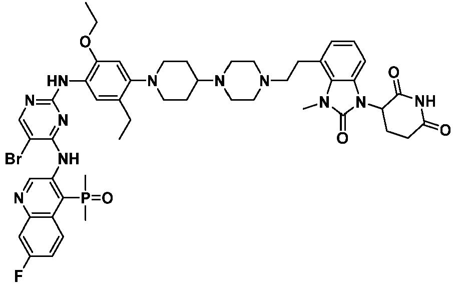 |
| 142 | 147 |
| 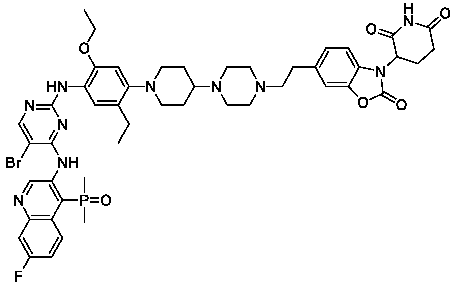 | 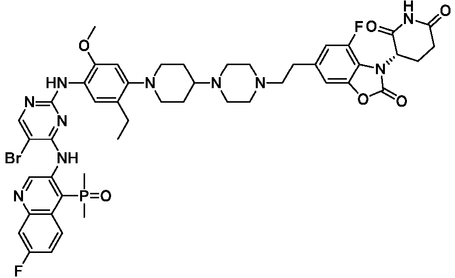 |
| 149 | 151 |
| 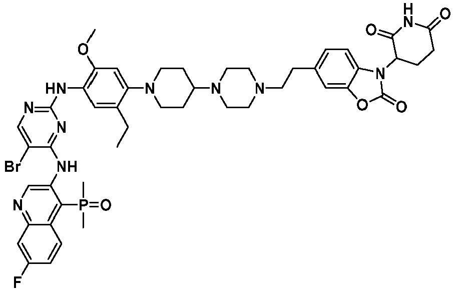 | 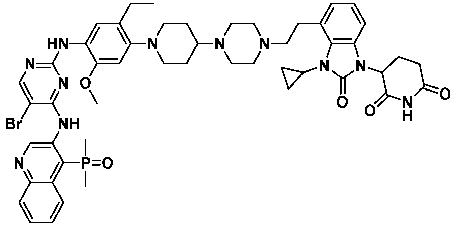 |
| 153 | 155 |
| | 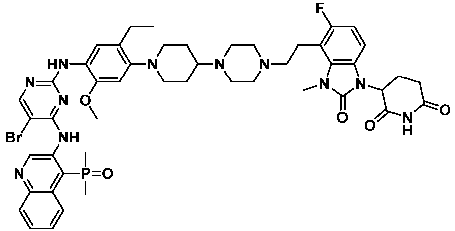 |
| | 159 |
| 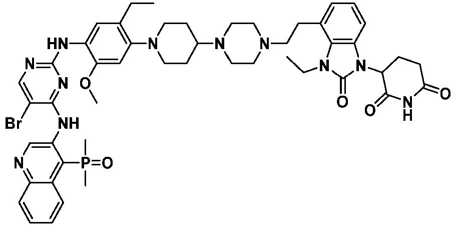 | 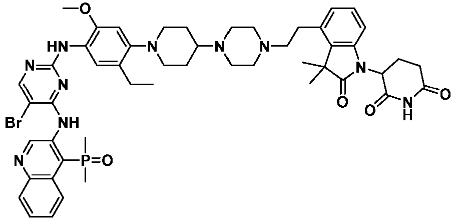 |
| 163 | 164 |
| 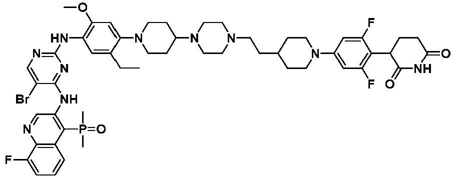 | 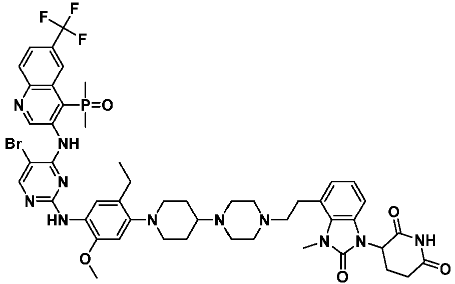 |
| 165 | 166 |
| 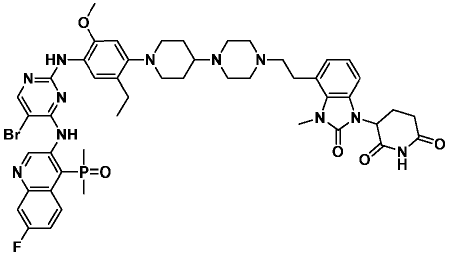 | 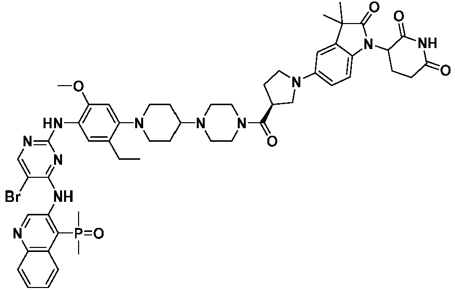 |
| 167 | 168 |
| 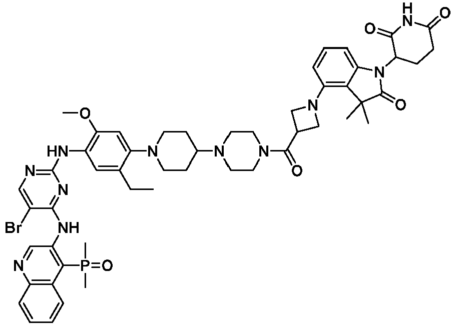 | 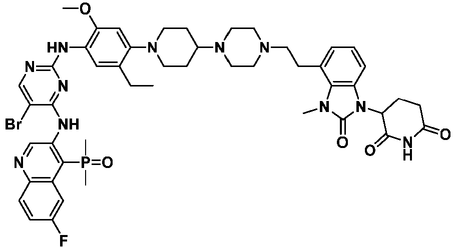 |
| 169 | 170 |
| 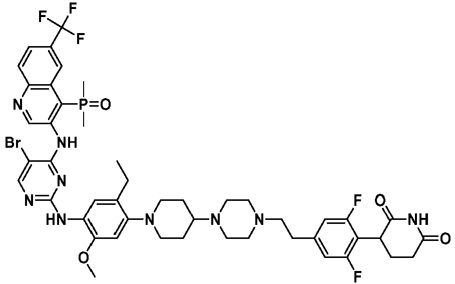 | 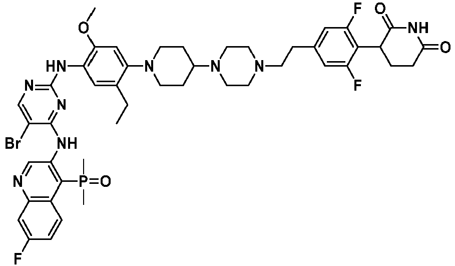 |
| 171 | 172 |
| 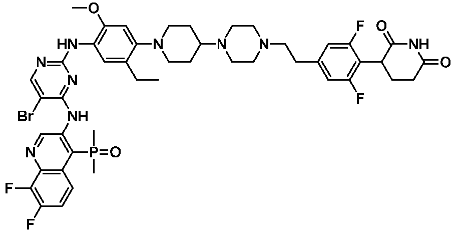 | 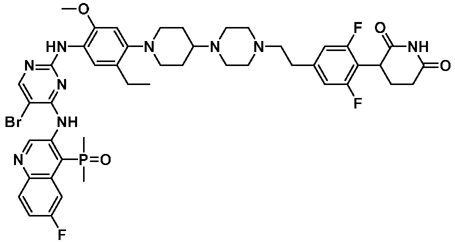 |
| 173 | 174 |
| 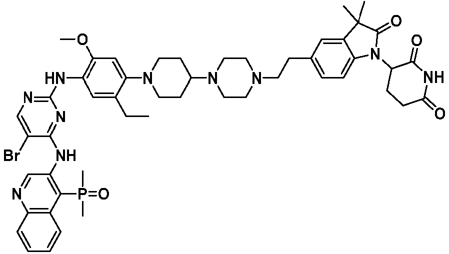 | 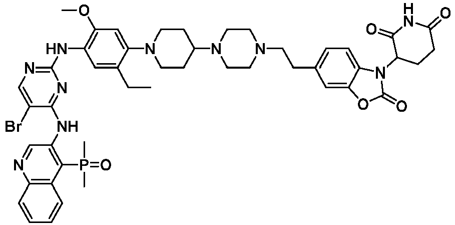 |
| 175 | 176 |
| | 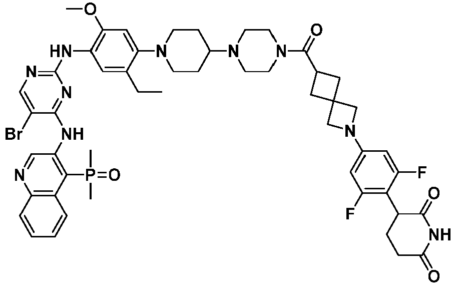 |
| | 178 |
| 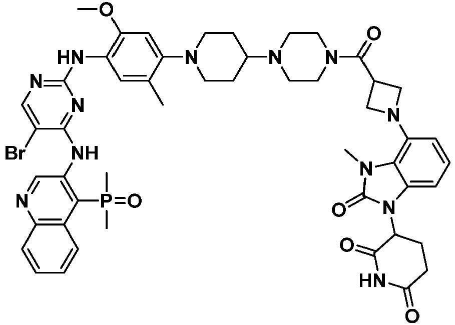 | 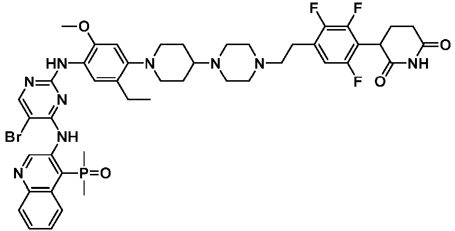 |
| 179 | 180 |
| 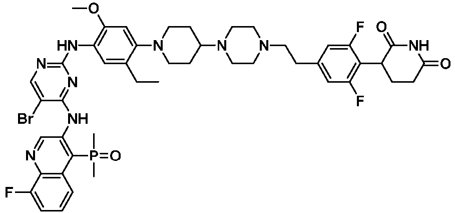 | 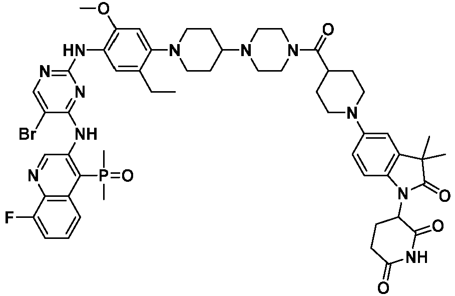 |
| 181 | 182 |
| 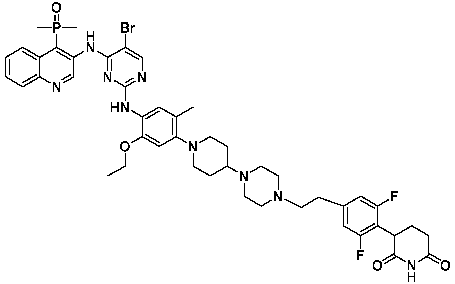 | 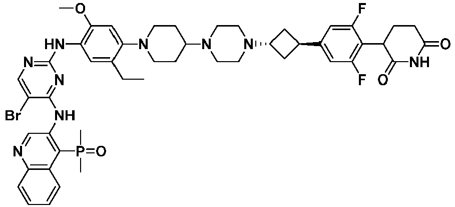 |
| 183 | 184 |
| 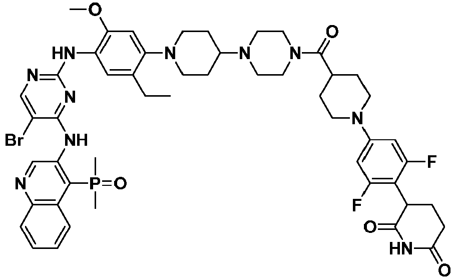 | 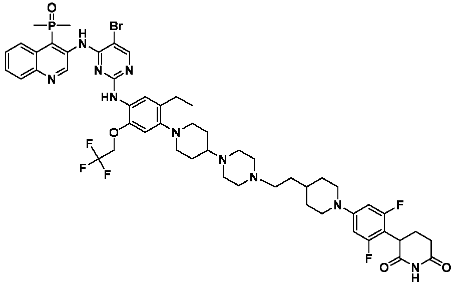 |
| 185 | 186 |
| 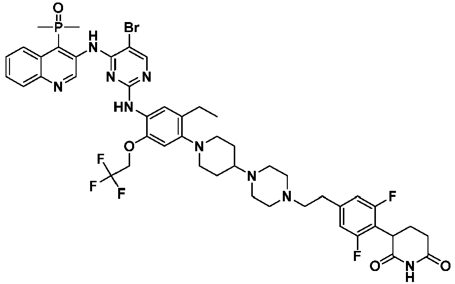 | 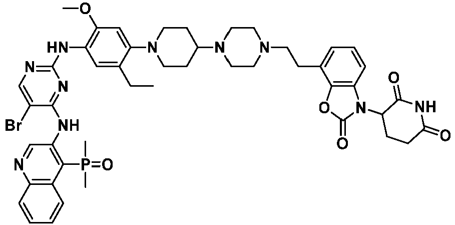 |
| 187 | 188, and |
| 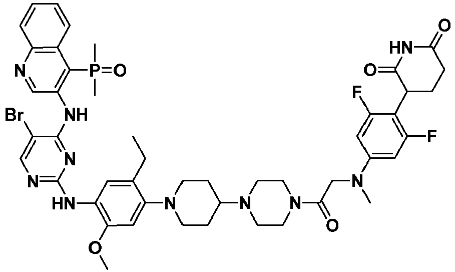 | |
| 189 | |

15. A compound according to any one of the above claims, for use in a method of:
(i) decreasing EGFR activity by inhibition and/or degradation, which comprises administering to an individual the compound, or a pharmaceutically acceptable salt thereof;
or
(ii) treating a disease or disorder in a patient comprising administering to the patient a therapeutically effective amount of the compound, or a pharmaceutically acceptable salt thereof as a EGFR kinase inhibitor and/or degrader, wherein the disease or disorder is associated with inhibition of EGFR.

16. The compound for use according to claim 15, wherein the disease is selected cancer, preferably pancreatic cancer, breast cancer, glioblastoma multiforme, head and neck cancer, or non-small cell lung cancer.

## Patentansprüche

1. Verbindung der Formel (X): 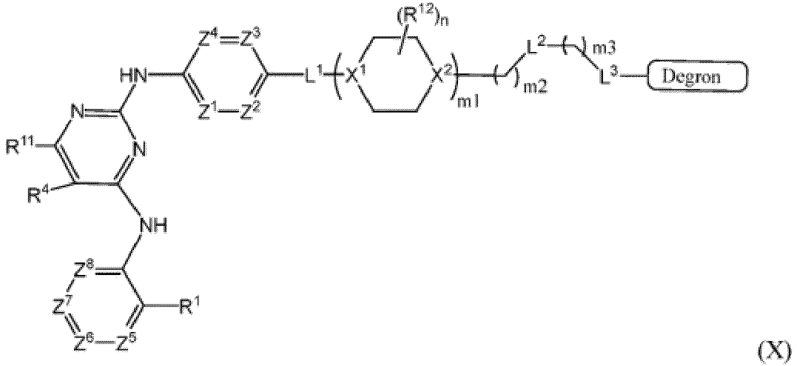 oder ein N-Oxid davon oder ein pharmazeutisch annehmbares Salz davon oder ein Stereoisomer davon oder ein deuteriertes Analogon davon, worin:
R¹ -P(O)R^{1a}R^{1b} ist, worin R^{1a} und R^{1b} jeweils unabhängig voneinander aus -CH₃, -CH₂CH₃, -CH₂CH₂CH₃, -iso-C₃H₇, -CH₂CH₂CH₂CH₃, -iso-C₄H₉, -sec-C₄H₉ und -tert-C₄H₉, Cyclopropyl, Cyclobutyl und Cyclopentyl ausgewählt sind;
Z⁵ -CR² ist;
Z⁶ -CR³ ist;
Z⁷ N ist;
Z⁸ -CR¹⁰ ist;
R² und R³ zusammen mit den Kohlenstoffatomen, an die sie gebunden sind, einen 5-bis 6-gliedrigen aromatischen Ring bilden, wobei der Ring 0 bis 3 Heteroatome umfasst, die jeweils unabhängig aus Stickstoff, Sauerstoff und Schwefel ausgewählt sind, wobei der Ring gegebenenfalls mit zumindest einem Substituenten R^{2e} substituiert ist;
R^{2e} bei jedem Vorkommen jeweils unabhängig Wasserstoff, -F, -Cl, -Br, -I, Methyl, Ethyl, Propyl, Butyl, Pentyl, Cyclopropyl, Cyclobutyl oder Cyclopentyl ist, wobei das Methyl, Ethyl, Propyl, Butyl, Pentyl, Cyclopropyl, Cyclobutyl oder Cyclopentyl jeweils gegebenenfalls mit zumindest einem R^{2d} substituiert ist; und
R^{2d} bei jedem Vorkommen jeweils unabhängig -F, -Cl, -Br, -I, -OH, Methyl, Ethyl, Propyl, Butyl, Pentyl, Hexyl, Heptyl, Octyl, Methoxy, Ethoxy, Propoxy, Butoxy, Pentoxy, Hexoxy, Heptoxy, Octoxy, Cyclopropyl, Cyclobutyl, Cyclopentyl, Cyclohexyl, Cycloheptyl, Cyclooctyl oder Phenyl ist;
R⁴ aus Wasserstoff, Halogen, -C₁₋₈-Alkyl, -C₂₋₈-Alkenyl, -C₂₋₈-Alkinyl, C₁₋₈-Alkoxy, -C₃₋₈-Cycloalkyl, 3- bis 8-gliedrigem Heterocyclyl, -C₆₋₁₂-Aryl, 5- bis 12-gliedrigem Heteroaryl, -CN, -SO₂R⁴, -SO₂NR⁴R^{4b}, -COR⁴, -CO₂R^{4a}, -CONR⁴R^{4b}, -NR^{4a}R^{4b}, -NR^{4a}COR^{4b}, -NR^{4a}CO₂R^{4b} und -NR⁴SO₂R^{4b} ausgewählt ist, wobei das -C₁₋₈-Alkyl, -C₂₋₈-Alkenyl, -C₂₋₈-Alkinyl, -C₁₋₈-Alkoxy, -C₃₋₈-Cycloalkyl, 3- bis 8-gliedrige Heterocyclyl, -C₆₋₁₂-Aryl oder 5- bis 12-gliedrige Heteroaryl jeweils gegebenenfalls mit Halogen, -C₁₋₈-Alkoxy, -C₁₋₈-Alkyl, -C₂₋₈-Alkenyl, -C₂₋₈-Alkinyl, -C₃₋₈-Cycloalkyl, 3- bis 8-gliedrigem Heterocyclyl, C₆₋₁₂-Aryl, 5- bis 12-gliedrigem Heteroaryl, Oxo, -CN, -OR^{4c}, -SO₂R^{4c}, -SO₂NR^{4c}R^{4d}, -COR^{4c}, -CO₂R^{4c}, -CONR^{4c}R^{4d}, -NR^{4c}R^{4d}-, -NR^{4c}COR^{4d}, -NR^{4c}CO₂R^{4d} oder -NR^{4c}SO₂R^{4d} substituiert ist,
worin R^{4a}, R^{4b}, R⁴, und R^{4d} jeweils unabhängig voneinander Wasserstoff, -C₁₋₈-Alkyl, -C₂₋₈-Alkenyl, -C₂₋₈-Alkinyl, C₃₋₈-Cycloalkyl, 3- bis 8-gliedriges Heterocyclyl, -C₆₋₁₂-Aryl oder 5-bis 12-gliedriges Heteroaryl sind;
R¹⁹, R¹¹ und R¹² jeweils unabhängig voneinander aus Wasserstoff, -F, -Cl, -Br, -I, Methyl, Ethyl, Propyl, Butyl, Pentyl, Hexyl, Heptyl, Octyl, Methoxy, Ethoxy, Propoxy, Butoxy, Pentoxy, Hexoxy, Heptoxy, Octoxy, Cyclopropyl, Cyclobutyl, Cyclopentyl, Cyclohexyl, Cycloheptyl, Cyclooctyl, -NH₂ und Oxo ausgewählt sind, wobei das Methyl, Ethyl, Propyl, Butyl, Pentyl, Hexyl, Heptyl, Octyl, Methoxy, Ethoxy, Propoxy, Butoxy, Pentoxy, Hexoxy, Heptoxy, Octoxy, Cyclopropyl, Cyclobutyl, Cyclopentyl, Cyclohexyl, Cycloheptyl oder Cyclooctyl jeweils gegebenenfalls mit zumindest einem Substituenten R^{9c} substituiert ist, oder
zwei R¹² zusammen mit den Kohlenstoffatomen, an die sie gebunden sind, einen 3-, 4-, 5-, 6-, 7- oder 8-gliedrigen Ring bilden, wobei der Ring 0, 1, 2 oder 3 Heteroatome umfasst, die jeweils unabhängig aus Stickstoff, Sauerstoff und Schwefel ausgewählt sind; wobei der Ring gegebenenfalls mit zumindest einem Substituenten R^{9c} substituiert ist,
worin die R^{9c} jeweils unabhängig voneinander -F, -Cl, -Br, -I, Hydroxy, Methyl, Ethyl, Propyl, Butyl, Pentyl, Hexyl, Heptyl, Octyl, Methoxy, Ethoxy, Propoxy, Butoxy, Pentoxy, Hexoxy, Heptoxy, Octoxy, Cyclopropyl, Cyclobutyl, Cyclopentyl, Cyclohexyl, Cycloheptyl, Cyclooctyl, -C₂₋₈-Alkenyl, -C₂₋₈-Alkinyl, 3- bis 8-gliedriges Heterocyclyl, Phenyl, 5- bis 12-gliedriges Heteroaryl, NH₂ oder -NHCH₃ sind;
Z¹, Z², Z³ und Z⁴ jeweils unabhängig voneinander -CR^{z} sind,
worin R^{Z} bei jedem Vorkommen jeweils unabhängig aus Wasserstoff, Halogen, -C₁₋₈-Alkyl, -C₂₋₈-Alkenyl, -C₂₋₈-Alkinyl, -NR^{Za}R^{Zb}, -OR^{Za}, -SR^{Za}, C₃₋₈-Cycloalkyl, 3- bis 8-gliedrigem Heterocyclyl, C₆₋₁₂-Aryl, 5- bis 12-gliedrigem Heteroaryl und CN ausgewählt ist, wobei das -C₁₋₈-Alkyl, -C₂₋₈-Alkenyl, -C₂₋₈-Alkinyl, C₃₋₈-Cycloalkyl, 3- bis 8-gliedrige Heterocyclyl, C₆₋₁₂-Aryl oder 5- bis 12-gliedrige Heteroaryl jeweils gegebenenfalls mit zumindest einem R^{Zc} substituiert ist,
worin R^{Za} und R^{Zb} jeweils unabhängig voneinander aus Wasserstoff, Methyl, Ethyl, Propyl, Butyl, Pentyl, Hexyl, Heptyl, Octyl, -C₂₋₈-Alkenyl, -C₂₋₈-Alkinyl, Cyclopropyl, Cyclobutyl, Cyclopentyl, Cyclohexyl, Cycloheptyl, Cyclooctyl, 3- bis 8-gliedrigem Heterocyclyl, Phenyl und 5- bis 12-gliedrigem Heteroaryl ausgewählt sind, wobei das Methyl, Ethyl, Propyl, Butyl, Pentyl, Hexyl, Heptyl, Octyl, -C₂₋₈-Alkenyl, -C₂₋₈-Alkinyl, Cyclopropyl, Cyclobutyl, Cyclopentyl, Cyclohexyl, Cycloheptyl, Cyclooctyl, 3- bis 8-gliedrige Heterocyclyl, Phenyl oder 5- bis 12-gliedrige Heteroaryl jeweils gegebenenfalls mit zumindest einem Substituenten R^{Zd} substituiert ist, und
worin R^{Zc} und R^{Zd} jeweils unabhängig voneinander -F, -Cl, Br, -I, Hydroxy, Methyl, Ethyl, Propyl, Butyl, Pentyl, Hexyl, Heptyl, Octyl, -C₁₋₈-Alkoxy, -C₂₋₈-Alkenyl, -C₂₋₈-Alkinyl, Cyclopropyl, Cyclobutyl, Cyclopentyl, Cyclohexyl, Cycloheptyl, Cyclooctyl, 3- bis 8-gliedriges Heterocyclyl, Phenyl oder 5- bis 12-gliedriges Heteroaryl sind;
L¹ aus einer Einfachbindung, -O-, -SO₂-, -C(O)-, -NR^{L1a}-, -C₃₋₈-Cycloalkylen-, *^{L1}-O-C₁₋₈-alkylen-**^{L1}, *^{L1}-C₁₋₈-Alkylen-O-**^{L1}, *^{L1}-SO₂-C₁₋₈-Alkylen-**^{L1}, *^{L1}-C₁₋₈-Alkylen-SO₂-**^{L1}, *^{L1}-CO-C₁₋₈-Alkylen-**^{L1}, *^{L1}-C₁₋₈-Alkylen-CO-**^{L1}, *^{L1}-NR^{L1a}-C₁₋₈-Alkylen-**^{L1}, *^{L1}-C₁₋₈-Alkylen-NRL^{1a}-**L¹, *^{L1}-NR^{L1a}C(O)-**^{L1}, *^{L1}-C(O)NR^{L1a}-**^{L1}, -C₁₋₈-Alkylen-, -C₂₋₈-Alkenylen-, C₂₋₈-Alkinylen, -[O(CR^{L1a}R^{L1b})ₘ₄]ₘ₅-, 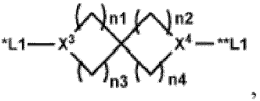 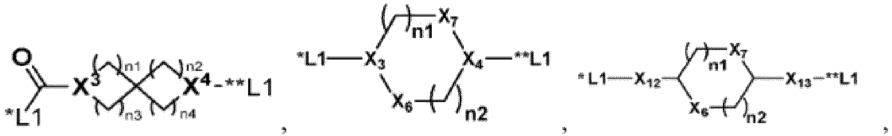 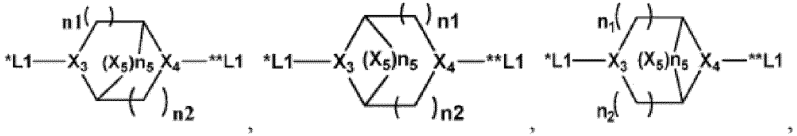 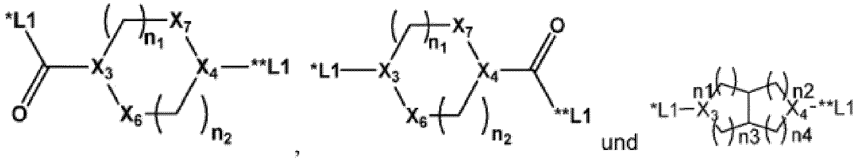 ausgewählt ist,
**wobei das** -C₃₋₈-Cycloalkylen-, *^{L1}-O-C₁₋₈-Alkylen-**^{L1}, *^{L1}-C₁₋₈-Alkylen-O-**^{L1}, *^{L1}-SO₂-C₁₋₈-Alkylen-**⁻¹, *^{L1}-C₁₋₈-Alkylen-SO₂-**^{L1}, *^{L1}-CO-C₁₋₈-Alkylen-**^{L1}, *^{L1}-C₁₋₈-Alkylen-CO-**^{L1}, *^{L1}-NR^{L1a}-C₁₋₈-Alkylen-**^{L1}, *^{L1}-C₁₋₈-Alkylen-NR^{L1a}-**^{L1}, -C₁₋₈-Alkylen-, -C₂₋₈-Alkenylen-, -C₂₋₈-Alkinylen, 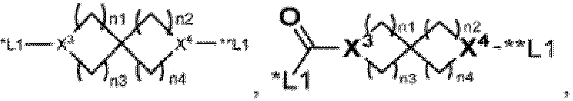 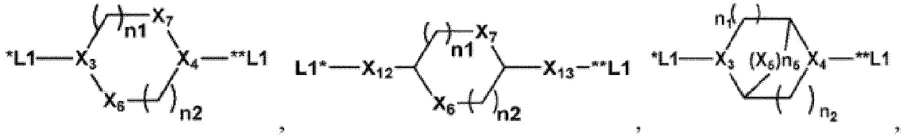 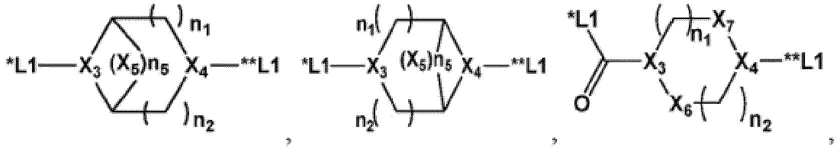 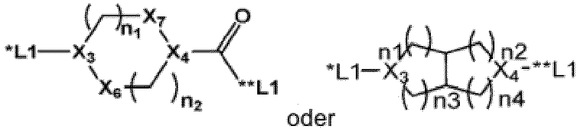 jeweils gegebenenfalls mit zumindest einem R^{L1c} substituiert ist,
worin sich *^{L1} auf die an die Gruppierung 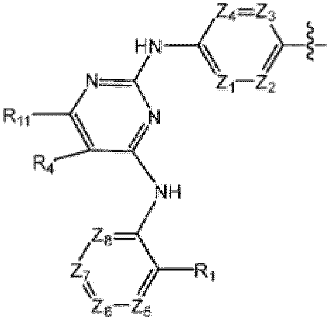 gebundene Position bezieht und sich **^{L1} auf die an die Gruppierung 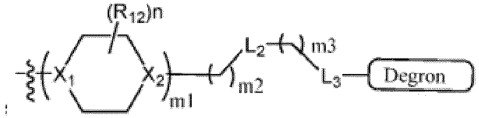 gebundene Position bezieht,
worin R^{L1a} und R^{1b} jeweils unabhängig voneinander aus Wasserstoff, -C₁₋₈-Alkyl, -C₂₋₈-Alkenyl, -C₂₋₈-Alkinyl, C₃₋₈-Cycloalkyl, 3- bis 8-gliedrigem Heterocyclyl, C₆₋₁₂-Aryl und 5- bis 12-gliedrigem Heteroaryl ausgewählt sind, wobei das -C₁₋₈-Alkyl, -C₂₋₈-Alkenyl, -C₂₋₈-Alkinyl, C₃₋₈-Cycloalkyl, 3- bis 8-gliedrige Heterocyclyl, C₆₋₁₂-Aryl oder 5- bis 12-gliedrige Heteroaryl jeweils gegebenenfalls mit zumindest einem Substituenten R^{L1d} substituiert ist,
worin R^{L1c} und R^{L1d} jeweils unabhängig voneinander Halogen, Hydroxy, -C₁₋₈-Alkyl, -C₁₋₈-Alkoxy, -C₁₋₈-Alkoxy-C₁₋₈-alkyl-, -C₂₋₈-Alkenyl, -C₂₋₈-Alkinyl, C₃₋₈-Cycloalkyl, 3- bis 8-gliedriges Heterocyclyl, C₆₋₁₂-Aryl, 5- bis 12-gliedriges Heteroaryl oder Oxo sind;
L² aus einer Einfachbindung, -O-, -SO₂-, -CO-, -NR^{L2a}-, -C₃₋₈-Cycloalkylen-, *^{L2}-O-C₁₋₈-Alkylen-**L², *^{L2}-C₁₋₈-Alkylen-O-**^{L2}, *^{L2}-SO₂-C₁₋₈-Alkylen-**^{L2}, *^{L2}-C₁₋₈-Alkylen-SO₂-**^{L2}, *^{L2}-CO-C₁₋₈-Alkylen-**^{L2}, *L²-C₁₋₈-Alkylen-CO-**L², *^{L2}-NR^{L2a}-C₁₋₈-Alkylen-**^{L2}, *^{L2}-C₁₋₈-Alkylen-NR^{L2a}-**^{L2}, *^{L2}-NR^{L2a}C(O)-**^{L2}, *^{L2}-C(O)NR^{L2a}-**^{L2}, -C₁₋₈-Alkylen-, -C₂₋₈-Alkenylen-, -C₂₋₈-Alkiny-**len-,** -[O(CR^{L2a}R^{L2b})ₘ₄]ₘ₅-, 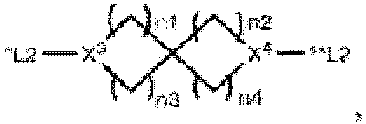 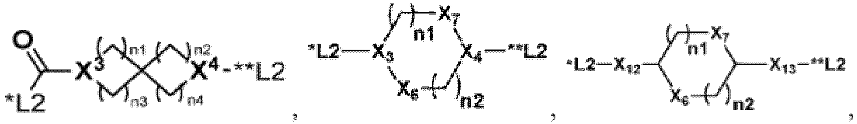 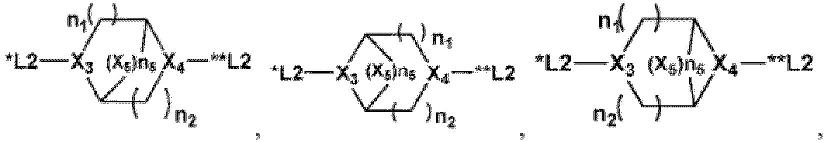 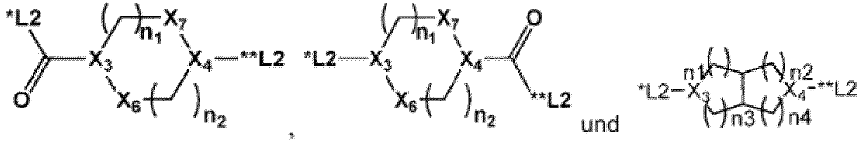 ausgewählt ist,
wobei das -C₃₋₈-Cycloalkylen-, *^{L2}-O-C₁₋₈-Alkylen-**^{L2}, *^{L2}-C₁₋₈-Alkylen-O-**^{L2}, *^{L2}-SO₂-C₁₋₈-Alkylen-**^{L2}, *^{L2}-C₁₋₈-Alkylen-SO₂-**^{L2}, *^{L2}-CO-C₁₋₈-Alkylen-**^{L2}, *^{L2}-C₁₋₈-Alkylen-CO-**^{L2}, *^{L2}-NR^{L2a}-C₁₋₈-Alkylen-**^{L2}, *^{L2}-C₁₋₈-Alkylen-NR^{L2a}- **^{L2}, -C₁₋₈-Alkylen-, -C₂₋₈-Alkenylen-, -C₂₋₈-Alkinylen-, 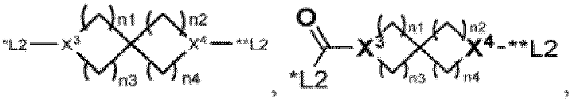 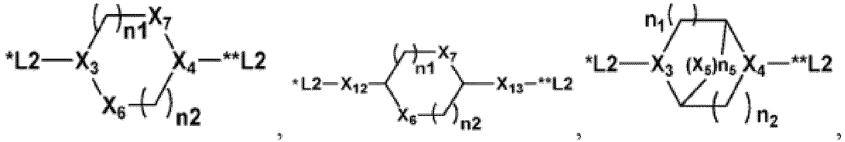 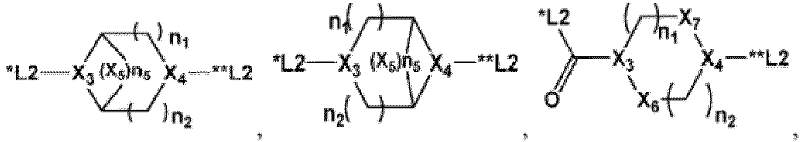 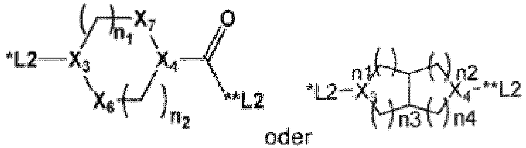 jeweils gegebenenfalls mit zumindest einem Substituenten R^{L2c} substituiert ist,
worin sich *^{L2} auf die an die Gruppierung 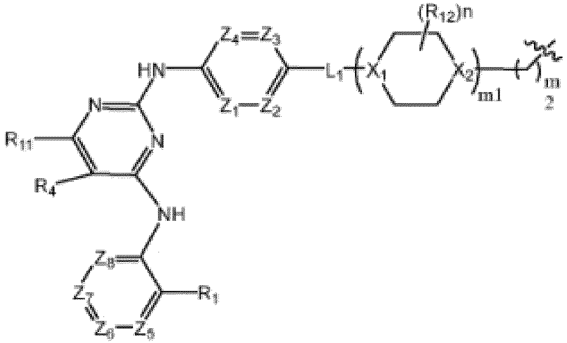 gebundene Position bezieht **und sich** **^{L2} auf die an die Gruppierung 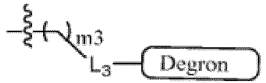 , gebundene Position bezieht,
worin R^{L2a} und R^{L2b} jeweils unabhängig voneinander aus Wasserstoff, -C₁₋₈-Alkyl, -C₂₋₈-Alkenyl, -C₂₋₈-Alkinyl, C₃₋₈-Cycloalkyl, 3- bis 8-gliedrigem Heterocyclyl, C₆₋₁₂-Aryl und 5- bis 12-gliedrigem Heteroaryl ausgewählt sind, wobei das -C₁₋₈-Alkyl, -C₂₋₈-Alkenyl, -C₂₋₈-Alkinyl, C₃₋₈-Cycloalkyl, 3- bis 8-gliedrige Heterocyclyl, C₆₋₁₂-Aryl oder 5- bis 12-gliedrige Heteroaryl jeweils gegebenenfalls mit zumindest einem Substituenten R^{L2d} substituiert ist,
worin R^{L2c} und R^{L2d} jeweils unabhängig voneinander Halogen, Hydroxy, -C₁₋₈-Alkyl, -C₁₋₈-Alkoxy, -C₁₋₈-Alkoxy-C₁₋₈-alkyl-, -C₂₋₈-Alkenyl, -C₂₋₈-Alkinyl, C₃₋₈-Cycloalkyl, 3- bis 8-gliedriges Heterocyclyl, C₆₋₁₂-Aryl, 5- bis 12-gliedriges Heteroaryl oder Oxo sind;
L³ aus einer Einfachbindung, -O-, -SO₂-, -CO-, -NR^{L3a}-, -C₃₋₈-Cycloalkylen-, *^{L3}-O-C₁₋₈-Alkylen-**^{L3}, *^{L3}-C₁₋₈-Alkylen-O-**^{L3}, *^{L3}-SO₂-C₁₋₈-Alkylen-**^{L3}, *^{L3}-C₁₋₈-Alkylen-SO₂-**^{L3}, *^{L3}-CO-C₁₋₈-Alkylen-**^{L3}, *^{L3}-C₁₋₈-Alkylen-CO-**^{L3}, *^{L3}-NR^{L3a}-C₁₋₈-Alkylen-**^{L3}, *^{L3}-C₁₋₈-Alkylen-NR^{L3a}-**^{L3}, *^{L3}-NR^{L3a}C(O)-**^{L3}, *^{L3}-C/O)NR^{L3a}-**^{L3}, -C₁₋₈-Alkylen-, -C₂₋₈-Alkenylen-, -C₂₋₈-Alkiny-**len-,** -[O(CR^{L3a}R^{L3b})ₘ₄]ₘ₅-, 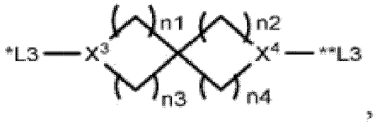 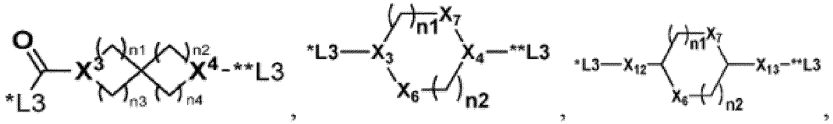 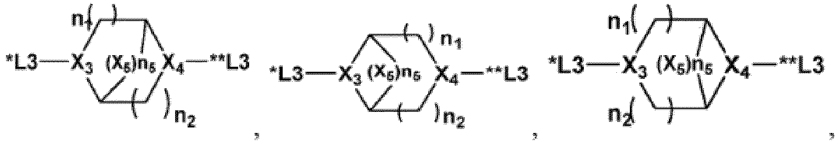 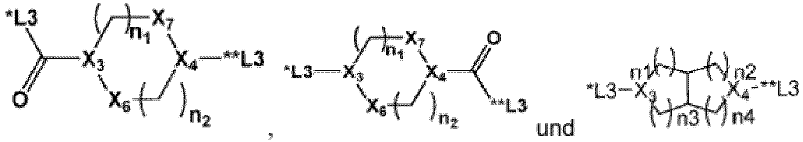 ausgewählt ist,
wobei das -C₃₋₈-Cycloalkylen-, *³-O-C₁₋₈-Alkylen-**^{L3}, *^{L3}-C₁₋₈-Alkylen-O-**^{L3}, *^{L3}-SO₂-C₁₋₈-Alkylen-**^{L3}, *^{L3}-C₁₋₈-Alkylen-SO₂-**^{L3}, *^{L3}-CO-C₁₋₈-Alkylen-**^{L3}, *^{L3}-C₁₋₈-Alkylen-CO-**^{L3}, *^{L3}-NR^{L3a}-C₁₋₈-Alkylen-**L³, *^{L3}-C₁₋₈-Alkylen-NR^{L3a}-**^{L3}, -C₁₋₈-Alkylen-, -C₂₋₈-Alkenylen-, -C₂₋₈-Alkinylen-, 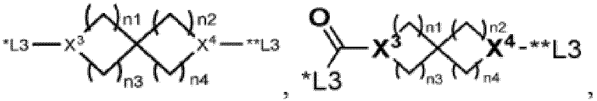 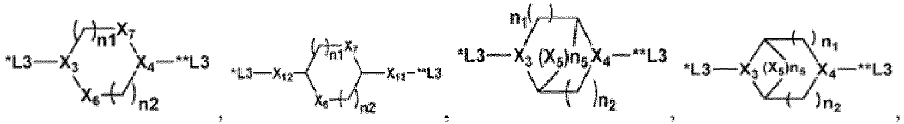 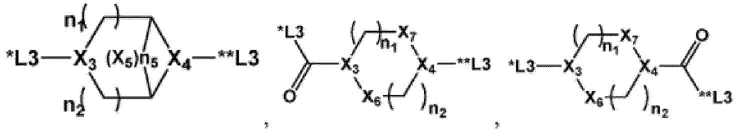 oder 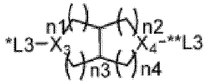 jeweils gegebenenfalls mit zumindest einem Substituenten R^{L3c} substituiert ist,
worin sich *^{L3} auf die an die Gruppierung 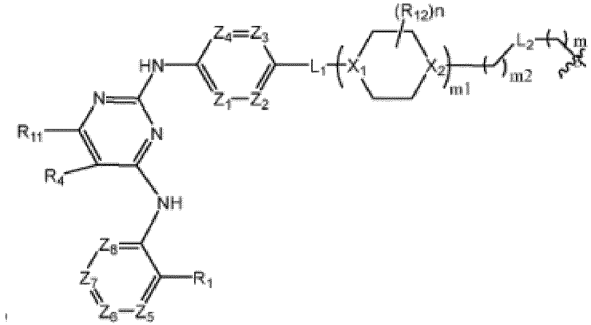 gebundene Position bezieht und sich **^{L3} auf die an die Gruppierung 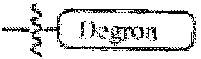 gebundene Position bezieht,
worin R^{L3a} und R^{L31} jeweils unabhängig voneinander aus Wasserstoff, -C₁₋₈-Alkyl, - C₂₋₈-Alkenyl, -C₂₋₈-Alkinyl, C₃₋₈-Cycloalkyl, 3- bis 8-gliedrigem Heterocyclyl, C₆₋₁₂-Aryl und 5- bis 12-gliedrigem Heteroaryl ausgewählt sind, wobei das -C₁₋₈-Alkyl, -C₂₋₈-Alkenyl, -C₂₋₈-Alkinyl, C₃₋₈-Cycloalkyl, 3- bis 8-gliedrige Heterocyclyl, C₆₋₁₂-Aryl oder 5- bis 12-gliedrige Heteroaryl jeweils gegebenenfalls mit zumindest einem Substituenten R^{L3d} substituiert ist,
worin R^{L3c} und L^{L3d} jeweils unabhängig voneinander Halogen, Hydroxy, -C₁₋₈-Alkyl, -C₁₋₈-Alkoxy, -C₁₋₈-Alkoxy-C₁₋₈-alkyl-, -C₂₋₈-Alkenyl, -C₂₋₈-Alkinyl, C₃₋₈-Cycloalkyl, 3- bis 8-gliedriges Heterocyclyl, C₆₋₁₂-Aryl, 5- bis 12-gliedriges Heteroaryl oder Oxo sind; 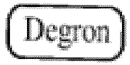 aus Folgendem ausgewählt ist: 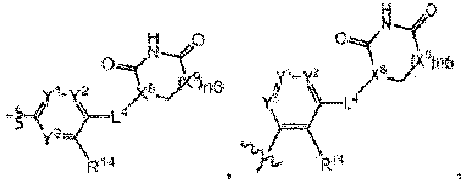 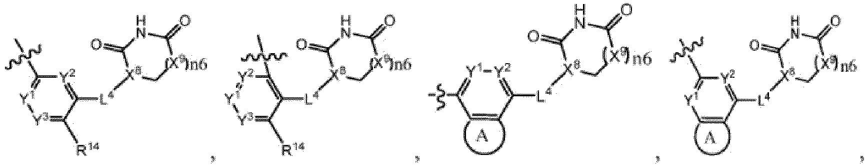 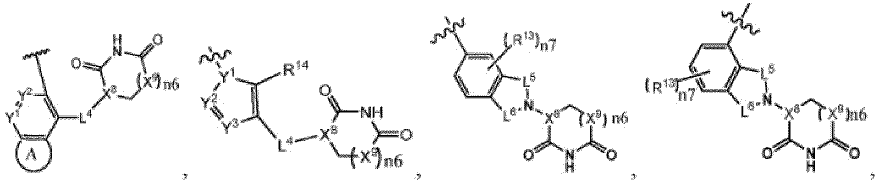 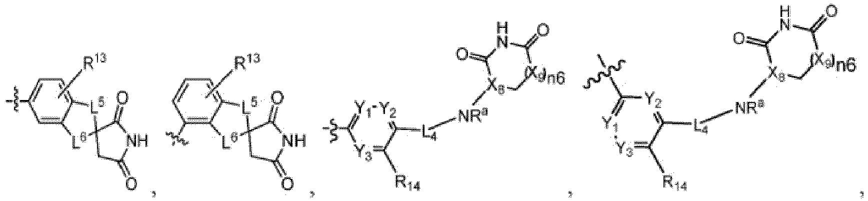 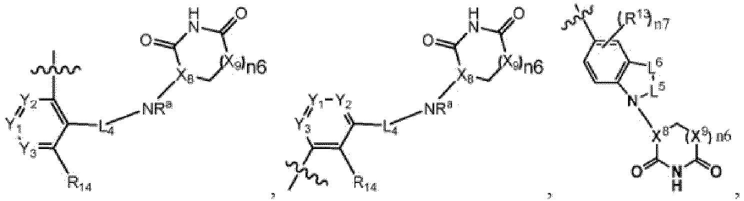 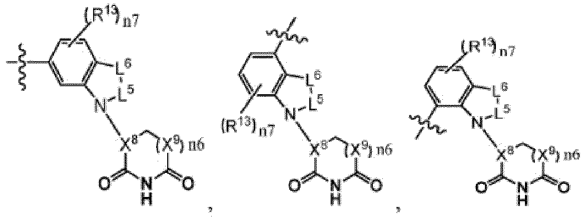 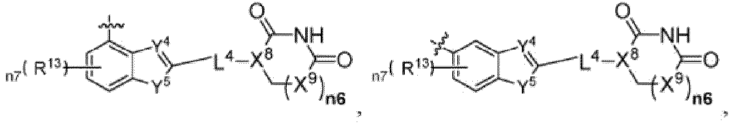 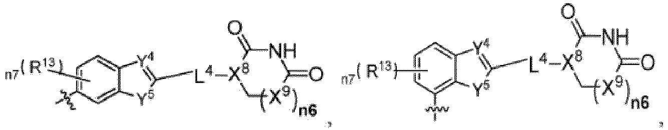 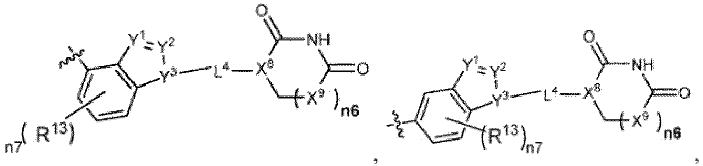 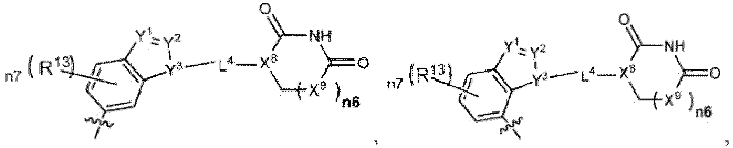 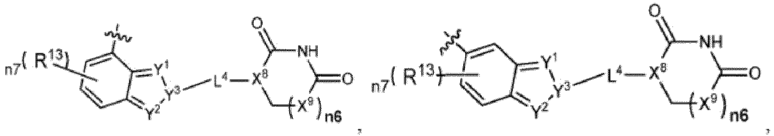 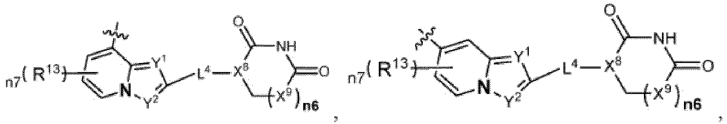 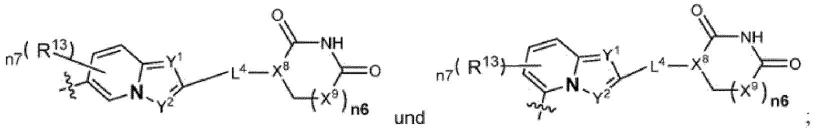
worin Ring A aus 3- bis 12-gliedrigem Cycloalkyl, 3- bis 12-gliedrigem Heterocyclyl, Aryl und Heteroaryl ausgewählt ist,
worin R¹³ und R¹⁴ jeweils unabhängig voneinander aus Wasserstoff, Halogen, CN, -C₁₋₈-Alkyl, -C₂₋₈-Alkenyl, -C₂₋₈-Alkinyl, -C₁₋₈-Alkoxy, C₃₋₈-Cycloalkyl, 3- bis 8-gliedrigem Heterocyclyl, C₆₋₁₂-Aryl und 5- bis 12-gliedrigem Heteroaryl ausgewählt sind, wobei das -C₁₋₈-Alkyl, -C₂₋₈-Alkenyl, -C₂₋₈-Alkinyl, -C₁₋₈-Alkoxy, C₃₋₈-Cycloalkyl, 3- bis 8-gliedrige Heterocyclyl, C₆₋₁₂-Aryl oder 5- bis 12-gliedrigen Heteroaryl jeweils gegebenenfalls mit zumindest einem Substituenten substituiert ist, der aus Halogen, -C₁₋₈-Alkyl, -C₁₋₈-Alkoxy-C₁₋₈-alkyl-, -C₂₋₈-Alkenyl, -C₂₋₈-Alkinyl, C₃₋₈-Cycloalkyl, 3- bis 8-gliedrigem Heterocyclyl, C₆₋₁₂-Aryl und 5- bis 12-gliedrigem Heteroaryl ausgewählt ist;
X¹, X², X³, X⁴ und X⁸ jeweils unabhängig voneinander aus -CR^{a} und N ausgewählt sind,
X⁵, X⁶, X⁷ und X⁹ jeweils unabhängig voneinander aus -NR^{a}-, -O-, -S- und -CR^{a}R^{b}-ausgewählt sind,
X¹² und X¹³ jeweils unabhängig voneinander aus -NR^{a}- und -O- ausgewählt sind,
L⁴, L⁵ und L⁶ jeweils unabhängig voneinander aus einer Einfachbindung, -O-, -NR^{a}-, -(CR^{a}R^{b})ₙ₈-, -O(CR^{a}R^{b}))ₙ₈-, -NR^{a}(CR^{a}R^{b})ₙ₈- und -C(O)- ausgewählt sind,
Y¹, Y², Y³ und Y⁴ jeweils unabhängig voneinander aus CR^{a} und N ausgewählt sind,
Y⁵ aus NR^{a}, O und S ausgewählt ist,
worin R^{a} und R^{b} jeweils unabhängig voneinander aus Wasserstoff (H, D oder T), Halogen, CN, -C₁₋₈-Alkyl, -C₁₋₈-Alkoxy, -C₂₋₈-Alkenyl, -C₂₋₈-Alkinyl, -C₃₋₈-Cycloalkyl, 3- bis 8-gliedrigem Heterocyclyl, -C₆₋₁₂-Aryl und 5- bis 12-gliedrigem Heteroaryl ausgewählt sind, wobei das -C₁₋₈-Alkyl, -C₁₋₈-Alkoxy, -C₂₋₈-Alkenyl, -C₂₋₈-Alkinyl, -C₃₋₈-Cycloalkyl, 3- bis 8-gliedrige Heterocyclyl, -C₆₋₁₂-Aryl oder 5- bis 12-gliedrige Heteroaryl jeweils gegebenenfalls mit zumindest einem Substituenten substituiert ist, der aus Halogen, Hydroxy, Halogen, -C₁₋₈-Alkyl, -C₁₋₈-Alkoxy, -C₂₋₈-Alkenyl, -C₂₋₈-Alkinyl, -C₃₋₈-Cycloalkyl, 3- bis 8-gliedrigem Heterocyclyl, -C₆₋₁₂-Aryl und 5- bis 12-gliedrigem Heteroaryl ausgewählt ist, oder
R^{a} und R^{b} zusammen mit den Kohlenstoffatomen, an die sie gebunden sind, einen 3-bis 12-gliedrigen Ring bilden, wobei der Ring 0 bis 3 Heteroatome umfasst, die jeweils unabhängig voneinander aus Stickstoff, Sauerstoff und Schwefel ausgewählt sind, wobei der Ring gegebenenfalls mit zumindest einem Substituenten substituiert ist, der aus Halogen, Hydroxy, -C₁₋₈-Alkyl, -C₂₋₈-Alkenyl, -C₂₋₈-Alkinyl, -C₁₋₈-Alkoxy, -C₂₋₈-Alkenyl, -C₂₋₈-Alkinyl, C₃₋₈-Cycloalkyl, 3- bis 8-gliedrigem Heterocyclyl, C₆₋₁₂-Aryl und 5- bis 12-gliedrigem Heteroaryl ausgewählt ist;
m₁ = 0 oder 1 ist;
m₂ und m₃ jeweils unabhängig voneinander 0, 1, 2, 3, 4, 5, 6, 7 oder 8 sind;
m₄ und m₅ jeweils unabhängig voneinander 0, 1, 2 oder 3 sind;
n, n₁, n₂, n₃, n₄ und n₅ jeweils unabhängig voneinander 0, 1, 2 oder 3 sind;
n₆ = 0, 1, 2, 3 oder 4 ist;
n₇ = 0, 1, 2 oder 3 ist; und
n₈ = 0, 1, 2, 3, 4, 5, 6, 7 oder 8 ist.

2. Verbindung nach Anspruch 1, worin R¹ -P(O)(CH₃)₂ ist.

3. Verbindung nach einem der Ansprüche 1 bis 2, worin
R² und R³ zusammen mit den Kohlenstoffatomen, an die sie gebunden sind, einen 5- oder 6-gliedrigen aromatischen Ring bilden, wobei der Ring 0, 1 oder 2 Heteroatome umfasst, die jeweils unabhängig voneinander aus Stickstoff, Sauerstoff und Schwefel ausgewählt sind, wobei der Ring gegebenenfalls mit zumindest einem Substituenten R^{2e} substituiert ist;
R^{2e} bei jedem Vorkommen jeweils unabhängig Wasserstoff, -F, -Cl, -Br, -I, Methyl, Ethyl, Propyl, Butyl, Pentyl, Hexyl, Heptyl, Octyl, Methoxy, Ethoxy, Propoxy, Butoxy, Pentoxy, Hexoxy, Heptoxy, Octoxy, Cyclopropyl, Cyclobutyl, Cyclopentyl, Cyclohexyl, Cycloheptyl, Cyclooctyl oder Oxo ist, wobei das Methyl, Ethyl, Propyl, Butyl, Pentyl, Hexyl, Heptyl, Octyl, Methoxy, Ethoxy, Propoxy, Butoxy, Pentoxy, Hexoxy, Heptoxy, Octoxy, Cyclopropyl, Cyclobutyl, Cyclopentyl, Cyclohexyl, Cycloheptyl oder Cyclooctyl jeweils gegebenenfalls mit zumindest einem Substituenten R^{2d} substituiert ist; und
R^{2d} bei jedem Vorkommen jeweils unabhängig -F, -Cl, Br, -I, -OH, Methyl, Ethyl, Propyl, Butyl, Pentyl, Hexyl, Heptyl, Octyl, Methoxy, Ethoxy, Propoxy, Butoxy, Pentoxy, Hexoxy, Heptoxy, Octoxy, Cyclopropyl, Cyclobutyl, Cyclopentyl, Cyclohexyl, Cycloheptyl, Cyclooctyl oder Phenyl ist.

4. Verbindung nach einem der Ansprüche 1 bis 3, worin die Gruppierung 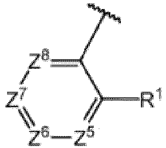 Folgendes ist: 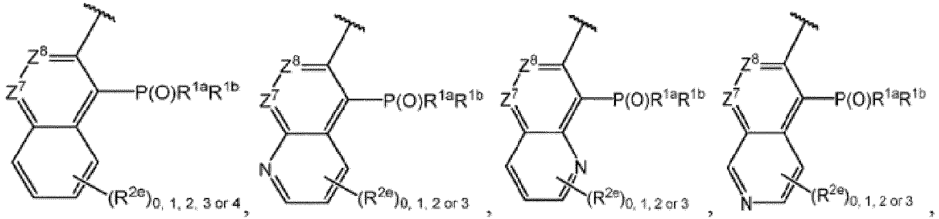 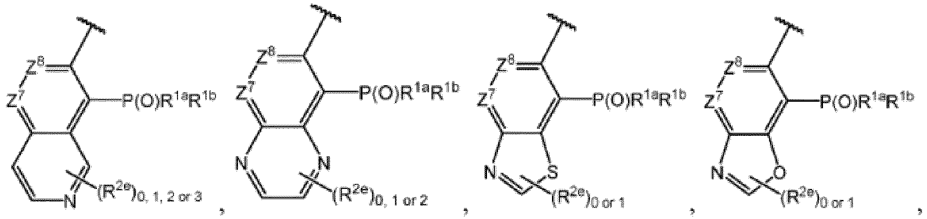 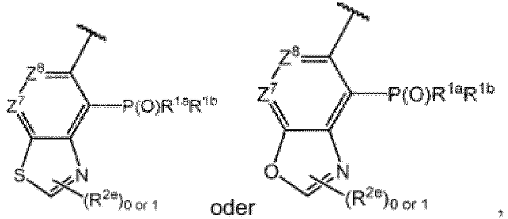 worin R^{2e}, Z⁷, Z⁸, R^{1a} und R^{1b} wie in Anspruch 1 definiert sind.

5. Verbindung nach einem der Ansprüche 1 bis 4, worin:
(i) R^{2e} bei jedem Vorkommen jeweils unabhängig Wasserstoff, -F, -Cl, -Br, -I, -CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CF₃, 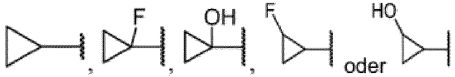 ist; und/oder
(ii) die Gruppierung 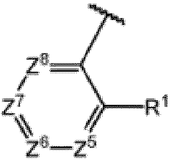 Folgendes ist:

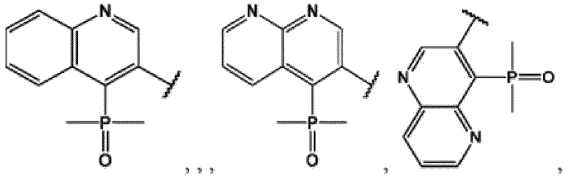

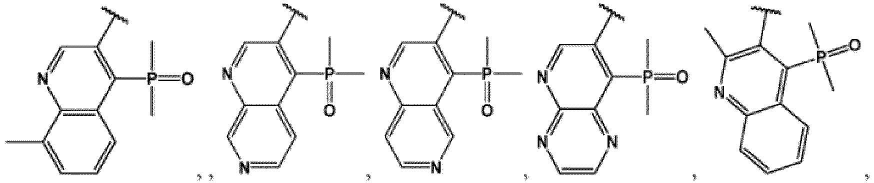

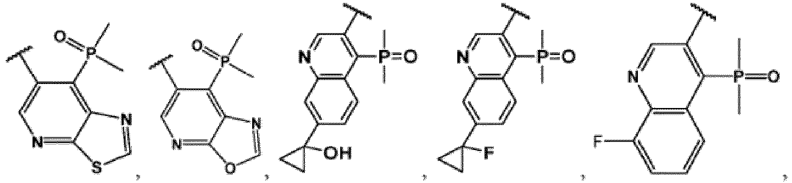

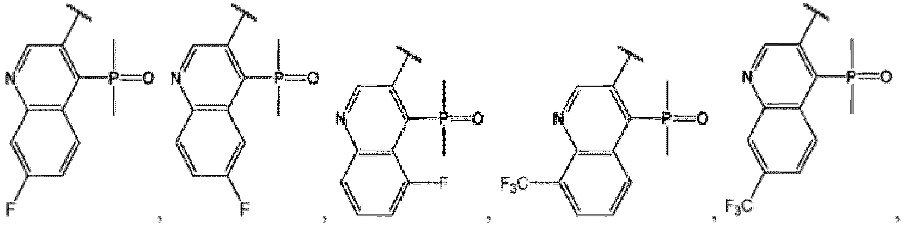

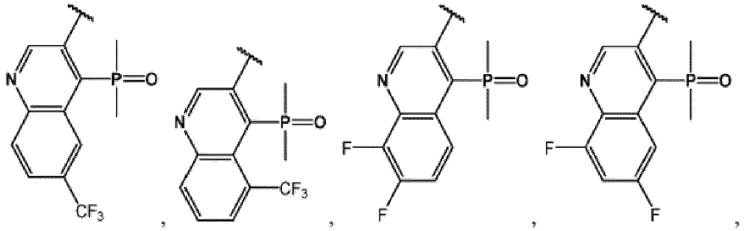

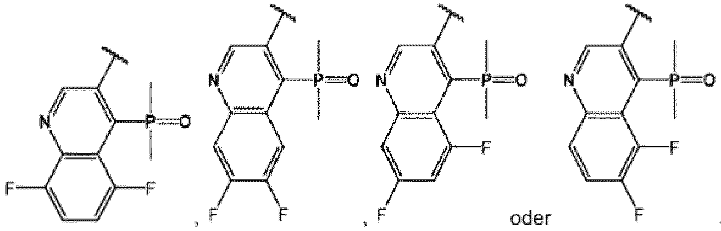

6. Verbindung nach einem der Ansprüche 1 bis 5, worin:
R¹⁰, R¹¹ und R¹² jeweils unabhängig voneinander aus Wasserstoff, F, Cl, Br, -NH₂, -CH₃, -C₂H₅, -C₃H₇, -CH₂F, -CHF₂, -CF₃, -C₄H₉, -C₅H₁₁, -OCH₃, -OC₂H₅, -OC₃H₇, -OC₄H₉, -OC₅H₁₁, -CN, Cyclopropyl und Oxo ausgewählt sind oder
zwei R¹² zusammen mit den Kohlenstoffatomen, an die sie gebunden sind, einen 3-, 4-, 5-, 6-, 7- oder 8-gliedrigen Ring bilden, wobei der Ring 0, 1, 2 oder 3 Heteroatome umfasst, die jeweils unabhängig voneinander aus Stickstoff, Sauerstoff und Schwefel ausgewählt sind, wobei der Ring gegebenenfalls mit zumindest einem Substituenten -H, -F, -Cl, Br, -I, Methyl, Ethyl, Propyl, Butyl, -NH₂, -NHCH₃, -OH, -OCH₃, -OC₂H₅, Cyclopropyl, Cyclobutyl, Cyclopentyl oder Cyclohexyl substituiert ist.

7. Verbindung nach einem der Ansprüche 1 bis 6, worin R⁴ aus -H, -F, -Cl, -Br, -I, -CH₃, -C₂H₅, -C₃H₇, -C₄H₉, Cyclopropyl und Cyclobutyl ausgewählt ist, wobei das -CH₃, -C₂H₅, -C₃H₇, -C₄H₉, Cyclopropyl oder Cyclobutyl gegebenenfalls mit -F, -Cl, -Br, -I, -C₁₋₈-Alkyl, -C₂₋₈-Alkenyl, -C₂₋₈-Alkinyl, -C₃₋₈-Cycloalkyl, 3- bis 8-gliedrigem Heterocyclyl, C₆₋₁₂-Aryl oder 5- bis 12-gliedrigem Heteroaryl, Oxo, -CN, -OR^{4c}, -SO₂R^{4c}, -SO₂NR^{4c}R^{4d}, -COR^{4c}, -CO₂R^{4c}, -CO-NR^{4c}R^{4d}, -NR^{4c}R^{4d}, -NR^{4c}COR^{4d}, -NR^{4c}CO₂R^{4d} oder -NR^{4c}SO₂R^{4d} substituiert ist,
worin R^{4c} und R^{4d} jeweils unabhängig voneinander Wasserstoff, -C₁₋₈-Alkyl, -C₂₋₈-Alkenyl, -C₂₋₈-Alkinyl, C₃₋₈-Cycloalkyl, 3- bis 8-gliedriges Heterocyclyl, C₆₋₁₂-Aryl oder 5- bis 12-gliedriges Heteroaryl sind.

8. Verbindung nach einem der Ansprüche 1 bis 7, worin:
(i) L¹ aus einer Einfachbindung, -O-, -SO₂-, -C(O)-, -NR^{L1a}-, -C₃₋₈-Cycloalkylen-, *^{L1}-O-C₁₋₈-Alkylen-**^{L1}, *^{L1}-C₁₋₈-Alkylen-O-**^{L1}, *^{L1}-SO₂-C₁₋₈-Alkylen-**¹, *^{L1}-C₁₋₈-Alkylen-SO₂-**^{L1}, *^{L1}-CO-C₁₋₈-Alkylen-**^{L1}, *^{L1}-C₁₋₈-Alkylen-CO-**^{L1}, *^{L1}-NR^{L1a}-C₁₋₈-Alkylen-**^{L1}, *^{L1}-C₁₋₈-Alkylen-NR^{L1a}-**^{L1}, *^{L1}-NR^{L1a}C(O)-**^{L1}, *^{L1}-C(O)NR^{L1a}-**^{L1}, -C₁₋₈-Alkylen-, -C₂₋₈-Alkenylen-, C₂₋₈-Alkiny-len, -[O(CR^{L1a}R^{L1b})ₘ₄]ₘ₅-, 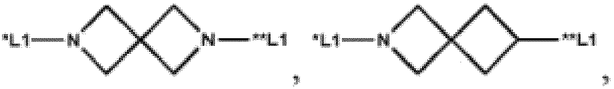 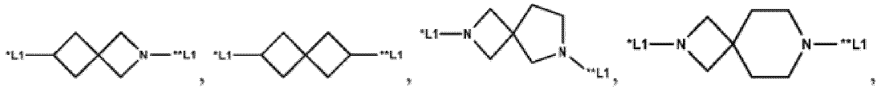 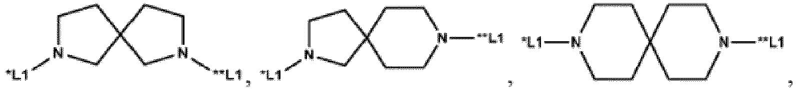 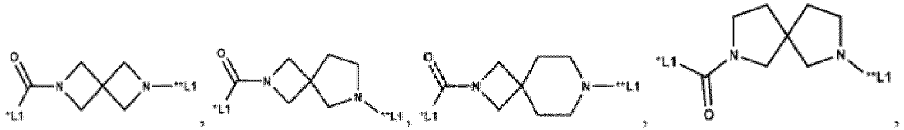 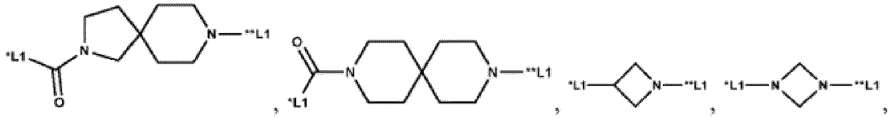 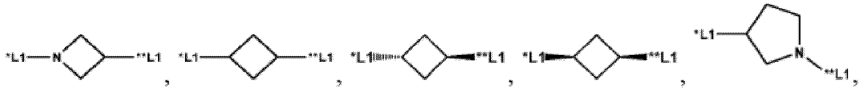 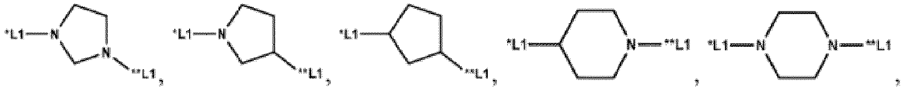 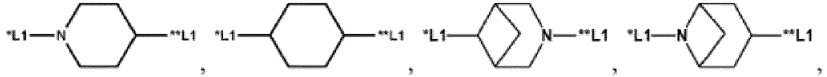 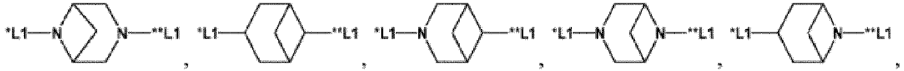 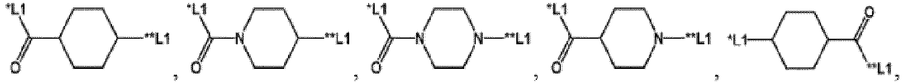 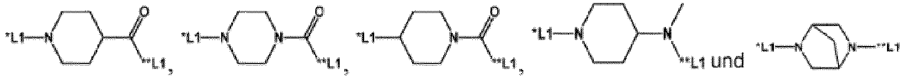 ausgewählt ist,
wobei das -C₃₋₈-Cycloalkylen-, *^{L1}-O-C₁₋₈-Alkylen-**^{L1}, *^{L1}-C₁₋₈-Alkylen-O-**^{L1}, *^{L1}-SO₂-C₁₋₈-Alkylen-**^{L1}, *^{L1}-C₁₋₈-Alkylen-SO₂-**^{L1}, *^{L1}-CO-C₄₋₈-Alkylen-**^{L1}, *^{L1}-C₁₋₈-Alkylen-CO-**^{L1}, *^{L1}-NR^{L1a}-C₁₋₈-Alkylen-**^{L1}, *^{L1}-C₁₋₈-Alkylen-NR^{L1a}-**^{L1}, -C₁₋₈-Alkylen-, -C₂₋₈-Alkenylen-, C₂₋₈-Alkinylen, 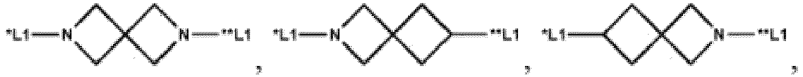 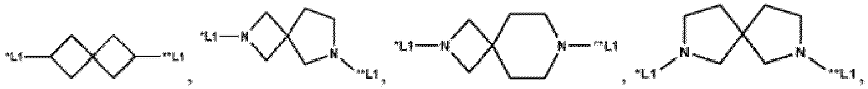 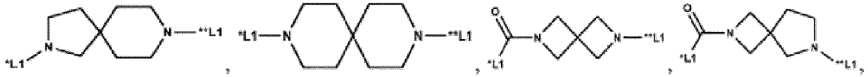 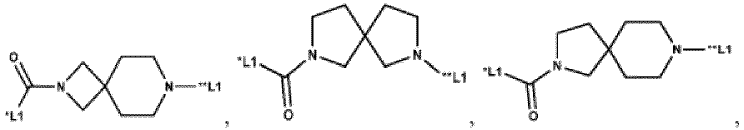 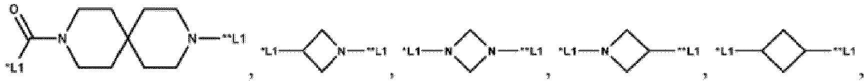 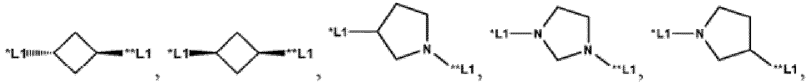 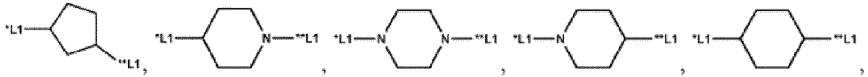 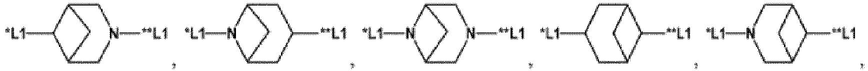 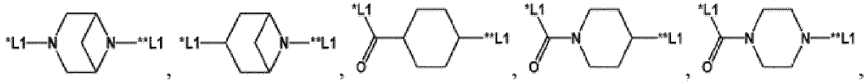 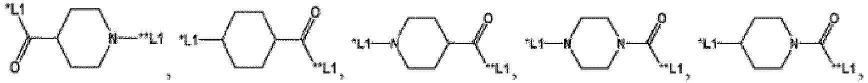 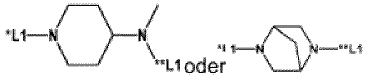 jeweils gegebenenfalls mit zumindest einem R^{L1c} substituiert ist,
worin R^{L1a} und R^{L1b} jeweils unabhängig voneinander aus Wasserstoff, Methyl, Ethyl, Propyl, Butyl, Pentyl, Hexyl, Heptyl, Octyl, Vinyl, Ethinyl, Cyclopropyl, Cyclobutyl, Cyclopentyl, Cyclohexyl, Cycloheptyl, Cyclooctyl, Oxazolidinyl, Imidazolidinyl, Thiazolidinyl, Pyrazolidinyl, Morpholinyl, Piperidinyl, Piperazinyl, Oxazinyl, Imidazolyl, Thiazolyl, Oxazolyl, Pyridyl, Pyrimidinyl, Pyrazinyl, Pyridazinyl, Phenyl, Pyrrolyl, Imidazolyl, Pyrazolyl, Thiazolyl, Oxazolyl, Triazolyl, Thiophenyl, Furanyl, Pyridyl, Pyrimidinyl und Pyrazinyl ausgewählt sind, wobei das Methyl, Ethyl, Propyl, Butyl, Pentyl, Hexyl, Heptyl, Octyl, Vinyl, Ethinyl, Cyclopropyl, Cyclobutyl, Cyclopentyl, Cyclohexyl, Cycloheptyl, Cyclooctyl, Oxazolidinyl, Imidazolidinyl, Thiazolidinyl, Pyrazolidinyl, Morpholinyl, Piperidinyl, Piperazinyl, Oxazinyl, Imidazolyl, Thiazolyl, Oxazolyl, Pyridyl, Pyrimidinyl, Pyrazinyl, Pyridazinyl, Phenyl, Pyrrolyl, Imidazolyl, Pyrazolyl, Thiazolyl, Oxazolyl, Triazolyl, Thiophenyl, Furanyl, Pyridyl, Pyrimidinyl oder Pyrazinyl jeweils gegebenenfalls mit zumindest einem Substituenten R^{L1d} substituiert ist und
worin R^{L1c} und R^{L1d} jeweils unabhängig voneinander -F, -Cl, Br, -I, -OH, Methyl, Ethyl, Propyl, Butyl, Pentyl, Hexyl, Heptyl, Octyl, Vinyl, Ethinyl, Methoxy, Ethoxy, Propoxy, Butoxy, Pentoxy, Hexoxy, Heptoxy, Octoxy, Cyclopropyl, Cyclobutyl, Cyclopentyl, Cyclohexyl, Cycloheptyl, Cyclooctyl, Oxazolidinyl, Imidazolidinyl, Thiazolidinyl, Pyrazolidinyl, Morpholinyl, Piperidinyl, Piperazinyl, Oxazinyl, Imidazolyl, Thiazolyl, Oxazolyl, Pyridyl, Pyrimidinyl, Pyrazinyl, Pyridazinyl, Phenyl, Pyrrolyl, Imidazolyl, Pyrazolyl, Thiazolyl, Oxazolyl, Triazolyl, Thiophenyl, Furanyl, Pyridyl, Pyrimidinyl, Pyrazinyl oder Oxo sind;
oder
(ii) L¹ aus einer Einfachbindung, -C₁₋₈-Alkylen- (vorzugsweise -CH₂-, -C₂H₄-, -C₃H₆-), -CO-, -O-, -N(CH₃)-, -NH-, 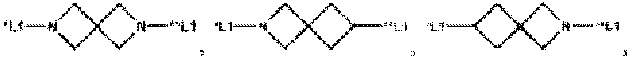 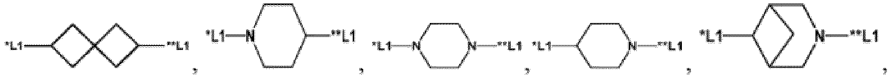 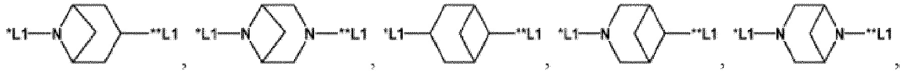 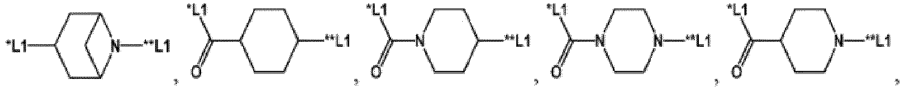 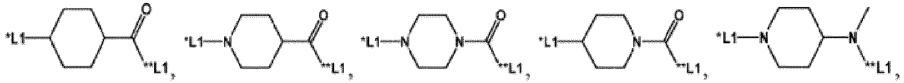 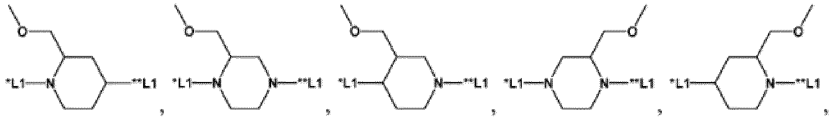 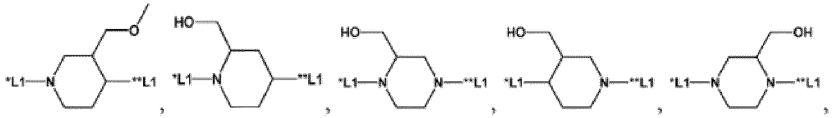 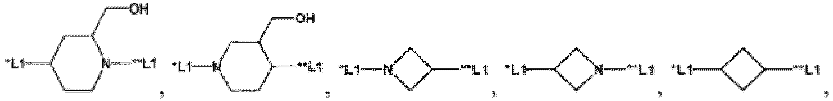 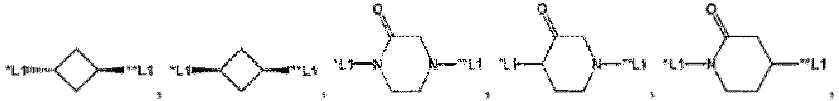 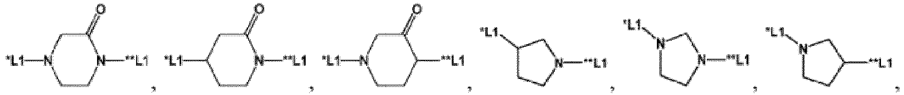 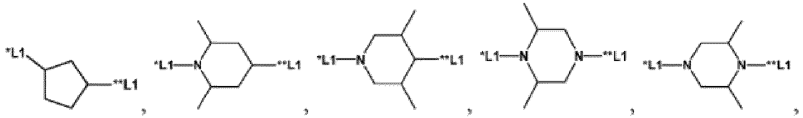 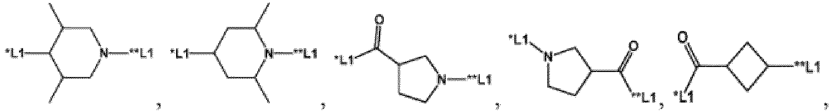 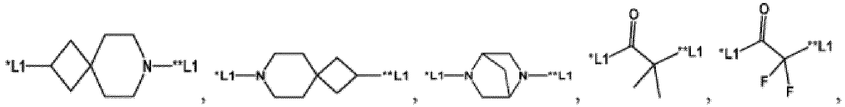 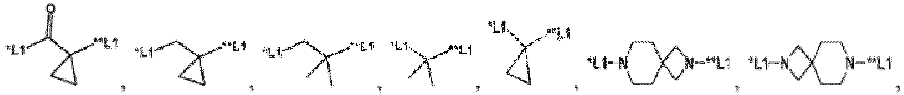 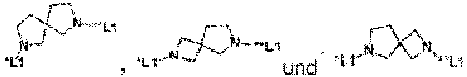 ausgewählt ist.

9. Verbindung nach einem der Ansprüche 1 bis 8, worin:
(i) X¹ und X² jeweils unabhängig aus -CR^{a} und N ausgewählt sind, worin R^{a} aus Wasserstoff, -F, -Cl, Br, -I, CN, Methyl, Ethyl, Methoxy, Ethoxy und Cyclopropyl ausgewählt ist, wobei das Methyl, Ethyl, Methoxy, Ethoxy oder Cyclopropyl jeweils gegebenenfalls mit zumindest einem Substituenten -F, -Cl, Br, -I, Hydroxy, Methyl oder Ethyl substituiert ist (wobei X¹ und X² vorzugsweise jeweils unabhängig aus CH, C(F), C(CH₃) oder N ausgewählt sind);
m₁ = 1 oder 0 ist; und
R¹² Wasserstoff, Oxo, Methoxymethyl, Hydroxymethyl, -CN oder -CH₃ ist;
und/oder
(ii) m1 = 1 ist, wobei die Gruppierung 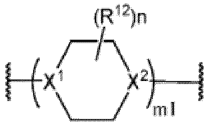 vorzugsweise 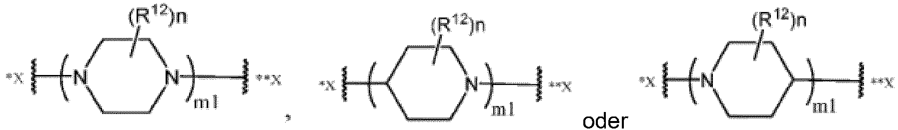 ist, worin sich *^{X} auf an die Gruppierung 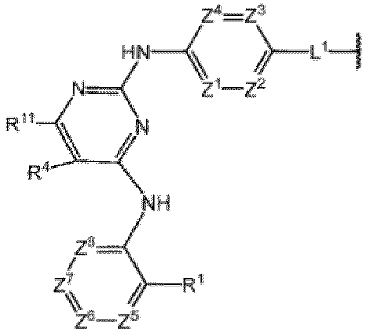 gebundene Position bezieht und sich **^{X} auf die an die Gruppierung 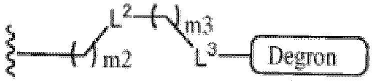 gebundene Position bezieht; und/oder
(iii) m₁ = 0 ist.

10. Verbindung nach einem der Ansprüche 1 bis 9, worin:
(i) die Gruppierung 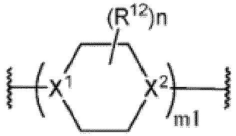 Folgendes ist: 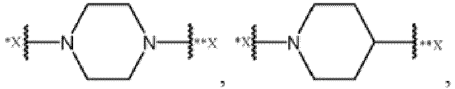 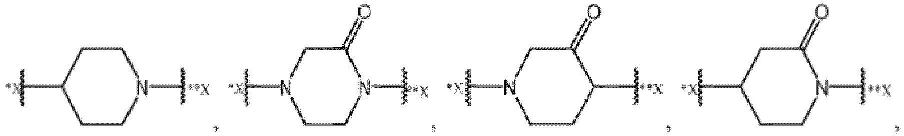 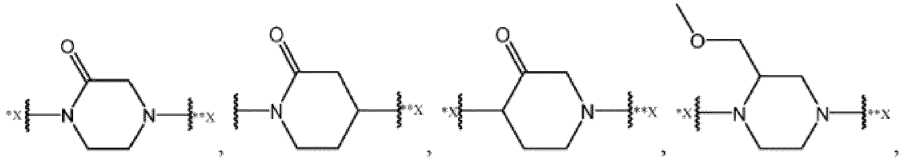 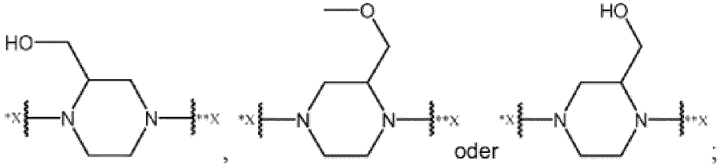 oder
(ii) m₂ aus 0, 1, 2, 3, 4 und 5 ausgewählt ist;
oder
(iii) L² aus einer Einfachbindung, -O-, -SO₂-, -CO-, -NR^{L2a}-, -C₃₋₈-Cycloalkylen-, *^{L2}-O-C₁₋₈-Alkylen-**^{L2}, *^{L2}-C₁₋₈-Alkylen-O-**^{L2}, *^{L2}-SO₂-C₁₋₈-Alkylen-**^{L2}, *^{L2}-C₁₋₈-Alkylen-SO₂-**^{L2}, *^{L2}-CO-C₁₋₈-Alkylen-**^{L2}, *L²-C₁₋₈-Alkylen-CO-**L², *^{L2}-NR^{L2a}-C₁₋₈-Alkylen-**^{L2}, *^{L2}-C₁₋₈-Alkylen-NR^{L2a}-**^{L2}, *^{L2}-NR^{L2a}C(O)-**^{L2} *^{L2}-C(O)NR^{L2a}-**^{L2}, -C₁₋₈-Alkylen-, -C₂₋₈-Alkenylen-, -C₂₋₈-Alkinylen-, -[O(CR^{L2a}R^{L2b})ₘ₄]ₘ₅-, 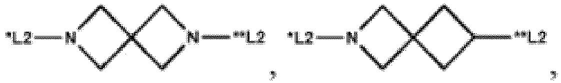 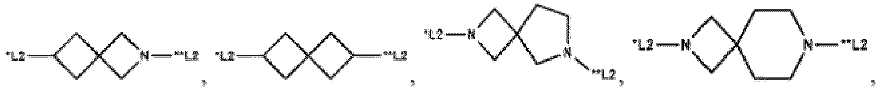 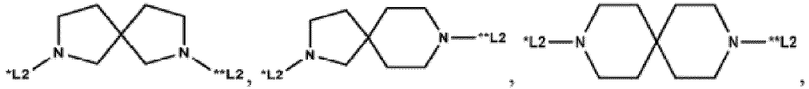 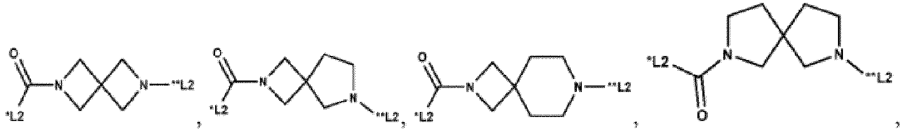 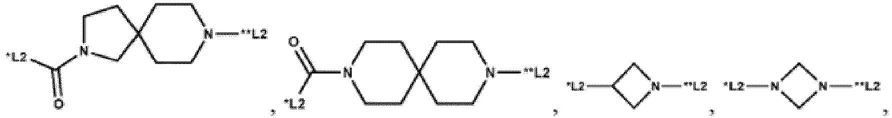 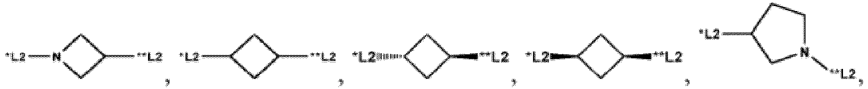 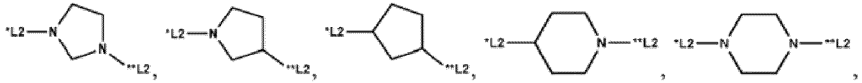 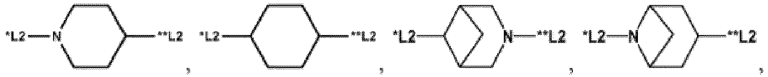 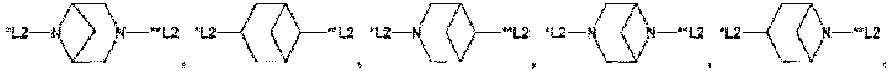 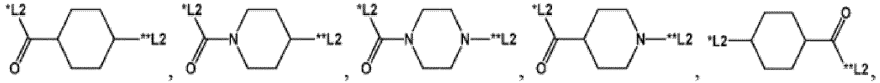 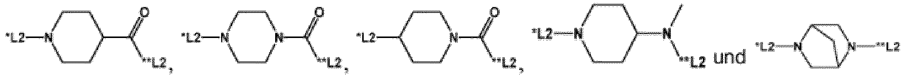 ausgewählt ist,
worin das -C₃₋₈-Cycloalkylen-, *^{L2}-O-C₁₋₈-Alkylen-**^{L2}, *^{L2}-C₁₋₈-Alkylen-O-**^{L2}, *^{L2}-SO₂-C₁₋₈-Alkylen-**^{L2}, *^{L2}-C₁₋₈-Alkylen-SO₂-**^{L2}, *^{L2}-CO-C₁₋₈-Alkylen-**^{L2}, *L²-C₁₋₈-Alkylen-CO-**L², *^{L2}-NR^{L2a}-C₁₋₈-Alkylen-**^{L2}, *^{L2}-C₁₋₈-Alkylen-NR^{L2a}-**^{L2}, -C₁₋₈-Alkylen-, -C₂₋₈-Alkenylen-, -C₂₋₈-Alkinylen-, 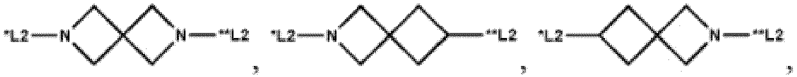 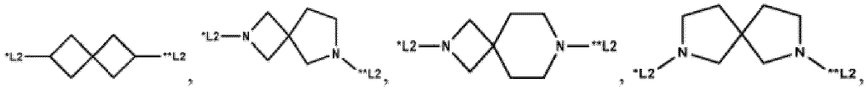 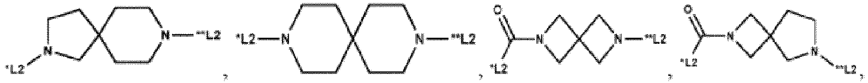 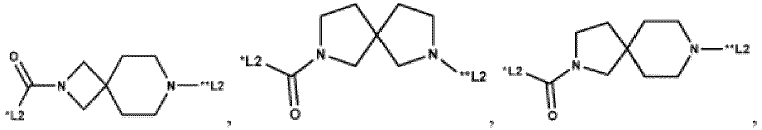 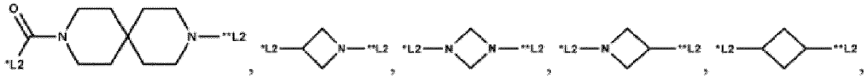 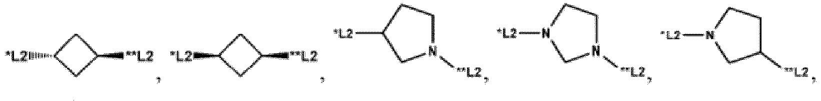 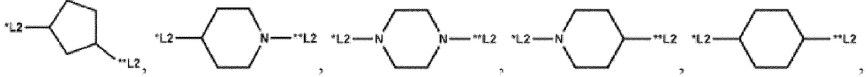 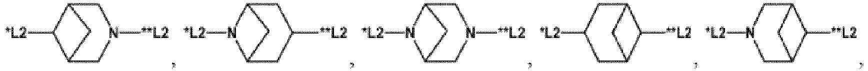 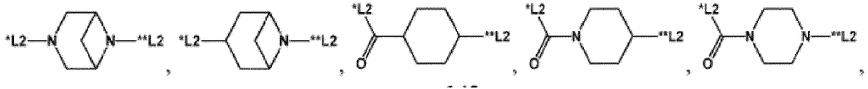 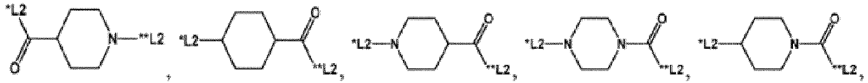 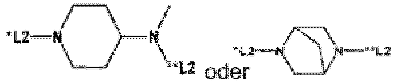 jeweils gegebenenfalls mit zumindest einem Substituenten R^{L2c} substituiert ist,
worin R^{L2a} und R^{L2b} jeweils unabhängig voneinander aus Wasserstoff, Methyl, Ethyl, Propyl, Butyl, Pentyl, Hexyl, Heptyl, Octyl, Vinyl, Ethinyl, Cyclopropyl, Cyclobutyl, Cyclopentyl, Cyclohexyl, Cycloheptyl, Cyclooctyl, Oxazolidinyl, Imidazolidinyl, Thiazolidinyl, Pyrazolidinyl, Morpholinyl, Piperidinyl, Piperazinyl, Oxazinyl, Imidazolyl, Thiazolyl, Oxazolyl, Pyridyl, Pyrimidinyl, Pyrazinyl, Pyridazinyl, Phenyl, Pyrrolyl, Imidazolyl, Pyrazolyl, Thiazolyl, Oxazolyl, Triazolyl, Thiophenyl, Furanyl, Pyridyl, Pyrimidinyl und Pyrazinyl ausgewählt sind, wobei das Methyl, Ethyl, Propyl, Butyl, Pentyl, Hexyl, Heptyl, Octyl, Vinyl, Ethinyl, Cyclopropyl, Cyclobutyl, Cyclopentyl, Cyclohexyl, Cycloheptyl, Cyclooctyl, Oxazolidinyl, Imidazolidinyl, Thiazolidinyl, Pyrazolidinyl, Morpholinyl, Piperidinyl, Piperazinyl, Oxazinyl, Imidazolyl, Thiazolyl, Oxazolyl, Pyridyl, Pyrimidinyl, Pyrazinyl, Pyridazinyl, Phenyl, Pyrrolyl, Imidazolyl, Pyrazolyl, Thiazolyl, Oxazolyl, Triazolyl, Thiophenyl, Furanyl, Pyridyl, Pyrimidinyl oder Pyrazinyl jeweils gegebenenfalls mit zumindest einem Substituenten R^{L2d} substituiert ist, und
worin R^{L2c} und R^{L2d} jeweils unabhängig voneinander -F, -Cl, Br, -I, -OH, Methyl, Ethyl, Propyl, Butyl, Pentyl, Hexyl, Heptyl, Octyl, Vinyl, Ethinyl, Methoxy, Ethoxy, Propoxy, Butoxy, Pentoxy, Hexoxy, Heptoxy, Octoxy, Cyclopropyl, Cyclobutyl, Cyclopentyl, Cyclohexyl, Cycloheptyl, Cyclooctyl, Oxazolidinyl, Imidazolidinyl, Thiazolidinyl, Pyrazolidinyl, Morpholinyl, Piperidinyl, Piperazinyl, Oxazinyl, Imidazolyl, Thiazolyl, Oxazolyl, Pyridyl, Pyrimidinyl, Pyrazinyl, Pyridazinyl, Phenyl, Pyrrolyl, Imidazolyl, Pyrazolyl, Thiazolyl, Oxazolyl, Triazolyl, Thiophenyl, Furanyl, Pyridyl, Pyrimidinyl, Pyrazinyl oder Oxo sind.

11. Verbindung nach einem der Ansprüche 1 bis 10, worin:
(i) L² aus einer Einfachbindung, -C₁₋₈-Alkylen- (vorzugsweise -CH₂-, C₂H₄-, -C₃H₆-), -CO-, -O-, -N(CH₃)-, -NH-, 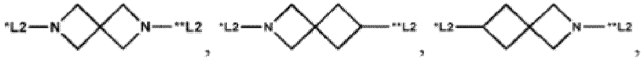 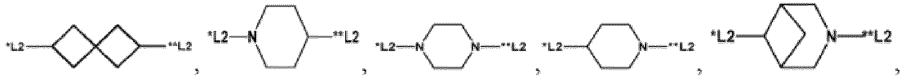 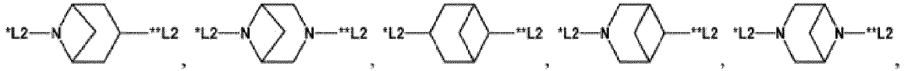 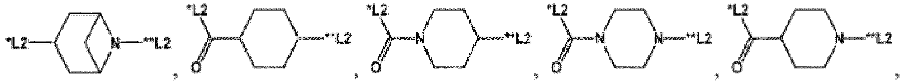 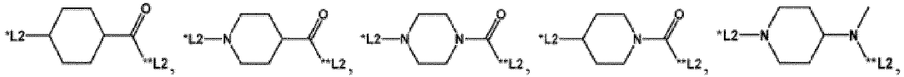 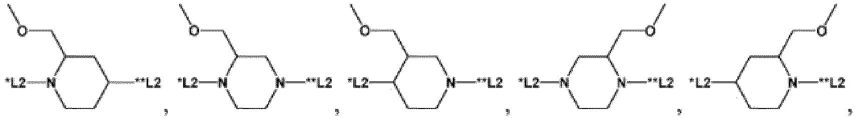 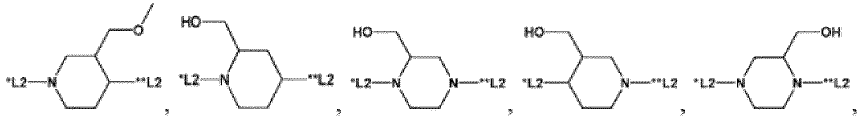 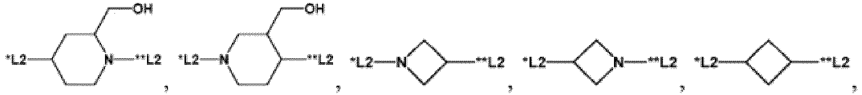 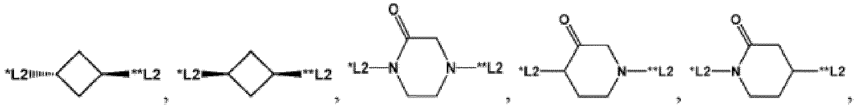 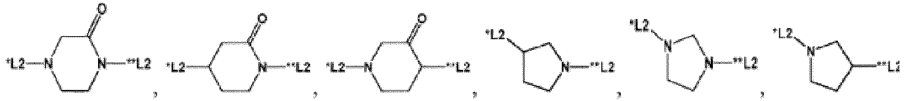 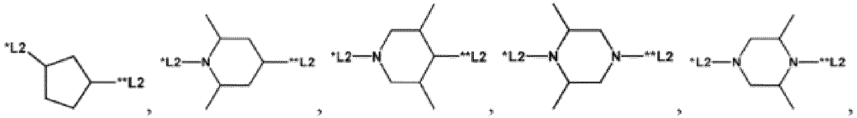 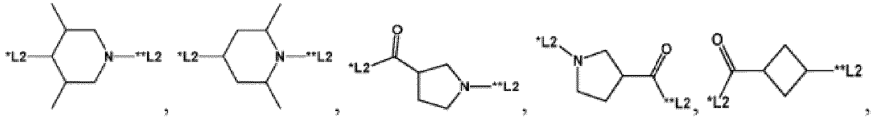 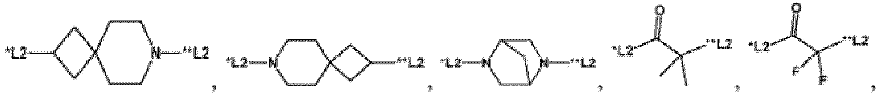 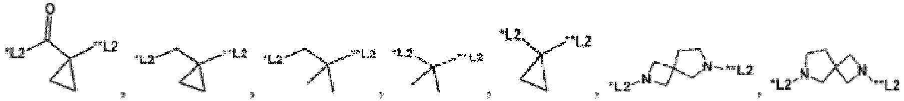 und 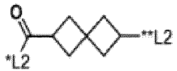 ausgewählt ist;
und/oder
(ii) m₃ = 0, 1, 2, 3, 4, 5 oder 6 ist;
und/oder
(iii) L³ aus einer Einfachbindung, -O-, -SO₂-, -CO-, -NR^{3a}-, -C₃₋₈-Cycloalkylen-, *^{L3}-O-C₁₋₈-Alkylen-**^{L3}, *^{L3}-C₁₋₈-Alkylen-O-**^{L3}, *^{L3}-SO₂-C₁₋₈-Alkylen-**^{L3}, *^{L3}-C₁₋₈-Alkylen-SO₂-**^{L3}, *^{L3}-CO-C₁₋₈-Alkylen-**^{L3}, *L³-C₁₋₈-Alkylen-CO-**^{L3}, *^{L3}-NR^{L3a}-C₁₋₈-Alkylen**^{L3}, *^{L3}-C₁₋₈-Alkylen-NR^{L3a}-**^{L3}, *^{L3}-NR^{L3a}C(O)-**^{L3}, *^{L3}-C(O)NR^{L3a}-**^{L3}, -C₁₋₈-Alkylen-, -C₂₋₈-Alkenylen-, -C₂₋₈-Alkiny-len-, -[O(CRL^{3a}RL^{3b})ₘ₄]ₘ₅-, 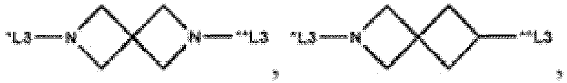 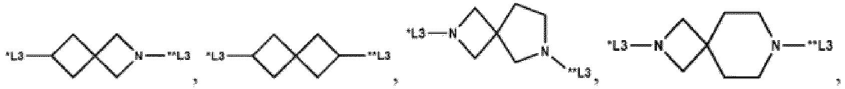 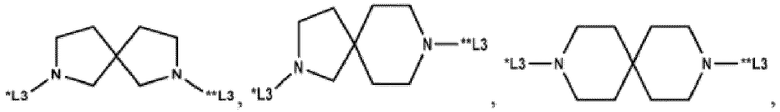 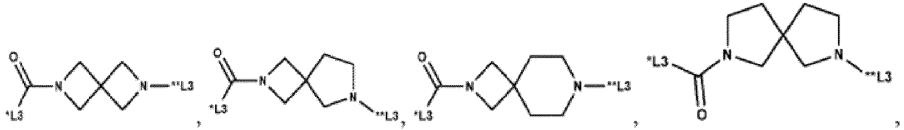 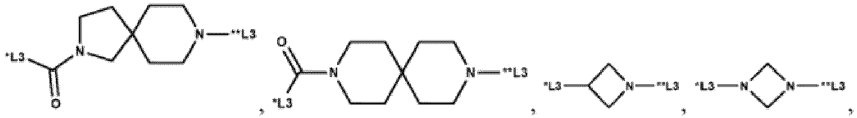 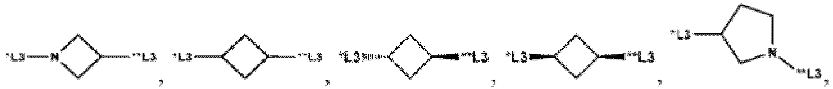 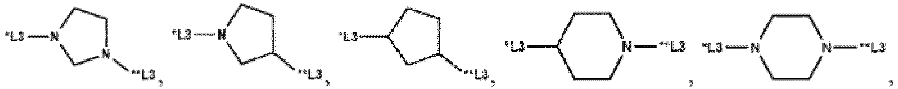 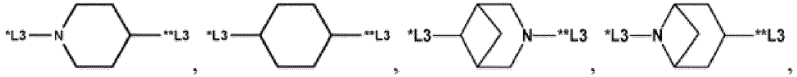 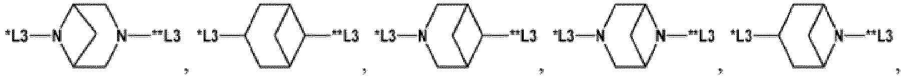 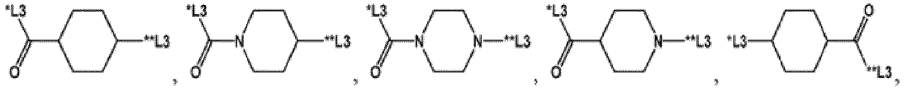 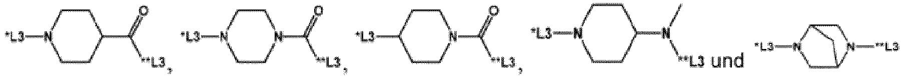 ausgewählt ist,
wobei das -C₃₋₈-Cycloalkylen-, *³-O-C₁₋₈-Alkylen-**^{L3}, *^{L3}-C₁₋₈-Alkylen-O-**^{L3}, *^{L3}-SO₂-C₁₋₈-Alkylen-**^{L3}, *^{L3}-C₁₋₈-Alkylen-SO₂-**^{L3}, *^{L3}-CO-C₁₋₈-Alkylen-**^{L3}, *^{L3}-C₁₋₈-Alkylen-CO-**^{L3}, *^{L3}-NR^{L3a}-C₁₋₈-Alkylen-**^{L3}, *L³-C₁₋₈-Alkylen-NR^{L3a}-**^{L3}, -C₁₋₈-Alkylen-, -C₂₋₈-Alkenylen-, -C₂₋₈-Alkinylen-, 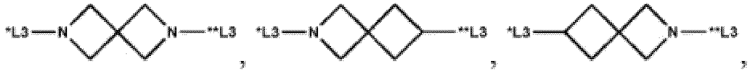 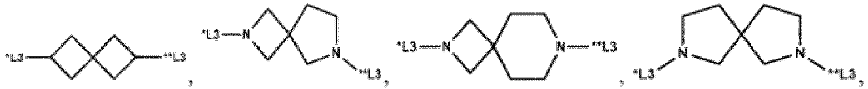 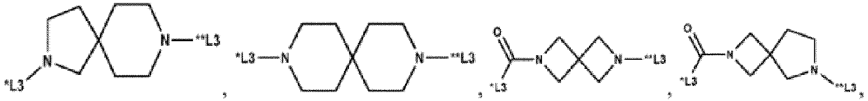 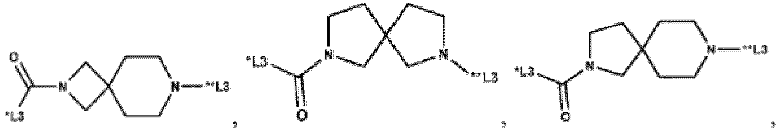 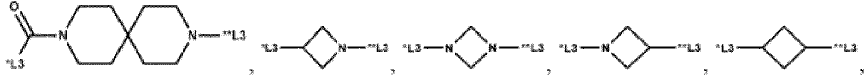 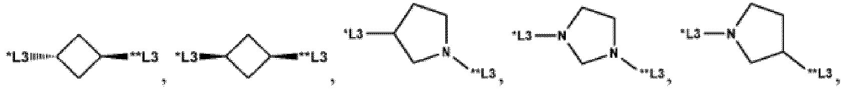 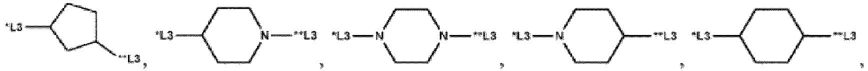 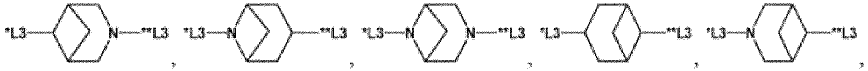 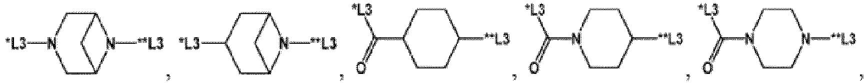 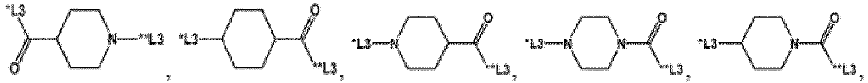 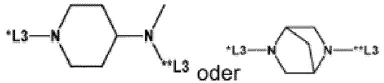 jeweils gegebenenfalls mit zumindest einem Substituenten R^{L3c} substituiert ist,
worin R^{L3a} und R^{L3b} jeweils unabhängig voneinander aus Wasserstoff, Methyl, Ethyl, Propyl, Butyl, Pentyl, Hexyl, Heptyl, Octyl, Vinyl, Ethinyl, Cyclopropyl, Cyclobutyl, Cyclopentyl, Cyclohexyl, Cycloheptyl, Cyclooctyl, Oxazolidinyl, Imidazolidinyl, Thiazolidinyl, Pyrazolidinyl, Morpholinyl, Piperidinyl, Piperazinyl, Oxazinyl, Imidazolyl, Thiazolyl, Oxazolyl, Pyridyl, Pyrimidinyl, Pyrazinyl, Pyridazinyl, Phenyl, Pyrrolyl, Imidazolyl, Pyrazolyl, Thiazolyl, Oxazolyl, Triazolyl, Thiophenyl, Furanyl, Pyridyl, Pyrimidinyl und Pyrazinyl ausgewählt sind, wobei das Methyl, Ethyl, Propyl, Butyl, Pentyl, Hexyl, Heptyl, Octyl, Vinyl, Ethinyl, Cyclopropyl, Cyclobutyl, Cyclopentyl, Cyclohexyl, Cycloheptyl, Cyclooctyl, Oxazolidinyl, Imidazolidinyl, Thiazolidinyl, Pyrazolidinyl, Morpholinyl, Piperidinyl, Piperazinyl, Oxazinyl, Imidazolyl, Thiazolyl, Oxazolyl, Pyridyl, Pyrimidinyl, Pyrazinyl, Pyridazinyl, Phenyl, Pyrrolyl, Imidazolyl, Pyrazolyl, Thiazolyl, Oxazolyl, Triazolyl, Thiophenyl, Furanyl, Pyridyl, Pyrimidinyl oder Pyrazinyl jeweils gegebenenfalls mit zumindest einem Substituenten R^{L3d} substituiert ist, und
worin R^{L3c} und R^{L3d} jeweils unabhängig voneinander -F, -Cl, Br, -I, -OH, Methyl, Ethyl, Propyl, Butyl, Pentyl, Hexyl, Heptyl, Octyl, Vinyl, Ethinyl, Methoxy, Ethoxy, Propoxy, Butoxy, Pentoxy, Hexoxy, Heptoxy, Octoxy, Cyclopropyl, Cyclobutyl, Cyclopentyl, Cyclohexyl, Cycloheptyl, Cyclooctyl, Oxazolidinyl, Imidazolidinyl, Thiazolidinyl, Pyrazolidinyl, Morpholinyl, Piperidinyl, Piperazinyl, Oxazinyl, Imidazolyl, Thiazolyl, Oxazolyl, Pyridyl, Pyrimidinyl, Pyrazinyl, Pyridazinyl, Phenyl, Pyrrolyl, Imidazolyl, Pyrazolyl, Thiazolyl, Oxazolyl, Triazolyl, Thiophenyl, Furanyl, Pyridyl, Pyrimidinyl, Pyrazinyl oder Oxo sind;
und/oder
(iv) L³ aus einer Einfachbindung, -C₁₋₈-Alkylen- (vorzugsweise -CH₂-, C₂H₄-, -C₃H₆-), - CO-, -O-, -N(CH₃)-, -NH-, 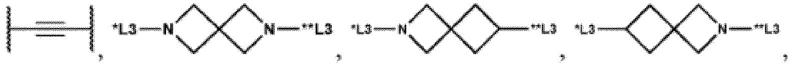 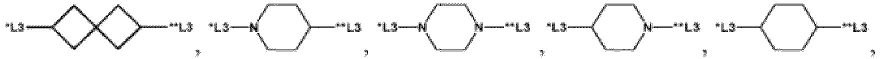 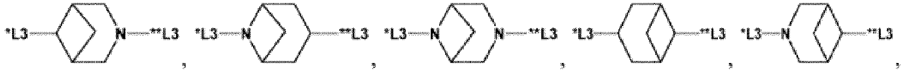 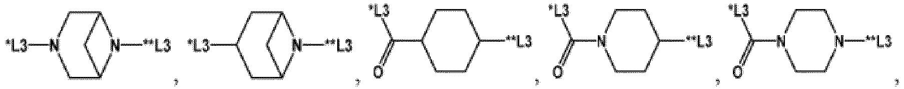 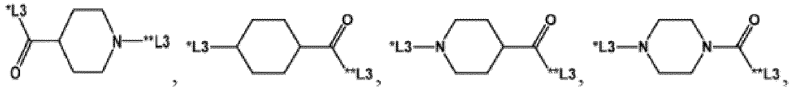 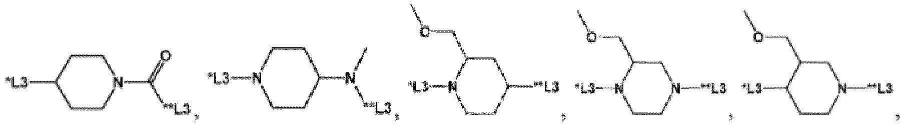 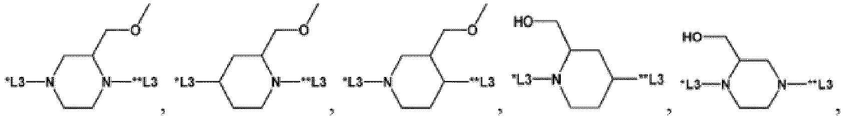 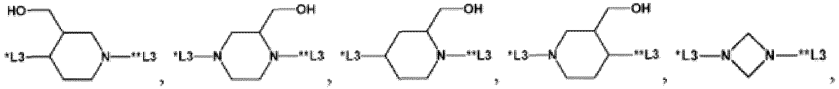 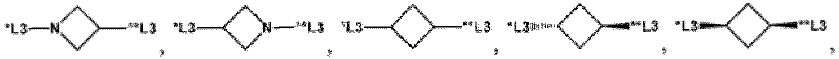 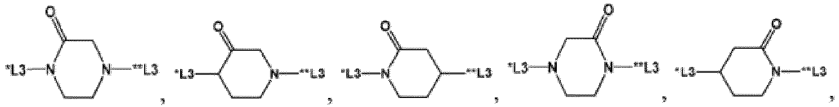 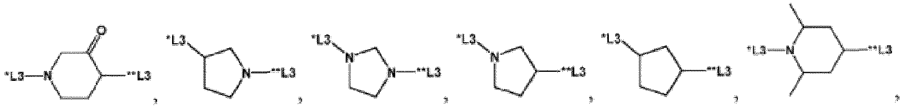 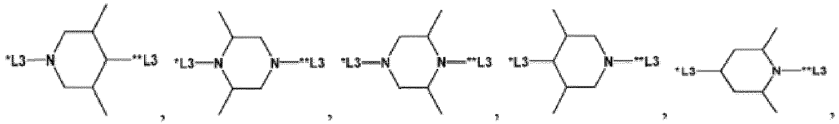 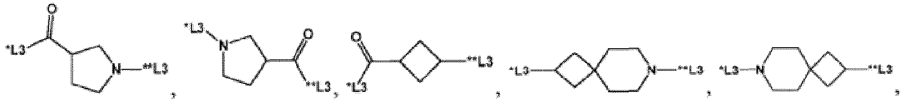 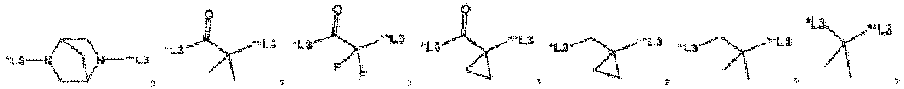 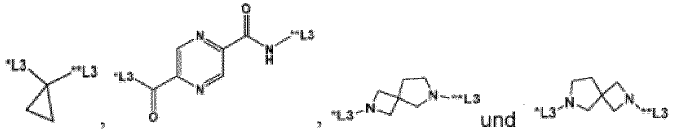 ausgewählt ist;
und/oder
(v) 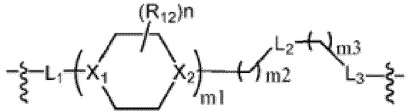 ausgewählt ist aus: -CH₂CH₂-, 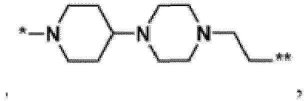 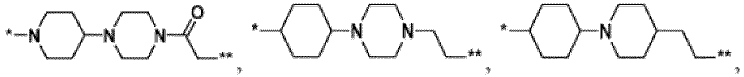 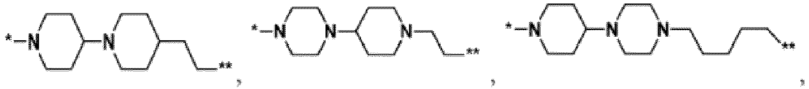 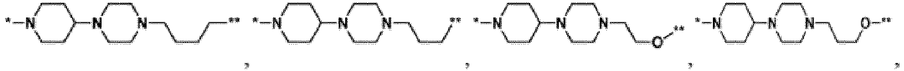 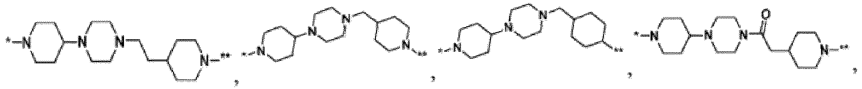 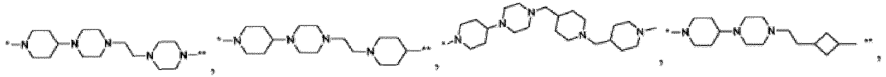 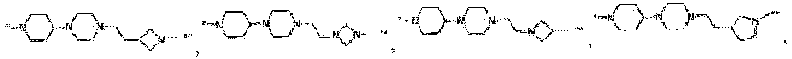 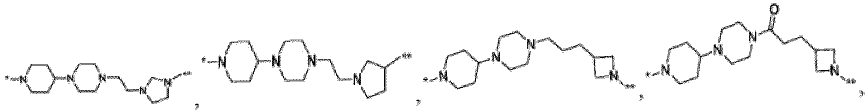 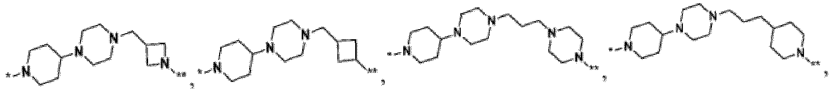 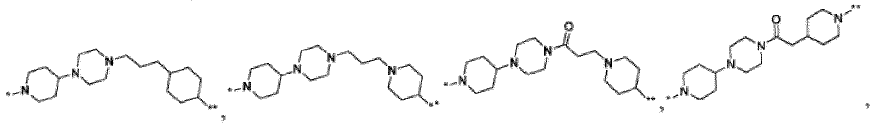 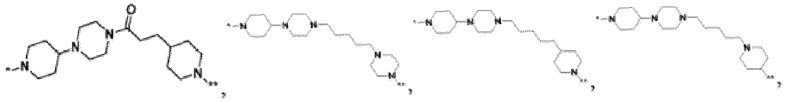 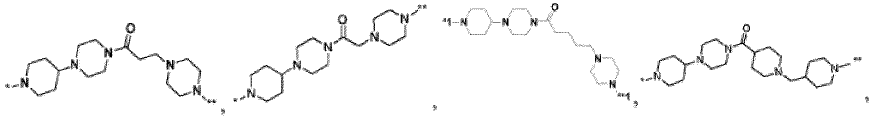 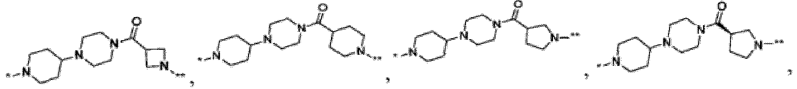 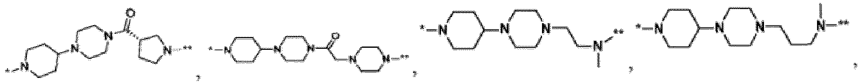 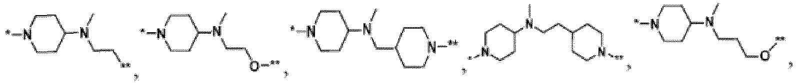 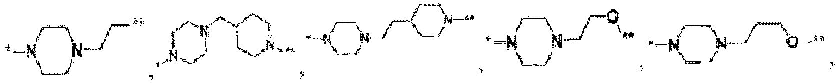 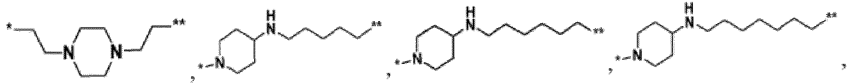 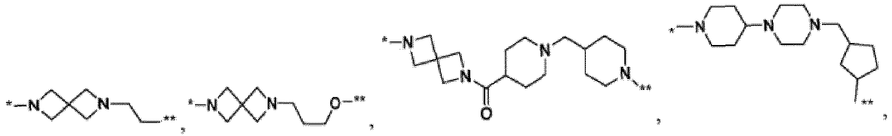 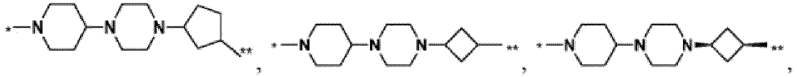 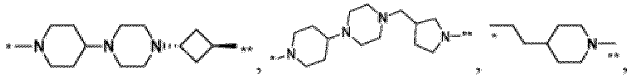 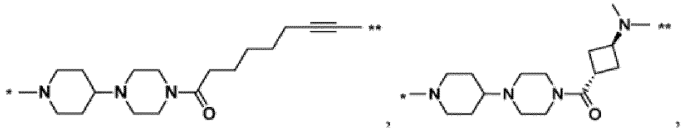 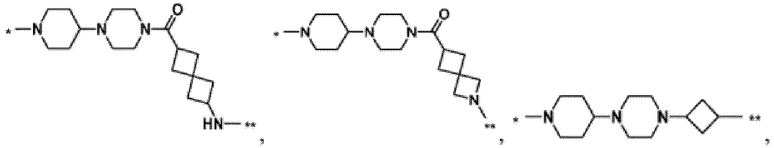 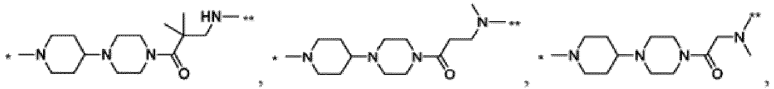 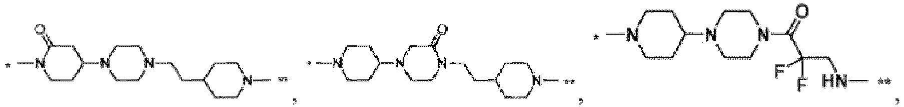 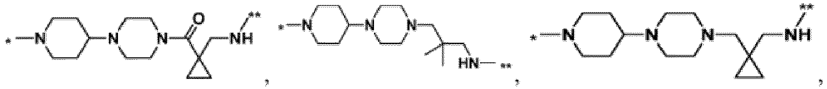 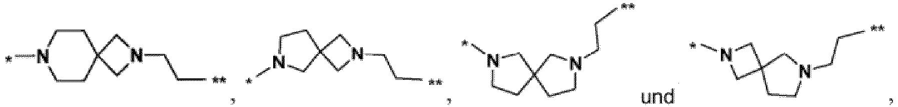 worin sich * auf die an die Gruppierung 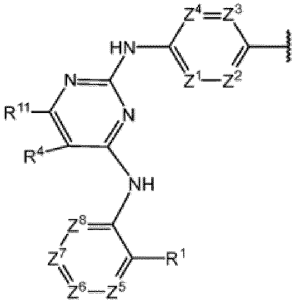
gebundene Position bezieht und sich ** auf die an die Gruppierung 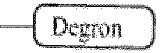 gebundene Position bezieht.

12. Verbindung nach einem der Ansprüche 1 bis 11, worin 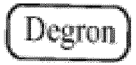 aus Folgendem ausgewählt ist:
(i) 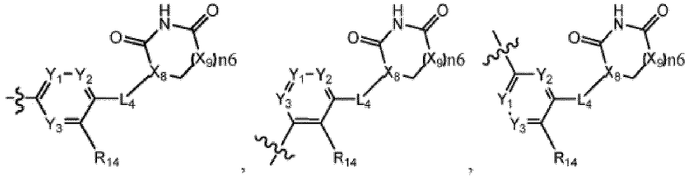 oder 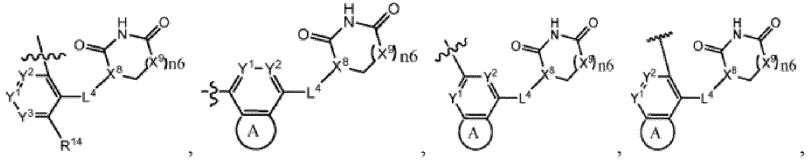 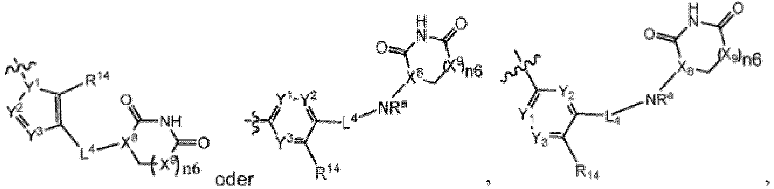 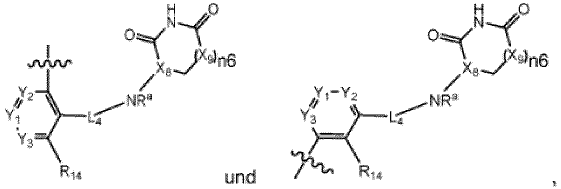
worin Ring A aus 5- bis 7-gliedrigem Cycloalkyl, 5- bis 7-gliedrigem Heterocyclyl, Aryl und Heteroaryl ausgewählt ist,
R¹⁴ jeweils unabhängig aus Wasserstoff, Halogen, -C₁₋₈-Alkyl, -C₁₋₈-Alkoxy und CN ausgewählt ist, wobei das -C₁₋₈-Alkyl oder -C₁₋₈-Alkoxy gegebenenfalls mit einem oder mehreren aus Halogen oder -C₁₋₈-Alkyl substituiert ist; wobei R¹⁴ vorzugsweise aus H, F, Cl, Br, I, CH₃, -OCH₃, CH₂F, CN, CHF₂ und CF₃ ausgewählt ist;
X⁸ jeweils unabhängig aus CH, CD, C(CH₃), C(C₂H₅), C(C₃H₇), C(CN) und N ausgewählt ist;
L⁴ jeweils unabhängig aus einer Einfachbindung, 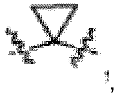 -O-, -NH-, -CH₂-, -CHF- und -CF₂- ausgewählt ist;
Y¹, Y² und Y³ jeweils unabhängig voneinander aus CR^{a} und N ausgewählt sind;
X⁹ CH₂ ist;
R^{a} jeweils unabhängig aus Wasserstoff, Halogen, -C₁₋₈-Alkyl und -C₁₋₈-Alkoxy ausgewählt ist, wobei das -C₁₋₈-Alkyl oder -C₁₋₃-Alkoxy gegebenenfalls mit zumindest einem oder mehreren aus Halogen, Hydroxy, Halogen, -C₁₋₈-Alkyl und -C₁₋₈-Alkoxy substituiert ist; und
n₆ jeweils unabhängig 0, 1 oder 2 ist;
oder
(ii) 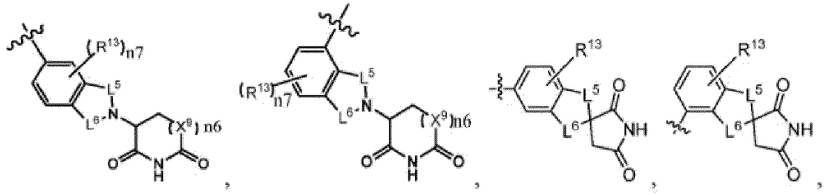 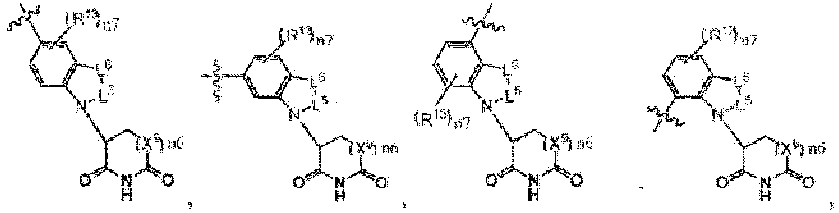 worin L⁵ und L⁶ jeweils unabhängig voneinander aus einer Einfachbindung, 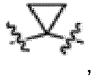 -O-, -NH-, -NMe-, -N(CH₂CH₃)-, -CH₂-, -CHF-, -CF₂-, -C(CH₃)₂- und -CO- ausgewählt sind (wobei vorzugsweise L⁵ -CO- oder -CH₂- ist und L⁶ 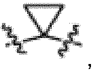 -O-, -NH-, -NMe-, -N(CH₂CH₃)-, -CH₂-, -CHF-, -CF₂-, -C(CH₃)₂- oder -CO- ist);
X⁹ CH₂ ist;
die R¹³ jeweils unabhängig voneinander aus Wasserstoff, -F, -Cl, -Br, -I, CN, -C₁₋₈-Alkyl und -C₁₋₈-Alkoxy ausgewählt sind;
n₆ = 0 oder 1 ist; und
n₇ = 0, 1 oder 2 ist;
oder
(iv) 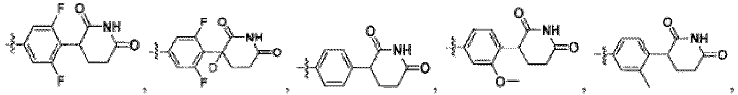 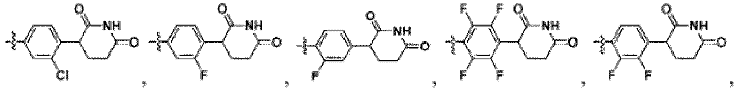 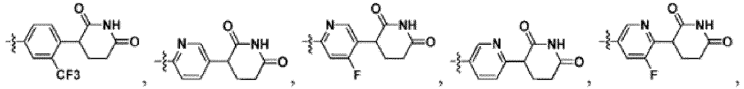 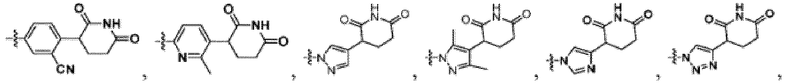 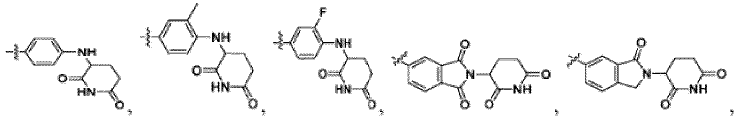 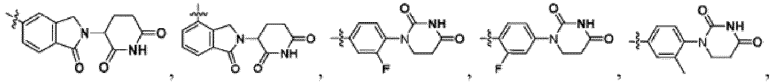 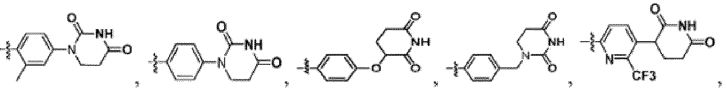 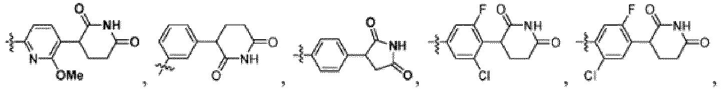 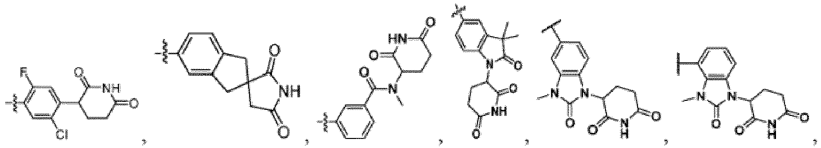 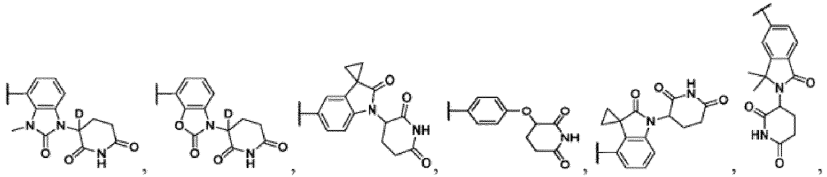 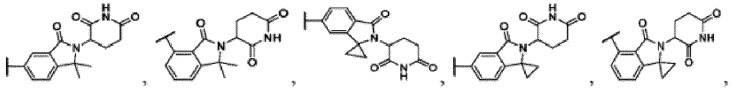 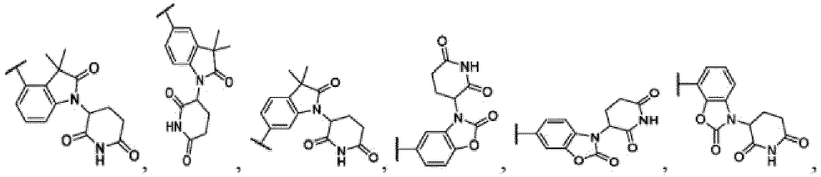 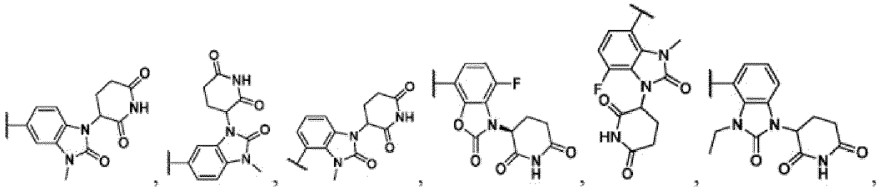 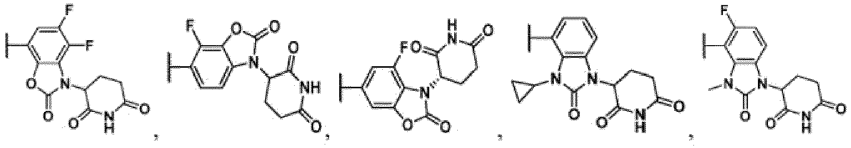 und

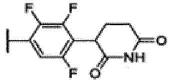

13. Verbindung nach einem der Ansprüche 1 bis 12, worin:
R^{z} jeweils unabhängig aus H, -CH₃, -C₂H₅, F, -CH₂F, -CHF₂, -CF₃, -OCH₃, -OC₂H₅, -C₃H₇, -OCH₂F, -OCHF₂, -OCH₂CF₃, -OCF₃, -SCF₃, -CF₃ und -CH(OH)CH₃ ausgewählt ist.

14. Verbindung, die aus den folgenden ausgewählt ist:
| | |
|---|---|
| 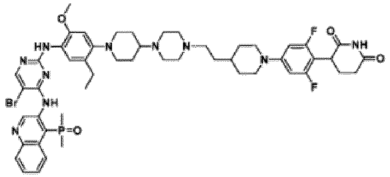 | 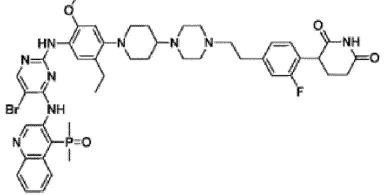 |
| 1 | 2 |
| 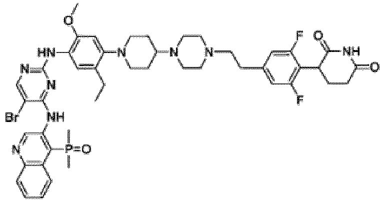 | 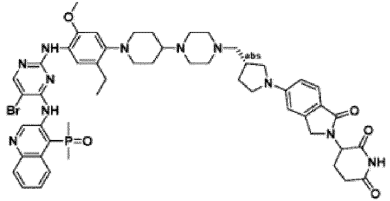 |
| 3 | 4 |
| 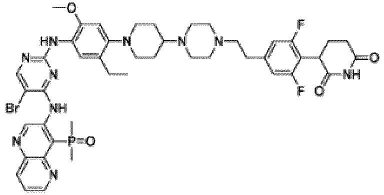 | 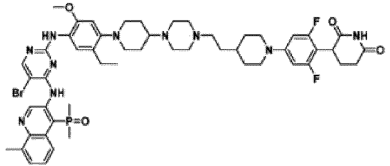 |
| 7 | 8 |
| 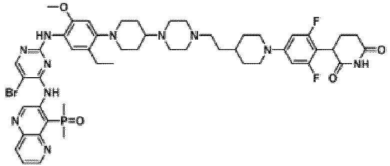 | 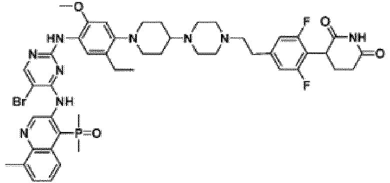 |
| 9 | 10 |
| 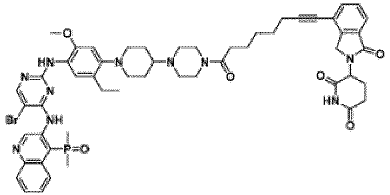 | 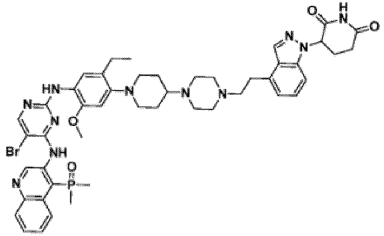 |
| 11 | 12 |
| 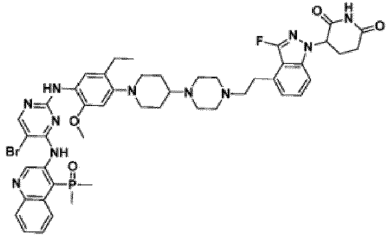 | 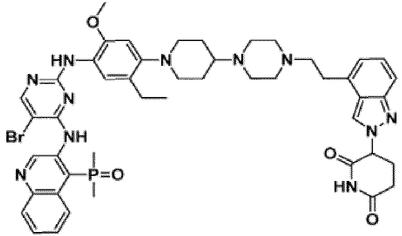 |
| 13 | 14 |
| 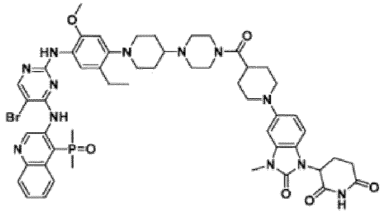 | 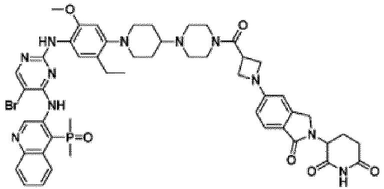 |
| 15 | 16 |
| 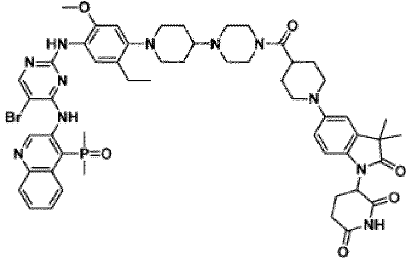 | 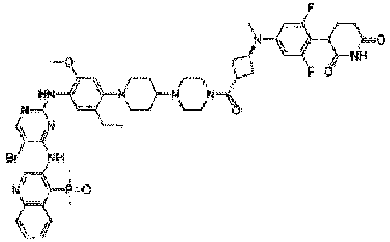 |
| 17 | 18 |
| 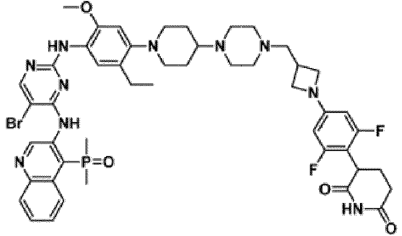 | 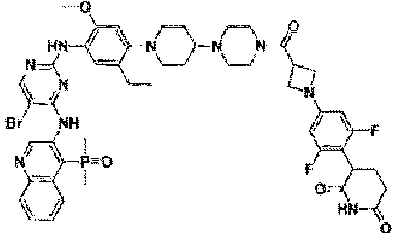 |
| 19 | 20 |
| 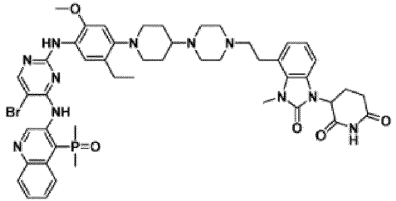 | 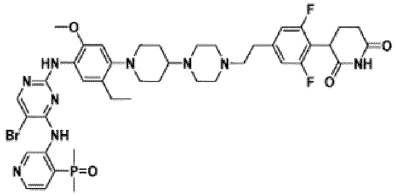 |
| 21 | 22 |
| 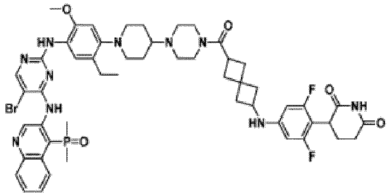 | 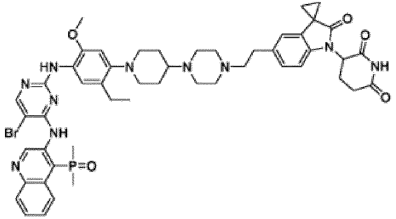 |
| 23 | 24 |
| 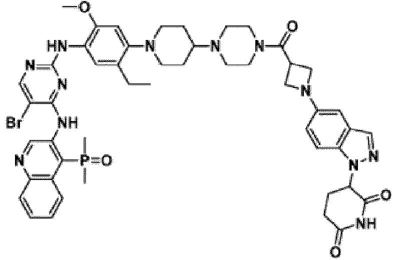 | 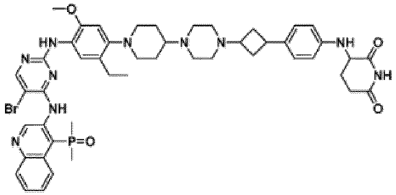 |
| 25 | 26 |
| 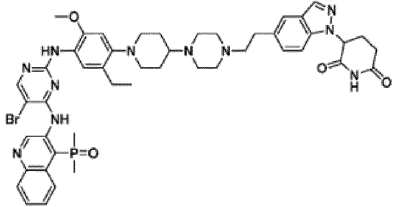 | 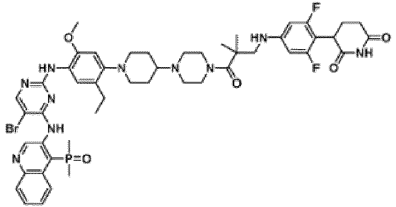 |
| 27 | 28 |
| 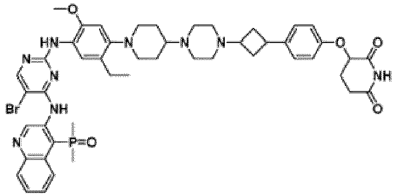 | 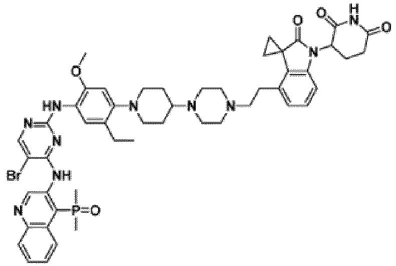 |
| 29 | 30 |
| 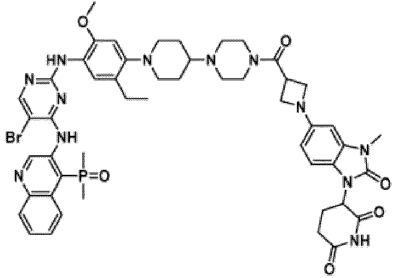 | 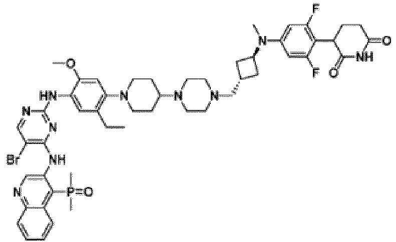 |
| 31 | 32 |
| 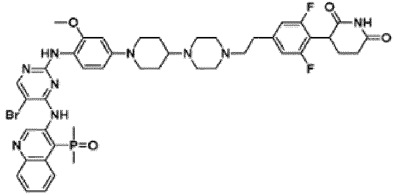 | 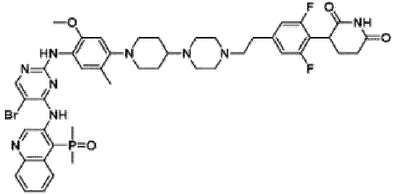 |
| 33 | 34 |
| 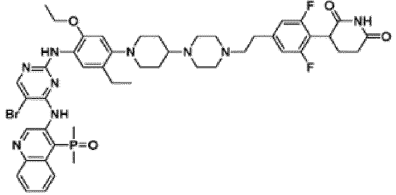 | 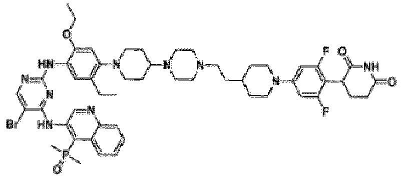 |
| 35 | 36 |
| 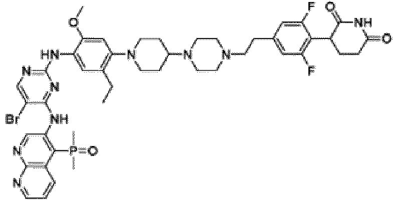 | 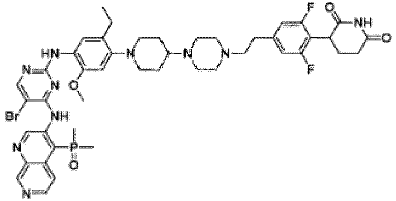 |
| 37 | 38 |
| | 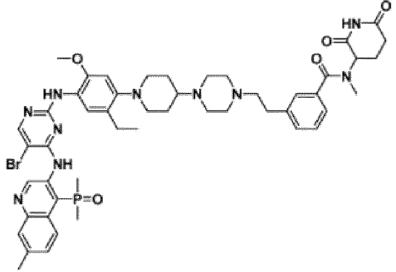 |
| | 40 |
| 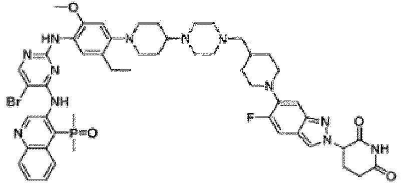 | 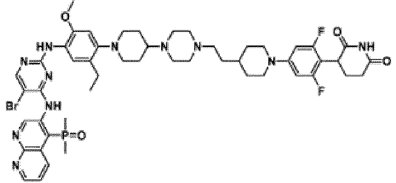 |
| 41 | 42 |
| 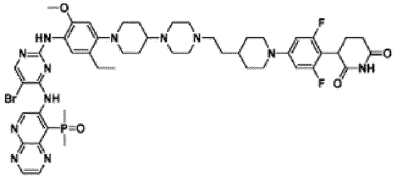 | 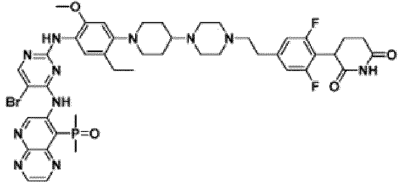 |
| 43 | 44 |
| 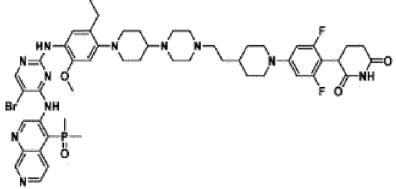 | 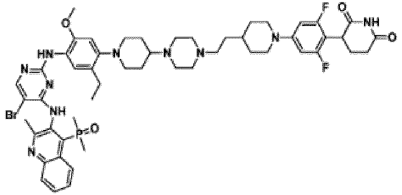 |
| 45 | 46 |
| 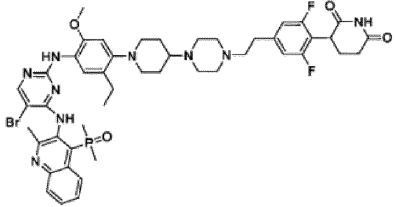 | 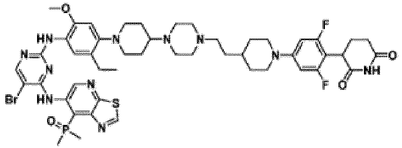 |
| 47 | 48 |
| 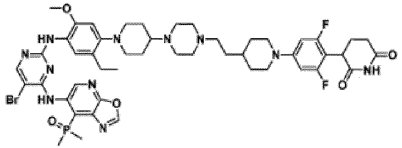 | 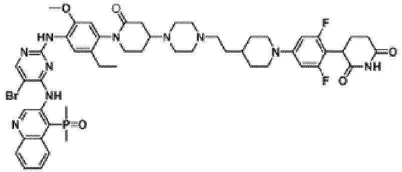 |
| 49 | 50 |
| 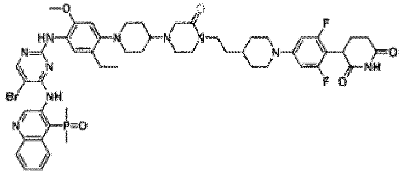 | 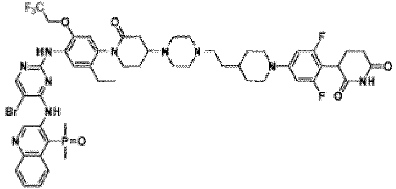 |
| 51 | 52 |
| 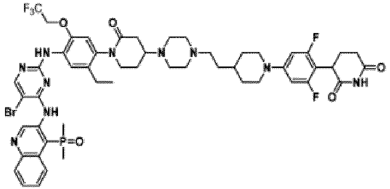 | 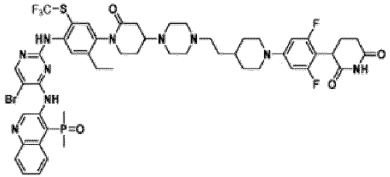 |
| 53 | 54 |
| 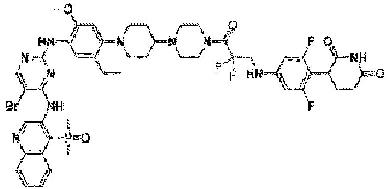 | 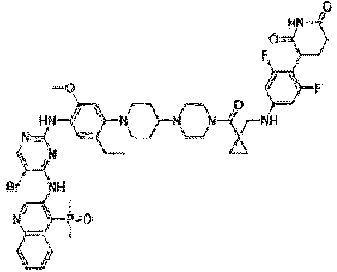 |
| 55 | 56 |
| 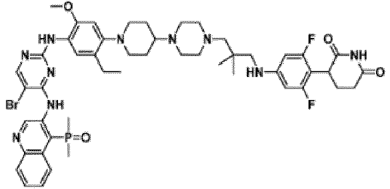 | 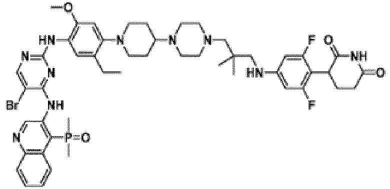 |
| 57 | 58 |
| 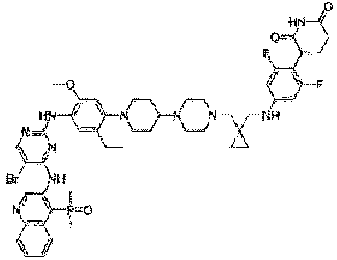 | 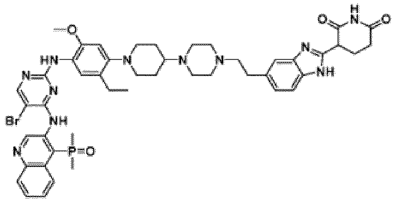 |
| 59 | 60 |
| 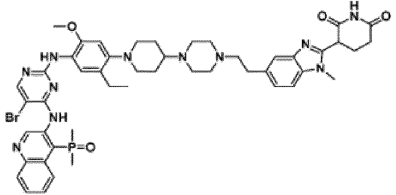 | 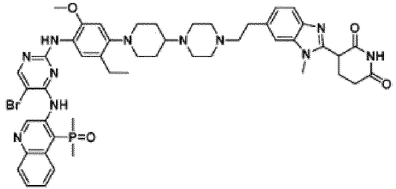 |
| 61 | 62 |
| 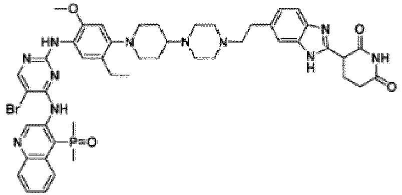 | 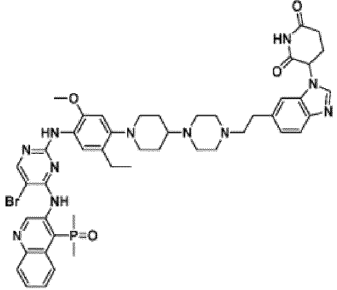 |
| 63 | 64 |
| 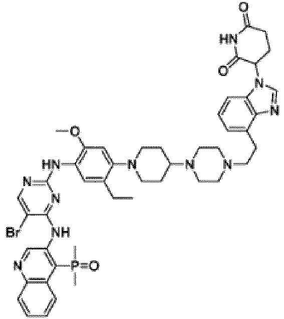 | 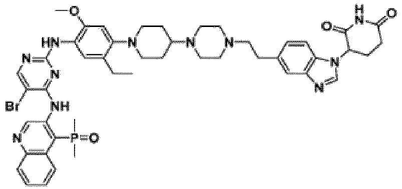 |
| 65 | 66 |
| 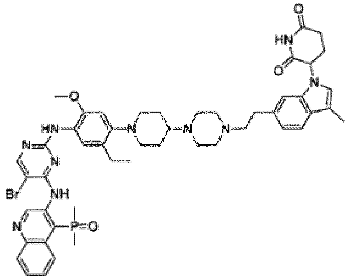 | 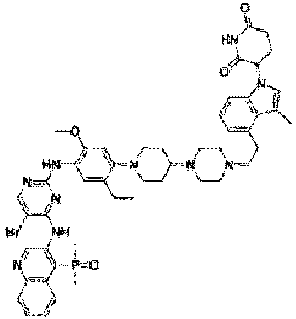 |
| 67 | 68 |
| 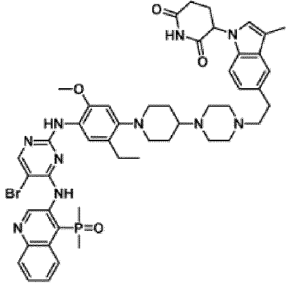 | 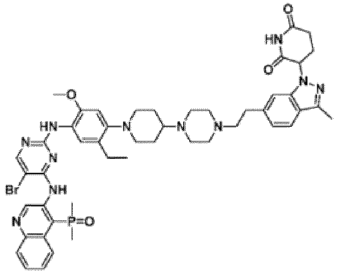 |
| 69 | 70 |
| 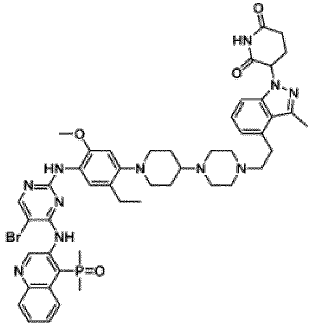 | 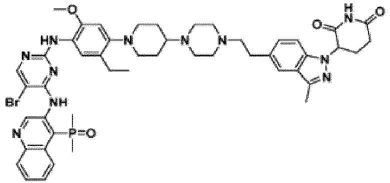 |
| 71 | 72 |
| 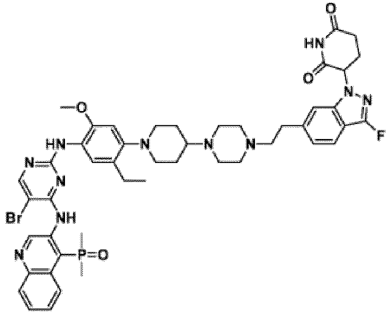 | 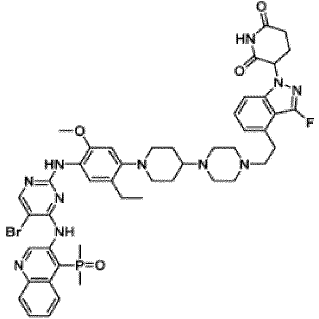 |
| 73 | 74 |
| 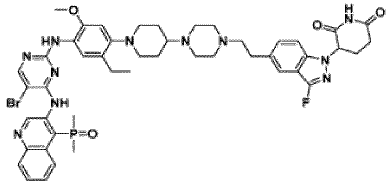 | 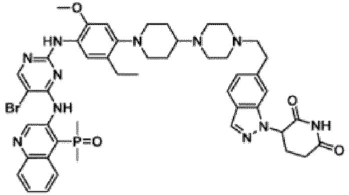 |
| 75 | 76 |
| 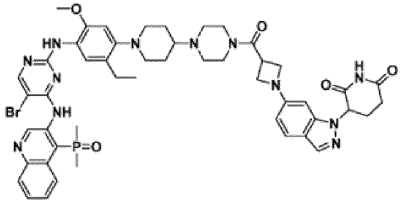 | 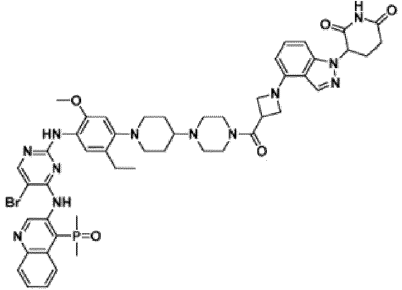 |
| 77 | 78 |
| 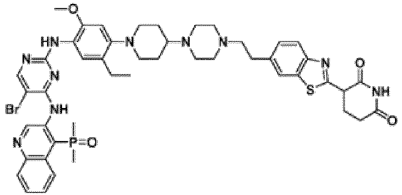 | 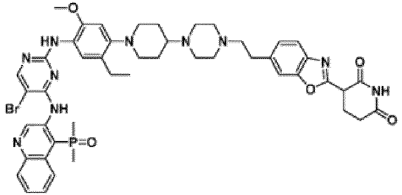 |
| 79 | 80 |
| 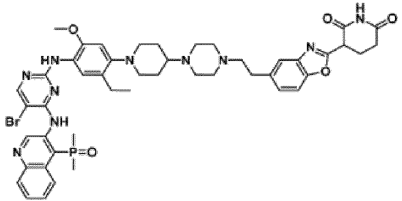 | 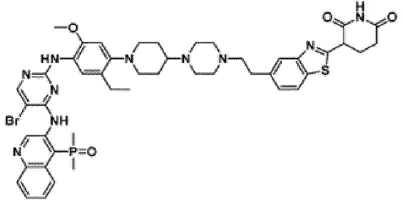 |
| 81 | 82 |
| 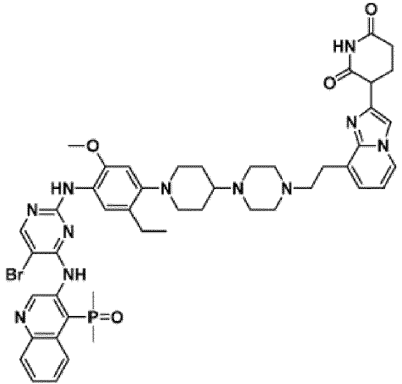 | 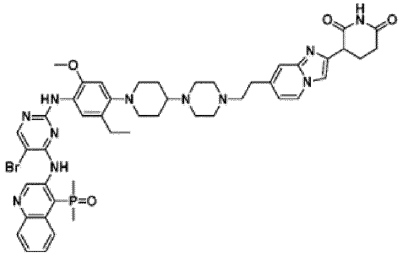 |
| 83 | 84 |
| 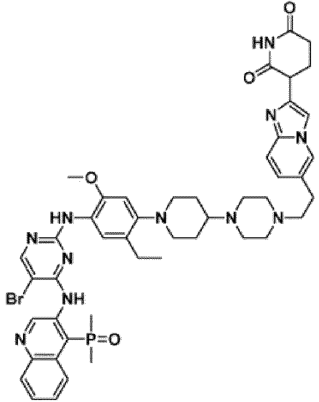 | 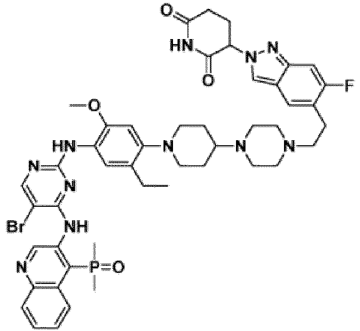 |
| 85 | 86 |
| 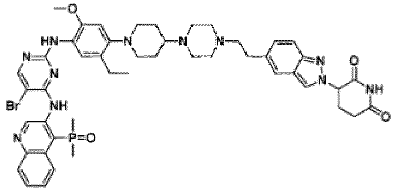 | 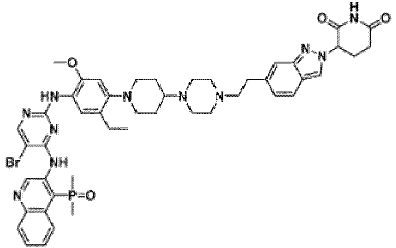 |
| 87 | 88 |
| 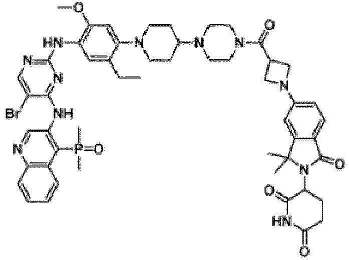 | 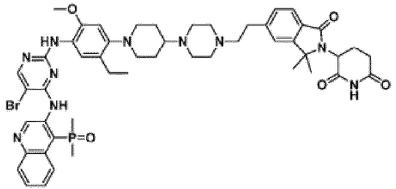 |
| 89 | 90 |
| 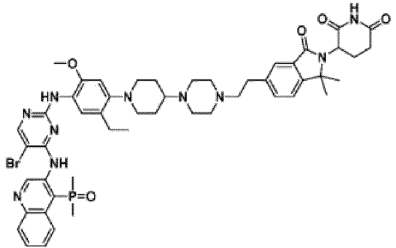 | 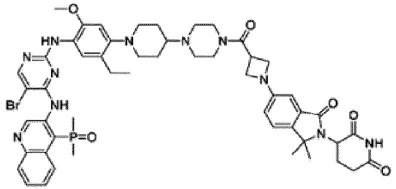 |
| 91 | 92 |
| 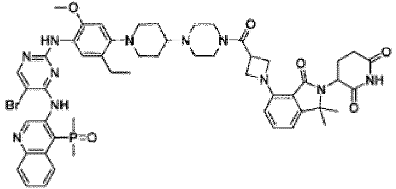 | 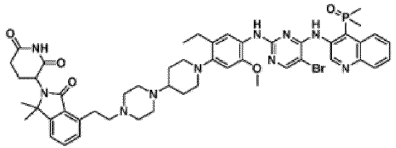 |
| 93 | 94 |
| 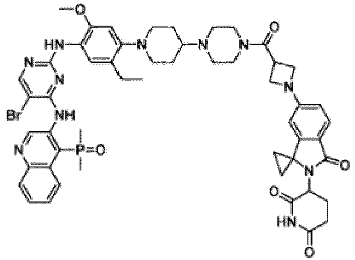 | 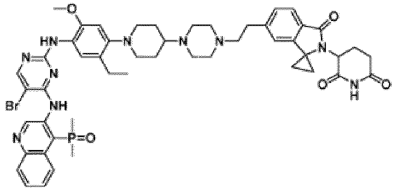 |
| 95 | 96 |
| 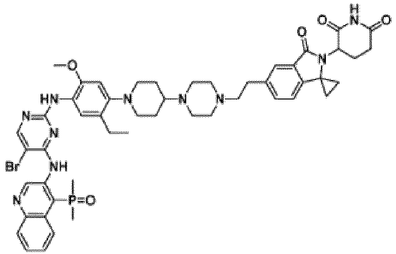 | 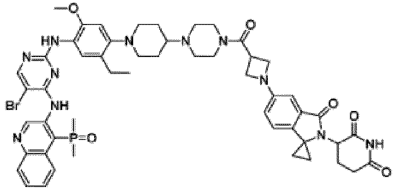 |
| 97 | 98 |
| 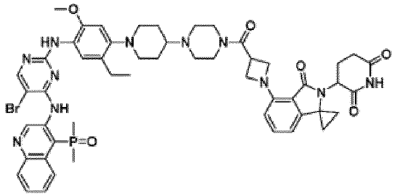 | 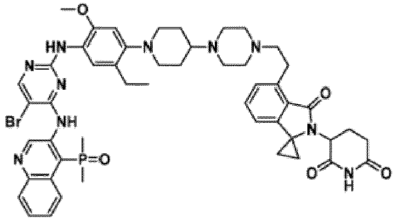 |
| 99 | 100 |
| 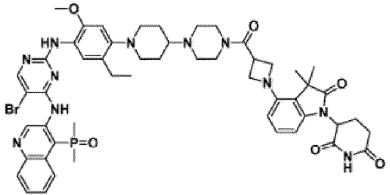 | 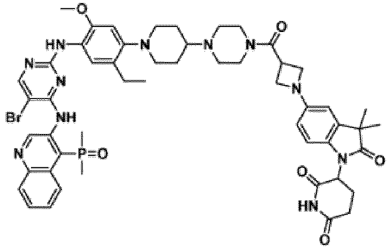 |
| 101 | 102 |
| 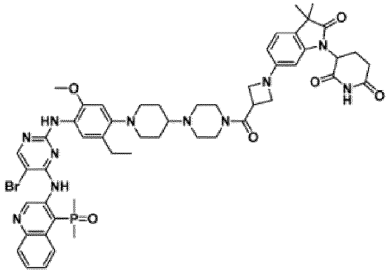 | 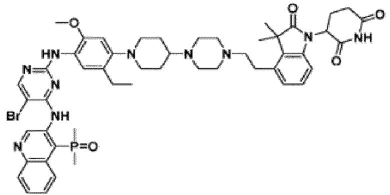 |
| 103 | 104 |
| 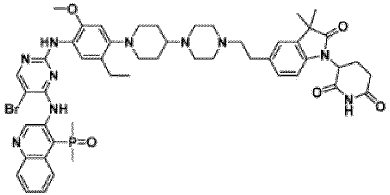 | 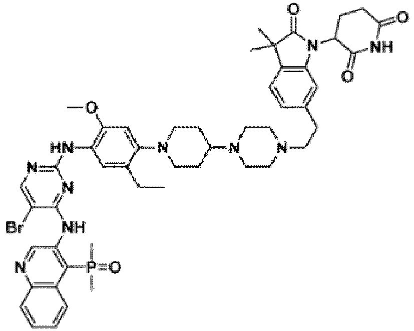 |
| 105 | 106 |
| 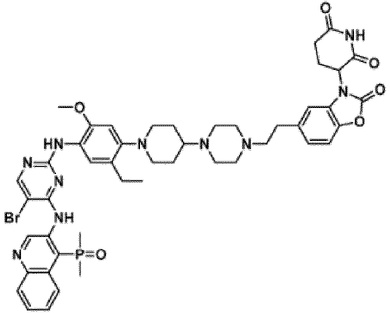 | 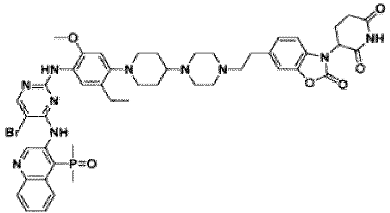 |
| 107 | 108 |
| 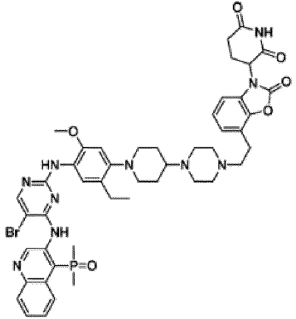 | 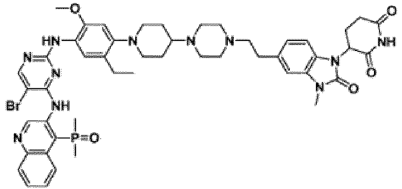 |
| 109 | 110 |
| 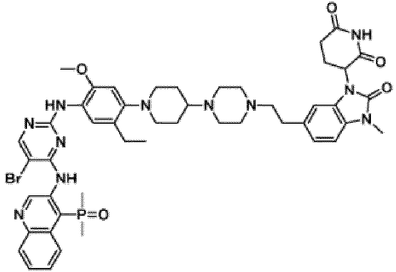 | 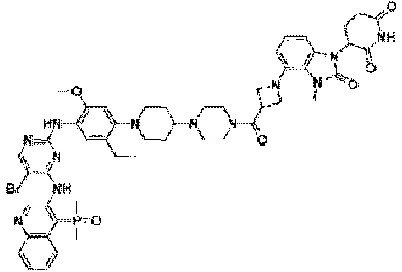 |
| 111 | 112 |
| 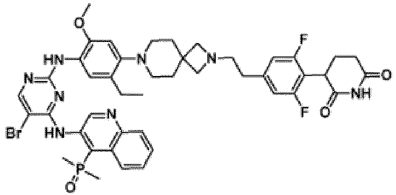 | 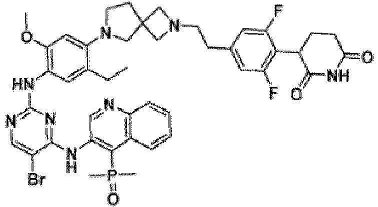 |
| 113 | 114 |
| 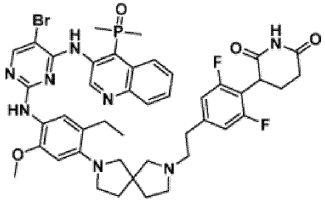 | 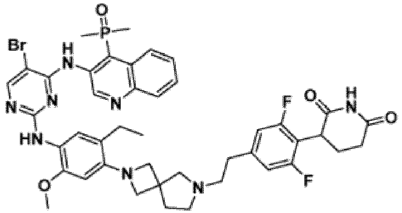 |
| 115 | 116 |
| 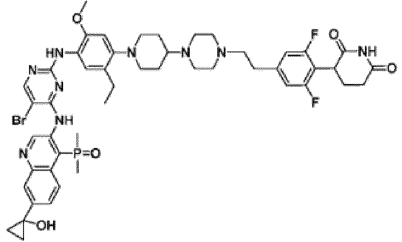 | 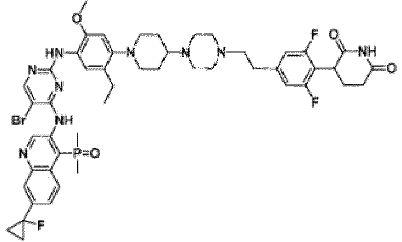 |
| 117 | 118 |
| 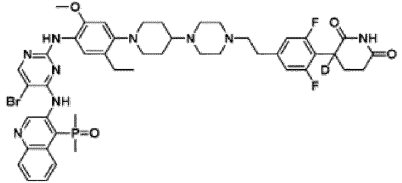 | 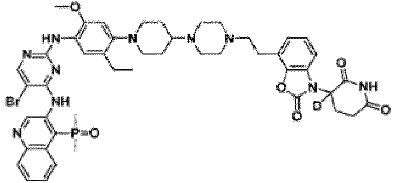 |
| 119 | 120 |
| 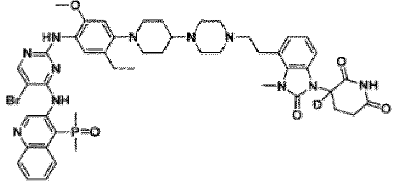 | 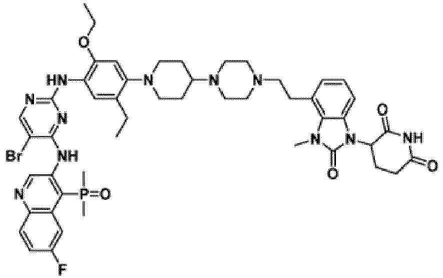 |
| 121 | 124 |
| 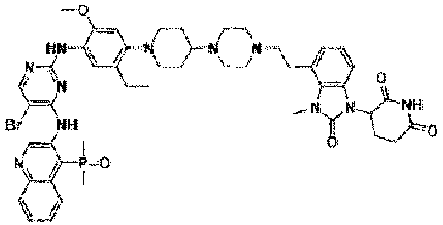 | 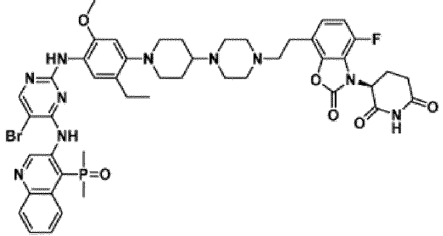 |
| 125 | 126 |
| 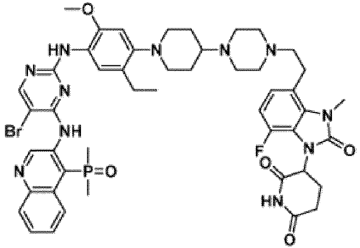 | 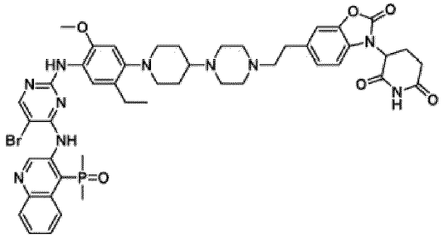 |
| 130 | 132 |
| 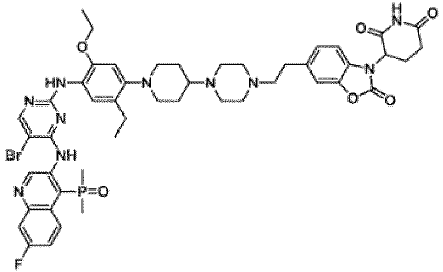 | 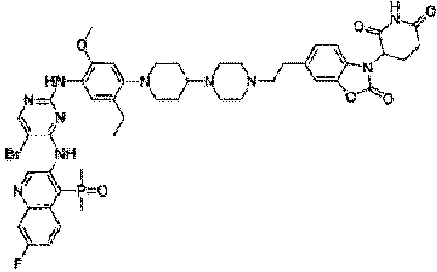 |
| 133 | 134 |
| 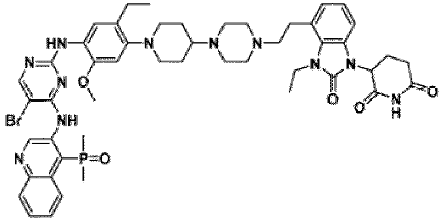 | 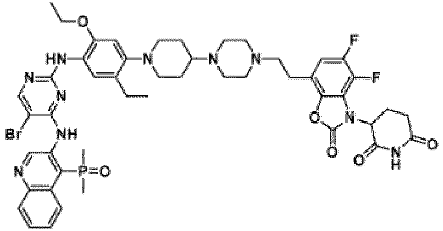 |
| 135 | 136 |
| 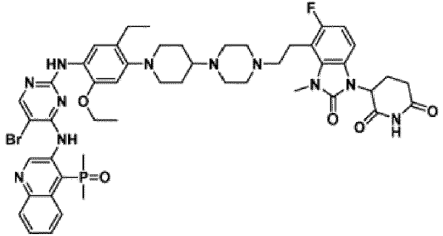 | 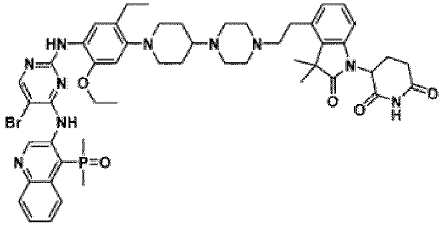 |
| 137 | 138 |
| 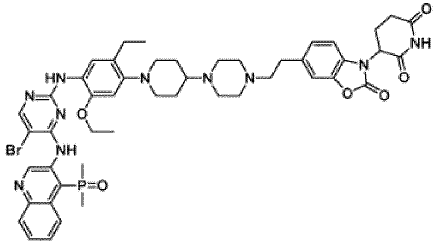 | 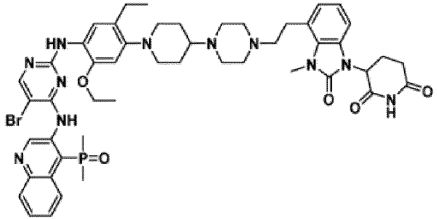 |
| 139 | 140 |
| 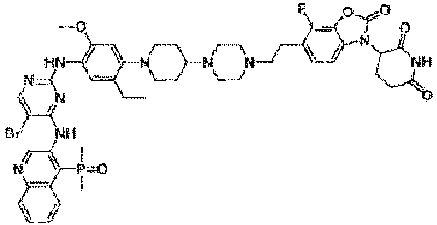 | 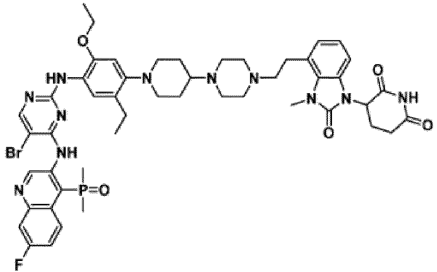 |
| 142 | 147 |
| 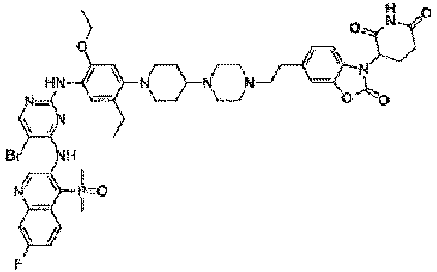 | 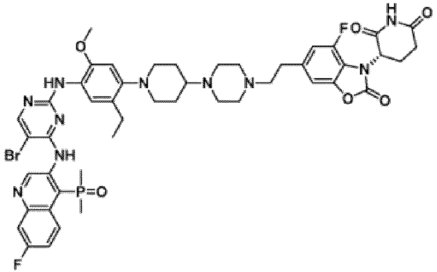 |
| 149 | 151 |
| 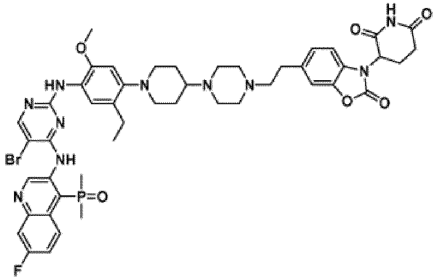 | 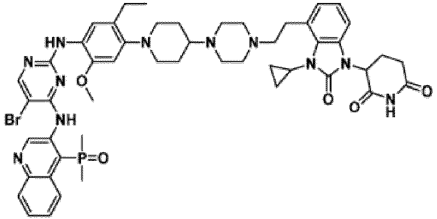 |
| 153 | 155 |
| | 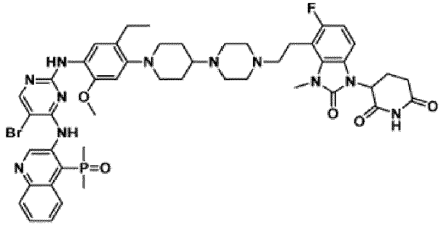 |
| | 159 |
| 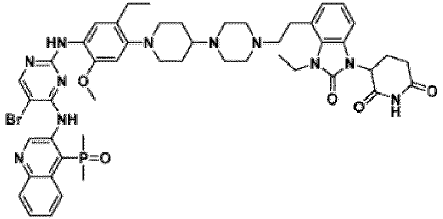 | 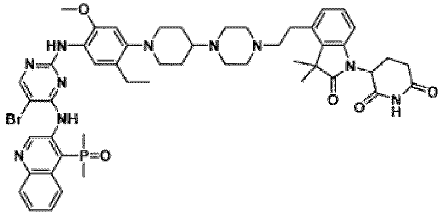 |
| 163 | 164 |
| 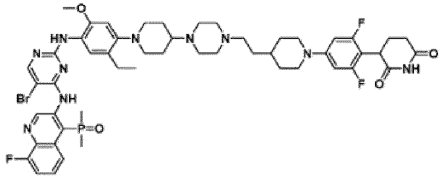 | 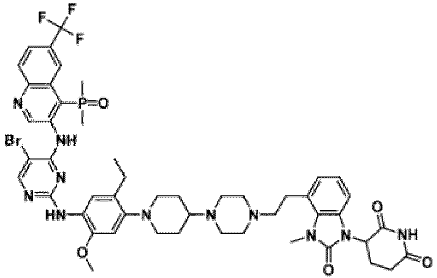 |
| 165 | 166 |
| 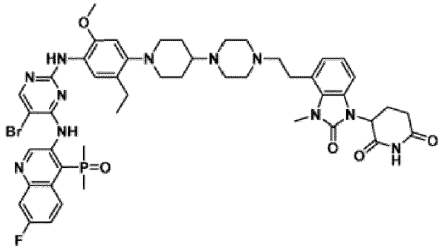 | 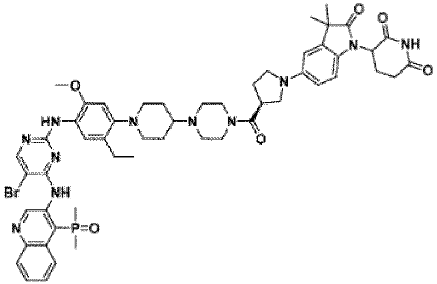 |
| 167 | 168 |
| 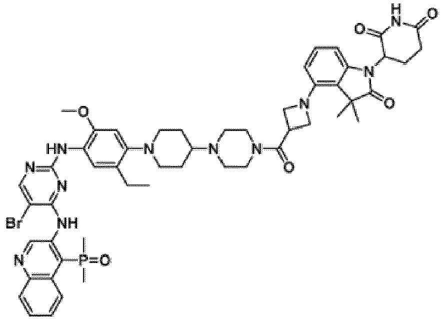 | 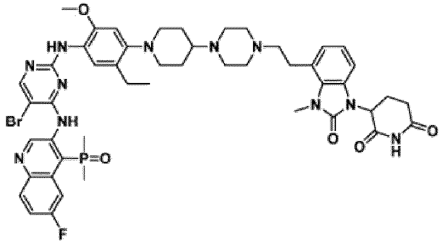 |
| 169 | 170 |
| 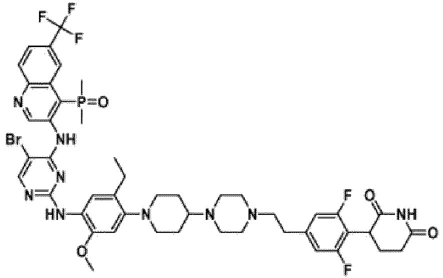 | 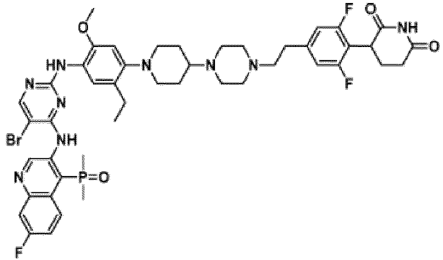 |
| 171 | 172 |
| 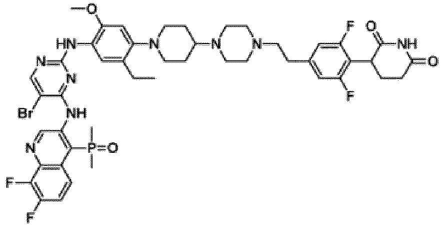 | 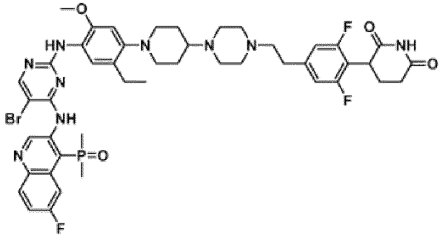 |
| 173 | 174 |
| 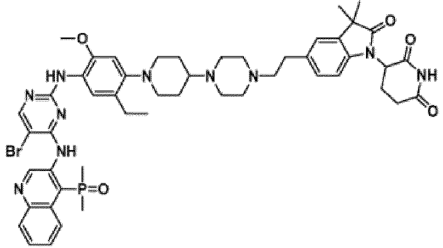 | 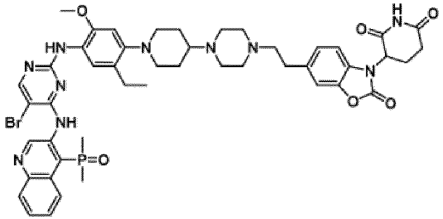 |
| 175 | 176 |
| | 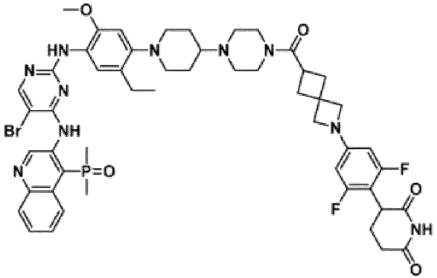 |
| | 178 |
| 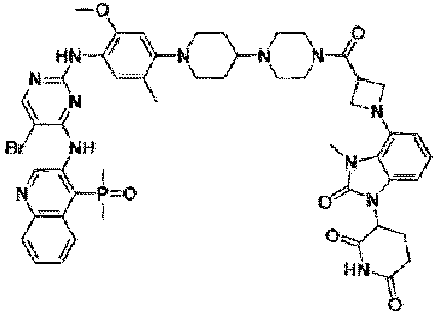 | 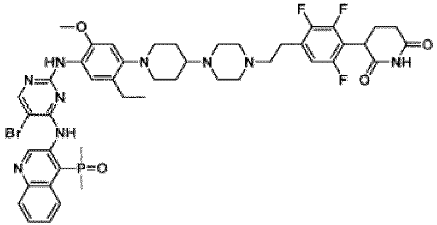 |
| 179 | 180 |
| 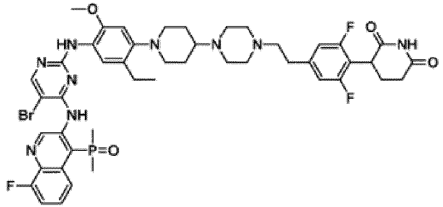 | 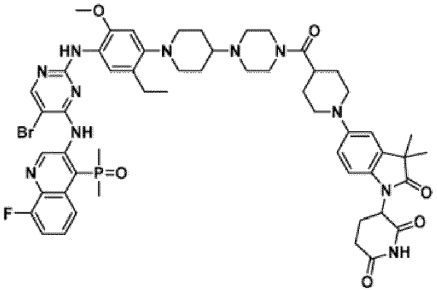 |
| 181 | 182 |
| 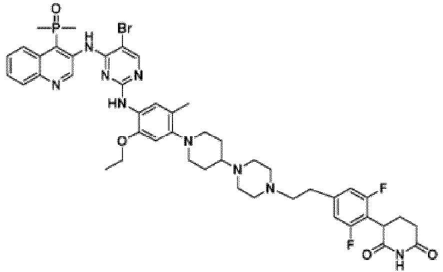 | 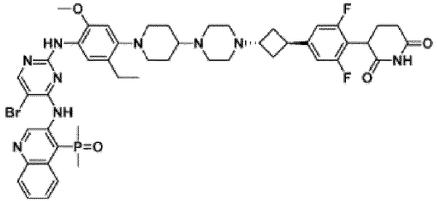 |
| 183 | 184 |
| 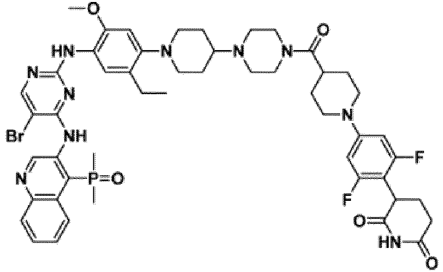 | 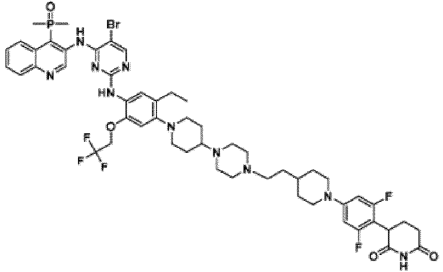 |
| 185 | 186 |
| 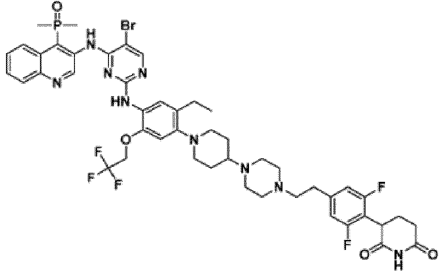 | 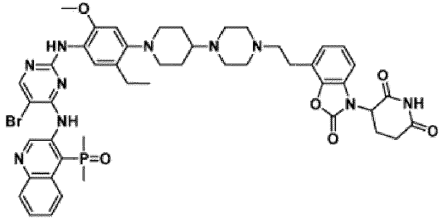 |
| 187 | 188 und |
| 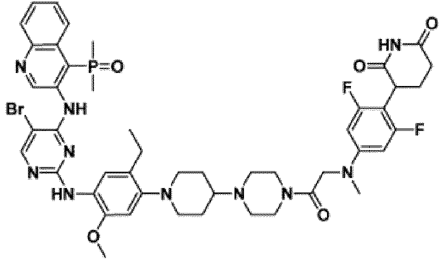 | |
| 189 | |

15. Verbindung nach einem der vorangegangenen Ansprüche zur Verwendung in einem Verfahren zum:
(i) Verringern der EGFR-Aktivität durch Inhibierung und/oder Abbau, welches das Verabreichen der Verbindung oder eines pharmazeutisch annehmbaren Salzes davon an ein Individuum umfasst,
(ii) Behandeln einer Erkrankung oder Störung bei einem Patienten, welches das Verabreichen einer therapeutisch wirksamen Menge der Verbindung oder eines pharmazeutisch annehmbaren Salzes davon als EGFR-Kinase-Inhibitor und/oder -Abbaumittel an den Patienten umfasst, wobei die Erkrankung oder die Störung mit der Inhibierung von EGFR im Zusammenhang steht.

16. Verbindung zur Verwendung nach Anspruch 15, wobei die Erkrankung aus Krebs, vorzugsweise Pankreaskrebs, Brustkrebs, Glioblastoma multiforme, Kopf- und Halskrebs und nichtkleinzelligem Lungenkrebs, ausgewählt ist.

## Revendications

1. Composé de formule (X) : 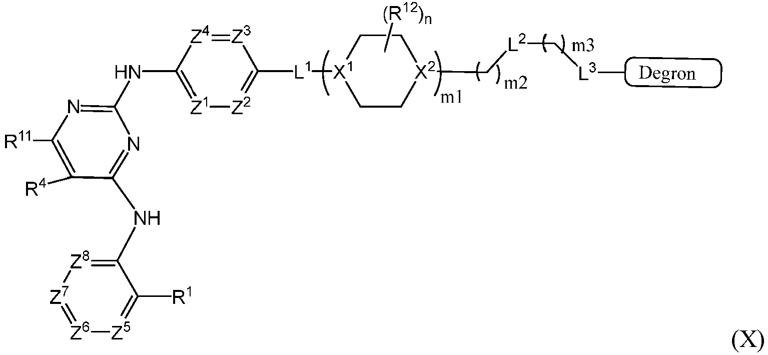 ou un N-oxyde de celui-ci, ou un sel pharmaceutiquement acceptable de celui-ci, ou un stéréoisomère de celui-ci, ou un analogue deutéré de celui-ci, dans lequel :
R¹ est -P(O)R^{1a}R^{1b}, où R^{1a} et R^{1b} sont chacun indépendamment choisis parmi -CH₃, -CH₂CH₃, -CH₂CH₂CH₃, *-iso-*C₃H₇, -CH₂CH₂CH₂CH₃, *-iso*-C₄H₉, -*sec*-C₄H₉ et -*tert*-C₄H₉, cyclopropyle, cyclobutyle et cyclopentyle ;
Z⁵ est -CR² ;
Z⁶ est -CR³ ;
Z⁷ est N ;
Z⁸ est -CR¹⁰ ;
R² et R³ forment, conjointement avec les atomes de carbone auxquels ils sont liés, un cycle aromatique à 5 ou 6 chaînons, ledit cycle comprenant 0 à 3 hétéroatomes indépendamment choisis parmi l'azote, l'oxygène et le soufre ; lequel cycle est éventuellement substitué par au moins un substituant R^{2e} ;
R^{2e}, à chaque occurrence, est indépendamment hydrogène, -F, -Cl, -Br, -I, méthyle, éthyle, propyle, butyle, pentyle, cyclopropyle, cyclobutyle ou cyclopentyle, où chacun des méthyle, éthyle, propyle, butyle, pentyle, cyclopropyle, cyclobutyle ou cyclopentyle est éventuellement substitué par au moins un R^{2d} ; et
R^{2d}, à chaque occurrence, est indépendamment -F, -Cl, - Br, -I, -OH, méthyle, éthyle, propyle, butyle, pentyle, hexyle, heptyle, octyle, méthoxy, éthoxy, propoxy, butoxy, pentoxy, hexoxy, heptoxy, octoxy, cyclopropyle, cyclobutyle, cyclopentyle, cyclohexyle, cycloheptyle, cyclooctyle ou phényle ;
R⁴ est choisi parmi hydrogène, halogène, -alkyle en C₁₋₈, -alcényle en C₂₋₈, -alcynyle en C₂₋₈, -alcoxy en C₁₋₈, -cycloalkyle en C₃-C₈, hétérocyclyle de 3 à 8 chaînons, -aryle en C₆-C₁₂, hétéroaryle de 5 à 12 chaînons, -CN, -SO₂R^{4a}, -SO₂NR^{4a}R^{4b}, -COR^{4a}, -CO₂R^{4a}, - CONR^{4a}R^{4b}, -NR^{4a}R^{4b}, -NR^{4a}COR^{4b}, -NR^{4a}CO₂R^{4b} et -NR^{4a}SO₂R^{4b}, où chacun des alkyle en C₁₋₈, -alcényle en C₂₋₈, -alcynyle en C₂₋₈, -alcoxy en C₁₋₈, -cycloalkyle en C₃-C₈, hétérocyclyle de 3 à 8 chaînons, -aryle en C₆-C₁₂, ou hétéroaryle de 5 à 12 chaînons est éventuellement substitué par halogène, -alcoxy en C₁₋₈, - alkyle en C₁₋₈, -alcényle en C₂₋₈, -alcynyle en C₂₋₈, - cycloalkyle en C₃-C₈, hétérocyclyle de 3 à 8 chaînons, aryle en C₆-C₁₂, hétéroaryle de 5 à 12 chaînons, oxo, -CN, -OR^{4c}, - SO₂R^{4c}, -SO₂NR^{4c}R^{4d}, -COR^{4c}, -CO₂R^{4c}, -CONR^{4c}R^{4d}, -NR^{4c}R^{4d}, - NR^{4c}COR^{4d}, -NR^{4c}CO₂R^{4d} ou -NR^{4c}SO₂R^{4d} ;
R^{4a}, R^{4b}, R^{4c} et R^{4d} sont chacun indépendamment hydrogène, -alkyle en C₁₋₈, -alcényle en C₂₋₈, -alcynyle en C₂₋₈, cycloalkyle en C₃-C₈, hétérocyclyle de 3 à 8 chaînons, -aryle en C₆-C₁₂ ou hétéroaryle de 5 à 12 chaînons ;
R¹⁰, R¹¹ et R¹² sont chacun indépendamment choisis parmi hydrogène, -F, -Cl, -Br, -I, méthyle, éthyle, propyle, butyle, pentyle, hexyle, heptyle, octyle, méthoxy, éthoxy, propoxy, butoxy, pentoxy, hexoxy, heptoxy, octoxy, cyclopropyle, cyclobutyle, cyclopentyle, cyclohexyle, cycloheptyle, cyclooctyle, -NH₂ et oxo, où chacun des méthyle, éthyle, propyle, butyle, pentyle, hexyle, heptyle, octyle, méthoxy, éthoxy, propoxy, butoxy, pentoxy, hexoxy, heptoxy, octoxy, cyclopropyle, cyclobutyle, cyclopentyle, cyclohexyle, cycloheptyle ou cyclooctyle est éventuellement substitué par au moins un substituant R^{9c} ; ou
deux R¹² forment, conjointement avec les atomes de carbone auxquels ils sont liés, un cycle à 3, 4, 5, 6, 7 ou 8 chaînons, ledit cycle comprenant 0, 1, 2 ou 3 hétéroatomes indépendamment choisis parmi l'azote, l'oxygène et le soufre ; lequel cycle est éventuellement substitué par au moins un substituant R^{9c} ; où
R^{9c} est indépendamment -F, -Cl, -Br, -I, hydroxy, méthyle, éthyle, propyle, butyle, pentyle, hexyle, heptyle, octyle, méthoxy, éthoxy, propoxy, butoxy, pentoxy, hexoxy, heptoxy, octoxy, cyclopropyle, cyclobutyle, cyclopentyle, cyclohexyle, cycloheptyle, cyclooctyle, -alcényle en C₂₋₈, - alcynyle en C₂₋₈, hétérocyclyle de 3 à 8 chaînons, phényle, hétéroaryle de 5 à 12 chaînons, NH₂ ou -NHCH₃ ;
Z¹, Z², Z³ et Z⁴ sont chacun indépendamment -CR^{z} ;
R^{Z}, à chaque occurrence, est indépendamment choisi parmi hydrogène, halogène, -alkyle en C₁₋₈, -alcényle en C₂₋₈, -alcynyle en C₂₋₈, -NR^{Za}R^{Zb}, -OR^{Za}, -SR^{Za}, cycloalkyle en C₃-C₈, hétérocyclyle de 3 à 8 chaînons, aryle en C₆-C₁₂, hétéroaryle de 5 à 12 chaînons et CN, où chacun des -alkyle en C₁₋₈, -alcényle en C₂₋₈, -alcynyle en C₂₋₈, cycloalkyle en C₃-C₈, hétérocyclyle de 3 à 8 chaînons, aryle en C₆-C₁₂ ou hétéroaryle de 5 à 12 chaînons est éventuellement substitué par au moins un R^{Zc} ;
R^{Za} et R^{Zb} sont indépendamment choisis parmi hydrogène, méthyle, éthyle, propyle, butyle, pentyle, hexyle, heptyle, octyle, -alcényle en C₂₋₈, -alcynyle en C₂₋₈, cyclopropyle, cyclobutyle, cyclopentyle, cyclohexyle, cycloheptyle, cyclooctyle, hétérocyclyle de 3 à 8 chaînons, phényle et hétéroaryle de 5 à 12 chaînons, où chacun desdits hydrogène, méthyle, éthyle, propyle, butyle, pentyle, hexyle, heptyle, octyle, -alcényle en C₂₋₈, -alcynyle en C₂₋₈, cyclopropyle, cyclobutyle, cyclopentyle, cyclohexyle, cycloheptyle, cyclooctyle, hétérocyclyle de 3 à 8 chaînons, phényle ou hétéroaryle de 5 à 12 chaînons est éventuellement substitué par au moins un substituant R^{Zd} ; et
R^{Zc} et R^{Zd} sont chacun indépendamment -F, -Cl, -Br, -I, hydroxy, méthyle, éthyle, propyle, butyle, pentyle, hexyle, heptyle, octyle, -alcoxy en C₁₋₈, -alcényle en C₂₋₈, -alcynyle en C₂₋₈, cyclopropyle, cyclobutyle, cyclopentyle, cyclohexyle, cycloheptyle, cyclooctyle, hétérocyclyle de 3 à 8 chaînons, phényle ou hétéroaryle de 5 à 12 chaînons ;
L¹ est choisi parmi une liaison simple, -O-, -SO₂-, -C(O)-, -NR^{L1a}-, - (cycloalkylène en C₃-C₈) -, *^{L1}-O- (alkylène en C₁₋₈)-**^{L1}, *^{L1}-(alkylène en C₁₋₈)-O-**^{L1}, *^{L1}-SO₂-(alkylène en C₁₋₈)-**^{L1}, *^{L1}-(alkylène en C₁₋₈)-SO₂-**^{L1}, *^{L1}-CO- (alkylène en C₁₋₈)-**^{L1}, *^{L1}- (alkylène en C₁₋₈) -CO-**^{L1}, *^{L1}-NR^{L1a}-(alkylène en C₁₋₈)-**^{L1}, *^{L1}-(alkylène en C₁₋₈) -NR^{L1a}-**^{L1}, *^{L1}-NR^{L1a}C(O)-**^{L1}, *^{L1}-C (O) NR^{L1a}-**^{L1}, -(alkylène en C₁₋₈)-, - (alcénylène en C₂₋₈)-, -(alcynylène en C₂₋₈)-, - [O(CR^{L1a}R^{L1b})ₘ₄]ₘ₅-, 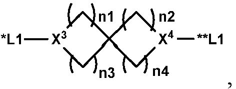 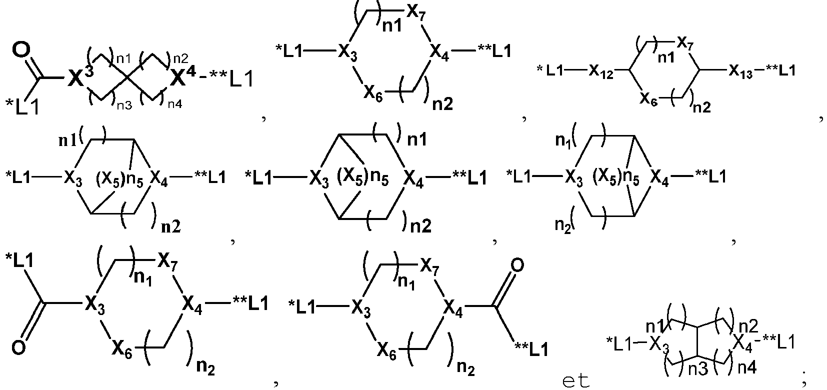
où chacun desdits - (cycloalkylène en C₃-C₈) -, *^{L1}-O-(alkylène en C₁₋₈)-**^{L1}, *^{L1}- (alkylène en C₁₋₈)-O-**^{L1}, *^{L1}-SO₂-(alkylène en C₁₋₈)-**^{L1}, *^{L1}-(alkylène en C₁₋₈)-SO₂-**^{L1}, *^{L1}-CO-(alkylène en C₁₋₈)-**^{L1}, *^{L1}- (alkylène en C₁₋₈)-CO-**^{L1}, *^{L1}-NR^{L1a}-(alkylène en C₁₋₈)-**^{L1}, *^{L1}-(alkylène en C₁₋₈) -NR^{L1a}-**^{L1}, - (alkylène en C₁₋₈)-, - (alcénylène en C₂₋₈)-, - (alcynylène en C₂₋₈)-, 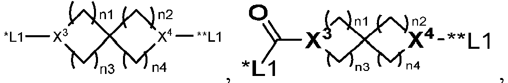 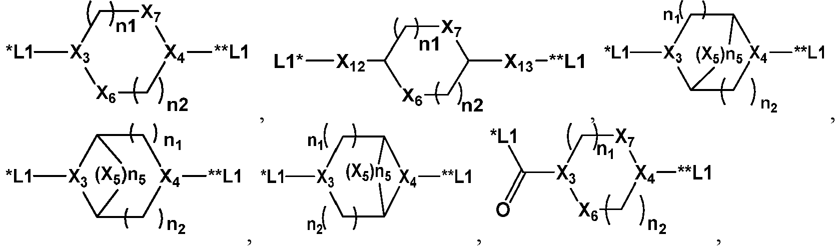 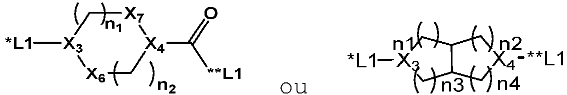 est éventuellement substitué par au moins un R^{L1c},
où *^{L1} désigne la position liée au groupement 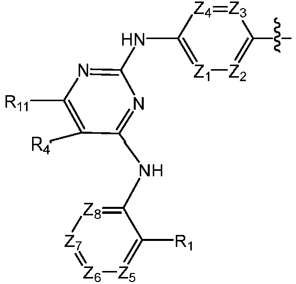 et **^{L1} désigne la position liée au groupement 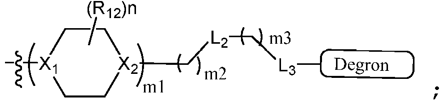
R^{L1a} et R^{L1b} sont chacun indépendamment choisis parmi hydrogène, -alkyle en C₁₋₈, -alcényle en C₂₋₈, -alcynyle en C₂₋₈, cycloalkyle en C₃-C₈, hétérocyclyle de 3 à 8 chaînons, aryle en C₆-C₁₂ et hétéroaryle de 5 à 12 chaînons, où chacun desdits
- alkyle en C₁₋₈, -alcényle en C₂₋₈, -alcynyle en C₂₋₈, cycloalkyle en C₃-C₈, hétérocyclyle de 3 à 8 chaînons, aryle en C₆-C₁₂ ou hétéroaryle de 5 à 12 chaînons est éventuellement substitué par au moins un substituant R^{L1d} ;
chacun desdits R^{L1c}, et R^{L1d} est indépendamment halogène, hydroxy, -alkyle en C₁₋₈, -alcoxy en C₁₋₈, (alcoxy en C₁₋₈)-(alkyle en C₁₋₈)-, -alcényle en C₂₋₈, -alcynyle en C₂₋₈, cycloalkyle en C₃-C₈, hétérocyclyle de 3 à 8 chaînons, aryle en C₆-C₁₂, hétéroaryle de 5 à 12 chaînons ou oxo ;
L² est choisi parmi une liaison simple, -O-, -SO₂-, -CO-, -NR^{L2a}-, - (cycloalkylène en C₃-C₈) -, *^{L2}-O- (alkylène en C₁₋₈)- **^{L2}, *^{L2}- (alkylène en C₁₋₈)-O-**^{L2}, *^{L2}-SO₂- (alkylène en C₁₋₈)- **^{L2}, *^{L2}- (alkylène en C₁₋₈)-SO₂-**^{L2}, *^{L2}-CO- (alkylène en C₁₋₈)- **^{L2}, *^{L2}-(alkylène en C₁₋₈)-CO-**^{L2}, *^{L2}-NR^{L2a}-(alkylène en C₁₋₈)- **^{L2}, *^{L2}-(alkylène en C₁₋₈)-NR^{L2a}-**^{L2}, *^{L2}-NR^{L2a}C(O)-**^{L2}, *^{L2}-C(O)NR^{L2a}-**^{L2}, -(alkylène en C₁₋₈)-, -(alcénylène en C₂₋₈)-, - (alcynylène en C₂₋₈)-, - [O(CR^{L2a}R^{L2b})ₘ₄]ₘ₅-, 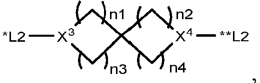 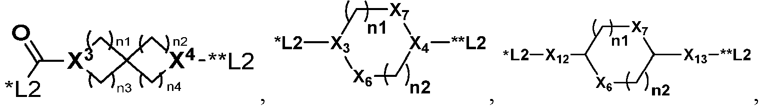 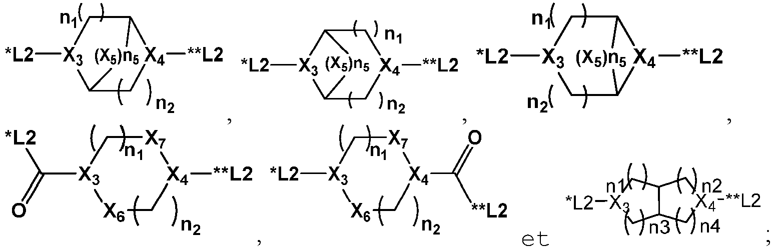
où chacun desdits - (cycloalkylène en C₃-C₈)-, *^{L2}-O-(alkylène en C₁₋₈)-**^{L2}, *^{L2}-(alkylène en C₁₋₈)-O-**^{L2}, *^{L2}-SO₂-(alkylène en C₁₋₈)-**^{L2}, *^{L2}- (alkylène en C₁₋₈)-SO₂-**^{L2}, *^{L2}-CO-(alkylène en C₁₋₈)-**^{L2}, *^{L2}- (alkylène en C₁₋₈)-CO-**^{L2,} *^{L2}-NRL^{2a}-(alkylène en C₁₋₈)-**^{L2}, *^{L2}- (alkylène en C₁₋₈)-NR^{L2a}-**^{L2}, - (alkylène en C₁₋₈)-, - (alcénylène en C₂₋₈)-, - (alcynylène en C₂₋₈)-, 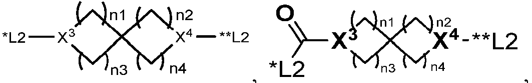 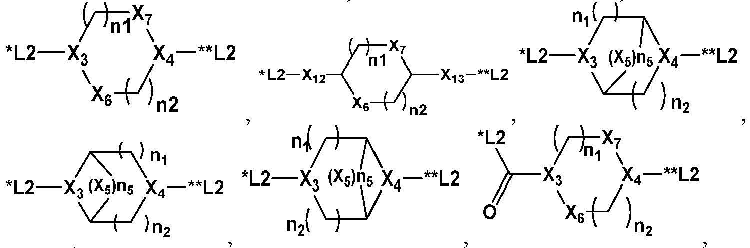 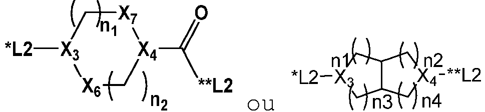 est éventuellement substitué par au moins un substituant R^{L2c} ;
où *^{L2} désigne la position liée au groupement 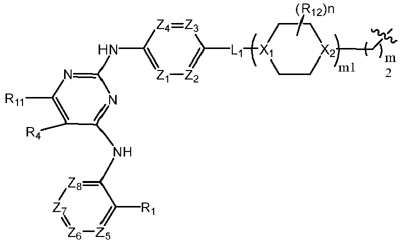 et **^{L2} désigne la position liée au groupement 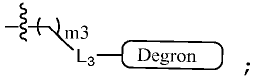
R^{L2a} et R^{L2b} sont chacun indépendamment choisis parmi un hydrogène, -alkyle en C₁₋₈, -alcényle en C₂₋₈, -alcynyle en C₂₋₈, cycloalkyle en C₃-C₈, hétérocyclyle de 3 à 8 chaînons, aryle en C₆-C₁₂ et hétéroaryle de 5 à 12 chaînons, où chacun desdits
- alkyle en C₁₋₈, -alcényle en C₂₋₈, -alcynyle en C₂₋₈, cycloalkyle en C₃-C₈, hétérocyclyle de 3 à 8 chaînons, aryle en C₆-C₁₂ ou hétéroaryle de 5 à 12 chaînons est éventuellement substitué par au moins un substituant R^{L2d} ;
chacun desdits R^{L2c} et R^{L2d} est indépendamment un halogène, hydroxy, -alkyle en C₁₋₈, -alcoxy en C₁₋₈, (alcoxy en C₁₋₈)-(alkyle en C₁₋₈)-, -alcényle en C₂₋₈, -alcynyle en C₂₋₈, cycloalkyle en C₃-C₈, hétérocyclyle de 3 à 8 chaînons, aryle en C₆-C₁₂, hétéroaryle de 5 à 12 chaînons ou oxo ;
L³ est choisi parmi une liaison simple, -O-, -SO₂-, -CO-, -NR^{L3a}-, - (cycloalkylène en C₃-C₈) -, *^{L3}-O- (alkylène en C₁₋₈)-**^{L3}, *^{L3}-(alkylène en C₁₋₈)-O-**^{L3}, *^{L3}-SO₂-(alkylène en C₁₋₈)-**^{L3}, *^{L3}-(alkylène en C₁₋₈)-SO₂-**^{L3}, *^{L3}-CO- (alkylène en C₁₋₈)- **^{L3}, *^{L3}- (alkylène en C₁₋₈)-Co-**^{L3}, *^{L3}-NR^{L3a}-(alkylène en C₁₋₈)- **^{L3}, *^{L3}-(alkylène en C₁₋₈)-NR^{L3a}-**^{L3}, *^{L3}-NR^{L3a}C(O)-**^{L3}, *^{L3}-C(O)NR^{L3a-}**^{L3}, -(alkylène en C₁₋₈)-, - (alcénylène en C₂₋₈)-, - (alcynylène en C₂₋₈)-, -[O(CR^{L3a}R^{L3b})ₘ₄]ₘ₅-, 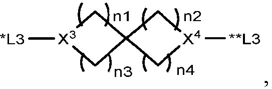 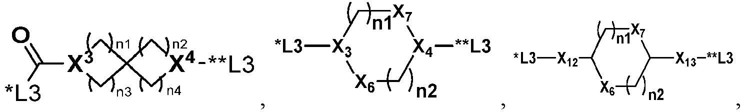 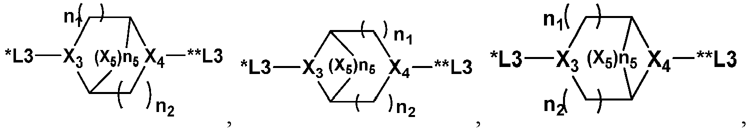 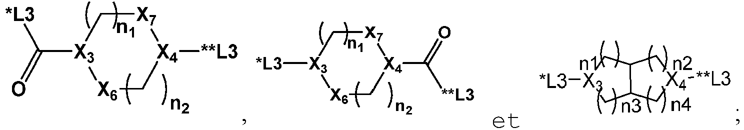
où chacun desdits - (cycloalkylène en C₃-C₈) -, *^{L3}-O-(alkylène en C₁₋₈)-**^{L3}, *^{L3}-(alkylène en C₁₋₈)-O-**^{L3}, *^{L3}-SO₂-(alkylène en C₁₋₈)-**^{L3}, *^{L3}-(alkylène en C₁₋₈)-SO₂-**^{L3}, *^{L3}-CO-(alkylène en C₁₋₈)-**^{L3}, *^{L3}-(alkylène en C₁₋₈)-CO-**^{L3}, *^{L3}-NR^{L3a}-(alkylène en C₁₋₈)-**^{L3}, *^{L3}-(alkylène en C₁₋₈)-NR^{L3a}-**^{L3}, - (alkylène en C₁₋₈)-, - (alcénylène en C₂₋₈)-, - (alcynylène en C₂₋₈)-, 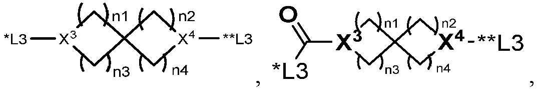 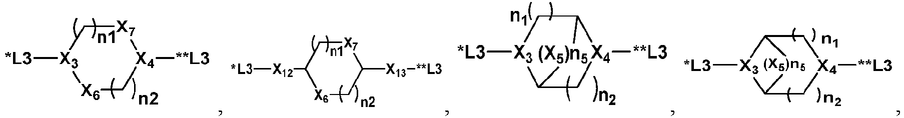 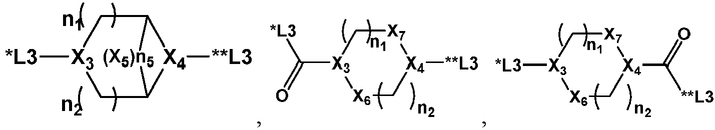 ou 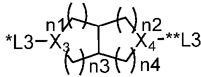 est éventuellement substitué par au moins un substituant R^{L3c} ;
où *^{L3} désigne la position liée au groupement 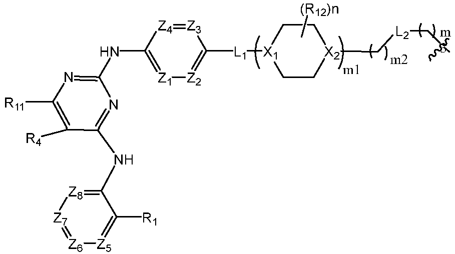 et **^{L3} désigne la position liée au groupement 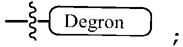
R^{L3a} et R^{L3b} sont chacun indépendamment choisis parmi un hydrogène, -alkyle en C₁₋₈, -alcényle en C₂₋₈, -alcynyle en C₂₋₈, cycloalkyle en C₃-C₈, hétérocyclyle de 3 à 8 chaînons, aryle en C₆-C₁₂ et hétéroaryle de 5 à 12 chaînons, où chacun desdits
- alkyle en C₁₋₈, -alcényle en C₂₋₈, -alcynyle en C₂₋₈, cycloalkyle en C₃-C₈, hétérocyclyle de 3 à 8 chaînons, aryle en C₆-C₁₂ ou hétéroaryle de 5 à 12 chaînons est éventuellement substitué par au moins un substituant R^{L3d} ;
chacun desdits R^{L3c} et R^{L3d} est indépendamment un halogène, hydroxy, -alkyle en C₁₋₈, -alcoxy en C₁₋₈, (alcoxy en C₁₋₈)-(alkyle en C₁₋₈)-, -alcényle en C₂₋₈, -alcynyle en C₂₋₈, cycloalkyle en C₃-C₈, hétérocyclyle de 3 à 8 chaînons, aryle en C₆-C₁₂, hétéroaryle de 5 à 12 chaînons ou oxo ; 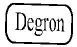
est choisi parmi 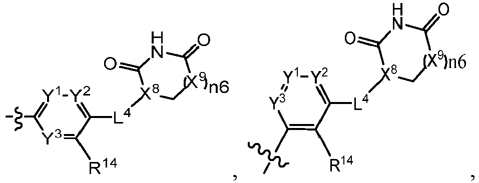 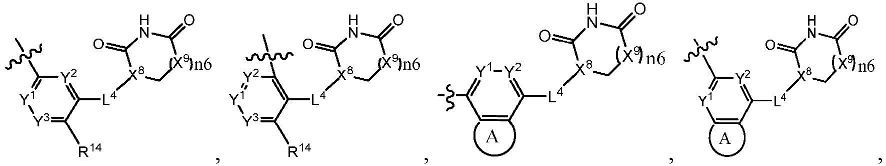 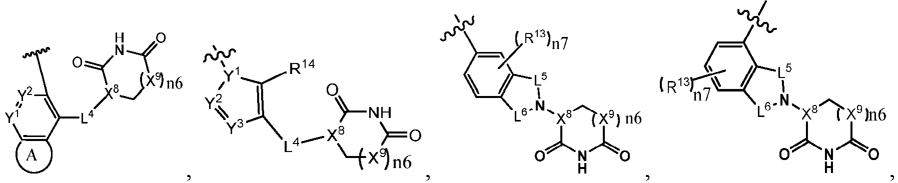 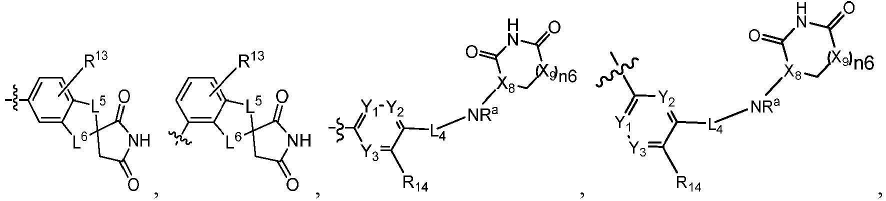 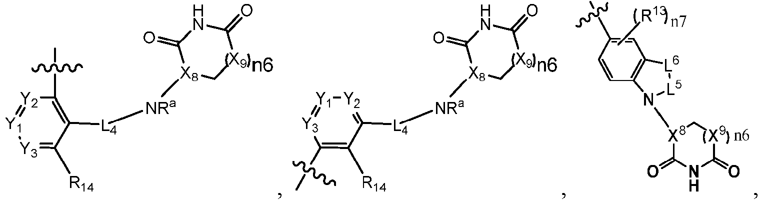 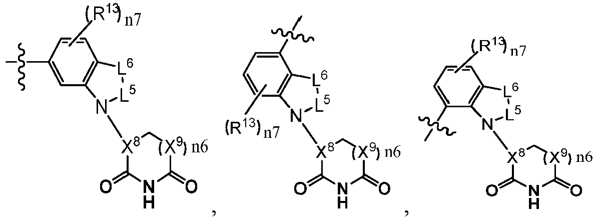 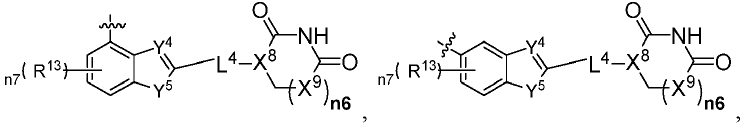 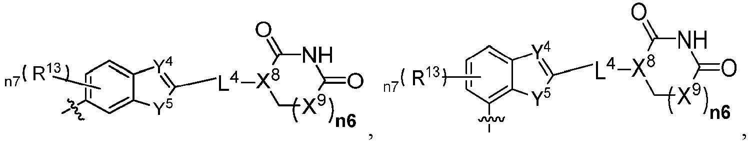 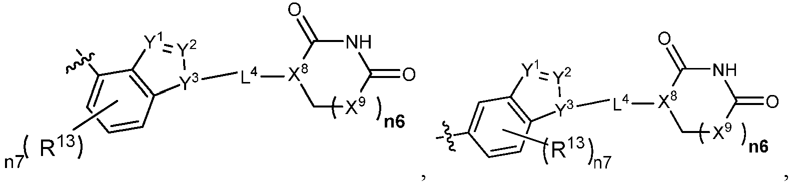 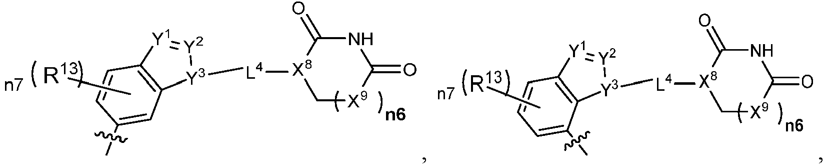 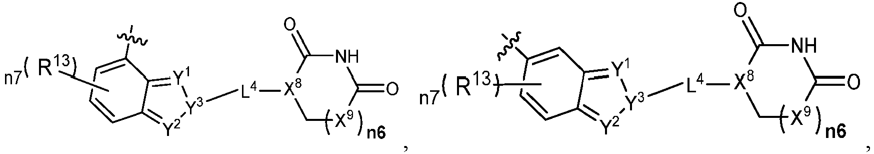 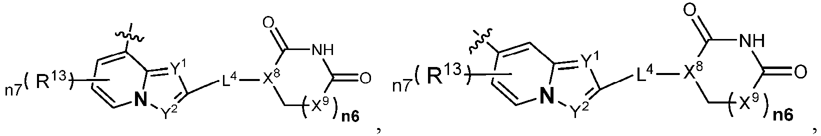 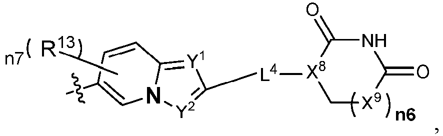 et 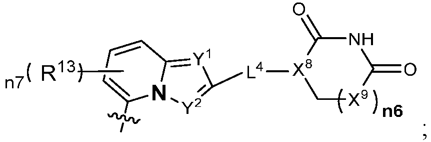
le cycle A est choisi parmi un cycloalkyle de 3 à 12 chaînons, un hétérocyclyle de 3 à 12 chaînons, un aryle et un hétéroaryle ;
R¹³ et R¹⁴ sont indépendamment choisis parmi un hydrogène, halogène, CN, -alkyle en C₁₋₈, -alcényle en C₂₋₈, -alcynyle en C₂₋₈, -alcoxy en C₁₋₈, cycloalkyle en C₃-C₈, hétérocyclyle de 3 à 8 chaînons, aryle en C₆-C₁₂ et hétéroaryle de 5 à 12 chaînons ; chaque dit -alkyle en C₁₋₈, -alcényle en C₂₋₈, - alcynyle en C₂₋₈, -alcoxy en C₁₋₈, cycloalkyle en C₃-C₈, hétérocyclyle de 3 à 8 chaînons, aryle en C₆-C₁₂ ou hétéroaryle de 5 à 12 chaînons est éventuellement substitué par au moins un substituant halogène, -alkyle en C₁₋₈, (alcoxy en C₁₋₈)-(alkyle en C₁₋₈)-, -alcényle en C₂₋₈, -alcynyle en C₂₋₈, cycloalkyle en C₃-C₈, hétérocyclyle de 3 à 8 chaînons, aryle en C₆-C₁₂ ou hétéroaryle de 5 à 12 chaînons ;
X¹, X², X³, X⁴ et X⁸ sont chacun indépendamment choisis parmi -CR^{a} et N ;
X⁵, X⁶, X⁷ et X⁹ sont chacun indépendamment choisis parmi -NR^{a}-, -O-, -S- et -CR^{a}R^{b}- ;
X¹² et X¹³ sont chacun indépendamment choisis parmi -NR^{a}- et -O- ;
L⁴, L⁵ et L⁶ sont chacun indépendamment choisis parmi une liaison simple, -O-, -NR^{a}-, -(CR^{a}R^{b})ₙ₈-, -O(CR^{a}R^{b})ₙ₈-, -NR^{a}(CR^{a}R^{b})ₙ₈- et -C(O)- ;
Y¹, Y², Y³ et Y⁴ sont chacun indépendamment choisis parmi CR^{a} et N ;
Y⁵ est choisi parmi NR^{a}, O et S ;
R^{a} et R^{b} sont chacun indépendamment choisis parmi un hydrogène (H, D ou T), halogène, CN, -alkyle en C₁₋₈, -alcoxy en C₁₋₈, -alcényle en C₂₋₈, -alcynyle en C₂₋₈, -cycloalkyle en C₃-C₈, hétérocyclyle de 3 à 8 chaînons, -aryle en C₆-C₁₂ et hétéroaryle de 5 à 12 chaînons, où chacun desdits -alkyle en C₁₋₈, -alcoxy en C₁₋₈, -alcényle en C₂₋₈, -alcynyle en C₂₋₈, -cycloalkyle en C₃-C₈, hétérocyclyle de 3 à 8 chaînons, -aryle en C₆-C₁₂ ou hétéroaryle de 5 à 12 chaînons est éventuellement substitué par au moins un substituant halogène, hydroxy, halogène, -alkyle en C₁₋₈, -alcoxy en C₁₋₈, -alcényle en C₂₋₈, -alcynyle en C₂₋₈, -cycloalkyle en C₃-C₈, hétérocyclyle de 3 à 8 chaînons, -aryle en C₆-C₁₂ ou hétéroaryle de 5 à 12 chaînons ; ou
R^{a} et R^{b} forment, conjointement avec les atomes de carbone auxquels ils sont liés, un cycle de 3 à 12 chaînons, ledit cycle comprenant 0 à 3 hétéroatomes indépendamment choisis parmi l'azote, l'oxygène et le soufre ; lequel cycle est éventuellement substitué par au moins un substituant halogène, hydroxy, -alkyle en C₁₋₈, -alcényle en C₂₋₈, - alcynyle en C₂₋₈, -alcoxy en C₁₋₈, -alcényle en C₂₋₈, -alcynyle en C₂₋₈, cycloalkyle en C₃-C₈, hétérocyclyle de 3 à 8 chaînons, aryle en C₆-C₁₂ ou hétéroaryle de 5 à 12 chaînons ;
m₁ vaut 0 ou 1 ;
m₂ et m₃ valent chacun indépendamment 0, 1, 2, 3, 4, 5, 6, 7 ou 8 ;
m₄ et m₅ valent chacun indépendamment 0, 1, 2 ou 3 ;
n, n₁, n₂, n₃, n₄ et n₅ valent chacun indépendamment 0, 1, 2 ou 3 ; et
n₆ vaut 0, 1, 2, 3 ou 4 ;
n₇ vaut 0, 1, 2 ou 3 ;
n₈ vaut 0, 1, 2, 3, 4, 5, 6, 7 ou 8.

2. Composé selon la revendication 1, dans lequel R¹ est
-P(O)(CH₃)₂.

3. Composé selon l'une quelconque des revendications 1 à 2, dans lequel :
R² et R³ forment, conjointement avec les atomes de carbone auxquels ils sont liés, un cycle aromatique à 5 ou 6 chaînons, ledit cycle comprenant 0, 1 ou 2 hétéroatomes indépendamment choisis parmi l'azote, l'oxygène et le soufre ; lequel cycle est éventuellement substitué par au moins un substituant R^{2e} ;
R^{2e}, à chaque occurrence, est indépendamment hydrogène, -F, -Cl, -Br, -I, méthyle, éthyle, propyle, butyle, pentyle, hexyle, heptyle, octyle, méthoxy, éthoxy, propoxy, butoxy, pentoxy, hexoxy, heptoxy, octoxy, cyclopropyle, cyclobutyle, cyclopentyle, cyclohexyle, cycloheptyle, cyclooctyle ou oxo, où chacun parmi méthyle, éthyle, propyle, butyle, pentyle, hexyle, heptyle, octyle, méthoxy, éthoxy, propoxy, butoxy, pentoxy, hexoxy, heptoxy, octoxy, cyclopropyle, cyclobutyle, cyclopentyle, cyclohexyle, cycloheptyle ou cyclooctyle est éventuellement substitué par au moins un substituant R^{2d} ; et
R^{2d}, à chaque occurrence, est indépendamment -F, -Cl, - Br, -I, -OH, méthyle, éthyle, propyle, butyle, pentyle, hexyle, heptyle, octyle, méthoxy, éthoxy, propoxy, butoxy, pentoxy, hexoxy, heptoxy, octoxy, cyclopropyle, cyclobutyle, cyclopentyle, cyclohexyle, cycloheptyle, cyclooctyle ou phényle.

4. Composé selon l'une quelconque des revendications 1 à 3, dans lequel le groupement 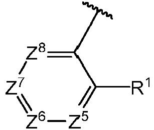 est 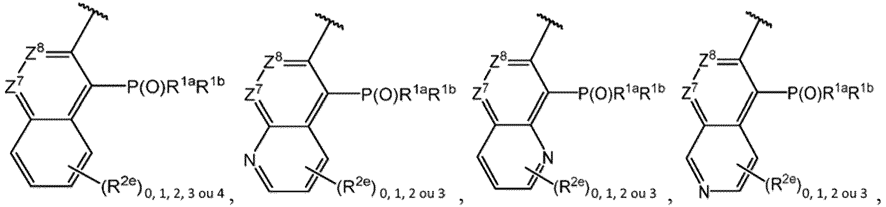 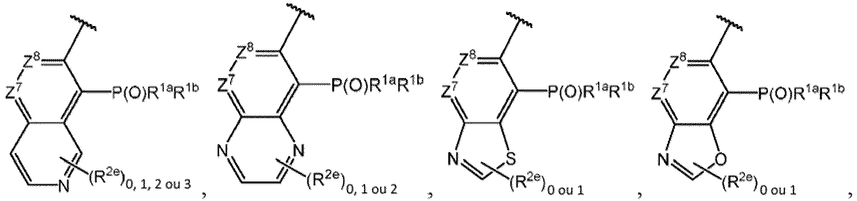 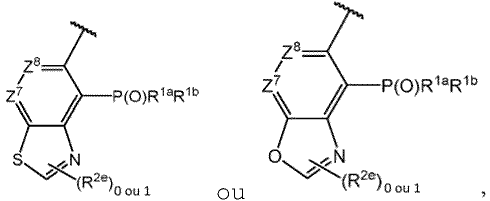 où R^{2e}, Z⁷, Z⁸, R^{1a} et R^{1b} sont tels que définis dans la revendication 1.

5. Composé selon l'une quelconque des revendications 1 à 4, dans lequel :
(i) R^{2e}, à chaque occurrence, est indépendamment hydrogène, -F, -Cl, -Br, -I, -CH₃, -CH₂CH₃, -CH₂CH₂CH₃, - CH(CH₃)₂, -CH₂CH₂CH₂CH₃, -CH(CH₃)CH₂CH₃, -CH₂CH(CH₃)₂, -C(CH₃)₃, -CF₃, 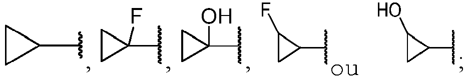 et/ou
(ii) le groupement 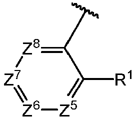 est

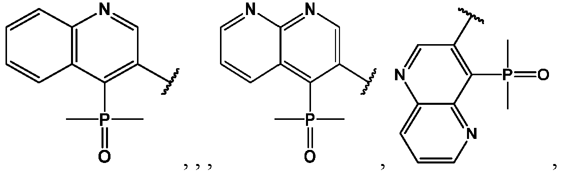

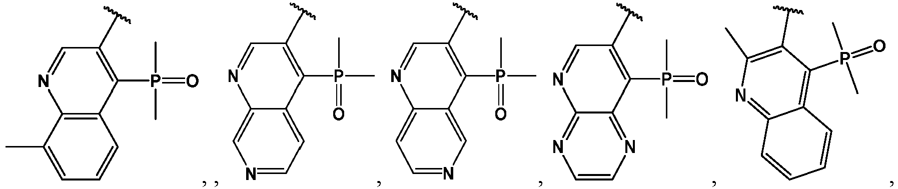

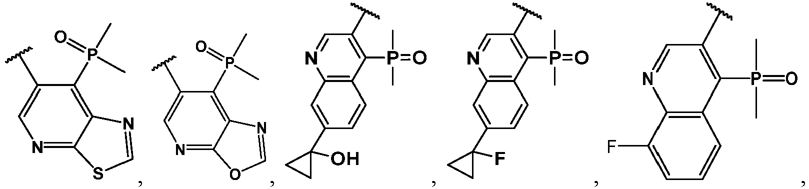

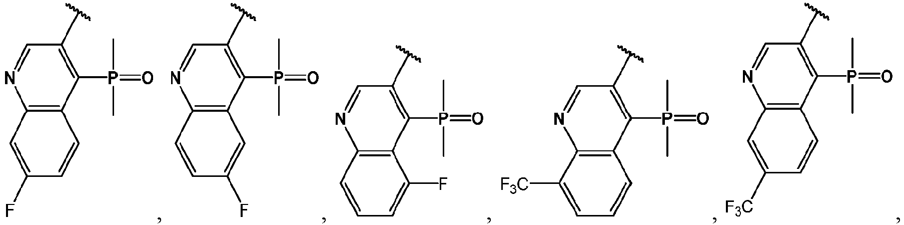

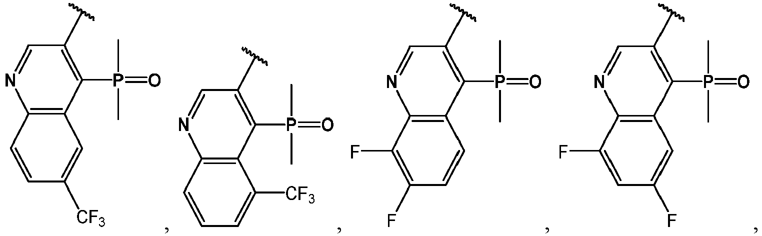

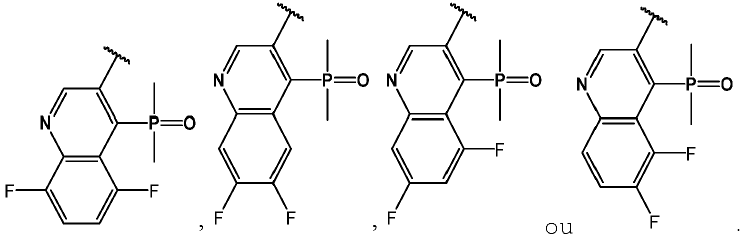

6. Composé selon l'une quelconque des revendications 1 à 5, dans lequel :
R¹⁰, R¹¹ et R¹² sont chacun indépendamment choisis parmi hydrogène, F, Cl, Br, -NH₂, -CH₃, -C₂H₅, -C₃H₇, -CH₂F, -CHF₂, -CF₃, -C₄H₉, -C₅H₁₁, -OCH₃, -OC₂H₅, -OC₃H₇, -OC₄H₉, -OC₅H₁₁, -CN, cyclopropyle et oxo ; ou
deux R¹² forment, conjointement avec les atomes de carbone auxquels ils sont liés, un cycle à 3, 4, 5, 6, 7 ou 8 chaînons, ledit cycle comprenant 0, 1, 2 ou 3 hétéroatomes indépendamment choisis parmi l'azote, l'oxygène et le soufre ; lequel cycle est éventuellement substitué par au moins un substituant -H, -F, -Cl, -Br, -I, méthyle, éthyle, propyle, butyle, -NH₂, -NHCH₃, -OH, -OCH₃, -OC₂H₅, cyclopropyle, cyclobutyle, cyclopentyle ou cyclohexyle.

7. Composé selon l'une quelconque des revendications 1 à 6, dans lequel R⁴ est choisi parmi -H, -F, -Cl, -Br, -I, -CH₃, -C₂H₅, -C₃H₇, -C₄H₉, cyclopropyle et cyclobutyle, où chacun parmi -CH₃, -C₂H₅, -C₃H₇, -C₄H₉, cyclopropyle ou cyclobutyle est éventuellement substitué par -F, -Cl, -Br, - I, -alkyle en C₁₋₈, -alcényle en C₂₋₈, -alcynyle en C₂₋₈, - cycloalkyle en C₃-C₈, hétérocyclyle de 3 à 8 chaînons, aryle en C₆-C₁₂, hétéroaryle de 5 à 12 chaînons, oxo, -CN, -OR^{4c}, - SO₂R^{4c}, -SO₂NR^{4c}R^{4d}, -COR^{4c}, -CO₂R^{4c}, -CONR^{4c}R^{4d}, -NR^{4c}R^{4d}, - NR^{4c}COR^{4d}, -NR^{4c}CO₂R^{4d} ou -NR^{4c}SO₂R^{4d} ;
R^{4c} et R^{4d} sont chacun indépendamment hydrogène, -alkyle en C₁₋₈, -alcényle en C₂₋₈, -alcynyle en C₂₋₈, cycloalkyle en C₃-C₈, hétérocyclyle de 3 à 8 chaînons, aryle en C₆-C₁₂ ou hétéroaryle de 5 à 12 chaînons.

8. Composé selon l'une quelconque des revendications 1 à 7, dans lequel :
(i) L¹ est choisi parmi une liaison simple, -O-, -SO₂-, -C(O)-, -NR^{L1a}-, - (cycloalkylène en C₃-C₈)-, *^{L1}-O-(alkylène en C₁₋₈)-**^{L1}, *^{L1}- (alkylène en C₁₋₈)-O-**^{L1}, *^{L1}-SO₂-(alkylène en C₁₋₈)-**^{L1}, *^{L1}- (alkylène en C₁₋₈)-SO₂-*^{L1}, *^{L1}-CO-(alkylène en C₁₋₈)-**^{L1}, *^{L1}- (alkylène en C₁₋₈)-CO-**^{L1}, *^{L1}-NR^{L1a}-(alkylène en C₁₋₈)-**^{L1}, *^{L1}-(alkylène en C₁₋₈)-NR^{L1a}-**^{L1}, *^{L1}-NR^{L1a}C(O)-**^{L1}, *^{L1}-C(O)NR^{L1a}-**^{L1}, - (alkylène en C₁₋₈)-, - (alcénylène en C₂₋₈)-, -(alcynylène en C₂₋₈)-, -[O(CR^{L1a}R^{L1b})ₘ₄]ₘ₅-, 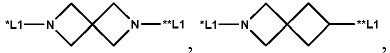 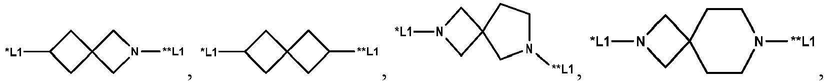 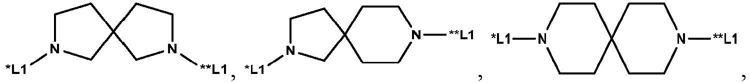 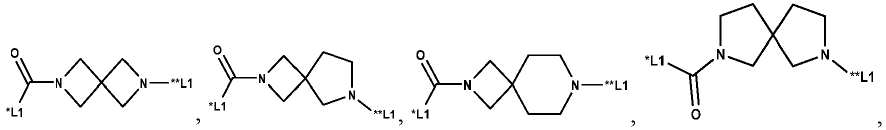 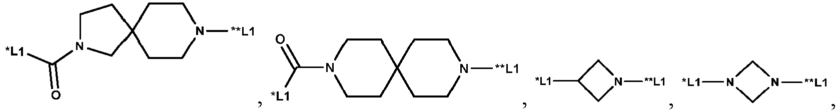 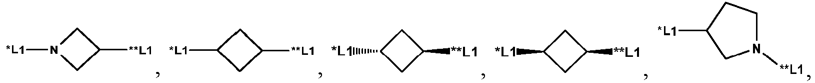 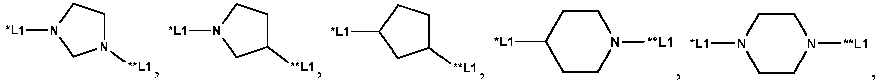 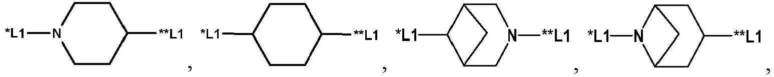 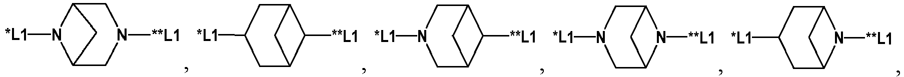 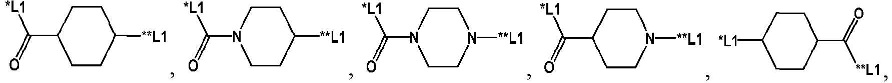 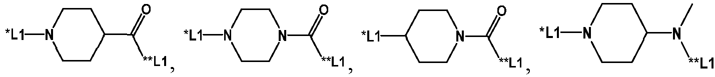 et 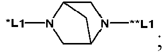
où chacun desdits - (cycloalkylène en C₃-C₈) -, *^{L1}-O-(alkylène en C₁₋₈)-**^{L1}, *^{L1}-(alkylène en C₁₋₈)-O-**^{L1}, *^{L1}-SO₂-(alkylène en C₁₋₈)-**^{L1}, *^{L1}- (alkylène en C₁₋₈)-SO₂-**^{L1}, *^{L1}-CO-(alkylène en C₁₋₈)-**^{L1}, *^{L1}-(alkylène en C₁₋₈)-CO-**^{L1}, *^{L1}-NR^{L1a}-(alkylène en C₁₋₈)-**^{L1}, *^{L1}-(alkylène en C₁₋₈)-NR^{L1a}-**^{L1} - (alkylène en C₁₋₈)-, - (alcénylène en C₂₋₈)-, - (alcynylène en C₂₋₈)-, 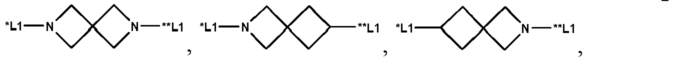 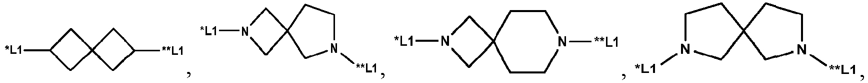 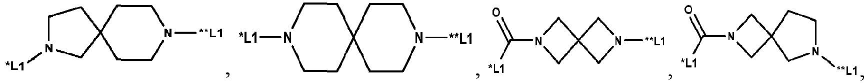 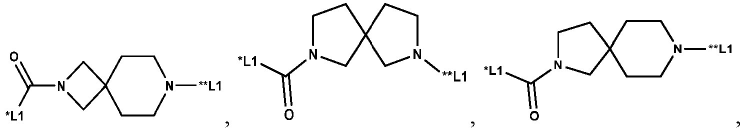 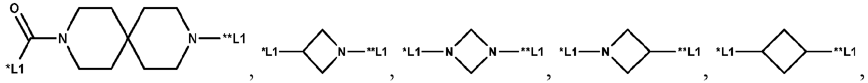 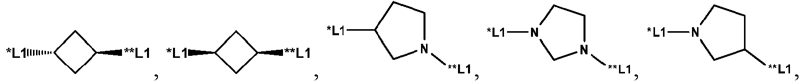 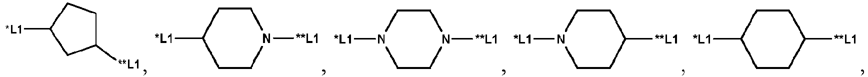 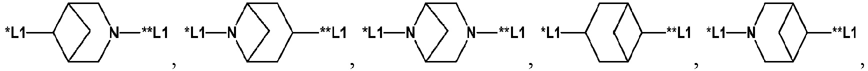 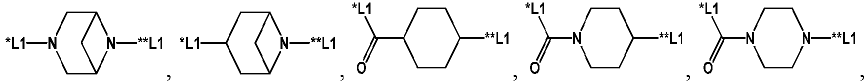 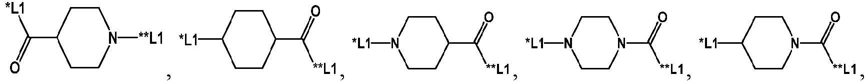 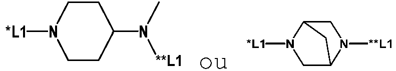 est éventuellement substitué par au moins un R^{L1c} ;
R^{L1a} et R^{L1b} sont chacun indépendamment choisis parmi hydrogène, méthyle, éthyle, propyle, butyle, pentyle, hexyle, heptyle, octyle, vinyle, éthynyle, cyclopropyle, cyclobutyle, cyclopentyle, cyclohexyle, cycloheptyle, cyclooctyle, oxazolidinyle, imidazolidinyle, thiazolidinyle, pyrazolidinyle, morpholinyle, pipéridinyle, pipérazinyle, oxazinyle, imidazolyle, thiazolyle, oxazolyle, pyridyle, pyrimidinyle, pyrazinyle, pyridazinyle, phényle, pyrrolyle, imidazolyle, pyrazolyle, thiazolyle, oxazolyle, triazolyle, thiophényle, furanyle, pyridyle, pyrimidinyle et pyrazinyle, où chacun desdits méthyle, éthyle, propyle, butyle, pentyle, hexyle, heptyle, octyle, vinyle, éthynyle, cyclopropyle, cyclobutyle, cyclopentyle, cyclohexyle, cycloheptyle, cyclooctyle, oxazolidinyle, imidazolidinyle, thiazolidinyle, pyrazolidinyle, morpholinyle, pipéridinyle, pipérazinyle, oxazinyle, imidazolyle, thiazolyle, oxazolyle, pyridyle, pyrimidinyle, pyrazinyle, pyridazinyle, phényle, pyrrolyle, imidazolyle, pyrazolyle, thiazolyle, oxazolyle, triazolyle, thiophényle, furanyle, pyridyle, pyrimidinyle ou pyrazinyle est éventuellement substitué par au moins un substituant R^{L1d} ; et
chacun desdits R^{L1c}, et R^{L1d} sont indépendamment -F, -Cl, -Br, -I, -OH, méthyle, éthyle, propyle, butyle, pentyle, hexyle, heptyle, octyle, vinyle, éthynyle, méthoxy, éthoxy, propoxy, butoxy, pentoxy, hexoxy, heptoxy, octoxy, cyclopropyle, cyclobutyle, cyclopentyle, cyclohexyle, cycloheptyle, cyclooctyle, oxazolidinyle, imidazolidinyle, thiazolidinyle, pyrazolidinyle, morpholinyle, pipéridinyle, pipérazinyle, oxazinyle, imidazolyle, thiazolyle, oxazolyle, pyridyle, pyrimidinyle, pyrazinyle, pyridazinyle, phényle, pyrrolyle, imidazolyle, pyrazolyle, thiazolyle, oxazolyle, triazolyle, thiophényle, furanyle, pyridyle, pyrimidinyle, pyrazinyle ou oxo ;
ou
(ii) L¹ est choisi parmi une liaison simple, -(alkylène en C₁₋₈)- (de préférence -CH₂-, -C₂H₄-, -C₃H₆-), -CO-, -O-, - N(CH₃)-, -NH-,

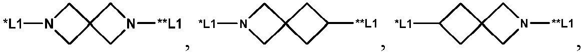

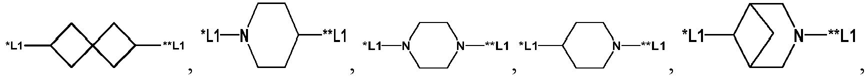

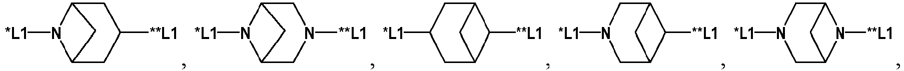

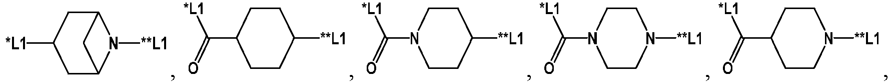

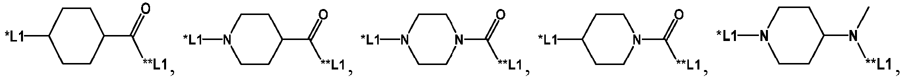

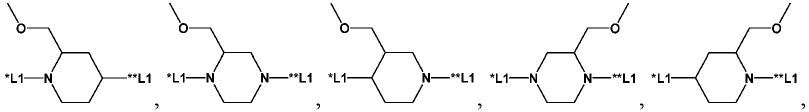

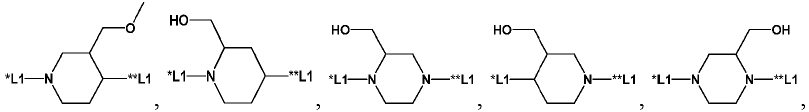

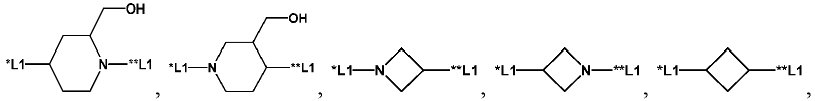

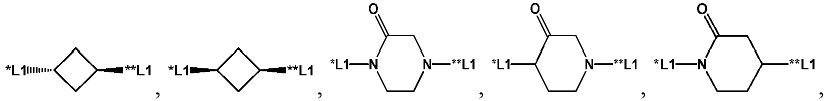

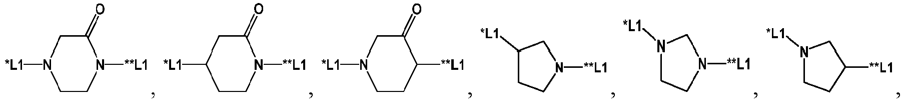

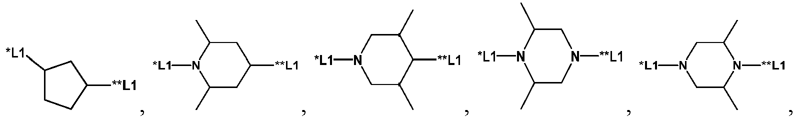

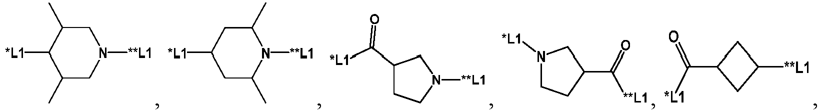

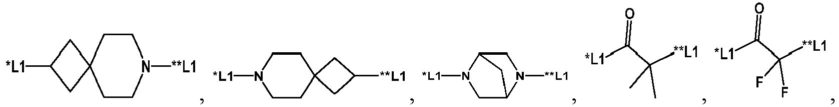

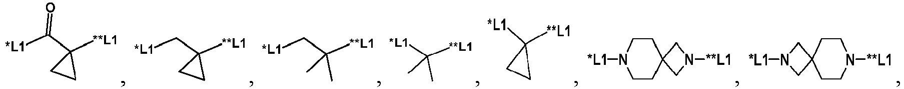

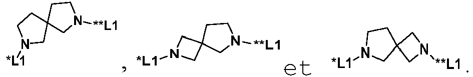

9. Composé selon l'une quelconque des revendications 1 à 8, dans lequel :
(i) X¹ et X² sont chacun indépendamment choisis parmi - CR^{a} et N ;
R^{a} est choisi parmi hydrogène, -F, -Cl, -Br, -I, CN, méthyle, éthyle, méthoxy, éthoxy et cyclopropyle, où chacun desdits méthyle, éthyle, méthoxy, éthoxy ou cyclopropyle est éventuellement substitué par au moins un substituant -F, - Cl, -Br, -I, hydroxy, méthyle, éthyle (de préférence, X¹ et X² sont chacun indépendamment choisis parmi CH, C(F), C(CH₃) et N) ;
m₁ vaut 1 ou 0 ; et
R¹² est hydrogène, oxo, méthoxyméthyle, hydroxyméthyle, -CN ou -CH₃ ;
et/ou
(ii) m1 vaut 1 ; de préférence, le groupement 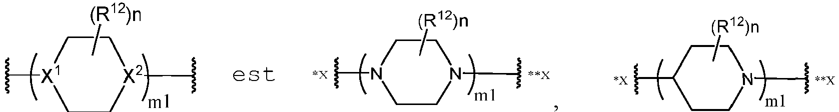 ou 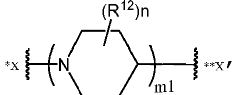 où *^{x} désigne la position liée au groupement 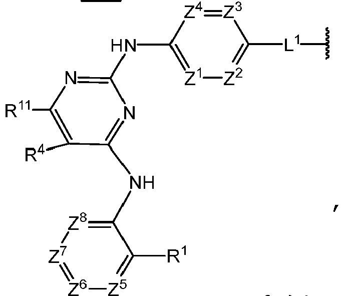 et **^{x} désigne la position liée au 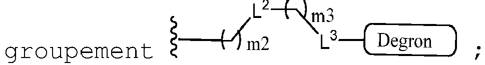 et/ou
(iii) m₁ vaut 0.

10. Composé selon l'une quelconque des revendications 1 à 9, dans lequel :
(i) le groupement 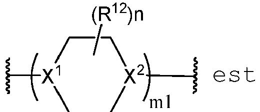 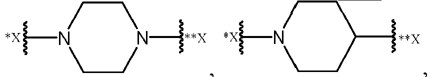 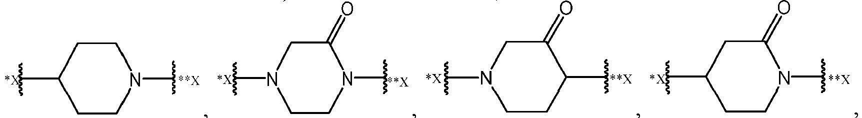 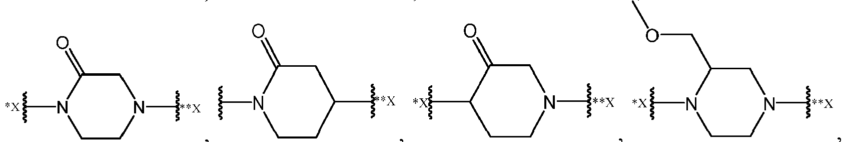 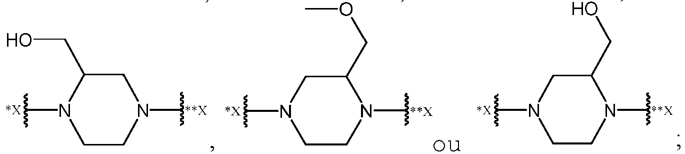 ou
(ii) m₂ est choisi parmi 0, 1, 2, 3, 4 et 5 ;
ou
(iii) L² est choisi parmi une liaison simple, -O-, -SO₂-, -CO-, -NR^{L2a}-, -(cycloalkylène en C₃-C₈)-, *^{L2}-O-(alkylène en C₁₋₈)-**^{L2}, *^{L2}- (alkylène en C₁₋₈)-O-**^{L2}, *^{L2}-SO₂- (alkylène en C₁₋₈)-**^{L2}, *^{L2}- (alkylène en C₁₋₈)-SO₂-**^{L2} *^{L2}-CO- (alkylène en C₁₋₈)-**^{L2}, *^{L2}- (alkylène en C₁₋₈)-CO-**^{L2}, *^{L2}-NR^{L2a}-(alkylène en C₁₋₈)-**^{L2}, *^{L2}-(alkylène en C₁₋₈)-NR^{L2a}-**^{L2}, *^{L2}-NR^{L2a}C(O)-**^{L2}, *^{L2}-C(O)NR^{L2a}-**^{L2}, -(alkylène en C₁₋₈)-, - (alcénylène en C₂₋₈)-, -(alcynylène en C₂₋₈)-, -[O(CR^{L2a}R^{L2b})ₘ₄]ₘ₅-, 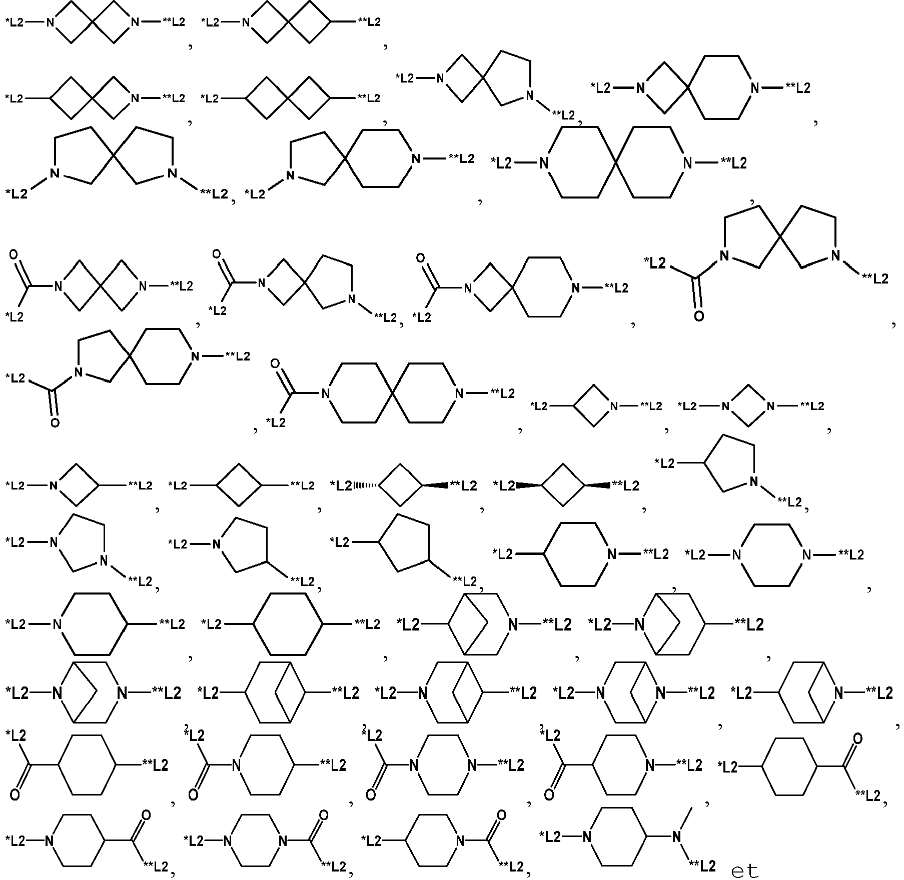 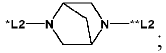
où chacun desdits - (cycloalkylène en C₃-C₈) -, *^{L2}-O-(alkylène en C₁₋₈)-**^{L2}, *^{L2}-(alkylène en C₁₋₈)-O-**^{L2}, *^{L2}-SO₂-(alkylène en C₁₋₈)-**^{L2}, *^{L2}-(alkylène en C₁₋₈)-SO₂-**^{L2}, *^{L2}-CO-(alkylène en C₁₋₈)-**^{L2}, *^{L2}-(alkylène en C₁₋₈)-CO-**^{L2}, *^{L2}-NR^{L2a}-(alkylène en C₁₋₈)-**^{L2}, *^{L2}-(alkylène en C₁₋₈)-NR^{L2a}-**^{L2}, - (alkylène en C₁₋₈)-, - (alcénylène en C₂₋₈)-, - (alcynylène en C₂₋₈)-, 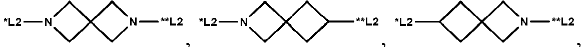 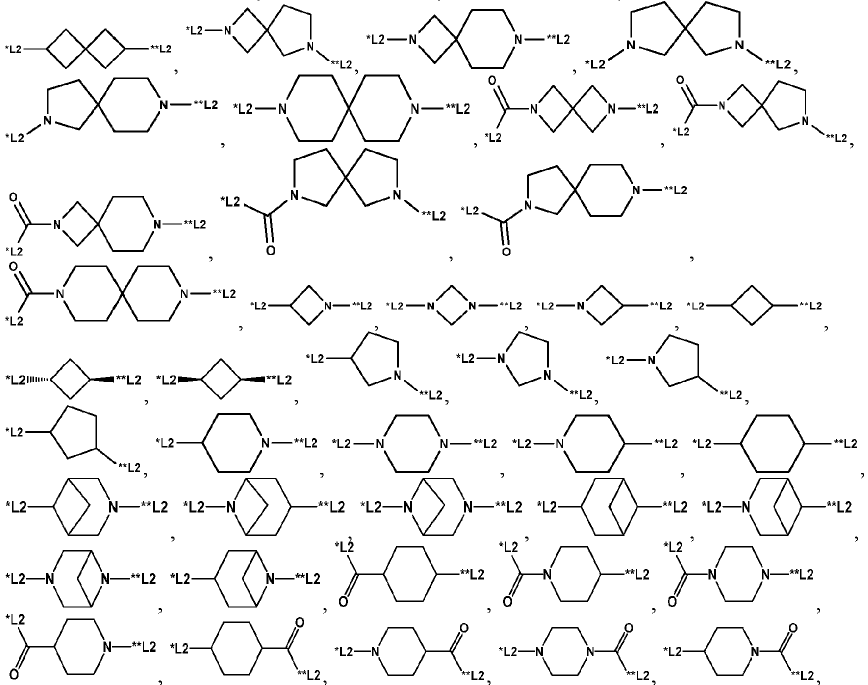 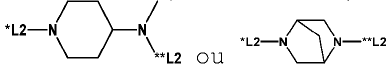 est éventuellement substitué par au moins un substituant R^{L2c} ;
R^{L2a} et R^{L2b} sont chacun indépendamment choisis parmi hydrogène, méthyle, éthyle, propyle, butyle, pentyle, hexyle, heptyle, octyle, vinyle, éthynyle, cyclopropyle, cyclobutyle, cyclopentyle, cyclohexyle, cycloheptyle, cyclooctyle, oxazolidinyle, imidazolidinyle, thiazolidinyle, pyrazolidinyle, morpholinyle, pipéridinyle, pipérazinyle, oxazinyle, imidazolyle, thiazolyle, oxazolyle, pyridyle, pyrimidinyle, pyrazinyle, pyridazinyle, phényle, pyrrolyle, imidazolyle, pyrazolyle, thiazolyle, oxazolyle, triazolyle, thiophényle, furanyle, pyridyle, pyrimidinyle et pyrazinyle, où chacun desdits méthyle, éthyle, propyle, butyle, pentyle, hexyle, heptyle, octyle, vinyle, éthynyle, cyclopropyle, cyclobutyle, cyclopentyle, cyclohexyle, cycloheptyle, cyclooctyle, oxazolidinyle, imidazolidinyle, thiazolidinyle, pyrazolidinyle, morpholinyle, pipéridinyle, pipérazinyle, oxazinyle, imidazolyle, thiazolyle, oxazolyle, pyridyle, pyrimidinyle, pyrazinyle, pyridazinyle, phényle, pyrrolyle, imidazolyle, pyrazolyle, thiazolyle, oxazolyle, triazolyle, thiophényle, furanyle, pyridyle, pyrimidinyle ou pyrazinyle est éventuellement substitué par au moins un substituant R^{L2d} ; et
chacun desdits R^{L2c} et R^{L2d} est indépendamment -F, -Cl, -Br, -I, -OH, méthyle, éthyle, propyle, butyle, pentyle, hexyle, heptyle, octyle, vinyle, éthynyle, cyclopropyle, cyclobutyle, cyclopentyle, cyclohexyle, cycloheptyle, cyclooctyle, oxazolidinyle, imidazolidinyle, thiazolidinyle, pyrazolidinyle, morpholinyle, pipéridinyle, pipérazinyle, oxazinyle, imidazolyle, thiazolyle, oxazolyle, pyridyle, pyrimidinyle, pyrazinyle, pyridazinyle, phényle, pyrrolyle, imidazolyle, pyrazolyle, thiazolyle, oxazolyle, triazolyle, thiophényle, furanyle, pyridyle, pyrimidinyle, pyrazinyle ou oxo.

11. Composé selon l'une quelconque des revendications 1 à 10, dans lequel :
(i) L² est choisi parmi une liaison simple, - (alkylène en C₁₋₈)- (de préférence -CH₂-, -C₂H₄-, -C₃H₆-), -CO-, -O-, - N(CH₃)-, -NH-, 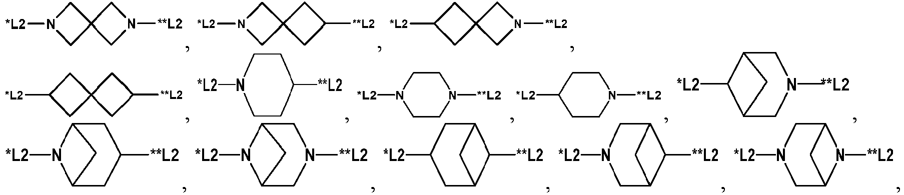 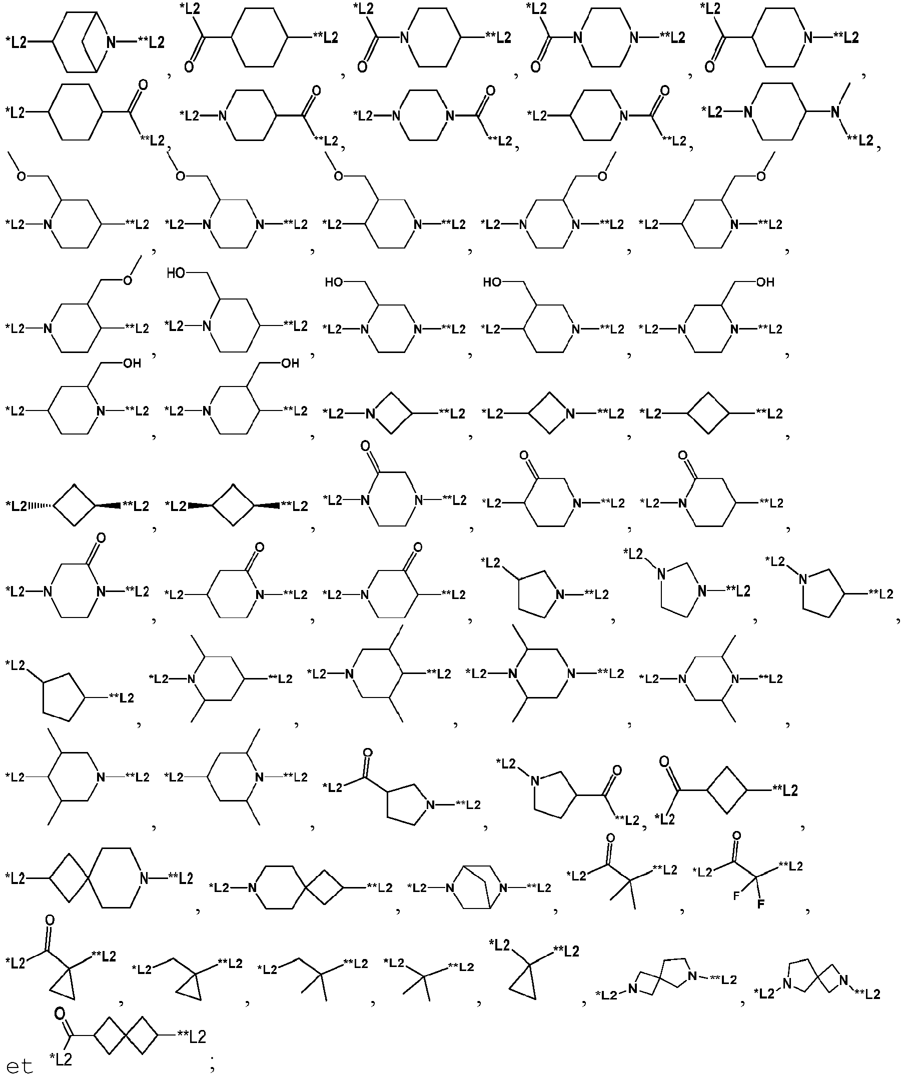 et/ou
(ii) m₃ est 0, 1, 2, 3, 4, 5 ou 6 ;
et/ou
(iii) L³ est choisi parmi une liaison simple, -O-, -SO₂-, - CO-, -NR^{L3a}-, - (cycloalkylène en C₃-C₈) -, *^{L3}-O- (alkylène en C₁₋₈)-**^{L3}, *^{L3}- (alkylène en C₁₋₈)-O-**^{L3}, *^{L3}-SO₂- (alkylène en C₁₋₈)-**^{L3}, *^{L3}- (alkylène en C₁₋₈)-SO₂-**^{L3}, *^{L3}-CO- (alkylène en C₁₋₈)-**^{L3}, *^{L3}- (alkylène en C₁₋₈)-CO-**^{L3}, *^{L3}-NR^{L3a}-(alkylène en C₁₋₈) -**^{L3}, *^{L3}- (alkylène en C₁₋₈) -NR^{L3a}-**^{L3}, *^{L3}-NR^{L3a}C(O)-**^{L3}, *^{L3}-C(O)NR^{L3a}-**^{L3}, -(alkylène en C₁₋₈)-, - (alcénylène en C₂₋₈)-, - (alcynylène en C₂₋₈)-, -[O(CR^{L3a}R^{L3b})ₘ₄]ₘ₅- 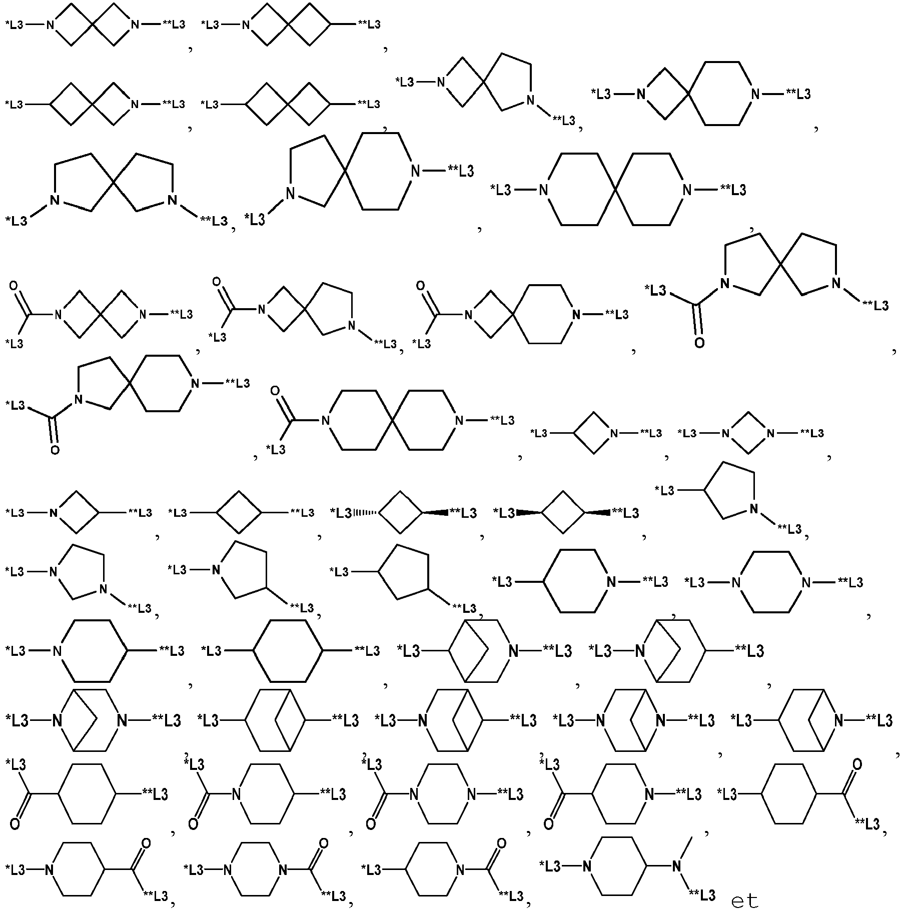 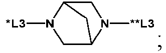
où chacun desdits - (cycloalkylène en C₃-C₈) -, *^{L3}-O-(alkylène en C₁₋₈)-**^{L3}, *^{L3}- (alkylène en C₁₋₈)-O-**^{L3}, *^{L3}-SO₂-(alkylène en C₁₋₈)-**^{L3}, *^{L3}- (alkylène en C₁₋₈)-SO₂-**^{L3}, *^{L3}-CO-(alkylène en C₁₋₈)-**^{L3}, *^{L3}- (alkylène en C₁₋₈)-CO-**^{L3}, *^{L3}-NR^{L3a}-(alkylène en C₁₋₈)-**^{L3}, *^{L3}- (alkylène en C₁₋₈)-NR^{L3a}-**^{L3}, - (alkylène en C₁₋₈)-, - (alcénylène en C₂₋₈)-, - (alcynylène en C₂₋₈)-, 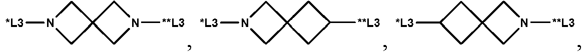 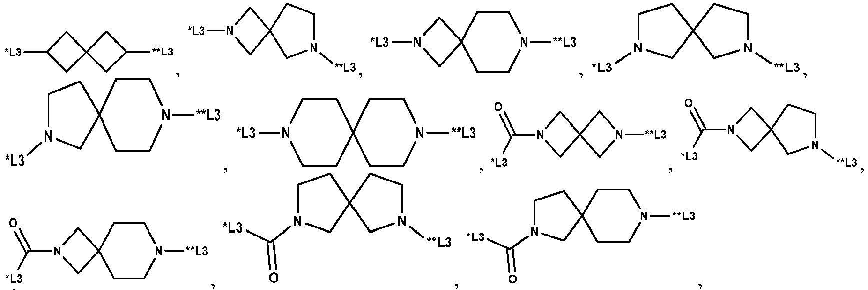 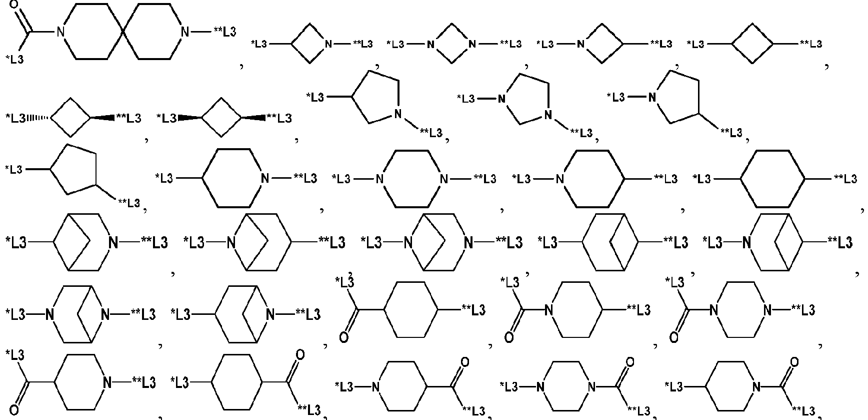 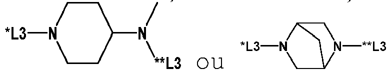 est éventuellement substitué par au moins un substituant R^{L3c} ;
R^{L3a} et R^{L3b} sont chacun indépendamment choisis parmi hydrogène, méthyle, éthyle, propyle, butyle, pentyle, hexyle, heptyle, octyle, vinyle, éthynyle, cyclopropyle, cyclobutyle, cyclopentyle, cyclohexyle, cycloheptyle, cyclooctyle, oxazolidinyle, imidazolidinyle, thiazolidinyle, pyrazolidinyle, morpholinyle, pipéridinyle, pipérazinyle, oxazinyle, imidazolyle, thiazolyle, oxazolyle, pyridyle, pyrimidinyle, pyrazinyle, pyridazinyle, phényle, pyrrolyle, imidazolyle, pyrazolyle, thiazolyle, oxazolyle, triazolyle, thiophényle, furanyle, pyridyle, pyrimidinyle et pyrazinyle, où chacun desdits méthyle, éthyle, propyle, butyle, pentyle, hexyle, heptyle, octyle, vinyle, éthynyle, cyclopropyle, cyclobutyle, cyclopentyle, cyclohexyle, cycloheptyle, cyclooctyle, oxazolidinyle, imidazolidinyle, thiazolidinyle,
pyrazolidinyle, morpholinyle, pipéridinyle, pipérazinyle, oxazinyle, imidazolyle, thiazolyle, oxazolyle, pyridyle, pyrimidinyle, pyrazinyle, pyridazinyle, phényle, pyrrolyle, imidazolyle, pyrazolyle, thiazolyle, oxazolyle, triazolyle, thiophényle, furanyle, pyridyle, pyrimidinyle ou pyrazinyle est éventuellement substitué par au moins un substituant R^{L3d} ; et
chacun desdits R^{L3c} et R^{L3d} est indépendamment -F, -Cl, -Br, -I, -OH, méthyle, éthyle, propyle, butyle, pentyle, hexyle, heptyle, octyle, vinyle, éthynyle, cyclopropyle, cyclobutyle, cyclopentyle, cyclohexyle, cycloheptyle, cyclooctyle, oxazolidinyle, imidazolidinyle, thiazolidinyle, pyrazolidinyle, morpholinyle, pipéridinyle, pipérazinyle, oxazinyle, imidazolyle, thiazolyle, oxazolyle, pyridyle, pyrimidinyle, pyrazinyle, pyridazinyle, phényle, pyrrolyle, imidazolyle, pyrazolyle, thiazolyle, oxazolyle, triazolyle, thiophényle, furanyle, pyridyle, pyrimidinyle, pyrazinyle ou oxo ;
et/ou
(iv) L³ est choisi parmi une liaison simple, - (alkylène en C₁₋₈)- (de préférence -CH₂-, -C₂H₄-, -C₃H₆-), -CO-, -O-, - N(CH₃)-, -NH-, 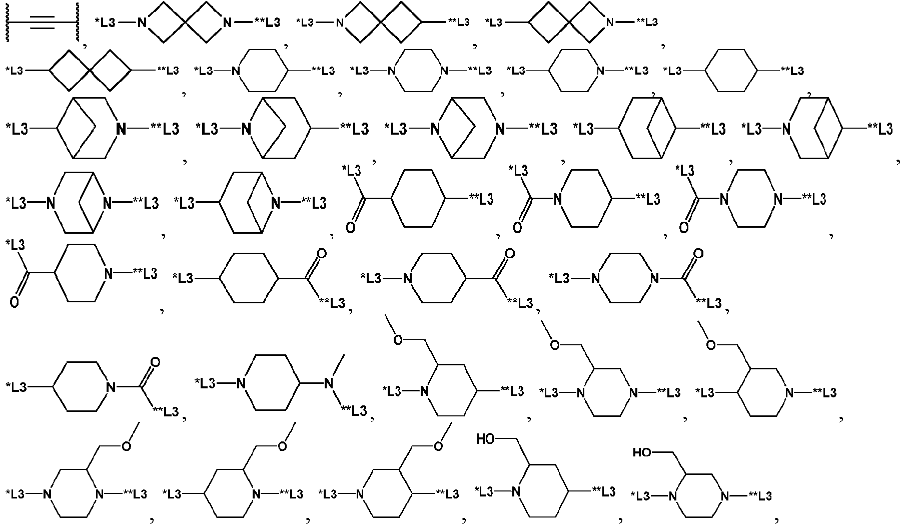 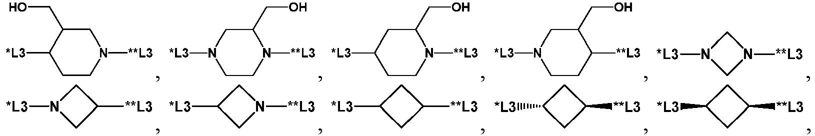 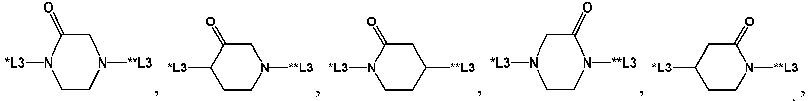 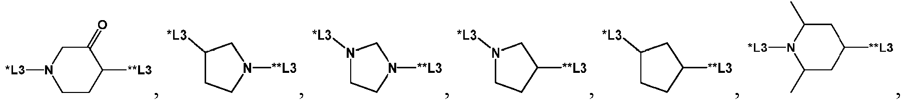 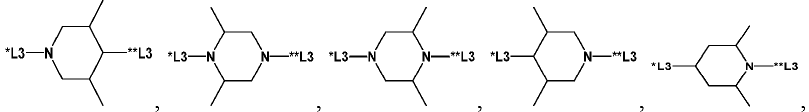 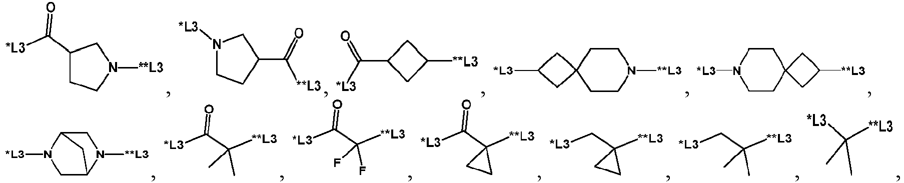 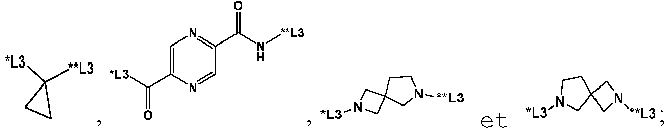 et/ou
(v) 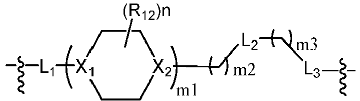 est choisi parmi -CH₂CH₂-, 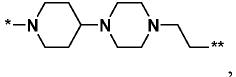 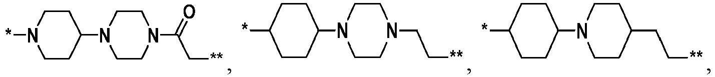 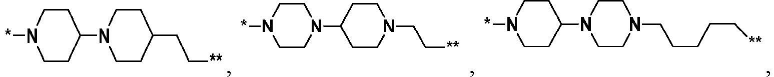 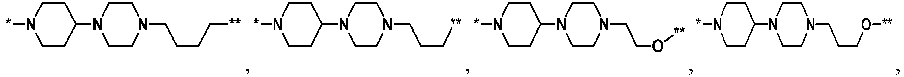 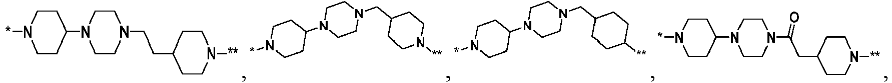 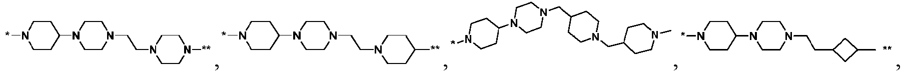 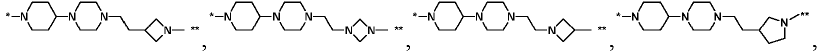 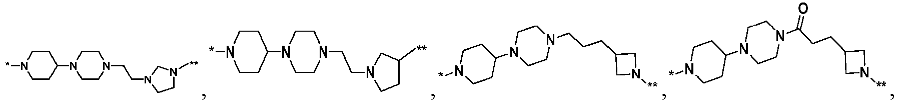 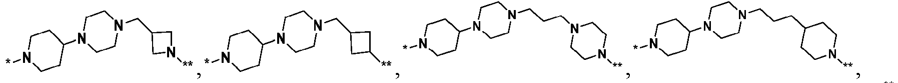 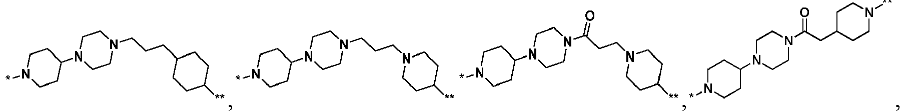 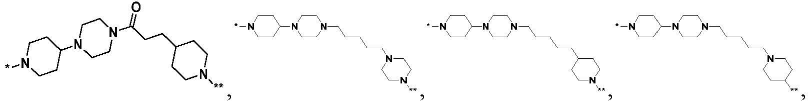 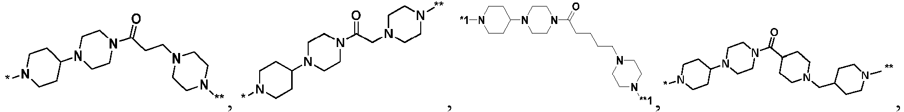 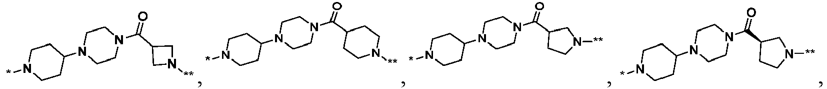 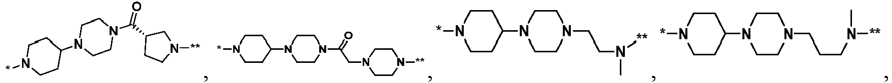 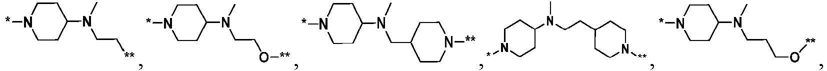 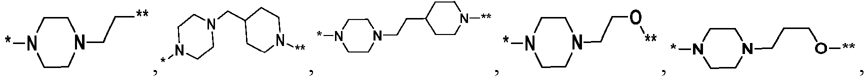 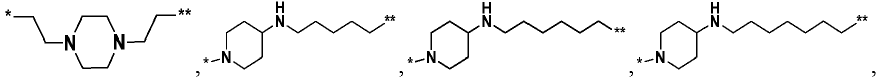 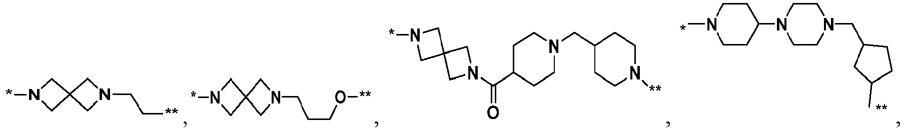 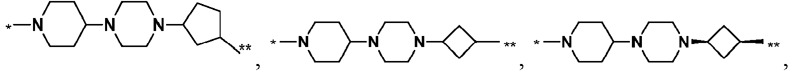 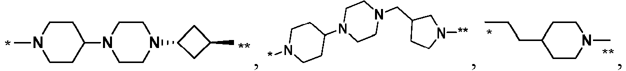 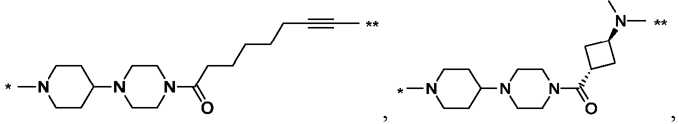 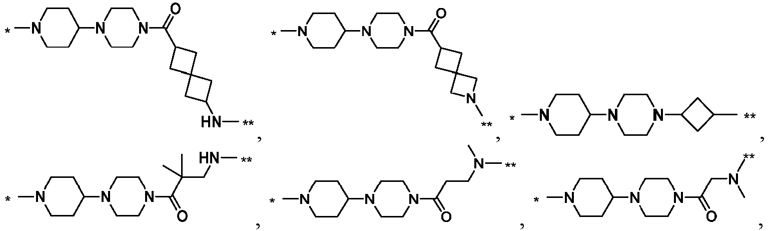 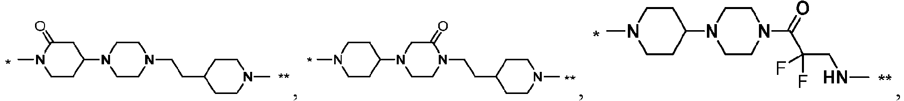 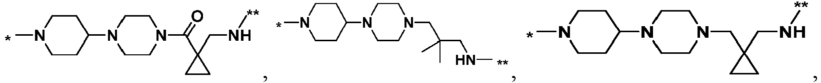 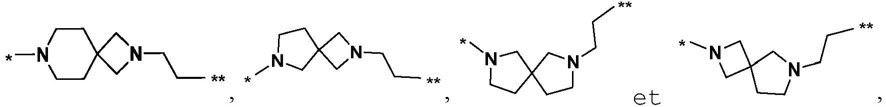 où * désigne la position liée au groupement 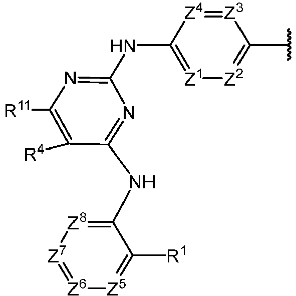 et ** désigne la position liée au groupement

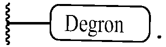

12. Composé selon l'une quelconque des revendications 1 à 11, dans lequel
(i) 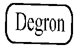 est choisi parmi : 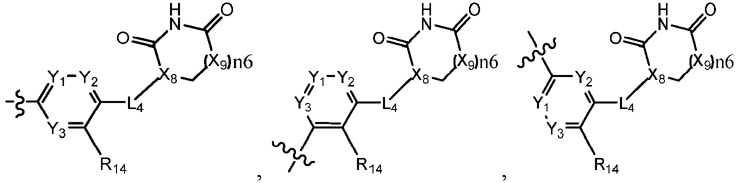 ou 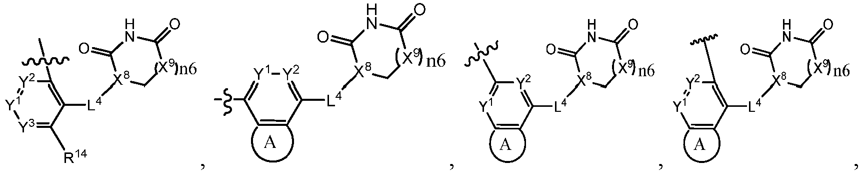 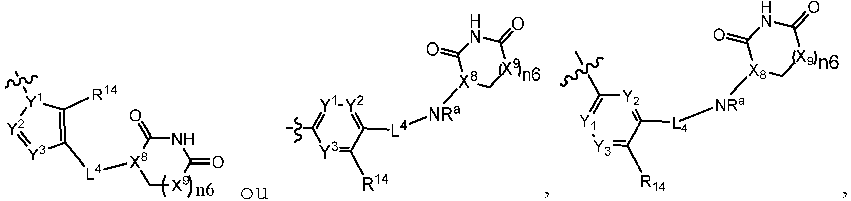 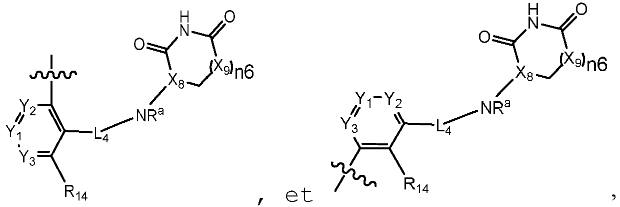
où le cycle A est choisi parmi un cycloalkyle de 5 à 7 chaînons, un hétérocyclyle de 5 à 7 chaînons, un aryle et un hétéroaryle ;
R¹⁴ est indépendamment choisi parmi hydrogène, halogène, -alkyle en C₁₋₈, -alcoxy en C₁₋₈ et CN ; chacun desdits -alkyle en C₁₋₈ ou -alcoxy en C₁₋₈ est éventuellement substitué par un ou plusieurs halogènes ou -alkyle en C₁₋₈ ; de préférence R¹⁴ est indépendamment choisi parmi H, F, Cl, Br, I, CH₃, -OCH₃, CH₂F, CN, CHF₂ et CF₃ ;
X⁸ est indépendamment choisi parmi CH, CD, C(CH₃), C(C₂H₅), C(C₃H₇), C(CN) et N ;
L⁴ est indépendamment choisi parmi une liaison simple, 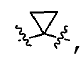 , -O-, -NH-, -CH₂-, -CHF- et -CF₂- ;
Y¹, Y² et Y³ sont chacun indépendamment choisis parmi CR^{a} et N ;
X⁹ est CH₂ ;
R^{a} est chacun indépendamment choisi parmi hydrogène, halogène, -alkyle en C₁₋₈ et -alcoxy en C₁₋₈, où chacun desdits -alkyle en C₁₋₈ ou -alcoxy en C₁₋₈ est éventuellement substitué par au moins un ou plusieurs halogène, hydroxy, halogène, -alkyle en C₁₋₈ ou -alcoxy en C₁₋₈ ; et
n₆ est indépendamment 0, 1 ou 2 ; 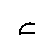 ou
(ii) 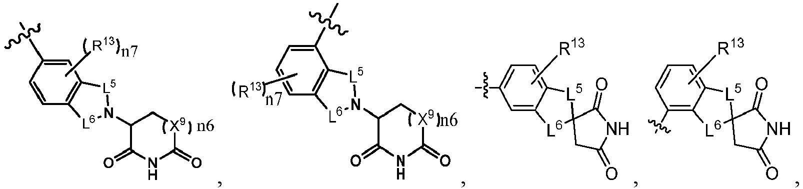 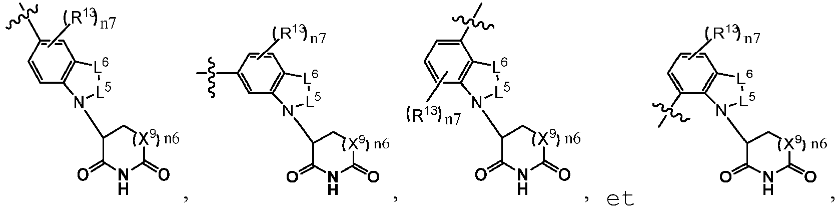
où L⁵ et L⁶ sont indépendamment choisis parmi une liaison simple, 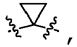 -O-, -NH-, -NMe-, -N(CH₂CH₃)-, -CH₂-, -CHF-, -CF₂-, -C(CH₃)₂- et -CO- (de préférence L⁵ est -CO- ou -CH₂-, et L⁶ est 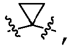 -O-, -NH-, -NMe-, -N(CH₂CH₃)-, -CH₂-, -CHF-, -CF₂-, -C(CH₃)₂- ou -CO-) ;
X⁹ est CH₂ ;
chaque R¹³ est indépendamment choisi parmi hydrogène, - F, -Cl, -Br, -I, CN, -alkyle en C₁₋₈ et -alcoxy en C₁₋₈ ;
n₆ est 0 ou 1 ; et
n₇ est 0, 1 ou 2 ;
ou
(iv) 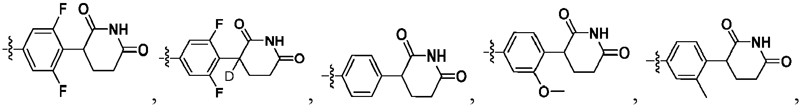 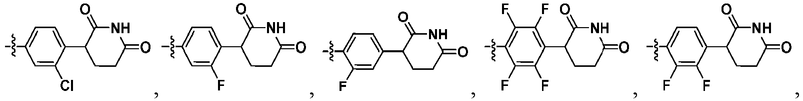 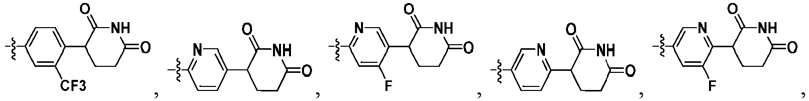 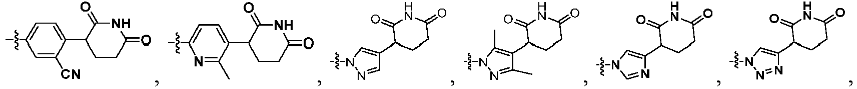 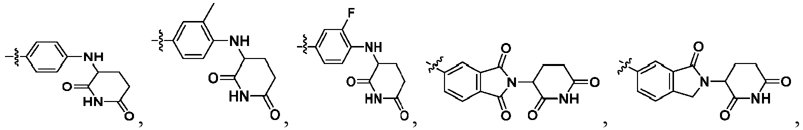 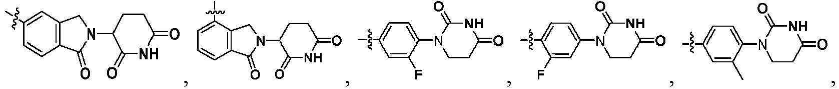 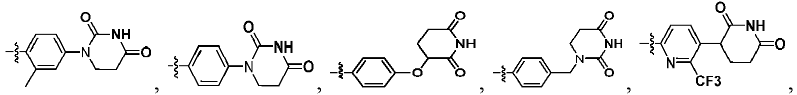 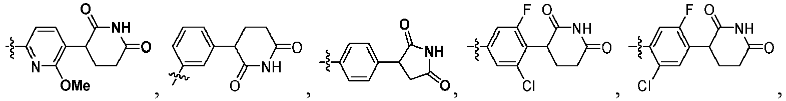 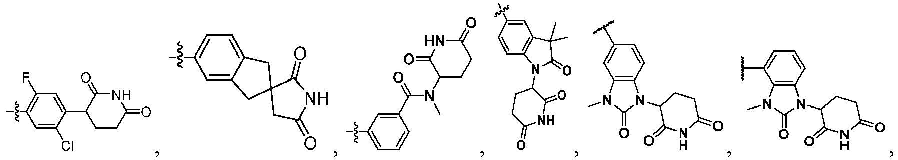 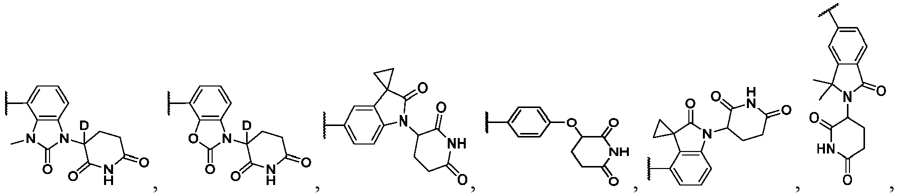 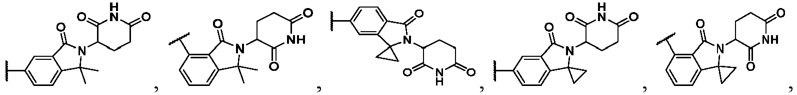 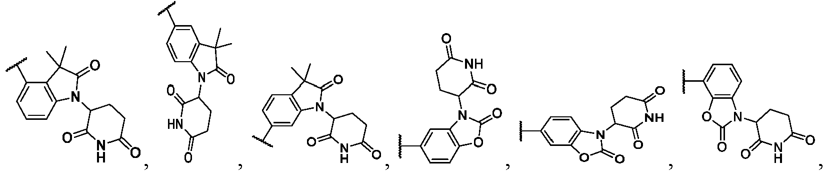 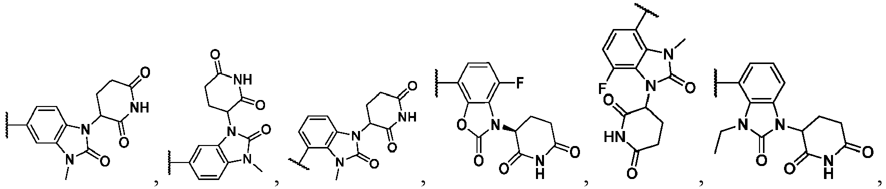 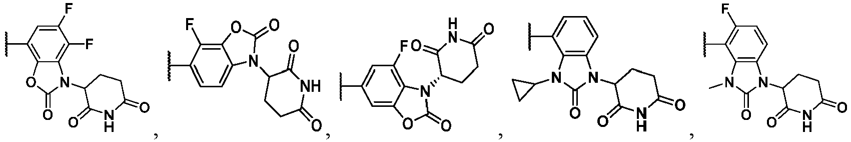 et

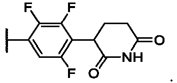

13. Composé selon l'une quelconque des revendications 1 a 12, dand lequel:
R^{z} est independent choisi parmi H, -CH₃, -C₂H_{5, F, -}CH₂F, -CHF₂, -CF₃, -OCH₃, -OC₂H₅, -OCH₂F, -OCHF₂, -OCH₂CF₃, -OCF₃, -SCF₃, -CF₃ et -CH(0H)CH₃.

14. Composé choisi parmi
| | |
|---|---|
| 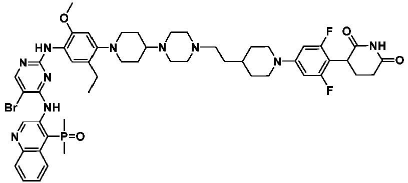 | 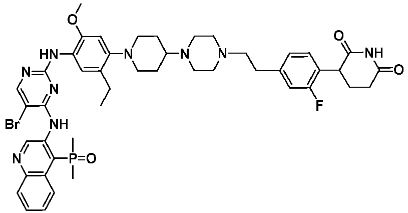 |
| 1 | 2 |
| 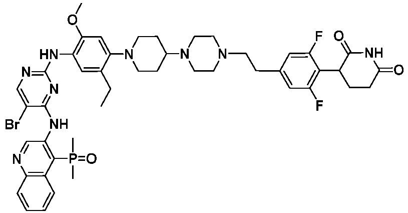 | 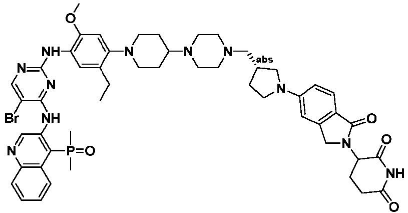 |
| 3 | 4 |
| 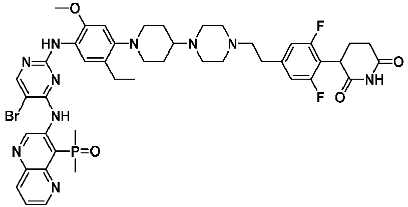 | 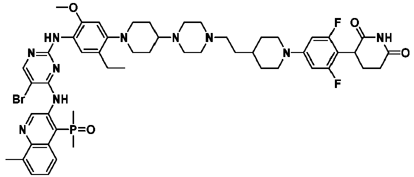 |
| 7 | 8 |
| 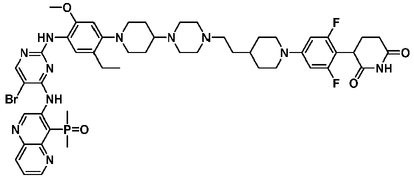 | 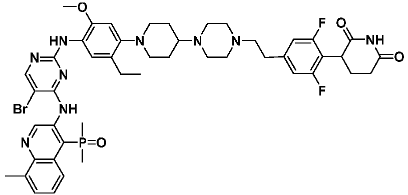 |
| 9 | 10 |
| 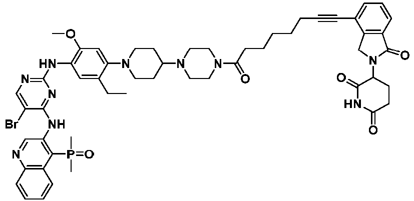 | 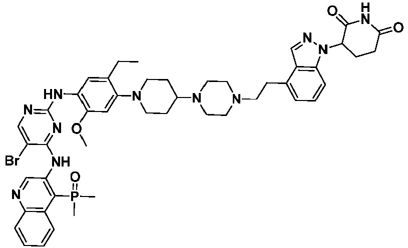 |
| 11 | 12 |
| 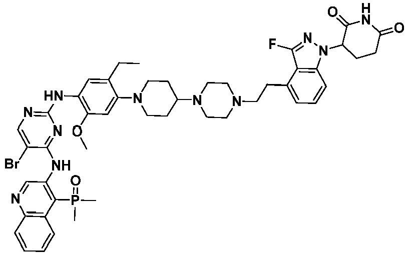 | 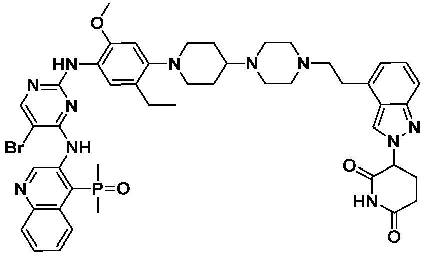 |
| 13 | 14 |
| 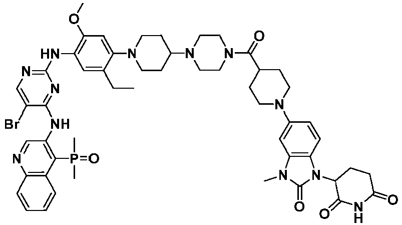 | 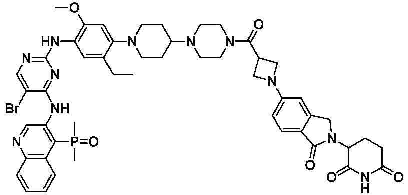 |
| 15 | 16 |
| 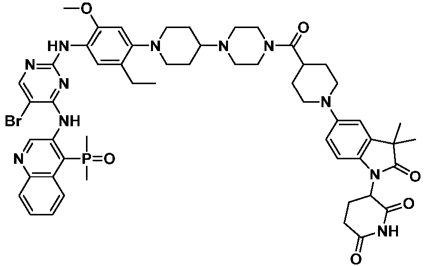 | 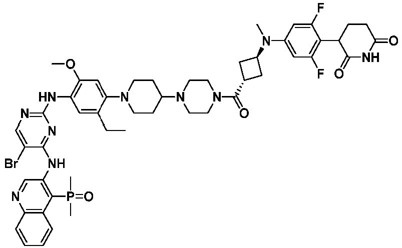 |
| 17 | 18 |
| 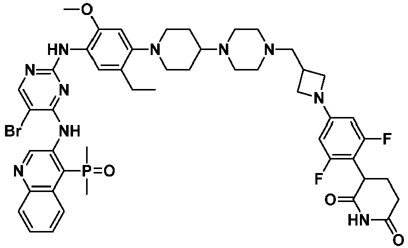 | 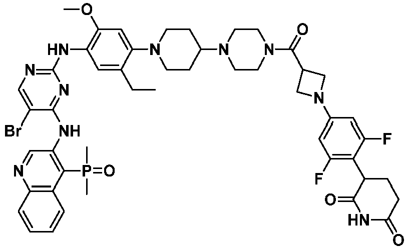 |
| 19 | 20 |
| 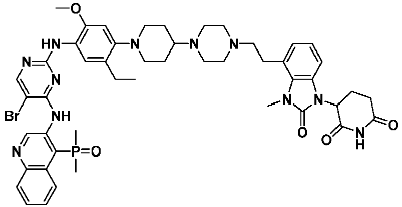 | 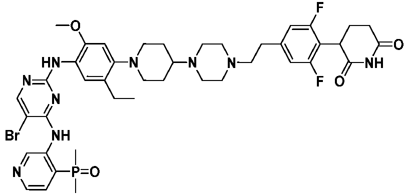 |
| 21 | 22 |
| 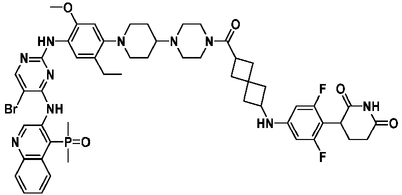 | 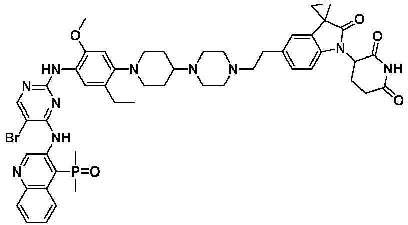 |
| 23 | 24 |
| 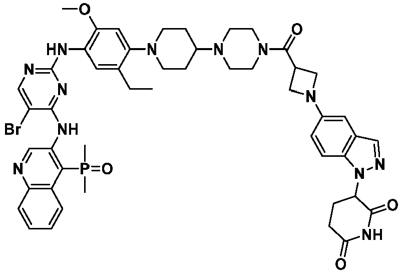 | 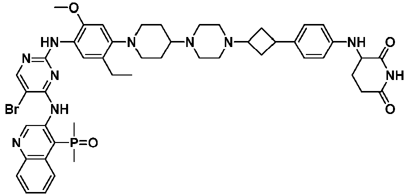 |
| 25 | 26 |
| 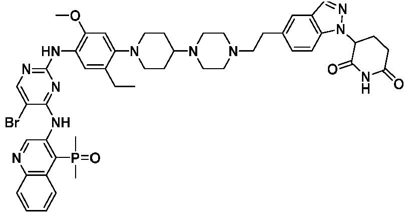 | 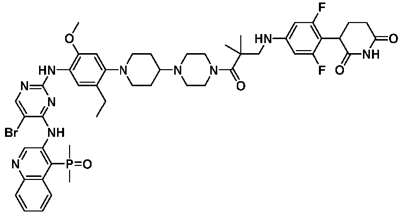 |
| 27 | 28 |
| 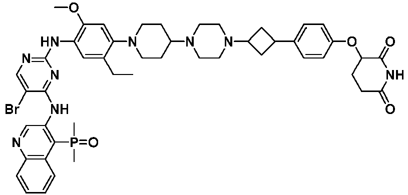 | 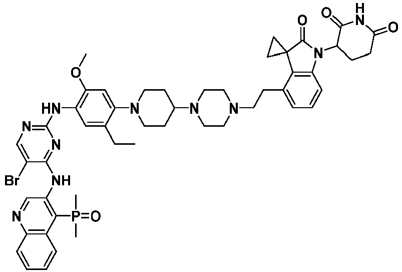 |
| 29 | 30 |
| 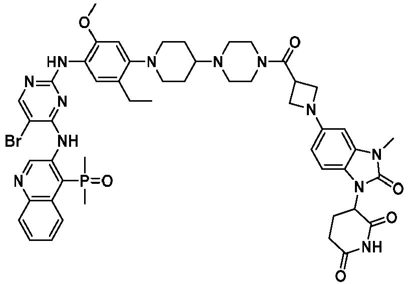 | 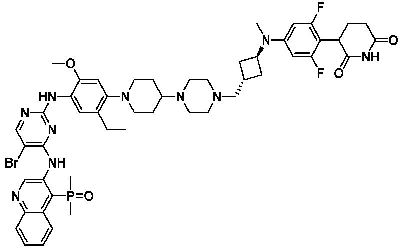 |
| 31 | 32 |
| 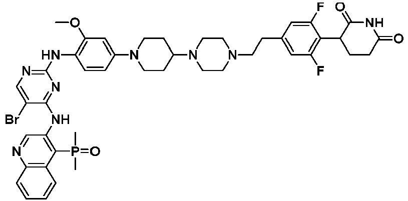 | 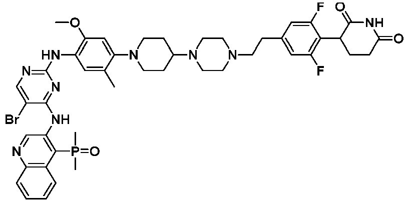 |
| 33 | 34 |
| 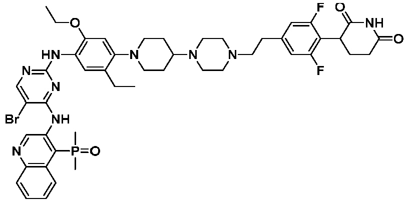 | 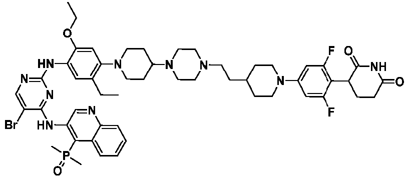 |
| 35 | 36 |
| 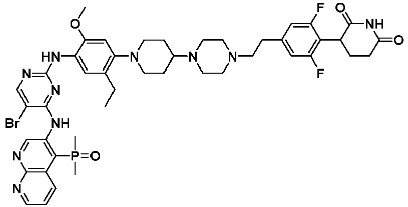 | 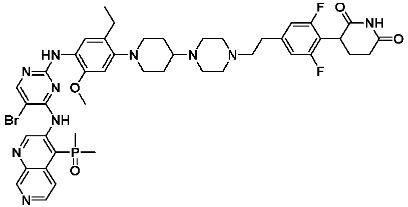 |
| 37 | 38 |
| | 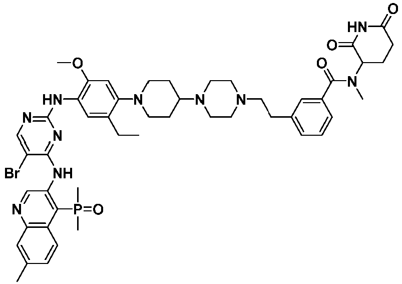 |
| | 40 |
| 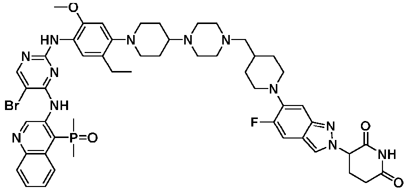 | 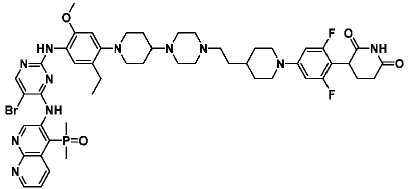 |
| 41 | 42 |
| 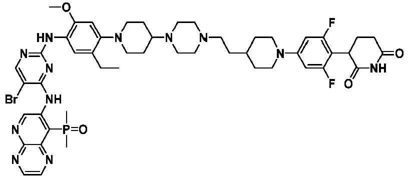 | 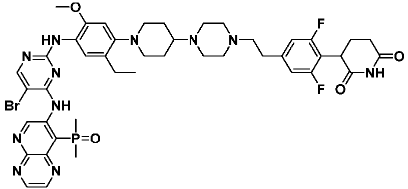 |
| 43 | 44 |
| 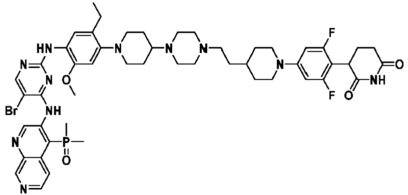 | 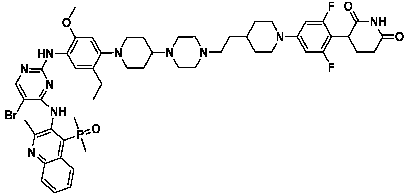 |
| 45 | 46 |
| 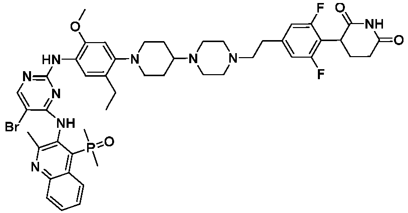 | 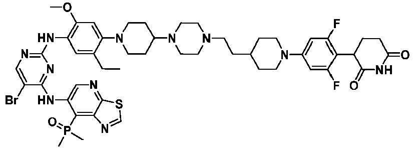 |
| 47 | 48 |
| 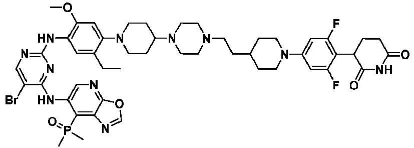 | 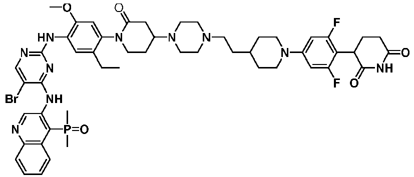 |
| 49 | 50 |
| 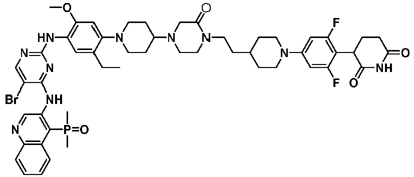 | 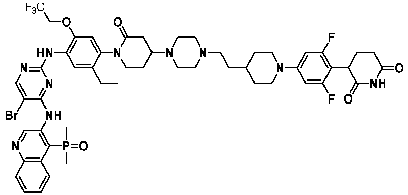 |
| 51 | 52 |
| 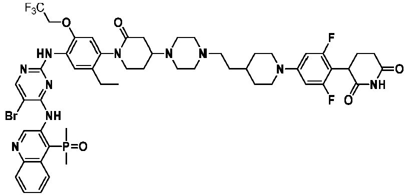 | 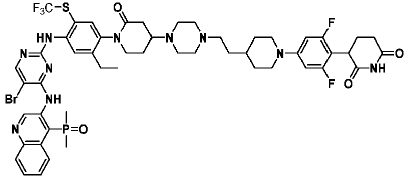 |
| 53 | 54 |
| 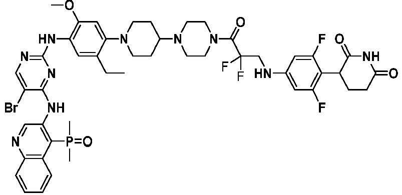 | 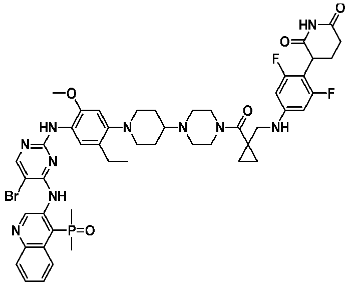 |
| 55 | 56 |
| 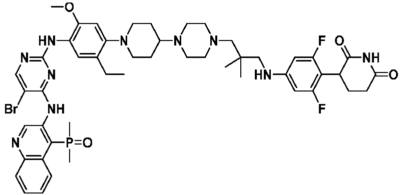 | 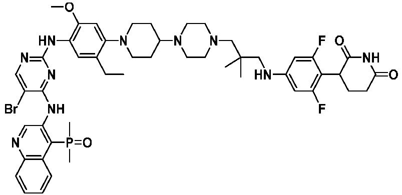 |
| 57 | 58 |
| 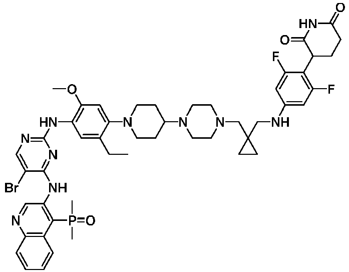 | 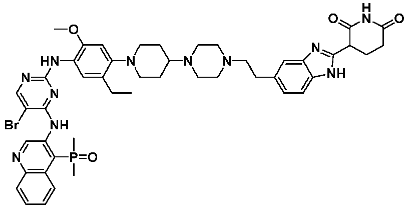 |
| 59 | 60 |
| 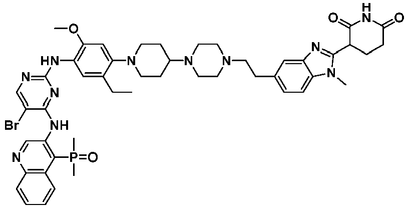 | 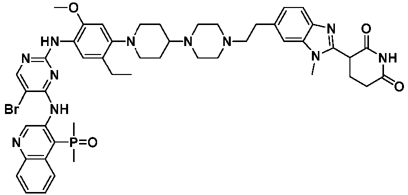 |
| 61 | 62 |
| 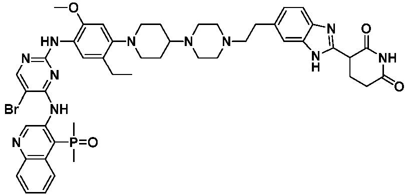 | 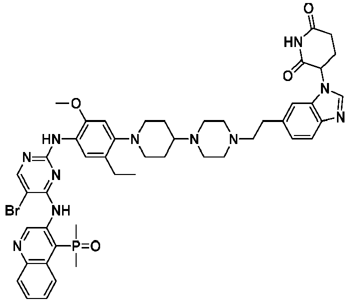 |
| 63 | 64 |
| 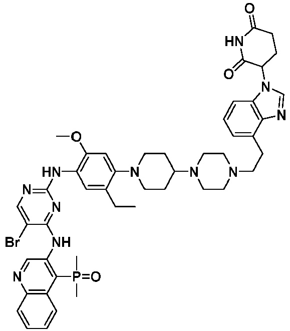 | 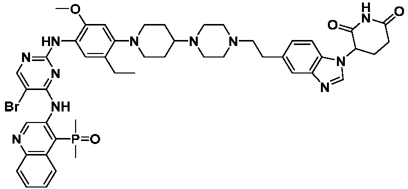 |
| 65 | 66 |
| 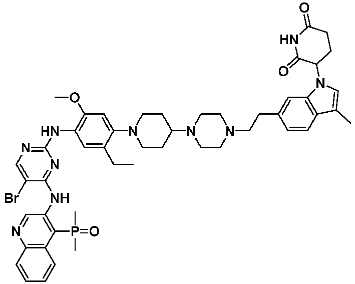 | 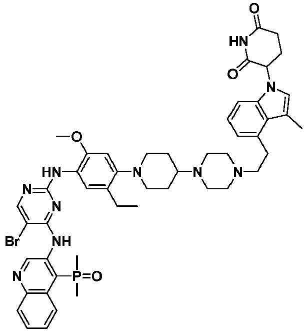 |
| 67 | 68 |
| 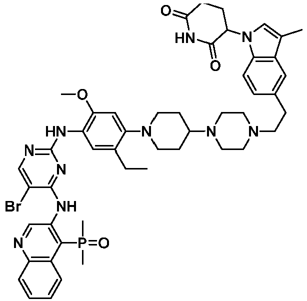 | 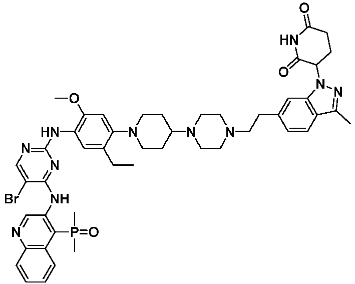 |
| 69 | 70 |
| 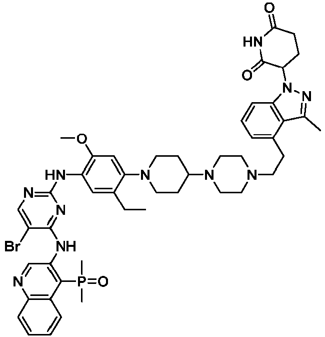 | 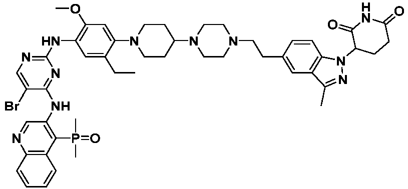 |
| 71 | 72 |
| 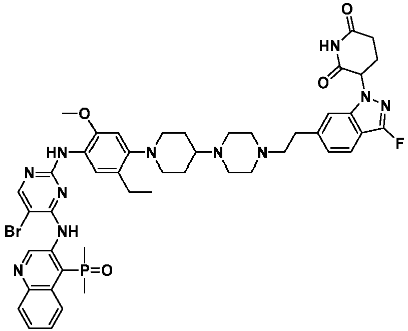 | 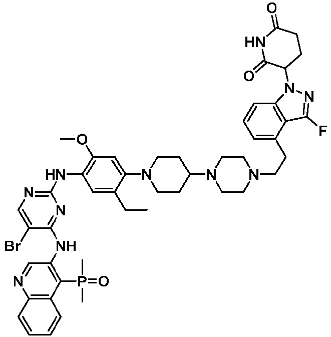 |
| 73 | 74 |
| 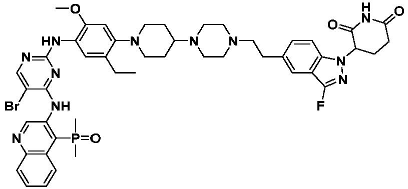 | 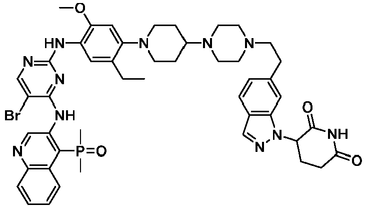 |
| 75 | 76 |
| 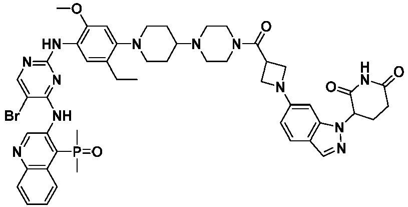 | 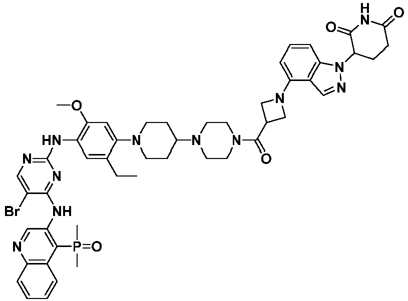 |
| 77 | 78 |
| 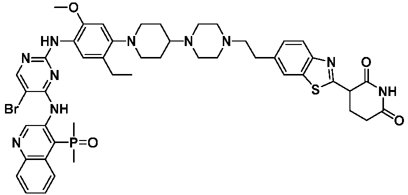 | 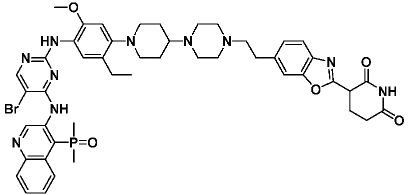 |
| 79 | 80 |
| 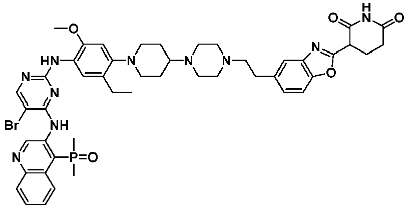 | 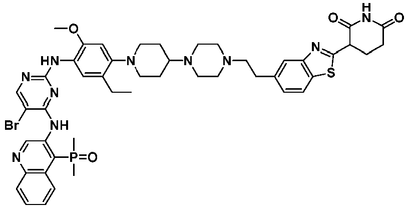 |
| 81 | 82 |
| 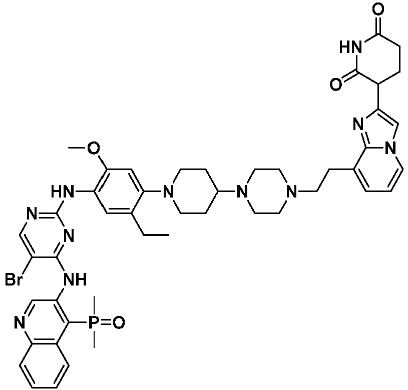 | 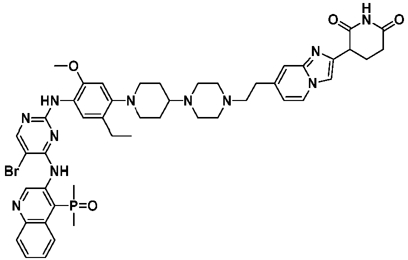 |
| 83 | 84 |
| 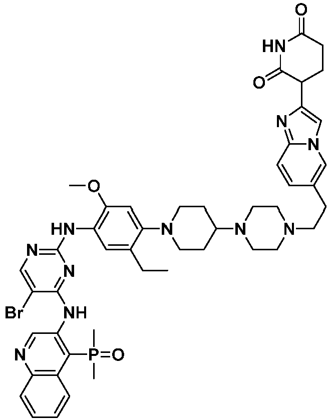 | 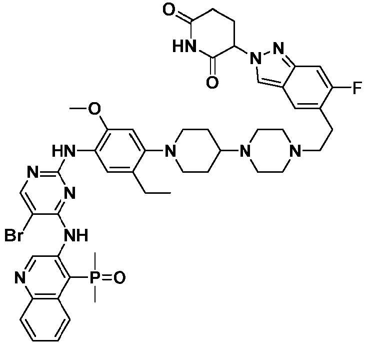 |
| 85 | 86 |
| 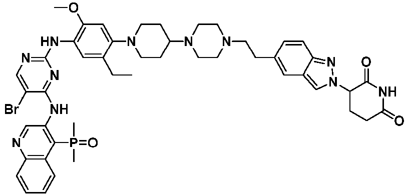 | 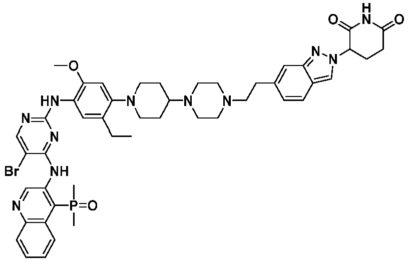 |
| 87 | 88 |
| 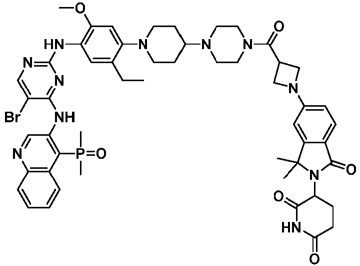 | 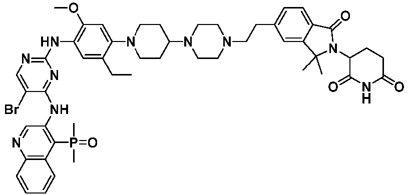 |
| 89 | 90 |
| 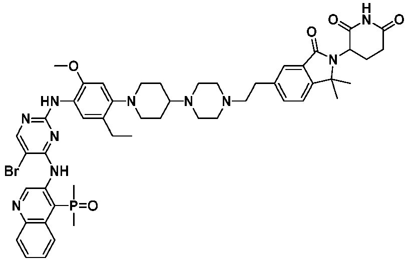 | 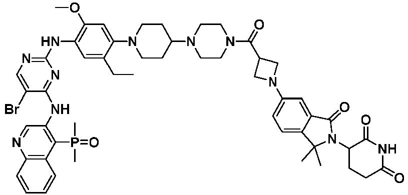 |
| 91 | 92 |
| 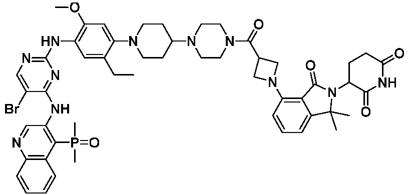 | 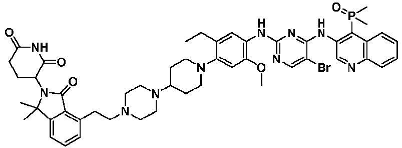 |
| 93 | 94 |
| 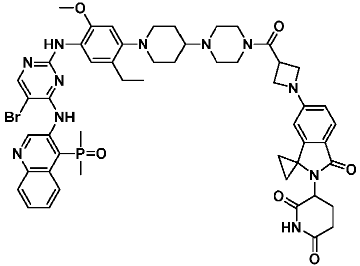 | 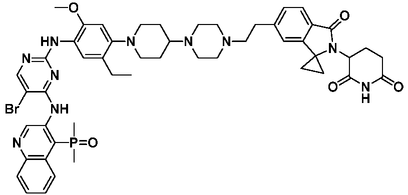 |
| 95 | 96 |
| 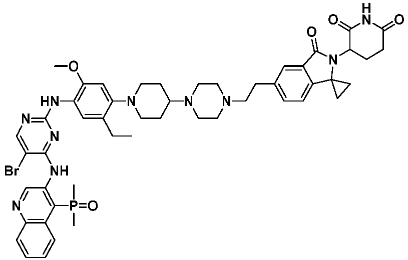 | 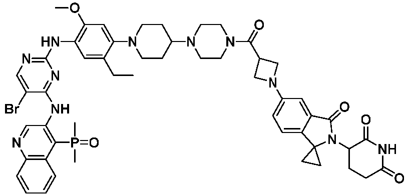 |
| 97 | 98 |
| 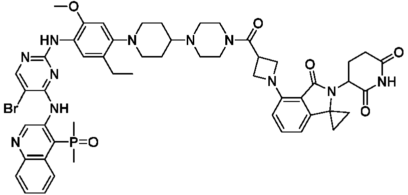 | 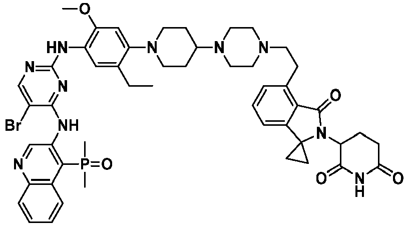 |
| 99 | 100 |
| 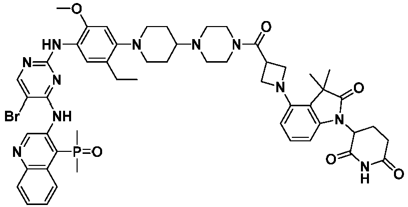 | 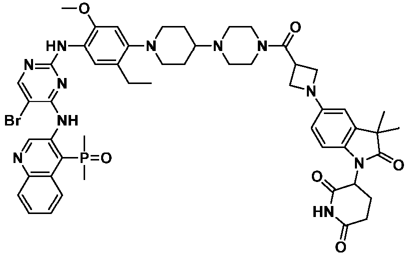 |
| 101 | 102 |
| 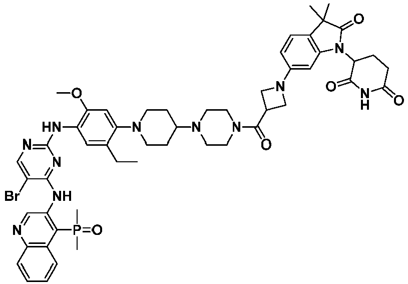 | 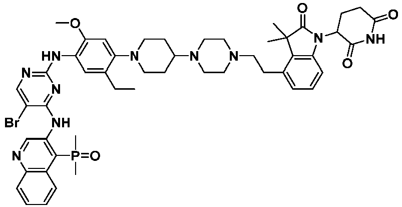 |
| 103 | 104 |
| 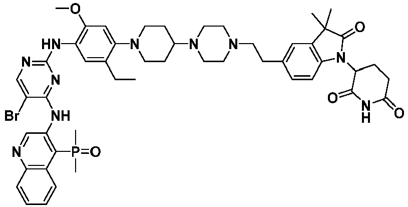 | 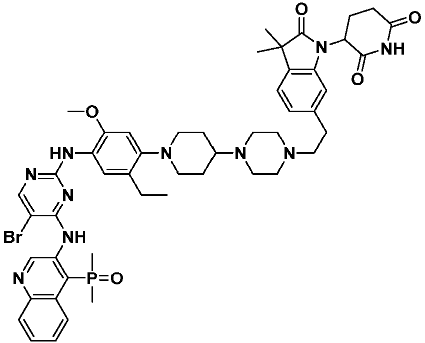 |
| 105 | 106 |
| 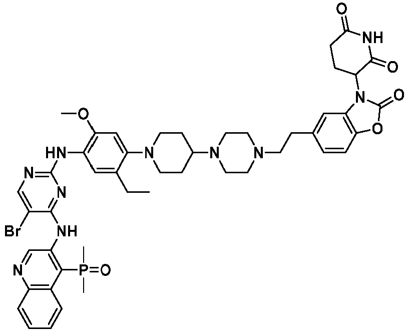 | 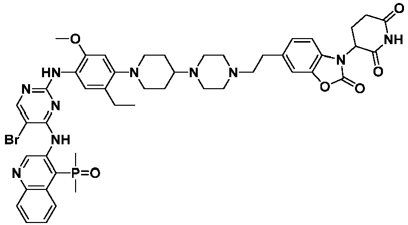 |
| 107 | 108 |
| 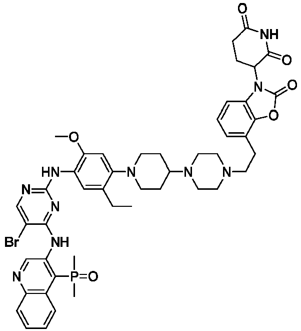 | 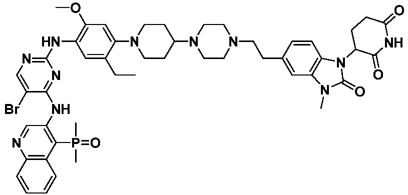 |
| 109 | 110 |
| 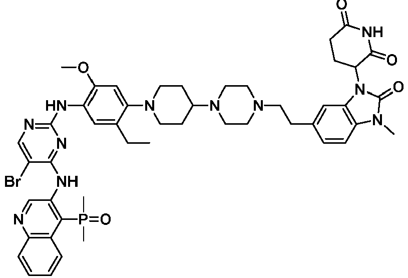 | 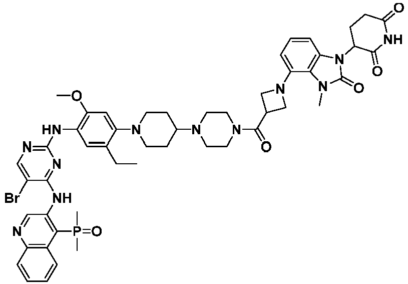 |
| 111 | 112 |
| 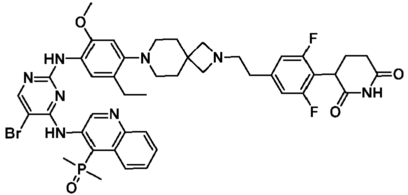 | 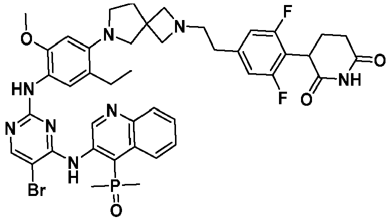 |
| 113 | 114 |
| 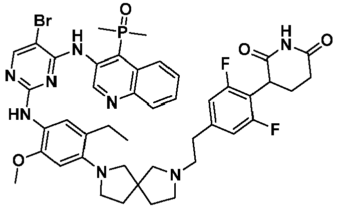 | 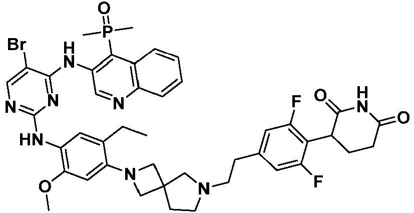 |
| 115 | 116 |
| 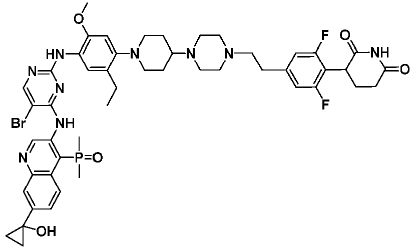 | 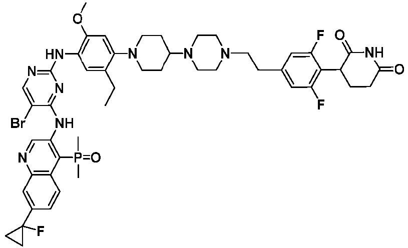 |
| 117 | 118 |
| 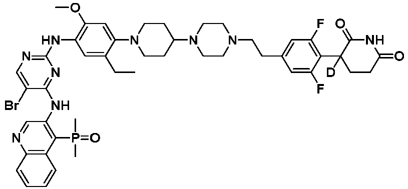 | 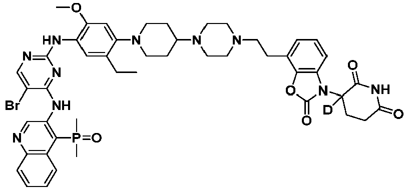 |
| 119 | 120 |
| 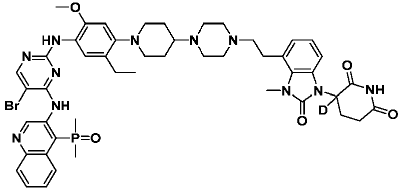 | 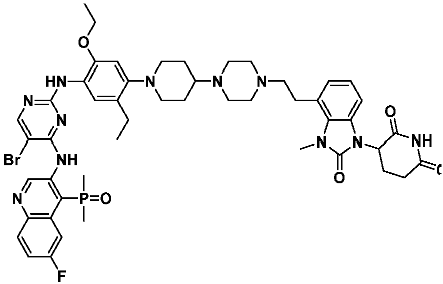 |
| 121 | 124 |
| 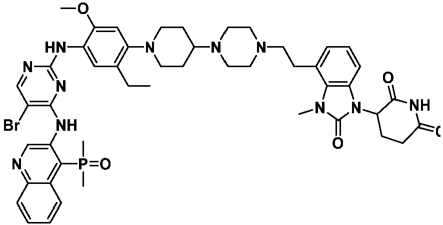 | 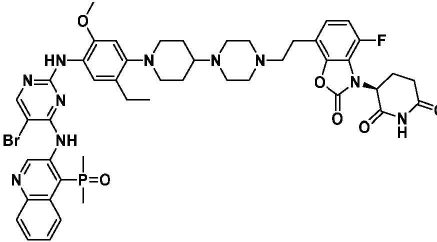 |
| 125 | 126 |
| 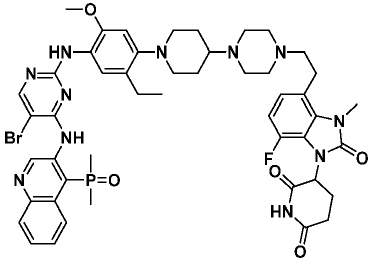 | 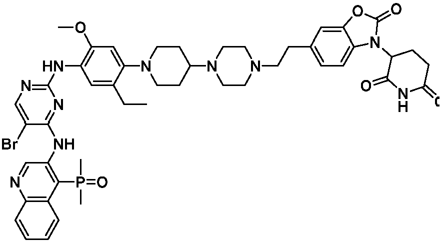 |
| 130 | 132 |
| 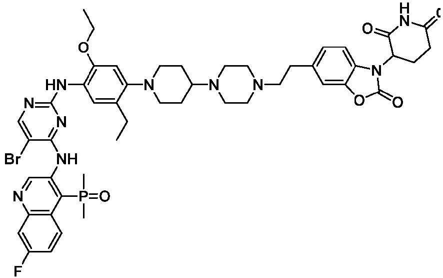 | 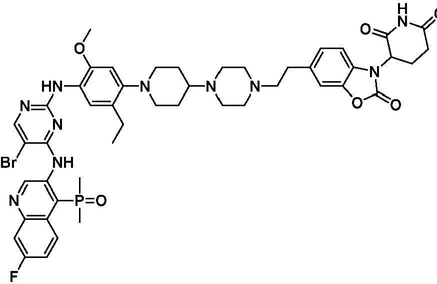 |
| 133 | 134 |
| 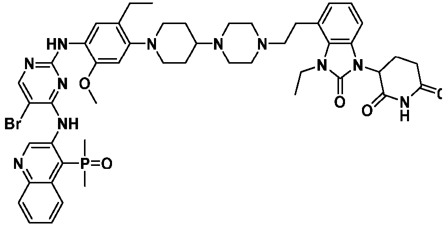 | 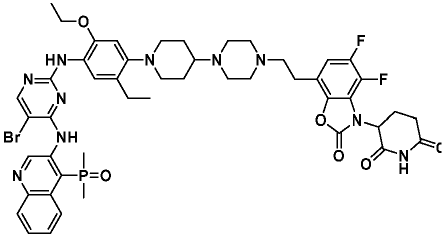 |
| 135 | 136 |
| 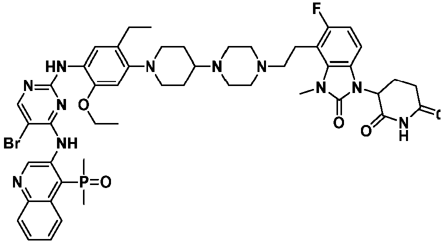 | 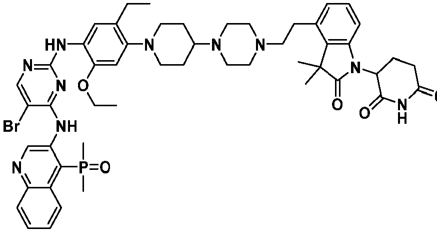 |
| 137 | 138 |
| 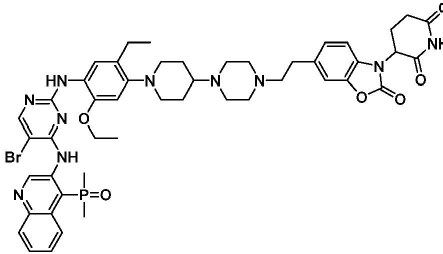 | 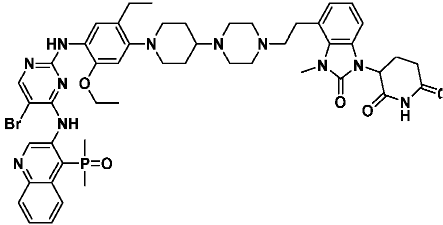 |
| 139 | 140 |
| 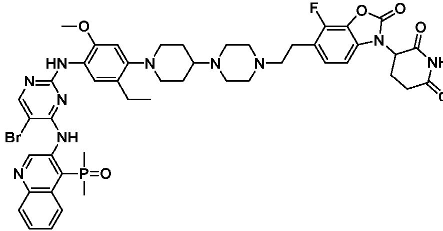 | 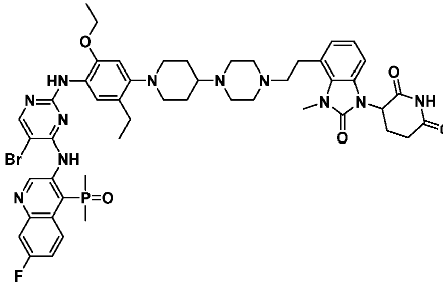 |
| 142 | 147 |
| 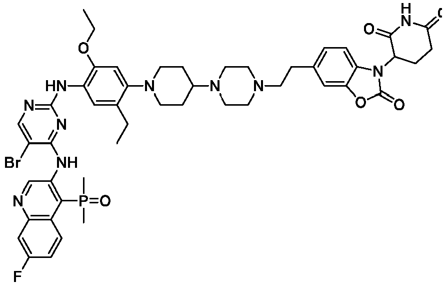 | 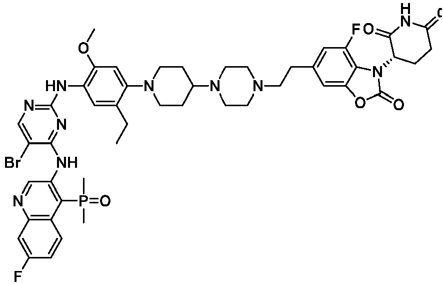 |
| 149 | 151 |
| 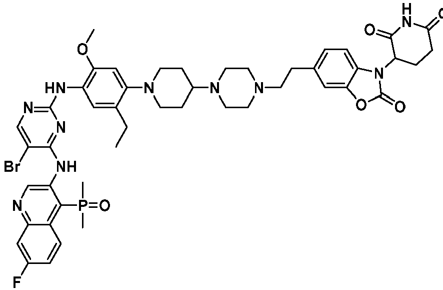 | 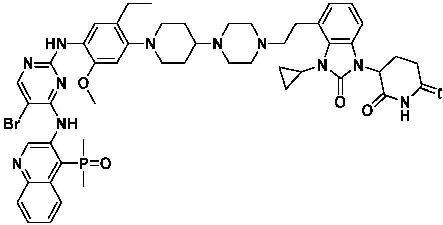 |
| 153 | 155 |
| | 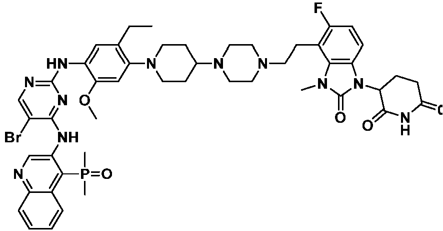 |
| | 159 |
| 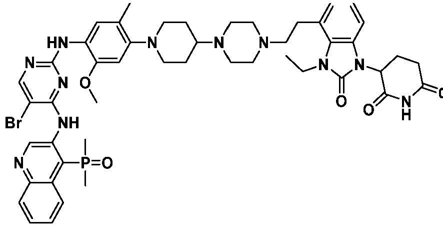 | 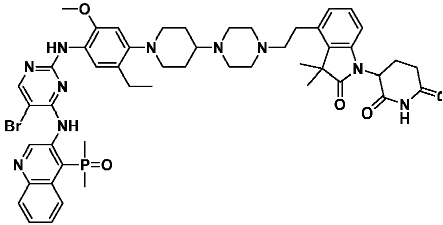 |
| 163 | 164 |
| 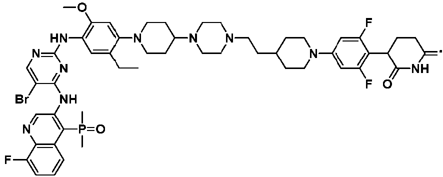 | 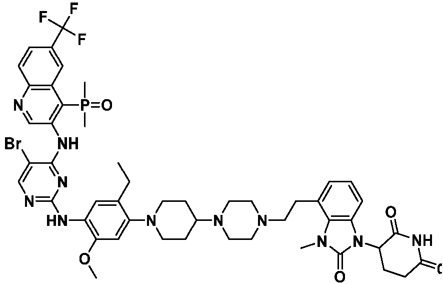 |
| 165 | 166 |
| 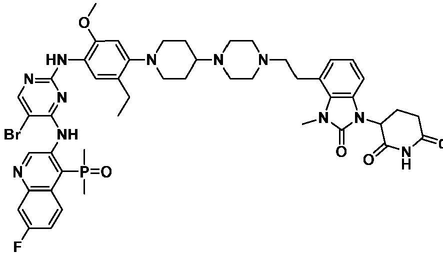 | 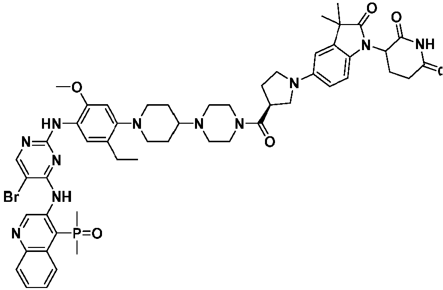 |
| 167 | 168 |
| 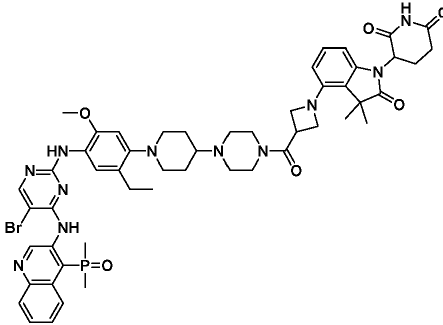 | 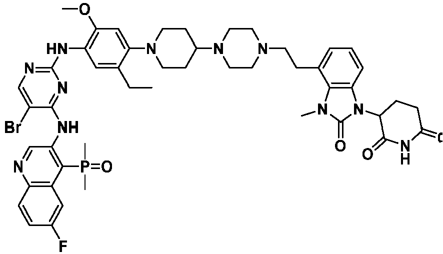 |
| 169 | 170 |
| 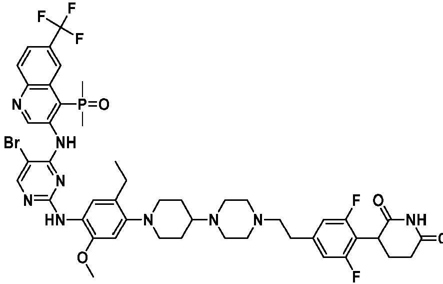 | 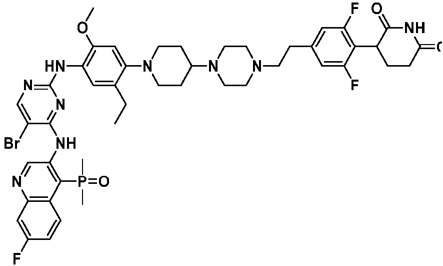 |
| 171 | 172 |
| 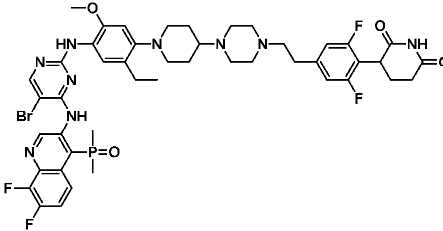 | 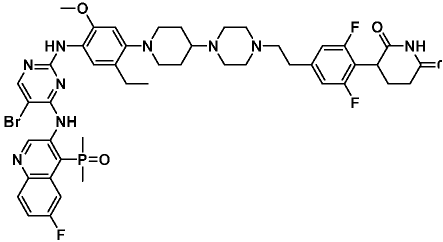 |
| 173 | 174 |
| 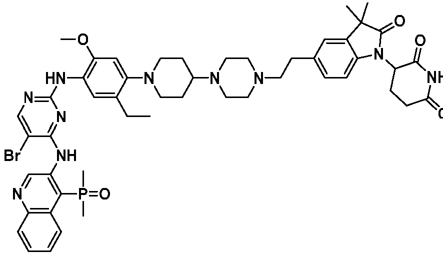 | 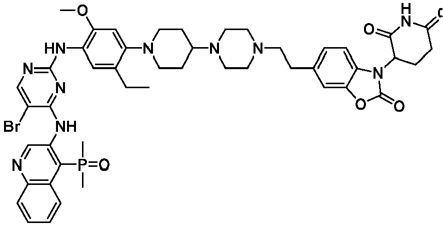 |
| 175 | 176 |
| | 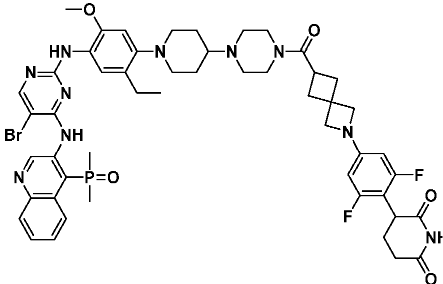 |
| | 178 |
| 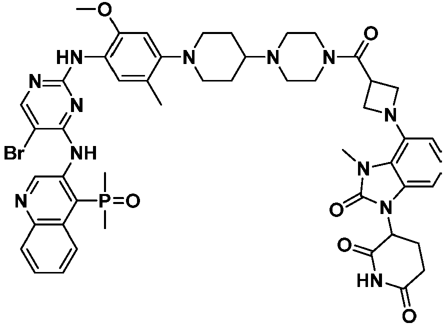 | 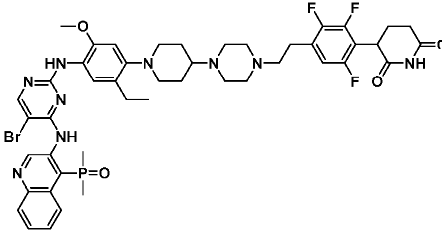 |
| 179 | 180 |
| 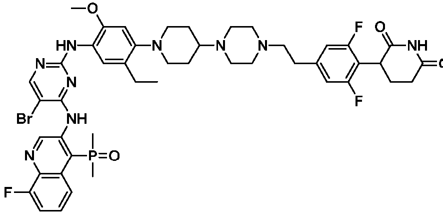 | 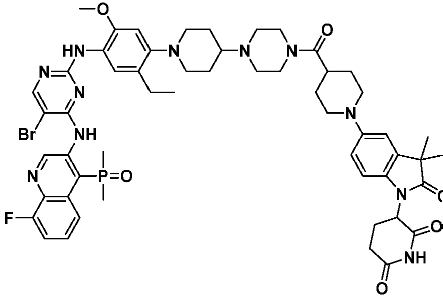 |
| 181 | 182 |
| 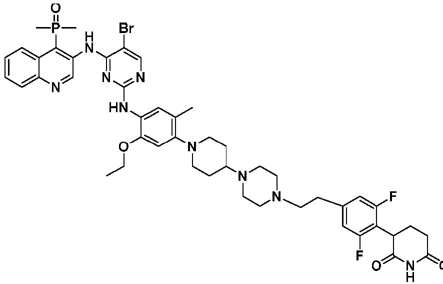 | 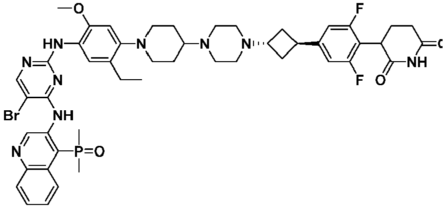 |
| 183 | 184 |
| 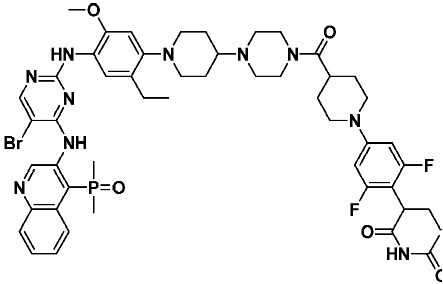 | 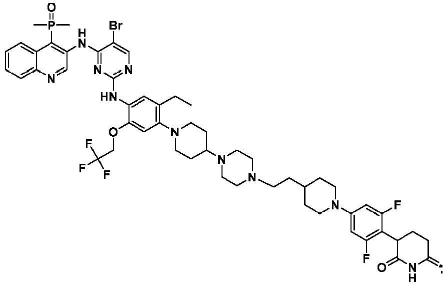 |
| 185 | 186 |
| 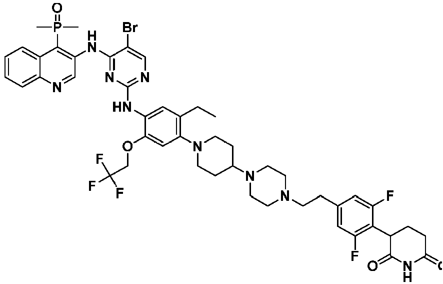 | 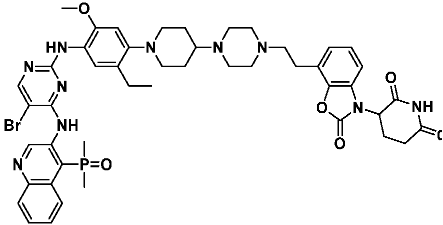 |
| 187 | 188, et |
| 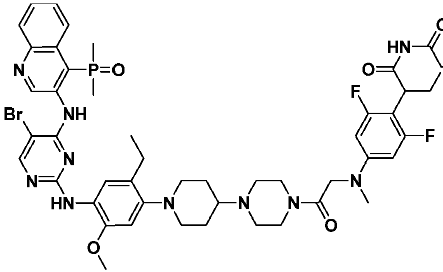 | |
| 189 | |

15. Composé selon l'une quelconque des revendications précédentes, pour une utilisation dans une méthode visant à :
(i) diminuer l'activité de l'EGFR par inhibition et/ou dégradation, qui comprend l'administration à un individu du composé, ou d'un sel pharmaceutiquement acceptable de celui-ci ;
ou
(ii) traiter une maladie ou un trouble chez un patient, comprenant l'administration au patient d'une quantité thérapeutiquement efficace du composé, ou d'un sel pharmaceutiquement acceptable de celui-ci, en tant qu'inhibiteur et/ou agent de dégradation de la kinase de l'EGFR, où la maladie ou le trouble est associé à l'inhibition de l'EGFR.

16. Composé pour une utilisation selon la revendication 15, dans lequel la maladie est choisie parmi un cancer, de préférence un cancer du pancréas, un cancer du sein, un glioblastome multiforme, un cancer de la tête et du cou et un cancer du poumon non à petites cellules.
